# EUROPEAN PATENT SPECIFICATION

(11) **EP 4 161 642 B1**
(45) Date of publication and mention of the grant of the patent: **27.05.2026**
(21) Application number: 21733165.1
(22) Date of filing: 04.06.2021
(51) Int. Cl.: A61P 9/10, A61P 11/06, A61P 35/00, A61P 37/02, C07D 451/00, C07D 471/08, C07D 487/04, A61K 31/46

(54) **N-PHENYLAMINOCARBONYL PYRIDINO- , PYRIMIDINO AND BENZO-TROPANES AS MODULATORS OF GPR65**
N-PHENYLAMINOCARBONYL PYRIDINO-, PYRIMIDINO UND BENZO-TROPANE ALS GPR65 MODULATOREN
PYRIDINO-, PYRIMIDINO- ET BENZO-TROPANES N-PHENYLAMINOCARBONYLÉS EN TANT QUE MODULATEURS DU GPR65

(30) Priority: 05.06.2020 GB 202008531; 18.01.2021 GB 202100645; 08.04.2021 GB 202105000
(43) Date of publication of application: 12.04.2023
(73) Proprietor: Pathios Therapeutics Limited, Milton, Abingdon OX14 4RY (GB)
(72) Inventor: MCCARTHY, Tom, Oxford, OX2 6HJ (GB); MILNE, Gavin, Oxford, OX2 6HJ (GB); MOECHEL, Tobias, Oxford, OX2 6HJ (GB); NAYLOR, Alan, Oxford, OX2 6HJ (GB); MILLER, David, Oxford, OX2 6HJ (GB)
(74) Representative: D Young & Co LLP
(86) International application number: PCT/GB2021/051397
(87) International publication number: WO 2021/245427

(56) References cited:
- WO-A1-2008/101354
- HUANG XI-PING ET AL: "Allosteric ligands for the pharmacologically dark receptors GPR68 and GPR65", vol. 527, no. 7579, 1 November 2015 (2015-11-01), London, pages 477 - 483, XP055830467, ISSN: 0028-0836, Retrieved from the Internet <URL:https://www.ncbi.nlm.nih.gov/pmc/articles/PMC4796946/pdf/nihms760070.pdf> DOI: 10.1038/nature15699
- DATABASE REGISTRY [online] CHEMICAL ABSTRACTS SERVICE, COLUMBUS, OHIO, US; 8 January 2020 (2020-01-08), UKRORGSYNTEZ LTD., XP002803888, Database accession no. 2402662-26-2
- DATABASE REGISTRY [online] CHEMICAL ABSTRACTS SERVICE, COLUMBUS, OHIO, US; 26 January 2017 (2017-01-26), AURORA FINE CHEMICALS, XP002803889, Database accession no. 2059740-21-3
- DATABASE REGISTRY [online] CHEMICAL ABSTRACTS SERVICE, COLUMBUS, OHIO, US; 25 January 2017 (2017-01-25), AURORA FINE CHEMICALS, XP002803890, Database accession no. 2058989-08-3
- DATABASE REGISTRY [online] CHEMICAL ABSTRACTS SERVICE, COLUMBUS, OHIO, US; 5 May 2016 (2016-05-05), AURORA FINE CHEMICALS, XP002803891, Database accession no. 1904233-31-3
- DATABASE REGISTRY [online] CHEMICAL ABSTRACTS SERVICE, COLUMBUS, OHIO, US; 5 May 2016 (2016-05-05), AURORA FINE CHEMICALS, XP002803892, Database accession no. 1903848-39-4

## Description

The present invention relates to compounds that are capable of modulating GPR65. The compounds have potential therapeutic applications in the treatment of a variety of disorders, including proliferative and immune disorders.

### BACKGROUND TO THE INVENTION

GPR65 is a Gs-coupled G protein-coupled receptor (GPCR) that is primarily expressed in immune cells and is activated by acidic extracellular pH to cause increases in cytoplasmic cyclic adenosine monophosphate (cAMP) (Wang, 2004). It has long been known that tumours typically undergo a switch in cellular metabolism from oxidative phosphorylation to aerobic glycolysis, which in turn results in an acidic extracellular microenvironment (Damaghi, 2013). Recently, it has been shown that this acidic microenvironment causes GPR65 activation in tumour-associated macrophages, resulting in an increase in cytoplasmic cAMP leading to transcription of the inducible cAMP early repressor (ICER). This, in turn, suppresses the secretion of tumour necrosis factor alpha (TNFα) to bias the macrophages toward an anti-inflammatory, tumour-permissive phenotype (Bohn, 2018). This GPR65-dependent pathway therefore appears to represent a mechanism by which tumours exploit their acidic microenvironment to evade detection by the immune system.

Autoimmune diseases are also often associated with an acidic local microenvironment (for instance, an inflamed joint). Recent studies also suggest that GPR65 acts through ICER in CD4+ T cells, to suppress IL-2 and hence bias cells toward an inflammatory Th17 phenotype, which is associated with increased pathogenicity in the context of autoimmune disease (Korn, 2009). Supporting this is the recent finding that ICER is required for Th17 differentiation (Yoshida, 2016) as well as that agonism of GPR65 leads to an increase in Th17 differentiation (Hernandez, 2018). Indeed, mutations in the GPR65 locus are associated with several autoimmune diseases, such as multiple sclerosis, ankylosing spondylitis, inflammatory bowel disease, and Crohn's disease (Gaublomme, 2015). One recent study found that mice with CD4+ T cells lacking GPR65 were protected from developing the disease autoimmune encephalomyelitis (EAE) (Gaublomme, 2015).

Thus, GPR65 appears to act through ICER to promote an anti-inflammatory and tumour-permissive phenotype in tumour associated macrophages and an inflammatory Th17 phenotype in CD4+ T cells that is associated with autoimmune disease. GPR65 signalling, therefore, represents an attractive pathway for therapeutic intervention for the treatment of both cancer and autoimmune diseases. There is therefore an ongoing need to develop new small molecule GPR65 modulators. Huang et al, Nature 527, 477-483 (2015) discloses allosteric ligands of GPR65. WO 2008/101354 discloses GPR65-specific antisense oligonucleotides.

The present invention seeks to provide compounds that are capable of modulating GPR65. As made clear from the above discussion, such compounds have potential therapeutic applications in the treatment of a variety of disorders, including proliferative disorders and immune disorders, as well as asthma and chronic obstructive pulmonary disease.

### STATEMENT OF INVENTION

A first aspect of the invention relates to a compound of formula (If), or a pharmaceutically acceptable salt or solvate thereof, 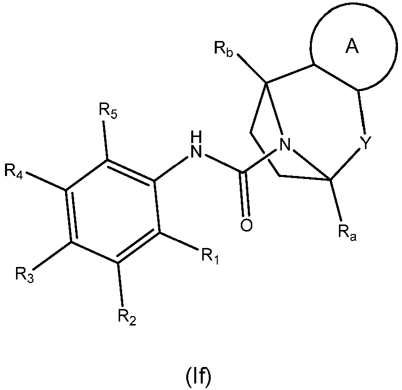 wherein:
ring A is a pyridinyl ring or tautomer thereof, or a phenyl ring, each of which may be optionally substituted with one or more substituents selected from H, F, CI, Br, I, CN, alkoxy, NR₁₁R₁₁', OH, alkyl, phenyl, and haloalkyl;
Y is selected from CH₂, C=N-OH, and CR₁₀R₁₀', wherein R₁₀ and R₁₀' are each independently selected from H, F, alkyl, and haloalkyl;
Rₐ and R_{b} are each independently selected from H and alkyl;
R₁, R₄, and R₅ are each independently selected from H, CN, alkyl, alkoxy, haloalkyl, OH, F, Cl, Br, and I;
R₂ and R₃ are each independently selected from H, F, CI, Br, I, CN, alkoxy, haloalkyl, haloalkoxy, alkyl, aryl, heteroaryl, O-aryl, NHCO-alkenyl and CO₂-alkyl, wherein said aryl, heteroaryl and O-aryl groups are each optionally further substituted by one or more groups independently selected from halo, alkyl and alkoxy; and
R₁₁ and R₁₁' are each independently selected from H, alkyl, haloalkyl, COR₁₂, CO₂R₁₂ and SO₂R₁₃, wherein R₁₂ and R₁₃ are both independently alkyl.

A second aspect of the invention relates to a compound of formula (Ib), or a pharmaceutically acceptable salt or solvate thereof, 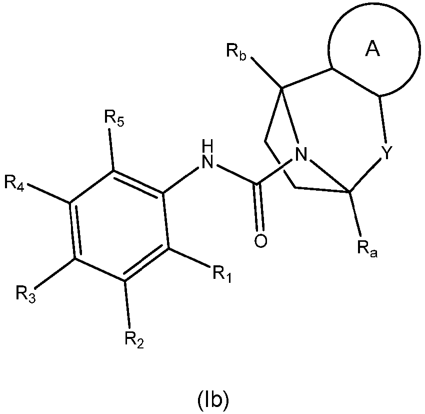 wherein:
ring A is selected from the groups (i)-(xx): 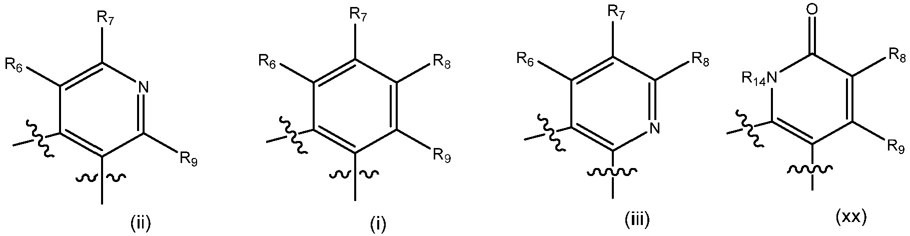 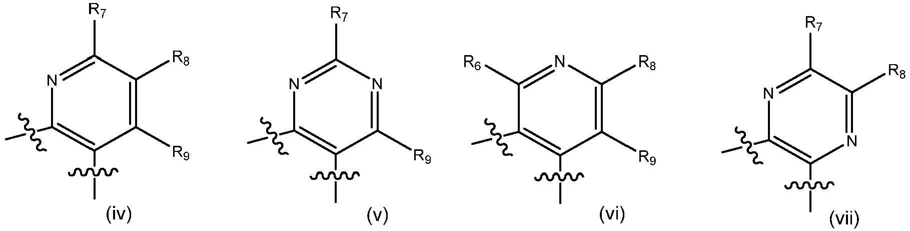 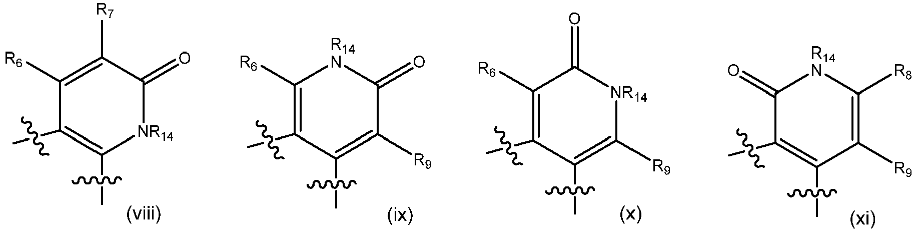 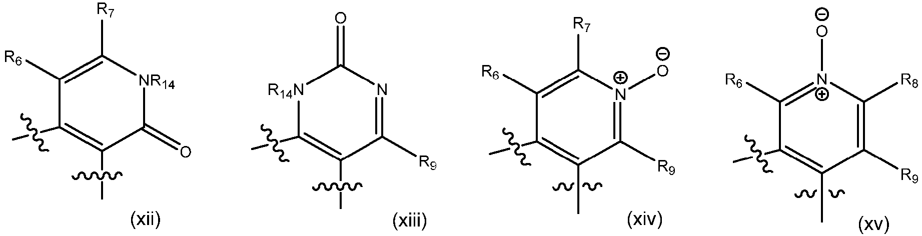 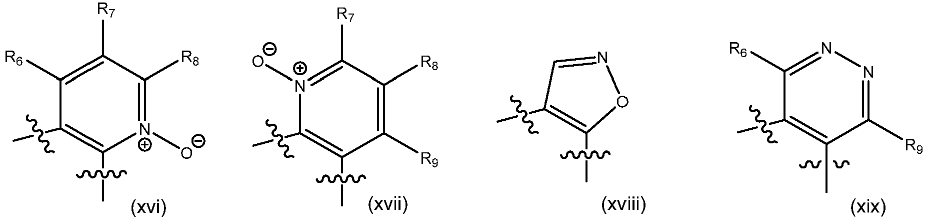 wherein R₆, R₇, R₈, and R₉ are each independently selected from H, F, CI, Br, I, CN, alkoxy, NR₁₁R₁₁', OH, alkyl, phenyl, and haloalkyl, and R₁₄ is H or alkyl;
Y is selected from CH₂, C=N-OH, and CR₁₀R₁₀', wherein R₁₀ and R₁₀' are each independently selected from H, F, alkyl, and haloalkyl;
Rₐ and R_{b} are each independently selected from H and alkyl;
R₁, R₄, and R₅ are each independently selected from H, CN, alkyl, haloalkyl, alkoxy, OH, F, Cl, Br, and I;
R₂ and R₃ are each independently selected from H, F, Cl, Br, I, CN, alkoxy, haloalkyl, haloalkoxy, alkyl, aryl, heteroaryl, O-aryl, NHCO-alkenyl and CO₂-alkyl, wherein said aryl, heteroaryl and O-aryl groups are each optionally further substituted by one or more groups independently selected from halo, alkyl and alkoxy; and
R₁₁ and R₁₁' are each independently selected from H, alkyl, haloalkyl, COR₁₂, CO₂R₁₂ and SO₂R₁₃, wherein R₁₂ and R₁₃ are both independently alkyl.

A third aspect of the invention relates to a compound of formula (le), or a pharmaceutically acceptable salt or solvate thereof, 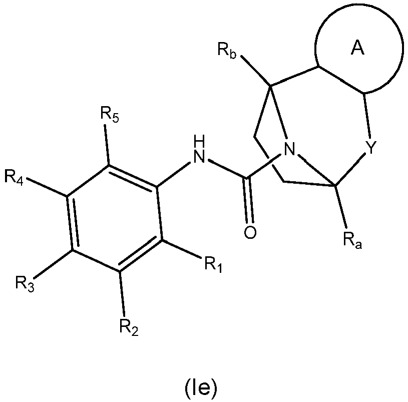 wherein:
ring A is 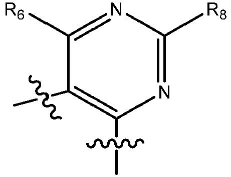 wherein R₆ and R₈ are each independently selected from H, F, CI, Br, I, CN, alkoxy, OH, phenyl, and haloalkyl (more preferably CF₃);
Y is selected from CH₂, C=N-OH, and CR₁₀R₁₀', wherein R₁₀ and R₁₀' are each independently selected from H, F, alkyl, and haloalkyl;
Rₐ and R_{b} are each independently selected from H and alkyl;
R₁, R₄, and R₅ are each independently selected from H, F, Cl, Br, and I; and
R₂ and R₃ are each independently selected from CI, Br, I, CN, and haloalkyl;
with the proviso that that when Y is CH₂, and Rₐ, R_{b}, R₁, R₄, R₅, R₆ and R₈ are all H:
   R₃ is not CN, when R₂ is Cl; and
   R₂ and R₃ are not both CI.

Another aspect of the invention related to a compound of formula (la), or a pharmaceutically acceptable salt or solvate thereof, 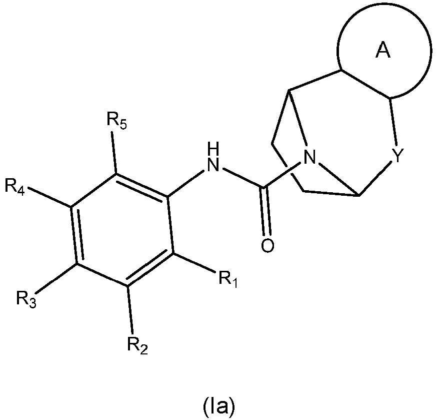 wherein:
ring A is a 5 or 6 membered aromatic or heteroaromatic ring, wherein said aromatic or heteroaromatic ring is optionally substituted with one or more substituents selected from F, CI, Br, I, CN, alkoxy, NR₁₁R₁₁', OH, alkyl, haloalkyl, aralkyl, aryl, and heteroaryl, and wherein said aryl and heteroaryl substituents are in turn optionally substituted with one or more substituents each independently selected from F, CI, Br, I, CN, alkoxy, NR₁₁R₁₁', OH, alkyl, haloalkyl, and aralkyl;
Y is selected from CH₂, C=N-OH, and CR₁₀R₁₀', wherein R₁₀ and R₁₀' are each independently selected from H, F, alkyl, and haloalkyl;
R₁, R₄, and R₅ are each independently selected from H, F, CI, Br, and I;
R₂ and R₃ are each independently selected from H, F, Cl, Br, I, CN, methoxy, and haloalkyl; and
R₁₁ and R₁₁' are each independently selected from H, alkyl, haloalkyl, COR₁₂, and SO₂R₁₃, wherein R₁₂ and R₁₃ are both alkyl;

wherein when Y is CH₂ and ring A is: 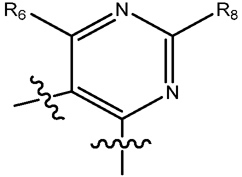
where R₆ and R₈ are both H,
   - R₂ and R₃ are not both CI, when R₁, R₄ and R₅ are all H;
   - R₃ is not Cl, when R₁, R₂, R₄ and R₅ are all H;
   - R₂ is not Cl, when R₁, R₃, R₄ and R₅ are all H;
   - R₁ is not Cl, when R₂, R₃, R₄ and R₅ are all H;
   - R₁ is not F, when R₄ is Cl and R₂, R₃ and R₅ are all H.

Advantageously, the presently claimed compounds are capable of modulating GPR65, thereby rendering the compounds of therapeutic interest in the treatment of various disorders, for example, in the field of oncology, immuno-oncology, and immunology.

Another aspect of the invention relates to compound of formula (Ic), or a pharmaceutically acceptable salt or solvate thereof, 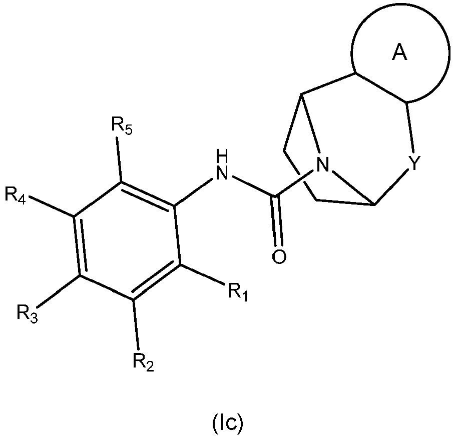 wherein:
ring A is 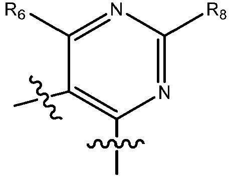 wherein R₆ and R₈ are each independently selected from H, F, Cl, Br, I, CN, alkoxy, NR₁₁R₁₁', OH, alkyl, phenyl, and haloalkyl, with the proviso that at least one of R₆ and R₈ is other than H;
Y is selected from CH₂, C=N-OH, and CR₁₀R₁₀', wherein R₁₀ and R₁₀' are each independently selected from H, F, alkyl, and haloalkyl;
R₁, R₄, and R₅ are each independently selected from H, F, CI, Br, and I;
R₂ and R₃ are each independently selected from H, F, Cl, Br, I, CN, methoxy, and haloalkyl; and
R₁₁ and R₁₁' are each independently selected from H, alkyl, haloalkyl, COR₁₂, and SO₂R₁₃, wherein R₁₂ and R₁₃ are both alkyl.

Another aspect of the invention relates to a compound of formula (Id), or a pharmaceutically acceptable salt or solvate thereof, 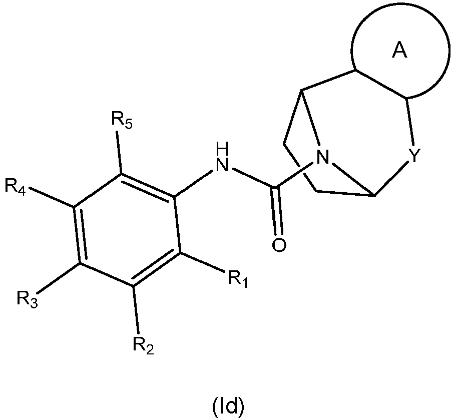 wherein:
ring A is 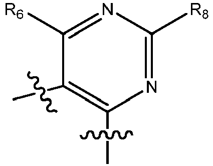 wherein R₆ and R₈ are each independently selected from H, F, Cl, Br, I, CN, alkoxy, NR₁₁R₁₁', OH, alkyl, phenyl, and haloalkyl;
Y is selected from CH₂, C=N-OH, and CR₁₀R₁₀', wherein R₁₀ and R₁₀' are each independently selected from H, F, alkyl, and haloalkyl;
R₁, R₄, and R₅ are each independently selected from H, F, CI, Br, and I;
R₂ and R₃ are each independently selected from H, F, Cl, Br, I, CN, methoxy, and haloalkyl, with the proviso that at least one of R₂ and R₃ is selected from CN, methoxy, and haloalkyl; and
R₁₁ and R₁₁' are each independently selected from H, alkyl, haloalkyl, COR₁₂, and SO₂R₁₃, wherein R₁₂ and R₁₃ are both alkyl.

Another aspect of the invention relates to a compound of formula (I), or a pharmaceutically acceptable salt or solvate thereof, 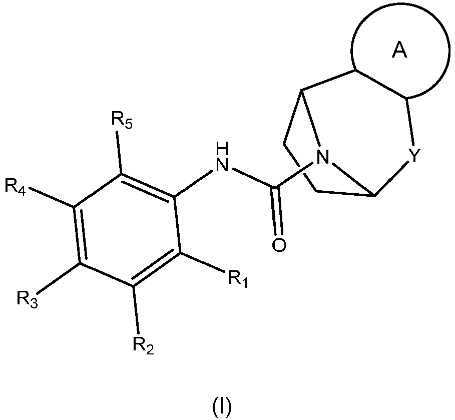 wherein:
ring A is a 5 or 6 membered aromatic or heteroaromatic ring, wherein said aromatic or heteroaromatic ring is optionally substituted with one or more substituents selected from F, CI, Br, I, CN, alkoxy, NR₁₁R₁₁', OH, alkyl, haloalkyl, aralkyl, aryl, and heteroaryl, and wherein said aryl and heteroaryl substituents are in turn optionally substituted with one or more substituents each independently selected from F, Cl, Br, I, CN, alkoxy, NR₁₁R₁₁', OH, alkyl, haloalkyl, and aralkyl;
Y is selected from CH₂, C=N-OH, and CR₁₀R₁₀', wherein R₁₀ and R₁₀' are each independently selected from H, F, alkyl, and haloalkyl;
R₁, R₄, and R₅ are each independently selected from H, F, CI, Br, and I;
R₂ and R₃ are each independently selected from H, F, Cl, Br, I, CN, methoxy, and haloalkyl; and
R₁₁ and R₁₁' are each independently selected from H, alkyl, haloalkyl, COR₁₂, and SO₂R₁₃, wherein R₁₂ and R₁₃ are both alkyl;
for use as a medicament.

Another aspect of the invention relates to a compound of formula (Ia), (Ib), (Ic), (Id), (le) or (If) as described above for use as a medicament.

Another aspect of the invention relates to a compound of formula (I), (Ia), (Ib), (Ic), (Id), (le) or (If) as described above for use in treating or preventing a disorder selected from a proliferative disorder, an immune disorder, asthma, chronic obstructive pulmonary disease (COPD) and acute respiratory distress syndrome (ARDS).

Another aspect of the invention relates to a compound for use as described above, which is of formula (I.1): 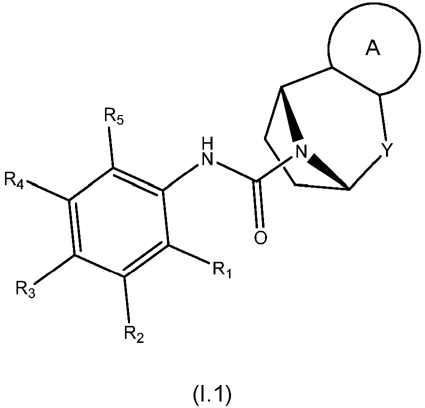 where A, Y and R₁-R₅ are as defined above.

Another aspect of the invention relates to a compound for use as described above, which is of formula (I.2): 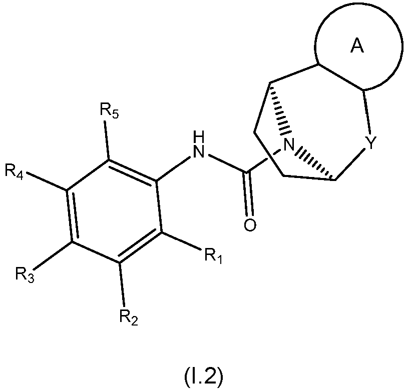 where A, Y and R₁-R₅ are as defined above.

Another aspect of the invention relates to a pharmaceutical composition comprising a compound as described above and a pharmaceutically acceptable diluent, excipient, or carrier.

Another aspect of the invention relates to a pharmaceutical composition as described above for use as a medicament.

Another aspect of the invention relates to a pharmaceutical composition as described above for use in treating or preventing a disorder selected from a proliferative disorder, an immune disorder, asthma, chronic obstructive pulmonary disease (COPD) and acute respiratory distress syndrome (ARDS).

Another aspect of the invention relates to a method of treating a disorder, comprising administering to a subject a compound or a pharmaceutical composition as described above.

### DETAILED DESCRIPTION

The present invention relates to compounds that are capable of modulating GPR65.

"Alkyl" is defined herein as a straight-chain or branched alkyl radical, preferably C₁₋₂₀ alkyl, more preferably C₁₋₁₂ alkyl, even more preferably C₁₋₁₀ alkyl or C₁₋₆ alkyl, for example, methyl, ethyl, propyl, isopropyl, butyl, isobutyl, tert-butyl, pentyl, hexyl. More preferably, the alkyl is a C₁₋₃ alkyl.

As used herein, the term "aryl" refers to a C₆₋₁₂ aromatic group, which may be benzocondensed, for example, phenyl or naphthyl.

"Haloalkyl" is defined herein as a straight-chain or branched alkyl radical as defined above, for example, methyl, ethyl, propyl, isopropyl, butyl, isobutyl, tert-butyl, pentyl, hexyl, that is substituted with one or more halogen atoms (that may be the same or different), such as fluorine, chlorine, bromine, and iodine. Preferably, the haloalkyl is a C₁₋₂₀ haloalkyl, more preferably a C₁₋₁₂ haloalkyl, even more preferably a C₁₋₁₀ haloalkyl or a C₁₋₆ haloalkyl, or a C₁₋₃ haloalkyl. Preferred examples are CF₃ and CHF₂, with CF₃ being particularly preferred.

"Alkoxy" is defined herein as an oxygen atom bonded to an alkyl group as defined above, for example methoxy, ethoxy, propoxy, isopropoxy, butoxy, isobutoxy, tert-butoxy, pentoxy and hexoxy. Preferably, the alkoxy is a C₁₋₂₀ alkoxy , more preferably a C₁₋₁₂ alkoxy, even more preferably C₁₋₁₀ alkoxy or a C₁₋₆ alkoxy, or a C₁₋₃ alkoxy. A particularly preferred example is methoxy (-OCH₃).

"Heteroaryl" is defined herein as a monocyclic or bicyclic C₂₋₁₂ aromatic ring comprising one or more heteroatoms (that may be the same or different), such as oxygen, nitrogen or sulphur. Examples of suitable heteroaryl groups include thienyl, furanyl, pyrrolyl, pyridinyl, oxazolyl, thiazolyl, imidazolyl, pyrazolyl, isoxazolyl, isothiazolyl, oxadiazolyl, triazolyl, thiadiazolyl etc. and benzo derivatives thereof, such as benzofuranyl, benzothienyl, benzimidazolyl, indolyl, isoindolyl, indazolyl etc.; or pyridyl, pyrazinyl, pyrimidinyl, pyridazinyl, triazinyl etc. and benzo derivatives thereof, such as quinolinyl, isoquinolinyl, cinnolinyl, phthalazinyl, quinazolinyl, quinoxalinyl, naphthyridinyl etc.

"Aralkyl' is defined herein as an alkyl group as defined above substituted by one or more aryl groups as defined above.

In formulae (la), (Ib), (Ic), (Id) and (le), preferably alkyl is C₁₋C₆ alkyl, haloalkyl is C₁₋C₆ haloalkyl, and alkoxy is C₁₋C₆ alkoxy.

### Structural representation of the compounds

The compounds of the invention comprise a structure wherein ring A is an optionally substituted 5- or 6-membererd aromatic or heteroaromatic ring fused to a bicyclic nitrogen-containing moiety to form a tricyclic structure. The resulting tricyclic structure can exist in two different configurations as depicted below (bridge substituents Rₐ and R_{b} are omitted in the following representations for clarity):

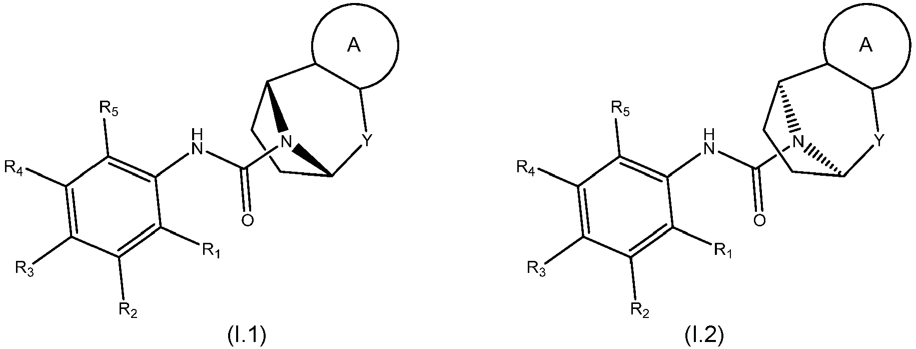

For the avoidance of doubt, the invention encompasses the compounds in either of the above configurations, as well as mixtures thereof, including racemic mixtures.

Alternatively, the structure can, for example, be represented, as follows:

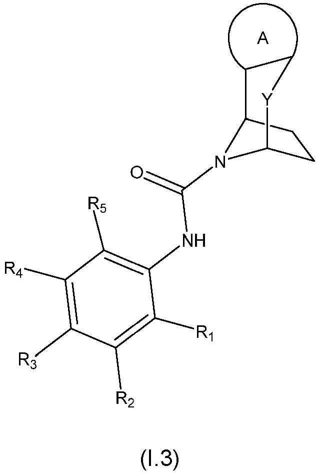

For the avoidance of doubt, the invention encompasses the compounds in the above configuration, as well the corresponding enantiomers thereof, and mixtures thereof, including racemic mixtures. As used throughout, and for ease of representation, *specific* examples of compounds according to the invention depicted in the above configuration (I.3) refer to mixtures of both enantiomers (in particular, the racemate), whereas the respective enantiomers - where these have been synthesised or separated - are depicted as either configuration (I.1) or configuration (I.2) with wedged bonds or dashed bonds respectively.

The compounds described herein contain an optionally substituted 5 or 6-membered aromatic or heteroaromatic ring A, which is fused to the bicyclic nitrogen-containing moiety to form a tricyclic structure. The optional substituents are selected from halo, CN, alkoxy, NR₁₁R₁₁', OH, alkyl, phenyl and haloalkyl. In some instances, ring A can exist in more than one tautomeric form. By way of illustration, where the heteroaromatic ring is substituted by an OH group, ring A can exist as two possible tautomers as shown below:

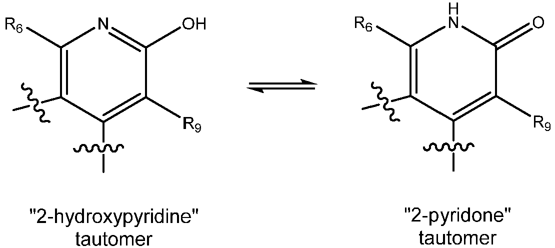

The 2-pyridone tautomer is believed to be the predominant solid state form. In solution, the energy difference between the two tautomeric forms is understood to be very small and is dependent on the polarity of the solvent. The skilled person would appreciate that other hydroxy substituted N-containing heteroaromatic groups (e.g. pyrimidine, other pyridine regioisomers) can be similarly represented in tautomeric form as shown above. The term "heteroaromatic" as used herein encompasses all tautomeric forms of the compounds.

Preferably, ring A is as defined herein, where the wavy lines denote attachment to the ring containing N and Y:

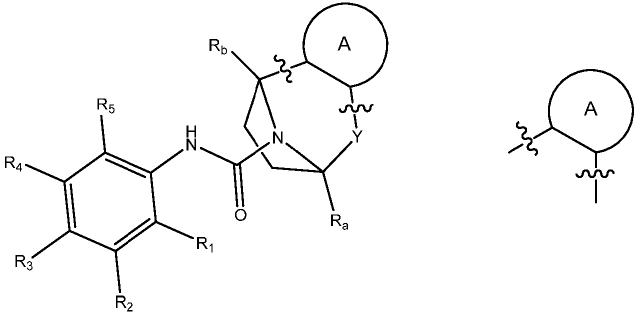

### Compounds of formula (If)

One aspect of the invention relates to a compound of formula (If), or a pharmaceutically acceptable salt or solvate thereof, 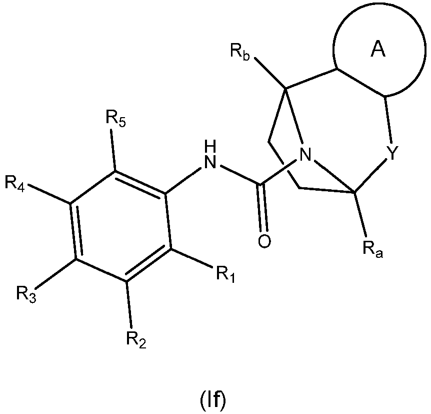 wherein:
ring A is a pyridinyl ring or tautomer thereof, or a phenyl ring, each of which may be optionally substituted with one or more substituents selected from H, F, CI, Br, I, CN, alkoxy, NR₁₁R₁₁', OH, alkyl, phenyl, and haloalkyl;
Y is selected from CH₂, C=N-OH, and CR₁₀R₁₀', wherein R₁₀ and R₁₀' are each independently selected from H, F, alkyl, and haloalkyl;
Rₐ and R_{b} are each independently selected from H and alkyl;
R₁, R₄, and R₅ are each independently selected from H, CN, alkyl, alkoxy, haloalkyl, OH, F, Cl, Br, and I;
R₂ and R₃ are each independently selected from H, F, CI, Br, I, CN, alkoxy, haloalkyl, haloalkoxy, alkyl, aryl, heteroaryl, O-aryl, NHCO-alkenyl and CO₂-alkyl, wherein said aryl, heteroaryl and O-aryl groups are each optionally further substituted by one or more groups independently selected from halo, alkyl and alkoxy; and
R₁₁ and R₁₁' are each independently selected from H, alkyl, haloalkyl, COR₁₂, CO₂R₁₂ and SO₂R₁₃, wherein R₁₂ and R₁₃ are both independently alkyl.

In one preferred embodiment, ring A is a hydroxy-substituted pyridinyl group which may exist in tautomeric form, for example, as depicted above in the section entitled "Structural representation of the compounds". The skilled person will recognise that other regioisomers of hydroxy-substituted pyridinyl groups can be similarly represented in tautomeric form (see groups (viii)-(xii) and (xx) below.

In a preferred embodiment, ring A is selected from the following: 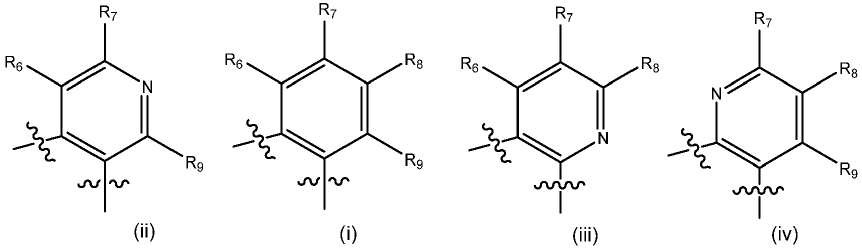 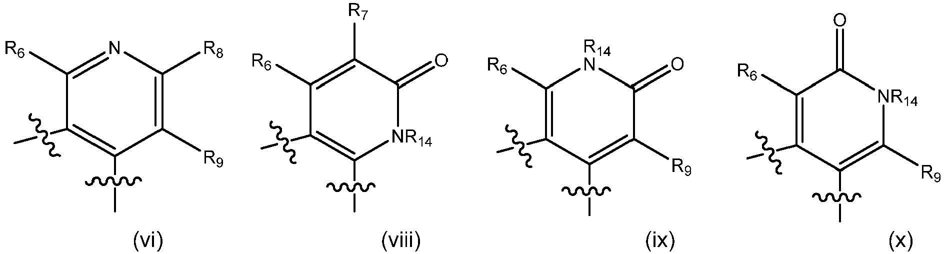 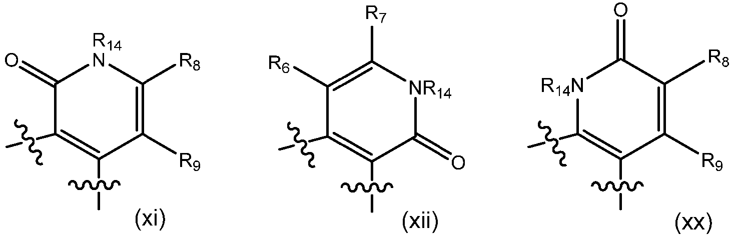 wherein R₆, R₇, R₈, and R₉ are each independently selected from H, F, CI, Br, I, CN, alkoxy, NR₁₁R₁₁', OH, alkyl, phenyl, and haloalkyl, and R₁₄ is H or alkyl, more preferably H.

Further preferred embodiments are set out below in relation to compounds of formula (Ib). In particular, preferred definitions for groups R₁₋₉, R₁₄, A, Y, Rₐ and R_{b} are as set out below for compounds of formula (Ib).

### Compounds of formula (Ib)

One aspect of the invention relates to a compound of formula (Ib), or a pharmaceutically acceptable salt or solvate thereof, 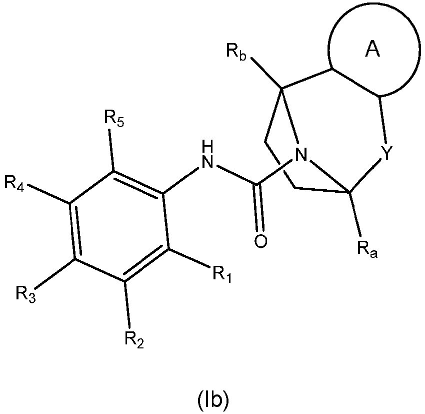 wherein:
ring A is selected from the groups (i)-(xx): 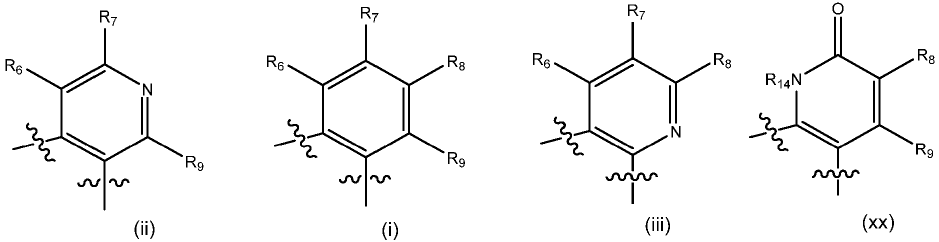 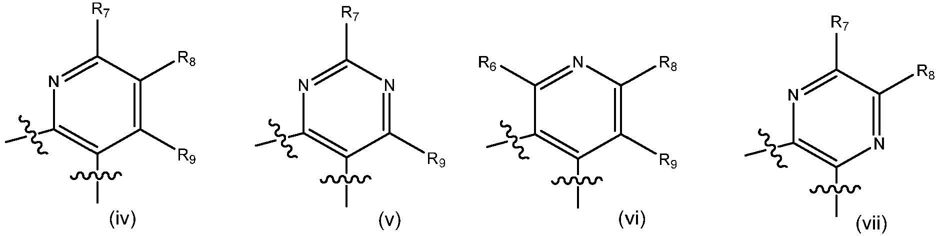 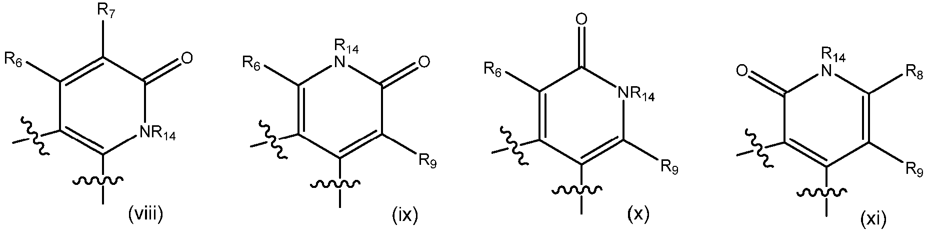 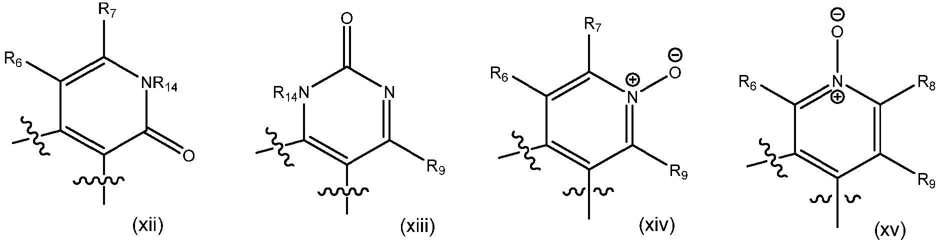 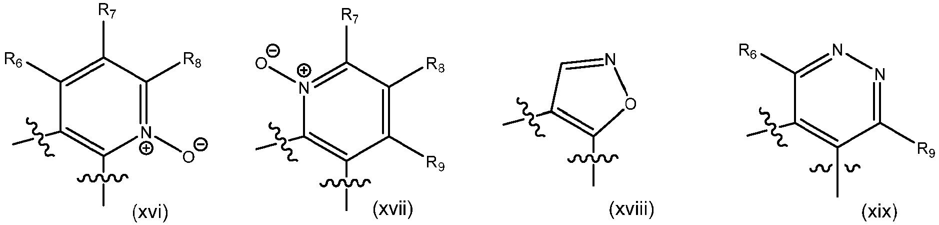 wherein R₆, R₇, R₈, and R₉ are each independently selected from H, F, CI, Br, I, CN, alkoxy, NR₁₁R₁₁', OH, alkyl, phenyl, and haloalkyl, and R₁₄ is H or alkyl;
Y is selected from CH₂, C=N-OH, and CR₁₀R₁₀', wherein R₁₀ and R₁₀' are each independently selected from H, F, alkyl, and haloalkyl;
Rₐ and R_{b} are each independently selected from H and alkyl;
R₁, R₄, and R₅ are each independently selected from H, CN, alkyl, haloalkyl, alkoxy, OH, F, Cl, Br, and I;
R₂ and R₃ are each independently selected from H, F, Cl, Br, I, CN, alkoxy, haloalkyl, haloalkoxy, alkyl, aryl, heteroaryl, O-aryl, NHCO-alkenyl, and CO₂-alkyl, wherein said aryl, heteroaryl and O-aryl groups are each optionally further substituted by one or more groups independently selected from halo, alkyl and alkoxy; and
R₁₁ and R₁₁' are each independently selected from H, alkyl, haloalkyl, COR₁₂, CO₂R₁₂ and SO₂R₁₃, wherein R₁₂ and R₁₃ are both alkyl.

In one preferred embodiment, ring A is selected from groups (i)-(xviii).

In one preferred embodiment, ring A is selected from groups (i), (ii), (iii), (iv), (v), (vi), (viii), (ix), (xiv), (xv) and (xix), and is preferably selected from (i), (ii), (iii), (v), (vi) and (ix), and is even more preferably selected from (i), (ii), (vi) and (ix).

In one preferred embodiment, ring A is selected from (i), (ii), (iii), (iv), (vi), (vii), (viii), (ix), (xiii), (xiv), (xv), (xvi), (xvii) and (xviii).

In one preferred embodiment, ring A is selected from (i), (ii), (vi), (ix) (xiv) and (xv), and is more preferably selected from (i), (ii) and (vi).

In one preferred embodiment, ring A is selected from (ii), (vi) and (ix).

In one preferred embodiment, ring A is (ii).

In one preferred embodiment, ring A is (vi).

In one preferred embodiment, ring A is (vi).

In one preferred embodiment the compound is of formula (Ib)-(ii): 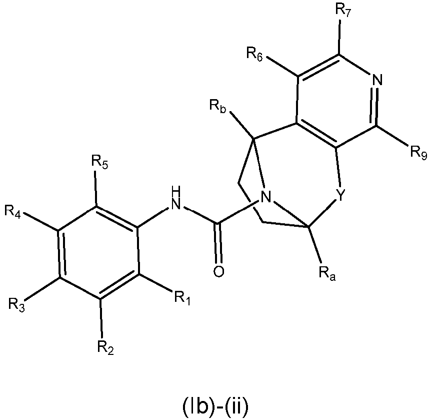 wherein Y, Rₐ, R_{b}, R₁-R₇ and R₉ are as defined above. In one preferred embodiment, R₆, R₇ and R₉ are all H. In one particularly preferred embodiment, R₉ is F and R₆ and R₇ are both H.

In one preferred embodiment, the compound is of formula (Ib)-(i): 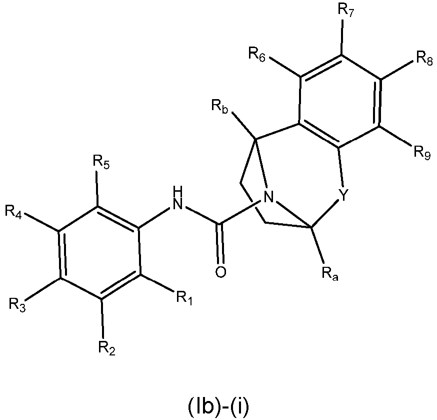 wherein Y, Rₐ, R_{b}, R₁-R₉ are as defined above. In one preferred embodiment, R₆, R₇ and R₉ are all H, and R₈ is selected from H, OMe, OH, CI, CN, F and NH₂.

In one preferred embodiment, the compound is of formula (Ib)-(vi): 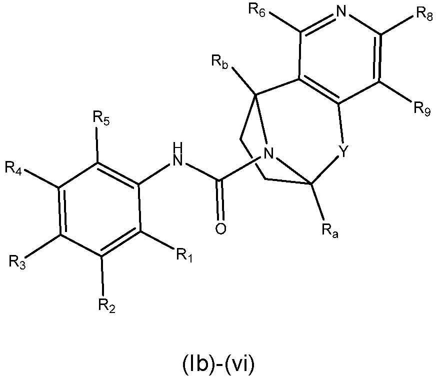 wherein Y, Rₐ, R_{b}, R₁-R₆, R₈ and R₉ are as defined above. In one preferred embodiment, R₆, R₈ and R₉ are each independently selected from H, F, Cl, NH₂, Br and OMe, and more preferably, from H and F. In one particularly preferred embodiment, R₆, R₈ and R₉ are all H.

In one preferred embodiment, the compound is of formula (Ib)-(ix): 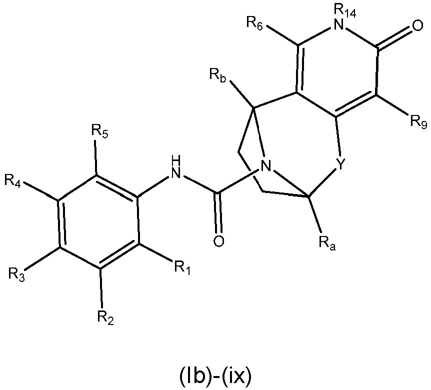 wherein Y, Rₐ, R_{b}, R₁-R₆, R₉ and R₁₄ are as defined above. In one preferred embodiment, R₆ and R₉ are both H, and R₁₄ is H or Me, more preferably Me. In one preferred embodiment, R₆ is H, R₉ is F, and R₁₄ is H or Me, more preferably Me.

In one preferred embodiment, Rₐ and R_{b} are each independently selected from H and methyl. In one preferred embodiment, one of Rₐ and R_{b} is alkyl (more preferably methyl) and the other is H. In one particularly preferred embodiment, Rₐ and R_{b} are both H.

In one preferred embodiment, R₁₁ and R₁₁' are selected from H and alkyl, and more preferably selected from H and Me, Even more preferably, R₁₁ and R₁₁' are both H.

In one preferred embodiment, Y is CR₁₀R₁₀', where R₁₀ and R₁₀' are each independently selected from H, F, Me and CF₃, and more preferably selected from H, F and Me. In one preferred embodiment, Y is selected from CH₂, CHF, CHMe and C=N-OH. In one preferred embodiment, Y is selected from CH₂ and C=N-OH. More preferably, Y is CH₂.

In one preferred embodiment, at least one of R₁, R₂, R₃, R₄ and R₅ is other than H.

In one preferred embodiment, one of R₁, R₂, R₃, R₄ and R₅ is other than H.

In one preferred embodiment, two of R₁, R₂, R₃, R₄ and R₅ are other than H.

In one preferred embodiment, three of R₁, R₂, R₃, R₄ and R₅ are other than H.

In one preferred embodiment, R₂ and R₃ are each independently selected from H, F, CI, Br, CN, methoxy, OCF₃, CF₃, OCHF₂, Me, Ph, pyrazolyl, oxazolyl, thiazolyl, OPh, NHCO-CH=CH₂ and CO₂Me, wherein said Ph, OPh, pyrazolyl, oxazolyl and thiazolyl groups are each optionally further substituted by one or more alkyl groups.

In one preferred embodiment, R₂ and R₃ are each independently selected from H, F, CI, Br, I, CN, methoxy, haloalkyl, haloalkoxy and CO₂-alkyl.

In one preferred embodiment, R₃ is selected from H, F, Cl, Br, CN, methoxy, OCF₃, CF₃, OCHF₂, Me, Ph, pyrazolyl, oxazolyl, thiazolyl, OPh, NHCO-CH=CH₂ and CO₂Me, wherein said Ph, OPh, pyrazolyl, oxazolyl and thiazolyl groups are each optionally further substituted by one or more alkyl groups. Preferably the pyrazolyl is a 1H-pyrazol-1-yl group. Preferably, the oxazolyl group is an oxazol-5-yl group. Preferably, the thiazolyl group is a thiazol-4-yl group.

In one preferred embodiment, R₂ is selected from H, F, Cl, Br, CN, Me, methoxy, OCF₃, CF₃, OCHF₂, Ph, pyrazolyl and CO₂Me, wherein said Ph and pyrazolyl groups are each optionally further substituted by one or more alkyl groups. Preferably the pyrazolyl is a *1H-*pyrazol-1-yl group.

In one preferred embodiment, R₂ and R₃ are each independently selected from F, CI, Br, I, CN, CO₂-alkyl, C₁₋C₆ haloalkyl and C₁₋C₆ haloalkoxy.

In one preferred embodiment, R₂ and R₃ are each independently selected from F, Cl, Br, I, CN, and C₁-C₆ haloalkyl.

In one preferred embodiment, R₂ and R₃ are each independently selected from H, F, CI, Br, I, CN, methoxy, and haloalkyl.

In one preferred embodiment, R₂ and R₃ are each independently selected from CI, Br, and CF₃, and more preferably independently selected from Cl and CF₃.

In one preferred embodiment, one of R₂ and R₃ is Cl and the other is OCF₃ or OCHF₂.

In one preferred embodiment, one of R₂ and R₃ is Cl and the other is CO₂Me.

In one preferred embodiment, R₂ and R₃ are both Cl, or one of R₂ and R₃ is Cl and the other is CF₃.

In one preferred embodiment, R₂ and R₃ are both CI.

In one preferred embodiment, one of R₂ and R₃ is Cl, and the other is CF₃.

In one preferred embodiment, one of R₂ and R₃ is Cl and the other is selected from OCF₃, CO₂Me, OCHF₂ and CF₃.

In one preferred embodiment, R₁, R₄, and R₅ are each independently selected from H, alkyl, alkoxy, OH, F, CI, Br, and I.

In one preferred embodiment, R₁, R₄, and R₅ are each independently selected from H, Me, OMe, OH, F, CI, Br, and I.

In one preferred embodiment, R₁, R₄, and R₅ are each independently selected from H, F, Cl, Br, and I.

In one preferred embodiment R₅ is selected from H, F, Me, MeO and Cl, and is preferably H or F, more preferably H.

In one preferred embodiment R₅ is selected from H, F and Cl, and is preferably H or F, more preferably H.

In one preferred embodiment, R₅ is selected from H, F and CN, and is preferably H.

In one preferred embodiment, R₁ and R₄ are both H.

In one preferred embodiment, R₁ is H; R₂ is selected from H, F, Cl, Br, CN, Me, methoxy, OCF₃, CF₃, OCHF₂, Ph, pyrazolyl and CO₂Me; R₃ is selected from from H, F, Cl, Br, CN, methoxy, OCF₃, CF₃, OCHF₂, Me, Ph, pyrazolyl, oxazolyl, thiazolyl, OPh, NHCO-CH=CH₂ and CO₂Me, wherein said Ph, OPh, pyrazolyl, oxazolyl and thiazolyl groups are each optionally further substituted by one or more alkyl groups; R₄ is H or CF₃, more preferably H; R₅ is selected from H, F, Me, MeO, Cl, OH and CN, and is preferably H or F, more preferably H. Preferably, for this embodiment, at least one of R₂ and R₃ is other than H. Even more preferably, both R₂ and R₃ are other than H.

In another preferred embodiment, one of R₂ and R₃ is selected from aryl, O-aryl and heteroaryl, each of which is optionally substituted, and the other of R₂ and R₃ is H, and R₁, R₄ and R₅ are all H.

In one preferred embodiment, R₆, R₇, R₈, and R₉ are each independently selected from H, F, Cl, Br, CN, OMe, NR₁₁R₁₁' and OH.

In one preferred embodiment, R₆, R₇, R₈, and R₉ are each independently selected from H, F, Cl, Br, CN, OMe, NH₂, NHBu, NHCO₂Bu and OH.

In one preferred embodiment, R₁₄ is H or methyl. More preferably, R₁₄ is H.

In one particularly preferred embodiment, the compound is of formula (Ib.1): 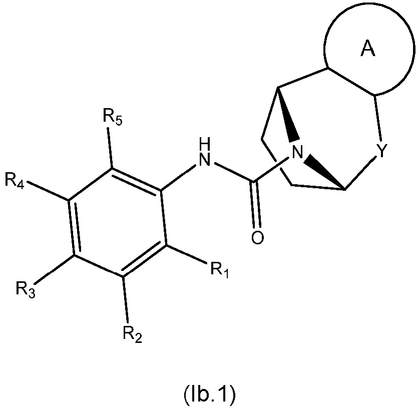 wherein ring A, and groups Y and R₁-R₅ are as described in any of the above embodiments.

In one preferred embodiment, the compound is in enantiomerically pure form. In one preferred embodiment, the compound is in the form of a mixture that is enantiomerically enriched with a compound of formula (Ib.1).

In another embodiment, the compound is of formula (Ib.2): 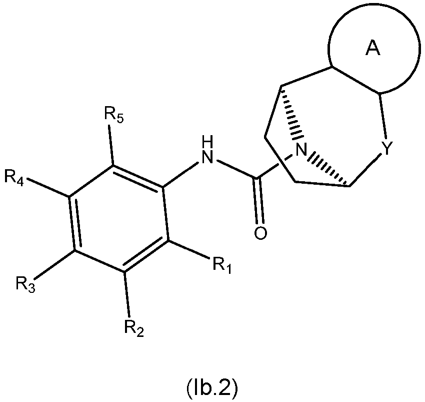 wherein ring A, and groups Y and R₁-R₅ are as described in any of the above embodiments.

In one preferred embodiment, the compound is in enantiomerically pure form. In one preferred embodiment, the compound is in the form of a mixture that is enantiomerically enriched with a compound of formula (Ib.2).

In one preferred embodiment, the compound is in the form of a mixture comprising a compound of formula (Ib.1) and its corresponding enantiomer of formula (Ib.2). In one preferred embodiment, the mixture is a racemic mixture, i.e. a 50:50 mixture of a compound of formula (Ib.1) and its corresponding enantiomer of formula (Ib.2).

Racemic mixtures can be used to prepare enantiomerically pure compounds of formula (Ib.1) or (Ib.2) by separating the compounds of formula (Ib.1) or (Ib.2) by standard methods, for example by chemical resolution using optically active acid or by the use of column chromatography or reverse-phase column chromatography using a substantially optically active (or "chiral") stationary phase as known to those skilled in the art. Racemic mixtures can also be used to prepare enantiomerically enriched mixtures of compounds of formula (Ib.1) or (Ib.2). Mixtures enriched with either a compound of formula (Ib.1) or (Ib.2) can also be obtained from the appropriate enantiomerically enriched precursors.

In one preferred embodiment of the invention, the compound is in the form of a mixture comprising enantiomers wherein the weight:weight ratio is at least approximately 2:1 or greater, preferably at least approximately 5:1 or greater, most preferably at least approximately 10:1 or greater in favour of the enantiomer that displays significant *in vitro* and/or *in vivo* activity (the eutomer).

In one particularly preferred embodiment, the compound is in the form of a mixture comprising a compound of formula (Ib.1) and its corresponding enantiomer of formula (Ib.2), wherein the weight:weight ratio of said compound of formula (Ib.1) to said compound of formula (Ib.2) is greater than 1.05:1, more preferably, greater than 2:1, even more preferably greater than 5:1, even more preferably greater than 10:1.

In one particularly preferred embodiment, the compound is in the form of a mixture comprising a compound of formula (Ib.1) and its corresponding enantiomer of formula (Ib.2), which is substantially enriched with said compound of formula (Ib.1).

In one embodiment, the compound is in the form of a mixture comprising a compound of formula (Ib.1) and its corresponding enantiomer of formula (Ib.2), wherein the weight:weight ratio of said compound of formula (Ib.2) to said compound of formula (Ib.1) is greater than 1.05:1, more preferably, greater than 2:1, even more preferably greater than 5:1, even more preferably greater than 10:1.

In one embodiment, the compound is in the form of a mixture comprising a compound of formula (Ib.1) and its corresponding enantiomer of formula (Ib.2), which is substantially enriched with said compound of formula (Ib.2).

In one preferred embodiment, the compound is selected from the following:

| | | | |
|---|---|---|---|
| 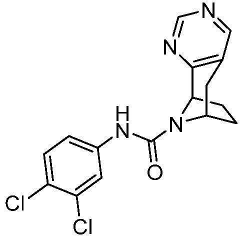 | (17) | 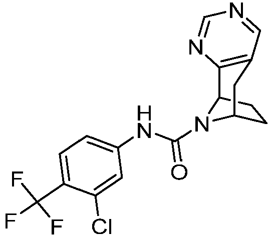 | (18) |
| 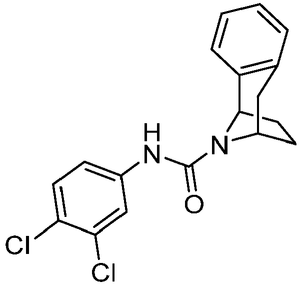 | (34) | 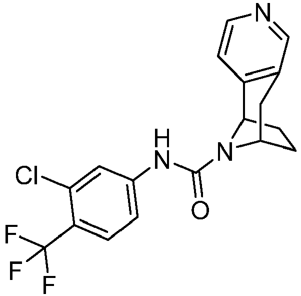 | (35) |
| 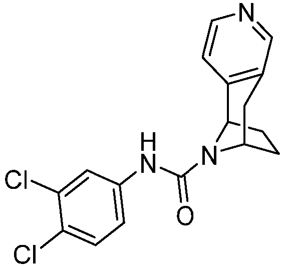 | (36) | 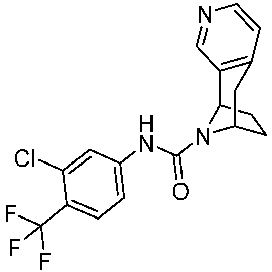 | (37) |
| | | 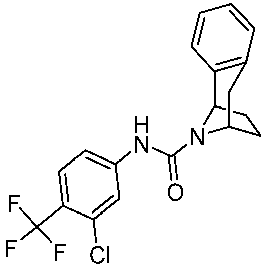 | (54) |
| 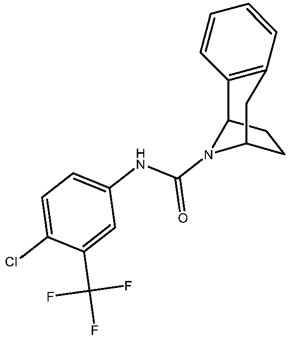 | (55) | 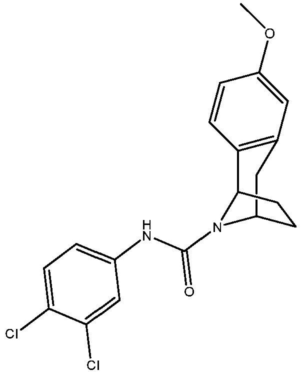 | (56) |
| 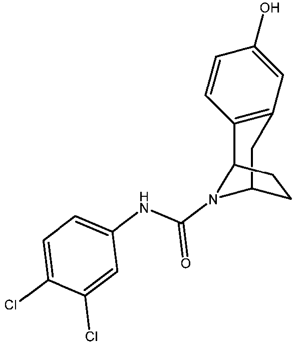 | (57) | 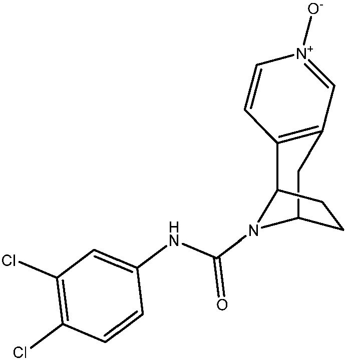 | (58) |
| 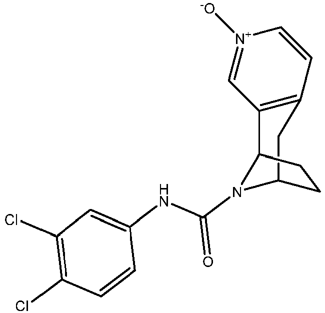 | (59) | 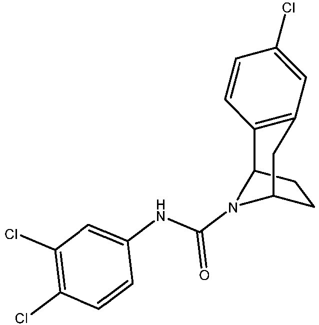 | (60) |
| 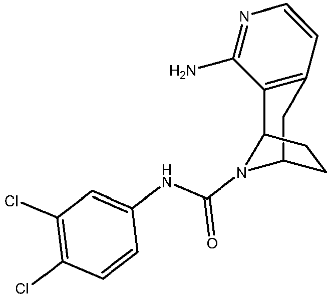 | (61) | 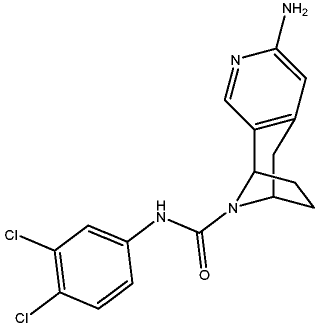 | (62) |
| 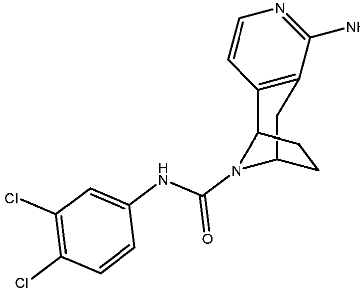 | (63) | 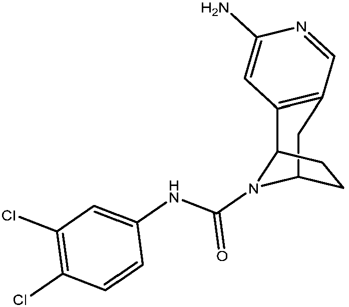 | (64) |
| 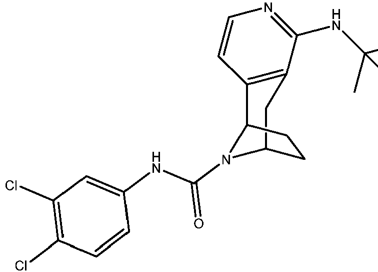 | (65) | 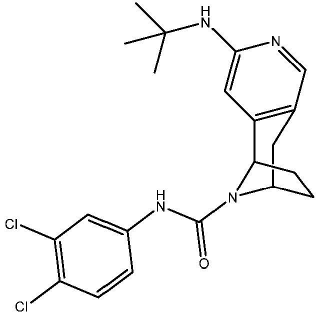 | (66) |
| 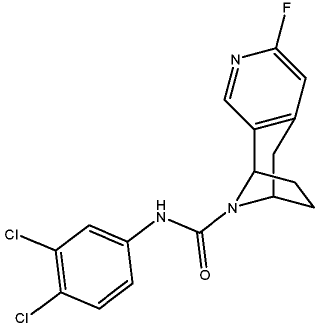 | (67) | 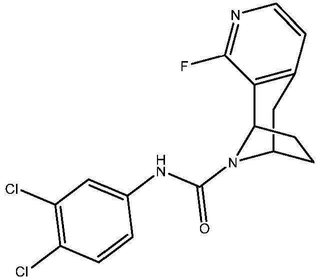 | (68) |
| 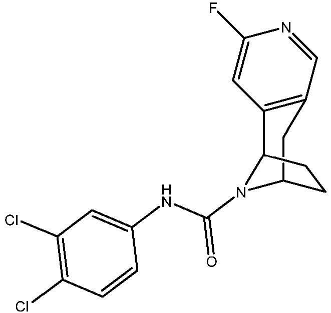 | (69) | 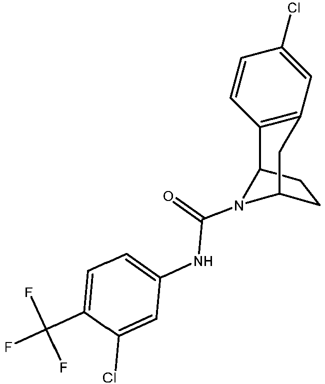 | (71) |
| 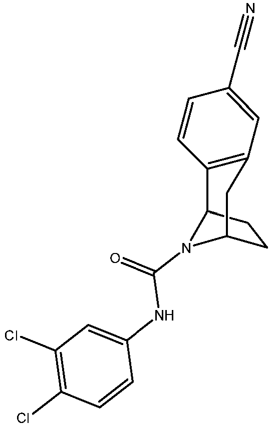 | (72) | 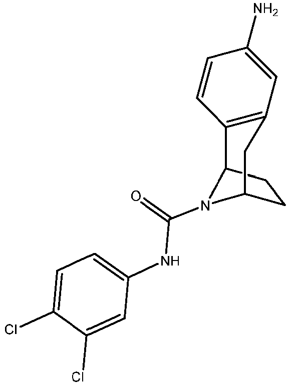 | (73) |
| 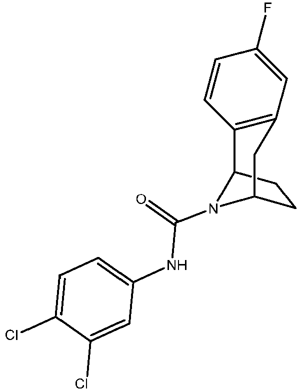 | (74) | 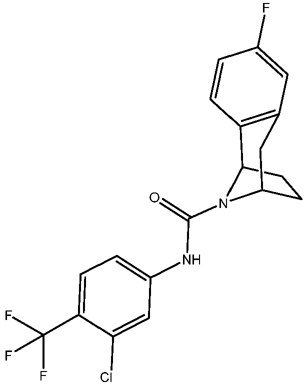 | (75) |
| 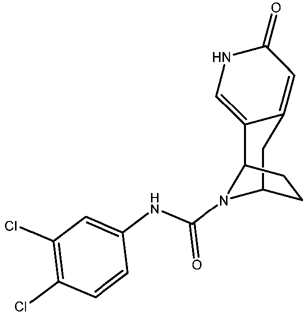 | (76) | 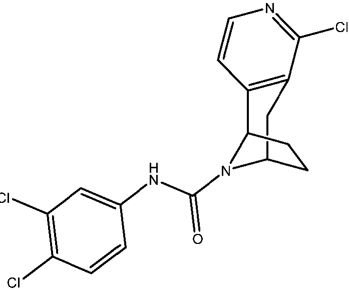 | (77) |
| 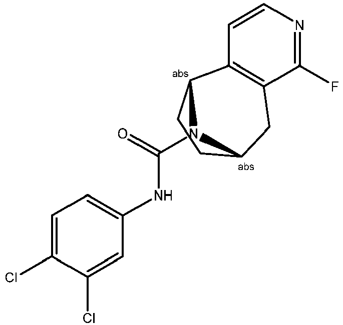 | (78) | 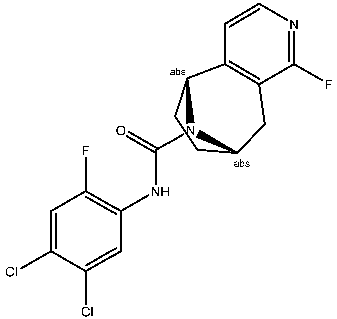 | (79) |
| 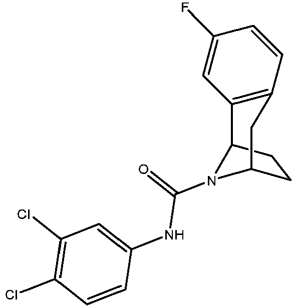 | (80) | 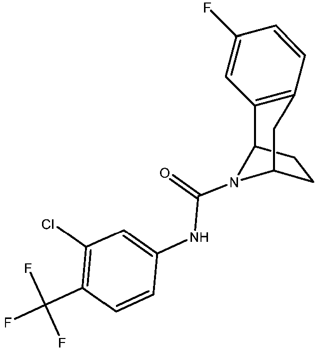 | (81) |
| 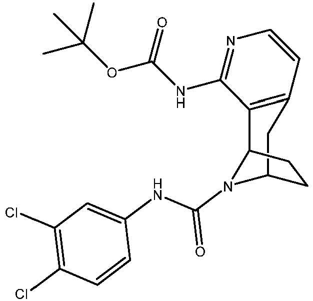 | (82) | 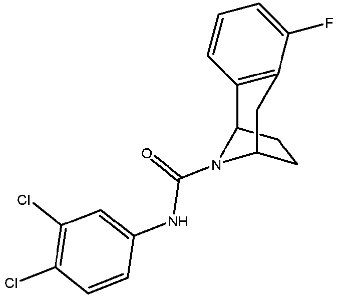 | (83) |
| 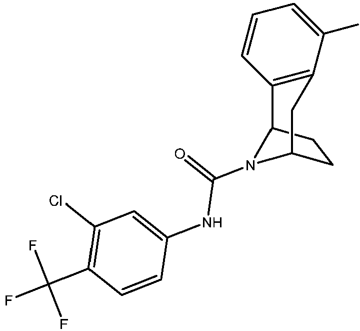 | (84) | 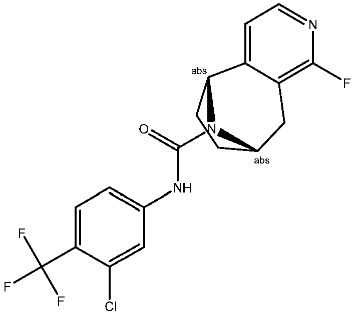 | (85) |
| 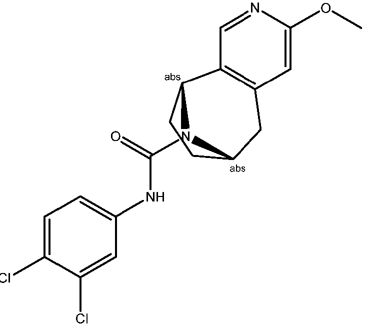 | (86) | 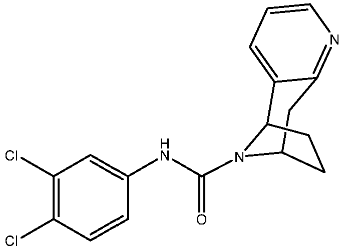 | (87) |
| 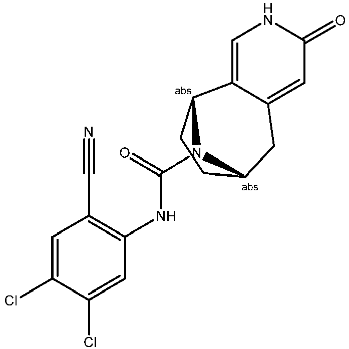 | (88) | 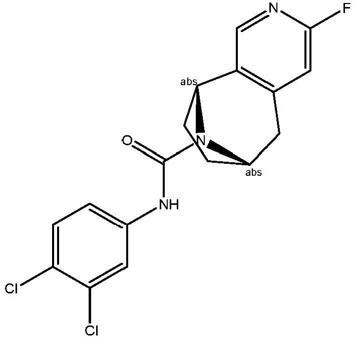 | (91) |
| 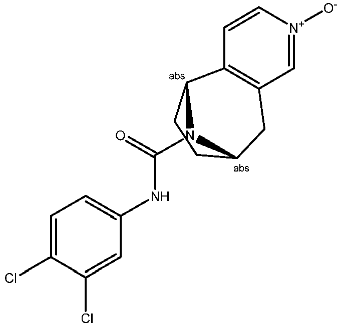 | (92) | 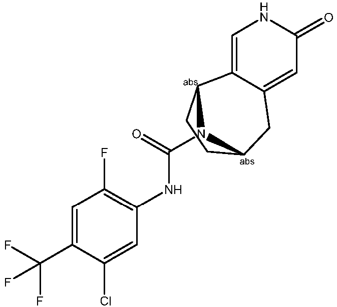 | (93) |
| 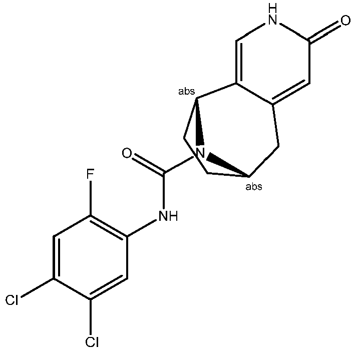 | (94) | 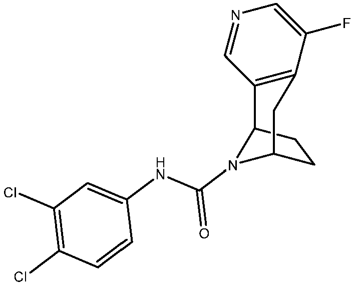 | (95) |
| 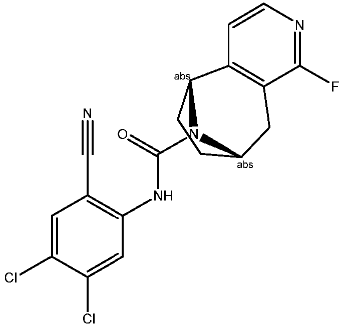 | (98) | 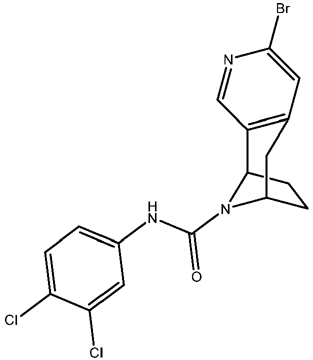 | (99) |
| 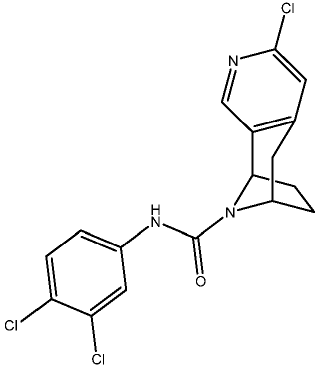 | (100) | 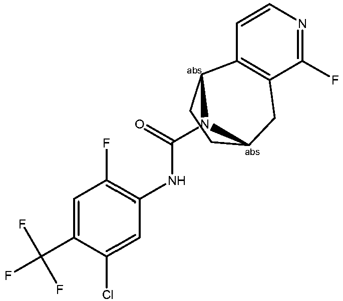 | (101) |
| 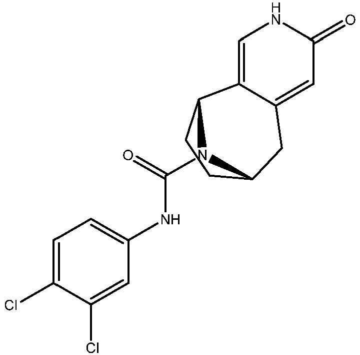 | (103) | 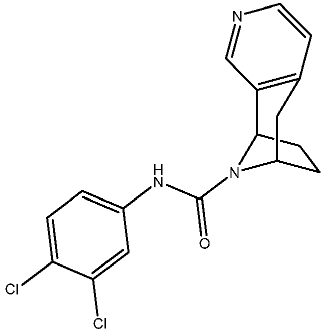 | (104) |
| 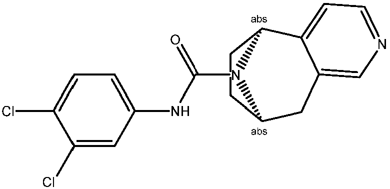 | (107) | 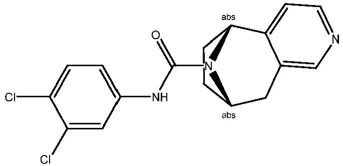 | (108) |
| 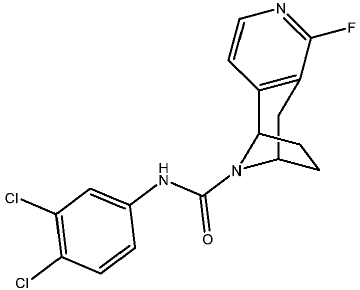 | (109) | 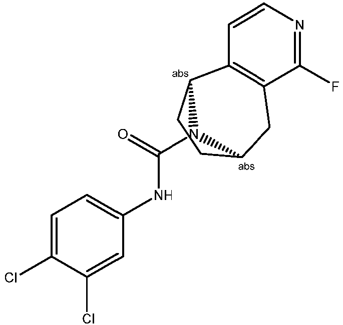 | (110) |
| 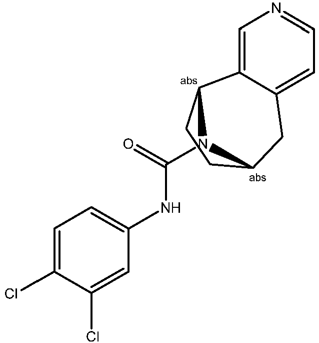 | (111) | 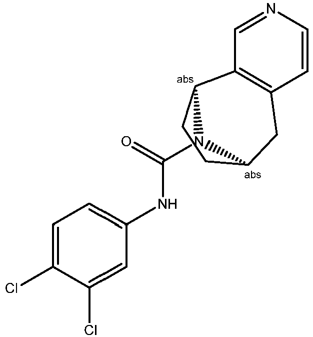 | (112) |
| 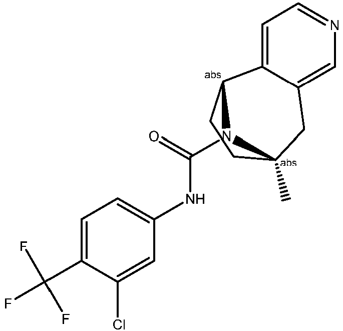 | (116) | 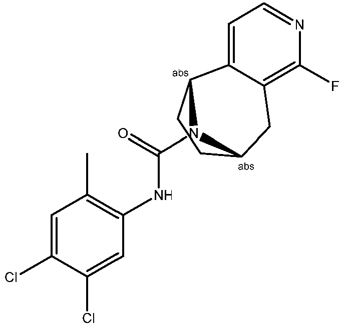 | (117) |
| 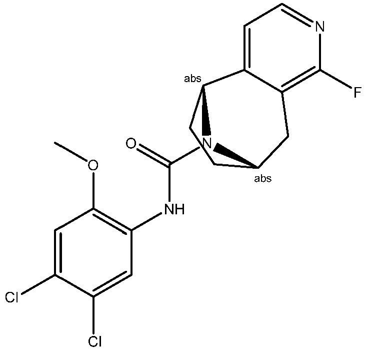 | (118) | 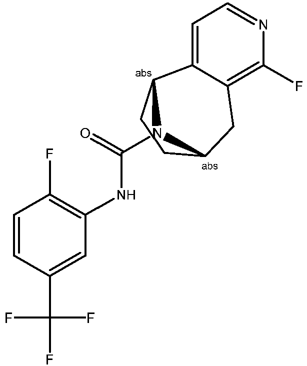 | (119) |
| 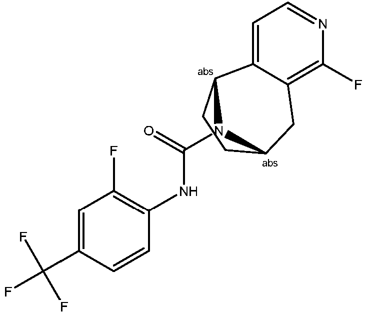 | (120) | 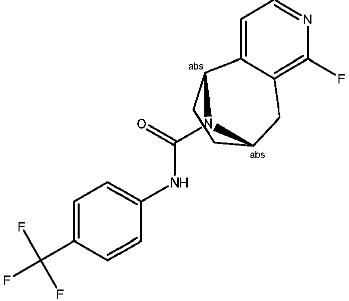 | (121) |
| 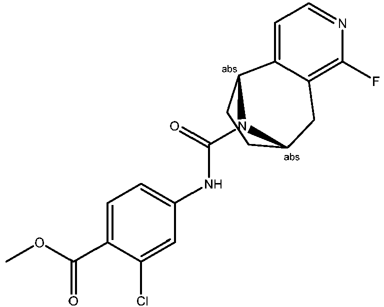 | (122) | 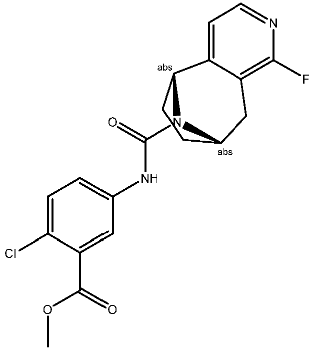 | (123) |
| 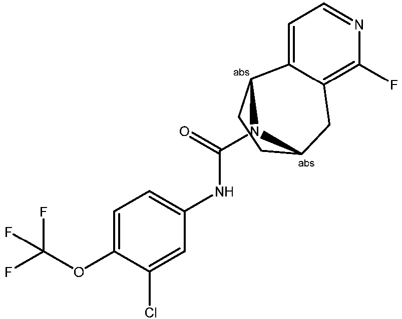 | (124) | 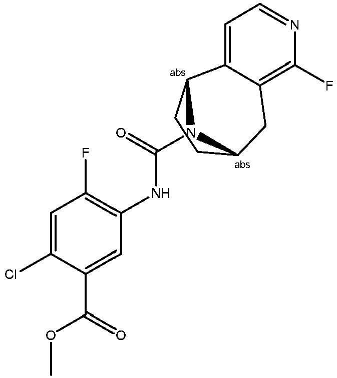 | (125) |
| 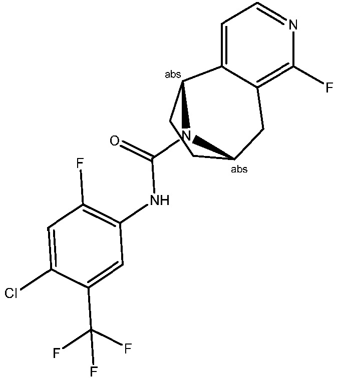 | (126) | 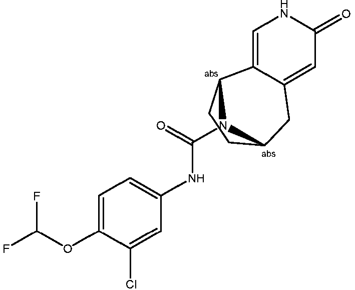 | (127) |
| 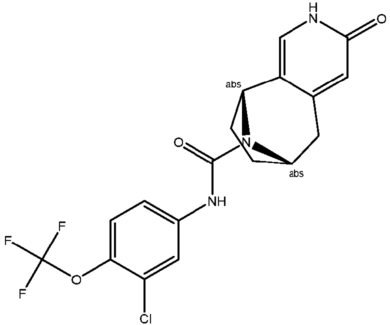 | (128) | 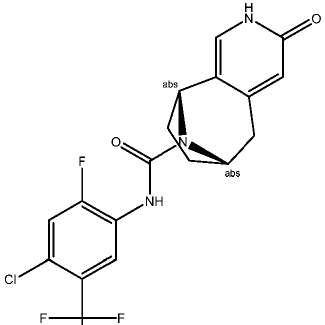 | (129) |
| 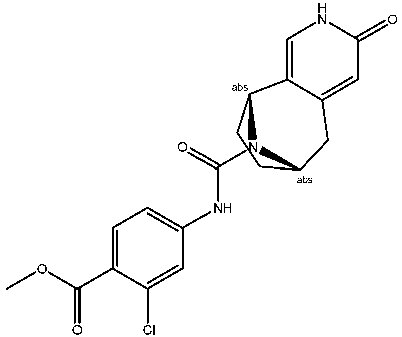 | (130) | 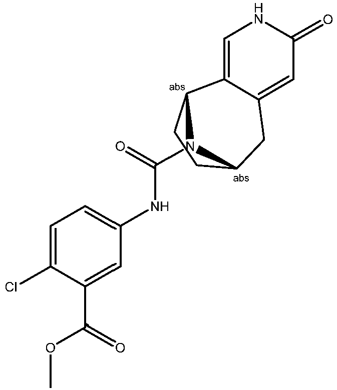 | (131) |
| 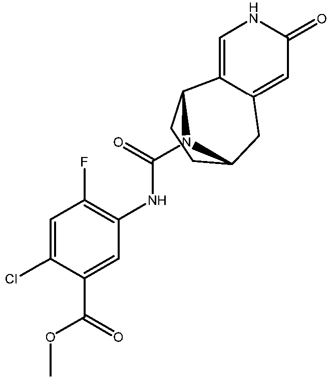 | (132) | 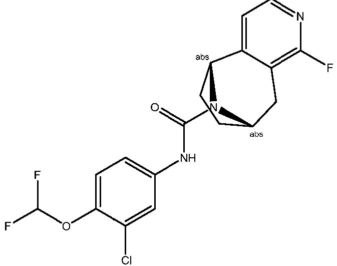 | (133) |
| 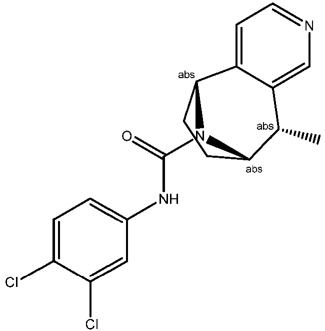 | (134) | 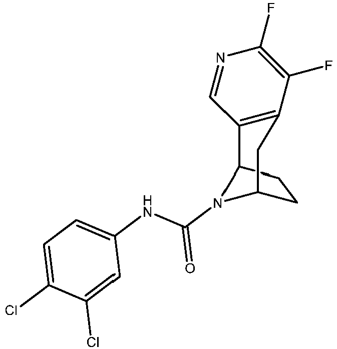 | (135) |
| 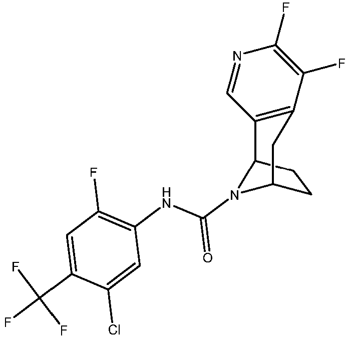 | (136) | 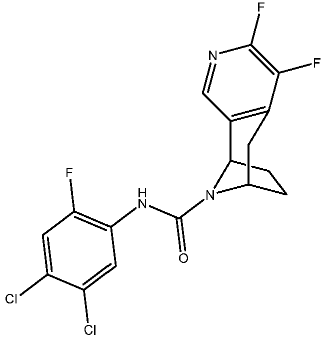 | (137) |
| 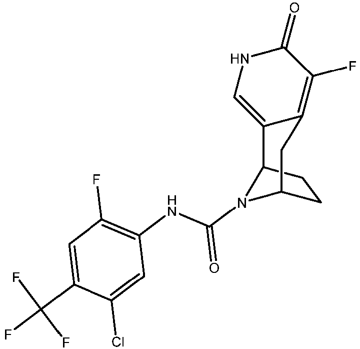 | (138) | 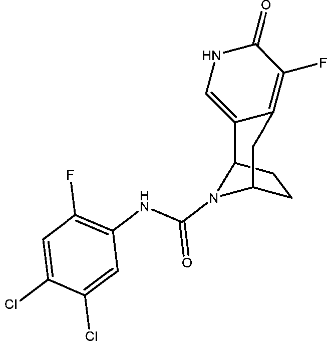 | (139) |
| 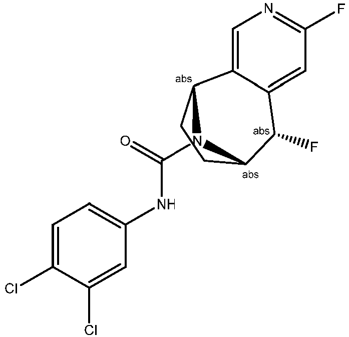 | (140) | 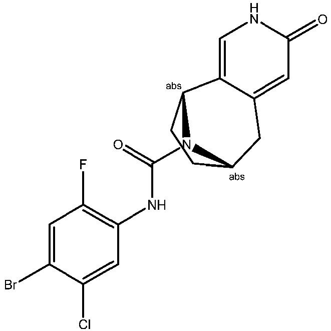 | (141) |
| 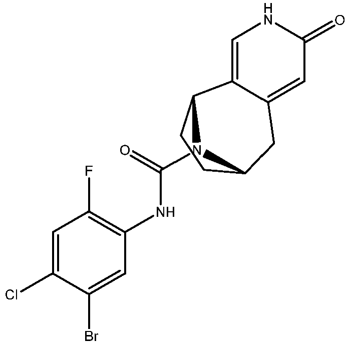 | (142) | 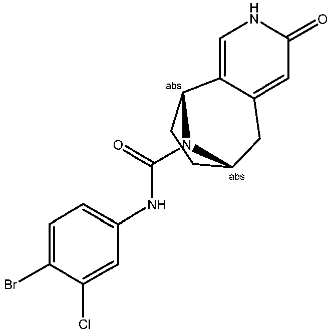 | (143) |
| 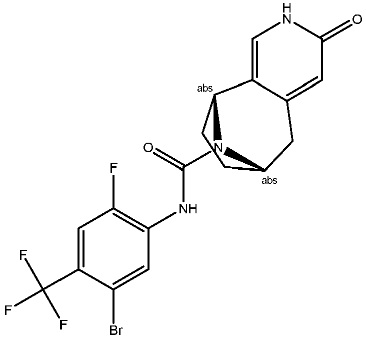 | (144) | 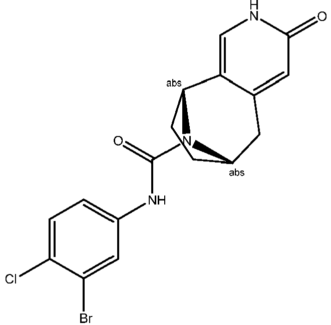 | (145) |
| 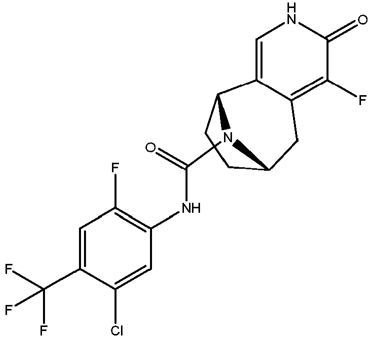 | (146) | 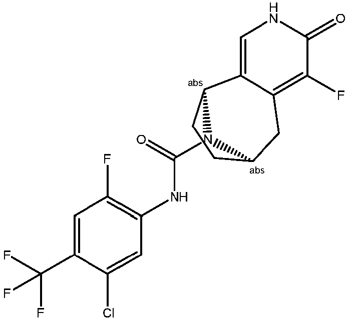 | (147) |
| 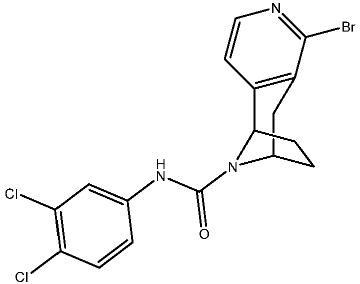 | (148) | 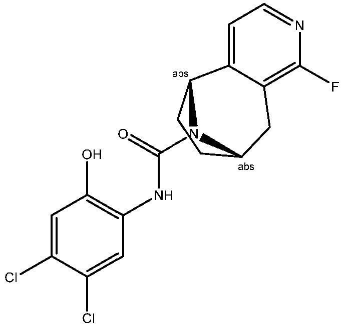 | (149) |
| 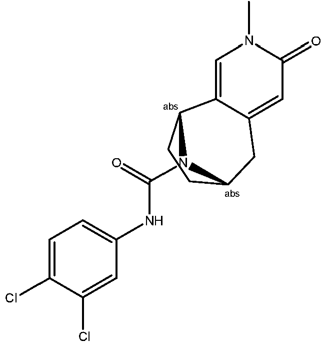 | (150) | | |
| 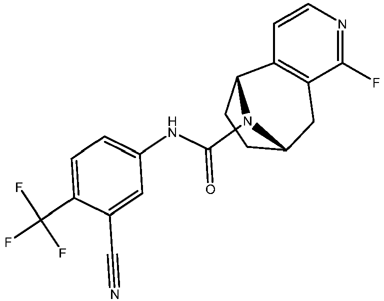 | (151) | 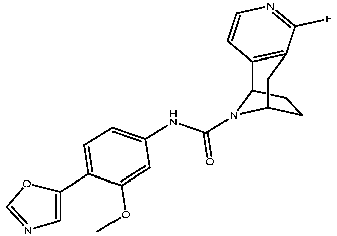 | (152) |
| 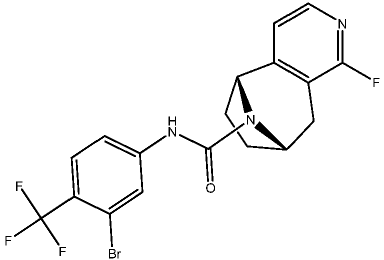 | (153) | 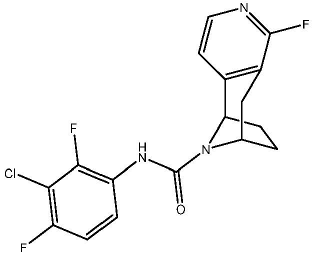 | (154) |
| 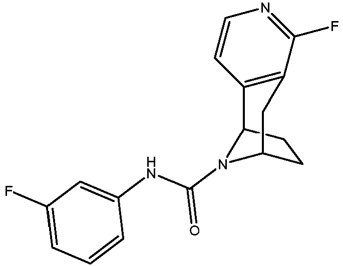 | (155) | 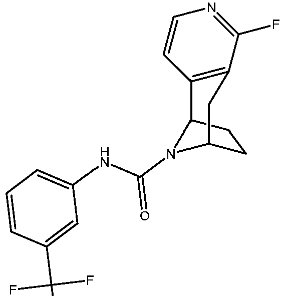 | (156) |
| 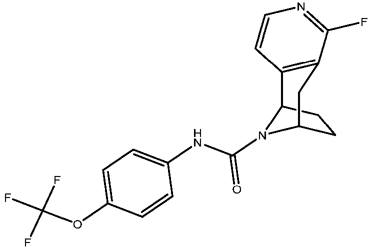 | (157) | 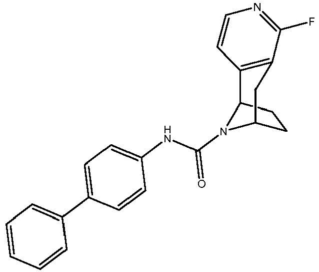 | (158) |
| 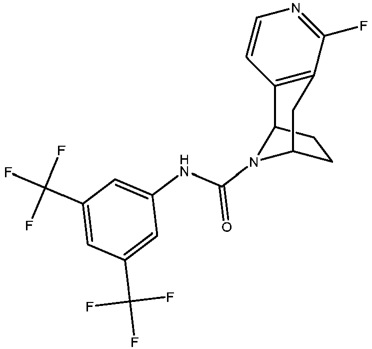 | (159) | 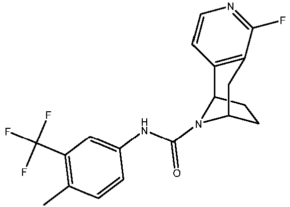 | (160) |
| 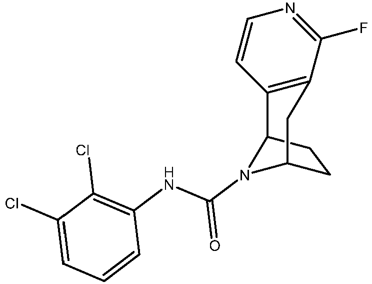 | (161) | 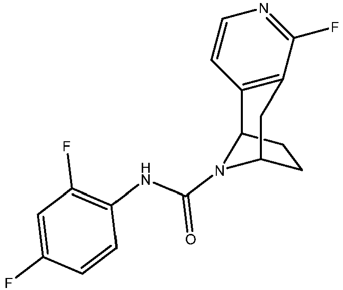 | (162) |
| 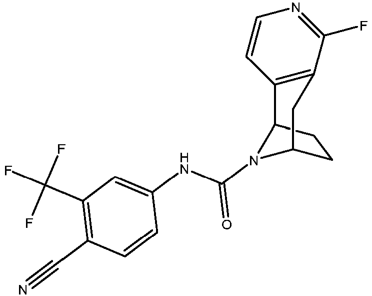 | (163) | 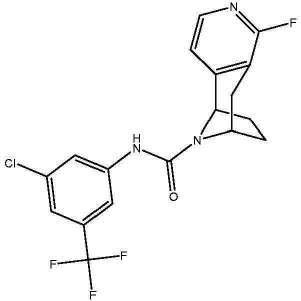 | (164) |
| 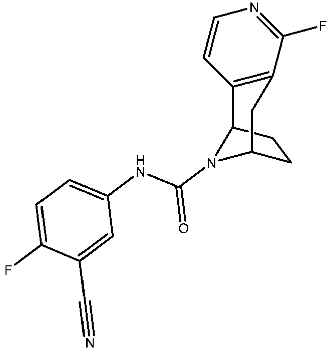 | (165) | 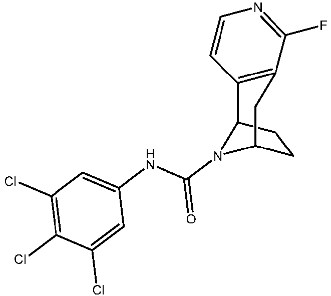 | (166) |
| 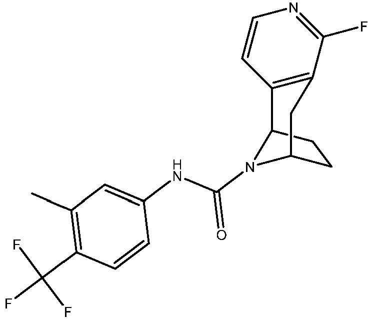 | (167) | 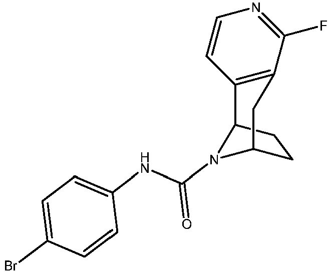 | (168) |
| 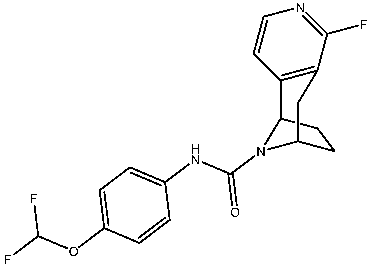 | (169) | 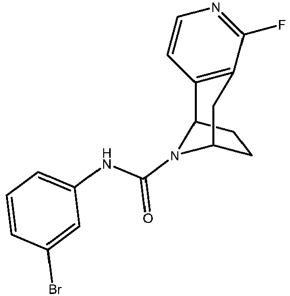 | (170) |
| 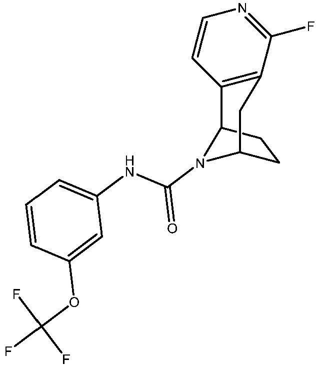 | (171) | 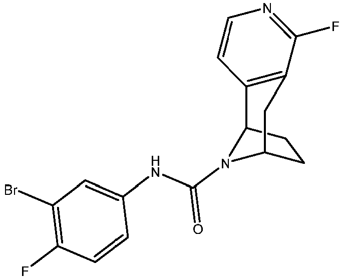 | (172) |
| 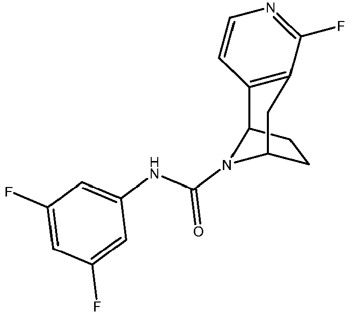 | (173) | 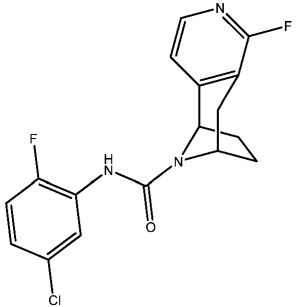 | (174) |
| 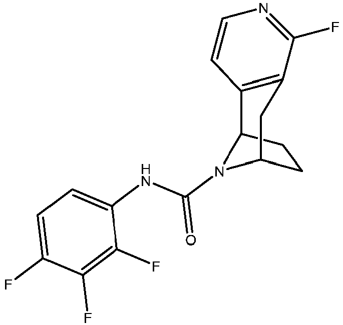 | (175) | 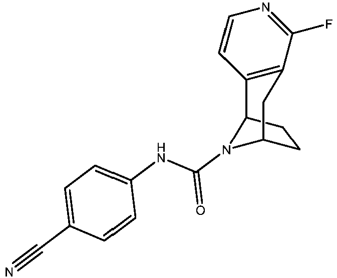 | (176) |
| 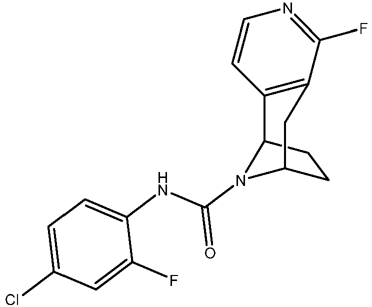 | (177) | 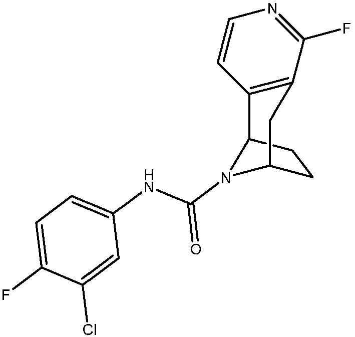 | (178) |
| 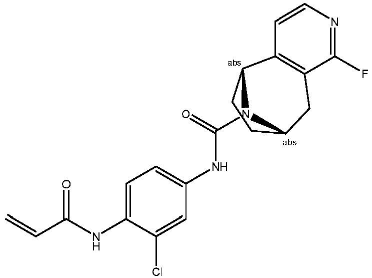 | (179) | 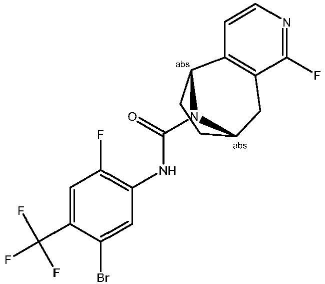 | (180) |
| 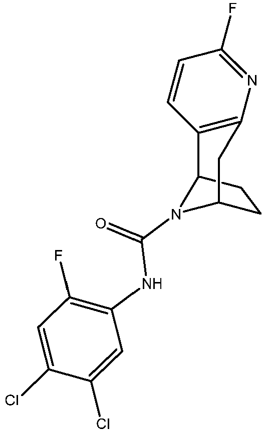 | (181) | 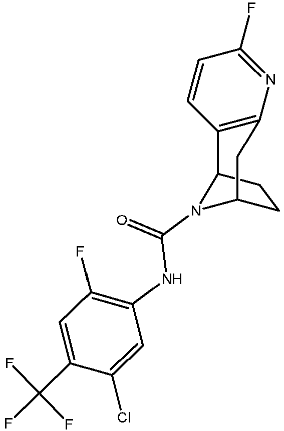 | (182) |
| 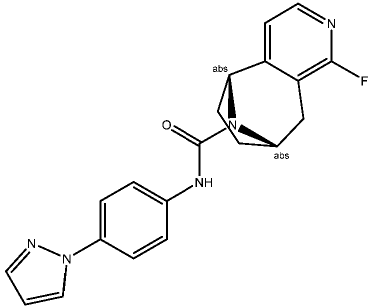 | (183) | 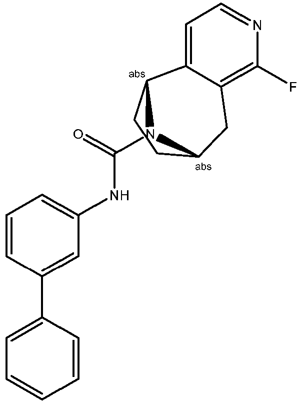 | (184) |
| 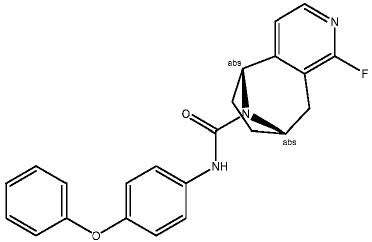 | (185) | 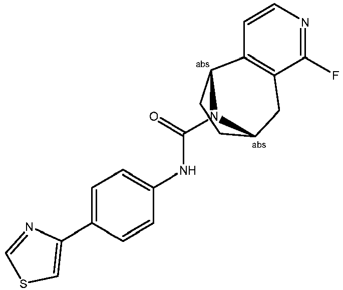 | (186) |
| 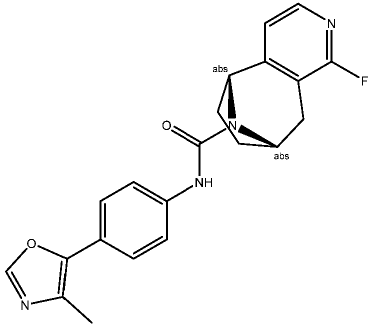 | (187) | 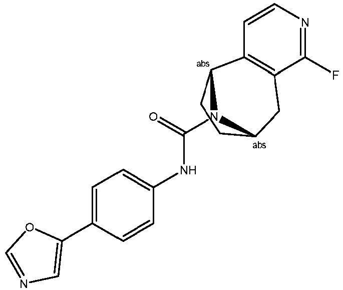 | (188) |
| 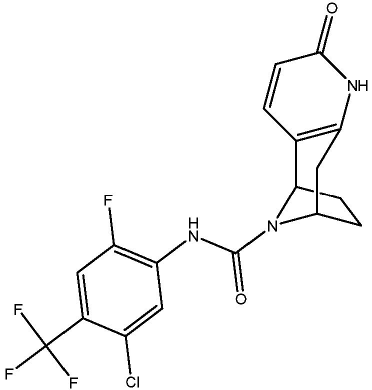 | (189) | 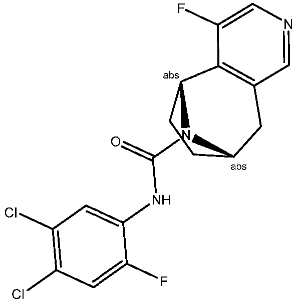 | (190) |
| 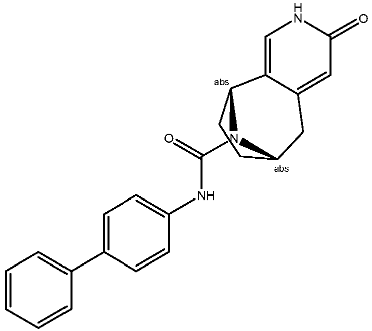 | (191) | 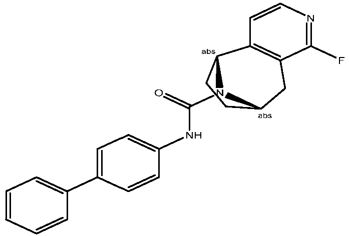 | (192) |
| 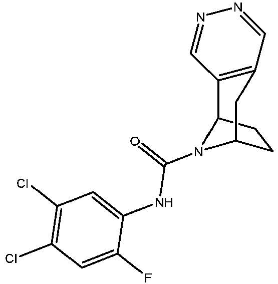 | (193) | 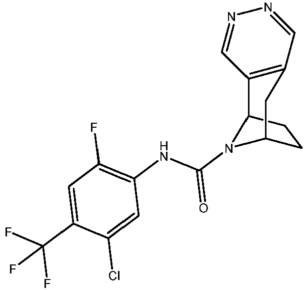 | (194) |
| | (195) | 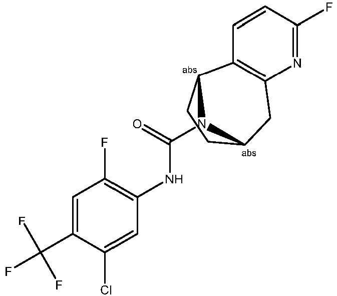 | (196) |
| 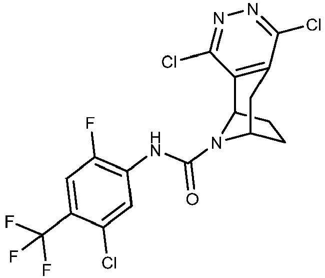 | | | |
| 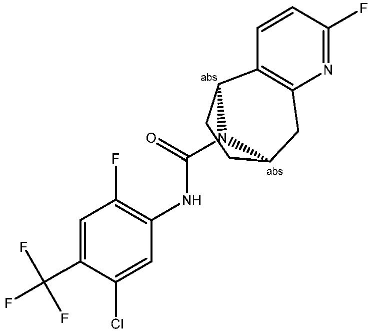 | (197) | 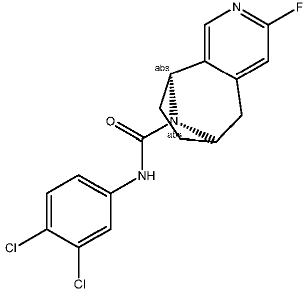 | (198) |
| 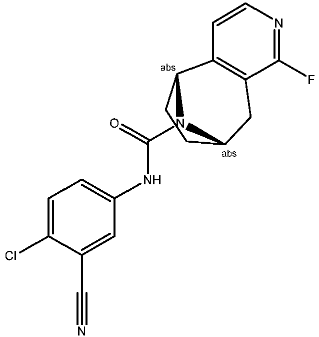 | (199) | 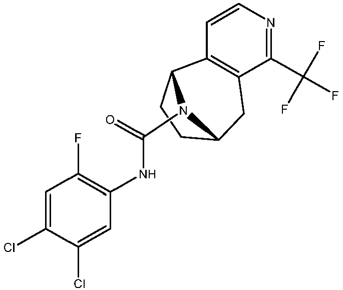 | 200 |

and enantiomers thereof, and mixtures of enantiomers thereof, including racemic mixtures, and pharmaceutically acceptable salts and solvates thereof.

### Compounds of formula (le)

Another aspect of the invention relates to a compound of formula (le), or a pharmaceutically acceptable salt or solvate thereof, 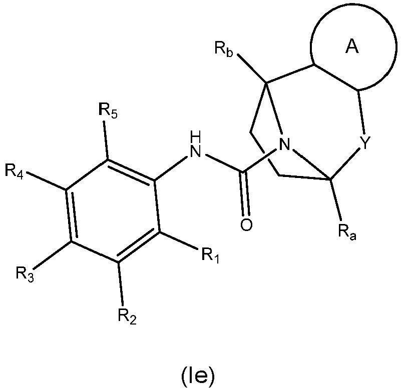 wherein:
ring A is 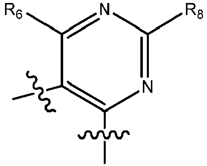 wherein R₆ and R₈ are each independently selected from H, F, CI, Br, I, CN, alkoxy, OH, phenyl, and haloalkyl (more preferably CF₃);
Y is selected from CH₂, C=N-OH, and CR₁₀R₁₀', wherein R₁₀ and R₁₀' are each independently selected from H, F, alkyl, and haloalkyl;
Rₐ and R_{b} are each independently selected from H and alkyl;
R₁, R₄, and R₅ are each independently selected from H, F, Cl, Br, and I; and
R₂ and R₃ are each independently selected from CI, Br, I, CN, and haloalkyl;
with the proviso that that when Y is CH₂, and Rₐ, R_{b}, R₁, R₄, R₅, R₆ and R₈ are all H:
   R₃ is not CN, when R₂ is Cl; and
   R₂ and R₃ are not both CI.

Thus, in one embodiment, the compound of the invention is of formula (le), or a pharmaceutically acceptable salt or solvate thereof: 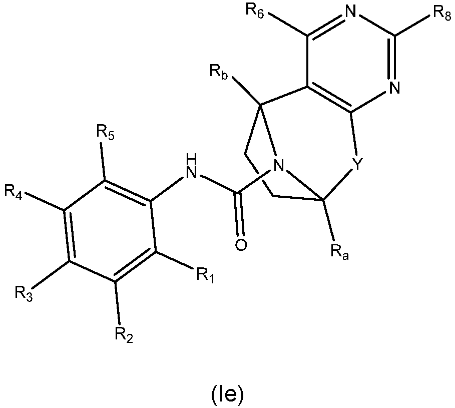 wherein Y, Rₐ, R_{b}, R₁-R₅, R₆ and R₈ are as defined above. Preferably, when Y is CH₂, Rₐ, R_{b}, R₁, R₄, R₅, R₆ and R₈ are H, R₃ is not CN, when R₂ is Cl. Preferably, when Y is CH₂, Rₐ, R_{b}, R₁, R₄, R₅, R₆ and R₈ are H, R₂ and R₃ are not both CI.

Another aspect of the invention relates to a compound, or a pharmaceutically acceptable salt or solvate thereof, having the structure of formula (le) shown above, wherein Y, Rₐ, R_{b}, R₁-R₅, R₆ and R₈ are as defined above, with the proviso that the compound is other than:

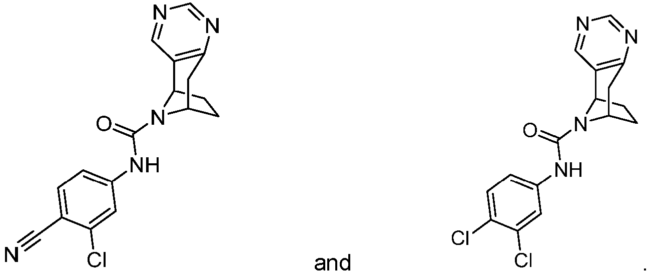

Another aspect of the invention relates to a compound, or a pharmaceutically acceptable salt or solvate thereof, having the structure of formula (le) shown above, wherein Y, Rₐ, R_{b}, R₁-R₅, R₆ and R₈ are as defined above, with the proviso that the compound is other than:
*N*-(3-chloro-4-cyanophenyl)-6,7,8,9-tetrahydro-5H-5,8-epiminocyclohepta[d]-pyrimidine-10-carboxamide; and
*N*-(3,4-dichlorophenyl)-6,7,8,9-tetrahydro-5H-5,8-epiminocyclohepta[d]pyrimidine-10-carboxamide.

Another aspect of the invention relates to a compound, or a pharmaceutically acceptable salt or solvate thereof, having the structure of formula (le) shown above, wherein Y, Rₐ, R_{b}, R₁-R₅, R₆ and R₈ are as defined above, with the proviso that the compound is other than:
*N*-(3-chloro-4-cyanophenyl)-6,7,8,9-tetrahydro-5H-5,8-epiminocyclohepta[d]-pyrimidine-10-carboxamide, and enantiomers thereof, and mixtures of enantiomers thereof; and
*N*-(3,4-dichlorophenyl)-6,7,8,9-tetrahydro-5H-5,8-epiminocyclohepta[d]pyrimidine-10-carboxamide, and enantiomers thereof, and mixtures of enantiomers thereof.

In one preferred embodiment, R₂ is selected from Cl, Br and CF₃, and is more preferably selected from Cl and CF₃.

In one preferred embodiment, R₃ is selected from Cl, Br, CN and CF₃, more preferably selected from Cl, Br and CF₃, and even more preferably selected from Cl and CF₃.

In one preferred embodiment, one of R₂ and R₃ is selected from Cl and the other is CF₃, or R₂ and R₃ are both CI.

In one preferred embodiment, R₁, R₄ and R₅ are all H.

In one preferred embodiment, Y is CR₁₀R₁₀', where R₁₀ and R₁₀' are each independently selected from H, F, Me and CF₃, and more preferably selected from H, F and Me. In one preferred embodiment, Y is selected from CH₂, CHF, CHMe and C=N-OH. In one preferred embodiment, Y is selected from CH₂ and C=N-OH. In one preferred embodiment, Y is selected from CH₂ and CHF. More preferably, Y is CH₂.

In one preferred embodiment, Rₐ and R_{b} are each independently selected from H and methyl. In one preferred embodiment, one of Rₐ and R_{b} is alkyl (more preferably methyl) and the other is H. In one particularly preferred embodiment, Rₐ and R_{b} are both H.

In one preferred embodiment, at least one of R₆ and R₈ is other than H.

In one preferred embodiment, R₆ is H.

In one preferred embodiment, R₈ is selected from H, CF₃, phenyl, OH and F.

In one preferred embodiment, R₈ is OH, and ring A is: 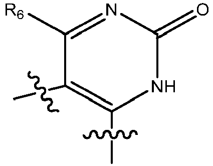 or its corresponding tautomer. Preferably, R₆ is H.

In one particularly preferred embodiment, the compound is of formula (le.1): 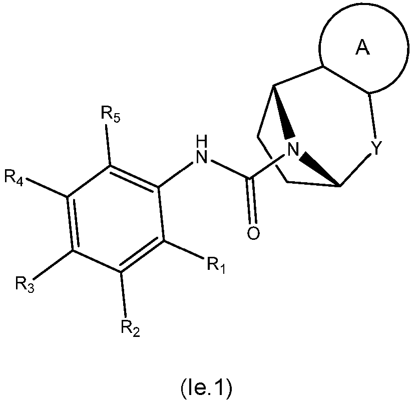 wherein ring A, and groups Y and R₁-R₅ are as described in any of the above embodiments for (le).

In one preferred embodiment, the compound is in enantiomerically pure form. In one preferred embodiment, the compound is in the form of a mixture that is enantiomerically enriched with a compound of formula (le.1).

In another embodiment, the compound is of formula (le.2): 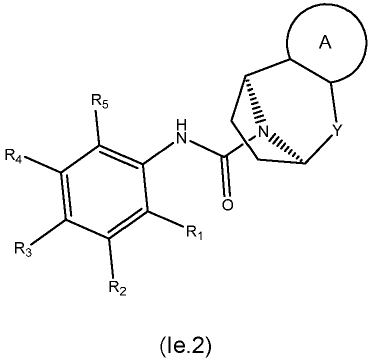 wherein ring A, and groups Y and R₁-R₅ are as described in any of the above embodiments for (le).

In one preferred embodiment, the compound is in enantiomerically pure form. In one preferred embodiment, the compound is in the form of a mixture that is enantiomerically enriched with a compound of formula (le.2).

In one preferred embodiment, the compound is in the form of a mixture comprising a compound of formula (le.1) and its corresponding enantiomer of formula (le.2). In one preferred embodiment, the mixture is a racemic mixture, i.e. a 50:50 mixture of a compound of formula (le.1) and its corresponding enantiomer of formula (le.2).

Racemic mixtures can be used to prepare enantiomerically pure compounds of formula (Ie.1) or (le.2) by separating the compounds of formula (le.1) or (le.2) by standard methods, for example by chemical resolution using optically active acid or by the use of column chromatography or reverse-phase column chromatography using a substantially optically active (or "chiral") stationary phase as known to those skilled in the art. Racemic mixtures can also be used to prepare enantiomerically enriched mixtures of compounds of formula (Ie.1) or (le.2). Mixtures enriched with either a compound of formula (le.1) or (le.2) can also be obtained from the appropriate enantiomerically enriched precursors.

In one preferred embodiment of the invention, the compound is in the form of a mixture comprising enantiomers wherein the weight:weight ratio is at least approximately 2:1 or greater, preferably at least approximately 5:1 or greater, most preferably at least approximately 10:1 or greater in favour of the enantiomer that displays significant *in vitro* and/or *in vivo* activity (the eutomer).

In one particularly preferred embodiment, the compound is in the form of a mixture comprising a compound of formula (le.1) and its corresponding enantiomer of formula (le.2), wherein the weight:weight ratio of said compound of formula (le.1) to said compound of formula (le.2) is greater than 1.05:1, more preferably, greater than 2:1, even more preferably greater than 5:1, even more preferably greater than 10:1.

In one particularly preferred embodiment, the compound is in the form of a mixture comprising a compound of formula (le.1) and its corresponding enantiomer of formula (le.2), which is substantially enriched with said compound of formula (le.1).

In one embodiment, the compound is in the form of a mixture comprising a compound of formula (le.1) and its corresponding enantiomer of formula (le.2), wherein the weight:weight ratio of said compound of formula (le.2) to said compound of formula (le.1) is greater than 1.05:1, more preferably, greater than 2:1, even more preferably greater than 5:1, even more preferably greater than 10:1.

In one embodiment, the compound is in the form of a mixture comprising a compound of formula (le.1) and its corresponding enantiomer of formula (le.2), which is substantially enriched with said compound of formula (le.2).

In one preferred embodiment, the compound is selected from the following:

| | | | |
|---|---|---|---|
| 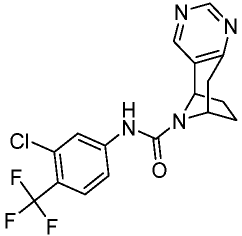 | (2) | | |
| 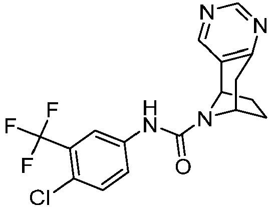 | (3) | 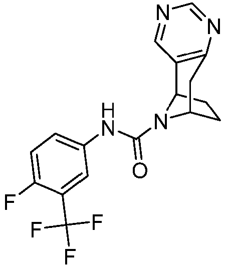 | (5) |
| 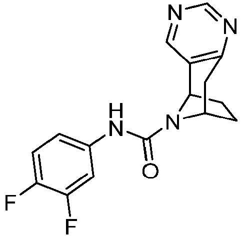 | (6) | 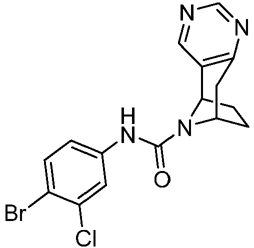 | (7) |
| 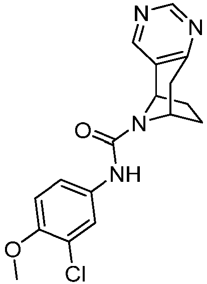 | (8) | 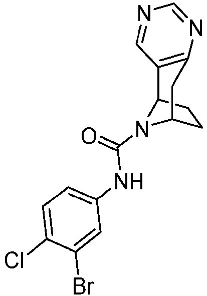 | (12) |
| 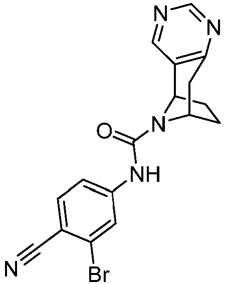 | (13) | 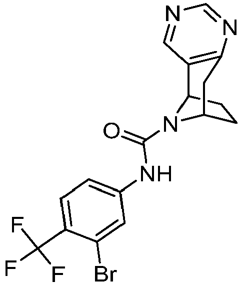 | (15) |
| 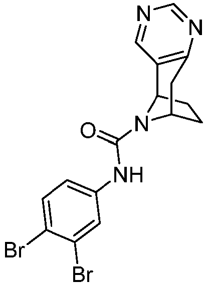 | (16) | 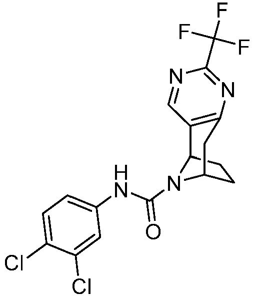 | (24) |
| 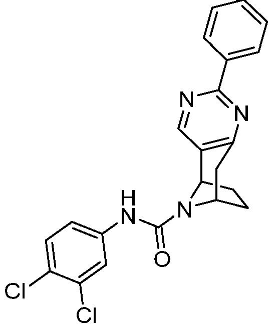 | (26) | 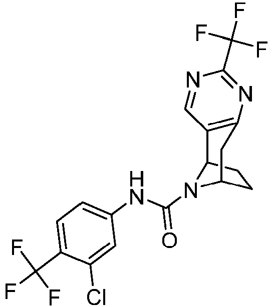 | (27) |
| 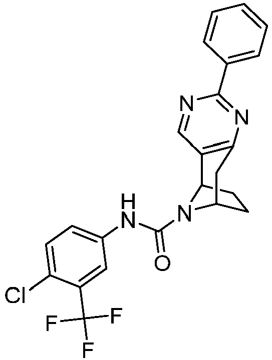 | (28) | 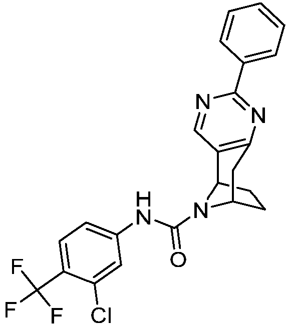 | (29) |
| 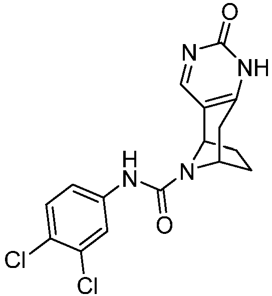 | (31) | 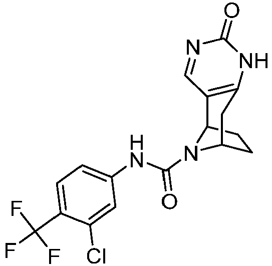 | (32) |
| | | 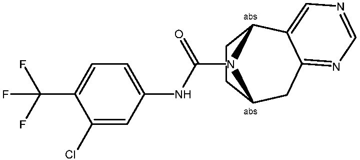 Enantiomer of (2) | (38) |
| 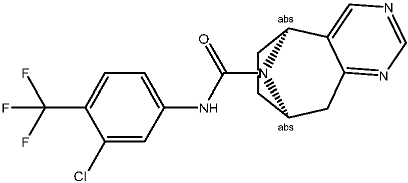 Enantiomer of (2) | (39) | 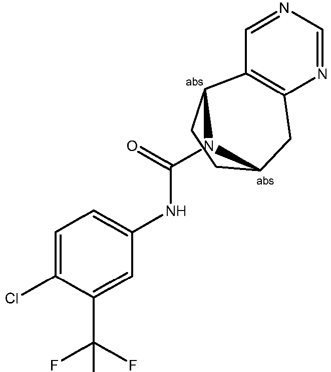 Enantiomer of (3) | (42) |
| 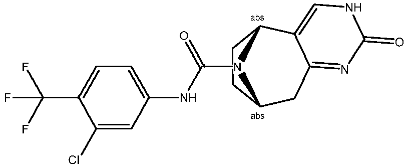 Enantiomer of (32) | (43) | 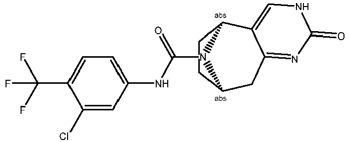 Enantiomer of (32) | (44) |
| 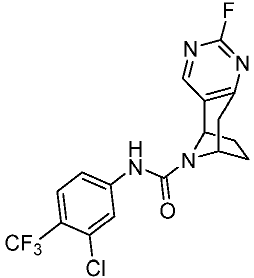 | (45) | 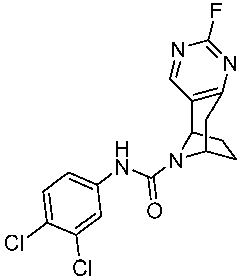 | (46) |
| 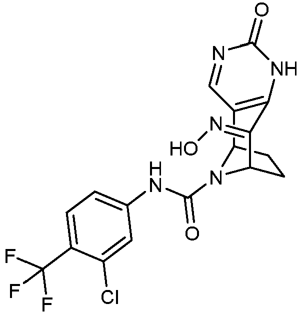 | (47) | 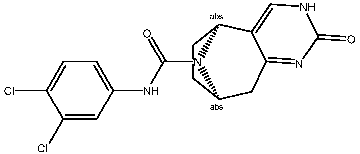 Enantiomer of (31) | (105) |
| 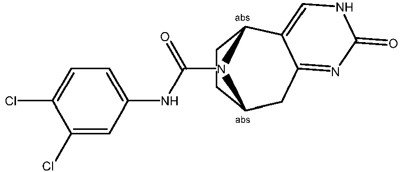 Enantiomer of (31) | (106) | 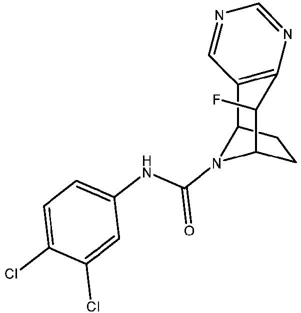 | (113) |
| 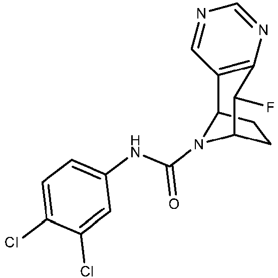 | (114) | 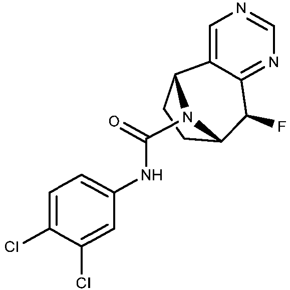 | (115) |

and enantiomers thereof, and mixtures of enantiomers thereof, including racemic mixtures, and pharmaceutically acceptable salts and solvates thereof.

### Compounds of formula (Ia)

One aspect of the invention relates to compounds of formula (la) or a pharmaceutically acceptable salt or solvate thereof, 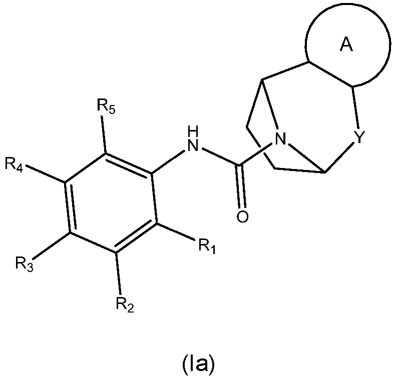 wherein:
ring A is a 5 or 6 membered aromatic or heteroaromatic ring, wherein said aromatic or heteroaromatic ring is optionally substituted with one or more substituents selected from F, Cl, Br, I, CN, alkoxy, NR₁₁R₁₁', OH, alkyl, haloalkyl, aralkyl, aryl, and heteroaryl, and wherein said aryl and heteroaryl substituents are in turn optionally substituted with one or more substituents each independently selected from F, Cl, Br, I, CN, alkoxy, NR₁₁R₁₁', OH, alkyl, haloalkyl, and aralkyl;
Y is selected from CH₂, C=N-OH, and CR₁₀R₁₀', wherein R₁₀ and R₁₀' are each independently selected from H, F, alkyl, and haloalkyl;
R₁, R₄, and R₅ are each independently selected from H, F, Cl, Br, and I;
R₂ and R₃ are each independently selected from H, F, Cl, Br, I, CN, methoxy, and haloalkyl; and
R₁₁ and R₁₁' are each independently selected from H, alkyl, haloalkyl, COR₁₂, and SO₂R₁₃, wherein R₁₂ and R₁₃ are both alkyl;

wherein when Y is CH₂ and ring A is: 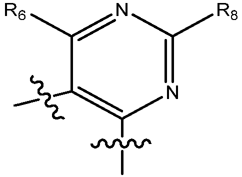
where R₆ and R₈ are both H,
   - R₂ and R₃ are not both Cl, when R₁, R₄ and R₅ are all H;
   - R₃ is not Cl, when R₁, R₂, R₄ and R₅ are all H;
   - R₂ is not Cl, when R₁, R₃, R₄ and R₅ are all H;
   - R₁ is not Cl, when R₂, R₃, R₄ and R₅ are all H;
   - R₁ is not F, when R₄ is Cl and R₂, R₃ and R₅ are all H.

In one preferred embodiment, the optionally substituted aromatic or heteroaromatic ring is a benzene, pyridine, pyridone, pyridine N-oxide, pyridazine, pyrimidine, pyrimidone, pyrazine, triazine, pyrrole, furan, thiophene, pyrazole, isoxazole, imidazole, oxazole, or thiazole ring. The term "heteroaromatic" as used herein also encompasses moieties that exist in tautomeric form, such as, but not limited to, pyridine, pyrimidone and the like. The aromatic or heteroaromatic ring A is fused with the adjacent nitrogen-containing bicyclic heterocyclic group to form a fused tricyclic ring system.

More preferably, the optionally substituted aromatic or heteroaromatic ring is a benzene, pyridine, pyridone, pyridine N-oxide, pyrimidine, pyrimidone, pyridazine, pyrazine, or isoxazole ring.

In one preferred embodiment, ring A is a benzene, pyridine, pyridone, pyridine N-oxide, pyrimidine, pyrimidone, pyridazine, pyrazine, or isoxazole ring that is optionally substituted with one or more substituents selected from F, Cl, Br, I, CN, C₁-C₆ alkoxy, NR₁₁R₁₁', OH, C₁-C₆ alkyl, phenyl, and C₁-C₆ haloalkyl.

In one embodiment, ring A is selected from: 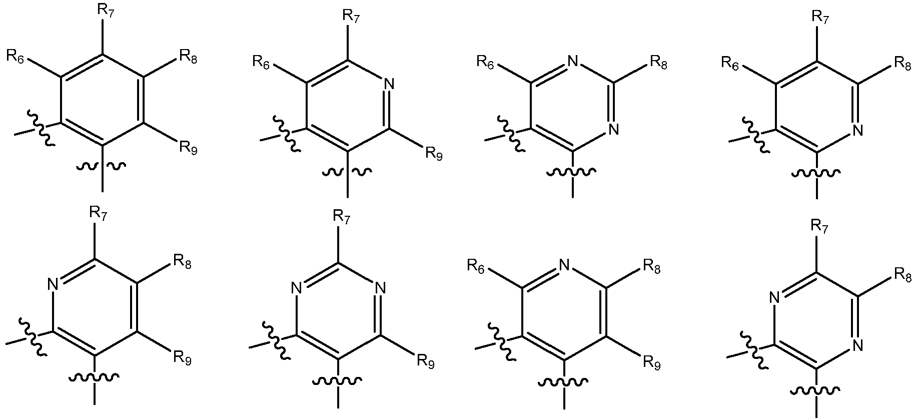 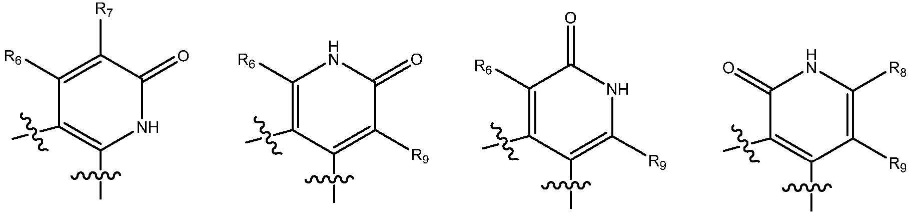 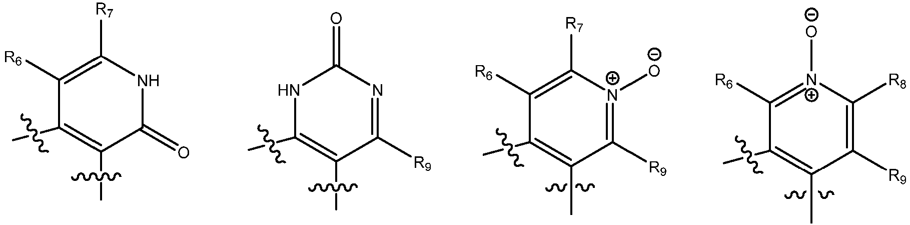 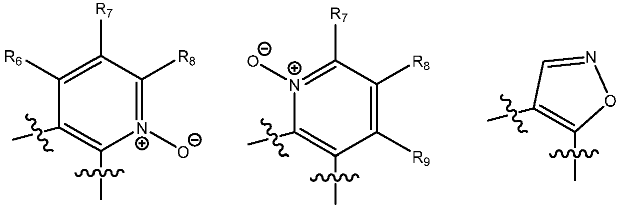 wherein R₆, R₇, R₈, and R₉ are each independently selected from H, F, Cl, Br, I, CN, C₁-C₆ alkoxy, NR₁₁R₁₁', OH, C₁-C₆ alkyl, phenyl, and C₁-C₆ haloalkyl.

In one preferred embodiment, ring A is selected from: 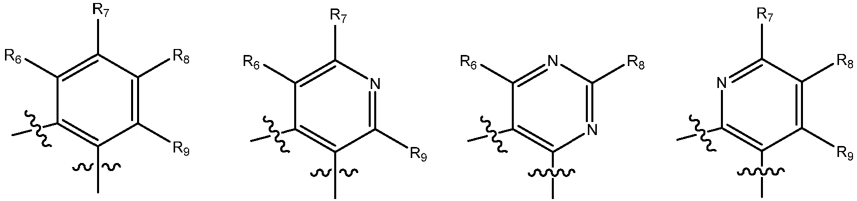 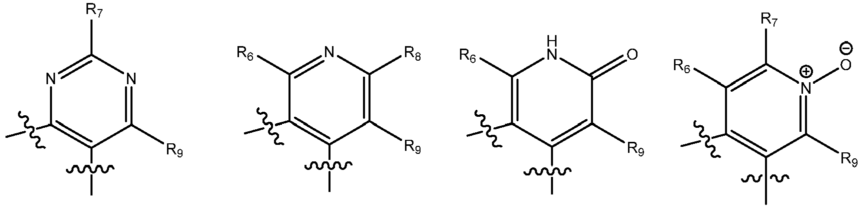 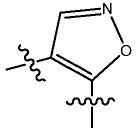 and is more preferably selected from:

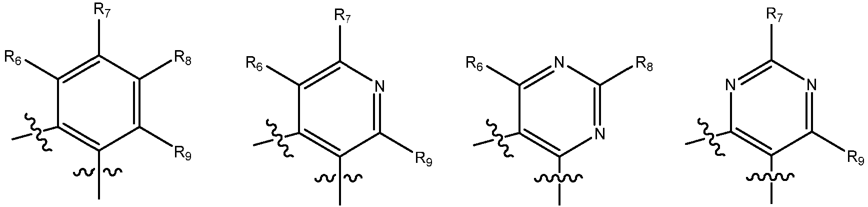

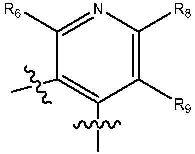

In one preferred embodiment, R₁₀ and R₁₀' are each independently selected from H and C₁-C₆ alkyl, preferably where C₁-C₆ alkyl is CH₃.

In one preferred embodiment, Y is chosen from CH₂ and C=N-OH, and is preferably CH₂.

In one preferred embodiment, R₁ is selected from H and F, and is preferably H.

In one preferred embodiment, R₂ and R₃ are each independently selected from F, Cl, Br, I, CN, and C₁-C₆ haloalkyl.

In one preferred embodiment, R₂ and R₃ are each independently selected from F, Cl, Br, I, CN, and CFₙH₃₋ₙ, where n is 1, 2, or 3, and is preferably 3.

In one preferred embodiment, R₂ and R₃ are each independently selected from Cl, Br, and CFₙH₃₋ₙ, where n is 1, 2, or 3, and is preferably 3.

In one preferred embodiment, R₂ and R₃ are each independently selected from Cl, Br, or CF₃, preferably wherein R₂ and R₃ are not both CF₃, and more preferably wherein R₂ is Cl or Br and R₃ is CF₃.

In one preferred embodiment, R₄ is selected from H and Cl, and is preferably H.

In one preferred embodiment, R₅ is H.

In one preferred embodiment, R₆ is selected from H, F, Cl, CN, methoxy, CH₃, NR₁₁R₁₁', and CF₃, wherein R₁₁ and R₁₁'are each independently selected from H and C₁-C₆ alkyl, and are preferably both H.

In one preferred embodiment, R₆ is selected from H, F, Cl, CN, methoxy, and CH₃, and is preferably H.

In one preferred embodiment, R₇ is selected from H, F, Cl, CN, methoxy, CH₃, NR₁₁R₁₁', and CF₃, wherein R₁₁ and R₁₁'are each independently selected from H and C₁-C₆ alkyl, and are preferably both H.

In one preferred embodiment, R₇ is selected from H, F, Cl, CN, methoxy, and CH₃, preferably H, F, or Cl, and is more preferably H.

In one preferred embodiment, R₈ is selected from H, F, OH, CN, methoxy, NR₁₁R₁₁', phenyl, CF₃, CF₂H, NHSO₂CH₃, NHCOCH₃, and NHCHF₂, wherein R₁₁ and R₁₁' are each independently selected from H and C₁-C₆ alkyl and are preferably both H.

In one preferred embodiment, R₈ is selected from H, F, Cl, CN, phenyl, and OH, preferably from H, F, and OH.

In one preferred embodiment, R₈ is OH.

In one preferred embodiment, R₉ is selected from H, F, Cl, CN, methoxy, CH₃, NR₁₁R₁₁', and CF₃, wherein R₁₁ and R₁₁'are each independently selected from H and C₁-C₆ alkyl, and are preferably both H.

In one preferred embodiment, R₉ is selected from H, F, Cl, CN, methoxy, and CH₃, preferably from H, F, and CN, and is more preferably H.

In one preferred embodiment, R₆, R₇, and R₉ are, if present, H.

In a particularly preferred embodiment, the compound of formula (Ia) is 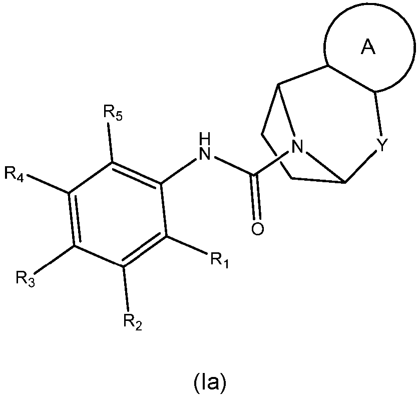 wherein:
ring A is selected from: 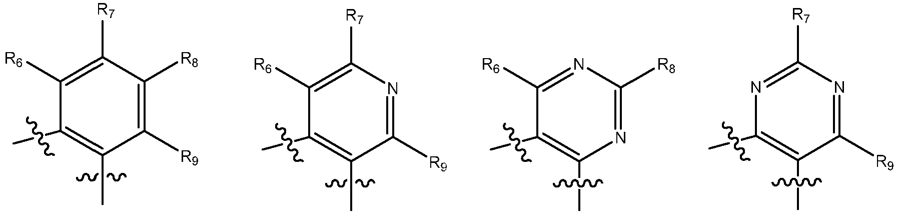 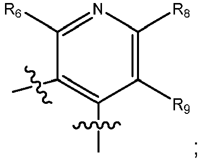
Y is CH₂;
R₁, R₄, R₅, R₆, R₇, and R₉ are all H;
R₂ and R₃ are each independently selected from Cl, Br, or CF₃, preferably wherein R₂ and R₃ are not both CF₃, and more preferably wherein R₂ is Cl or Br and R₃ is CF₃; and
R₈ is selected from H, F, Cl, CN, phenyl, and OH, preferably from H, F, and OH, and is more preferably OH;

preferably wherein when ring A is: 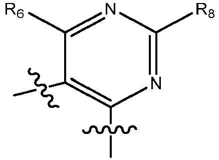
and R₈ is H: R₂ and R₃ are not both Cl.

In an even more preferred embodiment, the compound of formula (la) is 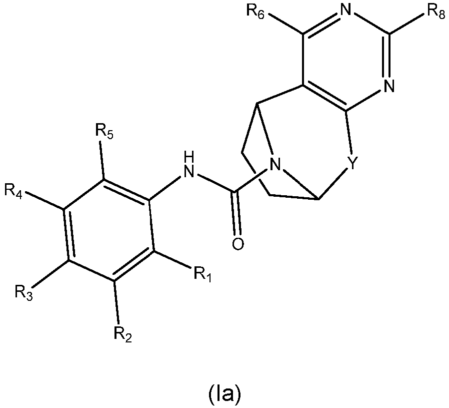 wherein:
Y is CH₂;
R₁, R₄, R₅, and R₆, are all H;
R₂ and R₃ are each independently selected from Cl, Br, or CF₃, preferably wherein R₂ and R₃ are not both CF₃, and more preferably wherein R₂ is Cl or Br and R₃ is CF₃; and
R₈ is selected from H, F, Cl, CN, phenyl, and OH, preferably from H, F, and OH, and is more preferably OH;
preferably wherein when R₈ is H: R₂ and R₃ are not both CI.

In one particularly preferred embodiment, the compound is of formula (la.1): 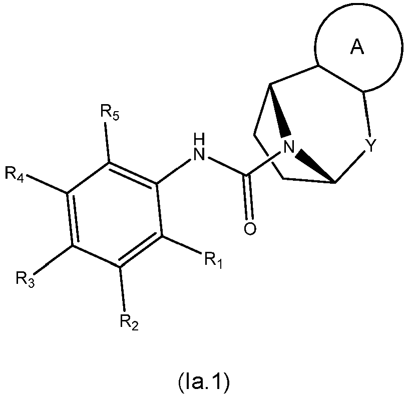 wherein ring A, and groups Y and R₁-R₁₃ are as described in any of the above embodiments. In one preferred embodiment, the compound is in enantiomerically pure form.

In another embodiment, the compound is of formula (la.2): 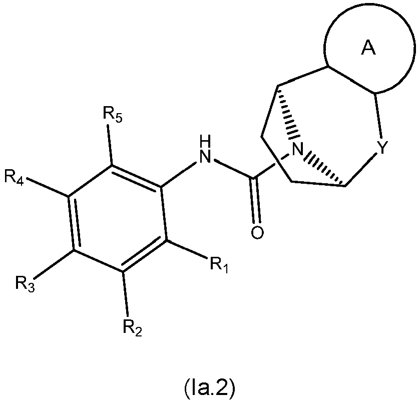 wherein ring A, and groups Y and R₁-R₁₃ are as described in any of the above embodiments. In one preferred embodiment, the compound is in enantiomerically pure form.

In one preferred embodiment, the compound is in the form of a mixture comprising a compound of formula (la.1) and its corresponding enantiomer of formula (la.2). In one preferred embodiment, the mixture is a racemic mixture, i.e. a 50:50 mixture of a compound of formula (la.1) and its corresponding enantiomer of formula (la.2).

Racemic mixtures can be used to prepare enantiomerically pure compounds of formula (Ia.1) or (la.2) by separating the compounds of formula (la.1) or (la.2) by standard methods, for example by chemical resolution using optically active acid or by the use of column chromatography or reverse-phase column chromatography using a substantially optically active (or "chiral") stationary phase as known to those skilled in the art. Racemic mixtures can also be used to prepare enantiomerically enriched mixtures of compounds of formula (Ia.1) or (la.2). Mixtures enriched with either a compound of formula (la.1) or (la.2) can also be obtained from the appropriate enantiomerically enriched precursors.

In one preferred embodiment of the invention, the compound is in the form of a mixture comprising enantiomers wherein the weight:weight ratio is at least approximately 2:1 or greater, preferably at least approximately 5:1 or greater, most preferably at least approximately 10:1 or greater in favour of the enantiomer that displays significant *in vitro* and/or *in vivo* activity (the eutomer).

In one particularly preferred embodiment, the compound is in the form of a mixture comprising a compound of formula (la.1) and its corresponding enantiomer of formula (la.2), wherein the weight:weight ratio of said compound of formula (la.1) to said compound of formula (la.2) is greater than 1.05:1, more preferably, greater than 2:1, even more preferably greater than 5:1, even more preferably greater than 10:1.

In one particularly preferred embodiment, the compound is in the form of a mixture comprising a compound of formula (la.1) and its corresponding enantiomer of formula (la.2), which is substantially enriched with said compound of formula (la.1).

In one embodiment, the compound is in the form of a mixture comprising a compound of formula (la.1) and its corresponding enantiomer of formula (la.2), wherein the weight:weight ratio of said compound of formula (la.2) to said compound of formula (la.1) is greater than 1.05:1, more preferably, greater than 2:1, even more preferably greater than 5:1, even more preferably greater than 10:1.

In one embodiment, the compound is in the form of a mixture comprising a compound of formula (la.1) and its corresponding enantiomer of formula (la.2), which is substantially enriched with said compound of formula (la.2).

In one preferred embodiment, the compound of formula (Ia) is selected from the following compounds as shown herein:
(1), (2), (3), (4), (5), (6), (7), (8), (11), (12), (13), (15), (16), (17), (18), (24), (25), (26), (27), (28), (29), (31), (32), (34), (35), (36), (37), (45), (46) and (47)
and enantiomers thereof, and mixtures of enantiomers thereof, including racemic mixtures, and pharmaceutically acceptable salts and solvates thereof.

In another preferred embodiment, the compound of formula (la) is selected from the following:
(±)-*N*-(4-(trifluoromethyl)phenyl)-6,7,8,9-tetrahydro-5H-5,8-epiminocyclohepta[d]pyrimidine-10-carboxamide;
(*5S*,*8*R)-*N*-(4-(trifluoromethyl)phenyl)-6,7,8,9-tetrahydro-5H-5,8-epiminocyclohepta[d]-pyrimidine-10-carboxamide;
(*5R 8S*)-*N*-(4-(trifluoromethyl)phenyl)-6,7,8,9-tetrahydro-5H-5,8-epiminocyclohepta[d]-pyrimidine-10-carboxamide;
(±)-*N*-(3-chloro-4-(trifluoromethyl)phenyl)-6,7,8,9-tetrahydro-5H-5,8-epiminocyclo-hepta[d]pyrimidine-10-carboxamide;
(*5S*,*8R*)-*N*-(3-chloro-4-(trifluoromethyl)phenyl)-6,7,8,9-tetrahydro-5H-5,8-epimino-cyclohepta[d]pyrimidine-10-carboxamide;
(*5R*,*8S*)-*N*-(3-chloro-4-(trifluoromethyl)phenyl)-6,7,8,9-tetrahydro-5H-5,8-epimino-cyclohepta[d]pyrimidine-10-carboxamide;
(±)-*N*-(4-chloro-3-(trifluoromethyl)phenyl)-6,7,8,9-tetrahydro-5H-5,8-epimino-cyclohepta[d]pyrimidine-10-carboxamide;
(*5*S,8R)-*N*-(4-chloro-3-(trifluoromethyl)phenyl)-6,7,8,9-tetrahydro-5H-5,8-epimino-cyclohepta[d]pyrimidine-10-carboxamide;
(*5R*,*8S*)-*N*-(4-chloro-3-(trifluoromethyl)phenyl)-6,7,8,9-tetrahydro-5H-5,8-epimino-cyclohepta[d]pyrimidine-10-carboxamide;
(±)-*N*-(3,5-dichlorophenyl)-6,7,8,9-tetrahydro-5H-5,8-epiminocyclohepta[d]pyrimidine-10-carboxamide;
(*5S*,*8R*)-*N*-(3,5-dichlorophenyl)-6,7,8,9-tetrahydro-5H-5,8-epiminocyclohepta[d]pyrimidine-10-carboxamide;
(*5R*,*8S*)-*N*-(3,5-dichlorophenyl)-6,7,8,9-tetrahydro-5H-5,8-epiminocyclohepta[d]pyrimidine-10-carboxamide;
(±)-*N*-(4-fluoro-3-(trifluoromethyl)phenyl)-6,7,8,9-tetrahydro-5H-5,8-epiminocyclohepta[d]-pyrimidine-10-carboxamide;
(*5S*,*8R*)*-N*-(4-fluoro-3-(trifluoromethyl)phenyl)-6,7,8,9-tetrahydro-5H-5,8-epimino-cyclohepta[d]pyrimidine-10-carboxamide;
(*5R*,*8S*)-*N*-(4-fluoro-3-(trifluoromethyl)phenyl)-6,7,8,9-tetrahydro-5H-5,8-epimino-cyclohepta[d]pyrimidine-10-carboxamide;
(±)-*N*-(3,4-difluorophenyl)-6,7,8,9-tetrahydro-5H-5,8-epiminocyclohepta[d]pyrimidine-10-carboxamide;
(*5S*,*8R*)-*N*-(3,4-difluorophenyl)-6,7,8,9-tetrahydro-5H-5,8-epiminocyclohepta[d]pyrimidine-10-carboxamide;
(*5R*,*8S*)-*N*-(3,4-difluorophenyl)-6,7,8,9-tetrahydro-5H-5,8-epiminocyclohepta[d]pyrimidine-10-carboxamide;
(±)-*N*-(4-bromo-3-chlorophenyl)-6,7,8,9-tetrahydro-5H-5,8-epiminocyclohepta[d]pyrimidine-10-carboxamide;
(5S, *8R*)-*N*-(4-bromo-3-chlorophenyl)-6,7,8,9-tetrahydro-5H-5,8-epiminocyclohepta[d]-pyrimidine-10-carboxamide;
(*5R*,*8S*)-*N*-(4-bromo-3-chlorophenyl)-6,7,8,9-tetrahydro-5H-5,8-epiminocyclohepta[d]-pyrimidine-10-carboxamide;
(±)-*N*-(3-chloro-4-methoxyphenyl)-6,7,8,9-tetrahydro-5H-5,8-epiminocyclohepta[d]-pyrimidine-10-carboxamide;
(*5S*,*8R*)-*N*-(3-chloro-4-methoxyphenyl)-6,7,8,9-tetrahydro-5H-5,8-epiminocyclohepta[d]-pyrimidine-10-carboxamide;
(*5R*,*8S*)-*N*-(3-chloro-4-methoxyphenyl)-6,7,8,9-tetrahydro-5H-5,8-epiminocyclohepta[d]-pyrimidine-10-carboxamide;
(±)-*N*-(3-bromo-4-chlorophenyl)-6,7,8,9-tetrahydro-5H-5,8-epiminocyclohepta[d]pyrimidine-10-carboxamide;
(*5S*,*8R*)-*N*-(3-bromo-4-chlorophenyl)-6,7,8,9-tetrahydro-5H-5,8-epiminocyclohepta[d]-pyrimidine-10-carboxamide;
(*5R*,*8S*)-*N*-(3-bromo-4-chlorophenyl)-6,7,8,9-tetrahydro-5H-5,8-epiminocyclohepta[d]-pyrimidine-10-carboxamide;
(±)-*N*-(3-bromo-4-cyanophenyl)-6,7,8,9-tetrahydro-5H-5,8-epiminocyclohepta[d]pyrimidine-10-carboxamide;
(*5S*,*8R*)-*N*-(3-bromo-4-cyanophenyl)-6,7,8,9-tetrahydro-5H-5,8-epiminocyclohepta[d]-pyrimidine-10-carboxamide;
(*5R*,*8S*)-*N*-(3-bromo-4-cyanophenyl)-6,7,8,9-tetrahydro-5H-5,8-epiminocyclohepta[d]-pyrimidine-10-carboxamide;
(±)-*N*-(3,4-dibromophenyl)-6,7,8,9-tetrahydro-5H-5,8-epiminocyclohepta[d]pyrimidine-10-carboxamide;
(*5S*,*8R*)-*N*-(3,4-dibromophenyl)-6,7,8,9-tetrahydro-5H-5,8-epiminocyclohepta[d]pyrimidine-10-carboxamide;
(*5R*,*8S*)-*N*-(3,4-dibromophenyl)-6,7,8,9-tetrahydro-5H-5,8-epiminocyclohepta[d]pyrimidine-10-carboxamide;
(±)-*N*-(3,4-dichlorophenyl)-6,7,8,9-tetrahydro-5H-6,9-epiminocyclohepta[d]pyrimidine-10-carboxamide;
(*5S*,*8R*)-*N*-(3,4-dichlorophenyl)-6,7,8,9-tetrahydro-5H-6,9-epiminocyclohepta[d]pyrimidine-10-carboxamide;
(*5R*,*8S*)-*N*-(3,4-dichlorophenyl)-6,7,8,9-tetrahydro-5H-6,9-epiminocyclohepta[d]pyrimidine-10-carboxamide;
(±)-*N*-(3-chloro-4-(trifluoromethyl)phenyl)-6,7,8,9-tetrahydro-5H-6,9-epiminocyclohepta[d]-pyrimidine-10-carboxamide;
(*5S,8R)*-*N*-(3-chloro-4-(trifluoromethyl)phenyl)-6,7,8,9-tetrahydro-5H-6,9-epimino-cyclohepta[d]pyrimidine-10-carboxamide;
(*5R,8S*)-*N*-(3-chloro-4-(trifluoromethyl)phenyl)-6,7,8,9-tetrahydro-5H-6,9*-*epimino-cyclohepta[d]pyrimidine-10-carboxamide;
(±)-*N*-(3,4-dichlorophenyl)-2-(trifluoromethyl)-6,7,8,9-tetrahydro-5H-5,8-epimino-cyclohepta[d]pyrimidine-10-carboxamide;
(*5S*,*8R*)-*N*-(3,4-dichlorophenyl)-2-(trifluoromethyl)-6,7,8,9-tetrahydro-5H-5,8-epimino-cyclohepta[d]pyrimidine-10-carboxamide;
(*5R,8S*)-*N*-(3,4-dichlorophenyl)-2-(trifluoromethyl)-6,7,8,9-tetrahydro-5H-5,8-epimino-cyclohepta[d]pyrimidine-10-carboxamide;
(±)-*N*-(3,4-dichlorophenyl)-2-methyl-6,7,8,9-tetrahydro-5H-5,8-epiminocyclohepta[d]-pyrimidine-10-carboxamide;
(*5S*,*8R*)-*N*-(3,4-dichlorophenyl)-2-methyl-6,7,8,9-tetrahydro-5H-5,8-epiminocyclohepta[d]-pyrimidine-10-carboxamide;
(*5R*,*8S*)-*N*-(3,4-dichlorophenyl)-2-methyl-6,7,8,9-tetrahydro-5H-5,8-epiminocyclohepta[d]-pyrimidine-10-carboxamide;
(±)-*N*-(3,4-dichlorophenyl)-2-phenyl-6,7,8,9-tetrahydro-5H-5,8-epiminocyclohepta[d]-pyrimidine-10-carboxamide;
(*5S*,*8R*)-*N*-(3,4-dichlorophenyl)-2-phenyl-6,7,8,9-tetrahydro-5H-5,8-epiminocyclohepta[d]-pyrimidine-10-carboxamide;
(*5R*,*8S*)-*N*-(3,4-dichlorophenyl)-2-phenyl-6,7,8,9-tetrahydro-5H-5,8-epiminocyclohepta[d]-pyrimidine-10-carboxamide;
(±)-*N*-(3-chloro-4-(trifluoromethyl)phenyl)-2-(trifluoromethyl)-6,7,8,9-tetrahydro-5H-5,8-epiminocyclohepta[d]pyrimidine-10-carboxamide;
(*5S*,*8R*)-*N*-(3-chloro-4-(trifluoromethyl)phenyl)-2-(trifluoromethyl)-6,7,8,9-tetrahydro-5H-5,8-epiminocyclohepta[d]pyrimidine-10-carboxamide;
(*5R*,*8S*)-*N*-(3-chloro-4-(trifluoromethyl)phenyl)-2-(trifluoromethyl)-6,7,8,9-tetrahydro-5H-5,8-epiminocyclohepta[d]pyrimidine-10-carboxamide;
(±)-*N*-(4-chloro-3-(trifluoromethyl)phenyl)-2-phenyl-6,7,8,9-tetrahydro-5H-5,8-epimino-cyclohepta[d]pyrimidine-10-carboxamide;
(*5S*,*8R*)-*N*-(4-chloro-3-(trifluoromethyl)phenyl)-2-phenyl-6,7,8,9-tetrahydro-5H-5,8-epimino-cyclohepta[d]pyrimidine-10-carboxamide;
(*5R*,*8S*)-*N*-(4-chloro-3-(trifluoromethyl)phenyl)-2-phenyl-6,7,8,9-tetrahydro-5H-5,8-epimino-cyclohepta[d]pyrimidine-10-carboxamide;
(±)-*N*-(3-chloro-4-(trifluoromethyl)phenyl)-2-phenyl-6,7,8,9-tetrahydro-5H-5,8-epimino-cyclohepta[d]pyrimidine-10-carboxamide;
(*5S*,*8R*)-*N*-(3-chloro-4-(trifluoromethyl)phenyl)-2-phenyl-6,7,8,9-tetrahydro-5H-5,8-epimino-cyclohepta[d]pyrimidine-10-carboxamide;
(*5R,8S*)-*N*-(3-chloro-4-(trifluoromethyl)phenyl)-2-phenyl-6,7,8,9-tetrahydro-5H-5,8-epimino-cyclohepta[d]pyrimidine-10-carboxamide;
(±)-*N*-(3,4-dichlorophenyl)-2-hydroxy-6,7,8,9-tetrahydro-5H-5,8-epiminocyclohepta[d]-pyrimidine-10-carboxamide;
(*5S*,*8R*)-*N*-(3,4-dichlorophenyl)-2-hydroxy-6,7,8,9-tetrahydro-5H-5,8-epiminocyclohepta[d]-pyrimidine-10-carboxamide;
(*5R,8S*)-*N*-(3,4-dichlorophenyl)-2-hydroxy-6,7,8,9-tetrahydro-5H-5,8-epiminocyclohepta[d]-pyrimidine-10-carboxamide;
(±)-*N*-(3-chloro-4-(trifluoromethyl)phenyl)-2-hydroxy-6,7,8,9-tetrahydro-5H-5,8-epimino-cyclohepta[d]pyrimidine-10-carboxamide;
(*5S*,*8R*)-*N*-(3-chloro-4-(trifluoromethyl)phenyl)-2-hydroxy-6,7,8,9-tetrahydro-5H-5,8-epimino-cyclohepta[d]pyrimidine-10-carboxamide;
(*5R*,*8S*)-*N*-(3-chloro-4-(trifluoromethyl)phenyl)-2-hydroxy-6,7,8,9-tetrahydro-5H-5,8-epimino-cyclohepta[d]pyrimidine-10-carboxamide;
(±)-*N*-(3,4-dichlorophenyl)-6,7,8,9-tetrahydro-5H-5,8-epiminobenzo[7]annulene-10-carboxamide;
(*5S*,*8R*)-*N*-(3,4-dichlorophenyl)-6,7,8,9-tetrahydro-5H-5,8-epiminobenzo[7]annulene-10-carboxamide;
(*5R*,*8S*)-*N*-(3,4-dichlorophenyl)-6,7,8,9-tetrahydro-5H-5,8-epiminobenzo[7]annulene-10-carboxamide;
(±)-*N*-(3-chloro-4-(trifluoromethyl)phenyl)-6,7,8,9-tetrahydro-5H-5,8-epiminocyclohepta[c]-pyridine-10-carboxamide;
(*5S,8R*)-*N*-(3-chloro-4-(trifluoromethyl)phenyl)-6,7,8,9-tetrahydro-5H-5,8-epimino-cyclohepta[c]-pyridine-10-carboxamide;
(*5R,8S*)-*N*-(3-chloro-4-(trifluoromethyl)phenyl)-6,7,8,9-tetrahydro-5H-5,8-epimino-cyclohepta[c]pyridine-10-carboxamide;
(±)-*N*-(3,4-dichlorophenyl)-6,7,8,9-tetrahydro-5H-5,8-epiminocyclohepta[c]pyridine-10-carboxamide;
(*5S,8R*)-*N*-(3,4-dichlorophenyl)-6,7,8,9-tetrahydro-5H-5,8-epiminocyclohepta[c]pyridine-10-carboxamide;
(*5R*,*8S*)-*N*-(3,4-dichlorophenyl)-6,7,8,9-tetrahydro-5H-5,8-epiminocyclohepta[c]pyridine-10-carboxamide;
(±)-*N*-(3-chloro-4-(trifluoromethyl)phenyl)-6,7,8,9-tetrahydro-5H-6,9-epiminocyclohepta[c]-pyridine-10-carboxamide;
(*5S*,*8R*)-*N*-(3-chloro-4-(trifluoromethyl)phenyl)-6,7,8,9-tetrahydro-5H-6,9-epimino-cyclohepta[c]pyridine-10-carboxamide;
(*5R*,*8S*)-*N-*(3-chloro-4-(trifluoromethyl)phenyl)-6,7,8,9-tetrahydro-5H-6,9-epimino-cyclohepta[c]pyridine-10-carboxamide;
(±)-*N*-(3-chloro-4-(trifluoromethyl)phenyl)-2-fluoro-6,7,8,9-tetrahydro-5H-5,8-epimino-cyclohepta[d]pyrimidine-10-carboxamide;
*(5S, 8R)-N-(3-chloro-4-(trifluoromethyl) phenyl)-2-fluoro-6, 7 ,8, 9-tetrahydro-5H-5, 8-*epiminocyclohepta[d]pyrimidine-10-carboxamide;
(*5R*,*8S*)-*N*-(3-chloro-4-(trifluoromethyl)phenyl)-2-fluoro-6,7,8,9-tetrahydro-5H-5,8-epiminocyclohepta[d]pyrimidine-10-carboxamide;
(±)-*N*-(3,4-dichlorophenyl)-2-fluoro-6,7,8,9-tetrahydro-5H-5,8-epiminocyclohepta[d]-pyrimidine-10-carboxamide;
(*5S,8R*)-*N*-(3,4-dichlorophenyl)-2-fluoro-6,7,8,9-tetrahydro-5H-5,8-epiminocyclohepta[d]-pyrimidine-10-carboxamide;
(*5R*,*8S*)-*N*-(3,4-dichlorophenyl)-2-fluoro-6,7,8,9-tetrahydro-5H-5,8-epiminocyclohepta[d]-pyrimidine-10-carboxamide;
(±)-*N*-(3-chloro-4-(trifluoromethyl)phenyl)-2-hydroxy-9-(hydroxyimino)-6,7,8,9-tetrahydro-5H-5,8-epiminocyclohepta[d]pyrimidine-10-carboxamide;
(*5S*,*8R*)-*N*-(3-chloro-4-(trifluoromethyl)phenyl)-2-hydroxy-9-(hydroxyimino)-6,7,8,9-tetrahydro-5H-5,8-epiminocyclohepta[d]pyrimidine-10-carboxamide;
(*5R*,*8S*)-*N-*(3-chloro-4-(trifluoromethyl)phenyl)-2-hydroxy-9-(hydroxyimino)-6,7,8,9-tetrahydro-5H-5,8-epiminocyclohepta[d]pyrimidine-10-carboxamide;
(±)-*N*-(3-chloro-4-cyanophenyl)-6,7,8,9-tetrahydro-5H-5,8-epiminocyclohepta[d]pyrimidine-10-carboxamide;
(*5S*,*8R*)-*N*-(3-chloro-4-cyanophenyl)-6,7,8,9-tetrahydro-5H-5,8-epiminocyclohepta[d]-pyrimidine-10-carboxamide;
(*5R,8S*)-*N*-(3-chloro-4-cyanophenyl)-6,7,8,9-tetrahydro-5H-5,8-epiminocyclohepta[d]-pyrimidine-10-carboxamide;
(±)-*N*-(3-bromo-4-(trifluoromethyl)phenyl)-6,7,8,9-tetrahydro-5H-5,8-epiminocyclohepta[d]-pyrimidine-10-carboxamide;
(*5S*,*8R*)-*N*-(3-bromo-4-(trifluoromethyl)phenyl)-6,7,8,9-tetrahydro-5H-5,8-epimino-cyclohepta[d]pyrimidine-10-carboxamide; and
(*5R,8S*)-*N*-(3-bromo-4-(trifluoromethyl)phenyl)-6,7,8,9-tetrahydro-5H-5,8-epimino-cyclohepta[d]pyrimidine-10-carboxamide;
and pharmaceutically acceptable salts and solvates thereof.

### Compounds of formula (Ic)

Another aspect of the invention relates to a compound of formula (Ic), or a pharmaceutically acceptable salt or solvate thereof, 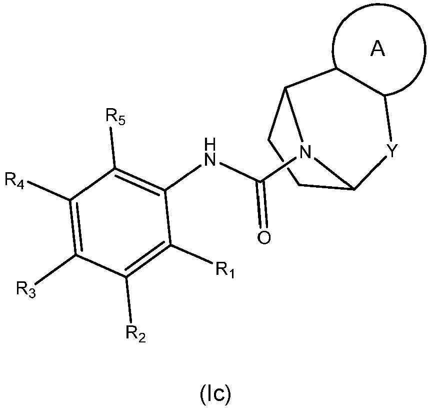 wherein:
ring A is 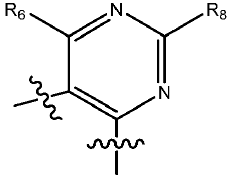 wherein R₆ and R₈ are each independently selected from H, F, Cl, Br, I, CN, alkoxy, NR₁₁R₁₁', OH, alkyl, phenyl, and haloalkyl, with the proviso that at least one of R₆ and R₈ is other than H;
Y is selected from CH₂, C=N-OH, and CR₁₀R₁₀', wherein R₁₀ and R₁₀' are each independently selected from H, F, alkyl, and haloalkyl;
R₁, R₄, and R₅ are each independently selected from H, F, Cl, Br, and I;
R₂ and R₃ are each independently selected from H, F, Cl, Br, I, CN, methoxy, and haloalkyl; and
R₁₁ and R₁₁' are each independently selected from H, alkyl, haloalkyl, COR₁₂, and SO₂R₁₃, wherein R₁₂ and R₁₃ are both alkyl.

Preferred definitions for groups Y and R₁₋₁₃ and preferred configurations of the ring system are as set out above for compounds of formula (Ia), (Ib), (Ie) and (If).

In one preferred embodiment, R₈ is OH, and ring A is in the form of a pyridine tautomer:

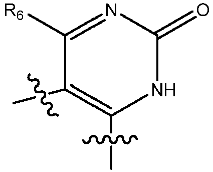

Highly preferred compounds of formula (Ic) include the following compounds as described herein: (24), (25), (26), (27), (28), (29), (31), (32), (45), (46) and (47), and enantiomers thereof, and mixtures of enantiomers thereof, including racemic mixtures, and pharmaceutically acceptable salts and solvates thereof.

### Compounds of formula (Id)

Another aspect of the invention relates to a compound of formula (Id), or a pharmaceutically acceptable salt or solvate thereof, 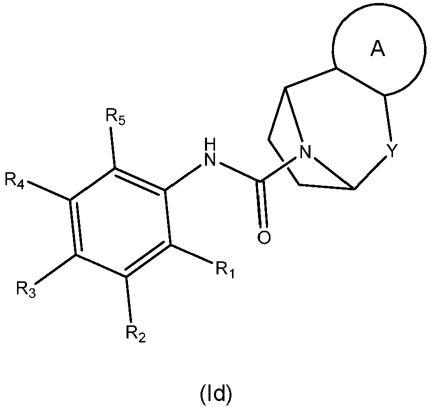 wherein:
ring A is 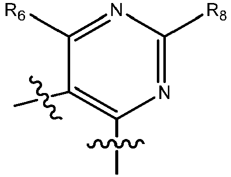 wherein R₆ and R₈ are each independently selected from H, F, Cl, Br, I, CN, alkoxy, NR₁₁R₁₁', OH, alkyl, phenyl, and haloalkyl;
Y is selected from CH₂, C=N-OH, and CR₁₀R₁₀', wherein R₁₀ and R₁₀' are each independently selected from H, F, alkyl, and haloalkyl;
R₁, R₄, and R₅ are each independently selected from H, F, Cl, Br, and I;
R₂ and R₃ are each independently selected from H, F, Cl, Br, I, CN, methoxy, and haloalkyl, with the proviso that at least one of R₂ and R₃ is selected from CN, methoxy, and haloalkyl; and
R₁₁ and R₁₁' are each independently selected from H, alkyl, haloalkyl, COR₁₂, and SO₂R₁₃, wherein R₁₂ and R₁₃ are both alkyl.

Preferred definitions for groups Y and R₁₋₁₃ and preferred configurations of the ring system are as set out above for compounds of formula (Ia).

Highly preferred compounds of formula (Id) include the following compounds as described herein: (1), (2), (3), (5), (8), (11), (13), (15), (18), (27), (28), (29), (32), (45) and (47), and enantiomers thereof, and mixtures of enantiomers thereof, including racemic mixtures, and pharmaceutically acceptable salts and solvates thereof.

### PROCESS

A further aspect of the invention relates to a process for preparing a compound as defined herein, said process comprising reacting a compound of formula II with a compound of formula III , where R¹⁻⁵, Y and A are as defined above, to form a compound of formula (la), (Ib), (Ic), (Id), (le) or (If):

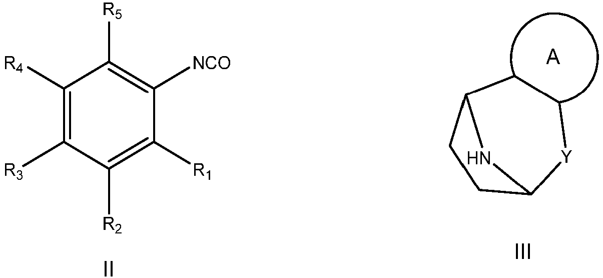

In one preferred embodiment, the reaction takes place in the presence of a base, preferably, *N,N*-diisopropylethylamine (DIPEA) or triethylamine. Preferably, the reaction takes place in an organic solvent. Suitable organic solvents include, but are not limited to, dichloromethane, tetrahydrofuran and dimethylformamide, or mixtures of two or more thereof. The skilled person would understand that other bases and solvents would also be suitable.

### THERAPEUTIC APPLICATIONS

A further aspect of the invention relates to compounds as described herein for use in medicine. The compounds have particular use in the field of oncology, immuno-oncology, and immunology as described in more detail below. In a preferred embodiment, the compound of the invention modulates GPR65, and more preferably inhibits GPR65 signalling.

Yet another aspect of the invention relates to compounds as described herein for use as a medicament, preferably for use in treating or preventing a disorder selected from a proliferative disorder and an immune disorder.

Another aspect of the invention relates to compounds as described herein for use in treating or preventing asthma and/or chronic obstructive pulmonary disease (COPD). GPR65 variant/SNP (rs6574978) has been shown to be associated with asthma/COPD syndrome with almost GWAS significant p value (1.18x10e-7) (Hardin, 2014). Furthermore, GPR65 activation by pH (pH is low/acidic in asthmatic lungs) promotes eosinophil viability in a cAMP-dependent manner, contributing to disease progression/exacerbation. It is further known that GPR65 KO mice have attenuated asthma symptoms (Kottyan, 2009).

Another aspect of the invention relates to compounds as described herein for use in treating or preventing acute respiratory distress syndrome (ARDS). GPR65 has been shown to be protective in a model of LPS-induced acute lung injury model (Tsurumaki, 2015).

One aspect of the invention relates to a compound as described herein for use in treating a proliferative disorder. Preferably, the proliferative disorder is a cancer or leukemia.

In one preferred embodiment, the cancer is a solid tumour and/or metastases thereof.

In another preferred embodiment, the cancer is selected from melanoma, renal cell carcinoma (RCC), gastric cancer, acute myeloid leukaemia (AML), triple negative breast cancer (TNBC), colorectal cancer, head and neck cancer, colorectal adenocarcinoma, pancreatic adenocarcinoma, lung cancer, sarcoma, ovarian cancer, and gliomas, preferably glioblastoma (GBM).

Without wishing to be bound by theory, it is understood that GPR65 modulators are capable of preventing the increase in cytoplasmic cAMP in tumour-associated macrophages (TAMs), natural killer (NK) cells and subsets of T cells that would typically result from their exposure to the acidic tumour microenvironment and concomitant GPR65 activation. This reduction in the level of cytoplasmic cAMP in turn reduces the levels of ICER and pro-inflammatory mediators such as CXCL10 and TNFα, preventing the polarization of TAMs and alteration of other immune cells that are associated with a non-inflammatory and tumour-permissive environment. Therefore, GPR65 modulators are expected to result in an increase in the visibility of the tumour to the immune system leading to increased immune-mediated tumour clearance. This suggests that modulation of GPR65 activity could be an effective treatment for cancer as stand-alone therapy or in combination with cancer immunotherapies (vaccines, agents that promote T cell mediated immune responses) or in patients that do not respond to immunomodulatory approaches such as PD1/PDL-1 blockade.

Another aspect of the invention relates to a compound as described herein for use in treating or preventing an immune disorder, preferably an autoimmune disease.

In one embodiment, the autoimmune disease is selected from psoriasis, psoriatic arthritis, rheumatoid arthritis (RA), multiple sclerosis (MS), systemic lupus erythematosus (SLE), autoimmune thyroiditis (Hashimoto's thyroiditis), Graves' disease, uveitis (including intermediate uveitis), ulcerative colitis, Crohn's disease, autoimmune uveoretinitis, systemic vasculitis, polymyositis-dermatomyositis, systemic sclerosis (scleroderma), Sjogren's Syndrome, ankylosing spondylitis and related spondyloarthropathies, sarcoidosis, autoimmune hemolytic anemia, immunological platelet disorders, autoimmune polyendocrinopathies, autoimmune myocarditis, type I diabetes and atopic dermatitis.

In a particularly preferred embodiment, the autoimmune disease is selected from psoriasis, psoriatic arthritis, ankylosing spondylitis, Crohn's disease, and multiple sclerosis (MS).

Without wishing to be bound by theory, it is understood that GPR65 modulators will prevent the upregulation of ICER in CD4+ T cells. This, in turn, is expected to prevent the ICER-associated suppression of IL-2 that biases CD4+ T cells toward the inflammatory Th17 phenotype associated with increased pathogenicity in the context of autoimmune disease. This is supported by the fact that mutations in the GPR65 locus are associated with several autoimmune diseases, such as multiple sclerosis, ankylosing spondylitis, inflammatory bowel disease, and Crohn's disease (Gaublomme, 2015). This suggests that modulation of GPR65 activity could be an effective treatment for autoimmune diseases.

Another aspect relates to a compound as described herein for use in treating or preventing a disorder caused by, associated with or accompanied by abnormal activity against GPR65.

Another aspect relates to a compound as described herein for use in treating or preventing a GPR65-associated disease or disorder.

Another aspect of the invention relates to a method of treating a disorder as described above comprising administering a compound as described herein to a subject.

Another aspect of the invention relates to a method of treating a GPR65-associated disease or disorder in a subject. The method according to this aspect of the present invention is effected by administering to a subject in need thereof a therapeutically effective amount of a compound of the present invention, as described hereinabove, either *per se*, or, more preferably, as a part of a pharmaceutical composition, mixed with, for example, a pharmaceutically acceptable carrier, as is detailed hereinafter.

Yet another aspect of the invention relates to a method of treating a subject having a disease state alleviated by modulation of GPR65 wherein the method comprises administering to the subject a therapeutically effective amount of a compound according to the invention.

Another aspect relates to a method of treating a disease state alleviated by modulation of GPR65, wherein the method comprises administering to a subject a therapeutically effective amount of a compound according to the invention.

Preferably, the subject is a mammal, more preferably a human.

The term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

Herein, the term "treating" includes abrogating, substantially inhibiting, slowing or reversing the progression of a disease or disorder, substantially ameliorating clinical symptoms of a disease or disorder or substantially preventing the appearance of clinical symptoms of a disease or disorder.

Herein, the term "preventing" refers to a method for barring an organism from acquiring a disorder or disease in the first place.

The term "therapeutically effective amount" refers to that amount of the compound being administered which will relieve to some extent one or more of the symptoms of the disease or disorder being treated.

For any compound used in this invention, a therapeutically effective amount, also referred to herein as a therapeutically effective dose, can be estimated initially from cell culture assays. For example, a dose can be formulated in animal models to achieve a circulating concentration range that includes the IC₅₀ or the IC₁₀₀ as determined in cell culture. Such information can be used to more accurately to determine useful doses in humans. Initial dosages can also be estimated from *in vivo* data. Using these initial guidelines one of ordinary skill in the art could determine an effective dosage in humans.

Moreover, toxicity and therapeutic efficacy of the compounds described herein can be determined by standard pharmaceutical procedures in cell cultures or experimental animals, e.g., by determining the LD₅₀ and the ED₅₀. The dose ratio between toxic and therapeutic effect is the therapeutic index and can be expressed as the ratio between LD₅₀ and ED₅₀. Compounds which exhibit high therapeutic indices are preferred. The data obtained from these cell cultures assays and animal studies can be used in formulating a dosage range that is not toxic for use in human. The dosage of such compounds lies preferably within a range of circulating concentrations that include the ED₅₀ with little or no toxicity. The dosage may vary within this range depending upon the dosage form employed and the route of administration utilized. The exact formulation, route of administration and dosage can be chosen by the individual physician in view of the patient's condition (see, e.g., Fingl *et al*, 1975, The Pharmacological Basis of Therapeutics, chapter 1, page 1).

Dosage amount and interval may be adjusted individually to provide plasma levels of the active compound which are sufficient to maintain therapeutic effect. Usual patient dosages for oral administration range from about 50-2000 mg/day, commonly from about 100-1000 mg/day, preferably from about 150-700 mg/day and most preferably from about 250-500 mg/day or from 50-100 mg/day. Preferably, therapeutically effective serum levels will be achieved by administering multiple doses each day. In cases of local administration or selective uptake, the effective local concentration of the drug may not be related to plasma concentration. One skilled in the art will be able to optimize therapeutically effective local dosages without undue experimentation.

As used herein, "GPR65-related disease or disorder" refers to a disease or disorder characterized by inappropriate GPR65 activity. Inappropriate GPR65 activity refers to either an increase or decrease in GPR65 activity as measured by enzyme or cellular assays, for example, compared to the activity in a healthy subject. Inappropriate activity could also be due to overexpression of GPR65 in diseased tissue compared with healthy adjacent tissue.

Preferred diseases or disorders that the compounds described herein may be useful in preventing include proliferative disorders and immune disorders as described hereinbefore, as well as asthma and chronic obstructive pulmonary disease.

Thus, the present invention further provides use of compounds as defined herein in the preparation of a medicament for the treatment of a disease where it is desirable to modulate GPR65. Such diseases include proliferative disorders and immune disorders as described hereinbefore, as well as asthma and chronic obstructive pulmonary disease.

As used herein the phrase "preparation of a medicament" includes the use of the components of the invention directly as the medicament in addition to their use in any stage of the preparation of such a medicament.

In one preferred embodiment, the compound prevents the increase in cytoplasmic cAMP levels expected following GPR65 activation at acidic pH. This prevention of cAMP accumulation in turn prevents downstream signalling through ICER, as described above. The "Human GPR65 cyclic adenosine monophosphate (cAMP) Homogeneous Time Resolved Fluorescence (HTRF) antagonist assay", or simply "cAMP assay", as described in the accompanying examples, can be used to measure the potency of GPR65 modulators, which is expressed as the concentration of compound required to reduce the increase in cAMP concentration upon GPR65 activation by 50% (i.e. an IC₅₀).

In one preferred embodiment, the compound exhibits an IC₅₀ value in the cAMP assay of less than about 25 µM. More preferably, the compound exhibits an IC₅₀ value in the cAMP assay of less than about 10 µM, more preferably, less than about 5 µM, even more preferably, less than about 1 µM, even more preferably, less than about 0.1 µM.

In another preferred embodiment, the compound exhibits an hGPR65 IC50 value of less than < 5 µM, more preferably less than < 500 nM in the aforementioned assay.

### Therapeutic use of compounds of Formula I

A further aspect of the invention relates to a compound of formula (I), or a pharmaceutically acceptable salt or solvate thereof, 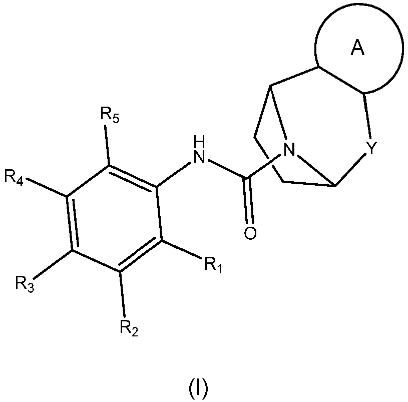 wherein:
ring A is a 5 or 6 membered aromatic or heteroaromatic ring, wherein said aromatic or heteroaromatic ring is optionally substituted with one or more substituents selected from F, Cl, Br, I, CN, alkoxy, NR₁₁R₁₁', OH, alkyl, haloalkyl, aralkyl, aryl, and heteroaryl, and wherein said aryl and heteroaryl substituents are in turn optionally substituted with one or more substituents each independently selected from F, Cl, Br, I, CN, alkoxy, NR₁₁R₁₁', OH, alkyl, haloalkyl, and aralkyl;
Y is selected from CH₂, C=N-OH, and CR₁₀R₁₀', wherein R₁₀ and R₁₀' are each independently selected from H, F, alkyl, and haloalkyl;
R₁, R₄, and R₅ are each independently selected from H, F, Cl, Br, and I;
R₂ and R₃ are each independently selected from H, F, Cl, Br, I, CN, methoxy, and haloalkyl; and
R₁₁ and R₁₁' are each independently selected from H, alkyl, haloalkyl, COR₁₂, and SO₂R₁₃, wherein R₁₂ and R₁₃ are both alkyl;
for use as a medicament.

Preferred definitions for ring A and groups Y and R₁₋₁₃ and preferred configurations of the ring system are as set out above for compounds of formula (la), (Ib), (Ic), (Id), (Ie) and (If).

Preferably, the compounds of formula (I) are for use in treating proliferative disorders and autoimmune disorders, as well as asthma and chronic obstructive pulmonary disease. Details of suitable proliferative disorders and autoimmune disorders are the same as those set forth above under the heading "Therapeutic Applications".

In one preferred embodiment, the compound of formula (I) for use as described above is selected from the following:
(1)-(8), (11)-(13), (15)-(18), (24)-(29), (31), (32), (34)-(37) and (45)-(53), and enantiomers thereof, and mixtures of enantiomers thereof, including racemic mixtures, and pharmaceutically acceptable salts and solvates thereof.

In one preferred embodiment, the compound of formula (I) for use as described above is selected from the following:

| | | | |
|---|---|---|---|
| 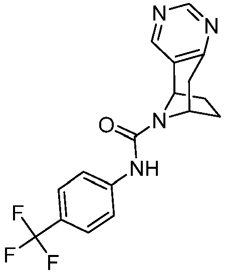 | (1) | 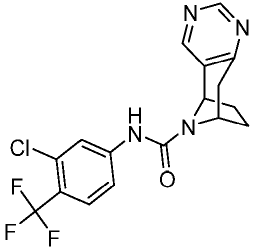 | (2) |
| 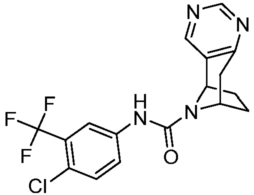 | (3) | 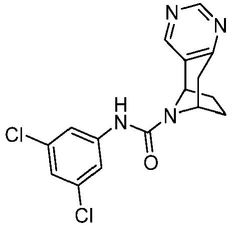 | (4) |
| 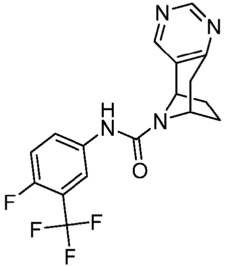 | (5) | 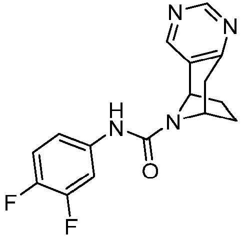 | (6) |
| 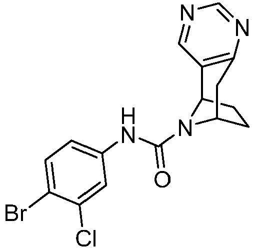 | (7) | 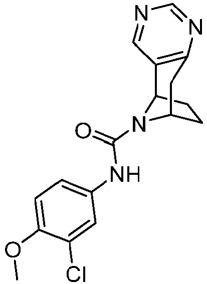 | (8) |
| 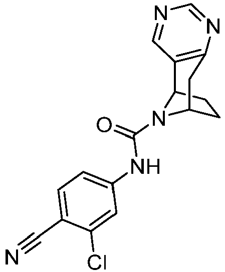 | (11) | 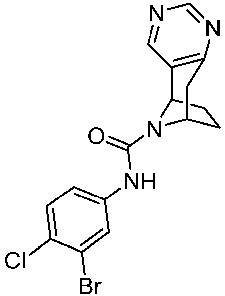 | (12) |
| 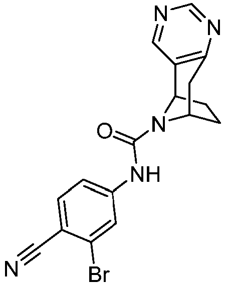 | (13) | 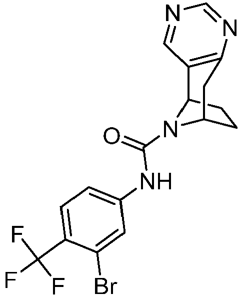 | (15) |
| 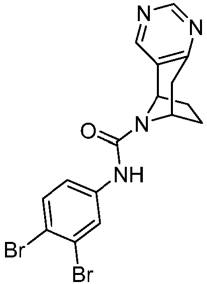 | (16) | 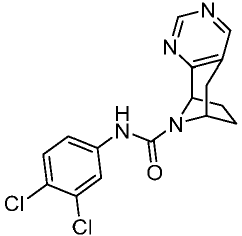 | (17) |
| 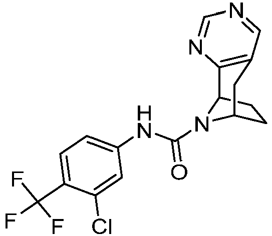 | (18) | 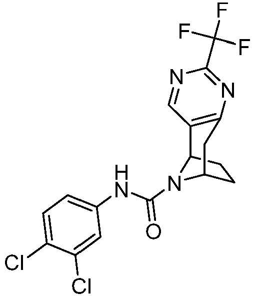 | (24) |
| 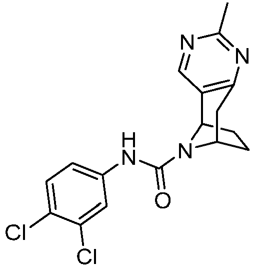 | (25) | 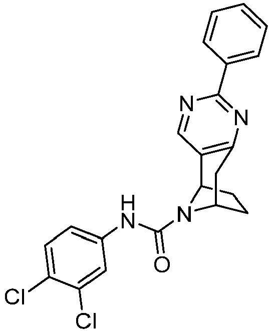 | (26) |
| 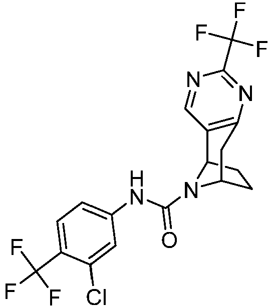 | (27) | 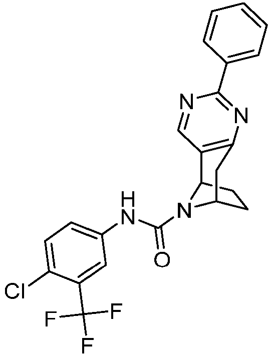 | (28) |
| 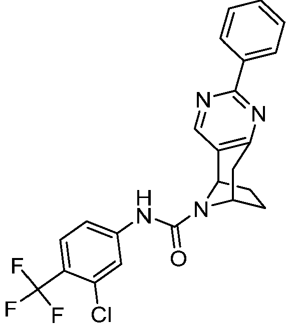 | (29) | 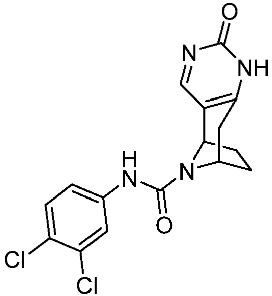 | (31) |
| 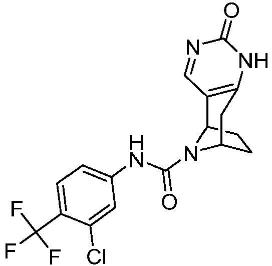 | (32) | 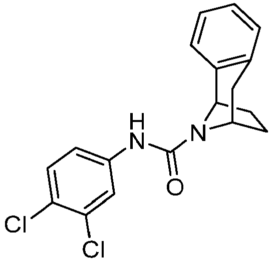 | (34) |
| 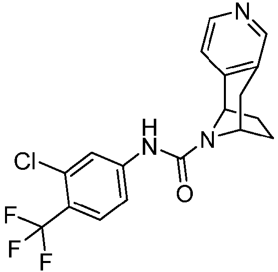 | (35) | 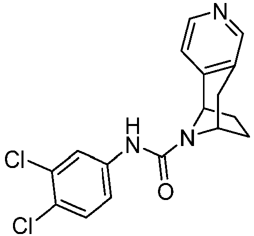 | (36) |
| 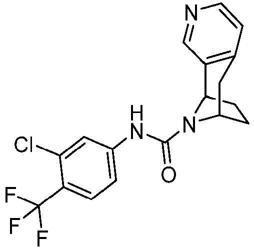 | (37) | 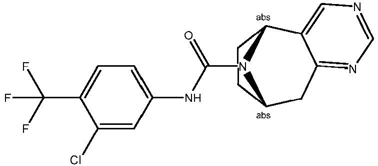 Enantiomer of (2) | (38) |
| 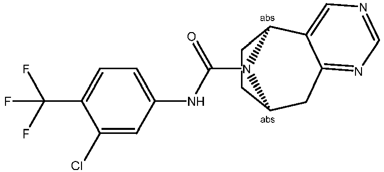 Enantiomer of (2) | (39) | 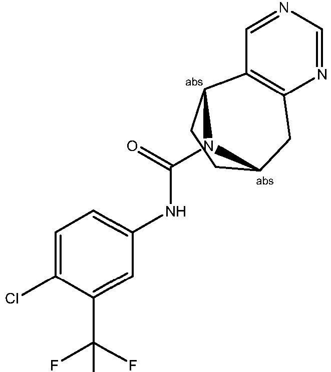 Enantiomer of (3) | (42) |
| 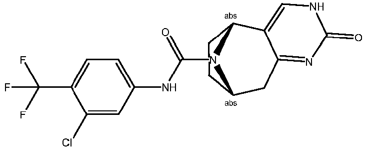 Enantiomer of (32) | (43) | 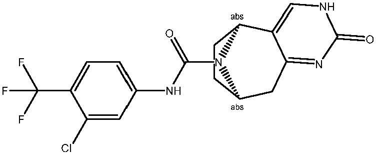 Enantiomer of (32) | (44) |
| 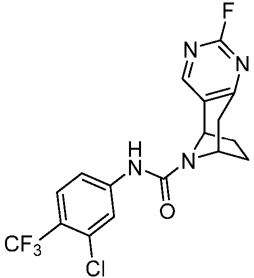 | (45) | 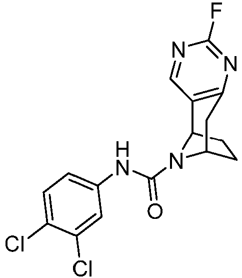 | (46) |
| 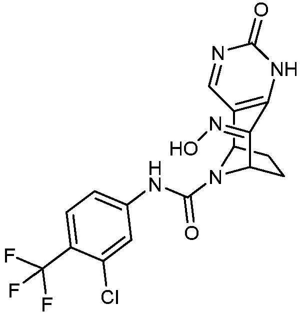 | (47) | 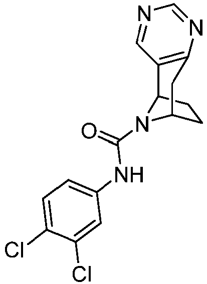 | (48) |
| 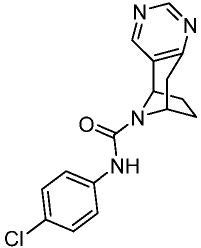 | (49) | 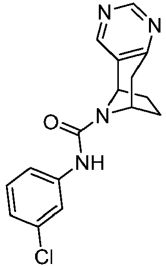 | (50) |
| 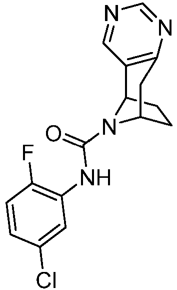 | (51) | 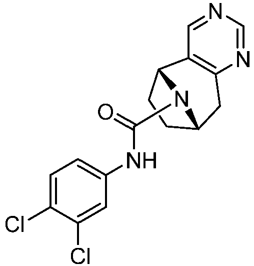 | (52) |
| 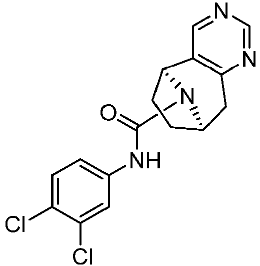 | (53) | 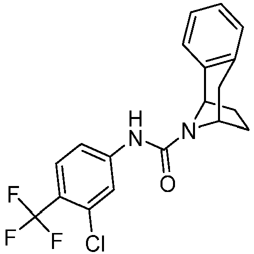 | (54) |
| 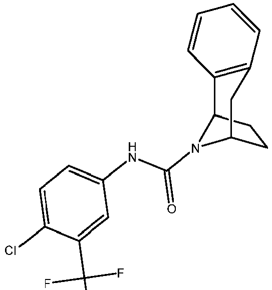 | (55) | 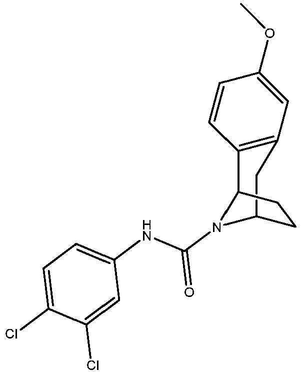 | (56) |
| 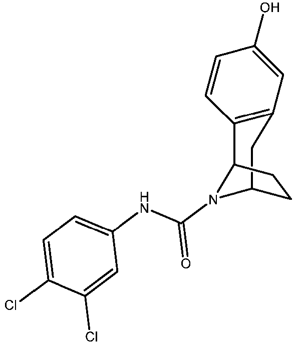 | (57) | 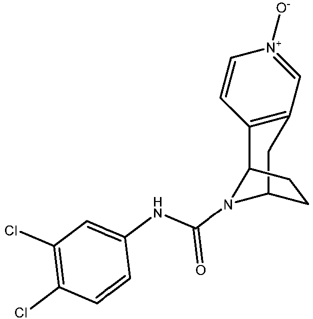 | (58) |
| 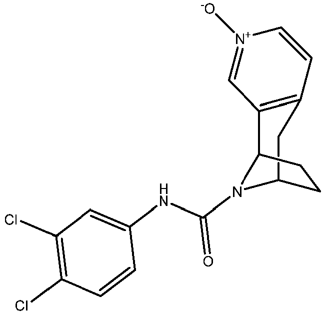 | (59) | 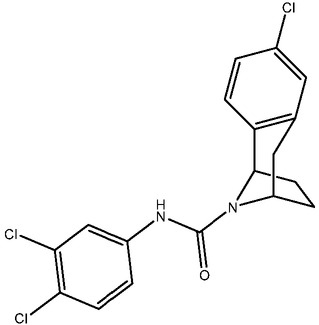 | (60) |
| 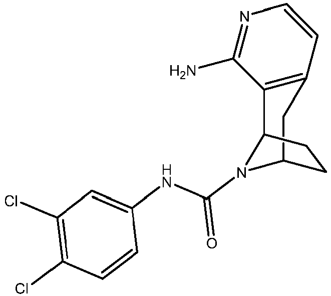 | (61) | 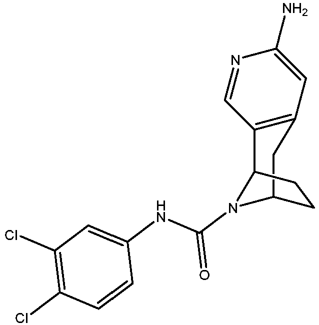 | (62) |
| 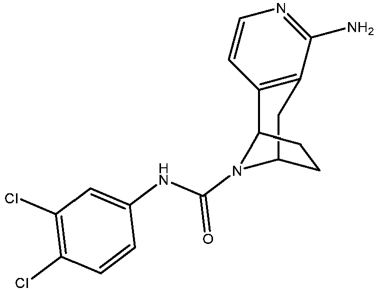 | (63) | 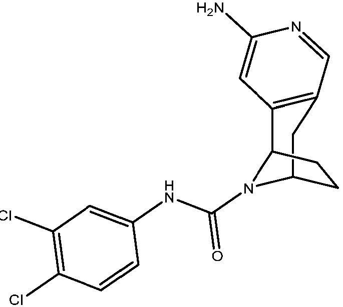 | (64) |
| 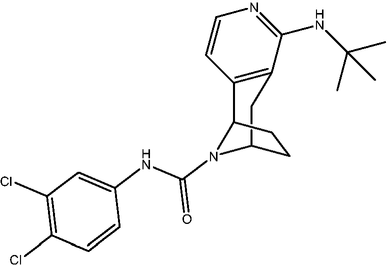 | (65) | 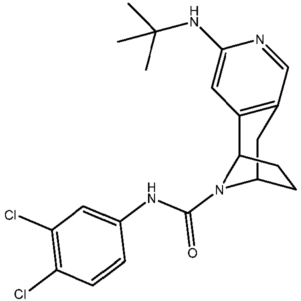 | (66) |
| 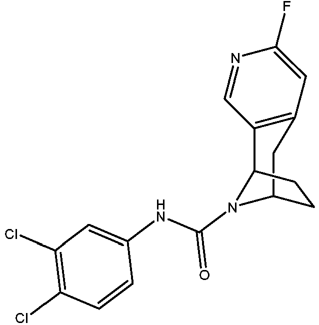 | (67) | 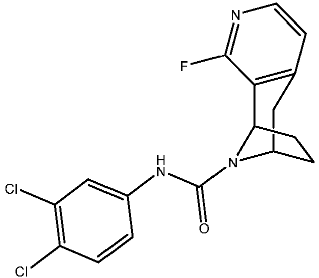 | (68) |
| 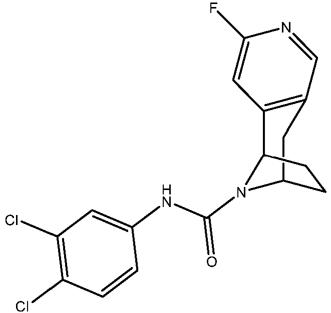 | (69) | 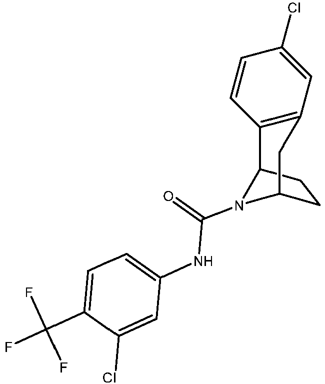 | (71) |
| 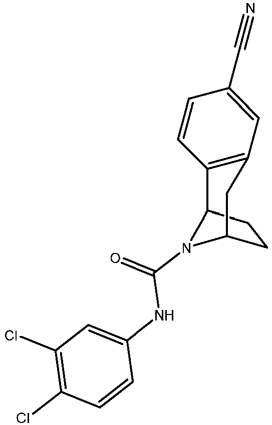 | (72) | 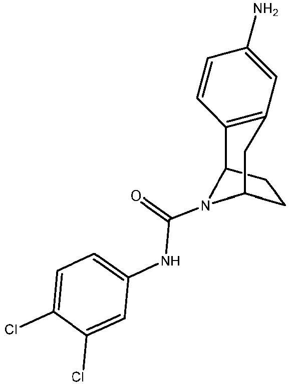 | (73) |
| 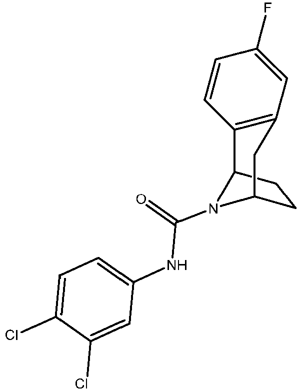 | (74) | 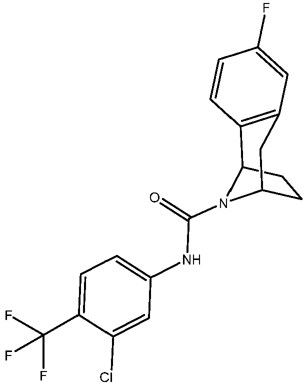 | (75) |
| 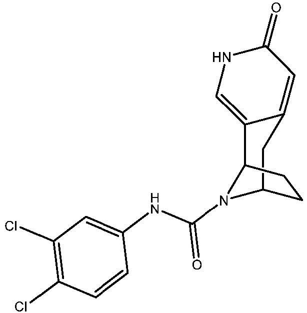 | (76) | 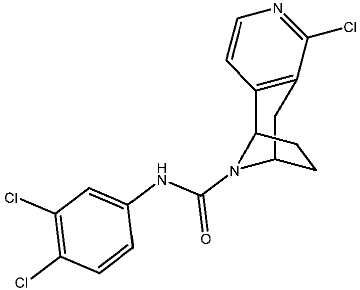 | (77) |
| 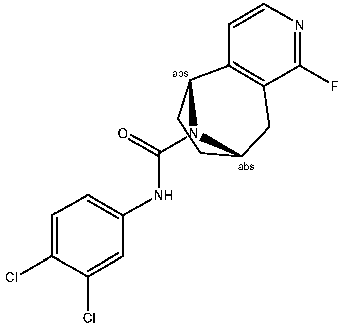 | (78) | 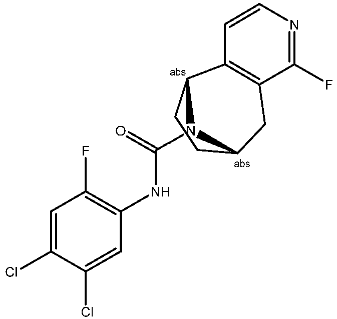 | (79) |
| 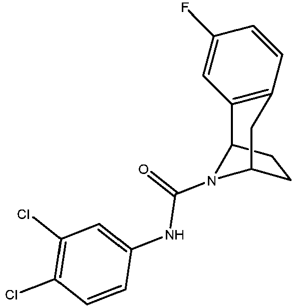 | (80) | 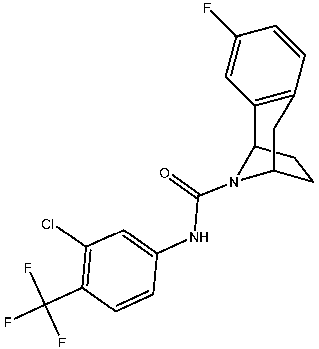 | (81) |
| 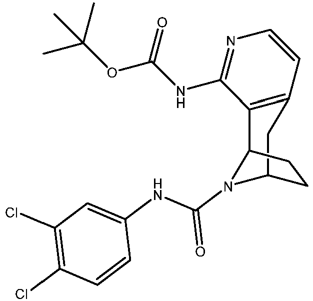 | (82) | 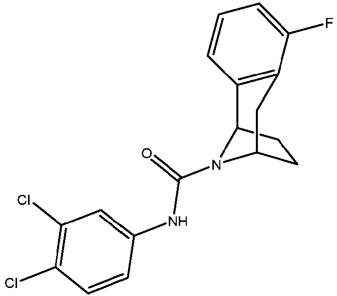 | (83) |
| 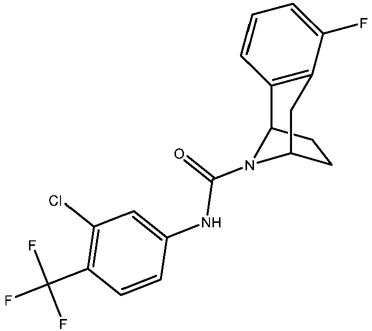 | (84) | 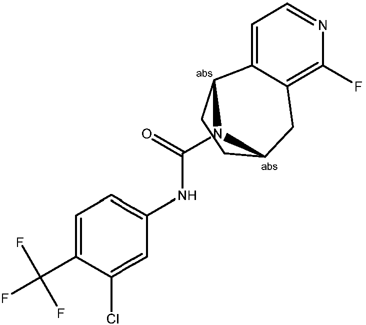 | (85) |
| 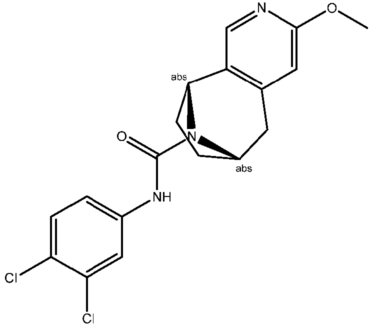 | (86) | 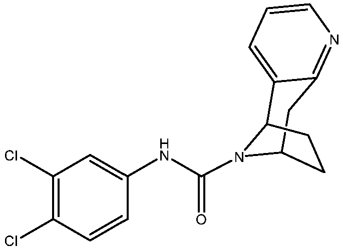 | (87) |
| 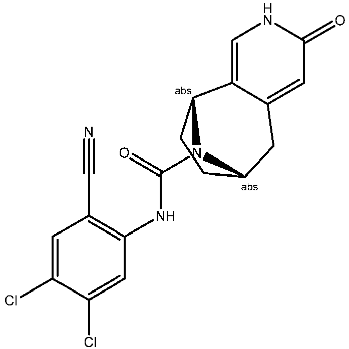 | (88) | 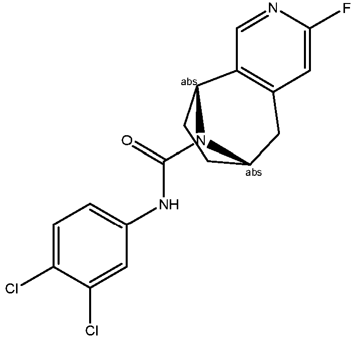 | (91) |
| 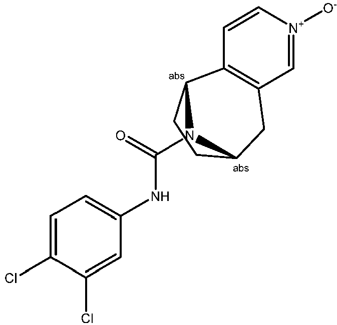 | (92) | 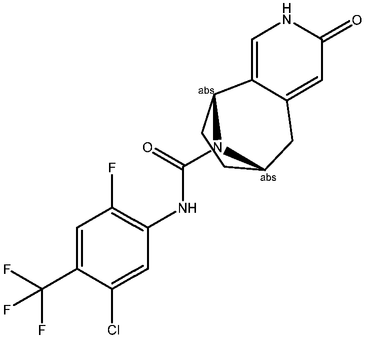 | (93) |
| 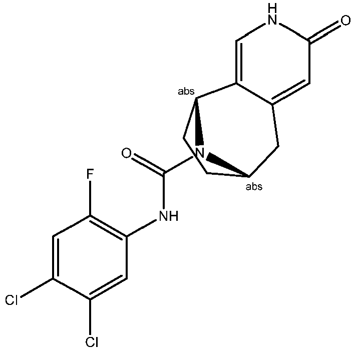 | (94) | 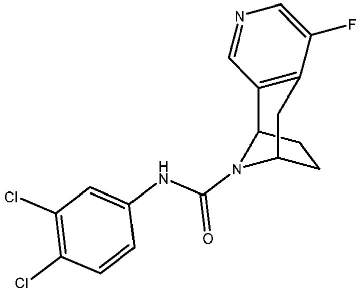 | (95) |
| 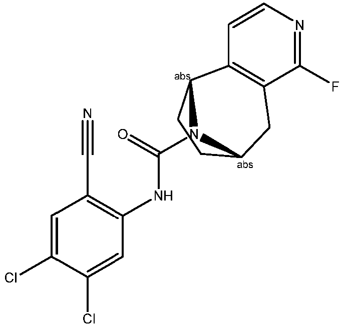 | (98) | 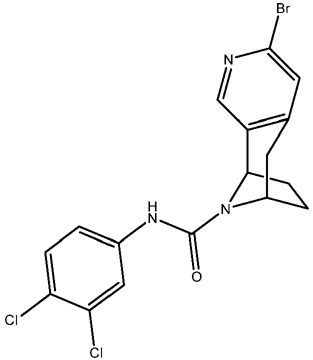 | (99) |
| 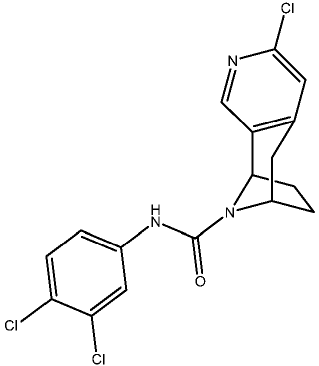 | (100) | 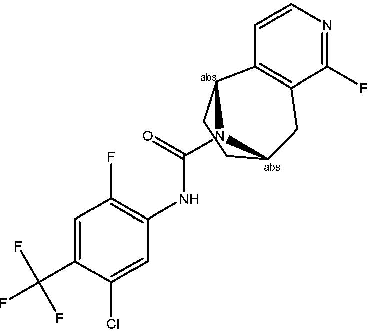 | (101) |
| 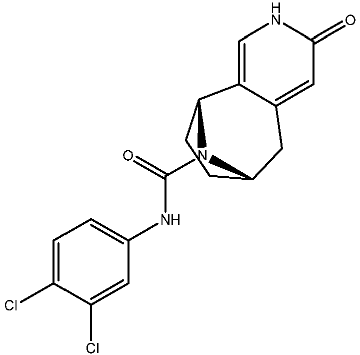 | (103) | 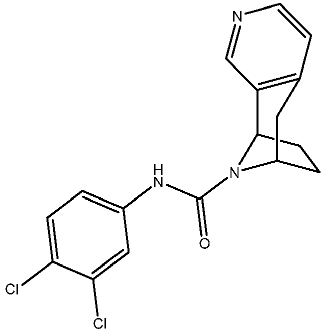 | (104) |
| 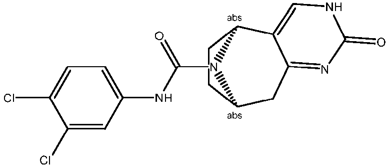 Enantiomer of (31) | (105) | 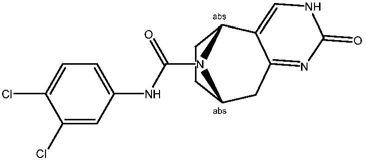 Enantiomer of (31) | (106) |
| 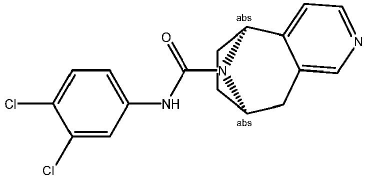 | (107) | 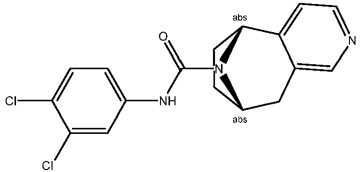 | (108) |
| 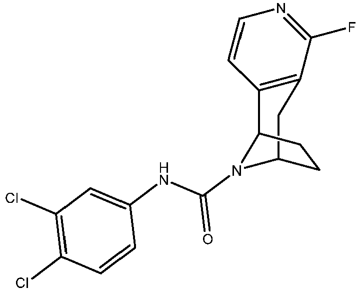 | (109) | 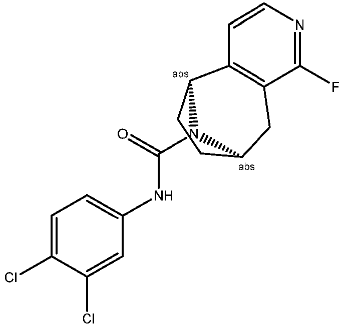 | (110) |
| 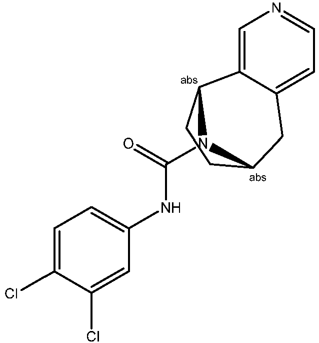 | (111) | 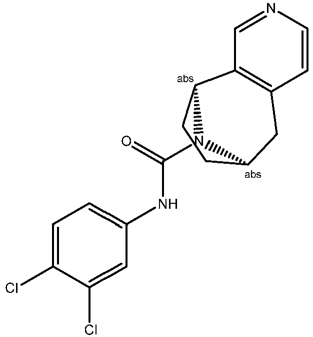 | (112) |
| 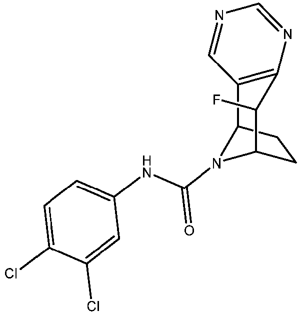 | (113) | 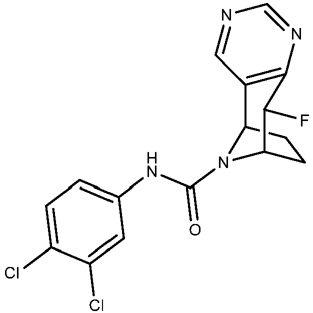 | (114) |
| 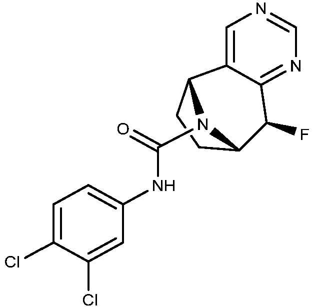 | (115) | | |
| 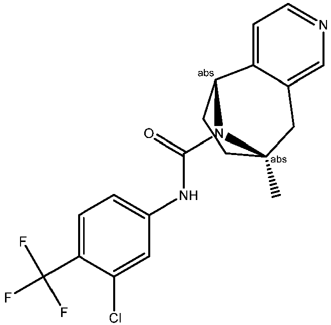 | (116) | 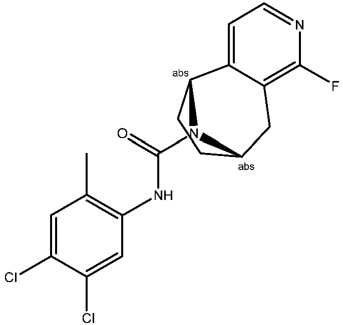 | (117) |
| 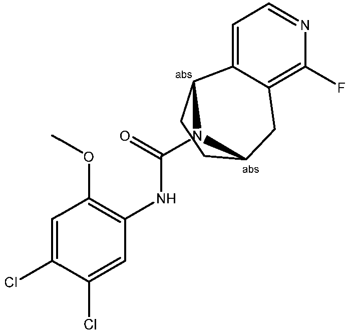 | (118) | 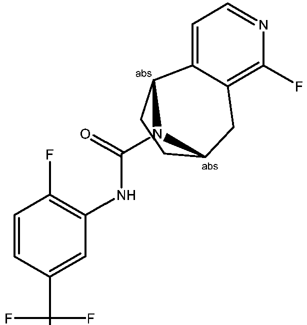 | (119) |
| 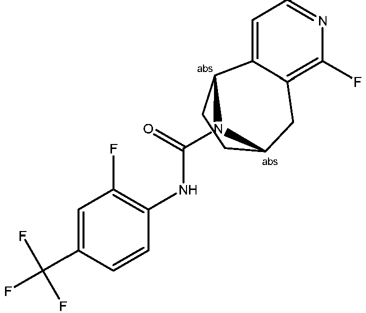 | (120) | 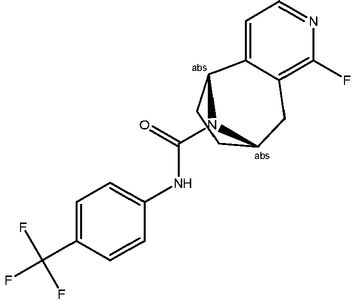 | (121) |
| 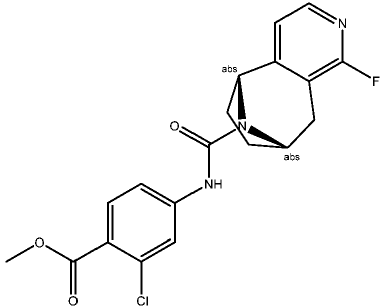 | (122) | 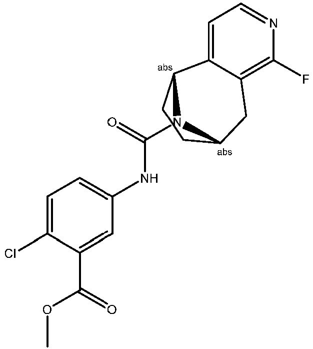 | (123) |
| 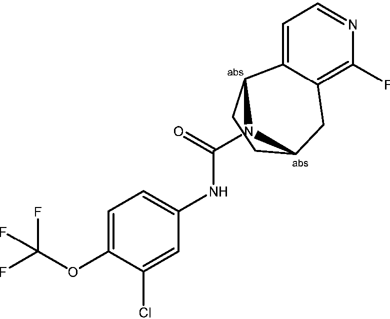 | (124) | 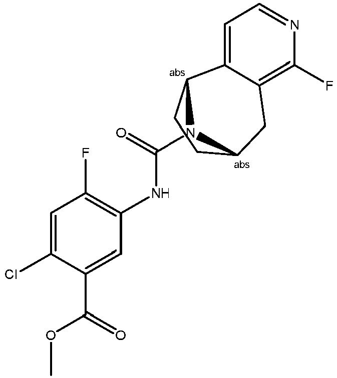 | (125) |
| 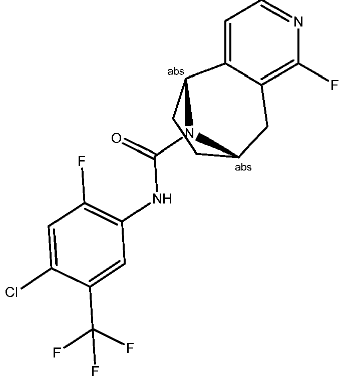 | (126) | 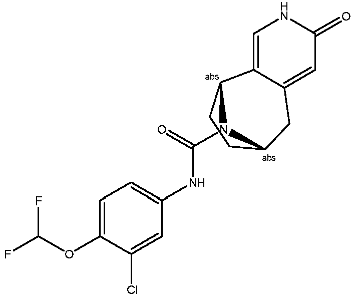 | (127) |
| 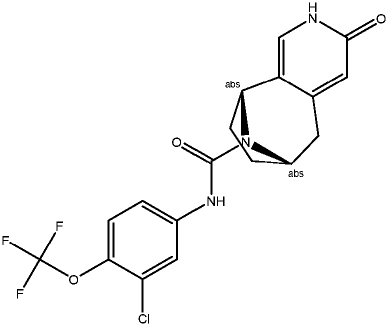 | (128) | 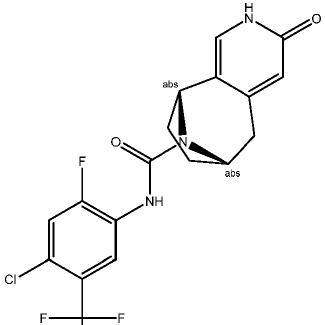 | (129) |
| 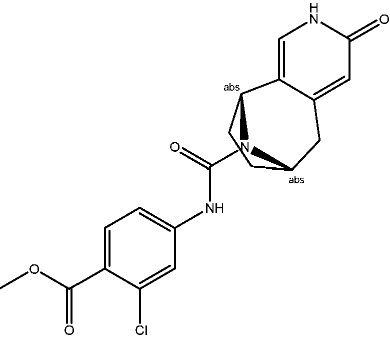 | (130) | 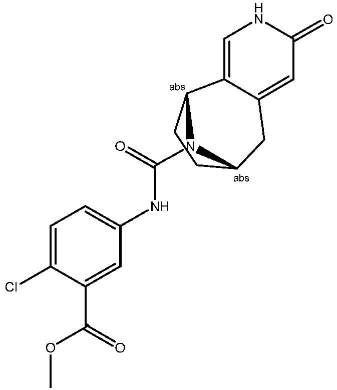 | (131) |
| 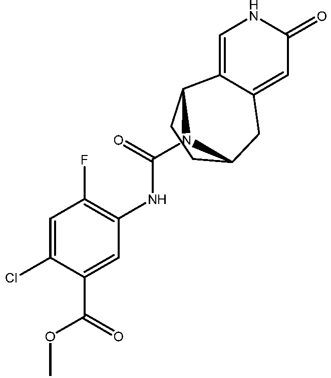 | (132) | 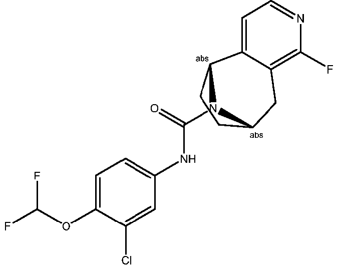 | (133) |
| 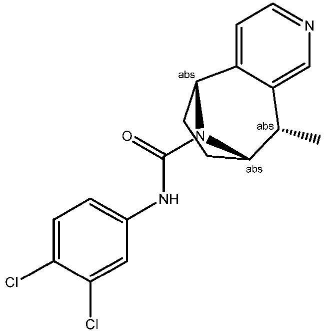 | (134) | 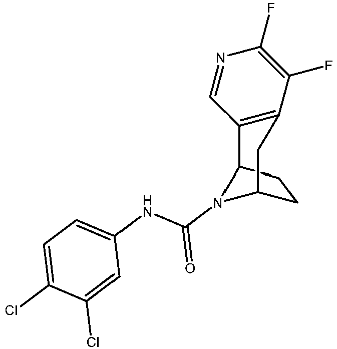 | (135) |
| 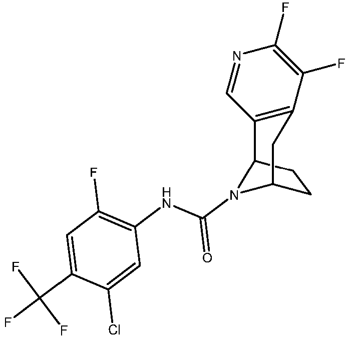 | (136) | 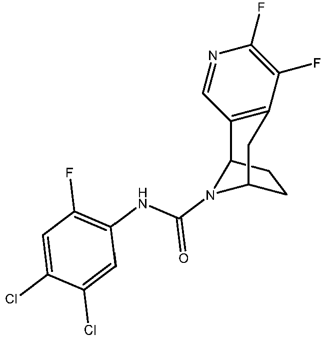 | (137) |
| 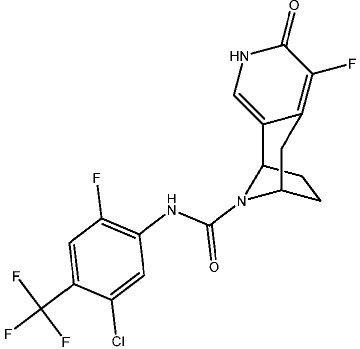 | (138) | 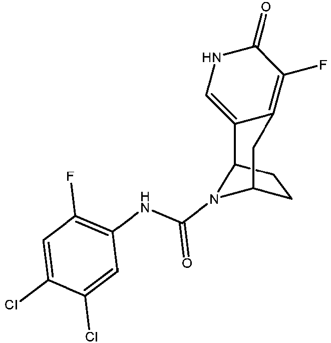 | (139) |
| 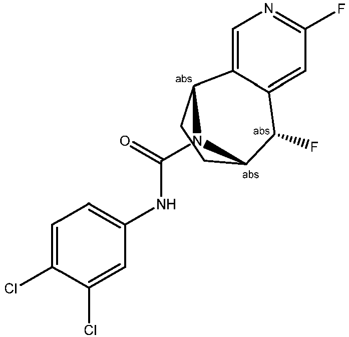 | (140) | 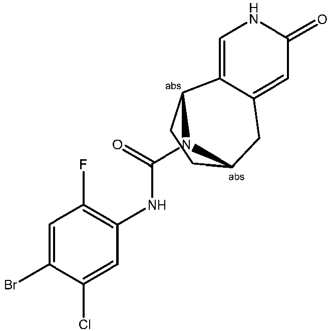 | (141) |
| 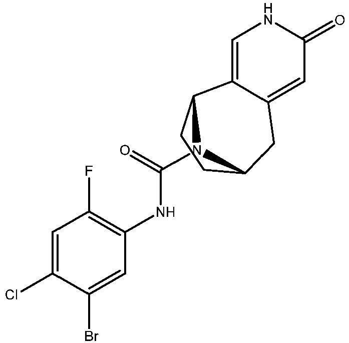 | (142) | 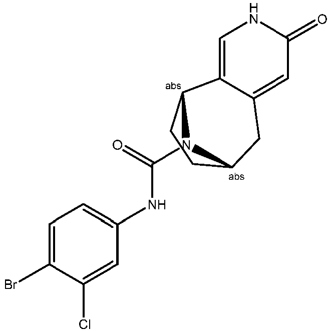 | (143) |
| 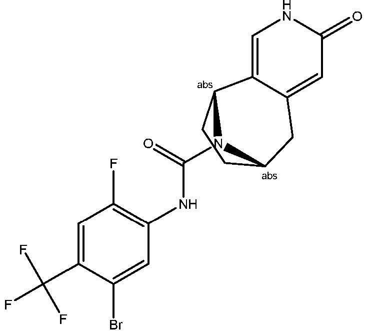 | (144) | 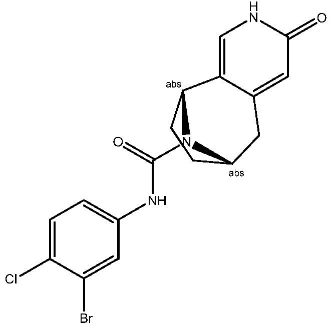 | (145) |
| 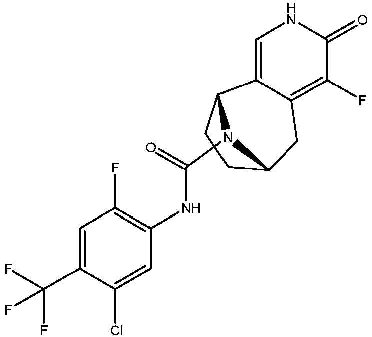 | (146) | 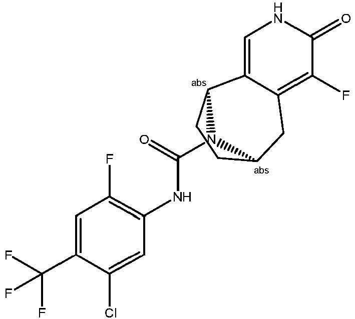 | (147) |
| 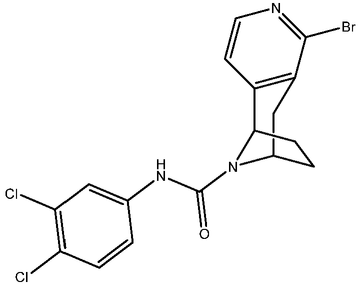 | (148) | 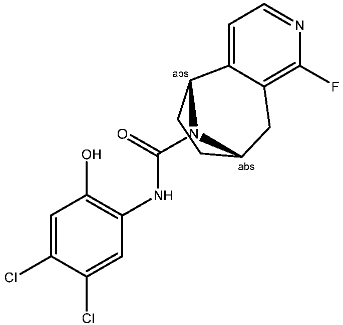 | (149) |
| 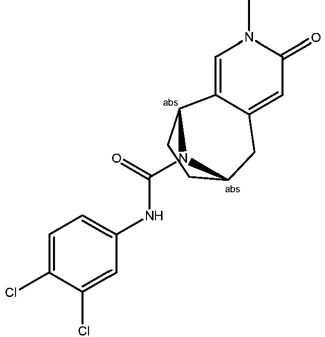 | (150) | | |
| 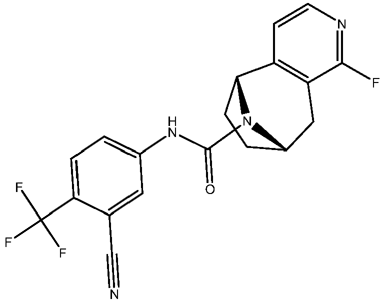 | (151) | 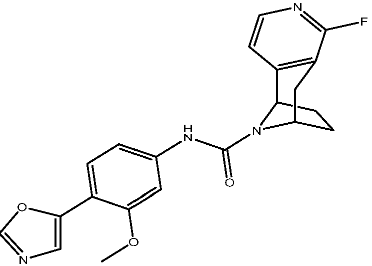 | (152) |
| 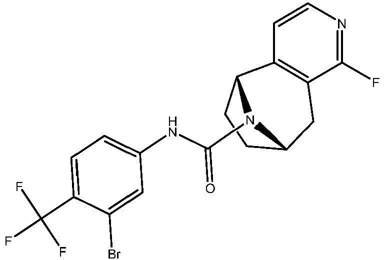 | (153) | 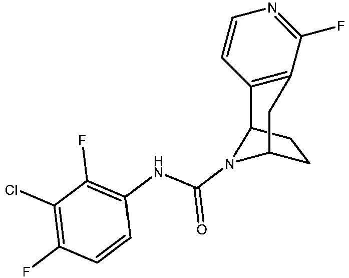 | (154) |
| 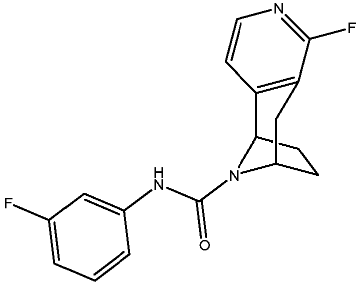 | (155) | 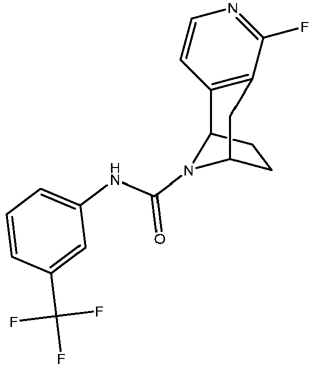 | (156) |
| 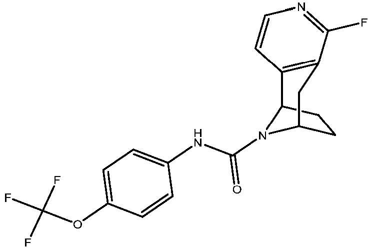 | (157) | 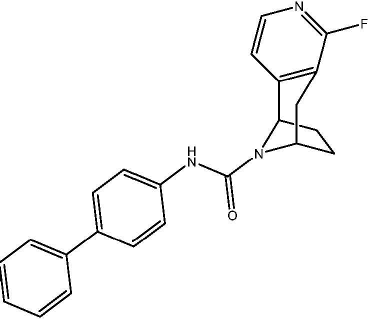 | (158) |
| 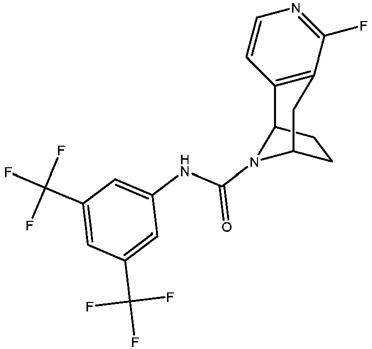 | (159) | 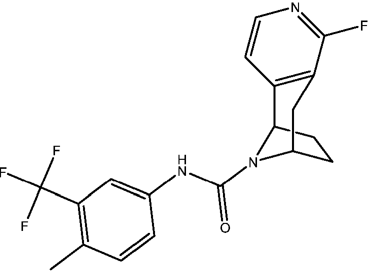 | (160) |
| 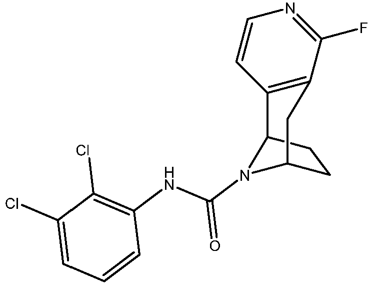 | (161) | 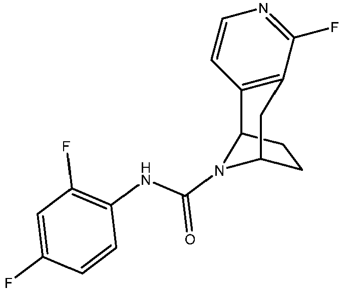 | (162) |
| 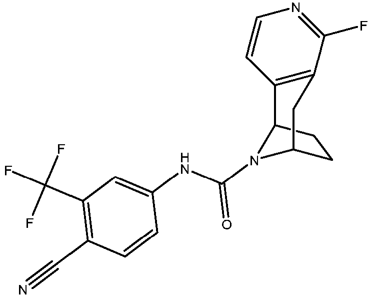 | (163) | 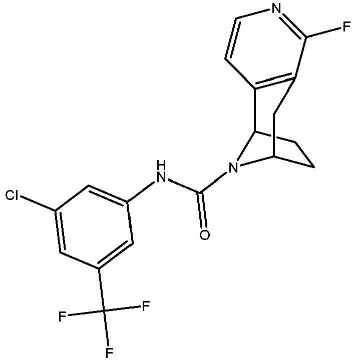 | (164) |
| 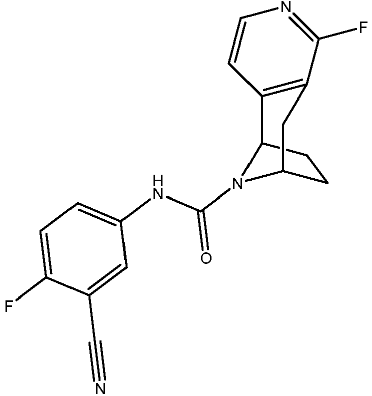 | (165) | 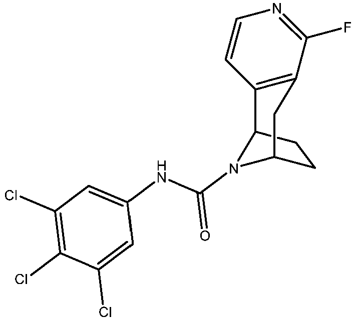 | (166) |
| 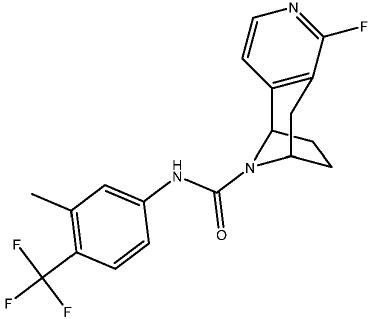 | (167) | 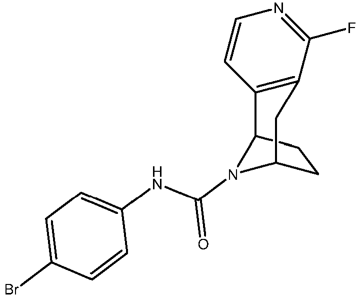 | (168) |
| 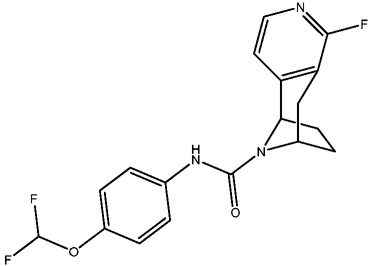 | (169) | 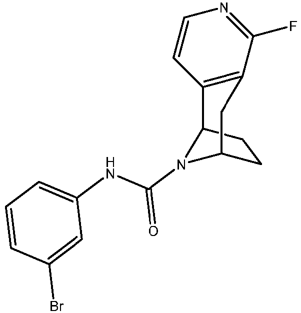 | (170) |
| 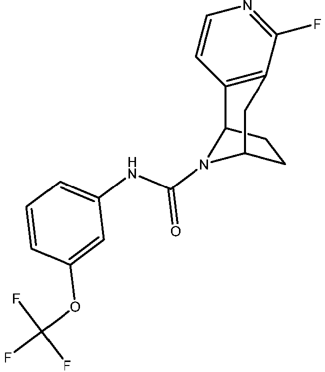 | (171) | 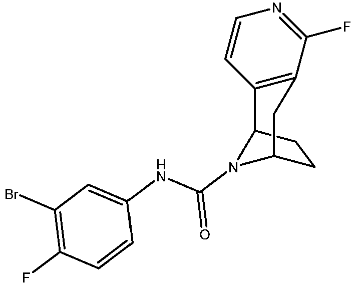 | (172) |
| 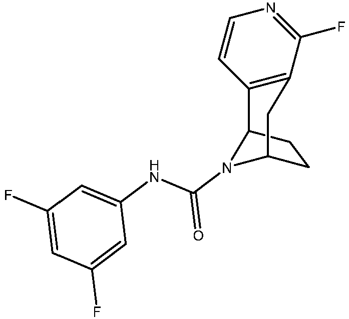 | (173) | 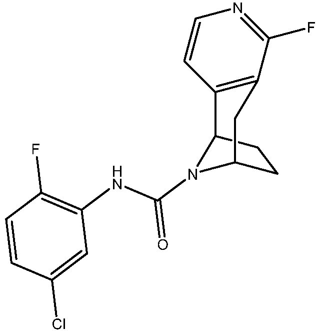 | (174) |
| 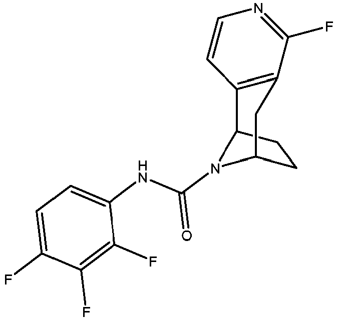 | (175) | 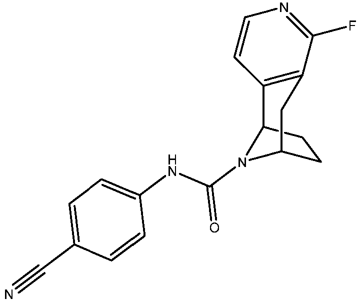 | (176) |
| 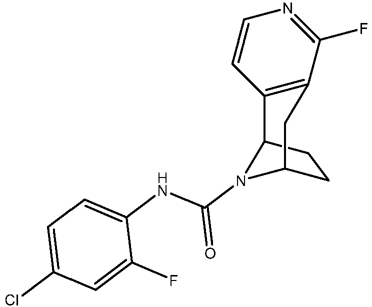 | (177) | 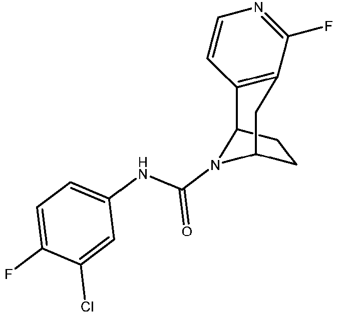 | (178) |
| 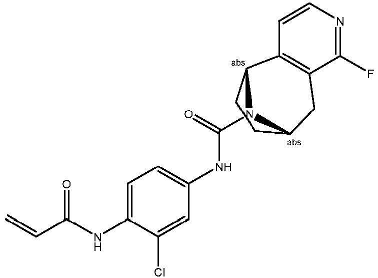 | (179) | 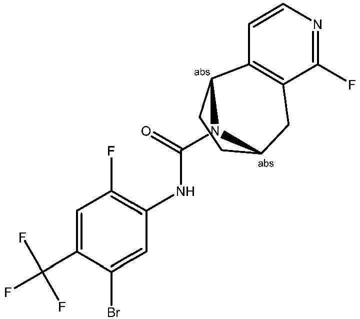 | (180) |
| 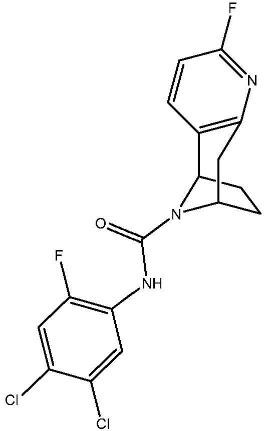 | (181) | 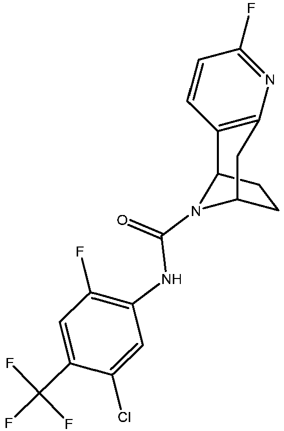 | (182) |
| 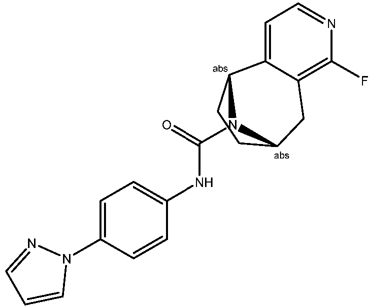 | (183) | 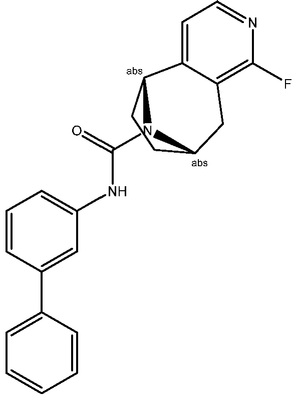 | (184) |
| 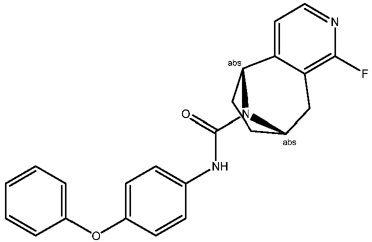 | (185) | 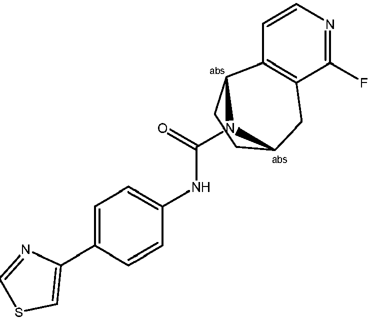 | (186) |
| 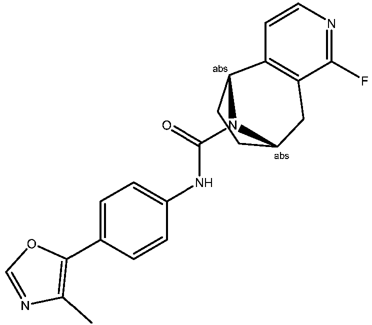 | (187) | 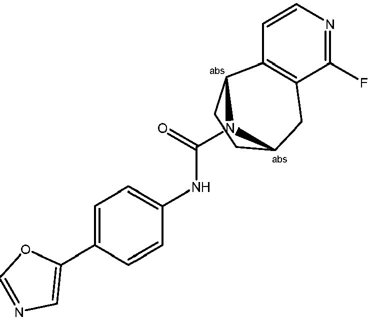 | (188) |
| 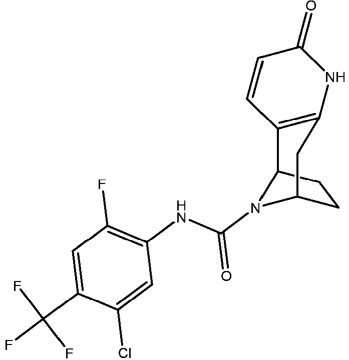 | (189) | 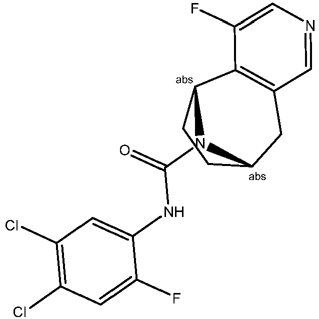 | (190) |
| 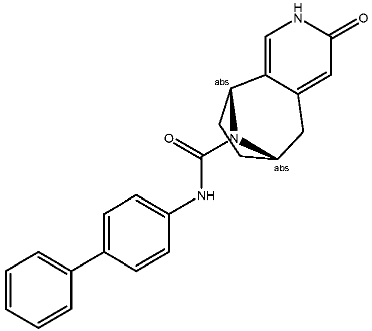 | (191) | 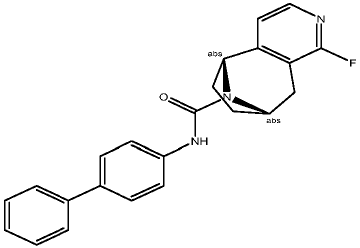 | (192) |
| 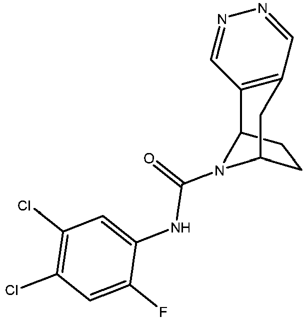 | (193) | 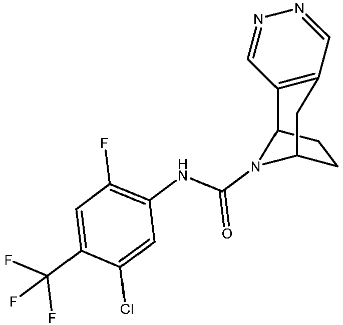 | (194) |
| 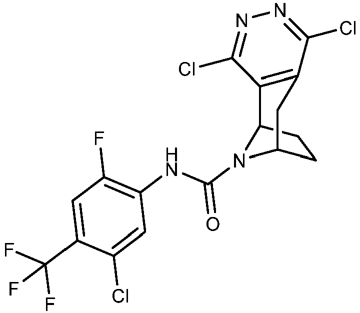 | (195) | 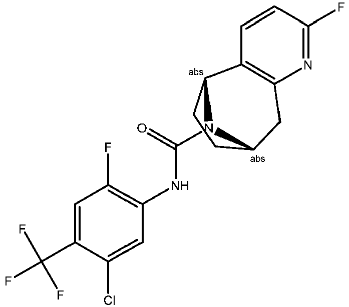 | (196) |
| 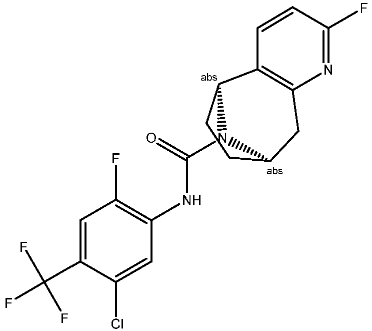 | (197) | 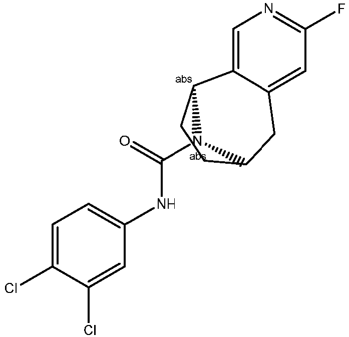 | (198) |
| 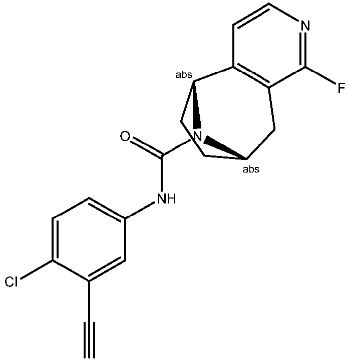 | (199) | 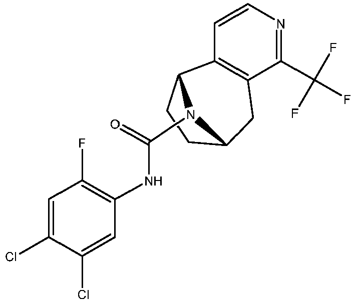 | 200 |

and enantiomers thereof, and mixtures of enantiomers thereof, including racemic mixtures, and pharmaceutically acceptable salts and solvates thereof.

Another aspect of the invention relates to the compounds as described above, and enantiomers thereof, and mixtures of enantiomers thereof, including racemic mixtures, and pharmaceutically acceptable salts and solvates thereof, for use as a medicament.

In another preferred embodiment, the compound according to the invention, or for use as described above, is selected from the following:
*N*-(4-(trifluoromethyl)phenyl)-6,7,8,9-tetrahydro-5H-5,8-epiminocyclohepta[d]pyrimidine-10-carboxamide
*N*-(3-chloro-4-(trifluoromethyl)phenyl)-6,7,8,9-tetrahydro-5H-5,8-epiminocyclohepta[d]-pyrimidine-10-carboxamide
*N*-(4-chloro-3-(trifluoromethyl)phenyl)-6,7,8,9-tetrahydro-5H-5,8-epiminocyclohepta[d]-pyrimidine-10-carboxamide
*N*-(3,5-dichlorophenyl)-6,7,8,9-tetrahydro-5H-5,8-epiminocyclohepta[d]pyrimidine-10-carboxamide
*N*-(4-fluoro-3-(trifluoromethyl)phenyl)-6,7,8,9-tetrahydro-5H-5,8-epiminocyclohepta[d]-pyrimidine-10-carboxamide
*N*-(3,4-difluorophenyl)-6,7,8,9-tetrahydro-5H-5,8-epiminocyclohepta[d]pyrimidine-10-carboxamide
*N*-(4-bromo-3-chlorophenyl)-6,7,8,9-tetrahydro-5H-5,8-epiminocyclohepta[d]pyrimidine-10-carboxamide
*N*-(3-chloro-4-methoxyphenyl)-6,7,8,9-tetrahydro-5H-5,8-epiminocyclohepta[d]pyrimidine-10-carboxamide
*N*-(3-chloro-4-cyanophenyl)-6,7,8,9-tetrahydro-5H-5,8-epiminocyclohepta[d]pyrimidine-10-carboxamide
*N*-(3-bromo-4-chlorophenyl)-6,7,8,9-tetrahydro-5H-5,8-epiminocyclohepta[d]pyrimidine-10-carboxamide
*N*-(3-bromo-4-cyanophenyl)-6,7,8,9-tetrahydro-5H-5,8-epiminocyclohepta[d]pyrimidine-10-carboxamide
*N*-(3-bromo-4-(trifluoromethyl)phenyl)-6,7,8,9-tetrahydro-5H-5,8-epiminocyclohepta[d]-pyrimidine-10-carboxamide
*N*-(3,4-dibromophenyl)-6,7,8,9-tetrahydro-5H-5,8-epiminocyclohepta[d]pyrimidine-10-carboxamide
*N*-(3,4-dichlorophenyl)-6,7,8,9-tetrahydro-5H-6,9-epiminocyclohepta[d]pyrimidine-10-carboxamide
*N*-(3-chloro-4-(trifluoromethyl)phenyl)-6,7,8,9-tetrahydro-5H-6,9-epiminocyclohepta[d]-pyrimidine-10-carboxamide
*N*-(3,4-dichlorophenyl)-2-(trifluoromethyl)-6,7,8,9-tetrahydro-5H-5,8-epiminocyclohepta[d]-pyrimidine-10-carboxamide
*N*-(3,4-dichlorophenyl)-2-methyl-6,7,8,9-tetrahydro-5H-5,8-epiminocyclohepta[d]-pyrimidine-10-carboxamide
*N*-(3,4-dichlorophenyl)-2-phenyl-6,7,8,9-tetrahydro-5H-5,8-epiminocyclohepta[d]-pyrimidine-10-carboxamide
*N*-(3-chloro-4-(trifluoromethyl)phenyl)-2-(trifluoromethyl)-6,7,8,9-tetrahydro-5H-5,8-epiminocyclohepta[d]pyrimidine-10-carboxamide
*N*-(4-chloro-3-(trifluoromethyl)phenyl)-2-phenyl-6,7,8,9-tetrahydro-5H-5,8-epiminocyclo-hepta[d]pyrimidine-10-carboxamide
*N*-(3-chloro-4-(trifluoromethyl)phenyl)-2-phenyl-6,7,8,9-tetrahydro-5H-5,8-epiminocyclo-hepta[d]pyrimidine-10-carboxamide
*N*-(3,4-dichlorophenyl)-2-hydroxy-6,7,8,9-tetrahydro-5H-5,8-epiminocyclohepta[d]-pyrimidine-10-carboxamide
*N*-(3-chloro-4-(trifluoromethyl)phenyl)-2-hydroxy-6,7,8,9-tetrahydro-5H-5,8-epiminocyclo-hepta[d]pyrimidine-10-carboxamide
*N*-(3,4-dichlorophenyl)-6,7,8,9-tetrahydro-5H-5,8-epiminobenzo[7]annulene-10-carboxamide
*N*-(3-chloro-4-(trifluoromethyl)phenyl)-6,7,8,9-tetrahydro-5H-5,8-epiminocyclo-hepta[c]pyridine-10-carboxamide
*N*-(3,4-dichlorophenyl)-6,7,8,9-tetrahydro-5H-5,8-epiminocyclohepta[c]pyridine-10-carboxamide
*N*-(3-chloro-4-(trifluoromethyl)phenyl)-6,7,8,9-tetrahydro-5H-6,9-epiminocyclohepta[c]-pyridine-10-carboxamide
*N*-(3-chloro-4-(trifluoromethyl)phenyl)-6,7,8,9-tetrahydro-5H-5,8-epiminocyclohepta[d]-pyrimidine-10-carboxamide
*N*-(3-chloro-4-(trifluoromethyl)phenyl)-6,7,8,9-tetrahydro-5H-5,8-epiminocyclohepta[d]-pyrimidine-10-carboxamide
*N*-(4-chloro-3-(trifluoromethyl)phenyl)-6,7,8,9-tetrahydro-5H-5,8-epiminocyclohepta[d]-pyrimidine-10-carboxamide
*N*-(3-chloro-4-(trifluoromethyl)phenyl)-2-oxo-3,5,6,7,8,9-hexahydro-2H-5,8-epiminocyclo-hepta[d]pyrimidine-10-carboxamide
*N*-(3-chloro-4-(trifluoromethyl)phenyl)-2-oxo-3,5,6,7,8,9-hexahydro-2H-5,8-epiminocyclo-hepta[d]pyrimidine-10-carboxamide
*N*-(3-chloro-4-(trifluoromethyl)phenyl)-2-fluoro-6,7,8,9-tetrahydro-5H-5,8-epiminocyclo-hepta[d]pyrimidine-10-carboxamide
*N*-(3,4-dichlorophenyl)-2-fluoro-6,7,8,9-tetrahydro-5H-5,8-epiminocyclohepta[d]-pyrimidine-10-carboxamide
*N*-(3-chloro-4-(trifluoromethyl)phenyl)-2-hydroxy-9-(hydroxyimino)-6,7,8,9-tetrahydro-5H-5,8-epiminocyclohepta[d]pyrimidine-10-carboxamide
*N*-(3,4-dichlorophenyl)-6,7,8,9-tetrahydro-5H-5,8-epiminocyclohepta[d]pyrimidine-10-carboxamide
*N*-(4-chlorophenyl)-6,7,8,9-tetrahydro-5H-5,8-epiminocyclohepta[d]pyrimidine-10-carboxamide
*N*-(3-chlorophenyl)-6,7,8,9-tetrahydro-5H-5,8-epiminocyclohepta[d]pyrimidine-10-carboxamide
*N*-(5-chloro-2-fluorophenyl)-6,7,8,9-tetrahydro-5H-5,8-epiminocyclohepta[d]pyrimidine-10-carboxamide
*N*-(3,4-dichlorophenyl)-6,7,8,9-tetrahydro-5H-5,8-epiminocyclohepta[d]pyrimidine-10-carboxamide
*N*-(3,4-dichlorophenyl)-6,7,8,9-tetrahydro-5H-5,8-epiminocyclohepta[d]pyrimidine-10-carboxamide
*N*-(3-chloro-4-(trifluoromethyl)phenyl)-6,7,8,9-tetrahydro-5H-5,8-epiminobenzo[7]-annulene-10-carboxamide
*N*-(4-chloro-3-(trifluoromethyl)phenyl)-6,7,8,9-tetrahydro-5H-5,8-epiminobenzo[7]-annulene-10-carboxamide
*N*-(3,4-dichlorophenyl)-2-methoxy-6,7,8,9-tetrahydro-5H-5,8-epiminobenzo[7]annulene-10-carboxamide
*N*-(3,4-dichlorophenyl)-2-hydroxy-6,7,8,9-tetrahydro-5H-5,8-epiminobenzo[7]annulene-10-carboxamide
10-((3,4-dichlorophenyl)carbamoyl)-6,7,8,9-tetrahydro-5H-5,8-epiminocyclohepta[c]-pyridine 2-oxide
10-((3,4-dichlorophenyl)carbamoyl)-6,7,8,9-tetrahydro-5H-6,9-epiminocyclohepta[c]-pyridine 2-oxide
2-chloro-*N*-(3,4-dichlorophenyl)-6,7,8,9-tetrahydro-5H-5,8-epiminobenzo[7]annulene-10-carboxamide
1-amino-*N*-(3,4-dichlorophenyl)-6,7,8,9-tetrahydro-5H-6,9-epiminocyclohepta[c]pyridine-10-carboxamide
3-amino-*N*-(3,4-dichlorophenyl)-6,7,8,9-tetrahydro-5H-6,9-epiminocyclohepta[c]pyridine-10-carboxamide
1-amino-*N*-(3,4-dichlorophenyl)-6,7,8,9-tetrahydro-5H-5,8-epiminocyclohepta[c]pyridine-10-carboxamide
3-amino-*N*-(3,4-dichlorophenyl)-6,7,8,9-tetrahydro-5H-5,8-epiminocyclohepta[c]pyridine-10-carboxamide
1-(tert-butylamino)-*N*-(3,4-dichlorophenyl)-6,7,8,9-tetrahydro-5H-5,8-epiminocyclo-hepta[c]pyridine-10-carboxamide
3-(tert-butylamino)-*N*-(3,4-dichlorophenyl)-6,7,8,9-tetrahydro-5H-5,8-epiminocyclo-hepta[c]pyridine-10-carboxamide
*N*-(3,4-dichlorophenyl)-3-fluoro-6,7,8,9-tetrahydro-5H-6,9-epiminocyclohepta[c]pyridine-10-carboxamide
*N*-(3,4-dichlorophenyl)-1-fluoro-6,7,8,9-tetrahydro-5H-6,9-epiminocyclohepta[c]pyridine-10-carboxamide
*N*-(3,4-dichlorophenyl)-3-fluoro-6,7,8,9-tetrahydro-5H-5,8-epiminocyclohepta[c]pyridine-10-carboxamide
2-chloro-*N*-(3-chloro-4-(trifluoromethyl)phenyl)-6,7,8,9-tetrahydro-5H-5,8-epiminobenzo-[7]annulene-10-carboxamide
2-cyano-*N*-(3,4-dichlorophenyl)-6,7,8,9-tetrahydro-5H-5,8-epiminobenzo[7]annulene-10-carboxamide
2-amino-*N*-(3,4-dichlorophenyl)-6,7,8,9-tetrahydro-5H-5,8-epiminobenzo[7]annulene-10-carboxamide
*N*-(3,4-dichlorophenyl)-2-fluoro-6,7,8,9-tetrahydro-5H-5,8-epiminobenzo[7]annulene-10-carboxamide
*N*-(3-chloro-4-(trifluoromethyl)phenyl)-2-fluoro-6,7,8,9-tetrahydro-5H-5,8-epiminobenzo-[7]annulene-10-carboxamide
*N*-(3,4-dichlorophenyl)-3-oxo-3,5,6,7,8,9-hexahydro-2H-6,9-epiminocyclohepta[c]pyridine-10-carboxamide
1-chloro-*N*-(3,4-dichlorophenyl)-6,7,8,9-tetrahydro-5H-5,8-epiminocyclohepta[c]pyridine-10-carboxamide
*N*-(3,4-dichlorophenyl)-1-fluoro-6,7,8,9-tetrahydro-5H-5,8-epiminocyclohepta[c]pyridine-10-carboxamide
*N*-(4,5-dichloro-2-fluorophenyl)-1-fluoro-6,7,8,9-tetrahydro-5H-5,8-epiminocyclohepta[c]-pyridine-10-carboxamide
*N*-(3,4-dichlorophenyl)-3-fluoro-6,7,8,9-tetrahydro-5H-5,8-epiminobenzo[7]annulene-10-carboxamide
*N*-(3-chloro-4-(trifluoromethyl)phenyl)-3-fluoro-6,7,8,9-tetrahydro-5H-5,8-epiminobenzo-[7]annulene-10-carboxamide
tert-butyl-(10-((3,4-dichlorophenyl)carbamoyl)-6,7,8,9-tetrahydro-5H-6,9-epiminocyclo-hepta[c]pyridin-1-yl)carbamate
*N*-(3,4-dichlorophenyl)-1-fluoro-6,7,8,9-tetrahydro-5H-5,8-epiminobenzo[7]annulene-10-carboxamide
*N*-(3-chloro-4-(trifluoromethyl)phenyl)-1-fluoro-6,7,8,9-tetrahydro-5H-5,8-epiminobenzo[7]annulene-10-carboxamide
*N*-(3-chloro-4-(trifluoromethyl)phenyl)-1-fluoro-6,7,8,9-tetrahydro-5H-5,8-epiminocyclo-hepta[c]pyridine-10-carboxamide
*N*-(3,4-dichlorophenyl)-3-methoxy-6,7,8,9-tetrahydro-5H-6,9-epiminocyclohepta[c]-pyridine-10-carboxamide
*N*-(3,4-dichlorophenyl)-6,7,8,9-tetrahydro-5H-5,8-epiminocyclohepta[b]pyridine-10-carboxamide
*N*-(4,5-dichloro-2-cyanophenyl)-3-oxo-3,5,6,7,8,9-hexahydro-2H-6,9-epiminocyclo-hepta[c]pyridine-10-carboxamide
*N*-(3,4-dichlorophenyl)-3-fluoro-6,7,8,9-tetrahydro-5H-6,9-epiminocyclohepta[c]pyridine-10-carboxamide
10-((3,4-dichlorophenyl)carbamoyl)-6,7,8,9-tetrahydro-5H-5,8-epiminocyclohepta[c]-pyridine 2-oxide
*N*-(5-chloro-2-fluoro-4-(trifluoromethyl)phenyl)-3-oxo-3,5,6,7,8,9-hexahydro-2H-6,9-epiminocyclohepta[c]pyridine-10-carboxamide
*N*-(4,5-dichloro-2-fluorophenyl)-3-oxo-3,5,6,7,8,9-hexahydro-2H-6,9-epiminocyclo-hepta[c]pyridine-10-carboxamide
*N*-(3,4-dichlorophenyl)-4-fluoro-6,7,8,9-tetrahydro-5H-6,9-epiminocyclohepta[c]pyridine-10-carboxamide
*N*-(4,5-dichloro-2-cyanophenyl)-1-fluoro-6,7,8,9-tetrahydro-5H-5,8-epiminocyclohepta[c]-pyridine-10-carboxamide
3-bromo-*N*-(3,4-dichlorophenyl)-6,7,8,9-tetrahydro-5H-6,9-epiminocyclohepta[c]pyridine-10-carboxamide
3-chloro-*N*-(3,4-dichlorophenyl)-6,7,8,9-tetrahydro-5H-6,9-epiminocyclohepta[c]pyridine-10-carboxamide
*N*-(5-chloro-2-fluoro-4-(trifluoromethyl)phenyl)-1-fluoro-6,7,8,9-tetrahydro-5H-5,8-epiminocyclohepta[c]pyridine-10-carboxamide
*N*-(4-chloro-5-(trifluoromethyl)pyridin-2-yl)-1-fluoro-6,7,8,9-tetrahydro-5H-5,8-epimino-cyclohepta[c]pyridine-10-carboxamide
*N*-(3,4-dichlorophenyl)-3-oxo-3,5,6,7,8,9-hexahydro-2H-6,9-epiminocyclohepta[c]pyridine-10-carboxamide
*N*-(3,4-dichlorophenyl)-6,7,8,9-tetrahydro-5H-6,9-epiminocyclohepta[c]pyridine-10-carboxamide
*N*-(3,4-dichlorophenyl)-2-oxo-3,5,6,7,8,9-hexahydro-2H-5,8-epiminocyclohepta[d]-pyrimidine-10-carboxamide
*N*-(3,4-dichlorophenyl)-2-oxo-3,5,6,7,8,9-hexahydro-2H-5,8-epiminocyclohepta[d]-pyrimidine-10-carboxamide
*N*-(3,4-dichlorophenyl)-6,7,8,9-tetrahydro-5H-5,8-epiminocyclohepta[c]pyridine-10-carboxamide
*N*-(3,4-dichlorophenyl)-6,7,8,9-tetrahydro-5H-5,8-epiminocyclohepta[c]pyridine-10-carboxamide
*N*-(3,4-dichlorophenyl)-1-fluoro-6,7,8,9-tetrahydro-5H-5,8-epiminocyclohepta[c]pyridine-10-carboxamide
*N*-(3,4-dichlorophenyl)-1-fluoro-6,7,8,9-tetrahydro-5H-5,8-epiminocyclohepta[c]pyridine-10-carboxamide
*N*-(3,4-dichlorophenyl)-6,7,8,9-tetrahydro-5H-6,9-epiminocyclohepta[c]pyridine-10-carboxamide
*N*-(3,4-dichlorophenyl)-6,7,8,9-tetrahydro-5H-6,9-epiminocyclohepta[c]pyridine-10-carboxamide
*N*-(3,4-dichlorophenyl)-9-fluoro-6,7,8,9-tetrahydro-5H-5,8-epiminocyclohepta[d]-pyrimidine-10-carboxamide
*N*-(3,4-dichlorophenyl)-9-fluoro-6,7,8,9-tetrahydro-5H-5,8-epiminocyclohepta[d-]pyrimidine-10-carboxamide
*N*-(3,4-dichlorophenyl)-9-fluoro-6,7,8,9-tetrahydro-5H-5,8-epiminocyclohepta[d]-pyrimidine-10-carboxamide
*N*-(3-chloro-4-(trifluoromethyl)phenyl)-8-methyl-6,7,8,9-tetrahydro-5H-5,8-epiminocyclo-hepta[c]pyridine-10-carboxamide
*N*-(4,5-dichloro-2-methylphenyl)-1-fluoro-6,7,8,9-tetrahydro-5H-5,8-epiminocyclo-hepta[c]pyridine-10-carboxamide
*N*-(4,5-dichloro-2-methoxyphenyl)-1-fluoro-6,7,8,9-tetrahydro-5H-5,8-epiminocyclo-hepta[c]pyridine-10-carboxamide
1-fluoro-*N*-(2-fluoro-5-(trifluoromethyl)phenyl)-6,7,8,9-tetrahydro-5H-5,8-epiminocyclo-hepta[c]pyridine-10-carboxamide
1-fluoro-*N*-(2-fluoro-4-(trifluoromethyl)phenyl)-6,7,8,9-tetrahydro-5H-5,8-epiminocyclo-hepta[c]pyridine-10-carboxamide
1-fluoro-*N*-(4-(trifluoromethyl)phenyl)-6,7,8,9-tetrahydro-5H-5,8-epiminocyclohepta-[c]pyridine-10-carboxamide
methyl 2-chloro-4-(-1-fluoro-6,7,8,9-tetrahydro-5H-5,8-epiminocyclohepta[c]pyridine-10-carboxamido)benzoate
methyl 2-chloro-5-(-1-fluoro-6,7,8,9-tetrahydro-5H-5,8-epiminocyclohepta[c]pyridine-10-carboxamido)benzoate
*N*-(3-chloro-4-(trifluoromethoxy)phenyl)-1-fluoro-6,7,8,9-tetrahydro-5H-5,8-epiminocyclo-hepta[c]pyridine-10-carboxamide
methyl 2-chloro-4-fluoro-5-(1-fluoro-6,7,8,9-tetrahydro-5H-5,8-epiminocyclohepta[c]-pyridine-10-carboxamido)benzoate
*N*-(4-chloro-2-fluoro-5-(trifluoromethyl)phenyl)-1-fluoro-6,7,8,9-tetrahydro-5H-5,8-epiminocyclohepta[c]pyridine-10-carboxamide
*N*-(3-chloro-4-(difluoromethoxy)phenyl)-3-oxo-3,5,6,7,8,9-hexahydro-2H-6,9-epiminocyclohepta[c]pyridine-10-carboxamide
*N*-(3-chloro-4-(trifluoromethoxy)phenyl)-3-oxo-3,5,6,7,8,9-hexahydro-2H-6,9-epiminocyclohepta[c]pyridine-10-carboxamide
*N*-(4-chloro-2-fluoro-5-(trifluoromethyl)phenyl)-3-oxo-3,5,6,7,8,9-hexahydro-2H-6,9-epiminocyclohepta[c]pyridine-10-carboxamide
methyl 2-chloro-4-(3-oxo-3,5,6,7,8,9-hexahydro-2H-6,9-epiminocyclohepta[c]pyridine-10-carboxamido)benzoate
methyl 2-chloro-5-(3-oxo-3,5,6,7,8,9-hexahydro-2H-6,9-epiminocyclohepta[c]pyridine-10-carboxamido)benzoate
methyl 2-chloro-4-fluoro-5-(3-oxo-3,5,6,7,8,9-hexahydro-2H-6,9-epiminocyclohepta[c]-pyridine-10-carboxamido)benzoate
*N*-(3-chloro-4-(difluoromethoxy)phenyl)-1-fluoro-6,7,8,9-tetrahydro-5H-5,8-epiminocyclo-hepta[c]pyridine-10-carboxamide
*N*-(3,4-dichlorophenyl)-9-methyl-6,7,8,9-tetrahydro-5H-5,8-epiminocyclohepta[c]pyridine-10-carboxamide
*N*-(3,4-dichlorophenyl)-3,4-difluoro-6,7,8,9-tetrahydro-5H-6,9-epiminocyclohepta[c]-pyridine-10-carboxamide
*N*-(5-chloro-2-fluoro-4-(trifluoromethyl)phenyl)-3,4-difluoro-6,7,8,9-tetrahydro-5H-6,9-epiminocyclohepta[c]pyridine-10-carboxamide
*N*-(4,5-dichloro-2-fluorophenyl)-3,4-difluoro-6,7,8,9-tetrahydro-5H-6,9-epiminocyclo-hepta[c]pyridine-10-carboxamide
*N*-(5-chloro-2-fluoro-4-(trifluoromethyl)phenyl)-4-fluoro-3-oxo-3,5,6,7,8,9-hexahydro-2H-6,9-epiminocyclohepta[c]pyridine-10-carboxamide
*N*-(4,5-dichloro-2-fluorophenyl)-4-fluoro-3-oxo-3,5,6,7,8,9-hexahydro-2H-6,9-epiminocyclohepta[c]pyridine-10-carboxamide
*N*-(3,4-dichlorophenyl)-3,5-difluoro-6,7,8,9-tetrahydro-5H-6,9-epiminocyclohepta[c]-pyridine-10-carboxamide
*N*-(4-bromo-5-chloro-2-fluorophenyl)-3-oxo-3,5,6,7,8,9-hexahydro-2H-6,9-epiminocyclo-hepta[c]pyridine-10-carboxamide
*N*-(5-bromo-4-chloro-2-fluorophenyl)-3-oxo-3,5,6,7,8,9-hexahydro-2H-6,9-epiminocyclo-hepta[c]pyridine-10-carboxamide
*N*-(4-bromo-3-chlorophenyl)-3-oxo-3,5,6,7,8,9-hexahydro-2H-6,9-epiminocyclohepta[c]-pyridine-10-carboxamide
*N*-(5-bromo-2-fluoro-4-(trifluoromethyl)phenyl)-3-oxo-3,5,6,7,8,9-hexahydro-2H-6,9-epiminocyclohepta[c]pyridine-10-carboxamide
*N*-(3-bromo-4-chlorophenyl)-3-oxo-3,5,6,7,8,9-hexahydro-2H-6,9-epiminocyclohepta[c]-pyridine-10-carboxamide
*N*-(5-chloro-2-fluoro-4-(trifluoromethyl)phenyl)-4-fluoro-3-oxo-3,5,6,7,8,9-hexahydro-2H-6,9-epiminocyclohepta[c]pyridine-10-carboxamide
*N*-(5-chloro-2-fluoro-4-(trifluoromethyl)phenyl)-4-fluoro-3-oxo-3,5,6,7,8,9-hexahydro-2H-6,9-epiminocyclohepta[c]pyridine-10-carboxamide
1-bromo-*N*-(3,4-dichlorophenyl)-6,7,8,9-tetrahydro-5H-5,8-epiminocyclohepta[c]pyridine-10-carboxamide
*N*-(4,5-dichloro-2-hydroxyphenyl)-1-fluoro-6,7,8,9-tetrahydro-5H-5,8-epiminocyclo-hepta[c]pyridine-10-carboxamide
*N*-(3,4-dichlorophenyl)-2-methyl-3-oxo-3,5,6,7,8,9-hexahydro-2H-6,9-epiminocyclo-hepta[c]pyridine-10-carboxamide
*N*-(3-cyano-4-(trifluoromethyl)phenyl)-1-fluoro-6,7,8,9-tetrahydro-5H-5,8-epiminocyclo-hepta[c]pyridine-10-carboxamide
1-fluoro-*N*-(3-methoxy-4-(oxazol-5-yl)phenyl)-6,7,8,9-tetrahydro-5H-5,8-epiminocyclo-hepta[c]pyridine-10-carboxamide
*N*-(3-bromo-4-(trifluoromethyl)phenyl)-1-fluoro-6,7,8,9-tetrahydro-5H-5,8-epiminocyclo-hepta[c]pyridine-10-carboxamide
*N*-(3-chloro-2,4-difluorophenyl)-1-fluoro-6,7,8,9-tetrahydro-5H-5,8-epiminocyclo-hepta[c]pyridine-10-carboxamide
1-fluoro-*N*-(3-fluorophenyl)-6,7,8,9-tetrahydro-5H-5,8-epiminocyclohepta[c]pyridine-10-carboxamide
1-fluoro-*N*-(3-(trifluoromethyl)phenyl)-6,7,8,9-tetrahydro-5H-5,8-epiminocyclohepta[c]-pyridine-10-carboxamide
1-fluoro-*N*-(4-(trifluoromethoxy)phenyl)-6,7,8,9-tetrahydro-5H-5,8-epiminocyclohepta[c]-pyridine-10-carboxamide
*N*-([1,1'-biphenyl]-4-yl)-1-fluoro-6,7,8,9-tetrahydro-5H-5,8-epiminocyclohepta[c]pyridine-10-carboxamide
*N*-(3,5-bis(trifluoromethyl)phenyl)-1-fluoro-6,7,8,9-tetrahydro-5H-5,8-epiminocyclohepta[c-]pyridine-10-carboxamide
1-fluoro-*N*-(4-methyl-3-(trifluoromethyl)phenyl)-6,7,8,9-tetrahydro-5H-5,8-epiminocyclo-hepta[c]pyridine-10-carboxamide
*N*-(2,3-dichlorophenyl)-1-fluoro-6,7,8,9-tetrahydro-5H-5,8-epiminocyclohepta[c]pyridine-10-carboxamide
*N*-(2,4-difluorophenyl)-1-fluoro-6,7,8,9-tetrahydro-5H-5,8-epiminocyclohepta[c]pyridine-10-carboxamide
*N*-(4-cyano-3-(trifluoromethyl)phenyl)-1-fluoro-6,7,8,9-tetrahydro-5H-5,8-epiminocyclo-hepta[c]pyridine-10-carboxamide
*N*-(3-chloro-5-(trifluoromethyl)phenyl)-1-fluoro-6,7,8,9-tetrahydro-5H-5,8-epiminocyclo-hepta[c]pyridine-10-carboxamide
*N*-(3-cyano-4-fluorophenyl)-1-fluoro-6,7,8,9-tetrahydro-5H-5,8-epiminocyclohepta[c]-pyridine-10-carboxamide
1-fluoro-*N*-(3,4,5-trichlorophenyl)-6,7,8,9-tetrahydro-5H-5,8-epiminocyclohepta[c]pyridine-10-carboxamide
1-fluoro-*N*-(3-methyl-4-(trifluoromethyl)phenyl)-6,7,8,9-tetrahydro-5H-5,8-epiminocyclo-hepta[c]pyridine-10-carboxamide
*N*-(4-bromophenyl)-1-fluoro-6,7,8,9-tetrahydro-5H-5,8-epiminocyclohepta[c]pyridine-10-carboxamide
*N*-(4-(difluoromethoxy)phenyl)-1-fluoro-6,7,8,9-tetrahydro-5H-5,8-epiminocyclohepta[c]-pyridine-10-carboxamide
*N*-(3-bromophenyl)-1-fluoro-6,7,8,9-tetrahydro-5H-5,8-epiminocyclohepta[c]pyridine-10-carboxamide
1-fluoro-*N*-(3-(trifluoromethoxy)phenyl)-6,7,8,9-tetrahydro-5H-5,8-epiminocyclo-hepta[c]pyridine-10-carboxamide
*N*-(3-bromo-4-fluorophenyl)-1-fluoro-6,7,8,9-tetrahydro-5H-5,8-epiminocyclo-hepta[c]pyridine-10-carboxamide
*N*-(3,5-difluorophenyl)-1-fluoro-6,7,8,9-tetrahydro-5H-5,8-epiminocyclohepta[c]pyridine-10-carboxamide
*N*-(5-chloro-2-fluorophenyl)-1-fluoro-6,7,8,9-tetrahydro-5H-5,8-epiminocyclohepta[c]-pyridine-10-carboxamide
1-fluoro-*N*-(2,3,4-trifluorophenyl)-6,7,8,9-tetrahydro-5H-5,8-epiminocyclohepta[c]pyridine-10-carboxamide
*N*-(4-cyanophenyl)-1-fluoro-6,7,8,9-tetrahydro-5H-5,8-epiminocyclohepta[c]pyridine-10-carboxamide
*N*-(4-chloro-2-fluorophenyl)-1-fluoro-6,7,8,9-tetrahydro-5H-5,8-epiminocyclohepta[c]-pyridine-10-carboxamide
*N*-(3-chloro-4-fluorophenyl)-1-fluoro-6,7,8,9-tetrahydro-5H-5,8-epiminocyclohepta[c]-pyridine-10-carboxamide
*N*-(4-acrylamido-3-chlorophenyl)-1-fluoro-6,7,8,9-tetrahydro-5H-5,8-epiminocyclohepta[c]-pyridine-10-carboxamide
*N*-(5-bromo-2-fluoro-4-(trifluoromethyl)phenyl)-1-fluoro-6,7,8,9-tetrahydro-5H-5,8-epiminocyclohepta[c]pyridine-10-carboxamide
*N*-(4,5-dichloro-2-fluorophenyl)-2-fluoro-6,7,8,9-tetrahydro-5H-5,8-epiminocyclohepta[b]-pyridine-10-carboxamide
*N*-(5-chloro-2-fluoro-4-(trifluoromethyl)phenyl)-2-fluoro-6,7,8,9-tetrahydro-5H-5,8-epiminocyclohepta[b]pyridine-10-carboxamide
*N*-(4-(1H-pyrazol-1-yl)phenyl)-1-fluoro-6,7,8,9-tetrahydro-5H-5,8-epiminocyclohepta[c]-pyridine-10-carboxamide
*N*-([1,1'-biphenyl]-3-yl)-1-fluoro-6,7,8,9-tetrahydro-5H-5,8-epiminocyclohepta[c]pyridine-10-carboxamide
1-fluoro-*N*-(4-phenoxyphenyl)-6,7,8,9-tetrahydro-5H-5,8-epiminocyclohepta[c]pyridine-10-carboxamide
1-fluoro-*N*-(4-(thiazol-4-yl)phenyl)-6,7,8,9-tetrahydro-5H-5,8-epiminocyclohepta[c]-pyridine-10-carboxamide
1-fluoro-*N*-(4-(4-methyloxazol-5-yl)phenyl)-6,7,8,9-tetrahydro-5H-5,8-epiminocyclo-hepta[c]pyridine-10-carboxamide
1-fluoro-*N*-(4-(oxazol-5-yl)phenyl)-6,7,8,9-tetrahydro-5H-5,8-epiminocyclohepta[c]-pyridine-10-carboxamide
*N*-(5-chloro-2-fluoro-4-(trifluoromethyl)phenyl)-2-oxo-2,5,6,7,8,9-hexahydro-1H-5,8-epiminocyclohepta[b]pyridine-10-carboxamide
*N*-(4,5-dichloro-2-fluorophenyl)-4-fluoro-6,7,8,9-tetrahydro-5H-5,8-epiminocyclo-hepta[c]pyridine-10-carboxamide
*N*-([1,1'-biphenyl]-4-yl)-3-oxo-3,5,6,7,8,9-hexahydro-2H-6,9-epiminocyclohepta[c]pyridine-10-carboxamide
*N*-([1,1'-biphenyl]-4-yl)-1-fluoro-6,7,8,9-tetrahydro-5H-5,8-epiminocyclohepta[c]pyridine-10-carboxamide
*N*-(4,5-dichloro-2-fluorophenyl)-6,7,8,9-tetrahydro-5H-5,8-epiminocyclohepta[d]-pyridazine-10-carboxamide
*N*-(5-chloro-2-fluoro-4-(trifluoromethyl)phenyl)-6,7,8,9-tetrahydro-5H-5,8-epiminocyclo-hepta[d]pyridazine-10-carboxamide
1,4-dichloro-*N*-(5-chloro-2-fluoro-4-(trifluoromethyl)phenyl)-6,7,8,9-tetrahydro-5H-5,8-epiminocyclohepta[d]pyridazine-10-carboxamide
*N*-(5-chloro-2-fluoro-4-(trifluoromethyl)phenyl)-2-fluoro-6,7,8,9-tetrahydro-5H-5,8-epiminocyclohepta[b]pyridine-10-carboxamide
*N*-(5-chloro-2-fluoro-4-(trifluoromethyl)phenyl)-2-fluoro-6,7,8,9-tetrahydro-5H-5,8-epiminocyclohepta[b]pyridine-10-carboxamide
*N*-(3,4-dichlorophenyl)-3-fluoro-6,7,8,9-tetrahydro-5H-6,9-epiminocyclohepta[c]pyridine-10-carboxamide
*N*-(4-chloro-3-cyanophenyl)-1-fluoro-6,7,8,9-tetrahydro-5H-5,8-epiminocyclohepta[c]-pyridine-10-carboxamide
*N*-(4,5-dichloro-2-fluorophenyl)-1-(trifluoromethyl)-6,7,8,9-tetrahydro-5H-5,8-epiminocyclohepta[c]pyridine-10-carboxamide
*N*-(3-chloro-4-(trifluoromethyl)phenyl)-6,7,8,9-tetrahydro-5H-5,8-epiminocyclohepta[d]-pyrimidine-10-carboxamide
*N*-(3-Chloro-4-(trifluoromethyl)phenyl)-9-(hydroxyimino)-2-oxo-3,5,6,7,8,9-hexahydro-2H-5,8-epiminocyclohepta[d]pyrimidine-10-carboxamide
*N*-(3,4-dichlorophenyl)-9-methyl-6,7,8,9-tetrahydro-5H-5,8-epiminocyclohepta[c]pyridine-10-carboxamide

(±)-*N*-(4-(trifluoromethyl)phenyl)-6,7,8,9-tetrahydro-5H-5,8-epiminocyclohepta[d]pyrimidine-10-carboxamide
(±)-*N*-(3-chloro-4-(trifluoromethyl)phenyl)-6,7,8,9-tetrahydro-5H-5,8-epiminocyclohepta[d]-pyrimidine-10-carboxamide
(±)-*N*-(4-chloro-3-(trifluoromethyl)phenyl)-6,7,8,9-tetrahydro-5H-5,8-epiminocyclohepta[d]-pyrimidine-10-carboxamide
(±)-*N*-(3,5-dichlorophenyl)-6,7,8,9-tetrahydro-5H-5,8-epiminocyclohepta[d]pyrimidine-10-carboxamide
(±)-*N*-(4-fluoro-3-(trifluoromethyl)phenyl)-6,7,8,9-tetrahydro-5H-5,8-epiminocyclohepta[d]-pyrimidine-10-carboxamide
(±)-*N*-(3,4-difluorophenyl)-6,7,8,9-tetrahydro-5H-5,8-epiminocyclohepta[d]pyrimidine-10-carboxamide
(±)-*N*-(4-bromo-3-chlorophenyl)-6,7,8,9-tetrahydro-5H-5,8-epiminocyclohepta[d]pyrimidine-10-carboxamide
(±)-*N*-(3-chloro-4-methoxyphenyl)-6,7,8,9-tetrahydro-5H-5,8-epiminocyclohepta[d]pyrimidine-10-carboxamide
(±)-*N*-(3-chloro-4-cyanophenyl)-6,7,8,9-tetrahydro-5H-5,8-epiminocyclohepta[d]pyrimidine-10-carboxamide
(±)-*N*-(3-bromo-4-chlorophenyl)-6,7,8,9-tetrahydro-5H-5,8-epiminocyclohepta[d]pyrimidine-10-carboxamide
(±)-*N*-(3-bromo-4-cyanophenyl)-6,7,8,9-tetrahydro-5H-5,8-epiminocyclohepta[d]pyrimidine-10-carboxamide
(±)-*N*-(3-bromo-4-(trifluoromethyl)phenyl)-6,7,8,9-tetrahydro-5H-5,8-epiminocyclohepta[d]-pyrimidine-10-carboxamide
(±)-*N*-(3,4-dibromophenyl)-6,7,8,9-tetrahydro-5H-5,8-epiminocyclohepta[d]pyrimidine-10-carboxamide
(±)-*N*-(3,4-dichlorophenyl)-6,7,8,9-tetrahydro-5H-6,9-epiminocyclohepta[d]pyrimidine-10-carboxamide
(±)-*N*-(3-chloro-4-(trifluoromethyl)phenyl)-6,7,8,9-tetrahydro-5H-6,9-epiminocyclohepta[d]-pyrimidine-10-carboxamide
(±)-*N*-(3,4-dichlorophenyl)-2-(trifluoromethyl)-6,7,8,9-tetrahydro-5H-5,8-epiminocyclohepta-[d]pyrimidine-10-carboxamide
(±)-*N*-(3,4-dichlorophenyl)-2-methyl-6,7,8,9-tetrahydro-5H-5,8-epiminocyclohepta[d]-pyrimidine-10-carboxamide
(±)-*N*-(3,4-dichlorophenyl)-2-phenyl-6,7,8,9-tetrahydro-5H-5,8-epiminocyclohepta[d]-pyrimidine-10-carboxamide
(±)-*N*-(3-chloro-4-(trifluoromethyl)phenyl)-2-(trifluoromethyl)-6,7,8,9-tetrahydro-5H-5,8-epiminocyclohepta[d]pyrimidine-10-carboxamide
(±)-*N*-(4-chloro-3-(trifluoromethyl)phenyl)-2-phenyl-6,7,8,9-tetrahydro-5H-5,8-epimino-cyclohepta[d]pyrimidine-10-carboxamide
(±)-*N*-(3-chloro-4-(trifluoromethyl)phenyl)-2-phenyl-6,7,8,9-tetrahydro-5H-5,8-epimino-cyclohepta[d]pyrimidine-10-carboxamide
(±)-*N*-(3,4-dichlorophenyl)-2-hydroxy-6,7,8,9-tetrahydro-5H-5,8-epiminocyclohepta[d]-pyrimidine-10-carboxamide
(±)-*N*-(3-chloro-4-(trifluoromethyl)phenyl)-2-hydroxy-6,7,8,9-tetrahydro-5H-5,8-epimino-cyclohepta[d]pyrimidine-10-carboxamide
(±)-*N*-(3,4-dichlorophenyl)-6,7,8,9-tetrahydro-5H-5,8-epiminobenzo[7]annulene-10-carboxamide
(±)-*N*-(3-chloro-4-(trifluoromethyl)phenyl)-6,7,8,9-tetrahydro-5H-5,8-epiminocyclohepta[c]-pyridine-10-carboxamide
(±)-*N*-(3,4-dichlorophenyl)-6,7,8,9-tetrahydro-5H-5,8-epiminocyclohepta[c]pyridine-10-carboxamide
(±)-*N*-(3-chloro-4-(trifluoromethyl)phenyl)-6,7,8,9-tetrahydro-5H-6,9-epiminocyclohepta-[c]pyridine-10-carboxamide
(±)-*N*-(3-chloro-4-(trifluoromethyl)phenyl)-6,7,8,9-tetrahydro-5H-5,8-epiminocyclohepta[d]-pyrimidine-10-carboxamide
(±)-*N*-(3-chloro-4-(trifluoromethyl)phenyl)-6,7,8,9-tetrahydro-5H-5,8-epiminocyclohepta[d]-pyrimidine-10-carboxamide
(±)-*N*-(4-chloro-3-(trifluoromethyl)phenyl)-6,7,8,9-tetrahydro-5H-5,8-epiminocyclohepta[d]-pyrimidine-10-carboxamide
(±)-*N*-(3-chloro-4-(trifluoromethyl)phenyl)-2-oxo-3,5,6,7,8,9-hexahydro-2H-5,8-epimino-cyclohepta[d]pyrimidine-10-carboxamide
(±)-*N*-(3-chloro-4-(trifluoromethyl)phenyl)-2-oxo-3,5,6,7,8,9-hexahydro-2H-5,8-epimino-cyclohepta[d]pyrimidine-10-carboxamide
(±)-*N*-(3-chloro-4-(trifluoromethyl)phenyl)-2-fluoro-6,7,8,9-tetrahydro-5H-5,8-epimino-cyclohepta[d]pyrimidine-10-carboxamide
(±)-*N*-(3,4-dichlorophenyl)-2-fluoro-6,7,8,9-tetrahydro-5H-5,8-epiminocyclohepta[d]-pyrimidine-10-carboxamide
(±)-*N*-(3-chloro-4-(trifluoromethyl)phenyl)-2-hydroxy-9-(hydroxyimino)-6,7,8,9-tetrahydro-5H-5,8-epiminocyclohepta[d]pyrimidine-10-carboxamide
(±)-*N*-(3,4-dichlorophenyl)-6,7,8,9-tetrahydro-5H-5,8-epiminocyclohepta[d]pyrimidine-10-carboxamide
(±)-*N*-(4-chlorophenyl)-6,7,8,9-tetrahydro-5H-5,8-epiminocyclohepta[d]pyrimidine-10-carboxamide
(±)-*N*-(3-chlorophenyl)-6,7,8,9-tetrahydro-5H-5,8-epiminocyclohepta[d]pyrimidine-10-carboxamide
(±)-*N*-(5-chloro-2-fluorophenyl)-6,7,8,9-tetrahydro-5H-5,8-epiminocyclohepta[d]pyrimidine-10-carboxamide
(±)-*N*-(3,4-dichlorophenyl)-6,7,8,9-tetrahydro-5H-5,8-epiminocyclohepta[d]pyrimidine-10-carboxamide
(±)-*N*-(3,4-dichlorophenyl)-6,7,8,9-tetrahydro-5H-5,8-epiminocyclohepta[d]pyrimidine-10-carboxamide
(±)-*N*-(3-chloro-4-(trifluoromethyl)phenyl)-6,7,8,9-tetrahydro-5H-5,8-epiminobenzo[7]-annulene-10-carboxamide
(±)-*N*-(4-chloro-3-(trifluoromethyl)phenyl)-6,7,8,9-tetrahydro-5H-5,8-epiminobenzo[7]-annulene-10-carboxamide
(±)-*N*-(3,4-dichlorophenyl)-2-methoxy-6,7,8,9-tetrahydro-5H-5,8-epiminobenzo[7]annulene-10-carboxamide
(±)-*N*-(3,4-dichlorophenyl)-2-hydroxy-6,7,8,9-tetrahydro-5H-5,8-epiminobenzo[7]annulene-10-carboxamide
(±)-10-((3,4-dichlorophenyl)carbamoyl)-6,7,8,9-tetrahydro-5H-5,8-epiminocyclohepta[c]-pyridine 2-oxide
(±)-10-((3,4-dichlorophenyl)carbamoyl)-6,7,8,9-tetrahydro-5H-6,9-epiminocyclohepta[c]-pyridine 2-oxide
(±)-2-chloro-*N*-(3,4-dichlorophenyl)-6,7,8,9-tetrahydro-5H-5,8-epiminobenzo[7]annulene-10-carboxamide
(±)-1-amino-*N*-(3,4-dichlorophenyl)-6,7,8,9-tetrahydro-5H-6,9-epiminocyclohepta[c]pyridine-10-carboxamide
(±)-3-amino-*N*-(3,4-dichlorophenyl)-6,7,8,9-tetrahydro-5H-6,9-epiminocyclohepta[c]pyridine-10-carboxamide
(±)-1-amino-*N*-(3,4-dichlorophenyl)-6,7,8,9-tetrahydro-5H-5,8-epiminocyclohepta[c]pyridine-10-carboxamide
(±)-3-amino-*N*-(3,4-dichlorophenyl)-6,7,8,9-tetrahydro-5H-5,8-epiminocyclohepta[c]pyridine-10-carboxamide
(±)-1-(tert-butylamino)-*N*-(3,4-dichlorophenyl)-6,7,8,9-tetrahydro-5H-5,8-epiminocyclo-hepta[c]pyridine-10-carboxamide
(±)-3-(tert-butylamino)-*N*-(3,4-dichlorophenyl)-6,7,8,9-tetrahydro-5H-5,8-epiminocyclohepta-[c]pyridine-10-carboxamide
(±)-*N*-(3,4-dichlorophenyl)-3-fluoro-6,7,8,9-tetrahydro-5H-6,9-epiminocyclohepta[c]pyridine-10-carboxamide
(±)-*N*-(3,4-dichlorophenyl)-1-fluoro-6,7,8,9-tetrahydro-5H-6,9-epiminocyclohepta[c]pyridine-10-carboxamide
(±)-*N*-(3,4-dichlorophenyl)-3-fluoro-6,7,8,9-tetrahydro-5H-5,8-epiminocyclohepta[c]pyridine-10-carboxamide
(±)-2-chloro-*N*-(3-chloro-4-(trifluoromethyl)phenyl)-6,7,8,9-tetrahydro-5H-5,8-epiminobenzo-[7]annulene-10-carboxamide
(±)-2-cyano-*N*-(3,4-dichlorophenyl)-6,7,8,9-tetrahydro-5H-5,8-epiminobenzo[7]annulene-10-carboxamide
(±)-2-amino-*N*-(3,4-dichlorophenyl)-6,7,8,9-tetrahydro-5H-5,8-epiminobenzo[7]annulene-10-carboxamide
(±)-*N*-(3,4-dichlorophenyl)-2-fluoro-6,7,8,9-tetrahydro-5H-5,8-epiminobenzo[7]annulene-10-carboxamide
(±)-*N*-(3-chloro-4-(trifluoromethyl)phenyl)-2-fluoro-6,7,8,9-tetrahydro-5H-5,8-epiminobenzo[7]annulene-10-carboxamide
(±)-*N*-(3,4-dichlorophenyl)-3-oxo-3,5,6,7,8,9-hexahydro-2H-6,9-epiminocyclohepta[c]-pyridine-10-carboxamide
(±)-1-chloro-*N*-(3,4-dichlorophenyl)-6,7,8,9-tetrahydro-5H-5,8-epiminocyclohepta[c]pyridine-10-carboxamide
(±)-*N*-(3,4-dichlorophenyl)-1-fluoro-6,7,8,9-tetrahydro-5H-5,8-epiminocyclohepta[c]pyridine-10-carboxamide
(±)-*N*-(4,5-dichloro-2-fluorophenyl)-1-fluoro-6,7,8,9-tetrahydro-5H-5,8-epiminocyclohepta[c]-pyridine-10-carboxamide
(±)-*N*-(3,4-dichlorophenyl)-3-fluoro-6,7,8,9-tetrahydro-5H-5,8-epiminobenzo[7]annulene-10-carboxamide
(±)-*N*-(3-chloro-4-(trifluoromethyl)phenyl)-3-fluoro-6,7,8,9-tetrahydro-5H-5,8-epimino-benzo[7]annulene-10-carboxamide
tert-butyl ((±)-10-((3,4-dichlorophenyl)carbamoyl)-6,7,8,9-tetrahydro-5H-6,9-epimino-cyclohepta[c]pyridin-1-yl)carbamate
(±)-*N*-(3,4-dichlorophenyl)-1-fluoro-6,7,8,9-tetrahydro-5H-5,8-epiminobenzo[7]annulene-10-carboxamide
(±)-*N*-(3-chloro-4-(trifluoromethyl)phenyl)-1-fluoro-6,7,8,9-tetrahydro-5H-5,8-epimino-benzo[7]annulene-10-carboxamide
(±)-*N*-(3-chloro-4-(trifluoromethyl)phenyl)-1-fluoro-6,7,8,9-tetrahydro-5H-5,8-epimino-cyclohepta[c]pyridine-10-carboxamide
(±)-*N*-(3,4-dichlorophenyl)-3-methoxy-6,7,8,9-tetrahydro-5H-6,9-epiminocyclohepta[c]-pyridine-10-carboxamide
(±)-*N*-(3,4-dichlorophenyl)-6,7,8,9-tetrahydro-5H-5,8-epiminocyclohepta[b]pyridine-10-carboxamide
(±)-*N*-(4,5-dichloro-2-cyanophenyl)-3-oxo-3,5,6,7,8,9-hexahydro-2H-6,9-epimino-cyclohepta[c]pyridine-10-carboxamide
(±)-*N*-(3,4-dichlorophenyl)-3-fluoro-6,7,8,9-tetrahydro-5H-6,9-epiminocyclohepta[c]pyridine-10-carboxamide
(±)-10-((3,4-dichlorophenyl)carbamoyl)-6,7,8,9-tetrahydro-5H-5,8-epiminocyclohepta[c]-pyridine 2-oxide
(±)-*N*-(5-chloro-2-fluoro-4-(trifluoromethyl)phenyl)-3-oxo-3,5,6,7,8,9-hexahydro-2H-6,9-epiminocyclohepta[c]pyridine-10-carboxamide
(±)-*N*-(4,5-dichloro-2-fluorophenyl)-3-oxo-3,5,6,7,8,9-hexahydro-2H-6,9-epimin-ocyclohepta[c]pyridine-10-carboxamide
(±)-*N*-(3,4-dichlorophenyl)-4-fluoro-6,7,8,9-tetrahydro-5H-6,9-epiminocyclohepta[c]pyridine-10-carboxamide
(±)-*N*-(4,5-dichloro-2-cyanophenyl)-1-fluoro-6,7,8,9-tetrahydro-5H-5,8-epiminocyclohepta[c]-pyridine-10-carboxamide
(±)-3-bromo-*N*-(3,4-dichlorophenyl)-6,7,8,9-tetrahydro-5H-6,9-epiminocyclohepta[c]pyridine-10-carboxamide
(±)-3-chloro-*N*-(3,4-dichlorophenyl)-6,7,8,9-tetrahydro-5H-6,9-epiminocyclohepta[c]pyridine-10-carboxamide
(±)-*N*-(5-chloro-2-fluoro-4-(trifluoromethyl)phenyl)-1-fluoro-6,7,8,9-tetrahydro-5H-5,8-epiminocyclohepta[c]pyridine-10-carboxamide
(±)-*N*-(4-chloro-5-(trifluoromethyl)pyridin-2-yl)-1-fluoro-6,7,8,9-tetrahydro-5H-5,8-epimino-cyclohepta[c]pyridine-10-carboxamide
(±)-*N*-(3,4-dichlorophenyl)-3-oxo-3,5,6,7,8,9-hexahydro-2H-6,9-epiminocyclohepta[c]-pyridine-10-carboxamide
(±)-*N*-(3,4-dichlorophenyl)-6,7,8,9-tetrahydro-5H-6,9-epiminocyclohepta[c]pyridine-10-carboxamide
(±)-*N*-(3,4-dichlorophenyl)-2-oxo-3,5,6,7,8,9-hexahydro-2H-5,8-epiminocyclohepta[d]-pyrimidine-10-carboxamide
(±)-*N*-(3,4-dichlorophenyl)-2-oxo-3,5,6,7,8,9-hexahydro-2H-5,8-epiminocyclohepta[d]-pyrimidine-10-carboxamide
(±)-*N*-(3,4-dichlorophenyl)-6,7,8,9-tetrahydro-5H-5,8-epiminocyclohepta[c]pyridine-10-carboxamide
(±)-*N*-(3,4-dichlorophenyl)-6,7,8,9-tetrahydro-5H-5,8-epiminocyclohepta[c]pyridine-10-carboxamide
(±)-*N*-(3,4-dichlorophenyl)-1-fluoro-6,7,8,9-tetrahydro-5H-5,8-epiminocyclohepta[c]pyridine-10-carboxamide
(±)-*N*-(3,4-dichlorophenyl)-1-fluoro-6,7,8,9-tetrahydro-5H-5,8-epiminocyclohepta[c]pyridine-10-carboxamide
(±)-*N*-(3,4-dichlorophenyl)-6,7,8,9-tetrahydro-5H-6,9-epiminocyclohepta[c]pyridine-10-carboxamide
(±)-*N*-(3,4-dichlorophenyl)-6,7,8,9-tetrahydro-5H-6,9-epiminocyclohepta[c]pyridine-10-carboxamide
(±)-*N*-(3,4-dichlorophenyl)-9-fluoro-6,7,8,9-tetrahydro-5H-5,8-epiminocyclohepta[d]-pyrimidine-10-carboxamide
   (±)-*N*-(3-cyano-4-(trifluoromethyl)phenyl)-1-fluoro-6,7,8,9-tetrahydro-5H-5,8-epiminocyclo-hepta[c]pyridine-10-carboxamide
   (±)-1-fluoro-*N*-(3-methoxy-4-(oxazol-5-yl)phenyl)-6,7,8,9-tetrahydro-5H-5,8-epiminocyclo-hepta[c]pyridine-10-carboxamide
   (±)-*N*-(3-bromo-4-(trifluoromethyl)phenyl)-1-fluoro-6,7,8,9-tetrahydro-5H-5,8-epiminocyclohepta[c]pyridine-10-carboxamide
   (±)-*N*-(3-chloro-2,4-difluorophenyl)-1-fluoro-6,7,8,9-tetrahydro-5H-5,8-epiminocyclo-hepta[c]pyridine-10-carboxamide
   (±)-1-fluoro-*N*-(3-fluorophenyl)-6,7,8,9-tetrahydro-5H-5,8-epiminocyclohepta[c]pyridine-10-carboxamide
   (±)-1-fluoro-*N*-(3-(trifluoromethyl)phenyl)-6,7,8,9-tetrahydro-5H-5,8-epiminocyclohepta[c]-pyridine-10-carboxamide
   (±)-1-fluoro-*N*-(4-(trifluoromethoxy)phenyl)-6,7,8,9-tetrahydro-5H-5,8-epiminocyclo-hepta[c]pyridine-10-carboxamide
   (±)-*N*-([1,1'-biphenyl]-4-yl)-1-fluoro-6,7,8,9-tetrahydro-5H-5,8-epiminocyclohepta[c]-pyridine-10-carboxamide
   (±)-*N*-(3,5-bis(trifluoromethyl)phenyl)-1-fluoro-6,7,8,9-tetrahydro-5H-5,8-epiminocyclo-hepta[c]pyridine-10-carboxamide
   (±)-1-fluoro-*N*-(4-methyl-3-(trifluoromethyl)phenyl)-6,7,8,9-tetrahydro-5H-5,8-epiminocyclohepta[c]pyridine-10-carboxamide
   (±)-*N*-(2,3-dichlorophenyl)-1-fluoro-6,7,8,9-tetrahydro-5H-5,8-epiminocyclohepta[c]-pyridine-10-carboxamide
   (±)-*N*-(2,4-difluorophenyl)-1-fluoro-6,7,8,9-tetrahydro-5H-5,8-epiminocyclohepta[c]-pyridine-10-carboxamide
   (±)-*N*-(4-cyano-3-(trifluoromethyl)phenyl)-1-fluoro-6,7,8,9-tetrahydro-5H-5,8-epiminocyclo-hepta[c]pyridine-10-carboxamide
   (±)-*N*-(3-chloro-5-(trifluoromethyl)phenyl)-1-fluoro-6,7,8,9-tetrahydro-5H-5,8-epiminocyclo-hepta[c]pyridine-10-carboxamide
   (±)-*N*-(3-cyano-4-fluorophenyl)-1-fluoro-6,7,8,9-tetrahydro-5H-5,8-epiminocyclo-hepta[c]pyridine-10-carboxamide
   (±)-1-fluoro-*N*-(3,4,5-trichlorophenyl)-6,7,8,9-tetrahydro-5H-5,8-epiminocyclohepta[c]-pyridine-10-carboxamide
   (±)-1-fluoro-*N*-(3-methyl-4-(trifluoromethyl)phenyl)-6,7,8,9-tetrahydro-5H-5,8-epiminocyclohepta[c]pyridine-10-carboxamide
   (±)-*N*-(4-bromophenyl)-1-fluoro-6,7,8,9-tetrahydro-5H-5,8-epiminocyclohepta[c]pyridine-10-carboxamide
   (±)-*N*-(4-(difluoromethoxy)phenyl)-1-fluoro-6,7,8,9-tetrahydro-5H-5,8-epiminocyclo-hepta[c]pyridine-10-carboxamide
   (±)-*N*-(3-bromophenyl)-1-fluoro-6,7,8,9-tetrahydro-5H-5,8-epiminocyclohepta[c]pyridine-10-carboxamide
   (±)-1-fluoro-*N*-(3-(trifluoromethoxy)phenyl)-6,7,8,9-tetrahydro-5H-5,8-epiminocyclo-hepta[c]pyridine-10-carboxamide
   (±)-*N*-(3-bromo-4-fluorophenyl)-1-fluoro-6,7,8,9-tetrahydro-5H-5,8-epiminocyclo-hepta[c]pyridine-10-carboxamide
   (±)-*N*-(3,5-difluorophenyl)-1-fluoro-6,7,8,9-tetrahydro-5H-5,8-epiminocyclohepta[c]-pyridine-10-carboxamide
   (±)-*N*-(5-chloro-2-fluorophenyl)-1-fluoro-6,7,8,9-tetrahydro-5H-5,8-epiminocyclohepta[c]-pyridine-10-carboxamide
   (±)-1-fluoro-*N*-(2,3,4-trifluorophenyl)-6,7,8,9-tetrahydro-5H-5,8-epiminocyclohepta[c]-pyridine-10-carboxamide
   (±)-*N*-(4-cyanophenyl)-1-fluoro-6,7,8,9-tetrahydro-5H-5,8-epiminocyclohepta[c]pyridine-10-carboxamide
   (±)-*N*-(4-chloro-2-fluorophenyl)-1-fluoro-6,7,8,9-tetrahydro-5H-5,8-epiminocyclohepta[c]-pyridine-10-carboxamide
   (±)-*N*-(3-chloro-4-fluorophenyl)-1-fluoro-6,7,8,9-tetrahydro-5H-5,8-epiminocyclohepta[c]-pyridine-10-carboxamide
   (±)-*N*-(4-acrylamido-3-chlorophenyl)-1-fluoro-6,7,8,9-tetrahydro-5H-5,8-epiminocyclo-hepta[c]pyridine-10-carboxamide
   (±)-*N*-(5-bromo-2-fluoro-4-(trifluoromethyl)phenyl)-1-fluoro-6,7,8,9-tetrahydro-5H-5,8-epiminocyclohepta[c]pyridine-10-carboxamide
   (±)-*N*-(4,5-dichloro-2-fluorophenyl)-2-fluoro-6,7,8,9-tetrahydro-5H-5,8-epiminocyclo-hepta[b]pyridine-10-carboxamide
   (±)-*N*-(5-chloro-2-fluoro-4-(trifluoromethyl)phenyl)-2-fluoro-6,7,8,9-tetrahydro-5H-5,8-epiminocyclohepta[b]pyridine-10-carboxamide
   (±)-*N*-(4-(1H-pyrazol-1-yl)phenyl)-1-fluoro-6,7,8,9-tetrahydro-5H-5,8-epiminocyclo-hepta[c]pyridine-10-carboxamide
   (±)-*N*-([1,1'-biphenyl]-3-yl)-1-fluoro-6,7,8,9-tetrahydro-5H-5,8-epiminocyclohepta[c]-pyridine-10-carboxamide
   (±)-1-fluoro-*N*-(4-phenoxyphenyl)-6,7,8,9-tetrahydro-5H-5,8-epiminocyclohepta[c]pyridine-10-carboxamide
   (±)-1-fluoro-*N*-(4-(thiazol-4-yl)phenyl)-6,7,8,9-tetrahydro-5H-5,8-epiminocyclohepta[c]-pyridine-10-carboxamide
   (±)-1-fluoro-*N*-(4-(4-methyloxazol-5-yl)phenyl)-6,7,8,9-tetrahydro-5H-5,8-epiminocyclo-hepta[c]pyridine-10-carboxamide
   (±)-1-fluoro-*N*-(4-(oxazol-5-yl)phenyl)-6,7,8,9-tetrahydro-5H-5,8-epiminocyclohepta[c]-pyridine-10-carboxamide
   (±)-*N*-(5-chloro-2-fluoro-4-(trifluoromethyl)phenyl)-2-oxo-2,5,6,7,8,9-hexahydro-1H-5,8-epiminocyclohepta[b]pyridine-10-carboxamide
   (±)-*N*-(4,5-dichloro-2-fluorophenyl)-4-fluoro-6,7,8,9-tetrahydro-5H-5,8-epiminocyclo-hepta[c]pyridine-10-carboxamide
   (±)-*N*-([1,1'-biphenyl]-4-yl)-3-oxo-3,5,6,7,8,9-hexahydro-2H-6,9-epiminocyclo-hepta[c]pyridine-10-carboxamide
   (±)-*N*-([1,1'-biphenyl]-4-yl)-1-fluoro-6,7,8,9-tetrahydro-5H-5,8-epiminocyclo-hepta[c]pyridine-10-carboxamide
   (±)-*N*-(4,5-dichloro-2-fluorophenyl)-6,7,8,9-tetrahydro-5H-5,8-epiminocyclohepta[d]-pyridazine-10-carboxamide
   (±)-*N*-(5-chloro-2-fluoro-4-(trifluoromethyl)phenyl)-6,7,8,9-tetrahydro-5H-5,8-epiminocyclohepta[d]pyridazine-10-carboxamide
   (±)-1,4-dichloro-*N*-(5-chloro-2-fluoro-4-(trifluoromethyl)phenyl)-6,7,8,9-tetrahydro-5H-5,8-epiminocyclohepta[d]pyridazine-10-carboxamide
   (±)-*N*-(5-chloro-2-fluoro-4-(trifluoromethyl)phenyl)-2-fluoro-6,7,8,9-tetrahydro-5H-5,8-epiminocyclohepta[b]pyridine-10-carboxamide
   (±)-*N*-(5-chloro-2-fluoro-4-(trifluoromethyl)phenyl)-2-fluoro-6,7,8,9-tetrahydro-5H-5,8-epiminocyclohepta[b]pyridine-10-carboxamide
   (±)-*N*-(3,4-dichlorophenyl)-3-fluoro-6,7,8,9-tetrahydro-5H-6,9-epiminocyclohepta[c]-pyridine-10-carboxamide
   (±)-*N*-(4-chloro-3-cyanophenyl)-1-fluoro-6,7,8,9-tetrahydro-5H-5,8-epiminocyclohepta[c]-pyridine-10-carboxamide
   (±)-*N*-(3-cyano-4-(trifluoromethyl)phenyl)-1-fluoro-6,7,8,9-tetrahydro-5H-5,8-epiminocyclo-hepta[c]pyridine-10-carboxamide
   (±)-1-fluoro-*N*-(3-methoxy-4-(oxazol-5-yl)phenyl)-6,7,8,9-tetrahydro-5H-5,8-epiminocyclo-hepta[c]pyridine-10-carboxamide
   (±)-*N*-(3-bromo-4-(trifluoromethyl)phenyl)-1-fluoro-6,7,8,9-tetrahydro-5H-5,8-epiminocyclohepta[c]pyridine-10-carboxamide
   (±)-*N*-(3-chloro-2,4-difluorophenyl)-1-fluoro-6,7,8,9-tetrahydro-5H-5,8-epiminocyclo-hepta[c]pyridine-10-carboxamide
   (±)-1-fluoro-*N*-(3-fluorophenyl)-6,7,8,9-tetrahydro-5H-5,8-epiminocyclohepta[c]pyridine-10-carboxamide
   (±)-1-fluoro-*N*-(3-(trifluoromethyl)phenyl)-6,7,8,9-tetrahydro-5H-5,8-epiminocyclo-hepta[c]pyridine-10-carboxamide
   (±)-1-fluoro-*N*-(4-(trifluoromethoxy)phenyl)-6,7,8,9-tetrahydro-5H-5,8-epiminocyclo-hepta[c]pyridine-10-carboxamide
   (±)-*N*-([1,1'-biphenyl]-4-yl)-1-fluoro-6,7,8,9-tetrahydro-5H-5,8-epiminocyclohepta[c]-pyridine-10-carboxamide
   (±)-*N*-(3,5-bis(trifluoromethyl)phenyl)-1-fluoro-6,7,8,9-tetrahydro-5H-5,8-epiminocyclo-hepta[c]pyridine-10-carboxamide
   (±)-1-fluoro-*N*-(4-methyl-3-(trifluoromethyl)phenyl)-6,7,8,9-tetrahydro-5H-5,8-epiminocyclohepta[c]pyridine-10-carboxamide
   (±)-*N*-(2,3-dichlorophenyl)-1-fluoro-6,7,8,9-tetrahydro-5H-5,8-epiminocyclohepta[c]-pyridine-10-carboxamide
   (±)-*N*-(2,4-difluorophenyl)-1-fluoro-6,7,8,9-tetrahydro-5H-5,8-epiminocyclohepta[c]-pyridine-10-carboxamide
   (±)-*N*-(4-cyano-3-(trifluoromethyl)phenyl)-1-fluoro-6,7,8,9-tetrahydro-5H-5,8-epiminocyclohepta[c]pyridine-10-carboxamide
   (±)-*N*-(3-chloro-5-(trifluoromethyl)phenyl)-1-fluoro-6,7,8,9-tetrahydro-5H-5,8-epiminocyclohepta[c]pyridine-10-carboxamide
   (±)-*N*-(3-cyano-4-fluorophenyl)-1-fluoro-6,7,8,9-tetrahydro-5H-5,8-epiminocyclohepta[c]-pyridine-10-carboxamide
   (±)-1-fluoro-*N*-(3,4,5-trichlorophenyl)-6,7,8,9-tetrahydro-5H-5,8-epiminocyclohepta[c]-pyridine-10-carboxamide
   (±)-1-fluoro-*N*-(3-methyl-4-(trifluoromethyl)phenyl)-6,7,8,9-tetrahydro-5H-5,8-epiminocyclohepta[c]pyridine-10-carboxamide
   (±)-*N*-(4-bromophenyl)-1-fluoro-6,7,8,9-tetrahydro-5H-5,8-epiminocyclohepta[c]pyridine-10-carboxamide
   (±)-*N*-(4-(difluoromethoxy)phenyl)-1-fluoro-6,7,8,9-tetrahydro-5H-5,8-epiminocyclo-hepta[c]pyridine-10-carboxamide
   (±)-*N*-(3-bromophenyl)-1-fluoro-6,7,8,9-tetrahydro-5H-5,8-epiminocyclohepta[c]pyridine-10-carboxamide
   (±)-1-fluoro-*N*-(3-(trifluoromethoxy)phenyl)-6,7,8,9-tetrahydro-5H-5,8-epiminocyclo-hepta[c]pyridine-10-carboxamide
   (±)-*N*-(3-bromo-4-fluorophenyl)-1-fluoro-6,7,8,9-tetrahydro-5H-5,8-epiminocyclo-hepta[c]pyridine-10-carboxamide
   (±)-*N*-(3,5-difluorophenyl)-1-fluoro-6,7,8,9-tetrahydro-5H-5,8-epiminocyclo-hepta[c]pyridine-10-carboxamide
   (±)-*N*-(5-chloro-2-fluorophenyl)-1-fluoro-6,7,8,9-tetrahydro-5H-5,8-epiminocyclo-hepta[c]pyridine-10-carboxamide
   (±)-1-fluoro-*N*-(2,3,4-trifluorophenyl)-6,7,8,9-tetrahydro-5H-5,8-epiminocyclo-hepta[c]pyridine-10-carboxamide
   (±)-*N*-(4-cyanophenyl)-1-fluoro-6,7,8,9-tetrahydro-5H-5,8-epiminocyclohepta[c]pyridine-10-carboxamide
   (±)-*N*-(4-chloro-2-fluorophenyl)-1-fluoro-6,7,8,9-tetrahydro-5H-5,8-epiminocyclohepta[c]-pyridine-10-carboxamide
   (±)-*N*-(3-chloro-4-fluorophenyl)-1-fluoro-6,7,8,9-tetrahydro-5H-5,8-epiminocyclohepta[c]-pyridine-10-carboxamide
   (±)-*N*-(4-acrylamido-3-chlorophenyl)-1-fluoro-6,7,8,9-tetrahydro-5H-5,8-epiminocyclo-hepta[c]pyridine-10-carboxamide
   (±)-*N*-(5-bromo-2-fluoro-4-(trifluoromethyl)phenyl)-1-fluoro-6,7,8,9-tetrahydro-5H-5,8-epiminocyclohepta[c]pyridine-10-carboxamide
   (±)-*N*-(4,5-dichloro-2-fluorophenyl)-2-fluoro-6,7,8,9-tetrahydro-5H-5,8-epiminocyclohepta[b]pyridine-10-carboxamide
   (±)-*N*-(5-chloro-2-fluoro-4-(trifluoromethyl)phenyl)-2-fluoro-6,7,8,9-tetrahydro-5H-5,8-epiminocyclohepta[b]pyridine-10-carboxamide
   (±)-*N*-(4-(1H-pyrazol-1-yl)phenyl)-1-fluoro-6,7,8,9-tetrahydro-5H-5,8-epiminocyclo-hepta[c]pyridine-10-carboxamide
   (±)-*N*-([1,1'-biphenyl]-3-yl)-1-fluoro-6,7,8,9-tetrahydro-5H-5,8-epiminocyclohepta[c]-pyridine-10-carboxamide
   (±)-1-fluoro-*N*-(4-phenoxyphenyl)-6,7,8,9-tetrahydro-5H-5,8-epiminocyclohepta[c]pyridine-10-carboxamide
   (±)-1-fluoro-*N*-(4-(thiazol-4-yl)phenyl)-6,7,8,9-tetrahydro-5H-5,8-epiminocyclohepta[c]-pyridine-10-carboxamide
   (±)-1-fluoro-*N*-(4-(4-methyloxazol-5-yl)phenyl)-6,7,8,9-tetrahydro-5H-5,8-epiminocyclo-hepta[c]pyridine-10-carboxamide
   (±)-1-fluoro-*N*-(4-(oxazol-5-yl)phenyl)-6,7,8,9-tetrahydro-5H-5,8-epiminocyclohepta[c]-pyridine-10-carboxamide
   (±)-*N*-(5-chloro-2-fluoro-4-(trifluoromethyl)phenyl)-2-oxo-2,5,6,7,8,9-hexahydro-1H-5,8-epiminocyclohepta[b]pyridine-10-carboxamide
   (±)-*N*-(4,5-dichloro-2-fluorophenyl)-4-fluoro-6,7,8,9-tetrahydro-5H-5,8-epiminocyclo-hepta[c]pyridine-10-carboxamide
   (±)-*N*-([1,1'-biphenyl]-4-yl)-3-oxo-3,5,6,7,8,9-hexahydro-2H-6,9-epiminocyclo-hepta[c]pyridine-10-carboxamide
   (±)-*N*-([1,1'-biphenyl]-4-yl)-1-fluoro-6,7,8,9-tetrahydro-5H-5,8-epiminocyclohepta[c]-pyridine-10-carboxamide
   (±)-*N*-(4,5-dichloro-2-fluorophenyl)-6,7,8,9-tetrahydro-5H-5,8-epiminocyclohepta[d]-pyridazine-10-carboxamide
   (±)-*N*-(5-chloro-2-fluoro-4-(trifluoromethyl)phenyl)-6,7,8,9-tetrahydro-5H-5,8-epiminocyclohepta[d]pyridazine-10-carboxamide
   (±)-1,4-dichloro-*N*-(5-chloro-2-fluoro-4-(trifluoromethyl)phenyl)-6,7,8,9-tetrahydro-5H-5,8-epiminocyclohepta[d]pyridazine-10-carboxamide
   (±)-*N*-(5-chloro-2-fluoro-4-(trifluoromethyl)phenyl)-2-fluoro-6,7,8,9-tetrahydro-5H-5,8-epiminocyclohepta[b]pyridine-10-carboxamide
   (±)-*N*-(5-chloro-2-fluoro-4-(trifluoromethyl)phenyl)-2-fluoro-6,7,8,9-tetrahydro-5H-5,8-epiminocyclohepta[b]pyridine-10-carboxamide
   (±)-*N*-(3,4-dichlorophenyl)-3-fluoro-6,7,8,9-tetrahydro-5H-6,9-epiminocyclohepta[c]-pyridine-10-carboxamide
   (±)-*N*-(4-chloro-3-cyanophenyl)-1-fluoro-6,7,8,9-tetrahydro-5H-5,8-epiminocyclohepta[c]-pyridine-10-carboxamide
   (±)-*N*-(4,5-dichloro-2-fluorophenyl)-1-(trifluoromethyl)-6,7,8,9-tetrahydro-5H-5,8-epiminocyclohepta[c]pyridine-10-carboxamide
   (±)-*N*-(3-Chloro-4-(trifluoromethyl)phenyl)-9-(hydroxyimino)-2-oxo-3,5,6,7,8,9-hexahydro-2H-5,8-epiminocyclohepta[d]pyrimidine-10-carboxamide
   *cis*-(±)-*N*-(3,4-Dichlorophenyl)-9-fluoro-6,7,8,9-tetrahydro-5H-5,8-epiminocyclo-hepta[d]pyrimidine-10-carboxamide
   *trans*-(±)-*N*-(3,4-Dichlorophenyl)-9-fluoro-6,7,8,9-tetrahydro-5H-5,8-epiminocyclohepta-[d]pyrimidine-10-carboxamide
   (±)-*N*-(3-chloro-4-(trifluoromethyl)phenyl)-8-methyl-6,7,8,9-tetrahydro-5H-5,8-epiminocyclohepta[c]pyridine-10-carboxamide
   (±)-*N*-(4,5-dichloro-2-methylphenyl)-1-fluoro-6,7,8,9-tetrahydro-5H-5,8-epiminocyclo-hepta[c]pyridine-10-carboxamide
   (±)-*N* -(4,5-dichloro-2-methoxyphenyl)-1-fluoro-6,7,8,9-tetrahydro-5H-5,8-epimino-cyclohepta[c]pyridine-10-carboxamide
   (±)-1-fluoro-*N*-(2-fluoro-5-(trifluoromethyl)phenyl)-6,7,8,9-tetrahydro-5H-5,8-epimino-cyclohepta[c]pyridine-10-carboxamide
   (±)-1-fluoro-*N*-(2-fluoro-4-(trifluoromethyl)phenyl)-6,7,8,9-tetrahydro-5H-5,8-epimino-cyclohepta[c]pyridine-10-carboxamide
   (±)-1-fluoro-*N*-(4-(trifluoromethyl)phenyl)-6,7,8,9-tetrahydro-5H-5,8-epimino-cyclohepta[c]pyridine-10-carboxamide
   (*5R,8S*)-*N*-(4-(trifluoromethyl)phenyl)-6,7,8,9-tetrahydro-5H-5,8-epiminocyclohepta-[d]pyrimidine-10-carboxamide
   (*5R,8S*)-*N*-(3-chloro-4-(trifluoromethyl)phenyl)-6,7,8,9-tetrahydro-5H-5,8-epiminocyclo-hepta[d]pyrimidine-10-carboxamide
   (*5R,8S*)-*N*-(4-chloro-3-(trifluoromethyl)phenyl)-6,7,8,9-tetrahydro-5H-5,8-epiminocyclo-hepta[d]pyrimidine-10-carboxamide
   (*5R*,*8S*)-*N*-(3,5-dichlorophenyl)-6,7,8,9-tetrahydro-5H-5,8-epiminocyclohepta[d]pyrimidine-10-carboxamide
   (*5R*,*8S*)-*N*-(4-fluoro-3-(trifluoromethyl)phenyl)-6,7,8,9-tetrahydro-5H-5,8-epimino-cyclohepta[d]pyrimidine-10-carboxamide
   (*5R*,*8S*)-*N*-(3,4-difluorophenyl)-6,7,8,9-tetrahydro-5H-5,8-epiminocyclohepta[d]pyrimidine-10-carboxamide
   (*5R*,*8S*)-*N*-(4-bromo-3-chlorophenyl)-6,7,8,9-tetrahydro-5H-5,8-epiminocyclohepta[d]-pyrimidine-10-carboxamide
   (*5R*,*8S*)-*N*-(3-chloro-4-methoxyphenyl)-6,7,8,9-tetrahydro-5H-5,8-epiminocyclohepta[d]-pyrimidine-10-carboxamide
   (*5R*,*8S*)-*N*-(3-chloro-4-cyanophenyl)-6,7,8,9-tetrahydro-5H-5,8-epiminocyclohepta[d]=pyrimidine-10-carboxamide
   (*5R*,*8S*)-*N*-(3-bromo-4-chlorophenyl)-6,7,8,9-tetrahydro-5H-5,8-epiminocyclohepta[d]-pyrimidine-10-carboxamide
   (*5R*,*8S*)-*N*-(3-bromo-4-cyanophenyl)-6,7,8,9-tetrahydro-5H-5,8-epiminocyclohepta[d]-pyrimidine-10-carboxamide
   (*5R,8S*)-*N*-(3-bromo-4-(trifluoromethyl)phenyl)-6,7,8,9-tetrahydro-5H-5,8-epimino-cyclohepta[d]pyrimidine-10-carboxamide
   (*5R*,*8S*)-*N*-(3,4-dibromophenyl)-6,7,8,9-tetrahydro-5H-5,8-epiminocyclohepta[d]pyrimidine-10-carboxamide
   (*6S*,*9R*)-*N*-(3,4-dichlorophenyl)-6,7,8,9-tetrahydro-5H-6,9-epiminocyclohepta[d]pyrimidine-10-carboxamide
   (*6S,9R*)-*N*-(3-chloro-4-(trifluoromethyl)phenyl)-6,7,8,9-tetrahydro-5H-6,9-epimino-cyclohepta[d]pyrimidine-10-carboxamide
   (*5R*,*8S*)-*N*-(3,4-dichlorophenyl)-2-(trifluoromethyl)-6,7,8,9-tetrahydro-5H-5,8-epimino-cyclohepta[d]pyrimidine-10-carboxamide
   (*5R*,*8S*)-*N*-(3,4-dichlorophenyl)-2-methyl-6,7,8,9-tetrahydro-5H-5,8-epiminocyclohepta-[d]pyrimidine-10-carboxamide
   (*5R*,*8S*)-*N*-(3,4-dichlorophenyl)-2-phenyl-6,7,8,9-tetrahydro-5H-5,8-epiminocyclohepta[d]-pyrimidine-10-carboxamide
   (*5R,8S*)*-N-(3-chloro-4-(trifluoromethyl)phenyl)-2-(trifluoromethyl)-6,7,8,9-tetrahydro-5H-*5,8-epiminocyclohepta[d]pyrimidine-10-carboxamide
   (*5R,8S*)*-N-(4-chloro-3-(trifluoromethyl)phenyl)-2-phenyl-6,7,8,9-tetrahydro-5H-5,8-*epiminocyclohepta[d]pyrimidine-10-carboxamide
   (*5R,8S*)*-N-(3-chloro-4-(trifluoromethyl)phenyl)-2-phenyl-6,7,8,9-tetrahydro-5H-5,8-*epiminocyclohepta[d]pyrimidine-10-carboxamide
   (*5R*,*8S*)-*N*-(3,4-dichlorophenyl)-2-hydroxy-6,7,8,9-tetrahydro-5H-5,8-epiminocyclohepta[d]-pyrimidine-10-carboxamide
   (*5R,8S*)*-N-(3-chloro-4-(trifluoromethyl)phenyl)-2-hydroxy-6,7,8,9-tetrahydro-5H-5,8-*epiminocyclohepta[d]pyrimidine-10-carboxamide
   (*5R*,*8S*)-*N*-(3,4-dichlorophenyl)-6,7,8,9-tetrahydro-5H-5,8-epiminobenzo[7]annulene-10-carboxamide
   (*5R,8S*)-*N*-(3-chloro-4-(trifluoromethyl)phenyl)-6,7,8,9-tetrahydro-5H-5,8-epimin-ocyclohepta[c]pyridine-10-carboxamide
   (*5R*,*8S*)-*N*-(3,4-dichlorophenyl)-6,7,8,9-tetrahydro-5H-5,8-epiminocyclohepta[c]pyridine-10-carboxamide
   (*6S,9R*)-*N*-(3-chloro-4-(trifluoromethyl)phenyl)-6,7,8,9-tetrahydro-5H-6,9-epiminocyclo-hepta-[c]pyridine-10-carboxamide
   (*5R,8S*)-*N*-(3-chloro-4-(trifluoromethyl)phenyl)-6,7,8,9-tetrahydro-5H-5,8-epiminocyclo-hepta-[d]pyrimidine-10-carboxamide
   (*5S,8R*)-*N*-(3-chloro-4-(trifluoromethyl)phenyl)-6,7,8,9-tetrahydro-5H-5,8-epiminocyclo-hepta-[d]pyrimidine-10-carboxamide
   (*5R,8S*)-*N*-(4-chloro-3-(trifluoromethyl)phenyl)-6,7,8,9-tetrahydro-5H-5,8-epiminocyclo-hepta-[d]pyrimidine-10-carboxamide
   *(5R,8S)-N-(3-chloro-4-(trifluoromethyl)phenyl)-2-oxo-3,5,6,7,8,9-hexahydro-2H-5,8-*epiminocyclohepta[d]pyrimidine-10-carboxamide
   *(5S, 8R)- N-(3-chl oro-4-(trifl uoromethyl) phenyl)-2 -oxo-3, 5,6,7,8, 9-hexahydro-2 H -5, 8-*epiminocyclohepta[d]pyrimidine-10-carboxamide
   *(5R,8S)-N-(3-chloro-4-(trifluoromethyl)phenyl)-2-fluoro-6,7,8,9-tetrahydro-5H-5,8-*epiminocyclohepta[d]pyrimidine-10-carboxamide
   (*5R*,*8S*)-*N*-(3,4-dichlorophenyl)-2-fluoro-6,7,8,9-tetrahydro-5H-5,8-epiminocyclohepta[d]-pyrimidine-10-carboxamide
   (*5R,8S,*E)-*N*-(3-chloro-4-(trifluoromethyl)phenyl)-2-hydroxy-9-(hydroxyimino)-6,7,8,9-tetrahydro-5H-5,8-epiminocyclohepta[d]pyrimidine-10-carboxamide
   (*5R*,*8S*)-*N*-(3,4-dichlorophenyl)-6,7,8,9-tetrahydro-5H-5,8-epiminocyclohepta[d]pyrimidine-10-carboxamide
   (*5R*,*8S*)-*N*-(4-chlorophenyl)-6,7,8,9-tetrahydro-5H-5,8-epiminocyclohepta[d]pyrimidine-10-carboxamide
   (*5R*,*8S*)-*N*-(3-chlorophenyl)-6,7,8,9-tetrahydro-5H-5,8-epiminocyclohepta[d]pyrimidine-10-carboxamide
   (*5R*,*8S*)-*N*-(5-chloro-2-fluorophenyl)-6,7,8,9-tetrahydro-5H-5,8-epiminocyclohepta[d]-pyrimidine-10-carboxamide
   (*5R*,*8S*)-*N*-(3,4-dichlorophenyl)-6,7,8,9-tetrahydro-5H-5,8-epiminocyclohepta[d]pyrimidine-10-carboxamide
   (*5S*,*8R*)-*N*-(3,4-dichlorophenyl)-6,7,8,9-tetrahydro-5H-5,8-epiminocyclohepta[d]pyrimidine-10-carboxamide
   (*5R,8S*)-*N*-(3-chloro-4-(trifluoromethyl)phenyl)-6,7,8,9-tetrahydro-5H-5,8-epiminobenzo[7]-annulene-10-carboxamide
   (*5R,8S*)-*N*-(4-chloro-3-(trifluoromethyl)phenyl)-6,7,8,9-tetrahydro-5H-5,8-epiminobenzo[7]-annulene-10-carboxamide
   (*5R*,*8S*)-*N*-(3,4-dichlorophenyl)-2-methoxy-6,7,8,9-tetrahydro-5H-5,8-epiminobenzo[7]-annulene-10-carboxamide
   (*5R*,*8S*)-*N*-(3,4-dichlorophenyl)-2-hydroxy-6,7,8,9-tetrahydro-5H-5,8-epiminobenzo[7]-annulene-10-carboxamide
   (*5R,8S*)-10-((3,4-dichlorophenyl)carbamoyl)-6,7,8,9-tetrahydro-5H-5,8-epiminocyclohepta-[c]pyridine 2-oxide
   (*6S*,*9R*)-10-((3,4-dichlorophenyl)carbamoyl)-6,7,8,9-tetrahydro-5H-6,9-epiminocyclohepta[c]-pyridine 2-oxide
   (*5R*,*8S*)-2-chloro-*N*-(3,4-dichlorophenyl)-6,7,8,9-tetrahydro-5H-5,8-epiminobenzo[7]-annulene-10-carboxamide
   (*6S*,*9R*)-1-amino-*N*-(3,4-dichlorophenyl)-6,7,8,9-tetrahydro-5H-6,9-epiminocyclohepta[c]-pyridine-10-carboxamide
   (*6S*,*9R*)-3-amino-*N*-(3,4-dichlorophenyl)-6,7,8,9-tetrahydro-5H-6,9-epiminocyclohepta[c]-pyridine-10-carboxamide
   (*5R*,*8S*)-1-amino-*N*-(3,4-dichlorophenyl)-6,7,8,9-tetrahydro-5H-5,8-epiminocyclohepta[c]-pyridine-10-carboxamide
   (*5R*,*8S*)-3-amino-*N*-(3,4-dichlorophenyl)-6,7,8,9-tetrahydro-5H-5,8-epiminocyclohepta[c]-pyridine-10-carboxamide
   (*5R*,*8S*)-1-(tert-butylamino)-*N*-(3,4-dichlorophenyl)-6,7,8,9-tetrahydro-5H-5,8-epimino-cyclohepta[c]pyridine-10-carboxamide
   (*5R*,*8S*)-3-(tert-butylamino)-*N*-(3,4-dichlorophenyl)-6,7,8,9-tetrahydro-5H-5,8-epimino-cyclohepta[c]pyridine-10-carboxamide
   (*6S*,*9R*)-*N*-(3,4-dichlorophenyl)-3-fluoro-6,7,8,9-tetrahydro-5H-6,9-epiminocyclohepta[c-]pyridine-10-carboxamide
   (*6S*,*9R*)-*N*-(3,4-dichlorophenyl)-1-fluoro-6,7,8,9-tetrahydro-5H-6,9-epiminocyclohepta[c]-pyridine-10-carboxamide
   (*5R*,*8S*)-*N*-(3,4-dichlorophenyl)-3-fluoro-6,7,8,9-tetrahydro-5H-5,8-epiminocyclohepta[c]-pyridine-10-carboxamide
   (*5R*,*8S*)-2-chloro-*N*-(3-chloro-4-(trifluoromethyl)phenyl)-6,7,8,9-tetrahydro-5H-5,8-epimino-benzo[7]annulene-10-carboxamide
   (*5R*,*8S*)-2-cyano-*N*-(3,4-dichlorophenyl)-6,7,8,9-tetrahydro-5H-5,8-epiminobenzo[7]-annulene-10-carboxamide
   (*5R*,*8S*)-2-amino-*N*-(3,4-dichlorophenyl)-6,7,8,9-tetrahydro-5H-5,8-epiminobenzo[7]-annulene-10-carboxamide
   (*5R*,*8S*)-*N*-(3,4-dichlorophenyl)-2-fluoro-6,7,8,9-tetrahydro-5H-5,8-epiminobenzo[7]-annulene-10-carboxamide
   (*5R,8S*)-*N*-(3-chloro-4-(trifluoromethyl)phenyl)-2-fluoro-6,7,8,9-tetrahydro-5H-5,8-epimino-benzo[7]annulene-10-carboxamide
   (*6S*,*9R*)-*N*-(3,4-dichlorophenyl)-3-oxo-3,5,6,7,8,9-hexahydro-2H-6,9-epiminocyclohepta-[c]pyridine-10-carboxamide
   (*5R*,*8S*)-1-chloro-*N*-(3,4-dichlorophenyl)-6,7,8,9-tetrahydro-5H-5,8-epiminocyclohepta-[c]pyridine-10-carboxamide
   (*5R*,*8S*)-*N*-(3,4-dichlorophenyl)-1-fluoro-6,7,8,9-tetrahydro-5H-5,8-epiminocyclohepta[c]-pyridine-10-carboxamide
   (*5R*,*8S*)-*N*-(4,5-dichloro-2-fluorophenyl)-1-fluoro-6,7,8,9-tetrahydro-5H-5,8-epimino-cyclohepta[c]pyridine-10-carboxamide
   (*5R*,*8S*)-*N*-(3,4-dichlorophenyl)-3-fluoro-6,7,8,9-tetrahydro-5H-5,8-epiminobenzo[7]-annulene-10-carboxamide
   (*5R,8S*)-*N*-(3-chloro-4-(trifluoromethyl)phenyl)-3-fluoro-6,7,8,9-tetrahydro-5H-5,8-epimino-benzo[7]annulene-10-carboxamide
   tert-butyl ((*6S*,*9R*)-10-((3,4-dichlorophenyl)carbamoyl)-6,7,8,9-tetrahydro-5H-6,9-epimino-cyclohepta[c]pyridin-1-yl)carbamate
   (*5R*,*8S*)-*N*-(3,4-dichlorophenyl)-1-fluoro-6,7,8,9-tetrahydro-5H-5,8-epiminobenzo[7]-annulene-10-carboxamide
   *(5R, 8S)-N-(3-chloro-4-(trifluoromethyl)phenyl)-1-fluoro-6,7,8,9-tetrahydro-5H-5,8-*epiminobenzo[7]annulene-10-carboxamide
   *(5R, 8S)-N-(3-chloro-4-(trifluoromethyl)phenyl)-1-fluoro-6,7,8,9-tetrahydro-5H-5,8-*epiminocyclohepta[c]pyridine-10-carboxamide
   (*6S*,*9R*)-*N*-(3,4-dichlorophenyl)-3-methoxy-6,7,8,9-tetrahydro-5H-6,9-epiminocyclohepta[c]-pyridine-10-carboxamide
   (*5R*,*8S*)-*N*-(3,4-dichlorophenyl)-6,7,8,9-tetrahydro-5H-5,8-epiminocyclohepta[b]pyridine-10-carboxamide
   (*6S*,*9R*)-*N*-(4,5-dichloro-2-cyanophenyl)-3-oxo-3,5,6,7,8,9-hexahydro-2H-6,9-epimino-cyclohepta[c]pyridine-10-carboxamide
   (*6S*,*9R*)-*N*-(3,4-dichlorophenyl)-3-fluoro-6,7,8,9-tetrahydro-5H-6,9-epiminocyclohepta[c]-pyridine-10-carboxamide
   (5*R*,8*S*)-10-((3,4-dichlorophenyl)carbamoyl)-6,7,8,9-tetrahydro-5H-5,8-epiminocyclohepta[c]-pyridine 2-oxide
   *(6S,9R*)*-N-*(5-chloro-2-fluoro-4-(trifluoromethyl)phenyl)-3-oxo-3,5,6,7,8,9-hexahydro-2H-6,9-epiminocyclohepta[c]pyridine-10-carboxamide
   (*6S*,*9R*)-*N*-(4,5-dichloro-2-fluorophenyl)-3-oxo-3,5,6,7,8,9-hexahydro-2H-6,9-epimino-cyclohepta[c]pyridine-10-carboxamide
   (*6S*,*9R*)-*N*-(3,4-dichlorophenyl)-4-fluoro-6,7,8,9-tetrahydro-5H-6,9-epiminocyclohepta[c]-pyridine-10-carboxamide
   (5*R*,8*S*)-*N*-(4,5-dichloro-2-cyanophenyl)-1-fluoro-6,7,8,9-tetrahydro-5H-5,8-epimino-cyclohepta[c]pyridine-10-carboxamide
   (6*S*,9*R*)-3-bromo-*N*-(3,4-dichlorophenyl)-6,7,8,9-tetrahydro-5H-6,9-epiminocyclohepta[c]-pyridine-10-carboxamide
   (6*S*,9*R*)-3-chloro-*N*-(3,4-dichlorophenyl)-6,7,8,9-tetrahydro-5H-6,9-epiminocyclohepta[c]-pyridine-10-carboxamide
   *(5R,8S)-N-(5-chloro-2-fluoro-4-(trifluoromethyl)phenyl)-1-fluoro-6,7,8,9-tetrahydro-5H-5,8-*epiminocyclohepta[c]pyridine-10-carboxamide
   (5*R*,8*S*)-*N*-(4-chloro-5-(trifluoromethyl)pyridin-2-yl)-1-fluoro-6,7,8,9-tetrahydro-5H-5,8-epiminocyclohepta[c]pyridine-10-carboxamide
   (*6S*,*9R*)-*N*-(3,4-dichlorophenyl)-3-oxo-3,5,6,7,8,9-hexahydro-2H-6,9-epiminocyclohepta[c]-pyridine-10-carboxamide
   (*6S*,*9R*)-*N*-(3,4-dichlorophenyl)-6,7,8,9-tetrahydro-5H-6,9-epiminocyclohepta[c]pyridine-10-carboxamide
   (*5S*,*8R*)-*N*-(3,4-dichlorophenyl)-2-oxo-3,5,6,7,8,9-hexahydro-2H-5,8-epiminocyclohepta[d]-pyrimidine-10-carboxamide
   (*5R*,*8S*)-*N*-(3,4-dichlorophenyl)-2-oxo-3,5,6,7,8,9-hexahydro-2H-5,8-epiminocyclohepta[d]-pyrimidine-10-carboxamide
   (5*S*,8*R*)-*N*-(3,4-dichlorophenyl)-6,7,8,9-tetrahydro-5H-5,8-epiminocyclohepta[c]pyridine-10-carboxamide
   (5*R*,8*S*)-*N*-(3,4-dichlorophenyl)-6,7,8,9-tetrahydro-5H-5,8-epiminocyclohepta[c]pyridine-10-carboxamide
   (5*R*,8*S*)-*N*-(3,4-dichlorophenyl)-1-fluoro-6,7,8,9-tetrahydro-5H-5,8-epiminocyclohepta[c]-pyridine-10-carboxamide
   (5*S*,8*R*)-*N*-(3,4-dichlorophenyl)-1-fluoro-6,7,8,9-tetrahydro-5H-5,8-epiminocyclohepta[c]-pyridine-10-carboxamide
   (6*S*,9*R*)-*N*-(3,4-dichlorophenyl)-6,7,8,9-tetrahydro-5H-6,9-epiminocyclohepta[c]pyridine-10-carboxamide
   (6*R*,9*S*)-*N*-(3,4-dichlorophenyl)-6,7,8,9-tetrahydro-5H-6,9-epiminocyclohepta[c]pyridine-1 0-carboxamide
   (5*R*,8*S*,9*S*)-*N*-(3,4-dichlorophenyl)-9-fluoro-6,7,8,9-tetrahydro-5H-5,8-epiminocyclohepta[d]-pyrimidine-10-carboxamide
   (5*S*,8*R*)-*N*-(4-(trifluoromethyl)phenyl)-6,7,8,9-tetrahydro-5H-5,8-epiminocyclohepta[d]-pyrimidine-10-carboxamide
   (*5S*,*8R*)-*N*-(3-chloro-4-(trifluoromethyl)phenyl)-6,7,8,9-tetrahydro-5H-5,8-epimino-cyclohepta[d]pyrimidine-10-carboxamide
   (*5S,8R*)-*N*-(4-chloro-3-(trifluoromethyl)phenyl)-6, 7,8, 9-tetrahydro-5H-5, 8-epimino-cyclohepta[d]pyrimidine-10-carboxamide
   (*5S*,*8R*)-*N*-(3,5-dichlorophenyl)-6,7,8,9-tetrahydro-5H-5,8-epiminocyclohepta[d]pyrimidine-10-carboxamide
   (*5S*,*8R*)-*N*-(4-fluoro-3-(trifluoromethyl)phenyl)-6,7,8,9-tetrahydro-5H-5,8-epimino-cyclohepta[d]pyrimidine-10-carboxamide
   (*5S*,*8R*)-*N*-(3,4-difluorophenyl)-6,7,8,9-tetrahydro-5H-5,8-epiminocyclohepta[d]pyrimidine-10-carboxamide
   (*5S*,*8R*)-*N*-(4-bromo-3-chlorophenyl)-6,7,8,9-tetrahydro-5H-5,8-epiminocyclohepta[d]-pyrimidine-10-carboxamide
   (*5S*,*8R*)-*N*-(3-chloro-4-methoxyphenyl)-6,7,8,9-tetrahydro-5H-5,8-epiminocyclohepta[d]-pyrimidine-10-carboxamide
   (*5S*,*8R*)-*N*-(3-chloro-4-cyanophenyl)-6,7,8,9-tetrahydro-5H-5,8-epiminocyclohepta[d]-pyrimidine-10-carboxamide
   (*5S*,*8R*)-*N*-(3-bromo-4-chlorophenyl)-6,7,8,9-tetrahydro-5H-5,8-epiminocyclohepta[d]-pyrimidine-10-carboxamide
   (*5S*,*8R*)-*N*-(3-bromo-4-cyanophenyl)-6,7,8,9-tetrahydro-5H-5,8-epiminocyclohepta[d]-pyrimidine-10-carboxamide
   (*5S*,*8R*)-*N*-(3-bromo-4-(trifluoromethyl)phenyl)-6,7,8,9-tetrahydro-5H-5,8-epimino-cyclohepta[d]pyrimidine-10-carboxamide
   (*5S*,*8R*)-*N*-(3,4-dibromophenyl)-6,7,8,9-tetrahydro-5H-5,8-epiminocyclohepta[d]pyrimidine-10-carboxamide
   (*6R*,*9S*)-*N*-(3,4-dichlorophenyl)-6,7,8,9-tetrahydro-5H-6,9-epiminocyclohepta[d]pyrimidine-10-carboxamide
   (*6R*,*9S*)-*N*-(3-chloro-4-(trifluoromethyl)phenyl)-6,7,8,9-tetrahydro-5H-6,9-epimino-cyclohepta[d]pyrimidine-10-carboxamide
   (*5S*,*8R*)-*N*-(3,4-dichlorophenyl)-2-(trifluoromethyl)-6,7,8,9-tetrahydro-5H-5,8-epimino-cyclohepta[d]pyrimidine-10-carboxamide
   (*5S*,*8R*)-*N*-(3,4-dichlorophenyl)-2-methyl-6,7,8,9-tetrahydro-5H-5,8-epiminocyclohepta-[d]pyrimidine-10-carboxamide
   (*5S*,*8R*)-*N*-(3,4-dichlorophenyl)-2-phenyl-6,7,8,9-tetrahydro-5H-5,8-epiminocyclohepta-[d]pyrimidine-10-carboxamide
   *(5S, 8R)-N-(3-chloro-4-(trifluoromethyl)phenyl)-2-(trifluoromethyl)-6, 7,8,9- tetrahydro-5H-*5,8-epiminocyclohepta[d]pyrimidine-10-carboxamide
   (*5S*,*8R*)-*N*-(4-chloro-3-(trifluoromethyl)phenyl)-2-phenyl-6,7,8,9-tetrahydro-5H-5,8-epiminocyclohepta[d]pyrimidine-10-carboxamide
   (*5S*,*8R*)-*N*-(3-chloro-4-(trifluoromethyl)phenyl)-2-phenyl-6,7,8,9-tetrahydro-5H-5,8-epimino-cyclohepta[d]pyrimidine-10-carboxamide
   (*5S*,*8R*)-*N*-(3,4-dichlorophenyl)-2-hydroxy-6,7,8,9-tetrahydro-5H-5,8-epiminocyclohepta[d-]pyrimidine-10-carboxamide
   (*5S,8R*)-*N*-(3-chloro-4-(trifluoromethyl)phenyl)-2-hydroxy-6,7,8,9-tetrahydro-5H-5,8-epiminocyclohepta[d]pyrimidine-10-carboxamide
   (*5S*,*8R*)-*N*-(3,4-dichlorophenyl)-6,7,8,9-tetrahydro-5H-5,8-epiminobenzo[7]annulene-10-carboxamide
   (*5S,8R*)-*N*-(3-chloro-4-(trifluoromethyl)phenyl)-6,7,8,9-tetrahydro-5H-5,8-epimino-cyclohepta[c]pyridine-10-carboxamide
   (5*S*,8*R*)-*N*-(3,4-dichlorophenyl)-6,7,8,9-tetrahydro-5H-5,8-epiminocyclohepta[c]pyridine-10-carboxamide
   (6*R*,9*S*)-*N*-(3-chloro-4-(trifluoromethyl)phenyl)-6,7,8,9-tetrahydro-5H-6,9-epiminocyclo-hepta-[c]pyridine-10-carboxamide
   (*5S,8R*)*-N*-(3-chloro-4-(trifluoromethyl)phenyl)-6,7,8,9-tetrahydro-5H-5,8-epiminocyclo-hepta-[d]pyrimidine-10-carboxamide
   (*5R,8S*)-*N*-(3-chloro-4-(trifluoromethyl)phenyl)-6,7,8,9-tetrahydro-5H-5,8-epiminocyc-lohepta-[d]pyrimidine-10-carboxamide
   (*5S,8R*)-*N*-(4-chloro-3-(trifluoromethyl)phenyl)-6,7,8,9-tetrahydro-5H-5,8-epiminocyclo-hepta-[d]pyrimidine-10-carboxamide
   *(5S, 8R)- N-(3-chl oro-4-(trifl uoromethyl) phenyl)-2 -oxo-3, 5,6,7,8, 9-hexahydro-2 H-5, 8-*epimino-cyclohepta[d]pyrimidine-10-carboxamide
   *(5R,8S)-N-(3-chloro-4-(trifluoromethyl)phenyl)-2-oxo-3,5,6,7,8,9-hexahydro-2H-5,8-*epimino-cyclohepta[d]pyrimidine-10-carboxamide
   (*5S*,*8R*)-*N*-(3-chloro-4-(trifluoromethyl)phenyl)-2-fluoro-6,7,8,9-tetrahydro-5H-5,8-epimino-cyclohepta[d]pyrimidine-10-carboxamide
   (*5S*,*8R*)-*N*-(3,4-dichlorophenyl)-2-fluoro-6,7,8,9-tetrahydro-5H-5,8-epiminocyclohepta[d]-pyrimidine-10-carboxamide
   (*5S*,*8R*,*E*)-*N*-(3-chloro-4-(trifluoromethyl)phenyl)-2-hydroxy-9-(hydroxyimino)-6,7,8,9-tetrahydro-5H-5,8-epiminocyclohepta[d]pyrimidine-10-carboxamide
   (*5S*,*8R*)-*N*-(3,4-dichlorophenyl)-6,7,8,9-tetrahydro-5H-5,8-epiminocyclohepta[d]pyrimidine-10-carboxamide
   (*5S*,*8R*)-*N*-(4-chlorophenyl)-6,7,8,9-tetrahydro-5H-5,8-epiminocyclohepta[d]pyrimidine-10-carboxamide
   (*5S*,*8R*)-*N*-(3-chlorophenyl)-6,7,8,9-tetrahydro-5H-5,8-epiminocyclohepta[d]pyrimidine-10-carboxamide
   (*5S*,*8R*)-*N*-(5-chloro-2-fluorophenyl)-6,7,8,9-tetrahydro-5H-5,8-epiminocyclohepta[d]-pyrimidine-10-carboxamide
   (*5S*,*8R*)-*N*-(3,4-dichlorophenyl)-6,7,8,9-tetrahydro-5H-5,8-epiminocyclohepta[d]pyrimidine-10-carboxamide
   (*5R*,*8S*)-*N*-(3,4-dichlorophenyl)-6,7,8,9-tetrahydro-5H-5,8-epiminocyclohepta[d]pyrimidine-10-carboxamide
   (*5S,8R*)-*N*-(3-chloro-4-(trifluoromethyl)phenyl)-6,7,8,9-tetrahydro-5H-5,8-epiminobenzo[7]-annulene-10-carboxamide
   (*5S,8R*)-*N*-(4-chloro-3-(trifluoromethyl)phenyl)-6,7,8,9-tetrahydro-5H-5,8-epiminobenzo[7]-annulene-10-carboxamide
   (*5S*,*8R*)-*N*-(3,4-dichlorophenyl)-2-methoxy-6,7,8,9-tetrahydro-5H-5,8-epiminobenzo[7]-annulene-10-carboxamide
   (*5S*,*8R*)-*N*-(3,4-dichlorophenyl)-2-hydroxy-6,7,8,9-tetrahydro-5H-5,8-epiminobenzo[7]-annulene-10-carboxamide
   (*5S,8R*)-10-((3,4-dichlorophenyl)carbamoyl)-6,7,8,9-tetrahydro-5H-5,8-epiminocyclohepta-[c]pyridine 2-oxide
   (*6R*,*9S*)-10-((3,4-dichlorophenyl)carbamoyl)-6,7,8,9-tetrahydro-5H-6,9-epiminocyclohepta-[c]pyridine 2-oxide
   (*5S*,*8R*)-2-chloro-*N*-(3,4-dichlorophenyl)-6,7,8,9-tetrahydro-5*H*-5,8-epiminobenzo[7]-annulene-10-carboxamide
   (*6R,9S*)-1-amino-*N*-(3,4-dichlorophenyl)-6,7,8,9-tetrahydro-5H-6,9-epiminocyclohepta[c]-pyridine-10-carboxamide
   (*6R*,*9S*)-3-amino-*N*-(3,4-dichlorophenyl)-6,7,8,9-tetrahydro-5H-6,9-epiminocyclohepta[c]-pyridine-10-carboxamide
   (*5S*,*8R*)-1-amino-*N*-(3,4-dichlorophenyl)-6,7,8,9-tetrahydro-5H-5,8-epiminocyclohepta[c]-pyridine-10-carboxamide
   (*5S*,*8R*)-3-amino-*N*-(3,4-dichlorophenyl)-6,7,8,9-tetrahydro-5H-5,8-epiminocyclohepta[c]-pyridine-10-carboxamide
   (*5S*,*8R*)-1-(tert-butylamino)-*N*-(3,4-dichlorophenyl)-6,7,8,9-tetrahydro-5H-5,8-epimino-cyclohepta[c]pyridine-10-carboxamide
   (*5S*,*8R*)-3-(tert-butylamino)-*N*-(3,4-dichlorophenyl)-6,7,8,9-tetrahydro-5H-5,8-epimino-cyclohepta[c]pyridine-10-carboxamide
   (*6R*,*9S*)-*N*-(3,4-dichlorophenyl)-3-fluoro-6,7,8,9-tetrahydro-5H-6,9-epiminocyclohepta-[c]pyridine-10-carboxamide
   (*6R*,*9S*)-*N*-(3,4-dichlorophenyl)-1-fluoro-6,7,8,9-tetrahydro-5H-6,9-epiminocyclohepta-[c]pyridine-10-carboxamide
   (*5S*,*8R*)-*N*-(3,4-dichlorophenyl)-3-fluoro-6,7,8,9-tetrahydro-5H-5,8-epiminocyclohepta[c]-pyridine-10-carboxamide
   (*5S*,*8R*)-2-chloro-*N*-(3-chloro-4-(trifluoromethyl)phenyl)-6,7,8,9-tetrahydro-5H-5,8-epimino-benzo[7]annulene-10-carboxamide
   (*5S*,*8R*)-2-cyano-*N*-(3,4-dichlorophenyl)-6,7,8,9-tetrahydro-5H-5,8-epiminobenzo-[7]annulene-10-carboxamide
   (*5S*,*8R*)-2-amino-*N*-(3,4-dichlorophenyl)-6,7,8,9-tetrahydro-5H-5,8-epiminobenzo[7]-annulene-10-carboxamide
   (*5S*,*8R*)-*N*-(3,4-dichlorophenyl)-2-fluoro-6,7,8,9-tetrahydro-5H-5,8-epiminobenzo[7]-annulene-10-carboxamide
   (5*S*,8*R*)-*N*-(3-chloro-4-(trifluoromethyl)phenyl)-2-fluoro-6,7,8,9-tetrahydro-5H-5,8-epimino-benzo[7]annulene-10-carboxamide
   (*6R,9S*)-*N*-(3,4-dichlorophenyl)-3-oxo-3,5,6,7,8,9-hexahydro-2H-6,9-epiminocyclohepta[c]-pyridine-10-carboxamide
   (*5S*,*8R*)-1-chloro-*N*-(3,4-dichlorophenyl)-6,7,8,9-tetrahydro-5H-5,8-epiminocyclohepta[c]-pyridine-10-carboxamide
   (*5S*,*8R*)-*N*-(3,4-dichlorophenyl)-1-fluoro-6,7,8,9-tetrahydro-5H-5,8-epiminocyclohepta[c]-pyridine-10-carboxamide
   (*5S*,*8R*)-*N*-(4,5-dichloro-2-fluorophenyl)-1-fluoro-6,7,8,9-tetrahydro-5H-5,8-epimino-cyclohepta[c]pyridine-10-carboxamide
   (*5S*,*8R*)-*N*-(3,4-dichlorophenyl)-3-fluoro-6,7,8,9-tetrahydro-5H-5,8-epiminobenzo[7]-annulene-10-carboxamide
   (*5S,8R*)-*N*-(3-chloro-4-(trifluoromethyl)phenyl)-3-fluoro-6,7,8,9-tetrahydro-5H-5,8-epimino-benzo[7]annulene-10-carboxamide
   tert-butyl ((*6R*,*9S*)-10-((3,4-dichlorophenyl)carbamoyl)-6,7,8,9-tetrahydro-5H-6,9-epimino-cyclohepta[c]pyridin-1-yl)carbamate
   (*5S*,*8R*)-*N*-(3,4-dichlorophenyl)-1-fluoro-6,7,8,9-tetrahydro-5H-5,8-epiminobenzo[7]-annulene-10-carboxamide
   (*5S,8R*)-*N*-(3-chloro-4-(trifluoromethyl)phenyl)-1-fluoro-6,7,8,9-tetrahydro-5H-5,8-epimino-benzo[7]annulene-10-carboxamide
   (*5S*,*8R*)-*N*-(3-chloro-4-(trifluoromethyl)phenyl)-1-fluoro-6,7,8,9-tetrahydro-5H-5,8-epimino-cyclohepta[c]pyridine-10-carboxamide
   (*6R*,*9S*)-*N*-(3,4-dichlorophenyl)-3-methoxy-6,7,8,9-tetrahydro-5H-6,9-epiminocyclohepta-[c]pyridine-10-carboxamide
   (*5S*,*8R*)-*N*-(3,4-dichlorophenyl)-6,7,8,9-tetrahydro-5H-5,8-epiminocyclohepta[b]pyridine-10-carboxamide
   (*6R*,*9S*)-*N*-(4,5-dichloro-2-cyanophenyl)-3-oxo-3,5,6,7,8,9-hexahydro-2H-6,9-epimino-cyclohepta[c]pyridine-10-carboxamide
   (*6R*,*9S*)-*N*-(3,4-dichlorophenyl)-3-fluoro-6,7,8,9-tetrahydro-5H-6,9-epiminocyclohepta-[c]pyridine-10-carboxamide
   (*5S*,*8R*)-10-((3,4-dichlorophenyl)carbamoyl)-6,7,8,9-tetrahydro-5H-5,8-epiminocyclohepta-[c]-pyridine 2-oxide
   *(6R,9S)-N-(5-chloro-2-fluoro-4-(trifluoromethyl)phenyl)-3-oxo-3,5,6,7,8,9-hexahydro-2H-*6,9-epiminocyclohepta[c]pyridine-10-carboxamide
   (*6R*,*9S*)-*N*-(4,5-dichloro-2-fluorophenyl)-3-oxo-3,5,6,7,8,9-hexahydro-2H-6,9-epimino-cyclohepta[c]pyridine-10-carboxamide
   (*6R*,*9S*)-*N*-(3,4-dichlorophenyl)-4-fluoro-6,7,8,9-tetrahydro-5H-6,9-epiminocyclohepta[c]-pyridine-10-carboxamide
   (*5S*,*8R*)-*N*-(4,5-dichloro-2-cyanophenyl)-1-fluoro-6,7,8,9-tetrahydro-5H-5,8-epimino-cyclohepta[c]pyridine-10-carboxamide
   (*6R*,*9S*)-3-bromo-*N*-(3,4-dichlorophenyl)-6,7,8,9-tetrahydro-5H-6,9-epiminocyclohepta-[c]pyridine-10-carboxamide
   (*6R*,*9S*)-3-chloro-*N*-(3,4-dichlorophenyl)-6,7,8,9-tetrahydro-5H-6,9-epiminocyclohepta[c]-pyridine-10-carboxamide
   *(5S, 8R)-N-(5-chloro-2- fluoro-4-(trifluoromethyl)phenyl)-1-fluoro-6, 7 ,8, 9- tetrahydro-5H-5,8-*epiminocyclohepta[c]pyridine-10-carboxamide
   (*5S,8R*)-*N*-(4-chloro-5-(trifluoromethyl)pyridin-2-yl)-1-fluoro-6,7,8,9-tetrahydro-5H-5,8-epiminocyclohepta[c]pyridine-10-carboxamide
   (*6R*,*9S*)-*N*-(3,4-dichlorophenyl)-3-oxo-3,5,6,7,8,9-hexahydro-2H-6,9-epiminocyclohepta[c]-pyridine-10-carboxamide
   (*6R*,*9S*)-*N*-(3,4-dichlorophenyl)-6,7,8,9-tetrahydro-5H-6,9-epiminocyclohepta[c]pyridine-1 0-carboxamide
   (*5R*,*8S*)-*N*-(3,4-dichlorophenyl)-2-oxo-3,5,6,7,8,9-hexahydro-2H-5,8-epiminocyclohepta[d]-pyrimidine-10-carboxamide
   (*5S*,*8R*)-*N*-(3,4-dichlorophenyl)-2-oxo-3,5,6,7,8,9-hexahydro-2H-5,8-epiminocyclohepta[d]-pyrimidine-10-carboxamide
   (*5R*,*8S*)-*N*-(3,4-dichlorophenyl)-6,7,8,9-tetrahydro-5H-5,8-epiminocyclohepta[c]pyridine-10-carboxamide
   (*5S*,*8R*)-*N*-(3,4-dichlorophenyl)-6,7,8,9-tetrahydro-5H-5,8-epiminocyclohepta[c]pyridine-10-carboxamide
   (*5S*,*8R*)-*N*-(3,4-dichlorophenyl)-1-fluoro-6,7,8,9-tetrahydro-5H-5,8-epiminocyclohepta[c]-pyridine-10-carboxamide
   (*5R*,*8S*)-*N*-(3,4-dichlorophenyl)-1-fluoro-6,7,8,9-tetrahydro-5H-5,8-epiminocyclohepta[c]-pyridine-10-carboxamide
   (*6R*,*9S*)-*N*-(3,4-dichlorophenyl)-6,7,8,9-tetrahydro-5H-6,9-epiminocyclohepta[c]pyridine-1 0-carboxamide
   (*6S*,*9R*)-*N*-(3,4-dichlorophenyl)-6,7,8,9-tetrahydro-5H-6,9-epiminocyclohepta[c]pyridine-10-carboxamide
   *(5S*,*8R,9R*)-*N*-(3,4-dichlorophenyl)-9-fluoro-6,7,8,9-tetrahydro-5H-5,8-epiminocyclohepta[d]-pyrimidine-10-carboxamide
(*5R,8S*)-*N*-(3-chloro-4-(trifluoromethyl)phenyl)-8-methyl-6,7,8,9-tetrahydro-5H-5,8-epimin-ocyclohepta[c]pyridine-10-carboxamide
(*5R*,*8S*)*-N*-(4,5-dichloro-2-methylphenyl)-1-fluoro-6,7,8,9-tetrahydro-5H-5,8-epiminocyclo-hepta[c]pyridine-10-carboxamide
(*5R*,*8S*)-*N*-(4,5-dichloro-2-methoxyphenyl)-1-fluoro-6,7,8,9-tetrahydro-5H-5,8-epimino-cyclohepta[c]pyridine-10-carboxamide
(*5R,8S*)-1-fluoro-*N*-(2-fluoro-5-(trifluoromethyl)phenyl)-6,7,8,9-tetrahydro-5H-5,8-epimino-cyclohepta[c]pyridine-10-carboxamide
(*5R,8S*)-1-fluoro-*N*-(2-fluoro-4-(trifluoromethyl)phenyl)-6,7,8,9-tetrahydro-5H-5,8-epimino-cyclohepta[c]pyridine-10-carboxamide
(*5R,8S*)-1-fluoro-*N*-(4-(trifluoromethyl)phenyl)-6,7,8,9-tetrahydro-5H-5,8-epiminocyclo-hepta[c]pyridine-10-carboxamide
methyl 2-chloro-4-((*5R*,*8S*)-1-fluoro-6,7,8,9-tetrahydro-5H-5,8-epiminocyclohepta[c]-pyridine-10-carboxamido)benzoate
methyl 2-chloro-5-((*5R*,*8S*)-1-fluoro-6,7,8,9-tetrahydro-5H-5,8-epiminocyclohepta[c]-pyridine-10-carboxamido)benzoate
(*5R*,*8S*)-*N*-(3-chloro-4-(trifluoromethoxy)phenyl)-1-fluoro-6,7,8,9-tetrahydro-5H-5,8-epiminocyclohepta[c]pyridine-10-carboxamide
methyl 2-chloro-4-fluoro-5-((*5R*,*8S*)-1-fluoro-6,7,8,9-tetrahydro-5H-5,8-epiminocyclo-hepta[c]pyridine-10-carboxamido)benzoate
(*5R*,*8S*)-*N*-(4-chloro-2-fluoro-5-(trifluoromethyl)phenyl)-1-fluoro-6,7,8,9-tetrahydro-5H-5,8-epiminocyclohepta[c]pyridine-10-carboxamide
(*6S,9R*)-*N*-(3-chloro-4-(difluoromethoxy)phenyl)-3-oxo-3,5,6,7,8,9-hexahydro-2H-6,9-epiminocyclohepta[c]pyridine-10-carboxamide
(*6S,9R*)-*N*-(3-chloro-4-(trifluoromethoxy)phenyl)-3-oxo-3,5,6,7,8,9-hexahydro-2H-6,9-epiminocyclohepta[c]pyridine-10-carboxamide
(*6S,9R*)-*N*-(4-chloro-2-fluoro-5-(trifluoromethyl)phenyl)-3-oxo-3,5,6,7,8,9-hexahydro-2H-6,9-epiminocyclohepta[c]pyridine-10-carboxamide
methyl 2-chloro-4-((*6S*,*9R*)-3-oxo-3,5,6,7,8,9-hexahydro-2H-6,9-epiminocyclohepta[c]-pyridine-10-carboxamido)benzoate
methyl 2-chloro-5-((*6S*,*9R*)-3-oxo-3,5,6,7,8,9-hexahydro-2H-6,9-epiminocyclohepta[c]-pyridine-10-carboxamido)benzoate
methyl 2-chloro-4-fluoro-5-((*6S*,*9R*)-3-oxo-3,5,6,7,8,9-hexahydro-2H-6,9-epiminocyclo-hepta[c]pyridine-10-carboxamido)benzoate
(*5R*,*8S*)-*N*-(3-chloro-4-(difluoromethoxy)phenyl)-1-fluoro-6,7,8,9-tetrahydro-5H-5,8-epiminocyclohepta[c]pyridine-10-carboxamide
(*5R,8S,9S*)-*N*-(3,4-dichlorophenyl)-9-methyl-6,7,8,9-tetrahydro-5H-5,8-epiminocyclo-hepta[c]pyridine-10-carboxamide
(*6S*,*9R*)-*N*-(3,4-dichlorophenyl)-3,4-difluoro-6,7,8,9-tetrahydro-5H-6,9-epiminocyclo-hepta[c]pyridine-10-carboxamide
(*6S,9R*)-*N*-(5-chloro-2-fluoro-4-(trifluoromethyl)phenyl)-3,4-difluoro-6,7,8,9-tetrahydro-5H-6,9-epiminocyclohepta[c]pyridine-10-carboxamide
(*6S*,*9R*)-*N*-(4,5-dichloro-2-fluorophenyl)-3,4-difluoro-6,7,8,9-tetrahydro-5H-6,9-epimino-cyclohepta[c]pyridine-10-carboxamide
(*6S,9R*)-*N*-(5-chloro-2-fluoro-4-(trifluoromethyl)phenyl)-4-fluoro-3-oxo-3,5,6,7,8,9-hexahydro-2H-6,9-epiminocyclohepta[c]pyridine-10-carboxamide
(*6S,9R*)-*N*-(4,5-dichloro-2-fluorophenyl)-4-fluoro-3-oxo-3,5,6,7,8,9-hexahydro-2H-6,9-epiminocyclohepta[c]pyridine-10-carboxamide
(5S,*6S*,*9R*)-*N*-(3,4-dichlorophenyl)-3,5-difluoro-6,7,8,9-tetrahydro-5H-6,9-epiminocyclo-hepta[c]pyridine-10-carboxamide
(*6S*,*9R*)-*N*-(4-bromo-5-chloro-2-fluorophenyl)-3-oxo-3,5,6,7,8,9-hexahydro-2H-6,9-epiminocyclohepta[c]pyridine-10-carboxamide
(*6S*,*9R*)-*N*-(5-bromo-4-chloro-2-fluorophenyl)-3-oxo-3,5,6,7,8,9-hexahydro-2H-6,9-epiminocyclohepta[c]pyridine-10-carboxamide
(*6S*,*9R*)-*N*-(4-bromo-3-chlorophenyl)-3-oxo-3,5,6,7,8,9-hexahydro-2H-6,9-epiminocyclo-hepta[c]pyridine-10-carboxamide
(*6S,9R*)-*N*-(5-bromo-2-fluoro-4-(trifluoromethyl)phenyl)-3-oxo-3,5,6,7,8,9-hexahydro-2H-6,9-epiminocyclohepta[c]pyridine-10-carboxamide
(*6S*,*9R*)-*N*-(3-bromo-4-chlorophenyl)-3-oxo-3,5,6,7,8,9-hexahydro-2H-6,9-epiminocyclo-hepta[c]pyridine-10-carboxamide
(*6S,9R*)-*N*-(5-chloro-2-fluoro-4-(trifluoromethyl)phenyl)-4-fluoro-3-oxo-3,5,6,7,8,9-hexahydro-2H-6,9-epiminocyclohepta[c]pyridine-10-carboxamide
(*6R,9S*)-*N-(5-chloro-2-fluoro-4-(trifluoromethyl)phenyl)-4-fluoro-3-oxo-3,5,6,7,8,9-*hexahydro-2H-6,9-epiminocyclohepta[c]pyridine-10-carboxamide
(*5R,8S*)-1-bromo-*N*-(3,4-dichlorophenyl)-6,7,8,9-tetrahydro-5H-5,8-epiminocyclohepta[c]-pyridine-10-carboxamide
(*5R*,*8S*)-*N*-(4,5-dichloro-2-hydroxyphenyl)-1-fluoro-6,7,8,9-tetrahydro-5H-5,8-epimino-cyclohepta[c]pyridine-10-carboxamide
(*6S*,*9R*)-*N*-(3,4-dichlorophenyl)-2-methyl-3-oxo-3,5,6,7,8,9-hexahydro-2H-6,9-epimino-cyclohepta[c]pyridine-10-carboxamide
(*5S,8R*)-*N*-(3-chloro-4-(trifluoromethyl)phenyl)-8-methyl-6,7,8,9-tetrahydro-5H-5,8-epimin-ocyclohepta[c]pyridine-10-carboxamide
(*5S*,*8R*)-*N*-(4,5-dichloro-2-methylphenyl)-1-fluoro-6,7,8,9-tetrahydro-5H-5,8-epiminocyclo-hepta[c]pyridine-10-carboxamide
(*5S*,*8R*)-*N*-(4,5-dichloro-2-methoxyphenyl)-1-fluoro-6,7,8,9-tetrahydro-5H-5,8-epimino-cyclohepta[c]pyridine-10-carboxamide
(*5S,8R*)*-*1*-*fluoro-*N*-(2-fluoro-5-(trifluoromethyl)phenyl)-6,7,8,9-tetrahydro-5H-5,8-epimino-cyclohepta[c]pyridine-10-carboxamide
(*5S,8R*)-*1*-fluoro-*N*-(2-fluoro-4-(trifluoromethyl)phenyl)-6,7,8,9-tetrahydro-5H-5,8-epimino-cyclohepta[c]pyridine-10-carboxamide
(*5S,8R*)-1-fluoro-*N*-(*4*-(trifluoromethyl)phenyl)-6,7,8,9-tetrahydro-5H-5,8-epiminocyclo-hepta[c]pyridine-10-carboxamide
methyl 2-chloro-4-((*5S*,*8R*)-1-fluoro-6,7,8,9-tetrahydro-5H-5,8-epiminocyclohepta[c]-pyridine-10-carboxamido)benzoate
methyl 2-chloro-5-((*5S*,*8R*)-1-fluoro-6,7,8,9-tetrahydro-5H-5,8-epiminocyclohepta[c]-pyridine-10-carboxamido)benzoate
(*5S*,*8R*)-*N*-(3-chloro-4-(trifluoromethoxy)phenyl)-1-fluoro-6,7,8,9-tetrahydro-5H-5,8-epiminocyclohepta[c]pyridine-10-carboxamide
methyl 2-chloro-4-fluoro-5-((*5S*,*8R*)-1-fluoro-6,7,8,9-tetrahydro-5H-5,8-epiminocyclo-hepta[c]pyridine-10-carboxamido)benzoate
(*5S,8R*)-*N-(4-chloro-2-fluoro-5-(trifluoromethyl)phenyl)-1-fluoro-6,7,8,9-tetrahydro-5H-5,8-*epiminocyclohepta[c]pyridine-10-carboxamide
(*6R*,*9S*)-*N*-(3-chloro-4-(difluoromethoxy)phenyl)-3-oxo-3,5,6,7,8,9-hexahydro-2H-6,9-epiminocyclohepta[c]pyridine-10-carboxamide
(*6R*,*9S*)-*N*-(3-chloro-4-(trifluoromethoxy)phenyl)-3-oxo-3,5,6,7,8,9-hexahydro-2H-6,9-epiminocyclohepta[c]pyridine-10-carboxamide
(*6R,9S*)-*N-(4-chloro-2-fluoro-5-(trifluoromethyl)phenyl)-3-oxo-3,5,6,7,8,9-hexahydro-2H-*6,9-epiminocyclohepta[c]pyridine-10-carboxamide
methyl 2-chloro-4-((*6R,9S*)-3-oxo-3,5,6,7,8,9-hexahydro-2H-6,9-epiminocyclohepta[c]-pyridine-10-carboxamido)benzoate
methyl 2-chloro-5-((*6R*,*9S*)-3-oxo-3,5,6,7,8,9-hexahydro-2H-6,9-epiminocyclohepta[c]-pyridine-10-carboxamido)benzoate
methyl 2-chloro-4-fluoro-5-((*6R*,*9S*)-3-oxo-3,5,6,7,8,9-hexahydro-2H-6,9-epiminocyclo-hepta[c]pyridine-10-carboxamido)benzoate
(*5S*,*8R*)-*N*-(3-chloro-4-(difluoromethoxy)phenyl)-1-fluoro-6,7,8,9-tetrahydro-5H-5,8-epiminocyclohepta[c]pyridine-10-carboxamide
(*5S,8R,9S*)-*N-*(3,4-dichlorophenyl)-9-methyl-6,7,8,9-tetrahydro-5H-5,8-epiminocyclo-hepta[c]pyridine-10-carboxamide
(*6R*,*9S*)-*N*-(3,4-dichlorophenyl)-3,4-difluoro-6,7,8,9-tetrahydro-5H-6,9-epiminocyclo-hepta[c]pyridine-10-carboxamide
(*6R,9S*)-*N-(5-chloro-2-fluoro-4-(trifluoromethyl)phenyl)-3,4-difluoro-6,7,8,9-tetrahydro-5H-*6,9-epiminocyclohepta[c]pyridine-10-carboxamide
(*6R*,*9S*)-*N*-(4,5-dichloro-2-fluorophenyl)-3,4-difluoro-6,7,8,9-tetrahydro-5H-6,9-epimino-cyclohepta[c]pyridine-10-carboxamide
(*6R,9S*)*-N-(5-chloro-2-fluoro-4-(trifluoromethyl)phenyl)-4-fluoro-3-oxo-3,5,6,7,8,9-*hexahydro-2H-6,9-epiminocyclohepta[c]pyridine-10-carboxamide
(*6R*,*9S*)-*N*-(4,5-dichloro-2-fluorophenyl)-4-fluoro-3-oxo-3,5,6,7,8,9-hexahydro-2H-6,9-epiminocyclohepta[c]pyridine-10-carboxamide
(5S,*6R*,*9S*)-*N*-(3,4-dichlorophenyl)-3,5-difluoro-6,7,8,9-tetrahydro-5H-6,9-epiminocyclo-hepta[c]pyridine-10-carboxamide
(*6R*,*9S*)-*N*-(4-bromo-5-chloro-2-fluorophenyl)-3-oxo-3,5,6,7,8,9-hexahydro-2H-6,9-epiminocyclohepta[c]pyridine-10-carboxamide
(*6R*,*9S*)-*N*-(5-bromo-4-chloro-2-fluorophenyl)-3-oxo-3,5,6,7,8,9-hexahydro-2H-6,9-epiminocyclohepta[c]pyridine-10-carboxamide
(*6R*,*9S*)-*N*-(4-bromo-3-chlorophenyl)-3-oxo-3,5,6,7,8,9-hexahydro-2H-6,9-epiminocyclo-hepta[c]pyridine-10-carboxamide
(*6R,9S*)-*N*-(5-bromo-2-fluoro-4-(trifluoromethyl)phenyl)-3-oxo-3,5,6,7,8,9-hexahydro-2H-6,9-epiminocyclohepta[c]pyridine-10-carboxamide
(6*R*,9*S*)-*N*-(3-bromo-4-chlorophenyl)-3-oxo-3,5,6,7,8,9-hexahydro-2H-6,9-epiminocyclo-hepta[c]pyridine-10-carboxamide
(*6R,9S*)-*N*-(5-chloro-2-fluoro-4-(trifluoromethyl)phenyl)-4-fluoro-3-oxo-3,5,6,7,8,9-hexahydro-2H-6,9-epiminocyclohepta[c]pyridine-10-carboxamide
(*5S*,*8R*)-1-bromo-*N*-(3,4-dichlorophenyl)-6,7,8,9-tetrahydro-5H-5,8-epiminocyclohepta[c]-pyridine-10-carboxamide
(*5S*,*8R*)-*N*-(4,5-dichloro-2-hydroxyphenyl)-1-fluoro-6,7,8,9-tetrahydro-5H-5,8-epimino-cyclohepta[c]pyridine-10-carboxamide
(*6R*,*9S*)-*N*-(3,4-dichlorophenyl)-2-methyl-3-oxo-3,5,6,7,8,9-hexahydro-2H-6,9-epimino-cyclohepta[c]pyridine-10-carboxamide
(*5R*,*8S*)-*N*-(3-cyano-4-(trifluoromethyl)phenyl)-1-fluoro-6,7,8,9-tetrahydro-5H-5,8-epiminocyclohepta[c]pyridine-10-carboxamide
(*5R,8S*)-1-fluoro-*N*-(3-methoxy-4-(oxazol-5-yl)phenyl)-6,7,8,9-tetrahydro-5H-5,8-epiminocyclohepta[c]pyridine-10-carboxamide
(*5R*,*8S*)-*N*-(3-bromo-4-(trifluoromethyl)phenyl)-1-fluoro-6,7,8,9-tetrahydro-5H-5,8-epiminocyclohepta[c]pyridine-10-carboxamide
(*5R*,*8S*)-*N*-(3-chloro-2,4-difluorophenyl)-1-fluoro-6,7,8,9-tetrahydro-5H-5,8-epiminocyclo-hepta[c]pyridine-10-carboxamide
(*5R*,*8S*)-1-fluoro-*N*-(3-fluorophenyl)-6,7,8,9-tetrahydro-5H-5,8-epiminocyclohepta[c]-pyridine-10-carboxamide
(*5R,8S*)-1-fluoro-*N*-(3-(trifluoromethyl)phenyl)-6,7,8,9-tetrahydro-5H-5,8-epiminocyclo-hepta[c]pyridine-10-carboxamide
(*5R*,*8S*)-1-fluoro-*N*-(4-(trifluoromethoxy)phenyl)-6,7,8,9-tetrahydro-5H-5,8-epiminocyclo-hepta[c]pyridine-10-carboxamide
(*5R,8S*)-*N*-([1,1'-biphenyl]-4-yl)-1-fluoro-6,7,8,9-tetrahydro-5H-5,8-epiminocyclohepta[c]-pyridine-10-carboxamide
*(*5*R,8S*)-*N*-(3,*5*-bis(trifluoromethyl)phenyl)-1-fluoro-6,7,8,9-tetrahydro-5H-5,8-epiminocyclo-hepta[c]pyridine-10-carboxamide
(*5R,8S*)-1-fluoro-*N*-(4-methyl-3-(trifluoromethyl)phenyl)-6,7,8,9-tetrahydro-5H-5,8-epiminocyclohepta[c]pyridine-10-carboxamide
(*5R,8S*)-*N*-(2,3-dichlorophenyl)-1-fluoro-6,7,8,9-tetrahydro-5H-5,8-epiminocyclohepta[c]-pyridine-10-carboxamide
(*5R*,*8S*)-*N*-(2,4-difluorophenyl)-1-fluoro-6,7,8,9-tetrahydro-5H-5,8-epiminocyclohepta[c]-pyridine-10-carboxamide
(*5R,8S*)-*N*-(4-cyano-3-(trifluoromethyl)phenyl)-1-fluoro-6,7,8,9-tetrahydro-5H-5,8-epiminocyclohepta[c]pyridine-10-carboxamide
(*5R,8S*)-*N*-(3-chloro-5-(trifluoromethyl)phenyl)-1-fluoro-6,7,8,9-tetrahydro-5H-5,8-epiminocyclohepta[c]pyridine-10-carboxamide
(*5R,8S*)-*N*-(3-cyano-4-fluorophenyl)-1-fluoro-6,7,8,9-tetrahydro-5H-5,8-epiminocyclohepta[c]pyridine-10-carboxamide
(*5R,8S*)-1-fluoro-*N*-(3,4,5-trichlorophenyl)-6,7,8,9-tetrahydro-5H-5,8-epiminocyclohepta[c]-pyridine-10-carboxamide
(*5R,8S*)-1-fluoro-*N*-(3-methyl-4-(trifluoromethyl)phenyl)-6,7,8,9-tetrahydro-5H-5,8-epiminocyclo-hepta[c]pyridine-10-carboxamide
(*5R*,*8S*)-*N*-(4-bromophenyl)-1-fluoro-6,7,8,9-tetrahydro-5H-5,8-epiminocyclohepta[c]-pyridine-10-carboxamide
(*5R*,*8S*)-*N*-(4-(difluoromethoxy)phenyl)-1-fluoro-6,7,8,9-tetrahydro-5H-5,8-epiminocyclohepta[c]pyridine-10-carboxamide
(*5R*,*8S*)*-N-*(3-bromophenyl)-1-fluoro-6,7,8,9-tetrahydro-5H-5,8-epiminocyclohepta[c]-pyridine-10-carboxamide
(*5R,8S*)-1-fluoro-*N*-(3-(trifluoromethoxy)phenyl)-6,7,8,9-tetrahydro-5H-5,8-epiminocyclohepta[c]pyridine-10-carboxamide
(*5R*,*8S*)-*N*-(3-bromo-4-fluorophenyl)-1-fluoro-6,7,8,9-tetrahydro-5H-5,8-epiminocyclohepta[c]pyridine-10-carboxamide
(*5R*,*8S*)-*N*-(3,5-difluorophenyl)-1-fluoro-6,7,8,9-tetrahydro-5H-5,8-epiminocyclohepta[c]-pyridine-10-carboxamide
(*5R,8S*)*-N-*(5-chloro-2-fluorophenyl)-1-fluoro-6,7,8,9-tetrahydro-5H-5,8-epiminocyclohepta[c]pyridine-10-carboxamide
(*5R,8S*)-1-fluoro-*N*-(2,3,4-trifluorophenyl)-6,7,8,9-tetrahydro-5H-5,8-epiminocyclohepta[c]pyridine-10-carboxamide
(*5R*,*8S*)-*N*-(4-cyanophenyl)-1-fluoro-6,7,8,9-tetrahydro-5H-5,8-epiminocyclohepta[c]pyridine-10-carboxamide
(*5R,8S*)*-N-*(4-chloro-2-fluorophenyl)-1-fluoro-6,7,8,9-tetrahydro-5H-5,8-epiminocyclohepta[c]pyridine-10-carboxamide
(*5R,8S*)-*N*-(3-chloro-4-fluorophenyl)-1-fluoro-6,7,8,9-tetrahydro-5H-5,8-epiminocyclohepta[c]pyridine-10-carboxamide
(*5R*,*8S*)-*N*-(4-acrylamido-3-chlorophenyl)-1-fluoro-6,7,8,9-tetrahydro-5H-5,8-epiminocyclohepta[c]pyridine-10-carboxamide
(*5R,8S*)-*N*-(5-bromo-2-fluoro-4-(trifluoromethyl)phenyl)-1-fluoro-6,7,8,9-tetrahydro-5H-5,8-epiminocyclohepta[c]pyridine-10-carboxamide
(*5R,8S*)-*N*-(4,5-dichloro-2-fluorophenyl)-2-fluoro-6,7,8,9-tetrahydro-5H-5,8-epiminocyclohepta[b]pyridine-10-carboxamide
(*5R,8*S)-*N*-(5-chloro-2-fluoro-4-(trifluoromethyl)phenyl)-2-fluoro-6,7,8,9-tetrahydro-5H-5,8-epiminocyclohepta[b]pyridine-10-carboxamide
(*5R,8S*)-*N*-(4-(1H-pyrazol-1-yl)phenyl)-1-fluoro-6,7,8,9-tetrahydro-5H-5,8-epiminocyclohepta[c]pyridine-10-carboxamide
(*5R,8S*)-*N*-([1,1'-biphenyl]-3-yl)-1-fluoro-6,7,8,9-tetrahydro-5H-5,8-epiminocyclohepta[c]-pyridine-10-carboxamide
(*5R,8S*)-1-fluoro-*N*-(4-phenoxyphenyl)-6,7,8,9-tetrahydro-5H-5,8-epiminocyclohepta[c-]pyridine-10-carboxamide
(*5R,8S*)-1-fluoro-*N*-(4-(thiazol-4-yl)phenyl)-6,7,8,9-tetrahydro-5H-5,8-epiminocyclohepta[c]pyridine-10-carboxamide
(*5R,8S*)-1-fluoro-*N*-(4-(4-methyloxazol-5-yl)phenyl)-6,7,8,9-tetrahydro-5H-5,8-epiminocyclohepta[c]pyridine-10-carboxamide
(*5R,8S*)-1-fluoro-*N*-(4-(oxazol-5-yl)phenyl)-6,7,8,9-tetrahydro-5H-5,8-epiminocyclohepta[c]pyridine-10-carboxamide
(*5R,8S*)-*N*-(5-chloro-2-fluoro-4-(trifluoromethyl)phenyl)-2-oxo-2,5,6,7,8,9-hexahydro-1H-5,8-epiminocyclohepta[b]pyridine-1 0-carboxamide
(*5R,8S*)-*N*-(4,5-dichloro-2-fluorophenyl)-4-fluoro-6,7,8,9-tetrahydro-5H-5,8-epiminocyclohepta[c]pyridine-10-carboxamide
(*6S,9R*)-*N*-([1,1'-biphenyl]-4-yl)-3-oxo-3,5,6,7,8,9-hexahydro-2H-6,9-epiminocyclohepta[c]pyridine-10-carboxamide
(*5R,8S*)-*N*-([1,1'-biphenyl]-4-yl)-1-fluoro-6,7,8,9-tetrahydro-5H-5,8-epiminocyclohepta[c]pyridine-10-carboxamide
(*5R*,*8S*)-*N*-(4,5-dichloro-2-fluorophenyl)-6,7,8,9-tetrahydro-5H-5,8-epiminocyclohepta[d]pyridazine-10-carboxamide
(*5R,8S*)-*N*-(5-chloro-2-fluoro-4-(trifluoromethyl)phenyl)-6,7,8,9-tetrahydro-5H-5,8-epiminocyclohepta[d]pyridazine-10-carboxamide
(*5R,8S*)-1,4-dichloro-*N*-(5-chloro-2-fluoro-4-(trifluoromethyl)phenyl)-6,7,8,9-tetrahydro-5H-5,8-epiminocyclohepta[d]pyridazine-10-carboxamide
(*5R*,*8S*)-*N*-(5-chloro-2-fluoro-4-(trifluoromethyl)phenyl)-2-fluoro-6,7,8,9-tetrahydro-5H-5,8-epiminocyclohepta[b]pyridine-10-carboxamide
(*5S,8R*)-*N*-(5-chloro-2-fluoro-4-(trifluoromethyl)phenyl)-2-fluoro-6,7,8,9-tetrahydro-5H-5,8-epiminocyclohepta[b]pyridine-10-carboxamide
(*6R,9S*)-*N*-(3,4-dichlorophenyl)-3-fluoro-6,7,8,9-tetrahydro-5H-6,9-epiminocyclohepta[c]-pyridine-10-carboxamide
(5R,*8S*)-*N*-(4-chloro-3-cyanophenyl)-1-fluoro-6,7,8,9-tetrahydro-5H-5,8-epiminocyclohepta[c]pyridine-10-carboxamide
(*5S,8R*)-*N*-(3-cyano-4-(trifluoromethyl)phenyl)-1-fluoro-6,7,8,9-tetrahydro-5H-5,8-epiminocyclohepta[c]pyridine-10-carboxamide
(*5S,8R*)-1-fluoro-*N*-(3-methoxy-4-(oxazol-5-yl)phenyl)-6,7,8,9-tetrahydro-5H-5,8-epiminocyclohepta[c]pyridine-10-carboxamide
(*5S,8R*)-*N*-(3-bromo-4-(trifluoromethyl)phenyl)-1-fluoro-6,7,8,9-tetrahydro-5H-5,8-epiminocyclohepta[c]pyridine-10-carboxamide
(*5S,8R*)-*N*-(3-chloro-2,4-difluorophenyl)-1-fluoro-6,7,8,9-tetrahydro-5H-5,8-epiminocyclohepta[c]pyridine-10-carboxamide
(*5S,8R*)-1-fluoro-*N*-(3-fluorophenyl)-6,7,8,9-tetrahydro-5H-5,8-epiminocyclohepta[c]-pyridine-10-carboxamide
(*5S,8R*)-1-fluoro-*N*-(3-(trifluoromethyl)phenyl)-6,7,8,9-tetrahydro-5H-5,8-epiminocyclo-hepta[c]pyridine-10-carboxamide
(*5S,8R*)-1-fluoro-*N*-(4-(trifluoromethoxy)phenyl)-6,7,8,9-tetrahydro-5H-5,8-epiminocyclo-hepta[c]pyridine-10-carboxamide
(*5S,8R*)-*N*-([1,1'-biphenyl]-4-yl)-1-fluoro-6,7,8,9-tetrahydro-5H-5,8-epiminocyclohepta[c]pyridine-10-carboxamide
(*5S,8R*)-*N*-(3,5-bis(trifluoromethyl)phenyl)-1-fluoro-6,7,8,9-tetrahydro-5H-5,8-epiminocyclo-hepta[c]pyridine-10-carboxamide
(*5S,8R*)-1-fluoro-*N*-(4-methyl-3-(trifluoromethyl)phenyl)-6,7,8,9-tetrahydro-5H-5,8-epiminocyclohepta[c]pyridine-10-carboxamide
(*5S*,*8R*)-*N*-(2,3-dichlorophenyl)-1-fluoro-6,7,8,9-tetrahydro-5H-5,8-epiminocyclohepta[c]-pyridine-10-carboxamide
(*5S*,*8R*)-*N*-(2,4-difluorophenyl)-1-fluoro-6,7,8,9-tetrahydro-5H-5,8-epiminocyclohepta[c]-pyridine-10-carboxamide
(*5S,8R*)-*N*-(4-cyano-3-(trifluoromethyl)phenyl)-1-fluoro-6,7,8,9-tetrahydro-5H-5,8-epiminocyclohepta[c]pyridine-10-carboxamide
(*5S,8R*)-*N*-(3-chloro-5-(trifluoromethyl)phenyl)-1-fluoro-6,7,8,9-tetrahydro-5H-5,8-epiminocyclohepta[c]pyridine-10-carboxamide
(*5S,8R*)-*N*-(3-cyano-4-fluorophenyl)-1-fluoro-6,7,8,9-tetrahydro-5H-5,8-epiminocyclohepta[c]pyridine-10-carboxamide
(*5S,8R*)-1-fluoro-*N*-(3,4,5-trichlorophenyl)-6,7,8,9-tetrahydro-5H-5,8-epiminocyclohepta[c]pyridine-10-carboxamide
(*5S,8R*)-1-fluoro-*N*-(3-methyl-4-(trifluoromethyl)phenyl)-6,7,8,9-tetrahydro-5H-5,8-epiminocyclohepta[c]pyridine-10-carboxamide
(*5S,8R*)-*N*-(4-bromophenyl)-1-fluoro-6,7,8,9-tetrahydro-5H-5,8-epiminocyclohepta[c]-pyridine-10-carboxamide
(*5S,8R*)-*N*-(4-(difluoromethoxy)phenyl)-1-fluoro-6,7,8,9-tetrahydro-5H-5,8-epiminocyclohepta[c]pyridine-10-carboxamide
(*5S,8R*)-*N*-(3-bromophenyl)-1-fluoro-6,7,8,9-tetrahydro-5H-5,8-epiminocyclohepta[c]-pyridine-10-carboxamide
(*5S,8R*)-1-fluoro-*N*-(3-(trifluoromethoxy)phenyl)-6,7,8,9-tetrahydro-5H-5,8-epiminocyclohepta[c]pyridine-10-carboxamide
(*5S,8R*)-*N*-(3-bromo-4-fluorophenyl)-1-fluoro-6,7,8,9-tetrahydro-5H-5,8-epiminocyclohepta[c]pyridine-10-carboxamide
(*5S,8R*)-*N*-(3,5-difluorophenyl)-1-fluoro-6,7,8,9-tetrahydro-5H-5,8-epiminocyclohepta[c]-pyridine-10-carboxamide
(*5S,8R*)-*N*-(5-chloro-2-fluorophenyl)-1-fluoro-6,7,8,9-tetrahydro-5H-5,8-epiminocyclohepta[c]pyridine-10-carboxamide
*(5S, 8R*)-1-fluoro-*N-*(2,3,4-trifluorophenyl)-6,7,8,9-tetrahydro-5H-5,8-epiminocyclohepta[c]pyridine-10-carboxamide
(5S, *8R*)-*N*-(4-cyanophenyl)-1-fluoro-6,7,8,9-tetrahydro-5H-5,8-epiminocyclohepta[c]-pyridine-10-carboxamide
(5S, *8R*)-*N*-(4-chloro-2-fluorophenyl)-1-fluoro-6,7,8,9-tetrahydro-5H-5,8-epiminocyclohepta[c]pyridine-10-carboxamide
(5S, *8R*)-*N*-(3-chloro-4-fluorophenyl)-1-fluoro-6,7,8,9-tetrahydro-5H-5,8-epiminocyclohepta[c]pyridine-10-carboxamide
(5S, *8R*)-*N*-(4-acrylamido-3-chlorophenyl)-1-fluoro-6,7,8,9-tetrahydro-5H-5,8-epiminocyclohepta[c]pyridine-10-carboxamide
(5S, 8R)-N-(5-bromo-2-fluoro-4-(trifluoromethyl)phenyl)-1-fluoro-6,7,8,9-tetrahydro-5H-5,8-epiminocyclohepta[c]pyridine-10-carboxamide
(*5S,8R*)-*N*-(4,5-dichloro-2-fluorophenyl)-2-fluoro-6,7,8,9-tetrahydro-5H-5,8-epiminocyclohepta[b]pyridine-10-carboxamide
(5*S,8R*)-*N*-(5-chloro-2-fluoro-4-(trifluoromethyl)phenyl)-2-fluoro-6,7,8,9-tetrahydro-5H-5,8-epiminocyclohepta[b]pyridine-10-carboxamide
(*5S,8R*)-*N*-(4-(1H-pyrazol-1-yl)phenyl)-1-fluoro-6,7,8,9-tetrahydro-5H-5,8-epiminocyclohepta[c]pyridine-10-carboxamide
(*5S,8R*)-*N*-([1,1'-biphenyl]-3-yl)-1-fluoro-6,7,8,9-tetrahydro-5H-5,8-epiminocyclohepta[c]pyridine-10-carboxamide
(*5S,8R*)-1-fluoro-*N*-(4-phenoxyphenyl)-6,7,8,9-tetrahydro-5H-5,8-epiminocyclohepta[c]pyridine-10-carboxamide
(*5S,8R*)-1-fluoro-*N*-(4-(thiazol-4-yl)phenyl)-6,7,8,9-tetrahydro-5H-5,8-epiminocyclohepta[c]pyridine-10-carboxamide
(6*S*,8*R*)-1-fluoro-*N*-(4-(4-methyloxazol-5-yl)phenyl)-6,7,8,9-tetrahydro-5H-5,8-epiminocyclo-hepta[c]pyridine-10-carboxamide
(*5S,8R*)-1-fluoro-*N*-(4-(oxazol-5-yl)phenyl)-6,7,8,9-tetrahydro-5H-5,8-epiminocyclohepta[c]pyridine-10-carboxamide
(*5S,8R*)-*N*-(5-chloro-2-fluoro-4-(trifluoromethyl)phenyl)-2-oxo-2,5,6,7,8,9-hexahydro-1H-5,8-epiminocyclohepta[b]pyridine-10-carboxamide
(*5S,8R*)-*N*-(4,5-dichloro-2-fluorophenyl)-4-fluoro-6,7,8,9-tetrahydro-5H-5,8-epiminocyclohepta[c]pyridine-10-carboxamide
(*6R,9S*)-*N*-([1,1'-biphenyl]-4-yl)-3-oxo-3,5,6,7,8,9-hexahydro-2H-6,9-epiminocyclohepta[c]pyridine-10-carboxamide
(*5S,8R*)-*N*-([1,1'-biphenyl]-4-yl)-1-fluoro-6,7,8,9-tetrahydro-5H-5,8-epiminocyclohepta[c]pyridine-10-carboxamide
(5S, *8R*)-*N*-(4,5-dichloro-2-fluorophenyl)-6,7,8,9-tetrahydro-5H-5,8-epiminocyclohepta[d]pyridazine-10-carboxamide
(*5S,8R*)-*N*-(5-chloro-2-fluoro-4-(trifluoromethyl)phenyl)-6,7,8,9-tetrahydro-5H-5,8-epiminocyclohepta[d]pyridazine-10-carboxamide
(*5S,8R*)-1,4-dichloro-*N*-(5-chloro-2-fluoro-4-(trifluoromethyl)phenyl)-6,7,8,9-tetrahydro-5H-5,8-epiminocyclohepta[d]pyridazine-10-carboxamide
(*5S,8R*)-*N*-(5-chloro-2-fluoro-4-(trifluoromethyl)phenyl)-2-fluoro-6,7,8,9-tetrahydro-5H-5,8-epiminocyclohepta[b]pyridine-10-carboxamide
(*5S,8R*)-*N*-(5-chloro-2-fluoro-4-(trifluoromethyl)phenyl)-2-fluoro-6,7,8,9-tetrahydro-5H-5,8-epiminocyclohepta[b]pyridine-10-carboxamide
(*6S,9R*)-*N*-(3,4-dichlorophenyl)-3-fluoro-6,7,8,9-tetrahydro-5H-6,9-epiminocyclohepta[c]pyridine-10-carboxamide
(5S, *8R*)-*N*-(4-chloro-3-cyanophenyl)-1-fluoro-6,7,8,9-tetrahydro-5H-5,8-epiminocyclohepta[c]pyridine-10-carboxamide
(*5R,8S*)-*N*-(4,5-dichloro-2-fluorophenyl)-1-(trifluoromethyl)-6,7,8,9-tetrahydro-5H-5,8-epiminocyclohepta[c]pyridine-10-carboxamide
(*5S,8R*)-*N*-(4,5-dichloro-2-fluorophenyl)-1-(trifluoromethyl)-6,7,8,9-tetrahydro-5H-5,8-epiminocyclohepta[c]pyridine-10-carboxamide
(*5R,8S*)-*N*-(3-Chloro-4-(trifluoromethyl)phenyl)-6,7,8,9-tetrahydro-5H-5,8-epiminocyclohepta[d]pyrimidine-10-carboxamide
(*5S,8R*)-*N*-(3-Chloro-4-(trifluoromethyl)phenyl)-6,7,8,9-tetrahydro-5H-5,8-epiminocyclohepta[d]pyrimidine-10-carboxamide
(*5R,8S*)-*N*-(4-Chloro-3-(trifluoromethyl)phenyl)-6,7,8,9-tetrahydro-5H-5,8-epiminocyclohepta[d]pyrimidine-10-carboxamide
(*5S,8R*)-*N*-(4-Chloro-3-(trifluoromethyl)phenyl)-6,7,8,9-tetrahydro-5H-5,8-epiminocyclohepta[d]pyrimidine-10-carboxamide
(*5S,8R*)-*N-*(4-Fluoro-3-(trifluoromethyl)phenyl)-6,7,8,9-tetrahydro-5H-5,8-epiminocyclohepta[d]pyrimidine-10-carboxamide
(*5R,8S*)-*N*-(4-Fluoro-3-(trifluoromethyl)phenyl)-6,7,8,9-tetrahydro-5H-5,8-epiminocyclohepta[d]pyrimidine-10-carboxamide
(5R, *8S*)-*N*-(3-chloro-4-cyanophenyl)-6,7,8,9-tetrahydro-5H-5,8-epiminocyclohepta[d]-pyrimidine-10-carboxamide
(*5R,8S*)-*N*-(3-chloro-4-(trifluoromethyl)phenyl)-6,7,8,9-tetrahydro-5H-5,8-epiminocyclohepta[c]pyridine-10-carboxamide
(*5S,8R*)-*N*-(3-chloro-4-(trifluoromethyl)phenyl)-6,7,8,9-tetrahydro-5H-5,8-epiminocyclohepta[c]pyridine-10-carboxamide
(*5R,8S,9S*)-*N*-(3,4-Dichlorophenyl)-9-fluoro-6,7,8,9-tetrahydro-5H-5,8-epiminocyclohepta[d]pyrimidine-10-carboxamide
(*5R,8S,E*)-*N*-(3-chloro-4-(trifluoromethyl)phenyl)-9-(hydroxyimino)-2-oxo-3,5,6,7,8,9-hexahydro-2H-5,8-epiminocyclohepta[d]pyrimidine-10-carboxamide
(*5S,8R,E*)-*N*-(3-chloro-4-(trifluoromethyl)phenyl)-9-(hydroxyimino)-2-oxo-3,5,6,7,8,9-hexahydro-2H-5,8-epiminocyclohepta[d]pyrimidine-10-carboxamide
(*5S,8R*)-*N*-(4,5-Dichloro-2-cyanophenyl)-1-fluoro-6,7,8,9-tetrahydro-5H-5,8-epiminocyclohepta[c]pyridine-10-carboxamide
(*5R,8S*)-*N*-(4,5-Dichloro-2-cyanophenyl)-1-fluoro-6,7,8,9-tetrahydro-5H-5,8-epiminocyclohepta[c]pyridine-10-carboxamide
methyl 2-chloro-4-((*6S,9R*)-3-oxo-3,5,6,7,8,9-hexahydro-2H-6,9-epiminocyclohepta[c]pyridine-10-carboxamido)benzoate
methyl 2-chloro-4-((*6R,9S*)-3-oxo-3,5,6,7,8,9-hexahydro-2H-6,9-epiminocyclohepta[c]pyridine-10-carboxamido)benzoate
and pharmaceutically acceptable salts and solvates thereof.

In one preferred embodiment, the compound, or compound for use, according to the invention exhibits an IC50 of > 500 nM and < 5 µM in a Human GPR65 cyclic adenosine monophosphate (cAMP) Homogeneous Time Resolved Fluorescence (HTRF) antagonist assay as described in the accompanying examples. In one preferred embodiment, the compound is selected from those denoted "high" or "medium" in Table 1.

In a more preferred embodiment, the compound, or compound for use, according to the invention exhibits an IC50 of < 500 nM in a Human GPR65 cAMP HTRF antagonist assay as described in the accompanying examples. In one preferred embodiment, the compound is selected from those denoted "high" in Table 1.

### PHARMACEUTICAL COMPOSITIONS

For use according to the present invention, the compounds or physiologically acceptable salt, ester or other physiologically functional derivative thereof, described herein, may be presented as a pharmaceutical formulation, comprising the compounds or physiologically acceptable salt, ester or other physiologically functional derivative thereof, together with one or more pharmaceutically acceptable carriers, excipients or diluents therefor and optionally other therapeutic and/or prophylactic ingredients. The carrier(s) must be acceptable in the sense of being compatible with the other ingredients of the formulation and not deleterious to the recipient thereof. The pharmaceutical compositions may be for human or animal usage in human and veterinary medicine.

Examples of such suitable excipients for the various different forms of pharmaceutical compositions described herein may be found in the "Handbook of Pharmaceutical Excipients, 2nd Edition, (1994), Edited by A Wade and PJ Weller. The carrier, or, if more than one be present, each of the carriers, must be acceptable in the sense of being compatible with the other ingredients of the formulation and not deleterious to the recipient.

Acceptable carriers or diluents for therapeutic use are well known in the pharmaceutical art, and are described, for example, in Remington's Pharmaceutical Sciences, Mack Publishing Co. (A. R. Gennaro edit. 1985).

Examples of suitable carriers include lactose, starch, glucose, methyl cellulose, magnesium stearate, mannitol, sorbitol and the like. Examples of suitable diluents include ethanol, glycerol and water.

The choice of pharmaceutical carrier, excipient or diluent can be selected with regard to the intended route of administration and standard pharmaceutical practice. The pharmaceutical compositions may comprise as, or in addition to, the carrier, excipient or diluent any suitable binder(s), lubricant(s), suspending agent(s), coating agent(s), solubilising agent(s), buffer(s), flavouring agent(s), surface active agent(s), thickener(s), preservative(s) (including anti-oxidants) and the like, and substances included for the purpose of rendering the formulation isotonic with the blood of the intended recipient.

Examples of suitable binders include starch, gelatin, natural sugars such as glucose, anhydrous lactose, free-flow lactose, beta-lactose, corn sweeteners, natural and synthetic gums, such as acacia, tragacanth or sodium alginate, carboxymethyl cellulose and polyethylene glycol.

Examples of suitable lubricants include sodium oleate, sodium stearate, magnesium stearate, sodium benzoate, sodium acetate, sodium chloride and the like.

Preservatives, stabilizers, dyes and even flavoring agents may be provided in the pharmaceutical composition. Examples of preservatives include sodium benzoate, sorbic acid and esters of p-hydroxybenzoic acid. Antioxidants and suspending agents may be also used.

Pharmaceutical formulations include those suitable for oral, topical (including dermal, buccal and sublingual), rectal or parenteral (including subcutaneous, intradermal, intramuscular and intravenous), nasal and pulmonary administration e.g., by inhalation. The formulation may, where appropriate, be conveniently presented in discrete dosage units and may be prepared by any of the methods well known in the art of pharmacy. All methods include the step of bringing into association an active compound with liquid carriers or finely divided solid carriers or both and then, if necessary, shaping the product into the desired formulation.

Pharmaceutical formulations suitable for oral administration wherein the carrier is a solid are most preferably presented as unit dose formulations such as boluses, capsules or tablets each containing a predetermined amount of active compound. A tablet may be made by compression or moulding, optionally with one or more accessory ingredients. Compressed tablets may be prepared by compressing in a suitable machine an active compound in a free-flowing form such as a powder or granules optionally mixed with a binder, lubricant, inert diluent, lubricating agent, surface-active agent or dispersing agent. Moulded tablets may be made by moulding an active compound with an inert liquid diluent. Tablets may be optionally coated and, if uncoated, may optionally be scored. Capsules may be prepared by filling an active compound, either alone or in admixture with one or more accessory ingredients, into the capsule shells and then sealing them in the usual manner. Cachets are analogous to capsules wherein an active compound together with any accessory ingredient(s) is sealed in a rice paper envelope. An active compound may also be formulated as dispersible granules, which may for example be suspended in water before administration, or sprinkled on food. The granules may be packaged, e.g., in a sachet. Formulations suitable for oral administration wherein the carrier is a liquid may be presented as a solution or a suspension in an aqueous or non-aqueous liquid, or as an oil-in-water liquid emulsion.

Formulations for oral administration include controlled release dosage forms, e.g., tablets wherein an active compound is formulated in an appropriate release - controlling matrix, or is coated with a suitable release - controlling film. Such formulations may be particularly convenient for prophylactic use.

Pharmaceutical formulations suitable for rectal administration wherein the carrier is a solid are most preferably presented as unit dose suppositories. Suitable carriers include cocoa butter and other materials commonly used in the art. The suppositories may be conveniently formed by admixture of an active compound with the softened or melted carrier(s) followed by chilling and shaping in moulds. Pharmaceutical formulations suitable for parenteral administration include sterile solutions or suspensions of an active compound in aqueous or oleaginous vehicles.

Injectable preparations may be adapted for bolus injection or continuous infusion. Such preparations are conveniently presented in unit dose or multi-dose containers which are sealed after introduction of the formulation until required for use. Alternatively, an active compound may be in powder form which is constituted with a suitable vehicle, such as sterile, pyrogen-free water, before use.

An active compound may also be formulated as long-acting depot preparations, which may be administered by intramuscular injection or by implantation, e.g., subcutaneously or intramuscularly. Depot preparations may include, for example, suitable polymeric or hydrophobic materials, or ion-exchange resins. Such long-acting formulations are particularly convenient for prophylactic use.

Formulations suitable for pulmonary administration via the buccal cavity are presented such that particles containing an active compound and desirably having a diameter in the range of 0.5 to 7 microns are delivered in the bronchial tree of the recipient.

As one possibility such formulations are in the form of finely comminuted powders which may conveniently be presented either in a pierceable capsule, suitably of, for example, gelatin, for use in an inhalation device, or alternatively as a self-propelling formulation comprising an active compound, a suitable liquid or gaseous propellant and optionally other ingredients such as a surfactant and/or a solid diluent. Suitable liquid propellants include propane and the chlorofluorocarbons, and suitable gaseous propellants include carbon dioxide. Self-propelling formulations may also be employed wherein an active compound is dispensed in the form of droplets of solution or suspension.

Such self-propelling formulations are analogous to those known in the art and may be prepared by established procedures. Suitably they are presented in a container provided with either a manually-operable or automatically functioning valve having the desired spray characteristics; advantageously the valve is of a metered type delivering a fixed volume, for example, 25 to 100 microlitres, upon each operation thereof.

As a further possibility an active compound may be in the form of a solution or suspension for use in an atomizer or nebuliser whereby an accelerated airstream or ultrasonic agitation is employed to produce a fine droplet mist for inhalation.

Formulations suitable for nasal administration include preparations generally similar to those described above for pulmonary administration. When dispensed such formulations should desirably have a particle diameter in the range 10 to 200 microns to enable retention in the nasal cavity; this may be achieved by, as appropriate, use of a powder of a suitable particle size or choice of an appropriate valve. Other suitable formulations include coarse powders having a particle diameter in the range 20 to 500 microns, for administration by rapid inhalation through the nasal passage from a container held close up to the nose, and nasal drops comprising 0.2 to 5% w/v of an active compound in aqueous or oily solution or suspension.

Pharmaceutically acceptable carriers are well known to those skilled in the art and include, but are not limited to, 0.1 M and preferably 0.05 M phosphate buffer or 0.8% saline. Additionally, such pharmaceutically acceptable carriers may be aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's or fixed oils. Preservatives and other additives may also be present, such as, for example, antimicrobials, antioxidants, chelating agents, inert gases and the like.

Formulations suitable for topical formulation may be provided for example as gels, creams or ointments. Such preparations may be applied e.g. to a wound or ulcer either directly spread upon the surface of the wound or ulcer or carried on a suitable support such as a bandage, gauze, mesh or the like which may be applied to and over the area to be treated.

Liquid or powder formulations may also be provided which can be sprayed or sprinkled directly onto the site to be treated, e.g. a wound or ulcer. Alternatively, a carrier such as a bandage, gauze, mesh or the like can be sprayed or sprinkle with the formulation and then applied to the site to be treated.

According to a further aspect of the invention, there is provided a process for the preparation of a pharmaceutical or veterinary composition as described above, the process comprising bringing the active compound(s) into association with the carrier, for example by admixture.

In general, the formulations are prepared by uniformly and intimately bringing into association the active agent with liquid carriers or finely divided solid carriers or both, and then if necessary shaping the product. The invention extends to methods for preparing a pharmaceutical composition comprising bringing a compound as described herein into conjunction or association with a pharmaceutically or veterinarily acceptable carrier or vehicle.

### SALTS/ESTERS

The compounds of the invention can be present as salts or esters, in particular pharmaceutically and veterinarily acceptable salts or esters.

Pharmaceutically acceptable salts of the compounds of the invention include suitable acid addition or base salts thereof. A review of suitable pharmaceutical salts may be found in Berge et al, J Pharm Sci, 66, 1-19 (1977). Salts are formed, for example with strong inorganic acids such as mineral acids, e.g. hydrohalic acids such as hydrochloride, hydrobromide and hydroiodide, sulphuric acid, phosphoric acid sulphate, bisulphate, hemisulphate, thiocyanate, persulphate and sulphonic acids; with strong organic carboxylic acids, such as alkanecarboxylic acids of 1 to 4 carbon atoms which are unsubstituted or substituted (e.g., by halogen), such as acetic acid; with saturated or unsaturated dicarboxylic acids, for example oxalic, malonic, succinic, maleic, fumaric, phthalic or tetraphthalic; with hydroxycarboxylic acids, for example ascorbic, glycolic, lactic, malic, tartaric or citric acid; with aminoacids, for example aspartic or glutamic acid; with benzoic acid; or with organic sulfonic acids, such as (C₁-C₄)-alkyl- or aryl-sulfonic acids which are unsubstituted or substituted (for example, by a halogen) such as methane- or p-toluene sulfonic acid. Salts which are not pharmaceutically or veterinarily acceptable may still be valuable as intermediates.

Preferred salts include, for example, acetate, trifluoroacetate, lactate, gluconate, citrate, tartrate, maleate, malate, pantothenate, adipate, alginate, aspartate, benzoate, butyrate, digluconate, cyclopentanate, glucoheptanate, glycerophosphate, oxalate, heptanoate, hexanoate, fumarate, nicotinate, palmoate, pectinate, 3-phenylpropionate, picrate, pivalate, proprionate, tartrate, lactobionate, pivolate, camphorate, undecanoate and succinate, organic sulphonic acids such as methanesulphonate, ethanesulphonate, 2-hydroxyethane sulphonate, camphorsulphonate, 2-naphthalenesulphonate, benzenesulphonate, p-chlorobenzenesulphonate and p-toluenesulphonate; and inorganic acids such as hydrochloride, hydrobromide, hydroiodide, sulphate, bisulphate, hemisulphate, thiocyanate, persulphate, phosphoric and sulphonic acids.

Esters are formed either using organic acids or alcohols/hydroxides, depending on the functional group being esterified. Organic acids include carboxylic acids, such as alkanecarboxylic acids of 1 to 12 carbon atoms which are unsubstituted or substituted (e.g., by halogen), such as acetic acid; with saturated or unsaturated dicarboxylic acid, for example oxalic, malonic, succinic, maleic, fumaric, phthalic or tetraphthalic; with hydroxycarboxylic acids, for example ascorbic, glycolic, lactic, malic, tartaric or citric acid; with aminoacids, for example aspartic or glutamic acid; with benzoic acid; or with organic sulfonic acids, such as (C₁-C₄)-alkyl- or aryl-sulfonic acids which are unsubstituted or substituted (for example, by a halogen) such as methane- or p-toluene sulfonic acid. Suitable hydroxides include inorganic hydroxides, such as sodium hydroxide, potassium hydroxide, calcium hydroxide, aluminium hydroxide. Alcohols include alkanealcohols of 1-12 carbon atoms which may be unsubstituted or substituted, e.g. by a halogen).

### ENANTIOMERS/TAUTOMERS

In all aspects of the present invention previously discussed, the invention includes, where appropriate all enantiomers, diastereoisomers and tautomers of the compounds of the invention. The person skilled in the art will recognise compounds that possess optical properties (one or more chiral carbon atoms) or tautomeric characteristics. The corresponding enantiomers and/or tautomers may be isolated/prepared by methods known in the art.

Enantiomers are characterised by the absolute configuration of their chiral centres and described by the R- and S-sequencing rules of Cahn, Ingold and Prelog. Such conventions are well known in the art (e.g. see 'Advanced Organic Chemistry', 3rd edition, ed. March, J., John Wiley and Sons, New York, 1985).

Compounds of the invention containing a chiral centre may be used as a racemic mixture, an enantiomerically enriched mixture, or the racemic mixture may be separated using well-known techniques and an individual enantiomer may be used alone.

### STEREO ISOMERS

Some of the compounds of the invention may exist as stereoisomers e.g. they may possess one or more asymmetric centres and so may exist in two or more stereoisomeric forms. The present invention contemplates the use of all the individual stereoisomers of those compounds, and mixtures thereof. The terms used in the claims encompass these forms, provided said forms retain the appropriate functional activity (though not necessarily to the same degree).

The present invention also includes all suitable isotopic variations of the compound or a pharmaceutically acceptable salt thereof. An isotopic variation of a compound of the present invention or a pharmaceutically acceptable salt thereof is defined as one in which at least one atom is replaced by an atom having the same atomic number but an atomic mass different from the atomic mass usually found in nature. Examples of isotopes that can be incorporated into the agent and pharmaceutically acceptable salts thereof include isotopes of hydrogen, carbon, nitrogen, oxygen, phosphorus, sulphur, fluorine and chlorine such as ²H, ³H, ¹³C, ¹⁴C, ¹⁵N, ¹⁷O, ¹⁸O, ³¹P, ³²P, ³⁵S, ¹⁸F and ³⁶Cl, respectively. Certain isotopic variations of the agent and pharmaceutically acceptable salts thereof, for example, those in which a radioactive isotope such as ³H or ¹⁴C is incorporated, are useful in drug and/or substrate tissue distribution studies. Tritiated, i.e., ³H, and carbon-14, i.e., ¹⁴C, isotopes are particularly preferred for their ease of preparation and detectability. Further, substitution with isotopes such as deuterium, i.e., ²H, may afford certain therapeutic advantages resulting from greater metabolic stability, for example, increased *in vivo* half-life or reduced dosage requirements and hence may be preferred in some circumstances. For example, the invention includes compounds of general formula (I) where any hydrogen atom has been replaced by a deuterium atom. Isotopic variations of the agent of the present invention and pharmaceutically acceptable salts thereof of this invention can generally be prepared by conventional procedures using appropriate isotopic variations of suitable reagents.

### ATROPISOMERS

Some of the compounds of the invention may exist as atropisomers. Atropisomers are stereoisomers arising because of hindered rotation about a single bond, where energy differences due to steric strain or other contributors create a barrier to rotation that is high enough to allow for isolation of individual conformers. The invention encompasses all such atropisomers. The invention also covers rotamers of the compounds.

### PRODRUGS

The invention further includes the compounds of the present invention in prodrug form, i.e. covalently bonded compounds which release the active parent drug *in vivo.* Such prodrugs are generally compounds of the invention wherein one or more appropriate groups have been modified such that the modification may be reversed upon administration to a human or mammalian subject. Reversion is usually performed by an enzyme naturally present in such subject, though it is possible for a second agent to be administered together with such a prodrug in order to perform the reversion *in vivo.* Examples of such modifications include ester (for example, any of those described above), wherein the reversion may be carried out be an esterase etc. Other such systems will be well known to those skilled in the art.

### SOLVATES

The present invention also includes solvate forms of the compounds of the present invention. The terms used in the claims encompass these forms. Preferably, the solvate is a hydrate.

### COMBINATIONS

A further aspect of the inventiont relates to a combination comprising a compound as described herein and one or more additional active agents. In a particularly preferred embodiment, the one or more compounds of the invention are administered in combination with one or more additional active agents, for example, existing drugs available on the market. In such cases, the compounds of the invention may be administered consecutively, simultaneously or sequentially with the one or more other active agents.

Drugs in general are more effective when used in combination. In particular, combination therapy is desirable in order to avoid an overlap of major toxicities, mechanism of action and resistance mechanism(s). Furthermore, it is also desirable to administer most drugs at their maximum tolerated doses with minimum time intervals between such doses. The major advantages of combining chemotherapeutic drugs are that it may promote additive or possible synergistic effects through biochemical interactions and also may decrease the emergence of resistance.

Beneficial combinations may be suggested by studying the activity of the test compounds with agents known or suspected of being valuable in the treatment of a particular disorder. This procedure can also be used to determine the order of administration of the agents, i.e. before, simultaneously, or after delivery. Such scheduling may be a feature of all the active agents identified herein.

In the context of cancer, compounds of the invention can be used in combination with immunotherapies such as cancer vaccines and/or with other immune-modulators such as agents that block the PD1/PDL-1 interaction. Thus, inone preferred embodiment, the additional active agent is an immunotherapy agent, more preferably a cancer immunotherapy agent. An "immunotherapy agent" refers to a treatment that uses the subject's own immune system to fight diseases such as cancer. For other disorders the compounds of the invention can be used in combination agents that block or decrease inflammation such as antibodies that target pro-inflammatory cytokines.

### POLYMORPHS

The invention further relates to the compounds of the present invention in their various crystalline forms, polymorphic forms and (an)hydrous forms. It is well established within the pharmaceutical industry that chemical compounds may be isolated in any of such forms by slightly varying the method of purification and or isolation form the solvents used in the synthetic preparation of such compounds.

### ADMINISTRATION

The pharmaceutical compositions of the present invention may be adapted for rectal, nasal, intrabronchial, topical (including buccal and sublingual), vaginal or parenteral (including subcutaneous, intramuscular, intravenous, intraarterial and intradermal), intraperitoneal or intrathecal administration. Preferably the formulation is an orally administered formulation. The formulations may conveniently be presented in unit dosage form, i.e., in the form of discrete portions containing a unit dose, or a multiple or sub-unit of a unit dose. By way of example, the formulations may be in the form of tablets and sustained release capsules, and may be prepared by any method well known in the art of pharmacy.

Formulations for oral administration in the present invention may be presented as: discrete units such as capsules, gellules, drops, cachets, pills or tablets each containing a predetermined amount of the active agent; as a powder or granules; as a solution, emulsion or a suspension of the active agent in an aqueous liquid or a non-aqueous liquid; or as an oil-in-water liquid emulsion or a water-in-oil liquid emulsion; or as a bolus etc. Preferably, these compositions contain from 1 to 250 mg and more preferably from 10-100 mg, of active ingredient per dose.

For compositions for oral administration (e.g. tablets and capsules), the term "acceptable carrier" includes vehicles such as common excipients e.g. binding agents, for example syrup, acacia, gelatin, sorbitol, tragacanth, polyvinylpyrrolidone (Povidone), methylcellulose, ethylcellulose, sodium carboxymethylcellulose, hydroxypropyl-methylcellulose, sucrose and starch; fillers and carriers, for example corn starch, gelatin, lactose, sucrose, microcrystalline cellulose, kaolin, mannitol, dicalcium phosphate, sodium chloride and alginic acid; and lubricants such as magnesium stearate, sodium stearate and other metallic stearates, glycerol stearate stearic acid, silicone fluid, talc waxes, oils and colloidal silica. Flavouring agents such as peppermint, oil of wintergreen, cherry flavouring and the like can also be used. It may be desirable to add a colouring agent to make the dosage form readily identifiable. Tablets may also be coated by methods well known in the art.

A tablet may be made by compression or moulding, optionally with one or more accessory ingredients. Compressed tablets may be prepared by compressing in a suitable machine the active agent in a free flowing form such as a powder or granules, optionally mixed with a binder, lubricant, inert diluent, preservative, surface-active or dispersing agent. Moulded tablets may be made by moulding in a suitable machine a mixture of the powdered compound moistened with an inert liquid diluent. The tablets may be optionally be coated or scored and may be formulated so as to provide slow or controlled release of the active agent.

Other formulations suitable for oral administration include lozenges comprising the active agent in a flavoured base, usually sucrose and acacia or tragacanth; pastilles comprising the active agent in an inert base such as gelatin and glycerin, or sucrose and acacia; and mouthwashes comprising the active agent in a suitable liquid carrier.

Other forms of administration comprise solutions or emulsions which may be injected intravenously, intraarterially, intrathecally, subcutaneously, intradermally, intraperitoneally or intramuscularly, and which are prepared from sterile or sterilisable solutions. Injectable forms typically contain between 10 - 1000 mg, preferably between 10 - 250 mg, of active ingredient per dose.

The pharmaceutical compositions of the present invention may also be in form of suppositories, pessaries, suspensions, emulsions, lotions, ointments, creams, gels, sprays, solutions or dusting powders.

An alternative means of transdermal administration is by use of a skin patch. For example, the active ingredient can be incorporated into a cream consisting of an aqueous emulsion of polyethylene glycols or liquid paraffin. The active ingredient can also be incorporated, at a concentration of between 1 and 10% by weight, into an ointment consisting of a white wax or white soft paraffin base together with such stabilisers and preservatives as may be required.

### DOSAGE

A person of ordinary skill in the art can easily determine an appropriate dose of one of the instant compositions to administer to a subject without undue experimentation. Typically, a physician will determine the actual dosage which will be most suitable for an individual patient and it will depend on a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy. The dosages disclosed herein are exemplary of the average case. There can of course be individual instances where higher or lower dosage ranges are merited, and such are within the scope of this invention.

The dosage amount will further be modified according to the mode of administration of the compound. For example, to achieve an "effective amount" for acute therapy, parenteral administration of a compound is typically preferred. An intravenous infusion of the compound in 5% dextrose in water or normal saline, or a similar formulation with suitable excipients, is most effective, although an intramuscular bolus injection is also useful. Typically, the parenteral dose will be about 0.01 to about 100 mg; preferably between 0.1 and 20 mg, in a manner to maintain the concentration of drug in the plasma at a concentration effective to modulate GPR65. The compounds may be administered one to four times daily at a level to achieve a total daily dose of about 0.4 to about 400 mg. The precise amount of an inventive compound which is therapeutically effective, and the route by which such compound is best administered, is readily determined by one of ordinary skill in the art by comparing the blood level of the agent to the concentration required to have a therapeutic effect.

The compounds of this invention may also be administered orally to the patient, in a manner such that the concentration of drug is sufficient to achieve one or more of the therapeutic indications disclosed herein. Typically, a pharmaceutical composition containing the compound is administered at an oral dose of between about 0.1 to about 500 mg or about 0.1 to about 50 mg in a manner consistent with the condition of the patient. Preferably the oral dose would be about 0.5 to about 50 mg or about 0.5 to about 20 mg.

No unacceptable toxicological effects are expected when compounds of the present invention are administered in accordance with the present invention. The compounds of this invention, which may have good bioavailability, may be tested in one of several biological assays to determine the concentration of a compound which is required to have a given pharmacological effect.

The invention is further described with reference to the following figures, and non-limiting examples.

### FIGURES

Figure 1 depicts the solved X-ray crystal structure for (5R,8S)-N-(3,4-dichlorophenyl)-6,7,8,9-tetrahydro-5H-5,8-epiminocyclohepta[d]pyrimidine-10-carboxamide (compound 52).
Figure 2 depicts the solved X-ray crystal structure for (5S,8R)-N-(3,4-dichlorophenyl)-6,7,8,9-tetrahydro-5H-5,8-epiminocyclohepta[d]pyrimidine-10-carboxamide (compound 53).

### EXAMPLES

Where the preparation of starting materials is not described, these are commercially available, known in the literature, or readily obtainable by those skilled in the art using standard procedures. Where it is indicated that compounds were prepared analogously to earlier examples or intermediates, it will be appreciated by the skilled person that the reaction time, number of equivalents of reagents, solvent, concentration and temperature can be modified for each specific reaction and that it may be necessary or desirable to employ different work-up or purification techniques.

### General Schemes

### Abbreviations

A list of some common abbreviations is shown below - where other abbreviations are used which are not listed, these will be understood by the person skilled in the art.

AcOH: Acetic acid; Boc: tert-butyloxycarbonyl br.: broad; CAN: ceric ammonium nitrate; d: doublet; DCM: dichloromethane; DIPEA: N,N-diisopropylethylamine; DMF: N,N-dimethylformamide; DMSO: dimethylsulfoxide; (ES+): electrospray ionization positive mode; Et₃N: triethylamine; EtOAc: ethyl acetate; EtOH: ethanol; h: hours; HPLC: high performance liquid chromatography; HCl: hydrochloric acid; Hz: hertz; IPA: iso-propyl alcohol; J: coupling constant; I: litre; LDA: lithium diisopropylamide; M: molar; m: multiplet [M+H]+: protonated molecular ion; mCPBA: meta-chloroperoxybenzoic acid; MeCN: acetonitrile; MeOH: methanol; MHz: megahertz; min: minutes; ml: millilitres; MS: mass spectrometry; MTBE: methyl *tert-butyl* ether; m/z: mass-to-charge ratio; NFSI: N-Fluorobenzenesulfonimide; NMR: nuclear magnetic resonance; Pd-170: chloro(crotyl)(2-dicyclohexylphosphino-2',4',6'-triisopropyl-1,1'-biphenyl)palladium(ll); Pd-172: chloro(crotyl)(2-dicyclohexylphosphino-2',6'-dimethoxy-1,1'-biphenyl)palladium(II); Pd-175: allyl(2-di-tert-butylphosphino-3,6-dimethoxy-2',4',6'-triisopropyl-1,1'-biphenyl)palladium(II) triflate Pd-178: chloro(crotyl)(tricyclohexylphosphine)palladium(II); Pd₂(dba)₃:Tris(dibenzylideneacetone)dipalladium(0); PDA: photodiode array; PMP: *para-*methoxyphenyl; RT: room temperature; Rt: retention time; s: singlet; SFC: supercritical fluid chromatography; SCX: solid supported cation exchange (resin); S-Phos: 2-dicyclohexylphosphino-2',6'-dimethoxybiphenyl; t:triplet; TFA: trifluoroacetic acid; THF: tetrahydrofuran; TLC: thin layer chromatography; UPLC: ultra performance liquid chromatography; UV: ultra-violet.

Other abbreviations are intended to convey their generally accepted meaning.

### General experimental conditions

All starting materials and solvents were obtained either from commercial sources or prepared according to literature methods. The appropriate isocyanate and aniline starting materials were obtained from Sigma Aldrich, Fluorochem or Enamine store, or were synthesised as described herein. The appropriate tricyclic amine starting materials were obtained from Enamine store or were synthesised as described herein. Reaction mixtures were magnetically stirred and reactions performed at room temperature (approximately 20 °C) unless otherwise indicated.

Silica gel chromatography was performed on an automated flash chromatography system, such as CombiFlash Companion, CombiFlash Rf system or Reveleris X2 flash system using RediSep^{®} Rf or Reveleris^{®} or the GraceResolv^{™} pre-packed silica (230-400 mesh, 40-63 µm) cartridges.

Analytical UPLC-MS experiments to determine retention times and associated mass ions were performed using a Waters ACQUITY UPLC^{®} H-Class system, equipped with ACQUITY PDA Detector and ACQUITY QDa mass spectrometer or Waters SQD mass spectrometer, running the analytical method described below.

Analytical LC-MS experiments to determine retention times and associated mass ions were performed using an Agilent 1200 series HPLC system coupled to an Agilent 1956, 6100 or 6120 series single quadrupole mass spectrometer running one of the analytical methods described below or a Shimadzu-2020-P2 system consisting of a Shimadzu LC-20AD series LC system and a Shimadzu-2020, single quadrupole mass spectrometer running one of the analytical methods described below

Analytical SFC experiments to determine retention times were performed using a Waters SFC system UPC2 system with a column temperature of 40 °C and a back pressure (ABPR) of 1750 psi using one of the analytical methods described below.

Preparative HPLC purifications were performed either using either a Waters X-Bridge BEH C18, 5 µm, 19 x 50 mm column, or a Waters Xbridge Prep OBD C18, 10 µm, 40 x 150 mm column, or a Phenomenex Gemini-NX C18, 3 µm, 30 x 75 mm column using a gradient of MeCN and 10 mM aqueous ammonium bicarbonate. Fractions were collected following UV detection across all wavelengths with PDA and in some cases an SQD2 or ACQUITY QDa mass spectrometer.

Preparative SFC purifications were performed using a Waters SFC prep 15 system with either a: Phenomenex Lux^{®} Cellulose-4, Column 1 x 25 cm, 5 µm particle size column or a Chiralpak^{®} IG (Daicel Ltd.) column (1 x 25 cm, 5 µm particle size), flow rate 15ml/min eluting with a mixture of CO₂ and co-solvent (MeOH, EtOH or IPA). Fractions were collected following UV detection at 210 - 400 nm using a PDA.

NMR spectra were recorded using either a Bruker Avance III HD 500 MHz instrument or a Bruker Avance Neo 400 MHz, using either residual non-deuterated solvent, or tetra-methylsilane as reference or Varian Y 400 MHz instrument, using tetra-methylsilane as reference, or a QOne AS400 400 MHz spectrometer using either residual non-deuterated solvent, or tetra-methylsilane as reference.

In the absence of the absolute stereochemistry being explicitly indicated through wedged and dashed bonds, chemical structures disclosed throughout the examples (for example, in the configuration (I.3) described hereinabove) are to be interpreted as depicting the racemate. For the avoidance of doubt, the invention encompasses the compounds in either configuration, as well as mixtures thereof.

### Analytical methods

### Method 1 - Basic 3 min method

| | |
|---|---|
| Column: | Waters ACQUITY UPLC^{®} BEH C18, 1.7 µm, 2.1 x 30 mm at 40 °C |
| Detection: | UV at 210-400 nm unless otherwise indicated, MS by electrospray ionisation |
| Solvents: | A: 10 mM aqueous ammonium bicarbonate, B: MeCN |

### Gradient:

| Time | %A | %B | Flow rate (ml/min) |
|---|---|---|---|
| 0.00 | 95 | 5 | 0.77 |
| 0.11 | 95 | 5 | 0.77 |
| 2.15 | 5 | 95 | 0.77 |
| 2.56 | 5 | 95 | 0.77 |
| 2.83 | 95 | 5 | 0.77 |
| 3.00 | 95 | 5 | 0.77 |

### Method 2 - Basic 4 min method

| | |
|---|---|
| Column: | Waters X-Bridge BEH C18, 2.5 µm, 4.6 x 30 mm at 40 °C |
| Detection: | UV at 254 nm unless otherwise indicated, MS by electrospray ionisation |
| Solvents: | A: 10 mM aqueous ammonium bicarbonate B: MeCN |

### Gradient:

| Time | %A | %B | Flow rate (ml/min) |
|---|---|---|---|
| 0.0 | 95.0 | 5.0 | 2.5 |
| 3.0 | 5.0 | 95.0 | 2.5 |
| 3.01 | 5.0 | 95.0 | 4.5 |
| 3.6 | 5.0 | 95.0 | 4.5 |
| 3.7 | 95.0 | 5.0 | 2.5 |
| 4.0 | 95.0 | 5.0 | 2.5 |

### Method 3 - Lux^{®} Amylose-2

| | |
|---|---|
| Column: | Lux^{®} Amylose-2, LC column (150 x 2 mm, 3 µm particle size) at 40 °C and a flow rate of 1.5 ml/min |
| Detection: | UV detection by DAD at 220 - 400 nm |
| Solvents: | gradient or an isocratic mixture of a polar solvent, usually methanol, ethanol or IPA in CO₂: |

### Method 4 - CHIRALPAK^{®}IG-3 (Daicel Ltd.) column

| | |
|---|---|
| Column: | CHIRALPAK^{®}IG-3 (Daicel Ltd.) column (2.1 x 150 mm, 3 µm particle size) and a flow rate of 1.5 ml/min |
| Detection: | UV detection by DAD at 220 - 400nm |
| Solvents: | gradient or an isocratic mixture of a polar solvent, usually methanol, ethanol or IPA in CO₂: |

### Method 5 - Basic 4 min method

| | |
|---|---|
| Column: | Gemini LC Column C18 50 x 2 mm, 3 µm particle size at 40 °C |
| Detection: | UV at 220 nm unless otherwise indicated, MS by electrospray ionisation |
| Solvents: | A: 10 mM aqueous ammonium bicarbonate, B: MeCN |

### Gradient:

| Time | %A | %B | Flow rate (ml/min) |
|---|---|---|---|
| 0.0 | 100 | 0 | 0.6 |
| 0.4 | 100 | 0 | 0.6 |
| 3.00 | 10 | 90.0 | 0.6 |
| 3.85 | 0 | 100 | 0.6 |
| 3.86 | 100 | 0 | 0.6 |
| 4.0 | 100 | 0 | 0.6 |

### Method 6 - Basic 4 min method

| | |
|---|---|
| Column: | Waters Sunfire, 3.5 µm, 4.6 × 50 mm column at 25 °C |
| Detection: | ionisation UV at 214 and 254 nm unless otherwise indicated, MS by electrospray |
| Solvents: | A: 0.05% formic acid in water (*v*/*v*) B: 0.05% formic acid MeCN (*v*/*v*) |

### Gradient:

| Time | %A | %B | Flow rate (ml/min) |
|---|---|---|---|
| 0.00 | 85 | 15 | 1.0 |
| 0.50 | 85 | 15 | 1.0 |
| 4.00 | 0 | 100 | 1.0 |
| 4.50 | 0 | 100 | 1.0 |
| 4.51 | 85 | 15 | 1.0 |
| 5.00 | 85 | 15 | 1.0 |

### Experimental scheme 1

### Compound 1 (±)-N-(4-(Trifluoromethyl)phenyl)-6,7,8,9-tetrahydro-5H-5,8-epiminocyclohepta[d]pyrimidine-10-carboxamide

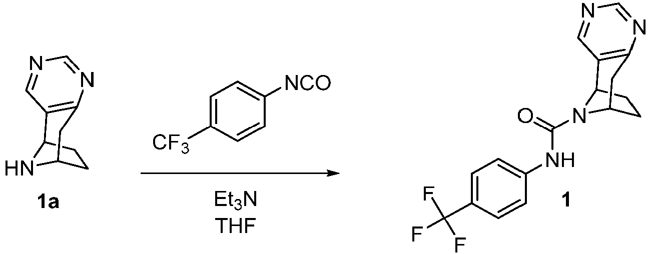

To a solution of 6,7,8,9-tetrahydro-5H-5,8-epiminocyclohepta[d]pyrimidine (0.075 g, 0.465 mmol) and Et₃N (97 µl, 0.698 mmol) in THF (4 ml) was added a solution of 1-isocyanato-4-(trifluoromethyl)benzene (0.131 g, 0.698 mmol) in THF (0.75 ml). The resultant mixture was stirred at RT for 20 h. MeOH (5 ml) was added and the crude product was concentrated *in vacuo.* The product was purified by chromatography on RP Flash C18 (15-75% MeCN/10 mM aqueous ammonium bicarbonate) to afford (±)-N-(4-(trifluoromethyl)phenyl)-6,7,8,9-tetrahydro-5H-5,8-epiminocyclohepta[d]pyrimidine-10-carboxamide as a colourless solid. LC-MS (method 1) m/z 349.3 (M+H)⁺ (ES⁺); at 1.18 min. ¹H NMR (500 MHz, DMSO-d6) δ 9.14 (s, 1H), 8.96 (s, 1H), 8.61 (s, 1H), 7.68 (d, *J* = 8.6 Hz, 2H), 7.58 (d, *J* = 8.6 Hz, 2H), 5.31 (d, *J* = 6.2 Hz, 1H), 4.84 (t, *J =* 6.5 Hz, 1H), 3.36 - 3.33 (m, 1H), 2.79 (d, *J =* 18.4 Hz, 1H), 2.33 - 2.24 (m, 1H), 2.20 (tt, J = 12.2, 6.2 Hz, 1H), 1.90 (ddd, J = 12.0, 9.2, 2.5 Hz, 1H), 1.81 - 1.72 (m, 1H).

The following compounds were prepared using appropriate starting materials in an analogous procedure to that described in Experimental Scheme 1. Where the starting materials are not described in the literature, their synthesis is described below.

Key: (a) Reaction performed in DMF (b) reaction performed in DCM (c) Reaction performed in mixture of DMF and DCM (d) product was purified by mass directed HPLC (MeCN/10 mM aqueous ammonium bicarbonate solution, C18) (e) product was purified by silica gel chromatography (0.7 M Ammonia/MeOH in DCM) (f) reaction performed in DMF/THF (g) product was purified by silica gel chromatography (DCM in heptane) (h) Reaction performed using DIPEA instead of Et₃N (i) product was purified by silica gel chromatography (EtOAc in isohexane) (j) ES⁻ [M-H]⁻, parent mass not observed in ES⁺

| Compound | Structure | LCMS method 1 Rt [M+H]⁺ | NMR |
|---|---|---|---|
| 2^{(a)(d)} | 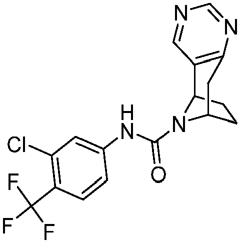 (±)-N-(3-Chloro-4-(trifluoromethyl)phenyl)-6,7,8,9-tetrahydro-5H-5,8-epiminocyclohepta[d]pyrimidine-10-carboxamide | 383.3, 385.3 at 1.31 min | ¹H NMR (500 MHz, DMSO-d6) δ 9.29 (s, 1H), 8.96 (s, 1H), 8.61 (s, 1H), 7.89 (d, *J* = 2.0 Hz, 1H), 7.71 (d, *J* = 8.8 Hz, 1H), 7.62 - 7.57 (m, 1H), 5.31 (d, *J = 6.2* Hz, 1H), 4.84 (t, *J =* 6.5 Hz, 1H), 3.31 (d, *J* = 3.8 Hz, 1H), 2.80 (d, *J* = 18.4 Hz, 1H), 2.34 - 2.15 (m, 2H), 1.95 - 1.86 (m, 1H), 1.81 - 1.69 (m, 1H). |
| 3^{(a)(d)} | 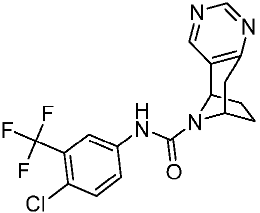 (±)-N-(4-Chloro-3-(trifluoromethyl)phenyl)-6,7,8,9-tetrahydro-5H-5,8-epiminocyclohepta[d]pyrimidine-10-carboxamide | 383.3, 385.3 at 1.29 min | ¹H NMR (500 MHz, DMSO-d6) δ 9.18 (s, 1H), 8.96 (s, 1H), 8.60 (s, 1H), 8.04 (d, *J* = 2.6 Hz, 1H), 7.79 (dd, *J* = 8.8, 2.6 Hz, 1H), 7.57 (d, *J* = 8.8 Hz, 1H), 5.30 (d, *J* = 6.2 Hz, 1H), 4.82 (t, *J* = 6.4 Hz, 1H), 3.31 (d, *J* = 4.8 Hz, 1H), 2.79 (d, *J* = 18.4 Hz, 1H), 2.34 - 2.13 (m, 2H), 1.89 (dd, *J =* 12.1, 2.7 Hz, 1H), 1.77 (dt, *J* = 15.5, 7.7 Hz, 1H). |
| 4^{(a)(d)} | 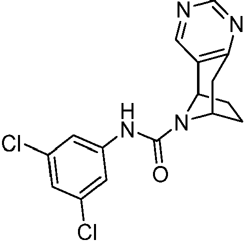 (±)-*N*-(3,5-Dichlorophenyl)-6,7,8,9-tetrahydro-5H-5,8-epiminocyclohepta[d]pyrimidine-10-carboxamide | 349.3, 351.3 at 1.26 min | ¹H NMR (500 MHz, DMSO-d6) δ 9.07 (s, 1H), 8.96 (s, 1H), 8.60 (s, 1H), 7.58 (d, *J* = 1.9 Hz, 2H), 7.14 (t, *J* = 1.9 Hz, 1H), 5.28 (d, *J* = 6.2 Hz, 1H), 4.80 (t, *J* = 6.5 Hz, 1H), 3.32 - 3.29 (m, 1H), 2.78 (d, *J* = 18.4 Hz, 1H), 2.28 (q, *J* = 11.2, 10.7 Hz, 1H), 2.19 (tt, *J* = 12.0, 6.2 Hz, 1H), 1.92 - 1.85 (m, 1H), 1.80 - 1.71 (m, 1H). |
| 5^{(b)(d)} | 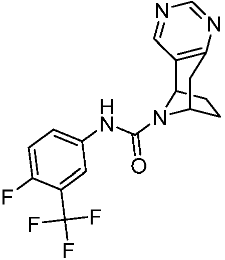 (±)-*N*-(4-Fluoro-3-(trifluoromethyl)phenyl)-6,7,8,9-tetrahydro-5H-5,8-epiminocyclohepta[d]pyrimidine-10-carboxamide | 367.3 at 1.2 min | ¹H NMR (500 MHz, DMSO-d6) δ 9.08 (s, 1H), 8.96 (s, 1H), 8.60 (s, 1H), 7.92 (dd, *J* = 6.5, 2.7 Hz, 1H), 7.77 (dt, *J* = 8.2, 3.8 Hz, 1H), 7.39 (t, *J* = 9.8 Hz, 1H), 5.29 (d, *J* = 6.2 Hz, 1H), 4.81 (t, *J* = 6.5 Hz, 1H), 3.37 - 3.34 (m, 1H), 2.78 (d, *J* = 18.2 Hz, 1H), 2.34 - 2.14 (m, 2H), 1.93 - 1.85 (m, 1H), 1.76 (dd, *J* = 18.2, 10.1 Hz, 1H). |
| 6^{(b)(d)} | 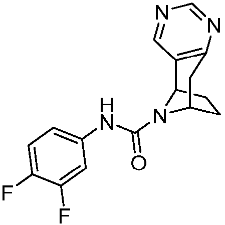 (±)-*N*-(3,4-Difluorophenyl)-6,7,8,9-tetrahydro-5H-5,8-epiminocyclohepta[d]pyrimidine-10-carboxamide | 317.3 at 1.01 min | ¹H NMR (500 MHz, DMSO-d6) δ 8.95 (s, 2H), 8.59 (s, 1H), 7.61 (ddd, *J* = 13.6, 7.5, 2.6 Hz, 1H), 7.30 (dt, J = 10.6, 9.1 Hz, 1H), 7.24 - 7.17 (m, 1H), 5.27 (d, *J* = 6.2 Hz, 1H), 4.80 (t, *J* = 6.4 Hz, 1H), 3.36 - 3.33 (m, 1H), 2.81 - 2.74 (m, 1H), 2.32 - 2.24 (m, 1H), 2.19 (tt, *J =* 12.1, 6.2 Hz, 1H), 1.93 - 1.84 (m, 1H), 1.80 - 1.71 (m, 1H). |
| 7^{(b)(d)} | 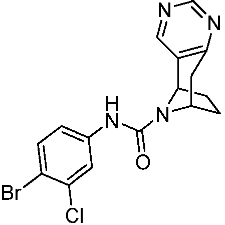 (±)-*N*-(4-Bromo-3-chlorophenyl)-6,7,8,9-tetrahydro-5H-5,8-epiminocyclohepta[d]pyrimidine-10-carboxamide | 393.2, 395.2, 396.3, 397.4 at 1.24 min | ¹H NMR (500 MHz, DMSO-d6) δ 9.03 (s, 1H), 8.96 (s, 1H), 8.60 (s, 1H), 7.83 (d, *J* = 2.5 Hz, 1H), 7.60 (d, *J* = 8.8 Hz, 1H), 7.38 (dd, J = 8.9, 2.5 Hz, 1H), 5.28 (d, *J* = 6.1 Hz, 1H), 4.80 (t, *J* = 6.5 Hz, 1H), 3.31 - 3.27 (m, 1H), 2.81 - 2.74 (m, 1H), 2.33 - 2.25 (m, 1H), 2.25 - 2.13 (m, 1H), 1.93 - 1.85 (m, 1H), 1.76 (dt, *J* = 15.5, 7.6 Hz, 1H). |
| 8(b)(_{d)} | 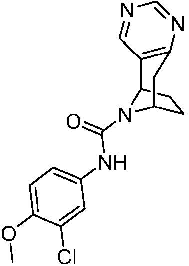 (±)-N-(3-Chloro-4-methoxyphenyl)-6,7,8,9-tetrahydro-5H-5,8-epiminocyclohepta[d]pyrimidine-10-carboxamide | 345.3, 347.4 at 1.00 min | ¹H NMR (500 MHz, DMSO-d6) δ 8.95 (s, 1H), 8.74 (s, 1H), 8.58 (s, 1H), 7.58 (d, *J* = 2.6 Hz, 1H), 7.33 (dd, *J* = 9.0, 2.6 Hz, 1H), 7.03 (d, *J* = 9.0 Hz, 1H), 5.25 (d, *J* = 6.2 Hz, 1H), 4.77 (t, *J* = 6.4 Hz, 1H), 3.78 (s, 3H), 3.31 - 3.28 (m, 1H), 2.75 (d, *J =* 18.4 Hz, 1H), 2.27 (q, *J* = 11.5, 10.8 Hz, 1H), 2.18 (tt, *J* = 12.1, 6.2 Hz, 1H), 1.92 - 1.83 (m, 1H), 1.75 (dt, *J* = 15.0, 7.6 Hz, 1H). |
| 12^{(d)} | 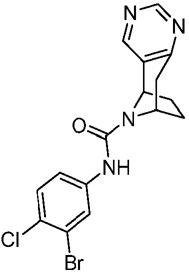 (±)-*N*-(3-Bromo-4-chlorophenyl)-6,7,8,9-tetrahydro-5H-5,8-epiminocyclohepta[d]pyrimidine-10-carboxamide | 393.3, 395.3 at 1.24 min | ¹H NMR (500 MHz, DMSO-d6) δ 9.01 (s, 1H), 8.96 (s, 1H), 8.59 (s, 1H), 7.95 (d, *J* = 2.4 Hz, 1H), 7.53 - 7.44 (m, 2H), 5.28 (d, *J* = 6.2 Hz, 1H), 4.83 - 4.77 (m, 1H), 3.35 - 3.33 (m, 1H), 2.78 (d, *J* = 18.3 Hz, 1H), 2.27 (d, *J =* 10.9 Hz, 1H), 2.19 (dt, *J* = 11.3, 5.4 Hz, 1H), 1.93 - 1.85 (m, 1H), 1.76 (s, 1H). |
| 13^{(d)} | 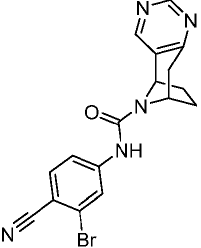 (±)-N-(3-Bromo-4-cyanophenyl)-6,7,8,9-tetrahydro-5H-5,8-epiminocyclohepta[d]pyrimidine-10-carboxamide | 384.3, 386.3 at 1.07 min | ¹H NMR (500 MHz, DMSO-d6) δ 9.35 (s, 1H), 8.96 (s, 1H), 8.61 (s, 1H), 8.04 (d, *J* = 2.0 Hz, 1H), 7.78 (d, *J* = 8.7 Hz, 1H), 7.64 (dd, *J* = 8.7, 2.1 Hz, 1H), 5.31 (d, *J* = 6.2 Hz, 1H), 4.87 - 4.80 (m, 1H), 3.34 (d, *J* = 4.7 Hz, 1H), 2.80 (d, *J* = 18.3 Hz, 1H), 2.31 - 2.14 (m, 2H), 1.94 - 1.86 (m, 1H), 1.76 (dd, *J* = 18.2, 10.1 Hz, 1H). |
| 16^{(b)(d)} | 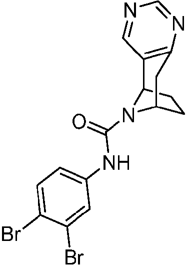 (±)-*N*-(3,4-Dibromophenyl)-6,7,8,9-tetrahydro-5H-5,8-epiminocyclohepta[d]pyrimidine-10-carboxamide | 437.3, 439.3, 441.3 at 1.27 min | ¹H NMR (500 MHz, DMSO-d6) δ 9.01 (s, 1H), 8.96 (s, 1H), 8.59 (s, 1H), 7.96 (d, *J* = 2.5 Hz, 1H), 7.59 (d, *J* = 8.8 Hz, 1H), 7.43 (dd, *J* = 8.8, 2.5 Hz, 1H), 5.28 (d, *J* = 6.1 Hz, 1H), 4.83 - 4.77 (m, 1H), 3.37 - 3.33 (m, 1H), 2.78 (d, *J* = 18.4 Hz, 1H), 2.27 (q, *J* = 10.9, 10.5 Hz, 1H), 2.19 (tt, *J* = 12.1, 6.2 Hz, 1H), 1.93 - 1.85 (m, 1H), 1.76 (dt, *J* = 15.9, 7.7 Hz, 1H). |
| 17^{(a)(e)} | 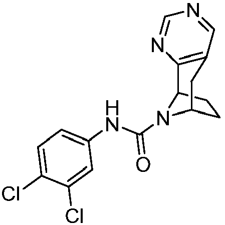 (±)-*N*-(3,4-Dichlorophenyl)-6,7,8,9-tetrahydro-5H-6,9-epiminocyclohepta[d]pyrimidine-10-carboxamide | 349.3, 351.3 at 1.24 min | ¹H NMR (500 MHz, DMSO-d6) δ 9.09 (s, 1H), 8.92 (s, 1H), 8.56 (s, 1H), 7.84 (d, *J* = 2.2 Hz, 1H), 7.49 - 7.42 (m, 2H), 5.12 (d, *J* = 6.1 Hz, 1H), 4.80 (t, *J* = 6.1 Hz, 1H), 3.31 - 3.24 (m, 1H), 2.68 (d, *J =* 17.2 Hz, 1H), 2.32 - 2.19 (m, 2H), 1.90 - 1.84 (m, 1H), 1.71 (dt, *J* = 9.7, 5.2 Hz, 1H). |
| 18^{(a)(e)} | 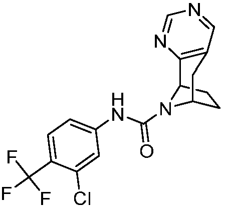 (±)-*N*-(3-Chloro-4-(trifluoromethyl)phenyl)-6,7,8,9-tetrahydro-5H-6,9-epiminocyclohepta[d]pyrimidine-10-carboxamide | 383.4, 385.3 at 1.35 min | ¹H NMR (500 MHz, DMSO-d6) δ 9.35 (s, 1H), 8.92 (s, 1H), 8.57 (s, 1H), 7.91 (d, *J* = 2.1 Hz, 1H), 7.71 (d, *J* = 8.8 Hz, 1H), 7.61 (d, *J* = 9.6 Hz, 1H), 5.15 (d, *J* = 6.0 Hz, 1H), 4.83 (t, *J=* 6.1 Hz, 1H), 3.29 (d, *J* = 7.3 Hz, 1H), 2.70 (d, *J* = 17.1 Hz, 1H), 2.33 - 2.17 (m, 2H), 1.94 - 1.86 (m, 1H), 1.72 (dt, *J* = 9.5, 4.9 Hz, 1H). |
| 24^{(b)(e)(h)} | 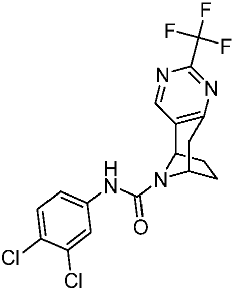 (±)-N-(3,4-Dichlorophenyl)-2-(trifluoromethyl)-6,7,8,9-tetrahydro-5H-5,8-epiminocyclohepta[d]pyrimidine-10-carboxamide | 417.3, 419.3 at 1.60 min | ¹H NMR (500 MHz, DMSO-d6) δ 9.08 (s, 1H), 8.87 (s, 1H), 7.82 (d, *J* = 2.4 Hz, 1H), 7.49 (d, *J* = 8.8 Hz, 1H), 7.44 (dd, *J* = 8.9, 2.4 Hz, 1H), 5.40 (d, *J =* 6.1 Hz, 1H), 4.85 (t, *J* = 6.3 Hz, 1H), 3.42 (dd, J = 18.8, 5.0 Hz, 1H), 2.93 (d, *J* = 18.8 Hz, 1H), 2.31 - 2.24 (m, 1H), 2.24 - 2.18 (m, 1H), 1.99 - 1.91 (m, 1H), 1.88 - 1.77 (m, 1H). |
| 25^{(b)(e)(h)} | 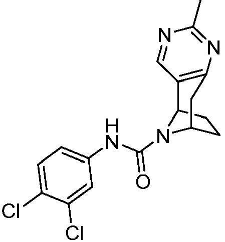 (±)-*N*-(3,4-Dichlorophenyl)-2-methyl-6,7,8,9-tetrahydro-5H-5,8-epiminocyclohepta[d]pyrimidine-10-carboxamide | 363.3, 365.3 at 1.23 min | ¹H NMR (500 MHz, DMSO-d6) δ 9.00 (s, 1H), 8.46 (s, 1H), 7.82 (d, *J* = 2.3 Hz, 1H), 7.50 - 7.42 (m, 2H), 5.24 (d, *J* = 6.1 Hz, 1H), 4.77 (t, *J* = 6.5 Hz, 1H), 3.27 (dd, *J* = 18.2, 5.2 Hz, 1H), 2.71 (d, *J* = 18.3 Hz, 1H), 2.53 (s, 3H), 2.26 (q, *J* = 11.1 Hz, 1H), 2.17 (tt, *J* = 12.0, 6.2 Hz, 1H), 1.85 (dd, *J* = 12.2, 9.1 Hz, 1H), 1.73 (dt, *J* = 15.2, 7.9 Hz, 1H). |
| 26^{(a)(e)(h)} | 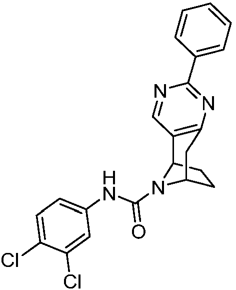 (±)-*N*-(3,4-Dichlorophenyl)-2-phenyl-6,7,8,9-tetrahydro-5H-5,8-epiminocyclohepta[d]pyrimidine-10-carboxamide | 425.3, 427.3 at 1.76 min | ¹H NMR (500 MHz, DMSO-d6) δ 9.05 (s, 1H), 8.70 (s, 1H), 8.38 - 8.31 (m, 2H), 7.84 (d, *J* = 2.1 Hz, 1H), 7.54 - 7.48 (m, 3H), 7.48 - 7.43 (m, 2H), 5.33 (d, *J* = 6.1 Hz, 1H), 4.84 (t, *J =* 6.5 Hz, 1H), 3.40 (dd, J = 18.4, 5.0 Hz, 1H), 2.87 (d, *J* = 18.2 Hz, 1H), 2.30 (q, *J* = 10.9, 10.4 Hz, 1H), 2.22 (tt, *J* = 12.0, 6.1 Hz, 1H), 1.94 (t, *J* = 10.8 Hz, 1H), 1.86 - 1.75 (m, 1H). |
| 27^{(b)(d)} | 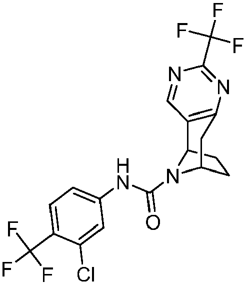 (±)-*N*-(3-Chloro-4-(trifluoromethyl)phenyl)-2-(trifluoromethyl)-6,7,8,9-tetrahydro-5H-5,8-epiminocyclohepta[d]pyrimidine-10-carboxamide | 450.4, 452.4 at 1.67 min | ¹H NMR (500 MHz, DMSO-d6) δ 9.34 (s, 1H), 8.89 (s, 1H), 7.89 (d, *J* = 2.0 Hz, 1H), 7.72 (d, *J* = 8.8 Hz, 1H), 7.60 (d, *J =* 9.0 Hz, 1H), 5.43 (d, *J* = 6.2 Hz, 1H), 4.88 (t, *J* = 6.4 Hz, 1H), 3.44 (dd, *J* = 19.0, 5.2 Hz, 1H), 2.95 (d, *J* = 18.8 Hz, 1H), 2.34 - 2.17 (m, 2H), 1.98 (dd, J = 12.1, 9.0 Hz, 1H), 1.88 - 1.80 (m, 1H). |
| 28^{(b)(e)(h)} | 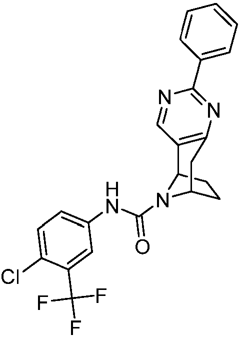 (±)-*N*-(4-Chloro-3-(trifluoromethyl)phenyl)-2-phenyl-6,7,8,9-tetrahydro-5H-5,8-epiminocyclohepta[d]pyrimidine-10-carboxamide | 459.3, 461.3 at 1.77 min | ¹H NMR (500 MHz, DMSO-d6) δ 9.20 (s, 1H), 8.71 (s, 1H), 8.34 (dd, *J* = 6.7, 3.1 Hz, 2H), 8.06 (d, *J* = 2.6 Hz, 1H), 7.80 (dd, *J* = 8.9, 2.6 Hz, 1H), 7.57 (d, *J* = 8.9 Hz, 1H), 7.54 - 7.48 (m, 3H), 5.35 (d, *J* = 6.1 Hz, 1H), 4.86 (t, *J* = 6.4 Hz, 1H), 3.42 (dd, *J* = 18.2, 5.0 Hz, 1H), 2.88 (d, *J* = 18.2 Hz, 1H), 2.36 - 2.27 (m, 1H), 2.27 - 2.17 (m, 1H), 1.95 (t, *J* = 10.6 Hz, 1H), 1.87 - 1.78 (m, 1H). |
| 29^{(b)(d)} | 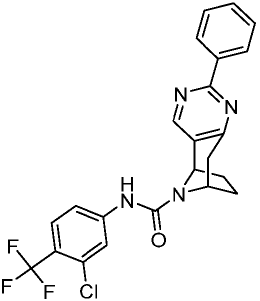 (±)-N-(3-Chloro-4-(trifluoromethyl)phenyl)-2-phenyl-6,7,8,9-tetrahydro-5H-5,8-epiminocyclohepta[d]pyrimidine-10-carboxamide | 459.4, 461.4 at 1.78 min | ¹H NMR (500 MHz, DMSO-d6) δ 9.31 (s, 1H), 8.71 (s, 1H), 8.34 (dd, J = 6.7, 3.0 Hz, 2H), 7.91 (d, *J* = 2.1 Hz, 1H), 7.71 (d, *J* = 8.7 Hz, 1H), 7.62 (d, J = 9.5 Hz, 1H), 7.53 - 7.48 (m, 3H), 5.36 (d, *J* = 6.1 Hz, 1H), 4.87 (t, *J* = 6.4 Hz, 1H), 3.41 (dd, *J* = 18.3, 5.1 Hz, 1H), 2.89 (d, *J* = 18.3 Hz, 1H), 2.34 - 2.18 (m, 2H), 1.95 (t, *J* = 10.5 Hz, 1H), 1.82 (d, *J* = 7.9 Hz, 1H). |
| 31 ^{(b)(e)(h)} | 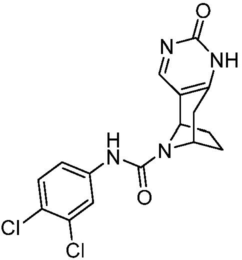 (±)-*N*-(3,4-Dichlorophenyl)-2-oxo-2,5,6,7,8,9-hexahydro-1H-5,8-epiminocyclohepta[d]pyrimidine-10-carboxamide | 365.3, 367.3 at 1.06 min | ¹H NMR (500 MHz, DMSO-d6) δ 11.68 (s, 1H), 8.96 (s, 1H), 7.85 (d, *J* = 2.1 Hz, 1H), 7.51 - 7.43 (m, 2H), 5.10 (d, *J* = 5.9 Hz, 1H), 4.68 (t, *J* = 6.4 Hz, 1H), 3.11 - 3.01 (m, 1H), 2.21 (q, *J* = 11.1 Hz, 1H), 2.06 (tt, *J* = 11.7, 6.1 Hz, 1H), 1.81 (t, *J* = 10.7 Hz, 1H), 1.75 - 1.68 (m, 1H). (2H underlying DMSO peak) |
| 32 ^{(b)(e)(h)} | 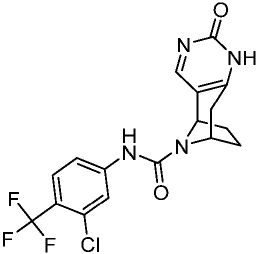 (±)-*N*-(3-Chloro-4-(trifluoromethyl)phenyl)-2-oxo-2,5,6,7,8,9-hexahydro-1H-5,8-epiminocyclohepta[d]pyrimidine-10-carboxamide | 399.3, 401.3 at 1.15 min | ¹H NMR (500 MHz, DMSO-d6) δ 11.73 (s, 1H), 9.20 (s, 1H), 7.91 (s, 2H), 7.72 (d, *J* = 8.8 Hz, 1H), 7.65 - 7.56 (m, 1H), 5.12 (d, *J* = 5.9 Hz, 1H), 4.71 (t, *J* = 6.6 Hz, 1H), 3.10 (dd, *J* = 18.6, 5.1 Hz, 1H), 2.56 (d, *J* = 18.5 Hz, 1H), 2.22 (q, *J* = 11.2 Hz, 1H), 2.12 - 2.02 (m, 1H), 1.82 (t, *J* = 10.6 Hz, 1H), 1.74 (dt, *J* = 15.7, 8.0 Hz, 1H) |
| 34^{(b)(g)(h)} | 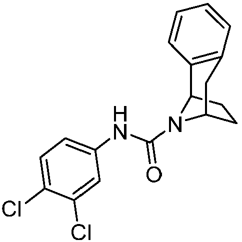 (±)-*N*-(3,4-Dichlorophenyl)-6,7,8,9-tetrahydro-5H-5,8-epiminobenzo[7]annulene-10-carboxamide | 347.3, 349.3 at 1.69 min | ¹H NMR (500 MHz, DMSO-d6) δ 8.87 (s, 1H), 7.86 (t, *JJ* = 1.3 Hz, 1H), 7.46 (d, *JJ* = 1.6 Hz, 2H), 7.19 - 7.12 (m, 3H), 7.10 (d, *JJ =* 6.8 Hz, 1H), 5.16 (d, *JJ* = 5.9 Hz, 1H), 4.70 (t, *JJ* = 6.2 Hz, 1H), 2.62 (d, *JJ* = 16.9 Hz, 1H), 2.26 - 2.09 (m, 2H), 1.83 - 1.75 (m, 1H), 1.67 (dt, *JJ =* 15.1, 7.4 Hz, 1H). (1H obscured by water peak) |
| 35^{(b)(d)(h)} | 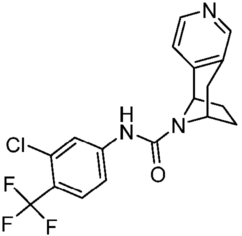 (±)-*N*-(3-Chloro-4-(trifluoromethyl)phenyl)-6,7,8,9-tetrahydro-5H-5,8-epiminocyclohepta[c]pyridine-10-carboxamide | 382.4, 384.3 at 1.40 min | ¹H NMR (500 MHz, DMSO-d6) δ 9.22 (s, 1H), 8.35 - 8.30 (m, 2H), 7.91 (d, *J* = 2.1 Hz, 1H), 7.70 (d, *J* = 8.8 Hz, 1H), 7.64 - 7.59 (m, 1H), 7.19 (d, *J* = 5.0 Hz, 1H), 5.19 (d, *J* = 6.3 Hz, 1H), 4.81 (t, *J* = 6.4 Hz, 1H), 3.28 (d, *J* = 4.9 Hz, 1H), 2.69 (d, *J* = 17.1 Hz, 1H), 2.31 - 2.13 (m, 2H), 1.87 - 1.78 (m, 1H), 1.76 - 1.67 (m, 1H) |
| 36^{(b)(d)(h)} | 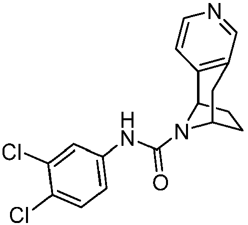 (±)-*N*-(3,4-Dichlorophenyl)-6,7,8,9-tetrahydro-5H-5,8-epiminocyclohepta[c]pyridine-10-carboxamide | 348.3, 350.3 at 1.33 min | ¹H NMR (500 MHz, DMSO-d6) δ 8.96 (s, 1H), 8.32 (t, *J =* 2.4 Hz, 2H), 7.84 (d, *J* = 2.1 Hz, 1H), 7.50 - 7.42 (m, 2H), 7.18 (d, *J* = 4.9 Hz, 1H), 5.16 (d, *J =* 6.3 Hz, 1H), 4.77 (t, *J* = 6.3 Hz, 1H), 3.31 - 3.25 (m, 1H), 2.67 (d, *J* = 17.1 Hz, 1H), 2.29 - 2.12 (m, 2H), 1.85 - 1.77 (m, 1H), 1.75 - 1.66 (m, 1H) |
| 37^{(b)(d)(h)} | 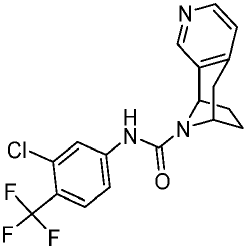 (±)-*N*-(3-Chloro-4-(trifluoromethyl)phenyl)-6,7,8,9-tetrahydro-5H-6,9-epiminocyclohepta[c]pyridine-10-carboxamide | 382.4, 384.3 at 1.42 min | 1H NMR (500 MHz, DMSO-d6) δ 9.21 (s, 1H), 8.37 (s, 1H), 8.33 (d, *J* = 5.0 Hz, 1H), 7.91 (d, *J* = 2.0 Hz, 1H), 7.70 (d, *J* = 8.8 Hz, 1H), 7.64 - 7.59 (m, 1H), 7.15 (d, *J* = 5.0 Hz, 1H), 5.28 (d, *J* = 6.0 Hz, 1H), 4.75 (t, *J = 6.2* Hz, 1H), 3.28 (d, *J =* 4.9 Hz, 1H), 2.69 (d,*J* = 17.7 Hz, 1H), 2.29 - 2.15 (m, 2H), 1.88 - 1.80 (m, 1H), 1.70 (t, *J* = 6.4 Hz, 1H) |
| 48^{(e)(h)} | 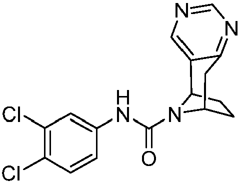 (±)-*N*-(3,4-Dichlorophenyl)-6,7,8,9-tetrahydro-5H-5,8-epiminocyclohepta[d]pyrimidine-10-carboxamide | 349.3, 351.3 at 1.22 min | ¹H NMR (500 MHz, DMSO-d6) δ 9.03 (s, 1H), 8.96 (s, 1H), 8.60 (s, 1H), 7.83 (d, *J* = 2.4 Hz, 1H), 7.51 - 7.41 (m, 2H), 5.28 (d, *J* = 6.2 Hz, 1H), 4.80 (t, *J* = 6.5 Hz, 1H), 3.29 (s, 1H), 2.78 (d, *J* = 18.4 Hz, 1H), 2.28 (dt, *J*= 11.0, 10.2 Hz, 1H), 2.19 (tt, *J* = 12.1, 6.2 Hz, 1H), 1.94 - 1.85 (m, 1H), 1.76 (dt, *J* = 15.5, 7.7 Hz, 1H). |
| 49 | 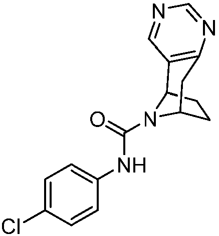 (±)-*N*-(4-Chlorophenyl)-6,7,8,9-tetrahydro-5H-5,8-epiminocyclohepta[d]pyrimidine-10-carboxamide | 315.2, 317.2 at 1.05 min | ¹H NMR (500 MHz, DMSO-d6) δ 8.95 (s, 1H), 8.89 (s, 1H), 8.59 (s, 1H), 7.48 (dd, J = 9.1, 2.6 Hz, 2H), 7.30 - 7.22 (m, 2H), 5.28 (d, *J* = 6.2 Hz, 1H), 4.80 (t, *J* = 6.4 Hz, 1H), 3.36 - 3.33 (m, 1H), 2.77 (d, *J* = 18.4 Hz, 1H), 2.32 - 2.23 (m, 1H), 2.19 (tt, *J* = 12.0, 6.1 Hz, 1H), 1.88 (ddd, *J* = 12.1, 9.3, 2.5 Hz, 1H), 1.75 (td, J = 10.5, 8.8, 6.1 Hz, 1H). |
| 50 | 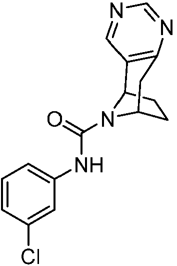 (±)-*N*-(3-Chlorophenyl)-6,7,8,9-tetrahydro-5H-5,8-epiminocyclohepta[d]pyrimidine-10-carboxamide | 315.2, 317.2 at 1.06 min | ¹H NMR (500 MHz, DMSO-d6) δ 8.96 (s, 1H), 8.94 (s, 1H), 8.60 (s, 1H), 7.64 (t, *J* = 2.1 Hz, 1H), 7.42 - 7.36 (m, 1H), 7.25 (t, *J = 8.1* Hz, 1H), 6.99 (dd, J *=* 8.0, 2.1 Hz, 1H), 5.28 (d, *J =* 6.2 Hz, 1H), 4.81 (t, *J* = 6.5 Hz, 1H), 3.30 (s, 1H), 2.77 (d, *J* = 18.4 Hz, 1H), 2.32 - 2.25 (m, 1H), 2.19 (td, *J* = 12.0, 6.2 Hz, 1H), 1.89 (ddd, *J* = 12.0, 9.2, 2.5 Hz, 1H), 1.76 (dt, *J* = 15.4, 8.2 Hz, 1H). |
| 51 | 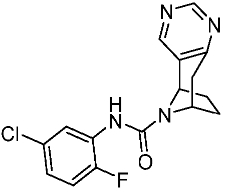 (±)-N-(5-Chloro-2-fluorophenyl)-6,7,8,9-tetrahydro-5H-5,8-epiminocyclohepta[d]pyrimidine-10-carboxamide | 333.3, 335.3 at 1.03 min | ¹H NMR (500 MHz, DMSO-d6) δ 8.96 (s, 1H), 8.78 (s, 1H), 8.59 (s, 1H), 7.60 (dd, J = 6.9, 2.7 Hz, 1H), 7.25 (dd, J = 10.5, 8.8 Hz, 1H), 7.15 (ddd, J = 8.9, 4.1, 2.7 Hz, 1H), 5.25 (d, *J =* 6.1 Hz, 1H), 4.78 (t, *J* = 6.5 Hz, 1H), 3.41 - 3.35 (m, 1H), 2.76 (d, *J* = 18.3 Hz, 1H), 2.33 - 2.14 (m, 2H), 1.93 - 1.82 (m, 1H), 1.80 - 1.70 (m, 1H). |
| 54 | 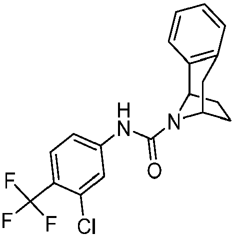 (±)-N-(3-Chloro-4-(trifluoromethyl)phenyl)-6,7,8,9-tetrahydro-5H-5,8-epiminobenzo[7]annulene-10-carboxamide | 381.3, 383.4 at 1.76 min | ¹H NMR (500 MHz, DMSO-d6) δ 9.14 (s, 1H), 7.92 (d, *J* = 2.0 Hz, 1H), 7.69 (d, *J* = 8.8 Hz, 1H), 7.62 (dd, J = 8.9, 2.1 Hz, 1H), 7.19 - 7.08 (m, 4H), 5.19 (d, *J* = 5.9 Hz, 1H), 4.76 - 4.70 (m, 1H), 3.37 - 3.28 (m, 1H), 2.64 (d, *J* = 16.9 Hz, 1H), 2.28 - 2.11 (m, 2H), 1.85 - 1.77 (m, 1H), 1.69 (dt, J = 15.2, 7.3 Hz, 1H) |
| 55 | 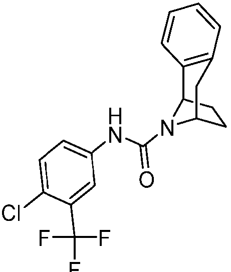 (±)-*N*-(4-Chloro-3-(trifluoromethyl)phenyl)-6,7,8,9-tetrahydro-5H-5,8-epiminobenzo[7]annulene-10-carboxamide | 381.3, 383.4 at 1.74 min | ¹H NMR (500 MHz, DMSO-d6) δ 9.02 (s, 1H), 8.08 (d, *J* = 2.6 Hz, 1H), 7.81 (dd, *J* = 8.9, 2.6 Hz, 1H), 7.55 (d, *J* = 8.9 Hz, 1H), 7.20 - 7.04 (m, 4H), 5.17 (d, *J* = 5.9 Hz, 1H), 4.79 - 4.66 (m, 1H), 3.39 - 3.35 (m, 1H), 2.63 (d, *J* = 16.8 Hz, 1H), 2.29 - 2.08 (m, 2H), 1.87 - 1.74 (m, 1H), 1.68 (dt, *J* = 14.4, 7.3 Hz, 1H |
| 56^{(b)(h)} | 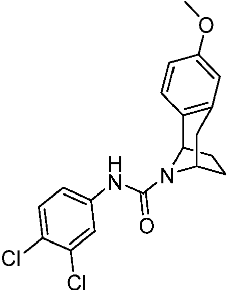 (±)-*N*-(3,4-Dichlorophenyl)-2-methoxy-6,7,8,9-tetrahydro-5H-5,8-epiminobenzo[7]annulene-10-carboxamide | 377.3, 379.3 at 1.65 min | ¹H NMR (500 MHz, DMSO-d6) δ 8.84 (s, 1H), 7.86 (s, 1H), 7.46 (s, 2H), 7.04 (d, *J* = 8.2 Hz, 1H), 6.82 - 6.55 (m, 2H), 5.12 (d, *J* = 5.9 Hz, 1H), 4.77 - 4.53 (m, 1H), 3.70 (s, 3H), 2.58 (d, *J* = 16.9 Hz, 1H), 2.24 - 2.06 (m, 2H), 1.80 - 1.70 (m, 1H), 1.69 - 1.60 (m, 1H), 1H obscured by residual water peak. |
| 57^{(b)(h)} | 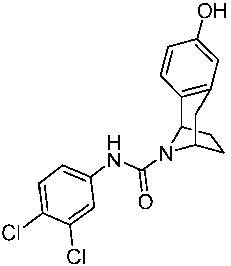 (±)-*N*-(3,4-Dichlorophenyl)-2-hydroxy-6,7,8,9-tetrahydro-5H-5,8-epiminobenzo[7]annulene-10-carboxamide | 363.3, 365.3 at 1.35 min | ¹H NMR (500 MHz, DMSO-d6) δ 9.18 (s, 1H), 8.81 (s, 1H), 7.86 (dd, *J* = 2.0, 0.8 Hz, 1H), 7.55-7.38 (m, 2H), 6.91 (d, *J =* 8.2 Hz, 1H), 6.51 (dd, *J* = 8.1, 2.5 Hz, 1H), 6.48 (d, *J* = 2.4 Hz, 1H), 5.07 (d, *J* = 5.9 Hz, 1H), 4.63 (t, *J* = 6.3 Hz, 1H), 3.24 (dd, *J* = 16.8, 4.9 Hz, 1H), 2.17 (q, *J =* 10.9 Hz, 1H), 2.13 - 2.02 (m, 1H), 1.81 - 1.69 (m, 1H), 1.68 - 1.54 (m, 1H), OH proton not visible |
| 73^{(b)(h)} | 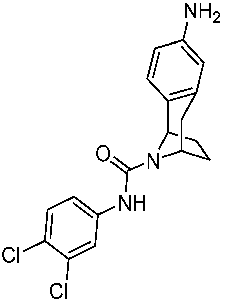 (±)-2-Amino-*N*-(3,4-dichlorophenyl)-6,7,8,9-tetrahydro-5H-5,8-epiminobenzo[7]annulene-10-carboxamide | 362.2, 364.5 at 1.39 min | ¹H NMR (500 MHz, DMSO-*d*₆) δ 8.77 (s, 1H), 7.86 (d, *J* = 2.2 Hz, 1H), 7.50 - 7.42 (m, 2H), 6.76 (d, *J* = 8.0 Hz, 1H), 6.32 (dd, *J* = 8.0, 2.3 Hz, 1H), 6.28 (d, *J* = 2.2 Hz, 1H), 5.00 (d, *J* = 5.8 Hz, 1H), 4.86 (s, 2H), 4.63 - 4.57 (m, 1H), 3.19 (dd, *J* = 16.5, 4.8 Hz, 1H), 2.44 (d, *J* = 16.6 Hz, 1H), 2.19 - 2.13 (m, 1H), 2.12 - 2.03 (m, 1H), 1.74 - 1.68 (m, 1H), 1.62 (br s, 1H). |
| 74^{(b)(i)} | 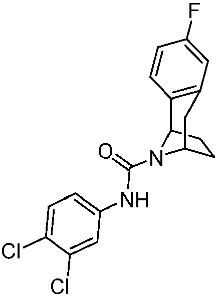 (±)-*N*-(3,4-Dichlorophenyl)-2-fluoro-6,7,8,9-tetrahydro-5H-5,8-epiminobenzo[7]annulene-10-carboxamide | 365.2, 367.4 at 1.68 min | ¹H NMR (500 MHz, DMSO-*d*₆) δ 8.88 (s, 1H), 7.84 (t, *J* = 1.4 Hz, 1H), 7.49 - 7.43 (m, 2H), 7.17 (dd, *J* = 9.2, 5.9 Hz, 1H), 6.95 (dd, *J* = 9.1, 6.1 Hz, 2H), 5.17 (d, *J* = 5.9 Hz, 1H), 4.68 (t, *J = 6.3* Hz, 1H), 2.63 (d, *J* = 17.2 Hz, 1H), 2.25 - 2.05 (m, 2H), 1.81 - 1.72 (m, 1H), 1.71 - 1.61 (m, 1H). (Exchangable - NH proton not seen). |
| 75^{(b)} | 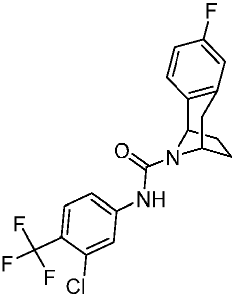 (±)-*N*-(3-Chloro-4-(trifluoromethyl)phenyl)-2-fluoro-6,7,8,9-tetrahydro-5H-5,8-epiminobenzo[7]annulene-10-carboxamide | 365.2, 399.3 at 1.75 min | ¹H NMR (500 MHz, DMSO-*d*₆) δ 9.14 (s, 1H), 7.91 (d, *J* = 2.0 Hz, 1H), 7.69 (d, *J* = 8.8 Hz, 1H), 7.61 (dd, *J* = 8.6, 2.1 Hz, 1H), 7.18 (dd, *J* = 9.3, 5.8 Hz, 1H), 7.01 - 6.72 (m, 2H), 5.20 (d, *J =* 6.0 Hz, 1H), 4.71 (t, *J =* 6.3 Hz, 1H), 2.65 (d, *J* = 17.3 Hz, 1H), 2.29 - 2.06 (m, 2H), 1.86 - 1.73 (m, 1H), 1.71 - 1.65 (m, 1H). (Exchangable -NH proton not visible). |
| 78^{(b)(i)} | 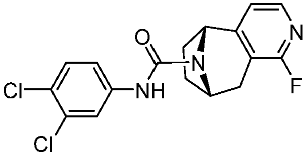 (5*R*,8*S*)-*N*-(3,4-Dichlorophenyl)-1-fluoro-6,7,8,9-tetrahydro-5H-5,8-epiminocyclohepta[c]pyridine-10-carboxamide | 366.1, 368.0 at 1.42 min | ¹H NMR (500 MHz, DMSO-d6) δ 9.00 (s, 1H), 8.01 (d, *J* = 5.0 Hz, 1H), 7.82 (d, *J* = 2.3 Hz, 1H), 7.47 (d, *J* = 8.8 Hz, 1H), 7.44 (dd, *J* = 8.9, 2.3 Hz, 1H), 7.20 (dd, *J* = 5.1, 1.6 Hz, 1H), 5.23 (d, *J =* 6.3 Hz, 1H), 4.98 - 4.64 (m, 1H), 3.14 (dd, *J* = 17.5, 5.0 Hz, 1H), 2.59 (d, *J* = 17.4 Hz, 1H), 2.38 - 2.04 (m, 2H), 1.91 - 1.80 (m, 1H), 1.80 - 1.69 (m, 1H). |
| 80^{(b)(d)} | 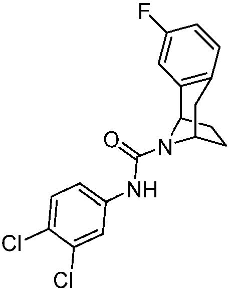 (±)-*N*-(3,4-Dichlorophenyl)-3-fluoro-6,7,8,9-tetrahydro-5H-5,8-epiminobenzo[7]annulene-10-carboxamide | 365.1, 367.1 at 1.64 min | ¹H NMR (500 MHz, DMSO-*d*₆) δ 8.89 (s, 1H), 7.84 (t, *J* = 1.4 Hz, 1H), 7.46 (s, 1H), 7.13 (dd, *J* = 8.3, 5.8 Hz, 1H), 7.03 - 6.84 (m, 2H), 5.14 (d, *J* = 6.1 Hz, 1H), 4.70 (t, *J* = 6.3 Hz, 1H), 3.25 (dd, *J* = 16.6, 4.9 Hz, 1H), 2.60 (d, *J* = 16.7 Hz, 1H), 2.28 - 2.09 (m, 2H), 1.88 - 1.74 (m, 1H), 1.72 - 1.56 (m, 1H). (Exchangeable NH not observed) |
| 81 ^{(b)(d)(h)} | 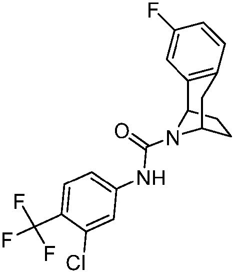 (±)-*N*-(3-Chloro-4-(trifluoromethyl)phenyl)-3-fluoro-6,7,8,9-tetrahydro-5H-5,8-epiminobenzo[7]annulene-10-carboxamide | 399.1, 401.1 at 1.71 min | ¹H NMR (500 MHz, DMSO-*d*₆) δ 9.16 (s, 1H), 7.91 (d, *J =* 2.1 Hz, 1H), 7.69 (d, *J* = 8.8 Hz, 1H), 7.61 (dd, *J* = 8.7, 2.1 Hz, 1H), 7.13 (dd, *J* = 8.4, 5.7 Hz, 1H), 7.04 - 6.94 (m, 2H), 5.17 (d, *J = 6.1* Hz, 1H), 4.77 - 4.71 (m, 1H), 3.26 (dd, *J* = 16.4, 4.8 Hz, 1H), 2.62 (d, *J* = 16.7 Hz, 1H), 2.27 - 2.05 (m, 2H), 1.84-1.79 (m, 1H), 1.68 -1.62 (m, 1H). |
| 83^{(b)(d)(h)} | 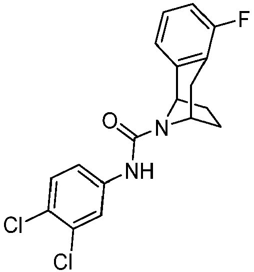 (±)-*N*-(3,4-Dichlorophenyl)-1-fluoro-6,7,8,9-tetrahydro-5H-5,8-epiminobenzo[7]annulene-10-carboxamide | 365.7, 367.5 at 1.69 min | ¹H NMR (500 MHz, DMSO-*d*₆) δ 8.92 (s, 1H), 7.84 (t, *J =* 1.4 Hz, 1H), 7.45 (d, *J* = 1.5 Hz, 2H), 7.19 (td, *J* = 7.9, 5.7 Hz, 1H), 7.00 (t, *J* = 8.0 Hz, 2H), 5.21 (d, *J =* 6.0 Hz, 1H), 4.75 (t, *J* = 6.3 Hz, 1H), 3.21 - 3.13 (m, 1H), 2.60 (d, *J* = 17.2 Hz, 1H), 2.27 - 2.19 (m, 1H), 2.18 - 2.13 (m, 1H), 1.84 - 1.76 (m, 1H), 1.74 - 1.65 (m, 1H). |
| 84^{(b)(d)(h)} | 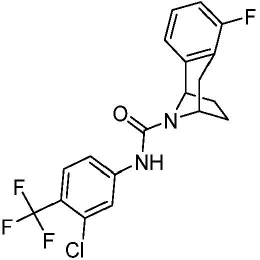 (±)-*N*-(3-Chloro-4-(trifluoromethyl)phenyl)-1-fluoro-6,7,8,9-tetrahydro-5H-5,8-epiminobenzo[7]annulene-10-carboxamide | 399.1, 401.1 at 1.72 min | ¹H NMR (500 MHz, DMSO-*d*₆) δ 9.18 (s, 1H), 7.91 (d, *J =* 2.1 Hz, 1H), 7.69 (d, *J* = 8.8 Hz, 1H), 7.61 (dd, *J* = 8.7, 2.1 Hz, 1H), 7.24 - 7.16 (m, 1H), 7.01 (dd, *J* = 9.7, 7.5 Hz, 2H), 5.24 (d, *J* = 6.0 Hz, 1H), 4.79 (t, *J* = 6.3 Hz, 1H), 3.22 - 3.14 (m, 1H), 2.63 (d, *J =* 17.1 Hz, 1H), 2.28 - 2.12 (m, 2H), 1.86 - 1.78 (m, 1H), 1.75 - 1.67 (m, 1H). |
| 85 ^{(b)(d)} | 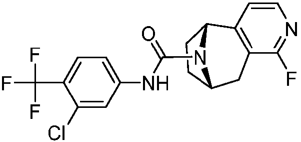 (5*R*,8*S*)-*N*-(3-Chloro-4-(trifluoromethyl)phenyl)-1-fluoro-6,7,8,9-tetrahydro-5H-5,8-epiminocyclohepta[c]pyridine-10-carboxamide | 400.1, 402.1 at 1.50 min | ¹H NMR (500 MHz, DMSO-d6) δ 9.26 (s, 1H), 8.01 (d, *J* = 5.0 Hz, 1H), 7.89 (d, *J* = 2.1 Hz, 1H), 7.70 (d, *J* = 8.8 Hz, 1H), 7.66 - 7.52 (m, 1H), 7.21 (dd, *J* = 5.1, 1.5 Hz, 1H), 5.26 (d, *J* = 6.3 Hz, 1H), 4.91 - 4.76 (m, 1H), 3.16 (dd, *J* = 17.4, 4.9 Hz, 1H), 2.61 (d, *J* = 17.4 Hz, 1H), 2.33 - 2.11 (m, 2H), 1.96 - 1.81 (m, 1H), 1.77 (dt, *J* = 15.2, 7.1 Hz, 1H). |
| 86^{(b)(h)(i)} | 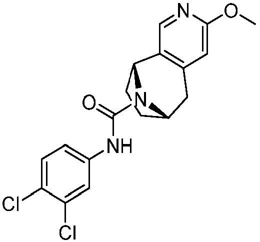 (6S,9R)-*N*-(3,4-Dichlorophenyl)-3-methoxy-6,7,8,9-tetrahydro-5H-6,9-epiminocyclohepta[c]pyridine-10-carboxamide | 378.3, 380.3 at 1.49 min | ¹H NMR (500 MHz, DMSO-d6) δ 8.89 (s, 1H), 7.94 (s, 1H), 7.83 (dd, *J* = 2.0, 0.8 Hz, 1H), 7.50 - 7.41 (m, 2H), 6.58 (s, 1H), 5.22 (d, *J* = 5.9 Hz, 1H), 4.66 (t, *J* = 6.2 Hz, 1H), 3.78 (s, 3H), 3.24 (dd, *J* = 17.7, 5.0 Hz, 1H), 2.64 (d, *J =* 17.7 Hz, 1H), 2.26 - 2.05 (m, 2H), 1.86 - 1.70 (m, 1H), 1.65 (dt, *J =* 12.0, 7.5 Hz, 1H). |
| 87^{(b)(e)(h)} | 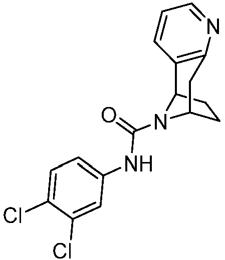 (±)-*N*-(3,4-Dichlorophenyl)-6,7,8,9-tetrahydro-5H-5,8-epiminocyclohepta[b]pyridine-10-carboxamide | 347.9, 350.3 at 1.31 min | ¹H NMR (500 MHz, DMSO-*d*₆) δ 8.96 (s, 1H), 8.35 (dd, *J =* 4.8, 1.7 Hz, 1H), 7.83 (dd, *J =* 2.0, 0.8 Hz, 1H), 7.55 (dd, *J =* 7.6, 1.8 Hz, 1H), 7.49 - 7.41 (m, 2H), 7.16 (dd, *J* = 7.6, 4.8 Hz, 1H), 5.20 (d, *J* = 6.1 Hz, 1H), 4.81 - 4.75 (m, 1H), 3.35 (d, *J = 5.1* Hz, 1H), 2.73 (d, *J* = 17.5 Hz, 1H), 2.25 (q, *J* = 11.0 Hz, 1H), 2.17 (tt, *J* = 11.9, 6.1 Hz, 1H), 1.86 - 1.78 (m, 1H), 1.72 (dt, *J* = 15.4, 7.7 Hz, 1H). |
| 91^{(b)(h)(i)} | 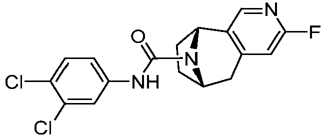 (6S,9R)-*N*-(3,4-Dichlorophenyl)-3-fluoro-6,7,8,9-tetrahydro-5H-6,9-epiminocyclohepta[c]pyridine-10-carboxamide | 366.4, 368.3, 370.2 at 1.45 min | ¹H NMR (500 MHz, DMSO-d6) δ 8.95 (s, 1H), 8.04 (s, 1H), 7.82 (d, *J* = 2.3 Hz, 1H), 7.47 (d, *J* = 8.8 Hz, 1H), 7.44 (dd, *J* = 8.9, 2.3 Hz, 1H), 6.96 (s, 1H), 5.29 (d, *J* = 6.0 Hz, 1H), 4.70 (t, *J* = 6.3 Hz, 1H), 3.34 - 3.27 (m, 1H), 2.75 (d, *J* = 18.1 Hz, 1H), 2.30 - 2.10 (m, 2H), 1.89 - 1.76 (m, 1H), 1.68 (dt, *J* = 14.8, 7.3 Hz, 1H). |
| 95^{(b)(d)(h)} | 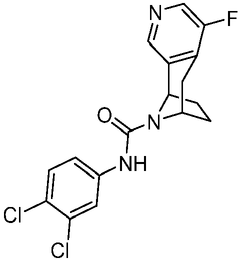 (±)-*N*-(3,4-Dichlorophenyl)-4-fluoro-6,7,8,9-tetrahydro-5H-6,9-epiminocyclohepta[c]pyridine-10-carboxamide | 366.1, 368.1, 370.2 at 1.44 min | ¹H NMR (500 MHz, DMSO-*d*₆) δ 8.98 (s, 1H), 8.35 (s, 1H), 8.28 (s, 1H), 7.82 (d, *J* = 2.3 Hz, 1H), 7.50 - 7.41 (m, 2H), 5.33 (d, *J* = 5.9 Hz, 1H), 4.78 (t, *J* = 6.2 Hz, 1H), 3.23 - 3.16 (m, 1H), 2.71 (d, *J* = 17.9 Hz, 1H), 2.29 - 2.15 (m, 2H), 1.85 (dd, *J* = 11.0, 8.3 Hz, 1H), 1.78 - 1.71 (m, 1H). |
| 103 | 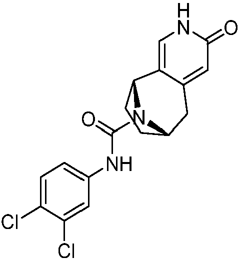 (6*S*,9*R*)-*N-*(3,4-Dichlorophenyl)-3-oxo-3,5,6,7,8,9-hexahydro-2H-6,9-epiminocyclohepta[c]pyridine-10-carboxamide | 364.3, 366.3 at 1.10 min | ¹H NMR (500 MHz, DMSO-d6) δ 11.27 (s, 1H), 8.83 (s, 1H), 7.86 - 7.82 (m, 1H), 7.49 - 7.44 (m, 2H), 7.19 (s, 1H), 6.09 (s, 1H), 5.06 (d, *J* = 6.0 Hz, 1H), 4.62 - 4.56 (m, 1H), 3.09 (dd, *J* = 17.9, 5.1 Hz, 1H), 2.55 (d, *J* = 17.8 Hz, 1H), 2.16 (q, *J* = 11.0 Hz, 1H), 2.05 (tt, J *=* 12.0, 6.3 Hz, 1H), 1.76 - 1.67 (m, 1H), 1.68 - 1.59 (m, 1H). |
| 104 | 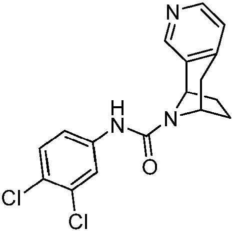 (±)-*N*-(3,4-Dichlorophenyl)-6,7,8,9-tetrahydro-5H-6,9-epiminocyclohepta[c]pyridine-10-carboxamide | 348.1, 350.1 at 1.29 min | 1H NMR: (400 MHz, DMSO-d6) δ 9.07-8.81 (m, 1H), 8.34 (s, 1H), 8.31 (d, *J =* 5.1 Hz, 1H), 7.83 (d, *J* = 1.1 Hz, 1H), 7.46-7.43 (m, 2H), 7.13 (d, *J =* 5.1 Hz, 1H), 5.24 (d, *J* = 5.7 Hz, 1H), 4.70 (t, *J =* 6.0 Hz, 1H), 3.30-3.22 (m, 1H), 2.66 (d, *J* = 17.9 Hz, 1H), 2.29-2.09 (m, 2H), 1.82 (t, *J =* 9.5 Hz, 1H), 1.68 (dd, *J* = 12.2, 5.8 Hz, 1H), |
| 111 | 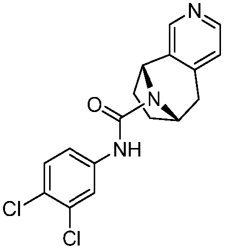 (6S,9R)-N-(3,4-Dichlorophenyl)-6,7,8,9-tetrahydro-5H-6,9-epiminocyclohepta[c]pyridine-10-carboxamide | 348.2, 350.3 at 1.31 min | ¹H NMR (500 MHz, DMSO-d6) δ 8.93 (s, 1H), 8.35 (s, 1H), 8.31 (d, *J* = 5.0 Hz, 1H), 7.83 (d, *J* = 2.1 Hz, 1H), 7.49 - 7.41 (m, 2H), 7.14 (d, *J* = 5.0 Hz, 1H), 5.25 (d, *J* = 5.9 Hz, 1H), 4.71 (t, *J* = 6.2 Hz, 1H), 3.31 - 3.24 (m, 1H), 2.67 (d, *J* = 17.8 Hz, 1H), 2.28 - 2.12 (m, 2H), 1.86 - 1.78 (m, 1H), 1.72 - 1.66 (m 1H). |
| 112^{(b)(e)(h)} | 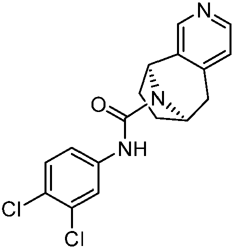 (6*R*,9*S*)-*N*-(3,4-Dichlorophenyl)-6,7,8,9-tetrahydro-5H-6,9-epiminocyclohepta[c]pyridine-10-carboxamide | 348.3, 350.2 at 1.31 min | ¹H NMR (500 MHz, DMSO-d6) δ 8.93 (s, 1H), 8.35 (s, 1H), 8.31 (d, *J* = 5.0 Hz, 1H), 7.83 (d, *J* = 2.1 Hz, 1H), 7.49 - 7.41 (m, 2H), 7.14 (d, *J* = 5.0 Hz, 1H), 5.25 (d, *J* = 5.9 Hz, 1H), 4.71 (t, *J* = 6.2 Hz, 1H), 3.31 - 3.24 (m, 1H), 2.67 (d, *J* = 17.8 Hz, 1H), 2.28 - 2.12 (m, 2H), 1.86 - 1.78 (m, 1H), 1.72 - 1.66 (m 1H). |
| 116^{(C)(i)} | 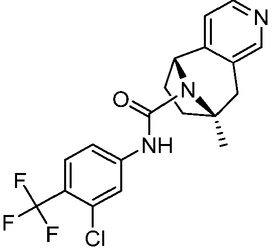 (5*R*,8*S*)-*N*-(3-Chloro-4-(trifluoromethyl)phenyl)-8-methyl-6,7,8,9-tetrahydro-5H-5,8-epiminocyclohepta[c]pyridine-10-carboxamide | 396.6, 398.4 at 1.54 min | ¹H NMR (500 MHz, DMSO-d6) δ 9.31 (s, 1H), 8.32 (s, 1H), 8.28 (d, *J =* 4.9 Hz, 1H), 7.77 (d, *J = 2.1* Hz, 1H), 7.65 (d, *J* = 8.9 Hz, 1H), 7.49 (d, *J =* 8.9 Hz, 1H), 7.11 (d, *J* = 5.0 Hz, 1H), 5.33 (d, *J* = 6.5 Hz, 1H), 3.51 (d, *J* = 16.8 Hz, 1H), 2.69 (d, *J =* 16.9 Hz, 1H), 2.31 - 2.20 (m, 1H), 1.92 (t, *J =* 8.0 Hz, 2H), 1.75 - 1.57 (m, 4H) |
| 134^{(b)(h)(i)} | 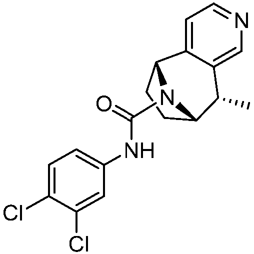 (5*R*,8*S*,9*S*)-*N*-(3,4-Dichlorophenyl)-9-methyl-6,7,8,9-tetrahydro-5H-5,8-epiminocyclohepta[c]pyridine-10-carboxamide | 362.3, 364.3 at 1.38 min | ¹H NMR (500 MHz, DMSO-d6) δ 8.95 (s, 1H), 8.48 (s, 1H), 8.32 (dd, *J* = 4.9, 0.9 Hz, 1H), 7.83 (dd, *J* = 2.0, 0.8 Hz, 1H), 7.59 - 7.38 (m, 2H), 7.14 (d, *J* = 4.9 Hz, 1H), 5.15 (d, *J* = 6.3 Hz, 1H), 4.65 - 4.39 (m, 1H), 3.64 - 3.39 (m, 1H), 2.13 (tt, J = 12.1, 6.6 Hz, 1H), 2.03 - 1.82 (m, 2H), 1.73 (ddd, *J* = 11.9, 8.9, 2.9 Hz, 1H), 1.32 (d, *J* = 7.1 Hz, 3H). |
| 135^{(b)(h)(i)} | 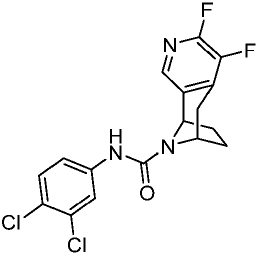 (±)-*N*-(3,4-Dichlorophenyl)-3,4-difluoro-6,7,8,9-tetrahydro-5H-6,9-epiminocyclohepta[c]pyridine-10-carboxamide | 384.3, 386.3, at 1.65 min | ¹H NMR (500 MHz, DMSO-d6) δ 8.99 (s, 1H), 7.91 (d, *J* = 1.4 Hz, 1H), 7.82 (d, *J* = 2.4 Hz, 1H), 7.50 - 7.40 (m, 2H), 5.35 (d, *J = 5.9* Hz, 1H), 4.77 (t, *J =* 6.2 Hz, 1H), 3.25 (dd, *J* = 18.2, 5.1 Hz, 1H), 2.82 (d, *J =* 18.1 Hz, 1H), 2.28 - 2.12 (m, 2H), 1.84 (t, *J =* 10.1 Hz, 1H), 1.75 (dt, *J* = 14.3, 6.9 Hz, 1H) |
| 140^{(b)(h)(i)} | 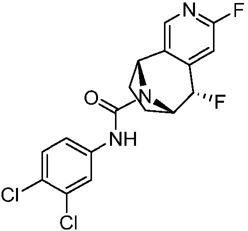 (5*S*,6*S*,9*R*)-*N*-(3,4-Dichlorophenyl)-3,5-difluoro-6,7,8,9-tetrahydro-5H-6,9-epiminocyclohepta[c]pyridine-10-carboxamide | 382.5, 384.2 at 1.64 min^{(j)} | ¹H NMR (500 MHz, DMSO-d6) δ 9.15 (s, 1H), 8.21 (s, 1H), 7.83 (d, *J* = 2.5 Hz, 1H), 7.50 (d, *J* = 8.8 Hz, 1H), 7.45 (dd, *J* = 8.9, 2.4 Hz, 1H), 7.25 (s, 1H), 6.05 (dd, *J* = 49.9, 5.4 Hz, 1H), 5.42 (d, *J* = 5.1 Hz, 1H), 4.94 (t, *J* = 6.2 Hz, 1H), 2.11 (m, 2H), 2.03 - 1.89 (m, 1H), 1.74 (t, *J* = 9.5 Hz, 1H). |
| 198 | 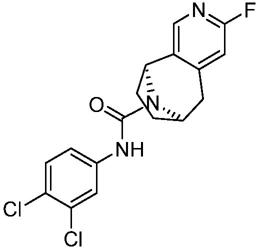 (6*R*,9*S*)-*N*-(3,4-dichlorophenyl)-3-fluoro-6,7,8,9-tetrahydro-5H-6,9-epiminocyclohepta[c]pyridine-10-carboxamide | 364.2, 366.2 at 1.49 min^{(j)} | ¹H NMR (500 MHz, DMSO-d6) δ 8.95 (s, 1H), 8.04 (s, 1H), 7.82 (d, J = 2.3 Hz, 1H), 7.53 - 7.37 (m, 2H), 6.96 (d, J = 1.7 Hz, 1H), 5.29 (d, J = 6.0 Hz, 1H), 4.70 (t, J = 6.2 Hz, 1H), 2.75 (d, J = 18.1 Hz, 1H), 2.18 (dtd, J = 24.0, 12.3, 7.3 Hz, 2H), 1.87 - 1.75 (m, 1H), 1.74 - 1.61 (m, 1H), ¹H under water peak. |

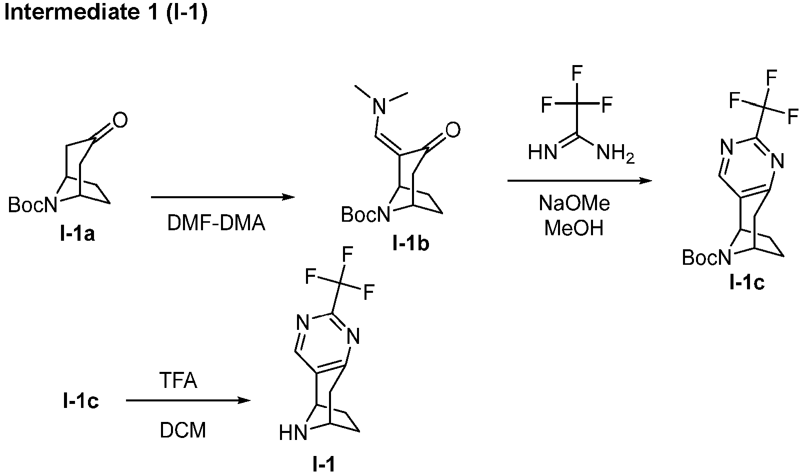

**Step** 1: *tert*-Butyl 3-oxo-8-azabicyclo[3.2.1]octane-8-carboxylate **I-1a** (10 g, 44 mmol) was dissolved in 1,1-dimethoxy-*N*,*N*-dimethylmethanamine (21 g, 24 ml, 0.18 mol) and the reaction mixture was heated at reflux for 16 h. The solvent was removed *in vacuo.* The product was purified by silica gel chromatography (0-5% (0.7 M ammonia/MeOH) in DCM) to afford *tert-*butyl (*E*)-2-((dimethylamino)methylene)-3-oxo-8-azabicyclo[3.2.1]octane-8-carboxylate **I-1b** as a yellow solid. LCMS (method 2) m/z 281.2 [M+H]⁺ (ES⁺); at 1.60 min. ¹H NMR (500 MHz, DMSO-d6) δ 7.25 - 7.14 (m, 1H), 5.20 (d, *J = 5.7* Hz, 1H), 4.21 (t, *J =* 6.3 Hz, 1H), 3.07 (s, 6H), 2.54 (s, 1H), 2.09 (s, 3H), 1.77 (t, *J =* 9.0 Hz, 1H), 1.68 - 1.56 (m, 1H), 1.38 (s, 9H).

**Step 2:** To a solution of 2,2,2-trifluoroacetimidamide (192 mg, 132 µl, 1.71 mmol) in MeOH (10 ml) was added a solution of sodium methoxide in MeOH (661 µl, 5.4 M, 3.57 mmol). A solution of *tert-*butyl (*E*)-2-((dimethylamino)methylene)-3-oxo-8-azabicyclo[3.2.1]octane-8-carboxylate **I-1b** (400 mg, 1.43 mmol) in MeOH (10 ml) was added and the reaction mixture was heated to 80 °C for 16 h. Water (5 ml) and saturated ammonium chloride solution (5 ml) was added. The product was extracted with 20% IPA in chloroform solution (50 ml). The product was purified by silica gel chromatography (0-3% (0.7 M Ammonia/MeOH) in DCM) to afford *tert-*butyl 2-(trifluoromethyl)-6,7,8,9-tetrahydro-5H-5,8-epiminocyclohepta[d]pyrimidine-10-carboxylate **I-1c** as a yellow oil. LCMS (method 2) m/z 330.1 [M+H]⁺ (ES⁺); at2.28 min. ¹H NMR (500 MHz, DMSO-d6) δ 5.12 (d, *J* = 5.8 Hz, 1H), 4.50 (s, 1H), 4.32 (s, 1H), 3.30 (s, 1H), 2.95 - 2.84 (m, 1H), 2.29 - 2.11 (m, 2H), 1.91 (t, *J =* 9.9 Hz, 1H), 1.77 - 1.68 (m, 1H), 1.41 - 1.23 (m, 9H).

**Step 3:** To a solution of *tert-*butyl 2-(trifluoromethyl)-6,7,8,9-tetrahydro-5H-5,8-epiminocyclohepta[d]pyrimidine-10-carboxylate **I-1c** (85 mg, 0.26 mmol) in DCM (2 ml) was added TFA (0.20 ml, 2.6 mmol). The reaction was stirred at RT for 16 h. The crude reaction mixture was concentrated under reduced pressure. 2 ml of 0.7 M ammonia in MeOH was added and the solvent was removed *in vacuo* to give 2-(trifluoromethyl)-6,7,8,9-tetrahydro-5H-5,8-epiminocyclohepta[d]pyrimidine as a yellow oil that was used in subsequent steps without further purification. LCMS (method 2) m/z 230.0 [M+H]⁺ (ES⁺); at 1.20 min.

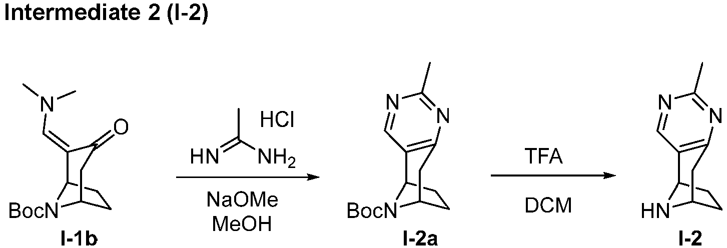

**Step 1:** *tert-*Butyl 2-methyl-6,7,8,9-tetrahydro-5H-5,8-epiminocyclohepta[d]pyrimidine-10-carboxylate **I-2a** was prepared from *tert-*butyl (*E*)-2-((dimethylamino)methylene)-3-oxo-8-azabicyclo[3.2.1]octane-8-carboxylate **I-1b** using a procedure essentially the same as for compound **I-1c:** LCMS (method 2) m/z 276.1 [M+H]⁺ (ES⁺); at 1.59 min. ¹H NMR (500 MHz, DMSO-d6) δ 8.47 (s, 1H), 4.93 (d, *J* = 6.0 Hz, 1H), 4.44 (s, 1H), 3.21 - 3.12 (m, 1H), 2.67 (d, *J* = 18.2 Hz, 1H), 2.53 (s, 3H), 2.21 (s, 1H), 2.13 (dq, *J* = 11.8, 5.8 Hz, 1H), 1.78 (t, *J* = 10.2 Hz, 1H), 1.66 (dt, *J* = 15.1, 7.6 Hz, 1H), 1.34 (s, 9H).

**Step 2:** 2-Methyl-6,7,8,9-tetrahydro-5H-5,8-epiminocyclohepta[d]pyrimidine **I-2** was prepared from *tert*-butyl 2-methyl-6,7,8,9-tetrahydro-5H-5,8-epiminocyclohepta[d]pyrimidine-10-carboxylate **I-2a** using a procedure essentially the same as for **I-1:** LCMS (method 2) m/z 176.1 [M+H]⁺ (ES⁺); at 0.70 min.

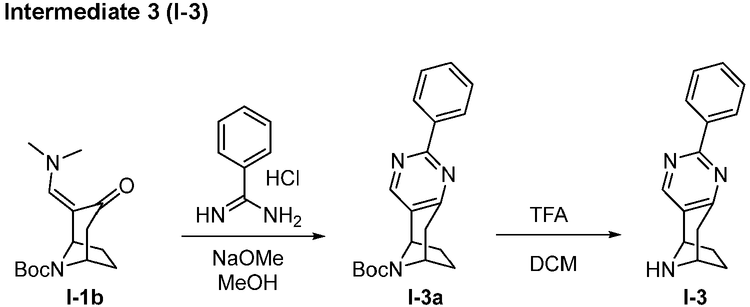

**Step 1:** *tert-*Butyl 2-phenyl-6,7,8,9-tetrahydro-5H-5,8-epiminocyclohepta[d]pyrimidine-10-carboxylate **(I-3a)** was prepared from tert-butyl (*E*)-2-((dimethylamino)methylene)-3-oxo-8-azabicyclo[3.2.1]octane-8-carboxylate **I-1b** using a procedure essentially the same as for **I-1c:** LCMS (method 2) m/z 338.2 [M+H]⁺ (ES⁺); at 2.55 min. ¹H NMR (500 MHz, DMSO-d6) δ 8.70 (s, 1H), 8.44 - 8.27 (m, 2H), 7.51 (p, *J* = 3.4, 3.0 Hz, 3H), 5.02 (d, *J* = 5.9 Hz, 1H), 4.50 (s, 1H), 3.28 (d, *J* = 23.9 Hz, 1H), 2.82 (d, *J* = 18.1 Hz, 1H), 2.32 - 2.11 (m, 2H), 1.87 (t, *J* = 10.1 Hz, 1H), 1.75 (dd, *J* = 12.7, 6.4 Hz, 1H), 1.40 (s, 9H).

**Step 2:** 2-Phenyl-6,7,8,9-tetrahydro-5H-5,8-epiminocyclohepta[d]pyrimidine **(I-3)** was prepared from *tert-*butyl 2-phenyl-6,7,8,9-tetrahydro-5H-5,8-epiminocyclohepta[d]pyrimidine-10-carboxylate **(I-3a)** using a procedure essentially the same as for **I-1:** LCMS (method 2) m/z 230.1 [M+H]⁺ (ES⁺); at 1.18 min.

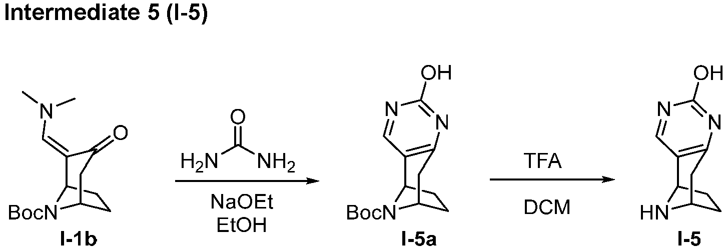

**Step 1:** To a solution of urea (0.2 g, 4 mmol) in EtOH (10 ml) was added a solution of sodium ethoxide (2 ml, 21% w/w in EtOH, 4 mmol). *tert*-Butyl (*E*)-2-((dimethylamino)methylene)-3-oxo-8-azabicyclo[3.2.1]octane-8-carboxylate I-1b (1 g, 4 mmol) in EtOH (10 ml) was added and the reaction mixture was heated to 90 °C for 16 h. Saturated ammonium chloride solution (5 ml) was added and the product was extracted with 10% (0.7 M ammonia/MeOH) in DCM solution (2 x 35 ml). The product was purified by silica gel chromatography (0-10% (0.7 M ammonia/MeOH) in DCM) to afford tert-butyl 2-hydroxy-6,7,8,9-tetrahydro-5H-5,8-epiminocyclohepta[d]pyrimidine-10-carboxylate **I-5a** as a white solid. LCMS (method 2) m/z 278.1 (M+H)⁺ (ES⁺), at 1.3 min. ¹H NMR (500 MHz, DMSO-d6) δ 11.68 (s, 1H), 7.96 (s, 1H), 4.79 (d, *J* = 6.0 Hz, 1H), 4.35 (s, 1H), 2.97 (d, *J =* 17.7 Hz, 1H), 2.16 (s, 1H), 2.02 (dt, *J* = 11.9, 5.6 Hz, 1H), 1.78 = 1.69 (m, 1H), 1.67 (d, *J* = 17.0 Hz, 1H), 1.36 (s, 9H). 1 proton under DMSO peak

**Step** 2: To a solution of *tert-butyl* 2-hydroxy-6,7,8,9-tetrahydro-5H-5,8-epiminocyclohepta[d]pyrimidine-10-carboxylate I-5a (361 mg, 1.30 mmol) in DCM (10 ml) was added TFA (1.00 ml, 13.0 mmol) slowly. The resultant mixture was stirred ON at RT. The reaction mixture was concentrated *in vacuo.* 0.7 M ammonia in MeOH (5 ml) was added and the mixture concentrated *in vacuo.* The material was purified by SCX eluting with 0.7 M ammonia in MeOH solution to provide 6,7,8,9-tetrahydro-5H-5,8-epiminocyclohepta[d]pyrimidin-2-ol **I-5** as a pale yellow solid. ¹H NMR (500 MHz, DMSO-d6) δ 7.79 (s, 1H), 7.27 (s, 1H), 6.67 (s, 1H), 4.05 (d, *J* = 5.7 Hz, 1H), 3.69 (t, *J* = 6.4 Hz, 1H), 2.88 - 2.78 (m, 1H), 2.34 (d, *J* = 18.5 Hz, 1H), 1.91 (d, *J* = 4.4 Hz, 1H), 1.89 - 1.81 (m, 1H), 1.74 - 1.62 (m, 1H), 1.56 - 1.46 (m, 1H).

### Intermediate 7 (I-7)

The following scheme makes use of synthetic methodology described in Schultz and Wolfe, Organic Letters, 2011, 13 (11), 2962-2965.

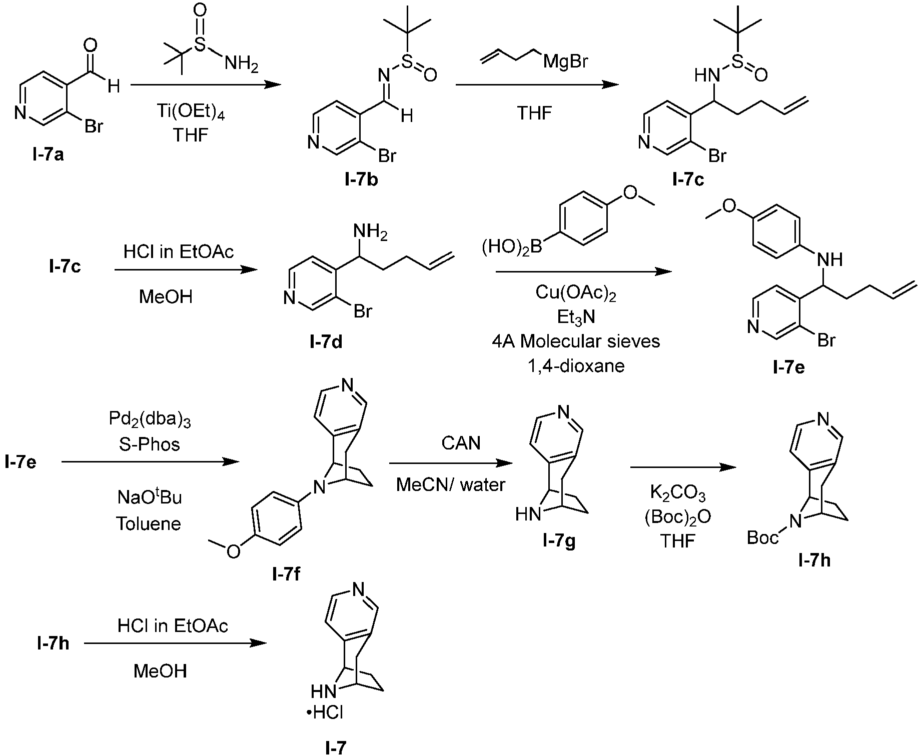

**Step 1:** To a solution of 3-bromo-4-pyridinecarboxaldehyde (42.0 g, 225 mmol) in THF (250 ml) was added titanium (IV) ethoxide (93.6 ml , 451 mmol) in one portion at RT. The mixture was stirred at RT for 5 min before (±)-*tert*-butylsulfinamide (30.1 g, 248 mmol) was added in one portion. The resulting mixture was stirred at RT for 16 h. Water (50 ml) was added at 0 °C, and the product was extracted with EtOAc (3 x 30 ml). The combined organic layers were concentrated *in vacuo.* The product was purified by silica gel chromatography (1-100% EtOAc/ petroleum ether to give (*E*)-*N*-((3-bromopyridin-4-yl)methylene)-2-methylpropane-2-sulfinamide **(I-7b)** as a yellow solid. ¹H NMR (400 MHz, CDCl₃) δ 8.91 (s, 1H), 8.86 (s, 1H), 8.62 (d, *J* = 4.8 Hz, 1H), 7.83 (d, *J* = 5.2 Hz, 1H), 1.29 (s, 9H).

**Step 2:** To a solution of (*E*)-*N*-((3-bromopyridin-4-yl)methylene)-2-methylpropane-2-sulfinamide **(I-7b)** (53.4 g, 185 mmol) in THF (300 ml) was added but-3-en-1-yl magnesium bromide (0.5 M, 1.59 L) dropwise at 0 °C. The mixture was warmed to RT over 1 h. Saturated ammonium chloride solution (200 ml) was added and the product was extracted with EtOAc (3 x 50 ml). The combined organics were washed with water (30 ml) and saturated brine (30 ml). The organics were dried with sodium sulfate and concentrated *in vacuo.* The product was purified by silica gel chromatography (1%-100% EtOAc/Petroleum ether) to give *N*-(1-(3-bromopyridin-4-yl)pent-4-en-1-yl)-2-methylpropane-2-sulfinamide **(I-7c)** as yellow oil. ¹H NMR (400 MHz, CDCl₃) δ 8.68 (s, 1H), 8.51-8.48 (m, 1H), 7.31 (d, *J* = 5.2 Hz, 1H), 5.83-5.76 (m, 1H), 5.10 (d, *J* = 1.2 Hz, 1H), 5.05 (d, *J* = 5.2 Hz, 1H), 4.87-4.83 (m, 1H), 3.56 (d, *J* = 3.2 Hz, 1H), 2.16-2.09 (m, 2H), 1.95-1.88 (m, 2H), 1.20 (d, *J* = 11.2 Hz , 9H).

**Step 3:** To a solution of *N*-(1-(3-bromopyridin-4-yl)pent-4-en-1-yl)-2-methylpropane-2-sulfinamide **(I-7c)** (54.3 g, 157 mmol) in MeOH (326 ml) was added a solution of HCl in EtOAc (4 M, 117 ml) at 0°C. The mixture was warmed to RT over 30 min. The reaction mixture was concentrated *in vacuo.* The residue was filtered and the filter cake was washed with EtOAc (2 x 10 ml) and dried *in vacuo* to give a pale yellow solid which was subsequently dissolved in water. The mixture was basified to pH > 11 with NaOH solution and extracted with DCM (3 x 30 ml). The combined organics were washed with brine (30 ml) and dried with sodium sulfate to give 1-(3-bromopyridin-4-yl)pent-4-en-1-amine **(I-7d)** as yellow-brown oil. ¹H NMR (400 MHz, CDCl₃) δ 8.64 (s, 1H), 8.48 (d, *J* = 5.6 Hz, 1H), 7.44 (d, *J* = 5.2 Hz, 1H), 5.86-5.81 (m, 1H), 5.07-4.98 (m, 2H), 4.32-4.29 (m, 1H), 2.20-2.13 (m, 2H), 1.81-1.83 (m, 1H), 1.52-1.64 (m, 1H), 1.52 (br. s, 2H).

**Step 4:** To a solution of 1-(3-bromopyridin-4-yl)pent-4-en-1-amine **(I-7d)** (26.5 g, 110 mmol), (4-methoxyphenyl)boronic acid (25.1 g, 165 mmol), 4 Å molecular sieves (53.0 g) and Cu(OAc)₂ (20.0 g, 110 mmol) in 1,4-dioxane (530 ml) was added Et₃N (20 ml, 143 mmol) dropwise. The mixture was stirred at 35 °C under O₂ for 16 h. The mixture was filtered and the filter cake was washed with EtOAc (3 x 15 ml). The filtrate was concentrated *in vacuo.* The product was purified by silica gel chromatography (1%-100% EtOAc/Petroleum ether) to give *N*-(1-(3-bromopyridin-4-yl)pent-4-en-1-yl)-4-methoxyaniline **(I-7e)** as a red-brown solid. ¹H NMR (400 MHz, CDCl₃) δ 8.68 (s, 1H), 8.40 (d, *J* = 5.2 Hz, 1H), 7.34 (d, *J* = 4.8 Hz, 1H), 6.71-6.67 (m, 2H), 6.36 (t, *J* = 2.0, 3.6 Hz, 2H), 5.87-5.83 (m, 1H), 5.09-5.02 (m, 2H), 4.65-4.62 (m, 1H), 3.94 (s, 1H), 3.69 (s, 3H), 2.31-2.23 (m, 2H), 1.94-1.91 (m, 1H), 1.74-1.71 (m, 1H).

**Step 5:** A flame-dried flask was cooled under a stream of N₂ and charged with Pd₂(dba)₃ (3.61 g, 3.95 mmol), S-Phos (3.24 g, 7.89 mmol) and NaO^{t}Bu (5.69 g, 59.2 mmol). The flask was purged with N₂ and a solution of *N*-(1-(3-bromopyridin-4-yl)pent-4-en-1-yl)-4-methoxyaniline **(I-7e)** (13.7 g, 39.5 mmol) in toluene (274 ml) was added dropwise. The resulting mixture was heated to 90 °C for 12 h. The reaction mixture was filtered and the filter cake was washed with EtOAc (3 x 10 ml). The filtrate was concentrated *in vacuo.* The product was purified by silica gel chromatography (1%-100% EtOAc/Petroleum ether to give 10-(4-methoxyphenyl)-6,7,8,9-tetrahydro-5H-5,8-epiminocyclohepta[c]pyridine **(I-7f)** as a pale yellow solid. ¹H NMR (400 MHz, CDCl₃) δ 8.33 (d, *J =* 4.8 Hz, 1H), 8.17 (s, 1H), 7.08 (d, *J =* 5.2 Hz, 1H), 6.77-6.71 (m, 4H), 4.61 (d, *J* = 6.0 Hz, 1H), 4.51 (t, *J =* 5.2, 6.8 Hz, 1H), 3.70 (s, 3H), 3.21 (dd, *J* = 4.8, 12.0 Hz, 1H), 2.43-2.35 (m, 3H), 1.95-1.91 (m, 1H), 1.79-1.78 (m, 1H).

**Step 6:** A solution of 10-(4-methoxyphenyl)-6,7,8,9-tetrahydro-5H-5,8-epiminocyclohepta[c]pyridine **(I-7f)** (6.32 g, 23.7 mmol) in MeCN (237 ml) was cooled to 0 °C before an aqueous solution of CAN (0.3 M, 316 ml) was added dropwise. After the addition was complete the reaction was stirred for 1 h at 0 °C. The reaction mixture was concentrated to dryness *in vacuo* to afford 6,7,8,9-tetrahydro-5H-5,8-epiminocyclohepta[c]pyridine **(I-7g)** as a brown oil, which was used in the subsequent step without any further purification.

**Step 7:** To a solution of 6,7,8,9-tetrahydro-5H-5,8-epiminocyclohepta[c]pyridine **(I-7g)** (3.82 g, 23.9 mmol) in THF (30 ml) was added K₂CO₃ (8.25 g, 59.7 mmol) and di-*tert*-butyl dicarbonate (26.0 g, 119 mmol). The mixture was stirred at RT for 16 h. The reaction mixture was filtered and the filter cake was washed with EtOAc (3 x 15 ml). Water (25 ml) was added to the filtrate. The aqueous layer was extracted with EtOAc (3 x 25 ml). The combined organic layers were dried over sodium sulfate and concentrated *in vacuo.* The product was purified by silica gel chromatography (1%-100% EtOAc/Petroleum ether) to give *tert*-butyl-6,7,8,9-tetrahydro-5H-5,8-epiminocyclohepta[c]pyridine-10-carboxylate **(I-7h)** as a red-brown solid. ¹H NMR (400 MHz, CDCl₃) δ 8.34-8.32 (m, 2H), 6.98 (s, 1H), 4.86 (d, *J* = 28.8 Hz, 1H), 4.58 (s, 1H), 3.36 (s, 1H), 2.56 (d, *J* = 16.8 Hz, 1H), 2.24-2.18 (m, 2H), 1.86-1.81 (m, 1H), 1.64-1.65 (m, 1H), 1.42 (s, 9H).

**Step** 8: A solution of HCl in EtOAc (4 M, 550 ml) was added to a solution of *tert-*butyl-6,7,8,9-tetrahydro-5H-5,8-epiminocyclohepta[c]pyridine-10-carboxylate **(I-7h)** (2.20 g, 8.45 mmol) in MeOH (400 ml) at RT. The mixture was stirred at RT for 2 h. The reaction mixture was concentrated *in vacuo.* The crude product was triturated with MTBE (20 ml) at RT for 10 min. The mixture was filtered, the filter cake was washed with MTBE (2 x 5 ml) and the cake was dried *in vacuo* to give 6,7,8,9-tetrahydro-5H-5,8-epiminocyclohepta[c]pyridine hydrochloride as a white solid. LCMS (method 5) m/z 161.2 (M+H)⁺ (ES⁺), at 2.44 min. ¹H NMR (400 MHz, CDCl₃) δ 9.92 (s, 1H), 9.65 (s, 1H), 8.64 (d, *J* = 18.0 Hz, 2H), 7.64 (s, 1H), 5.03 (s, 1H), 4.40 (s, 1H), 3.39 (s, 1H), 3.00 (d, *J* = 17.6 Hz, 1H), 2.36-2.18 (m, 2H), 1.99-2.02 (m, 1H), 1.86-1.75 (m, 1H).

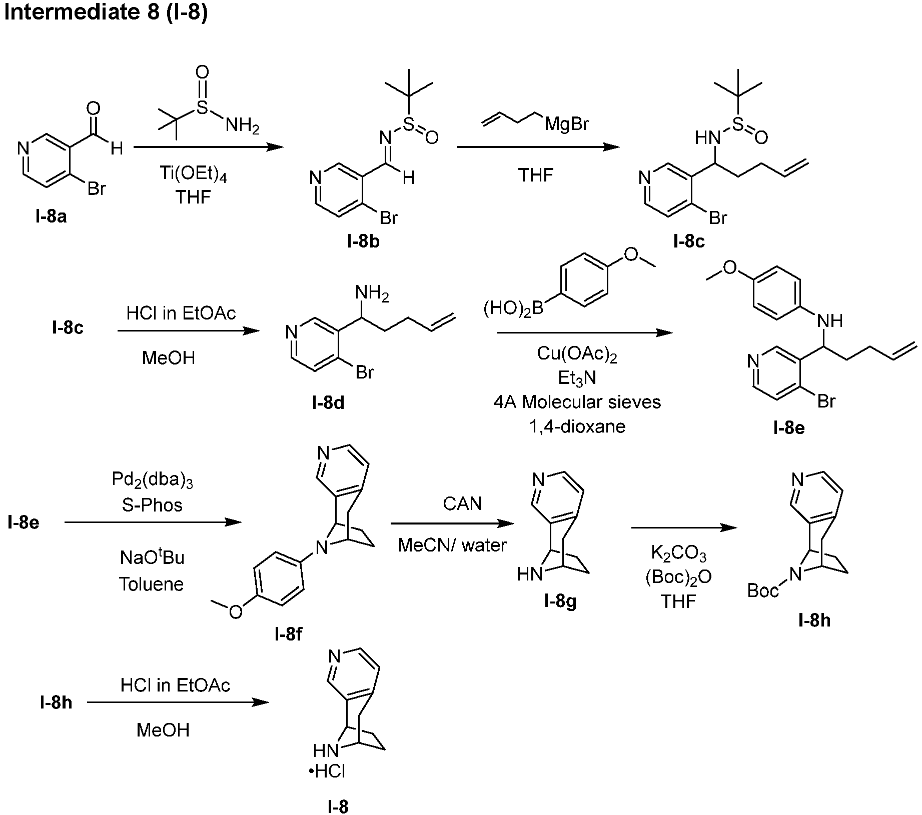

**Step 1:** (*E*)-*N*-((4-Bromopyridin-3-yl)methylene)-2-methylpropane-2-sulfinamide **(I-8b)** was synthesised from 4-bromo-3-pyridinecarboxaldehyde **(I-8a)** using a procedure essentially the same as for **I-7b.** ¹H NMR (400 MHz, CDCl₃) δ 9.14 (s, 1H), 8.93 (s, 1H), 8.46 (d, *J* = 5.2 Hz, 1H), 7.61 (d, *J* = 5.2 Hz, 1H), 1.30 (s, 9H).

**Step 2:** *N*-(1-(4-Bromopyridin-3-yl)pent-4-en-1-yl)-2-methylpropane-2-sulfinamide **(I-8c)** was synthesised from (*E*)-*N*-((4-bromopyridin-3-yl)methylene)-2-methylpropane-2-sulfinamide **(I-8b)** using a procedure essentially the same as for **I-7c.** ¹H NMR (400 MHz, CDCl₃) δ: 8.55 (s, 1H), 8.32-8.29 (m, 1H), 7.50 (d, *J* = 5.2 Hz, 1H), 5.85-5.76 (m, 1H), 5.10-5.02 (m, 2H), 4.92-4.90 (m, 1H), 3.57 (d, *J* = 3.6 Hz, 1H), 2.18-1.97 (m, 4H), 1.19 (s, 9H).

**Step 3:** 1-(4-Bromopyridin-3-yl)pent-4-en-1-amine **(I-8d)** was synthesised from *N*-(1-(4-bromopyridin-3-yl)pent-4-en-1-yl)-2-methylpropane-2-sulfinamide **(I-8c)** using a procedure essentially the same as for **I-7d.** ¹H NMR (400 MHz, CDCl₃) δ 8.66 (s, 1H), 8.29-8.24 (m, 1H), 7.48-7.45 (t, *J* = 8.8 Hz, 1H), 5.86-5.79 (m, 1H), 5.06-4.97 (m, 2H), 4.36-4.33 (m, 1H), 2.20-2.12 (m, 2H), 1.89-1.85 (m, 1H), 1.78-1.75 (m, 1H), 1.56 (s, 2H).

**Step 4:** *N*-(1-(4-Bromopyridin-3-yl)pent-4-en-1-yl)-4-methoxyaniline **(I-8e)** was synthesised from 1-(4-bromopyridin-3-yl)pent-4-en-1-amine **(I-8d)** using a procedure essentially the same as for **I-7e.** ¹H NMR (400 MHz, CDCl₃) δ 8.58 (d, *J* = 15.6 Hz, 1H), 8.26 (s, 1H), 7.49 (d, *J* = 5.2 Hz, 1H), 6.69 (d, *J* = 8.8 Hz, 2H), 6.44-6.41 (m, 2H), 5.88-5.82 (m, 1H), 5.08-5.01 (m, 2H), 4.72-4.68 (m, 1H), 3.86-3.84 (m, 1H), 3.69 (s, 3H), 2.30-2.21 (m, 2H), 2.02-1.89 (m, 1H), 1.88-1.76 (m, 1H).

**Step 5:** 10-(4-Methoxyphenyl)-6,7,8,9-tetrahydro-5H-6,9-epiminocyclohepta[c]pyridine **(I-8f)** was synthesised from *N*-(1-(4-bromopyridin-3-yl)pent-4-en-1-yl)-4-methoxyaniline (I-8e) using a procedure essentially the same as for **I-7f.** ¹H NMR (400 MHz, CDCl₃) δ 8.39 (s, 1H), 8.27 (d, *J* = 5.2 Hz, 1H), 6.84 (d, *J* = 4.4 Hz, 1H), 6.77-6.70 (m, 4H), 4.72 (d, *J* = 5.2 Hz, 1H), 4.65 (d, *J* = 5.2 Hz, 1H), 3.69 (s, 3H), 3.25-3.19 (m, 1H), 2.40 (dd, *J* = 3.6, 7.2 Hz, 3H), 1.97-1.92 (m, 1H), 1.79-1.75 (m, 1H).

**Step 6:** 6,7,8,9-Tetrahydro-5H-6,9-epiminocyclohepta[c]pyridine **(I-8g)** was synthesised from 10-(4-methoxyphenyl)-6,7,8,9-tetrahydro-5H-6,9-epiminocyclohepta[c]pyridine **(I-8f)** using a procedure essentially the same as for **I-7g.**

**Step 7:** *tert*-Butyl-6,7,8,9-tetrahydro-5H-6,9-epiminocyclohepta[c]pyridine-10-carboxylate **(I-8h)** was prepared from 6,7,8,9-tetrahydro-5H-6,9-epiminocyclohepta[c]pyridine **(I-8g)** using a procedure essentially the same as for **I-7h.** ¹H NMR (400 MHz, CDCl₃) δ 8.36 (d, *J* = 5.2 Hz, 1H), 8.31 (s, 1H), 7.01 (d, *J* = 4.8 Hz, 1H), 4.95 (s, 1H), 4.54 (s, 1H), 3.37 (s, 1H), 2.54 (d, *J* = 17.6 Hz, 1H), 2.33-2.16 (m, 2H), 1.93-1.81 (m, 1H), 1.70-1.59 (m, 1H), 1.47-1.35 (m, 9H).

**Step 8:** 6,7,8,9-Tetrahydro-5H-6,9-epiminocyclohepta[c]pyridine hydrochloride **(I-8)** was synthesised from *tert*-butyl-6,7,8,9-tetrahydro-5H-6,9-epiminocyclohepta[c]pyridine-10-carboxylate **(I-8h)** using a procedure essentially the same as for **I-7.** LCMS (method 5) m/z 161.2 (M+H)⁺ (ES⁺), at 2.42 min. ¹H NMR (400 MHz, CDCl₃) δ 10.26 (s, 1H), 9.90 (s, 1H), 8.79 (s, 1H), 8.71 (d, *J* = 6.0 Hz, 1H), 7.77 (d, *J* = 5.6 Hz, 1H), 5.10 (d, *J* = 6.0 Hz, 1H), 4.36 (s, 1H), 3.54 (dd, *J* = 4.8, 19.2 Hz, 1H), 3.19-3.07 (m, 1H), 2.41-2.18 (m, 2H), 2.08 (t, J = 11.2 Hz, 1H), 1.88-1.75 (m, 1H).

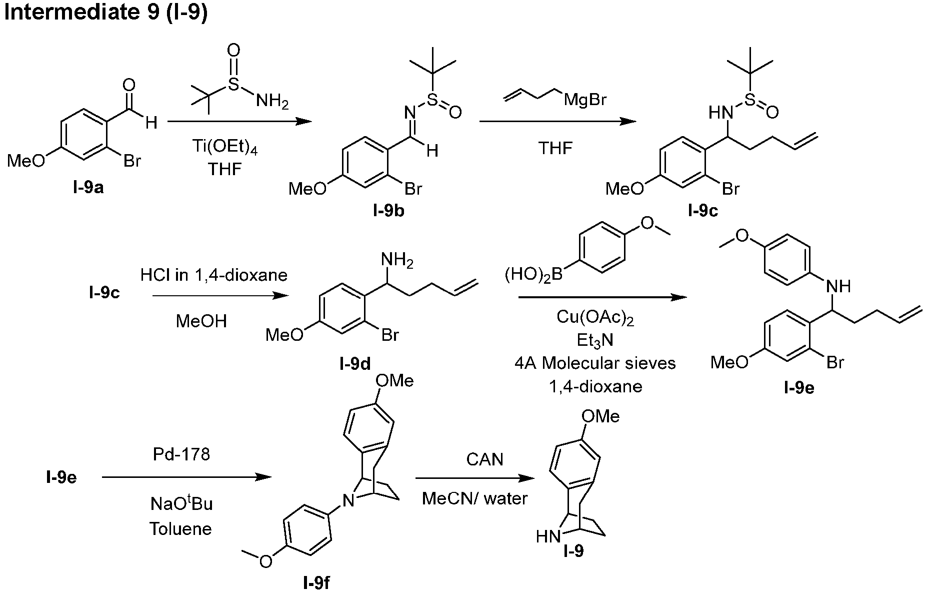

**Step** 1: (*E*)-*N*-(2-Bromo-4-methoxybenzylidene)-2-methylpropane-2- sulfinamide **(I-9b)** was synthesised from 2-bromo-4-methoxybenzaldehyde (**I-9a**) using a procedure essentially the same as for **I-7b.** ¹H NMR (500 MHz, d6-DMSO) δ 8.73 (s, 1H), 8.01 (d, *J* = 8.8 Hz, 1H), 7.37 (d, *J* = 2.5 Hz, 1H), 7.13 (dd, *J* = 8.8, 2.4 Hz, 1H), 3.88 (s, 3H), 1.18 (s, 9H).

**Step** 2: A solution of (*E*)-*N*-(2-bromo-4-methoxybenzylidene)-2-methylpropane-2-sulfinamide (13.0 g, 40.9 mmol) in THF (130 ml) was cooled down to -78 °C. But-3-en-1-ylmagnesium bromide (0.5 M in THF) (245 ml, 123 mmol) was added dropwise. The mixture was slowly warmed to RT and the mixture was stirred at RT for 16 h. Saturated aqueous ammonium chloride solution (150 ml) was added and the product was extracted with EtOAc (2 x 100 ml). The organics were combined, dried over magnesium sulfate and concentrated *in vacuo* to obtain *N*-(1-(2-bromo-4-methoxyphenyl)pent-4-en-1-yl)-2-methylpropane-2-sulfinamide **(I-9c)** as a light brown oil. The compound was taken to the next step without any further purification as a 2:1 mixture of diastereoisomers.

Major diastereoisomer :¹H NMR (500 MHz, d6-DMSO) δ 7.42 (d, *J =* 8.7 Hz, 1H), 7.11 (d, *J* = 2.5 Hz, 1H), 6.99 (dd, *J* = 8.7, 2.6 Hz, 1H), 5.82 (ddt, *J* = 16.9, 10.2, 6.5 Hz, 1H), 5.51 (d, *J =* 6.5 Hz, 1H), 5.06 (d, *J =* 17.4 Hz, 1H), 4.98 (d, *J =* 10.8 Hz, 1H), 4.57 (d, *J =* 6.8 Hz, 1H), 3.76 (s, 3H), 2.19-2.08 (m, 1H), 2.07-1.97 (m, 1H), 1.94-1.84 (m, 1H), 1.78-1.67 (m, 1H), 1.05 (s, 9H).

Minor diastereoisomer: ¹H NMR (500 MHz, d6-DMSO) δ 7.51 (d, *J* = 8.7 Hz, 1H), 7.11 (d, *J* = 2.5 Hz, 1H), 6.99 (dd, *J* = 8.5, 2.5 Hz, 1H), 5.87-5.78 (m, 2H), 5.09-4.9 (m, 2H), 4.56-4.45 (m, 1H), 3.77 (s, 3H), 2.23-2.09 (m, 1H), 2.08-1.97 (m, 1H), 1.83-1.70 (m, 1H), 1.68-1.59 (m, 1H), 1.10 (s, 9H).

**Step 3:** To a solution of *N*-(1-(2-bromo-4-methoxyphenyl)pent-4-en-1-yl)-2-methylpropane-2-sulfinamide (**I-9c**) (16.5 g, 44.16 mmol) in MeOH (65 ml) was added a solution of HCl (4M in 1,4-dioxane) (33 ml, 132.5 mmol) at RT. The reaction mixture was stirred at RT for 2.5 h. Water (200 ml) was added and the aqueous solution was washed with EtOAc (200 ml). The aqueous layer was adjusted to pH 8 by the addition of 2M NaOH aqueous solution. The aqueous layer was then extracted with EtOAc (2 x 100 ml). The organics were combined, dried over magnesium sulfate and concentrated *in vacuo* to afford 1-(2-bromo-4-methoxyphenyl)pent-4-en-1-amine (**I-9d**) as a pale brown liquid. The crude product was used in the next step without further purification.

**Step 4:** To a solution of 1-(2-bromo-4-methoxyphenyl)pent-4-en-1-amine (**I-9d**) (7.60 g, 28.1 mmol) in DCM (100 ml) was added (4-methoxyphenyl)boronic acid (12.8 g, 84.4 mmol) followed by copper(II) acetate (7.66 g, 42.2 mmol) and pyridine (11.4 ml, 141 mmol). The blue mixture was vigorously stirred at RT for 72 h. 2M NaOH aqueous solution (100 ml) was added and the mixture was stirred for 30 min. The copper salts were filtered through a pad of Celite. Water (100 ml) was added and the product was extracted with DCM (3 x 100 ml). The organic layers were combined, dried over magnesium sulfate, and concentrated *in vacuo.* The product was purified by chromatography on silica gel (5-25% EtOAc/Isohexane) to afford *N*-(1-(2-bromo-4-methoxyphenyl)pent-4-en-1-yl)-4-methoxyaniline(I-9e) as a yellow liquid. LCMS (method 2) m/z 255 (M-PMP)⁺ (ES⁺), at 1.91 min, ¹H NMR (500 MHz, DMSO-d6) δ 7.33 (d, *J* = 8.7 Hz, 1H), 7.12 (d, *J =* 2.6 Hz, 1H), 6.90 (dd, *J* = 8.7, 2.6 Hz, 1H), 6.62 (d, *J =* 8.9 Hz, 2H), 6.37 (d, *J =* 9.0 Hz, 2H), 5.92 (d, *J =* 8.1 Hz, 1H), 5.85 (ddt, *J* = 17.0, 10.2, 6.5, 6.5 Hz, 1H), 5.02 (dq, *J* = 17.2, 1.8, 1.7, 1.7 Hz, 1H), 4.97 (ddt, *J* = 10.2, 2.3, 1.3, 1.3 Hz, 1H), 4.51 (td, *J* = 8.2, 8.2, 5.0 Hz, 1H), 3.73 (s, 3H), 3.57 (s, 3H), 2.34 - 2.20 (m, 1H), 2.19 - 2.06 (m, 1H), 1.78 - 1.61 (m, 2H).

**Step 5:** To a flask was added Pd-178 (667 mg, 1.40 mmol) and NaO^{t}Bu (2.02 g, 21.0 mmol) and the flask was purged with nitrogen. A solution of N-(1-(2-bromo-4-methoxyphenyl)pent-4-en-1-yl)-4-methoxyaniline (**I-9e**) (5.26 g, 14.0 mmol) in degassed toluene (120 ml) was added *via* syringe at RT. The resulting mixture was heated at 95 °C for 2 h. The reaction mixture was cooled to RT and filtered through celite. The celite was washed with EtOAc (100 ml). The filtrate was concentrated *in vacuo.* The product was purified by chromatography on silica gel (0-25% EtOAc/isohexane) to afford (±)-2-methoxy-10-(4-methoxyphenyl)-6,7,8,9-tetrahydro-5H-5,8-epiminobenzo[7]annulene **(I-9f)** as a pale pink oil. LCMS (method 2) m/z 296 (M+H)⁺ (ES⁺), at 1.27 min,¹H NMR (500 MHz, DMSO-d6) δ 7.12 (d, *J* = 8.3 Hz, 1H), 6.76 (d, *J =* 9.1 Hz, 2H), 6.69 (d, *J* = 9.1 Hz, 2H), 6.65 (dd, *J* = 8.3, 2.7 Hz, 1H), 6.48 (d, *J =* 2.6 Hz, 1H), 4.70 (d, *J* = 5.6 Hz, 1H), 4.54 - 4.38 (m, 1H), 3.64 (s, 3H), 3.61 (s, 3H), 3.10 (dd, *J* = 17.0, 4.7 Hz, 1H), 2.36 (d, *J =* 17.0 Hz, 1H), 2.30 - 2.13 (m, 2H), 1.80 - 1.63 (m, 2H).

**Step 6:** To a solution of (±)-2-methoxy-10-(4-methoxyphenyl)-6,7,8,9-tetrahydro-5H-5,8-epiminobenzo[7]annulene **(I-9f)** (680.0 mg, 2.30 mmol) in MeCN (25 ml) was added a solution of CAN (3.79 g, 6.91 mmol) in water (25 ml) was added dropwise over 30 min at 0 °C. The mixture was stirred at 0 °C for 1 h, and at RT for 1 h. Water (10 ml) and 2M NaOH (10 ml) were added and the product was extracted with DCM (3 x 30 ml). The organic layers were combined and concentrated *in vacuo* to obtain (±)-2-methoxy-6,7,8,9-tetrahydro-5H-5,8-epiminobenzo[7]annulene (**I-9**) as a brown oil. The compound was used as is without further purification. LCMS (method 2) m/z 190 (M+H)⁺ (ES⁺), at 1.56 min, ¹H NMR (500 MHz, DMSO-d6) δ 6.89 (d, *J* = 8.2 Hz, 1H), 6.61 (dd, *J* = 8.2, 2.7 Hz, 1H), 6.58 (d, *J =* 2.6 Hz, 1H), 4.04 (d, *J =* 5.1 Hz, 1H), 3.73-3.66 (m, 4H), 3.01 (dd, *J* = 16.6, 5.0 Hz, 1H), 2.64 (br s, 1H), 2.44 (d, *J* = 16.6 Hz, 1H), 1.95 - 1.81 (m, 2H), 1.74 - 1.57 (m, 1H), 1.50 - 1.39 (m, 1H).

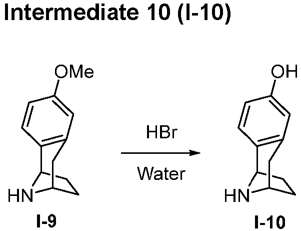

**Step 1:** (±)-2-Methoxy-6,7,8,9-tetrahydro-5H-5,8-epiminobenzo[7]annulene (**I-9**) (267 mg, 1.24 mmol) was added to aqueous HBr (48% solution in water) (1.4 ml, 48% w/w, 12.41 mmol) at RT, and then refluxed for 6 h. The mixture was diluted with MeOH (20 ml) and filtered to get a clear dark brown filtrate. SCX (7.00 g, 5.4 mmol) was added and the mixture was further stirred at RT for 1 h. The SCX was washed with MeOH (100 ml) and the product was eluted with 0.7 M ammonia in MeOH (50 ml). The eluant was concentrated *in vacuo* to obtain (±)-6,7,8,9-tetrahydro-5H-5,8-epiminobenzo[7]annulen-2-ol, as a dark brown oil. LCMS (method 2) m/z 176 (M+H)⁺ (ES⁺), at 0.61 min, 1H NMR (500 MHz, DMSO-d6) δ 9.00 (s, 1H), 6.77 (d, *J =* 8.1 Hz, 1H), 6.44 (dd, *J* = 8.1, 2.5 Hz, 1H), 6.41 (d, *J =* 2.5 Hz, 1H), 4.00 (d, *J* = 5.3 Hz, 1H), 3.67 (t, *J =* 5.8 Hz, 1H), 2.96 (dd, *J* = 16.5, 4.9 Hz, 1H), 2.37 (d, *J* = 16.5, 1H), 1.94-1.80 (m, 2H), 1.68-1.59 (m, 1H), 1.48-1.39 (m, 1H), 1 exchangeable proton not visible

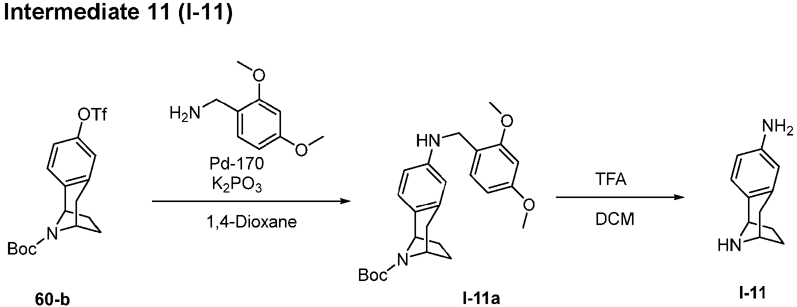

**Step 1:** A vial was charged with Pd-170 (1 mg, 1.45 µmol) and K₂PO₃ (23 mg, 145 µmol), sealed and evacuated/ backfilled with N₂ (3 times). A solution of *tert*-butyl (±)-2-(((trifluoromethyl)sulfonyl)oxy)-6,7,8,9-tetrahydro-5H-5,8-epiminobenzo[7]annulene-10-carboxylate (40 mg, 97.0 µmol) and (2,4-dimethoxyphenyl)methanamine (18 mg, 107 µmol) in 1,4-dioxane (1 ml) was added and the reaction mixture heated to 120 °C for 16 h. The reaction mixture was cooled to RT and filtered through a pad of celite washing with EtOAc. The filtrate was concentrated *in* vacuo. The product was purified by silica gel chromatography (0%-100% EtOAc/isohexane) to give *tert*-butyl (±)-2-((2,4-dimethoxybenzyl)amino)-6,7,8,9-tetrahydro-5H-5,8-epiminobenzo[7]annulene-10-carboxylate (**I-11a**) as yellow oil. LC-MS (method 1) m/z 447.1 (M+Na)⁺ (ES⁺) at 1.82 min, ¹H NMR (500 MHz, DMSO-d6) δ 7.18 (dd, *J =* 8.3, 1.9 Hz, 1H), 6.84 (br s, 1H), 6.53 - 6.33 (m, 4H), 4.90 - 4.72 (m, 1H), 4.60 - 4.37 (m, 1H), 3.82 (s, 3H), 3.79 (s, 3H), 3.39 - 3.18 (m, 1H), 2.45 (d, *J* = 16.5 Hz, 1H), 2.30 - 2.02 (m, 3H), 1.79 (t, *J =* 10.3 Hz, 1H), 1.62 (br s, 2H), 1.39 (s, 9H). (-NH proton not visible).

**Step 2:** To a solution of *tert*-butyl (±)-2-((2,4-dimethoxybenzyl)amino)-6,7,8,9-tetrahydro-5H-5,8-epiminobenzo[7]annulene-10-carboxylate **(I-11a)** (30 mg, 69.7 µmol) in DCM (1 ml) was added TFA (54 µl, 0.70 mmol) and the reaction mixture stirred at RT overnight. The reaction mixture was concentrated *in vacuo.* The product was purified by ion exchange using SCX (0.5 g) washing with MeOH and eluting with 0.7 M ammonia in MeOH to give (±)-6,7,8,9-tetrahydro-5H-5,8-epiminobenzo[7]annulen-2-amine **(I-11)** as a sticky yellow oil. LC-MS (method 1) m/z 175.8 (M+H)⁺ (ES⁺) at 0.71 min.

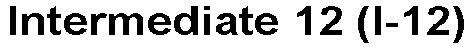

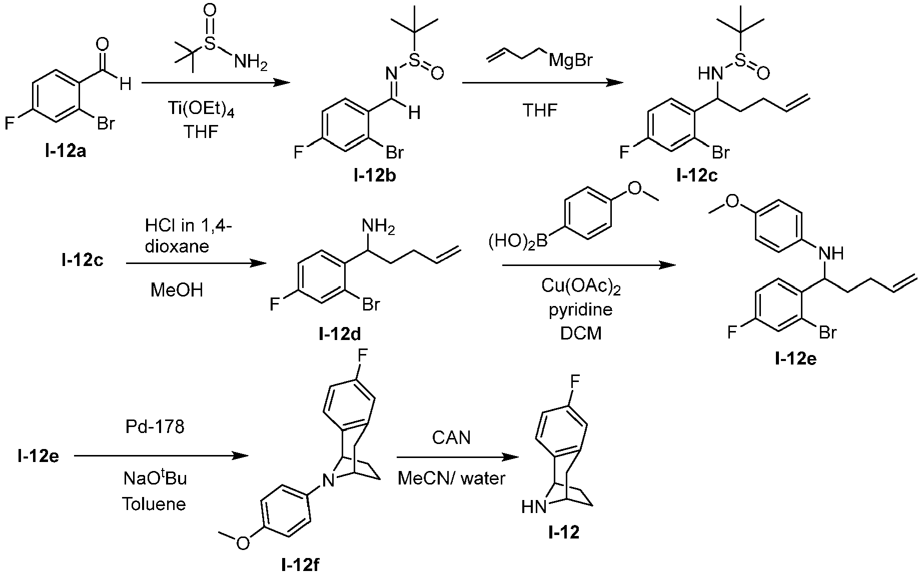

**Step 1:** To a solution of 2-bromo-4-fluorobenzaldehyde **I-12a** (10.0 g, 49.3 mmol) in THF (170 ml) was added titanium (IV) ethoxide (20.7 ml, 98.5 mmol) in one portion at RT. The mixture was stirred at RT for 5 min before (±)-*tert*-butylsulfinamide (5.97 g, 49.3 mmol) was added in one portion. The resulting mixture was stirred at RT for 24 h. Brine (100 ml) was added and the mixture stirred for 10 min then filtered through celite. The filtrate was extracted with EtOAc (2 x 100 ml). The combined organic layers were washed with brine (3 x 100 ml), dried over magnesium sulfate and concentrated *in vacuo* to give (*E*)-*N*-(2-bromo-4-fluorobenzylidene)-2-methylpropane-2-sulfinamide as pale yellow solid **(I-12b).** LCMS (method 1) m/z 306.2, 308.1 (M+H)⁺ (ES⁺), at 1.6 min. ¹H NMR (500 MHz, DMSO-*d*₆) δ 8.77 (s, 1H), 8.11 (dd, *J* = 8.8, 6.2 Hz, 1H), 7.81 (dd, *J* = 8.5, 2.6 Hz, 1H), 7.45 (td, *J* = 8.2, 2.6 Hz, 1H), 1.20 (s, 9H).

**Step 2:** To a solution of (*E*)-*N*-(2-bromo-4-fluorobenzylidene)-2-methylpropane-2-sulfinamide (13.00 g, 42.46 mmol) **(I-12b)** in THF (150 ml) was added but-3-en-1-yl magnesium bromide (2.17 M, 29.4 ml) dropwise at -78 °C. The mixture was warmed to RT over 16 h. Saturated ammonium chloride solution (50 ml) was added and the product was extracted with EtOAc (2 x 100 ml). The combined organics were dried with magnesium sulfate and concentrated *in vacuo,* to give *N*-(1-(2-bromo-4-fluorophenyl)pent-4-en-1-yl)-2-methylpropane-2-sulfinamide **(I-12c)** as an orange oil. LCMS (method 2) m/z 362.1, 364.1 (M+H)⁺ (ES⁺) at 2.48 min

**Step 3:** To a solution of *N*-(1-(2-bromo-4-fluorophenyl)pent-4-en-1-yl)-2-methylpropane-2-sulfinamide **(I-12c)** (15.0 g, 41.26 mmol) in MeOH (65 ml) was added a solution of HCl in 1,4-dioxane (4 M, 10.3 ml) at RT and stirred for 2.5 h. The reaction mixture was concentrated *in vacuo.* The residue was partitioned between water (200 ml) and EtOAc (200 ml). The aqueous phase was basified to pH 8 with NaOH solution and extracted with EtOAc (2 x 100 ml). The organic layer was diluted with water (50 ml) basified to pH 10 with NaOH and extracted with ethyl acetate (3 x 100 ml). The combined organics were dried over magnesium sulfate and concentrated *in vacuo* to give 1-(2-bromo-4-fluorophenyl)pent-4-en-1-amine **(I-12d)** as a colourless oil. LCMS (method 2) m/z 258.0, 260.0 (M+H)⁺ (ES⁺), at 2.20 min. ¹H NMR (500 MHz, DMSO-*d*₆) δ 7.66 (dd, *J* = 8.7, 6.4 Hz, 1H), 7.47 (ddt, *J* = 8.5, 3.0, 1.6 Hz, 1H), 7.30 - 7.22 (m, 1H), 5.81 (ddt, *J* = 16.9, 10.2, 6.6 Hz, 1H), 5.09-4.92 (m, 1H), 4.94 (ddt, *J* = 10.3, 2.2, 1.1 Hz, 1H), 4.13 (dd, *J* = 8.1, 5.0 Hz, 1H), 2.19 - 2.07 (m, 1H), 2.10 - 1.97 (m, 1H), 1.66-1.58 (m, 1H), 1.57-1.46 (m, 1H) (Exchangeable -NH₂ protons not visible).

**Step** 4: To a solution of 1-(2-bromo-4-fluorophenyl)pent-4-en-1-amine (9.60 g, 37.2 mmol) **(I-12d),** (4-methoxyphenyl)boronic acid (17.0 g, 112 mmol), and Cu(OAc)₂ (10.1 g, 55.8 mmol) in DCM (100 ml) was added pyridine (15.0 ml, 186 mmol) dropwise. The mixture was stirred at RT open to air for 16 h. 2 M NaOH aqueous solution (100 ml) was added, the mixture stirred for 30 min and filtered through celite. The filtrate was partitioned between water (100 ml) and DCM (100 ml) and the layers were separated. The aqueous layer was further extracted with DCM (2 x 100 ml). The combined organics were dried over magnesium sulfate and concentrated *in vacuo.* The product was purified by silica gel chromatography (0%-10% EtOAc/isohexane) followed by silica gel chromatography (0%-50% DCM/ isohexane) to give N-(1-(2-bromo-4-fluorophenyl)pent-4-en-1-yl)-4-methoxyaniline (**I-12e**) as a colourless oil. LCMS (method 2) m/z 364.3, 366.6 (M+H)⁺ (ES⁺), at 1.55 min. ¹H NMR (500 MHz, DMSO-*d*₆) δ 7.52 (dd, *J* = 8.5, 2.6 Hz, 1H), 7.47 (dd, *J* = 8.7, 6.3 Hz, 1H), 7.20 (td, *J* = 8.5, 2.7 Hz, 1H), 6.67 - 6.60 (m, 2H), 6.40 - 6.33 (m, 2H), 6.00 (d, *J* = 8.0 Hz, 1H), 5.90 - 5.79 (m, 1H), 5.06 - 4.94 (m, 2H), 4.55 (td, *J* = 8.2, 5.0 Hz, 1H), 3.57 (s, 3H), 2.29 (t, *J =* 11.2 Hz, 1H), 2.21 - 2.10 (m, 1H), 1.79 - 1.64 (m, 2H).

**Step 5:** A three-neck flask was charged with Pd-178 (448 mg, 939 µmol) and NaO^{t}Bu (3.99 g, 41.5 mmol) and purged with N₂. A solution of *N*-(1-(2-bromo-4-fluorophenyl)pent-4-en-1-yl)-4-methoxyaniline (**I-12e)** (3.42 g, 9.39 mmol) in toluene (30 ml) was added dropwise. The resulting mixture was heated to 95 °C for 2 h. The reaction mixture was cooled to RT and filtered through celite, washing with EtOAc (100 ml). The filtrate was concentrated *in vacuo.* The product was purified by silica gel chromatography (0%-5% EtOAc/heptane) to give (±)-2-fluoro-10-(4-methoxyphenyl)-6,7,8,9-tetrahydro-5H-5,8-epiminobenzo[7]annulene (**I-12f**) as a pink oil. LCMS (method 2) m/z 384.1 (M+H)⁺ (ES⁺), at 2.52 min. ¹H NMR (500 MHz, DMSO-*d*₆) δ 7.26 (dd, *J* = 8.3, 6.0 Hz, 1H), 6.90 (td, *J* = 8.7, 2.7 Hz, 1H), 6.80 - 6.73 (m, 3H), 6.73 - 6.66 (m, 2H), 4.79 (d, *J =* 5.6 Hz, 1H), 4.44 (t, *J =* 5.8 Hz, 1H), 3.61 (s, 3H), 3.11 (dd, *J* = 17.3, 4.8 Hz, 1H), 2.41 (d, *J =* 17.3 Hz, 1H), 2.26 - 2.18 (m, 2H), 1.84 - 1.62 (m, 2H).

**Step 6:** A solution of (±)-2-fluoro-10-(4-methoxyphenyl)-6,7,8,9-tetrahydro-5H-5,8-epiminobenzo[7]annulene **(I-12f)** (2.15 g, 7.59 mmol) in MeCN (28 ml) was cooled to 0 °C before a solution of CAN (12.5 g, 22.8 mmol) in water (100 ml) was added dropwise. After the addition was completed the reaction was stirred for 1 h at 0 °C, then allowed to warm to RT and stirred for 1 h. Water (30 ml) and 2 M aqueous sodium hydroxide (50 ml) was added and the product extracted with DCM (4 x 150 ml). The combined organic layers were dried over magnesium sulfate and concentrated *in vacuo.* The crude residue was redissolved in MeOH and purified by SCX (15 g) washing with MeOH and eluting with 0.7 M ammonia in MeOH to give (±)-2-fluoro-6,7,8,9-tetrahydro-5H-5,8-epiminobenzo[7]annulene (**I-12**) as a brown oil. LCMS (method 2) m/z 178.1 (M+H)⁺ (ES⁺), at 1.65 min. ¹H NMR (500 MHz, DMSO-*d*₆) δ 7.02 (dd, *J* = 6.3, 1.9 Hz, 1H), 6.94 - 6.81 (m, 2H), 4.12 - 4.07 (m, 1H), 3.68 (t, *J* = 5.5 Hz, 1H), 3.02 (dd, *J* = 16.8, 5.0 Hz, 1H), 2.50 - 2.43 (m, 2H), 1.95 - 1.75 (m, 2H), 1.73 - 1.61 (m, 1H), 1.52 - 1.39 (m, 1H).

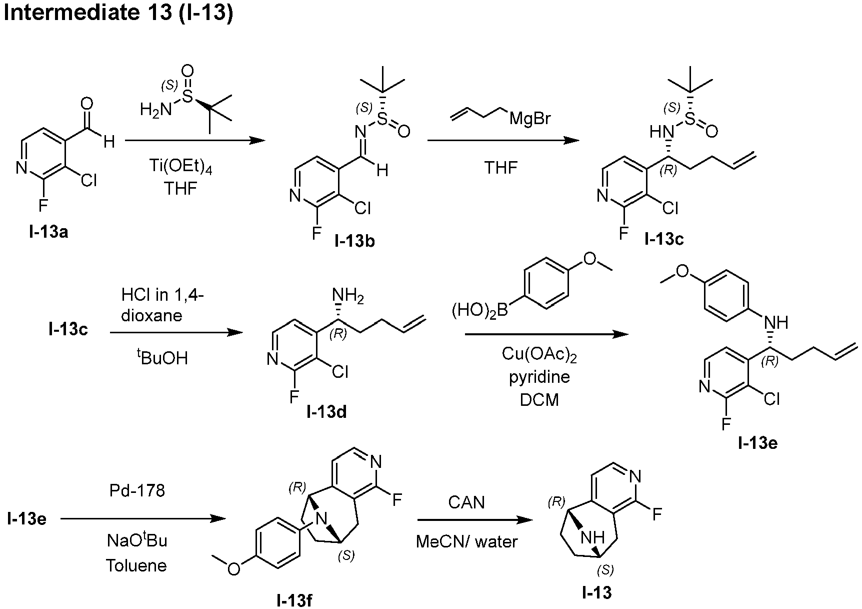

**Step 1:** To a solution of 3-chloro-2-fluoroisonicotinaldehyde **I-13a** (1.00 g, 6.27 mmol) in THF (17 ml) was added titanium (IV) ethoxide (2.63 ml, 12.5 mmol) in one portion at RT. The mixture was stirred at RT for 5 min before (*S*)-*tert*-butylsulfinamide (760 mg, 6.27 mmol) was added in one portion. The resulting mixture was stirred at RT for 16 h. Brine (30 ml) was added, and the mixture stirred for 10 min then filtered through celite. The filtrate was extracted with EtOAc (2 x 20 ml). The combined organics were dried over magnesium sulfate and concentrated *in vacuo* to give (*S*)-*N*-((3-chloro-2-fluoropyridin-4-yl)methylene)-2-methylpropane-2-sulfinamide **(I-13b)** as a pale yellow solid. LCMS (method 1) m/z 227.5 (M-Cl)⁺ (ES⁺), at 1.36 min. ¹H NMR (500 MHz, DMSO-d6) δ 8.81 (s, 1H), 8.33 (dt, *J* = 5.1, 0.8 Hz, 1H), 7.90 (d, *J =* 5.1 Hz, 1H), 1.22 (s, 9H).

**Step 2:** To a solution of (*S*)-*N*-((3-chloro-2-fluoropyridin-4-yl)methylene)-2-methylpropane-2-sulfinamide (1.38 g, 5.26 mmol) **(I-13b)** in THF (22 ml) was added but-3-en-1-yl magnesium bromide (0.5 M, 31.6 ml, 15.79 mmol) dropwise at -78 °C. The mixture was warmed to RT slowly and stirred for 72 h. Saturated ammonium chloride solution (10 ml) was added and the product was extracted with EtOAc (3 x 10 ml). The combined organics were dried with magnesium sulfate and concentrated *in vacuo.* The product was purified by silica gel chromatography (0-100% EtOAc/isohexane) to give (*S)*-*N*-*((R*)-1-(3-chloro-2-fluoropyridin-4-yl)pent-4-en-1-yl)-2-methylpropane-2-sulfinamide **(I-13c)** as a pale yellow solid. LCMS (method 1) m/z 316.7, 319.1 (M-H)⁻ (ES⁻), at 1.39 min. ¹H NMR (500 MHz, DMSO-d6) δ 8.21 (dd, *J* = 5.2, 0.8 Hz, 1H), 7.53 (d, *J =* 5.2 Hz, 1H), 5.90 (d, *J* = 7.3 Hz, 1H), 5.81 (dddd, *J =* 17.3, 10.2, 7.1, 6.1 Hz, 1H), 5.10 (dq, *J* = 17.2, 1.7 Hz, 1H), 5.02 (ddt, *J* = 10.2, 2.3, 1.2 Hz, 1H), 4.66 (ddd, *J* = 8.6, 7.3, 5.3 Hz, 1H), 2.27 - 2.08 (m, 2H), 1.97 - 1.87 (m, 1H), 1.80 - 1.70 (m, 1H), 1.07 (s, 9H).

**Step 3:** To a solution of (*S*)-*N*-((*R*)-1-(3-chloro-2-fluoropyridin-4-yl)pent-4-en-1-yl)-2-methylpropane-2-sulfinamide **(I-13c)** (714 mg, 2.015 mmol) in ^{t}BuOH (7.2 ml) was added a solution of HCl in 1,4-dioxane (4 M, 3.0 ml, 12.09 mmol) at RT and stirred for 2.5 h. The reaction mixture was quenched with saturated aqueous sodium bicarbonate solution (100 ml) and extracted with DCM (3 x 30 ml). The combined organics were dried over magnesium sulfate and concentrated *in vacuo* to give (*R*)-1-(3-chloro-2-fluoropyridin-4-yl)pent-4-en-1-amine as a pale yellow liquid **(I-13d)** as an orange/brown oil. LCMS (method 1): m/z 215.4, 217.3 (M+H)⁺ (ES⁺); at 1.17 min, 1H NMR (500 MHz, DMSO-d6) δ 8.16 (dd, *J* = 5.1, 0.9 Hz, 1H), 7.62 (d, *J =* 5.1 Hz, 1H), 5.80 (ddt, *J* = 16.9, 10.2, 6.6 Hz, 1H), 5.02 (dq, *J* = 17.4, 1.8 Hz, 1H), 4.96 (ddt, *J =* 10.2, 2.3, 1.3 Hz, 1H), 4.22 (dd, *J* = 8.1, 5.1 Hz, 1H), 2.23 - 2.01 (m, 2H), 1.76 - 1.50 (m, 2H), 2 NH protons not observed.

**Step 4:** To a solution of (*R*)-1-(3-chloro-2-fluoropyridin-4-yl)pent-4-en-1-amine **(I-13d)** (644.3 mg, 3.001 mmol), (4-methoxyphenyl)boronic acid (1.37 g, 9.0 mmol), and Cu(OAc)₂ (820 mg, 4.50 mmol) in DCM (100 ml) was added pyridine (1.2 ml, 15.0 mmol) dropwise. The mixture was stirred at RT, open to air for 16 h. 2M NaOH aqueous solution (20 ml) was added followed by water (20 ml) and the mixture extracted with DCM (3 x 20 ml). The combined organics were dried over magnesium sulfate and concentrated *in vacuo.* The product was purified by silica gel chromatography (0%-100% DCM/isohexane) to give (R)-*N*-(1-(3-chloro-2-fluoropyridin-4-yl)pent-4-en-1-yl)-4-methoxyaniline **(I-13e)** as a yellow oil. LCMS (method 1): m/z 321.1, 323.4 (M+H)⁺ (ES⁺); at 1.73 min, ¹H NMR (500 MHz, DMSO-d6) δ 8.10 (d, *J* = 5.1 Hz, 1H), 7.41 (d, *J* = 5.1 Hz, 1H), 6.65 (d, *J* = 8.9 Hz, 2H), 6.38 (d, *J* = 8.9 Hz, 2H), 6.09 (d, *J =* 8.3 Hz, 1H), 5.84 (ddt, *J* = 17.0, 10.2, 6.6 Hz, 1H), 5.10 - 4.88 (m, 2H), 4.68 (td, *J* = 8.5, 4.7 Hz, 1H), 3.57 (s, 3H), 2.34 - 2.24 (m, 1H), 2.23 - 2.12 (m, 1H), 1.87 - 1.69 (m, 2H).

**Step 5:** A three-neck flask was charged with Pd-178 (7.4 mg, 15.6 µmol) and sodium tert-butoxide (22.5 mg, 234 µmol) and purged with N₂. A solution of (*R*)-*N*-(1-(3-chloro-2-fluoropyridin-4-yl)pent-4-en-1-yl)-4-methoxyaniline **(I-13e)** (50.0 mg, 156 µmol) in toluene (1 ml) was added dropwise. The resulting mixture was heated to 95 °C for 1.5 h. The reaction mixture was cooled to RT and filtered through celite, washing with EtOAc (3 x 20 ml). The filtrate was concentrated *in vacuo.* The product was purified by silica gel chromatography (0%-50% EtOAc/heptane) to give (5*R*,8*S*)-1-fluoro-10-(4-methoxyphenyl)-6,7,8,9-tetrahydro-5H-5,8-epiminocyclohepta[c]pyridine **(I-13f)** a white solid. LCMS (method 1) m/z 285.3 (M+H)⁺ (ES⁺), at 1.35 min. ¹H NMR (500 MHz, DMSO-d6) δ 7.95 (d, *J =* 4.9 Hz, 1H), 7.29 (dd, *J* = 5.0, 1.7 Hz, 1H), 6.80 (d, *J* = 9.1 Hz, 2H), 6.71 (d, *J* = 9.1 Hz, 2H), 4.92 (d, *J =* 5.6 Hz, 1H), 4.56 (t, *J =* 5.9 Hz, 1H), 3.61 (s, 3H), 2.92 (dd, *J* = 17.6, 4.9 Hz, 1H), 2.35 (d, *J* = 17.5 Hz, 1H), 2.32 - 2.19 (m, 2H), 1.91 - 1.82 (m, 1H), 1.81 - 1.74 (m, 1H).

**Step 6:** A solution of (5*R*,8*S*)-1-fluoro-10-(4-methoxyphenyl)-6,7,8,9-tetrahydro-5H-5,8-epiminocyclohepta[c]pyridine **(I-13f)** (69 mg, 232 µmol) in MeCN (3.2 ml) was cooled to 0 °C before a solution of CAN (381 mg, 696 µmol) in water (3.2 ml) was added dropwise. After the addition was completed the reaction was stirred for 1 h at 0 °C. 2 M aqueous NaOH (5 ml) and water (5 ml) were added, and the mixture extracted with DCM (3 x 10 ml). The combined organics were dried over magnesium sulfate and concentrated *in vacuo* to give (*5R*,*8S*)-1-fluoro-6,7,8,9-tetrahydro-5H-5,8-epiminocyclohepta[c]pyridine (**I-13**) as a pale pink solid. LCMS (method 1): m/z 179.2 (M+H)⁺ (ES⁺); at 0.69 min, ¹H NMR (500 MHz, DMSO-d6) δ 7.91 (dd, *J* = 5.0, 0.9 Hz, 1H), 7.04 (dd, *J* = 5.0, 1.9 Hz, 1H), 4.17 (d, *J* = 5.3 Hz, 1H), 3.78 (t, *J =* 6.0 Hz, 1H), 2.85 (dd, *J* = 17.1, 5.2 Hz, 1H), 2.74 (s, 1H), 2.38 (d, *J =* 17.0 Hz, 1H), 2.01 - 1.85 (m, 2H), 1.73 (t, *J =* 9.1 Hz, 1H), 1.51 (dt, *J* = 9.4, 5.4 Hz, 1H).

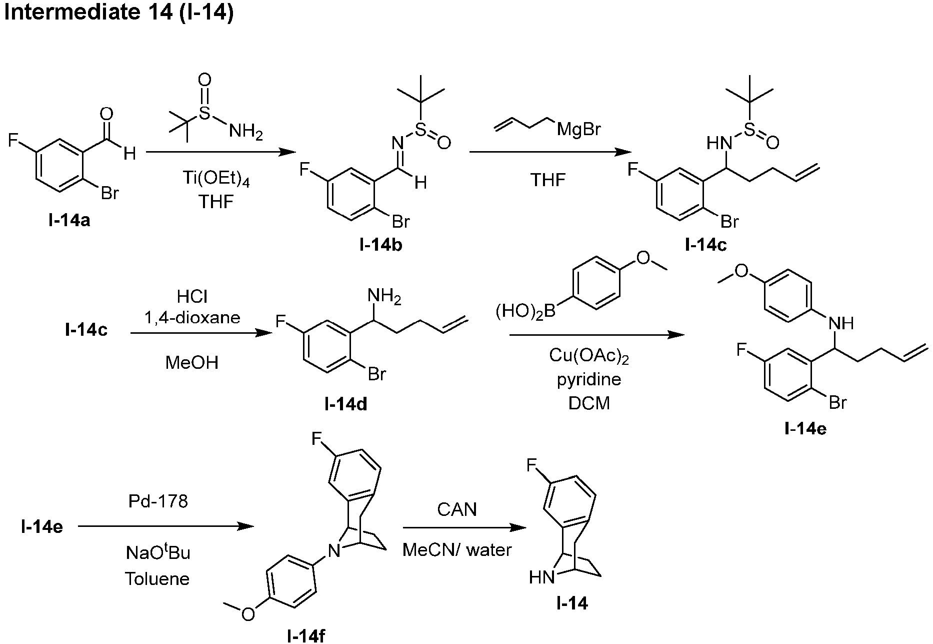

**Step 1:** *N*-(2-Bromo-5-fluorobenzylidene)-2-methylpropane-2-sulfinamide **I-14b** was synthesised from 2-bromo-5-fluorobenzaldehyde **I-14a** using a procedure essentially the same as for I-12b. LCMS (method 2): m/z 305.9, 308.0 (M+H)⁺ (ES⁺); at 2.80 min, ¹H NMR (500 MHz, DMSO-*d*₆) δ 8.76 (d, *J* = 2.2 Hz, 1H), 7.86 (dd, *J* = 8.9, 5.1 Hz, 1H), 7.80 (dd, *J* = 9.2, 3.2 Hz, 1H), 7.56 - 7.36 (m, 1H), 1.20 (s, 9H).

**Step 2:** *N*-(1-(2-Bromo-5-fluorophenyl)pent-4-en-1-yl)-2-methylpropane-2-sulfinamide **I-14c** was synthesised from N-(2-bromo-5-fluorobenzylidene)-2-methylpropane-2-sulfinamide I-14b using a procedure essentially the same as for I-12c. LCMS (method 2): m/z 362.0, 364.0 (M+H)⁺ (ES⁺); at 2.47 min, ¹H NMR (500 MHz, DMSO-*d*₆) δ 7.61 (ddd, *J* = 8.7, 5.4, 4.2 Hz, 1H), 7.38 (dd, *J* = 10.2, 3.2 Hz, 1H), 7.13 - 7.04 (m, 1H), 5.87 - 5.79 (m, 1H), 5.69 (d, *J* = 7.2 Hz, 1H), 5.13 - 5.02 (m, 1H), 5.02 - 4.95 (m, 1H), 4.62 - 4.46 (m, 1H), 2.26 - 2.17 (m, 1H), 2.17 - 2.04 (m, 1H), 1.86 (dtd, *J* = 13.9, 8.6, 5.4 Hz, 1H), 1.80 - 1.60 (m, 1H), 1.06 (s, 9H).

**Step** 3: To a solution of *N*-(1-(2-bromo-5-fluorophenyl)pent-4-en-1-yl)-2-methylpropane-2-sulfinamide **I-14c** (13.6 g, 37.5 mmol) in MeOH (65 ml) was added a solution of HCl in 1,4-dioxane (4 M, 28 ml, 113 mmol) at RT and stirred for 17 h. The reaction mixture was concentrated *in vacuo.* The residue was dissolved in water (200 ml) and washed with EtOAc (200 ml). The aqueous layer was basified to pH 8 with 2 M NaOH solution and extracted with EtOAc (2 x 100 ml). The combined organics were dried over magnesium sulfate and concentrated *in vacuo* to give *N*-(1-(2-bromo-5-fluorophenyl)pent-4-en-1-yl)-2-methylpropane-2-sulfinamide **I-14d** as an orange/brown oil. LCMS (method 2): m/z 258.0. 260.0 (M+H)⁺ (ES⁺); at 2.24 min, ¹H NMR (500 MHz, DMSO-*d*₆) δ 7.57 (dd, *J* = 8.7, 5.5 Hz, 1H), 7.48 (dd, *J* = 10.4, 3.2 Hz, 1H), 7.03 (td, *J* = 8.4, 3.2 Hz, 1H), 5.81 (ddt, *J* = 16.9, 10.2, 6.6 Hz, 1H), 5.02 (m, 1H), 4.94 (ddt, *J =* 10.2, 2.4, 1.3 Hz, 1H), 4.12 - 4.00 (m, 1H), 2.21 - 2.01 (m, 2H), 1.99 (s, 2H), 1.66 - 1.54 (m, 1H), 1.54 - 1.43 (m, 1H).

**Step 4:** *N*-(1-(2-Bromo-5-fluorophenyl)pent-4-en-1-yl)-4-methoxyaniline **I-14e** was synthesised from *N*-(1-(2-bromo-5-fluorophenyl)pent-4-en-1-amine **I-14d** using a procedure essentially the same as for **I-12e.** LCMS (method 2) :m/z 364.1, 366.0 (M+H)⁺ (ES⁺); at 2.90 min, ¹H NMR (500 MHz, DMSO-*d*₆) δ 7.61 (dd, *J* = 8.8, 5.3 Hz, 1H), 7.26 (dd, *J =* 10.1, 3.2 Hz, 1H), 7.02 (ddd, *J* = 8.8, 8.0, 3.1 Hz, 1H), 6.67 - 6.61 (m, 2H), 6.41 - 6.34 (m, 2H), 6.00 (d, *J* = 8.3 Hz, 1H), 5.90 -5.81 (m, 1H), 5.06 - 4.94 (m, 2H), 4.58 - 4.50 (m, 1H), 3.57 (s, 3H), 2.35 - 2.24 (m, 1H), 2.22 - 2.11 (m, 1H), 1.77 - 1.67 (m, 2H).

**Step 5:** (±)-3-Fluoro-10-(4-methoxyphenyl)-6,7,8,9-tetrahydro-5H-5,8-epiminobenzo[7]annulene **I-14f** was synthesised from N-(1-(2-bromo-5-fluorophenyl)pent-4-en-1-yl)-4-methoxyaniline **I-14e** using a procedure essentially the same as for **I-12f**. LCMS (method 2): m/z 284.1 (M+H)⁺ (ES⁺); at 2.67 min.

**Step 6:** A solution of (±)-3-fluoro-10-(4-methoxyphenyl)-6,7,8,9-tetrahydro-5H-5,8-epiminobenzo[7]annulene **I-14f** (2.31 g, 8.15 mmol) in MeCN (100 ml) was cooled to 0 °C before a solution of CAN (13.4 g, 24.5 mmol) in water (100 ml) was added dropwise. After the addition was completed the reaction was stirred for 1 h at 0 °C, then allowed to warm to RT and stirred for 1 h. 2 M aqueous NaOH solution (100 ml) and DCM (100 ml) were added and the mixture filtered through celite, washing with DCM (100 ml). The layers were separated and the aqueous layer was extracted with DCM (2 x 100 ml). The combined organics were dried over magnesium sulfate and concentrated *in vacuo* to give (±)-3-fluoro-6,7,8,9-tetrahydro-5H-5,8-epiminobenzo[7]annulene **I-14** as a sticky brown oil. LCMS (method 2): m/z 178.5 (M+H)⁺ (ES⁺); at 1.68 min, ¹H NMR (400 MHz, DMSO-*d*₆) δ 7.02 (dd, *J* = 8.3, 5.8 Hz, 1H), 6.87 (ddd, *J* = 18.0, 9.2, 2.8 Hz, 2H), 4.09 (d, *J* = 5.0 Hz, 1H), 3.70 (t, *J =* 5.8 Hz, 1H), 2.98 (dd, *J* = 16.6, 5.0 Hz, 1H), 2.44 (d, *J =* 16.4 Hz, 1H), 1.96 - 1.82 (m, 2H), 1.70 (t, *J* = 8.9 Hz, 1H), 1.53 - 1.37 (m, 1H). (1H obscured by water peak).

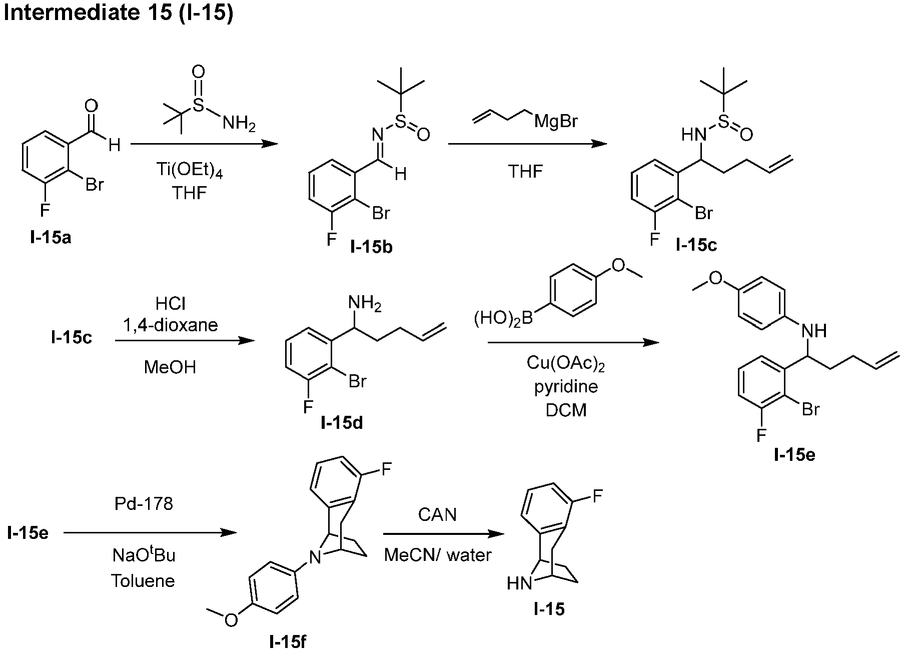

**Step 1:** *N*-(2-Bromo-3-fluorobenzylidene)-2-methylpropane-2-sulfinamide **I-15b** was synthesised from 2-bromo-3-fluorobenzaldehyde **I-15a** using a procedure essentially the same as for I-12b. LCMS (method 2): m/z 305.9, 308.0 (M+H)⁺ (ES⁺); at 2.42 min, ¹H NMR (500 MHz, DMSO-*d*₆) δ 8.81 (s, 1H), 7.88 (ddd, *J* = 7.6, 1.8, 0.9 Hz, 1H), 7.65 - 7.54 (m, 2H), 1.20 (s, 9H).

**Step 2:** *N*-(1-(2-Bromo-3-fluorophenyl)pent-4-en-1-yl)-2-methylpropane-2-sulfinamide I-15c was synthesised from N-(2-bromo-3-fluorobenzylidene)-2-methylpropane-2-sulfinamide I-15b using a procedure essentially the same as for I-12c. LCMS (method 2): m/z 362.0, 364.0 (M+H)⁺ (ES⁺); at 2.44 min, ¹H NMR (500 MHz, DMSO-*d*₆) δ 7.49 - 7.41 (m, 1H), 7.44 - 7.34 (m, 1H), 7.24 (td, *J* = 8.4, 1.6 Hz, 1H), 5.86 - 5.77 (m, 1H), 5.71 (d, *J* = 7.0 Hz, 1H), 5.12 - 4.99 (m, 1H), 5.02 - 4.95 (m, 1H), 4.70 - 4.57 (m, 1H), 2.25 - 2.14 (m, 1H), 2.17 - 2.03 (m, 1H), 1.95 - 1.60 (m, 2H), 1.08 (s, 9H).

**Step 3:** 1-(2-Bromo-3-fluorophenyl)pent-4-en-1-amine **I-15d** was synthesised from *N*-(1-(2-bromo-3-fluorophenyl)pent-4-en-1-yl)-2-methylpropane-2-sulfinamide **I-15c** using a procedure essentially the same as for **I-14d.** LCMS (method 2): m/z 258.0, 260.0 (M+H)⁺ (ES⁺); at 2.18 min, ¹H NMR (500 MHz, DMSO-*d*₆) δ 7.46 (dt, *J* = 7.8, 1.3 Hz, 1H), 7.40 (td, *J* = 7.9, 5.6 Hz, 1H), 7.20 (ddd, *J* = 8.9, 8.0, 1.6 Hz, 1H), 5.81 (ddt, *J* = 16.9, 10.2, 6.6 Hz, 1H), 5.06- 4.98 (m, 1H), 4.94 (ddt, *J* = 10.2, 2.4, 1.3 Hz, 1H), 4.17 (dd, *J* = 8.2, 4.9 Hz, 1H), 2.20 - 1.96 (m, 2H), 1.96 (s, 2H), 1.67 - 1.61 (m, 1H), 1.56 - 1.49 (m, 1H). .

**Step 4:** To a solution of 1-(2-bromo-3-fluorophenyl)pent-4-en-1-amine (10.3 g, 39.9 mmol) **I-15d**, (4-methoxyphenyl)boronic acid (18.2 g, 120 mmol), and Cu(OAc)₂ (10.9 g, 59.9 mmol) in DCM (100 ml) was added pyridine (16.1 ml, 200 mmol) dropwise. The mixture was stirred at RT open to air for 4 days. 2 M NaOH aqueous solution (100 ml) was added, the mixture was stirred for 30 min and filtered through celite. The filtrate was partitioned between water (100 ml) and DCM (100 ml) and the layers were separated. The aqueous layer was further extracted with DCM (2 x 100 ml). The combined organics were dried over magnesium sulfate and concentrated *in vacuo.* The product was purified by silica gel chromatography (0%-100% DCM/isohexane) to give *N*-(1-(2-bromo-3-fluorophenyl)pent-4-en-1-yl)-4-methoxyaniline **I-15e** as a sticky orange oil. ¹H NMR (500 MHz, DMSO-*d*₆) δ 7.36 - 7.31 (m, 1H), 7.27 (dd, *J* = 8.0, 1.7 Hz, 1H), 7.19 (ddd, *J* = 9.5, 8.0, 1.6 Hz, 1H), 6.66 - 6.58 (m, 2H), 6.40 - 6.33 (m, 2H), 6.04 (d, *J* = 8.1 Hz, 1H), 5.87 - 5.82 (m, 1H), 5.06 - 4.94 (m, 2H), 4.61 (td, *J* = 8.1, 5.2 Hz, 1H), 3.56 (s, 3H), 2.30 (dd, *J* = 14.3, 7.8 Hz, 1H), 2.23 - 2.12 (m, 1H), 1.80 - 1.67 (m, 2H).

**Step 5:** (±)-1-Fluoro-10-(4-methoxyphenyl)-6,7,8,9-tetrahydro-5H-5,8-epiminobenzo[7]annulene **I-15f** was synthesised from *N*-(1-(2-bromo-3-fluorophenyl)pent-4-en-1-yl)-4-methoxyaniline **I-15e** using a procedure essentially the same as for **I-12f**. LCMS (method 2): m/z 284.1 (M+H)⁺ (ES⁺); at 2.56 min, ¹H NMR (400 MHz, DMSO-*d*₆) δ 7.19 - 7.06 (m, 2H), 6.88 (ddd, *J* = 9.5, 7.9, 1.5 Hz, 1H), 6.83 - 6.74 (m, 2H), 6.73 - 6.65 (m, 2H), 4.83 (d, *J* = 5.3 Hz, 1H), 4.51 (t, *J =* 5.6 Hz, 1H), 3.60 (s, 3H), 2.96 (dd, *J* = 17.3, 4.8 Hz, 1H), 2.37 (d, *J* = 17.4 Hz, 1H), 2.33 - 2.17 (m, 2H), 1.88 - 1.65 (m, 2H).

**Step 6:** (±)-1-Fluoro-6,7,8,9-tetrahydro-5H-5,8-epiminobenzo[7]annulene **I-15** was synthesised from (±)-1-fluoro-10-(4-methoxyphenyl)-6,7,8,9-tetrahydro-5H-5,8-epiminobenzo[7]annulene **I-15f** using a procedure essentially the same as for **I-12.** LCMS (method 2): m/z 178.1 (M+H)⁺ (ES⁺); at 1.69 min, ¹H NMR (500 MHz, DMSO-*d*₆) δ 7.09 (td, *J* = 7.8, 5.9 Hz, 1H), 6.90 (ddd, *J* = 9.5, 8.2, 1.1 Hz, 1H), 6.86 (dd, *J* = 7.5, 1.1 Hz, 1H), 4.12 (d, *J* = 5.1 Hz, 1H), 3.75 (t, *J* = 6.0 Hz, 1H), 2.88 (dd, *J* = 16.9, 5.2 Hz, 1H), 2.57 (s, 1H), 2.42 (d, *J* = 16.9 Hz, 1H), 1.97 - 1.85 (m, 2H), 1.70 (t, *J* = 9.0 Hz, 1H), 1.52 - 1.41 (m, 1H).

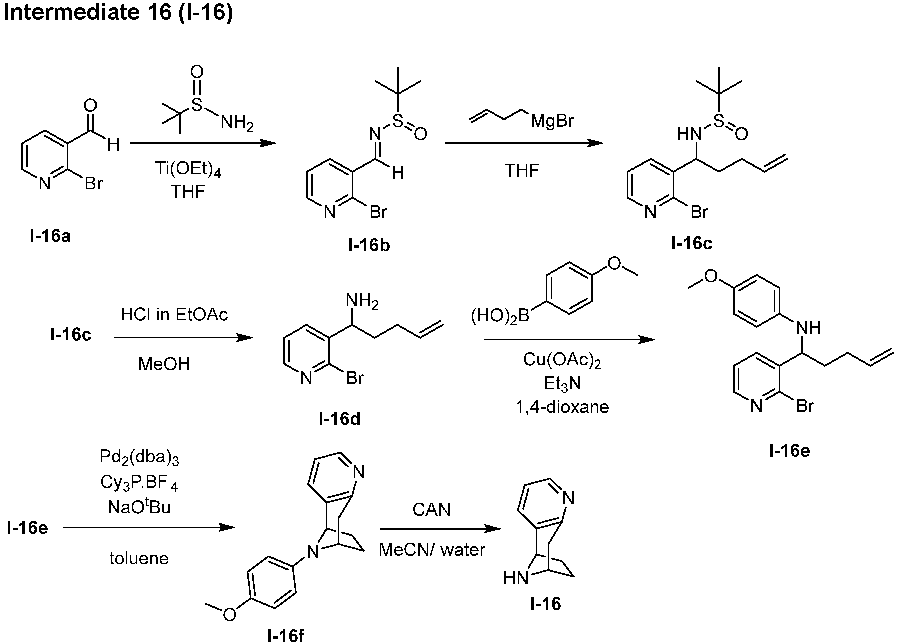

**Step 1:** To a mixture of 2-bromonicotinaldehyde **I-16a** (15.0 g, 80.6 mmol) in THF (90.0 ml) was added Ti(OEt)₄ (33.5 ml, 161 mmol) and *tert*-butylsulfinamide (10.8 g, 88.7 mmol). The resulting mixture was stirred at RT for 16 h. The reaction mixture was poured into water (100 ml) at 0 °C, and then extracted with EtOAc (3 x 75 ml). The combined organics were dried over sodium sulfate and concentrated *in vacuo.* The residue was purified by silica gel chromatography (0-100% EtOAc/petroleum ether) to give *N*-((2-bromopyridin-3-yl)methylene)-2-methylpropane-2-sulfinamide **I-16b** as a yellow solid. ¹H NMR (400 MHz, CDCl₃) δ 8.92 (s, 1H), 8.48 (t, *J =* 2.0 Hz, 1H), 8.31-8.29 (m, 1H), 7.40-7.37 (m, 1H), 1.25 (t, *J* = 3.2 Hz, 9H).

**Step** 2: To a solution of *N*-((2-bromopyridin-3-yl)methylene)-2-methylpropane-2-sulfinamide **I-16b** (19.0 g, 65.7 mmol) in THF (107 ml) was added but-3-en-1-yl magnesium bromide (0.5 M, 302 ml) dropwise at 0 °C. The mixture was warmed to RT for 1 h. The reaction mixture was cooled to 0 °C and water (50 ml) was added carefully. The product was extracted with EtOAc (3 x 100 ml). The combined organic layers were washed with brine (50 ml), dried over sodium sulfate and concentrated *in vacuo.* The residue was purified by silica gel chromatography (0-100% EtOAc/petroleum ether) to give *N*-(1-(2-bromopyridin-3-yl)pent-4-en-1-yl)-2-methylpropane-2-sulfinamide **I-16c** as a yellow solid. ¹H NMR (400 MHz, CDCl₃) δ 8.28 (t, *J* = 2.8 Hz, 1H), 7.69 (t, *J* = 2.5 Hz, 1H), 7.29-7.26 (m, 1H), 5.83-5.76 (t, *J* = 2.4 Hz, 1H), 5.08-5.00 (m, 2H), 4.84 (s, 1H), 2.23-2.10 (m, 2H), 1.97-1.93 (m, 2H), 1.18 (s, 9H). (NH not observed)

**Step 3:** To a solution of *N*-(1-(2-bromopyridin-3-yl)pent-4-en-1-yl)-2-methylpropane-2-sulfinamide I-16c (11.2 g, 32.5 mmol) in MeOH (70 ml) was added HCl in EtOAc (4 M, 24.4 ml, 97.6 mmol) at 0 °C and the mixture was warmed to RT for 30 min. The reaction mixture was concentrated *in vacuo,* triturated with MTBE (100 ml) and the solid collected by filtration, washed with MTBE (2 x 100 ml) and dried *in vacuo.* The solids were dissolved in water (50 ml), cooled to 0 °C and the mixture was basified to pH 11 with NaOH solution. The aqueous mixture was extracted with DCM (3 x 30 ml) and the combined organics were washed with brine (30 ml), dried over sodium sulfate and concentrated *in vacuo.* The residue was purified by silica gel chromatography (0-100% EtOAc/petroleum ether) to give 1-(2-bromopyridin-3-yl)pent-4-en-1-amine **I-16d** as a yellow solid. ¹H NMR (400 MHz, CDCl₃) δ 8.26-8.22 (m, 1H), 7.87-7.78 (m, 1H), 7.26 (t, *J* = 5.6 Hz, 1H), 5.85-5.76 (m, 1H), 5.05-4.96 (m, 2H), 4.35-4.30 (m, 1H), 2.21-2.10 (m, 2H), 1.83 (t, *J* = 6.4 Hz, 1H), 1.70-1.64 (m, 1H).(NH₂ not observed)

**Step 4:** To a mixture of 1-(2-bromopyridin-3-yl)pent-4-en-1-amine **I-16d** (6.50 g, 26.9 mmol), (4-methoxyphenyl)boronic acid (7.66 g, 50.4 mmol), 4 Å molecular Sieves (12.1 g) and Cu(OAc)₂ (11.4 g, 63.0 mmol) in 1,4-dioxane (100 ml) was added dropwise Et₃N (4.56 ml, 32.8 mmol). The resultant mixture was stirred at 35 °C under O₂ for 16 hrs. The reaction mixture was filtered and the solid was washed with EtOAc (3 x 10 ml). The filtrate was dried over sodium sulfate and concentrated *in vacuo.* The residue was purified by silica gel chromatography (0-100% EtOAc/petroleum ether) to give *N*-(1-(2-bromopyridin-3-yl)pent-4-en-1-yl)-4-methoxyaniline **I-16e** as a red-brown solid. ¹H NMR (400 MHz, CDCl₃) δ 8.26-8.23 (m, 1H), 7.83-7.70 (m, 1H) 7.26-7.18 (m, 1H), 6.69 (d, *J =* 4.8 Hz, 1H), 6.41-6.38 (m, 2H), 5.86 (d, *J* = 6.8 Hz, 2H), 5.08-5.00 (m, 2H), 4.64 (t, *J =* 4.4 Hz, 1H), 3.69 (s, 3H), 2.31-2.22 (m, 2H), 1.98 (d, *J* = 6.8 Hz, 1H), 1.73 (t, *J =* 8.4 Hz, 1H). (NH not observed)

**Step 5:** A three-neck flask was charged with Pd₂(dba)₃ (131 mg, 0.144 mmol), tricyclohexylphosphonium;tetrafluoroborate (106 mg, 0.288 mmol) and NaO^{t}Bu (207 mg, 2.16 mmol) and purged with argon. A solution of *N*-(1-(2-bromopyridin-3-yl)pent-4-en-1-yl)-4-methoxyaniline **I-16e** (0.50 g, 1.44 mmol) in 1,4-dioxane (1.8 ml) was added and the resulting mixture was heated to 95 °C for 12 h. The reaction mixture was filtered and the solid was washed with EtOAc (3 x 10 ml). The filtrate was dried over sodium sulfate and concentrated *in vacuo.* The residue was purified by silica gel chromatography (0-100% EtOAc/petroleum ether) to give (±)-10-(4-methoxyphenyl)-6,7,8,9-tetrahydro-5H-5,8-epiminocyclohepta[b]pyridine **I-16f** as a pale red-brown solid. ¹H NMR (400 MHz, CDCl₃) δ 8.32-8.31 (m, 1H), 7.45-7.43 (m, 1H), 6.75 (t, *J* = 2.8 Hz, 1H), 6.72 (t, *J =* 6.4 Hz, 4H), 4.67 (d, *J* = 6.8 Hz, 1H), 4.53 (t, *J =* 6.4 Hz, 1H), 3.69 (s, 3H), 3.36 (d, *J =* 17.6 Hz, 1H), 2.61 (d, *J* = 17.6 Hz, 1H), 2.38 (t, *J* = 5.6 Hz, 1H), 1.94-1.83 (m, 2H).

**Step** 6: (±)-6,7,8,9-tetrahydro-5H-5,8-epiminocyclohepta[b]pyridine **I-16** was synthesised from (±)-10-(4-methoxyphenyl)-6,7,8,9-tetrahydro-5H-5,8-epiminocyclohepta[b]pyridine **I-16f** using a procedure essentially the same as for **I-14**. LCMS (method 1): m/z 160.9 (M+H)⁺ (ES⁺); at 0.62 min.

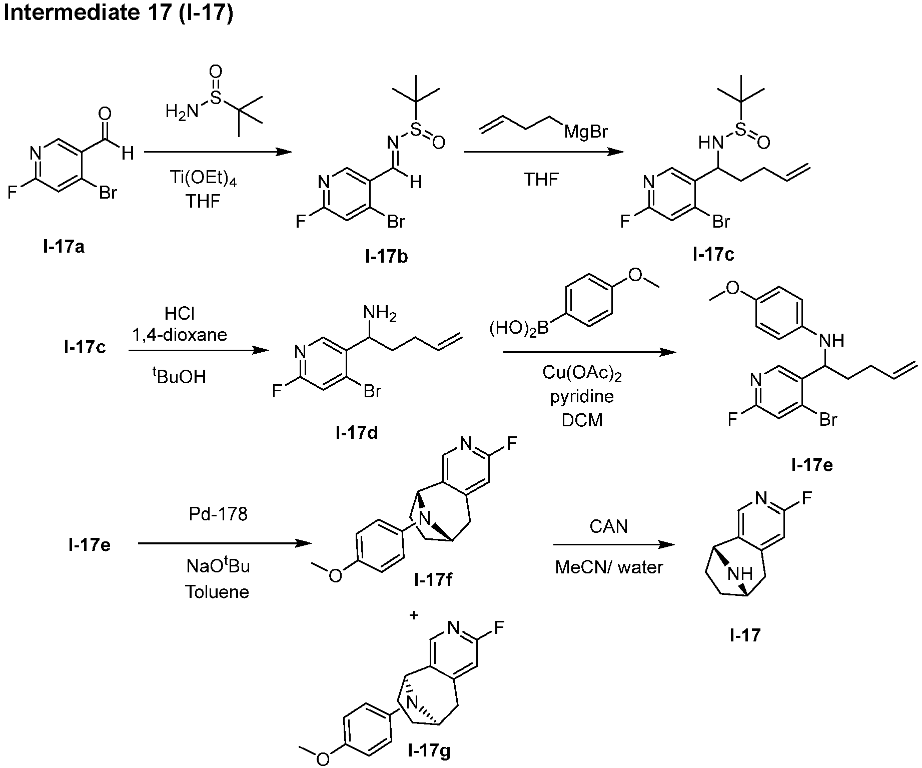

**Step 1:** (*S*)-*N*-((4-bromo-6-fluoropyridin-3-yl)methylene)-2-methylpropane-2-sulfinamide I-17b was synthesised from 4-bromo-6-fluoronicotinaldehyde **I-17a** using a procedure essentially the same as for **I-13b**. LCMS (method 2): m/z 307.0, 308.9 (M+H)⁺ (ES⁺); at 2.05 min, ¹H NMR (500 MHz, DMSO-d6) δ 8.81 (s, 1H), 8.73 (s, 1H), 7.88 (d, *J =* 2.4 Hz, 1H), 1.21 (s, 9H).

**Step 2:** To a solution of ((*S*)-*N*-((4-bromo-6-fluoropyridin-3-yl)methylene)-2-methylpropane-2-sulfinamide **I-17b** (7.00 g, 22.8 mmol) in THF (114 ml) was added but-3-en-1-yl magnesium bromide (2.54 M, 17.9 ml, 45.6 mmol) dropwise at -78 °C. The mixture was warmed to RT slowly and stirred for 16 h. Saturated ammonium chloride solution (10 ml) was added and the product was extracted with EtOAc (2 x 10 ml). The combined organics were dried with magnesium sulfate and concentrated *in vacuo.* The product was purified by silica gel chromatography (0-10% IPA/isohexane) to give a 2:1 mixture of the diastereomers of (±)-*N*-(1-(4-bromo-6-fluoropyridin-3-yl)pent-4-en-1-yl)-2-methylpropane-2-sulfinamide **I-17c** as a pale yellow oil. LCMS (method 1) m/z 363.3, 365.4 (M+H)⁺ (ES⁺), at 1.39 and 1.42 min.

**Major diastereomer:** 1H NMR (500 MHz, DMSO-d6) δ 8.43 (s, 1H), 7.60 (d, *J* = 2.6 Hz, 1H), 6.00 (d, *J* = 9.5 Hz, 1H), 5.82 (ddt, *J* = 16.7, 10.3, 6.6 Hz, 1H), 5.12 - 4.97 (m, 2H), 4.64-4.50 (m, 1H), 2.26 - 2.14 (m, 1H), 2.16 - 2.05 (m, 1H), 1.94 - 1.78 (m, 1H), 1.78-1.66 (m, 1H), 1.13 (s, 9H).

**Minor diastereomer:** 1H NMR (500 MHz, DMSO-d6) δ 8.33 (s, 1H), 7.61 (d, *J =* 2.5 Hz, 1H), 5.82 (ddt, *J* = 16.7, 10.3, 6.6 Hz, 1H), 5.76 (d, *J* = 7.0 Hz, 1H), 5.12 - 4.97 (m, 2H), 4.64-4.50 (m, 1H), 2.26 - 2.14 (m, 1H), 2.16 - 2.05 (m, 1H), 2.03 - 1.92 (m, 1H), 1.94 - 1.78 (m, 1H), 1.07 (s, 9H).

**Step 3:** (±)-1-(4-Bromo-6-fluoropyridin-3-yl)pent-4-en-1-amine **I-17d** was synthesised from (±)-*N*-(1-(4-bromo-6-fluoropyridin-3-yl)pent-4-en-1-yl)-2-methylpropane-2-sulfinamide **I-17c** using a procedure essentially the same as for **I-13d**. LCMS (method 1) m/z 259.2, 261.2 (M+H)⁺ (ES⁺), at 1.21 min. ¹H NMR (500 MHz, DMSO-d6) δ 8.41 (s, 1H), 7.55 (d, *J* = 2.7 Hz, 1H), 5.82 (ddt, *J* = 16.9, 10.2, 6.6 Hz, 1H), 5.03 (dq, *J* = 17.2, 1.8 Hz, 1H), 4.95 (ddt, *J* = 10.2, 2.3, 1.3 Hz, 1H), 4.12 (dd, *J* = 8.2, 4.9 Hz, 1H), 2.25 - 1.98 (m, 4H), 1.75-1.64 (m, 1H), 1.64-1.54 (m, 1H).

**Step 4:** (±)-*N*-(1-(4-Bromo-6-fluoropyridin-3-yl)pent-4-en-1-yl)-4-methoxyaniline **I-17e** was synthesised from (±)-1-(4-bromo-6-fluoropyridin-3-yl)pent-4-en-1-amine **I-17d** using a procedure essentially the same as for **I-13e**. LCMS (method 1) m/z 365.0, 367.1 (M+H)⁺ (ES⁺), at 1.76 min. ¹H NMR (500 MHz, DMSO-d6) δ 8.21 (s, 1H), 7.61 (d, *J* = 2.5 Hz, 1H), 6.66 (d, *J* = 8.9 Hz, 2H), 6.40 (d, *J* = 9.0 Hz, 2H), 6.04 (d, *J =* 8.3 Hz, 1H), 5.86 (ddt, *J =* 17.0, 10.2, 6.6 Hz, 1H), 5.08 - 4.90 (m, 2H), 4.59 (td, *J* = 8.5, 4.8 Hz, 1H), 3.58 (s, 3H), 2.35 - 2.26 (m, 1H), 2.23-2.13 (m, 1H), 1.92 - 1.71 (m, 2H).

**Step 5:** A three-neck flask was charged with Pd-178 (0.29 g, 0.61 mmol) and NaO^{t}Bu (0.88 g, 9.2 mmol) and purged with N₂. A solution of *N*-(1-(4-bromo-6-fluoropyridin-3-yl)pent-4-en-1-yl)-4-methoxyaniline **I-17e** (2.3 g, 6.1 mmol) in toluene (66 ml) was added dropwise. The resulting mixture was heated to 95 °C for 2 h. The reaction mixture was cooled to RT and filtered through celite, washing the solid with EtOAc (150 ml). The filtrate was concentrated *in vacuo.* The product was purified by silica gel chromatography (0%-50% EtOAc/isohexane) to give a mixture of enantiomers which were dissolved to 30 mg/ml in DCM:methanol (1:4) and was then separated by chiral SFC on a Waters prep 15 with UV detection at 210 nm, 40 °C, 100 bar on a Lux C3 column (21.2 mm x 250 mm, 5 µm particle size), flow rate 50 ml/min using 40% methanol to give (6S,9R)-3-fluoro-10-(4-methoxyphenyl)-6,7,8,9-tetrahydro-5H-6,9-epiminocyclohepta[c]pyridine **(I-17f)** as a pale yellow solid. LCMS (method 1) m/z 285.3 (M+H)+ (ES+), at 1.35 min. 1H NMR (500 MHz, DMSO-d6) δ 8.10 (s, 1H), 6.79 (t, *J* = 9.7 Hz, 3H), 6.70 (d, *J =* 9.1 Hz, 2H), 4.95 (d, *J* = 5.5 Hz, 1H), 4.47 (t, *J* = 6.0 Hz, 1H), 3.61 (s, 3H), 3.11 (dd, *J* = 18.2, 4.9 Hz, 1H), 2.31-2.19 (m, 2H), 1.88 - 1.55 (m, 2H), 1H obscured by residual DMSO peak.
and (6R,9S)-3-fluoro-10-(4-methoxyphenyl)-6,7,8,9-tetrahydro-5H-6,9-epiminocyclohepta[c]pyridine **(I-17g)** as a pale yellow solid. LCMS (method 1) m/z 285.3 (M+H)+ (ES+), at 1.35 min. 1H NMR (500 MHz, DMSO-d6) δ 8.10 (s, 1H), 6.79 (t, *J* = 9.7 Hz, 3H), 6.70 (d, *J* = 9.1 Hz, 2H), 4.95 (d, *J* = 5.5 Hz, 1H), 4.47 (t, *J =* 6.0 Hz, 1H), 3.61 (s, 3H), 3.11 (dd, *J* = 18.2, 4.9 Hz, 1H), 2.31-2.19 (m, 2H), 1.88 - 1.55 (m, 2H), 1H obscured by residual DMSO peak.

**Step 6:** (6*S*,9*R*)-3-fluoro-6,7,8,9-tetrahydro-5H-6,9-epiminocyclohepta[c]pyridine (**I-17**) was synthesised from (6*S*,9*R)*-3-fluoro-10-(4-methoxyphenyl)-6,7,8,9-tetrahydro-5H-6,9-epiminocyclohepta[c]pyridine **(I-17f)** using a procedure essentially the same as for **I-13**. LCMS (method 1) m/z 179.2 (M+H)⁺ (ES⁺), at 0.71 min.

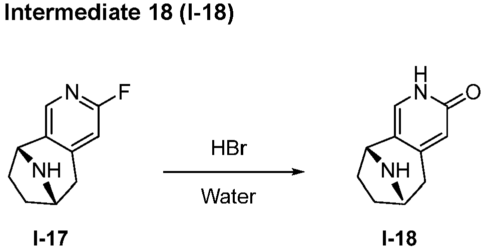

(6*S*,9*R*)-3-fluoro-6,7,8,9-tetrahydro-5H-6,9-epiminocyclohepta[c]pyridine **I-17** (300 mg, 1.68 mmol) was dissolved in HBr (1.90 ml, 48 % w/w in water, 16.8 mmol) and was heated at 100 °C for 18 h. The reaction mixture was cooled to RT, diluted with MeOH, loaded onto a SCX cartridge (20 g) and the product was eluted with ammonia in MeOH solution (0.7 M) to give (6*S*,9*R*)-2,5,6,7,8,9-hexahydro-3H-6,9-epiminocyclohepta[c]pyridin-3-one (**I-18**) as an off white solid. LCMS (method 1) m/z 176.9 (M+H)⁺ (ES⁺), at 0.41 min,

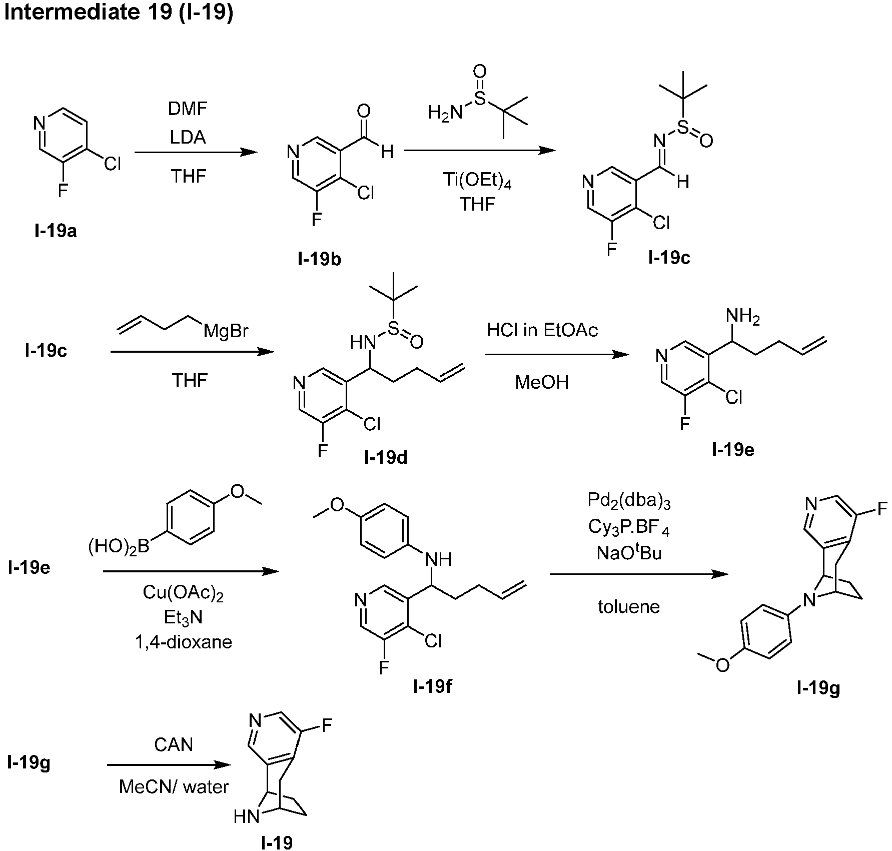

**Step 1:** To a solution of 4-chloro-3-fluoropyridine **I-19a** (25.9 ml, 190 mmol) in THF (120 ml) was added LDA (2 M, 114 ml, 228 mmol) at -70 °C. The mixture was stirred at -70 °C for 3 h, before DMF (17.5 ml, 228 mmol) in THF (50 ml) was added. The mixture was slowly warmed to RT and stirred at RT for 1 h. The reaction mixture was cooled to 0 °C and saturated ammonium chloride (100 ml) was added. The aqueous layer was adjusted to pH 6 with HCl (1 M) and extracted with MTBE (3 x 100 ml). The combined organic layers were washed with brine (100 ml), dried over sodium sulfate and concentrated *in vacuo* to give 4-chloro-5-fluoronicotinaldehyde **I-19b** as a yellow oil. ¹H NMR (400 MHz, CDCl₃) δ 10.4 (s, 1H), 8.87 (s, 1H), 8.69 (s, 1H).

### Step 2: N-((4-Chloro-5-fluoropyridin-3-yl)methylene)-2-methylpropane-2-sulfinamide

**I-19c** was synthesised from 4-chloro-5-fluoronicotinaldehyde **I-19b** using a procedure essentially the same as for **I-16b.** ¹H NMR (400 MHz, CDCl₃) δ 9.02 (s, 1H), 8.93 (s, 1H), 8.59 (s, 1H), 1.30 (s, 9H).

**Step 3:** To a solution of (*E*)-*N*-((4-chloro-5-fluoropyridin-3-yl)methylene)-2-methylpropane-2-sulfinamide **I-19c** (27.0 g, 102 mmol) in THF (150 ml), was added but-3-en-1-yl magnesium bromide (0.5 M, 822 ml) at 0 °C. The mixture was warmed and stirred at RT for 1 h. The reaction mixture was cooled to 0 °C and water (50 ml) was added. The product was extracted with EtOAc (3 x 100 ml). The organic layer was separated, washed with water (100 ml) and brine (100 ml), dried over sodium sulfate and concentrated *in vacuo.* The product was triturated with petroleum ether (50 ml) and the solid was collected by filtration washing with petroleum ether (2 x 20 ml). The solid was dried *in vacuo* to give *N*-(1-(4-chloro-5-fluoropyridin-3-yl)pent-4-en-1-yl)-2-methylpropane-2-sulfinamide **I-19d** as a yellow solid. ¹H NMR (400 MHz, CDCl₃)δ 8.43 (s, 2H), 5.84 - 5.76 (m, 1H), 5.10 - 5.03 (m, 2H), 4.94 - 4.90 (m, 1H), 3.58 (d, *J* = 2.4 Hz, 1H), 2.22 - 2.01 (m, 4H), 1.20 (s, 9H).

**Step 4:** 1-(4-Chloro-5-fluoropyridin-3-yl)pent-4-en-1-amine **I-19e** was synthesised from *N-*(1-(4-chloro-5-fluoropyridin-3-yl)pent-4-en-1-yl)-2-methylpropane-2-sulfinamide **I-19d** using a procedure essentially the same as for **I-16d.** ¹H NMR (400 MHz, CDCl₃)δ 8.52 (s, 1H), 8.38 (s, 1H), 5.83 - 5.77 (m, 1H), 5.05 - 4.97 (m, 2H), 4.39 - 4.35 (m, 1H), 2.16 - 2.11 (m, 2H), 1.86 - 1.60 (m, 2H).(NH₂ not observed)

**Step 5:** *N*-(1-(4-Chloro-5-fluoropyridin-3-yl)pent-4-en-1-yl)-4-methoxyaniline **I-19f** was synthesised from 1-(4-chloro-5-fluoropyridin-3-yl)pent-4-en-1-amine **I-19e** using a procedure essentially the same as for **I-16e.** ¹H NMR (400 MHz, CDCl₃)δ 8.43 (s, 1H), 8.37 (s, 1H), 6.72 - 6.69 (m, 2H), 6.44 (t, *J* = 3.6 Hz, 2H), 5.85 - 5.81 (m, 1H), 5.09 - 5.03 (m, 2H), 4.76 (t, *J =* 4.8 Hz, 1H), 3.96 (s, 1H), 3.70 (s, 3H), 2.30 - 2.22 (m, 2H), 1.97 - 1.87 (m, 2H).

**Step 6:** (±)-4-fluoro-10-(4-methoxyphenyl)-6,7,8,9-tetrahydro-5H-6,9-epiminocyclohepta[c]pyridine **I-19g** was synthesised from *N*-(1-(4-bromo-5-fluoropyridin-3-yl)pent-4-en-1-yl)-4-methoxyaniline **I-19f** using a procedure essentially the same as for **I-16f**. ¹H NMR (400 MHz, CDCl₃) 8.25 (s, 1H), 8.17 (s, 1H), 6.77 - 6.73 (m, 4H), 4.79 (d, *J* = 6.0 Hz, 1H), 4.52 (t, *J =* 5.2, 1H), 3.71 (s, 3H), 3.15 - 3.09 (m, 1H), 2.48 - 2.40 (m, 3H), 1.99 - 1.94 (m, 1H), 1.78 - 1.70 (m, 1H).

**Step 7:** A solution of (±)-4-fluoro-10-(4-methoxyphenyl)-6,7,8,9-tetrahydro-5H-6,9-epiminocyclohepta[c]pyridine **I-19g** (0.150 g, 528 µmol) in MeCN (10 ml) was cooled to 0 °C before a solution of CAN (925 mg, 1.69 mmol) in water (10 ml) was added dropwise. After the addition was completed the reaction was stirred for 1 h at 0 °C. Further CAN (925 mg, 1.69 mmol) in water (3 ml) was added and the reaction stirred for 1 h. The material was loaded onto an SCX cartridge (20 g), washed with MeOH (40 ml) and the product was eluted using 0.7 M ammonia in MeOH (100 ml) solution to give (6*S*,9*R*)-4-fluoro-6,7,8,9-tetrahydro-5H-6,9-epiminocyclohepta[c]pyridine as a yellow oil **I-19.** LCMS (method 1): m/z 178.9 (M+H)⁺ (ES⁺); at 0.71 min.

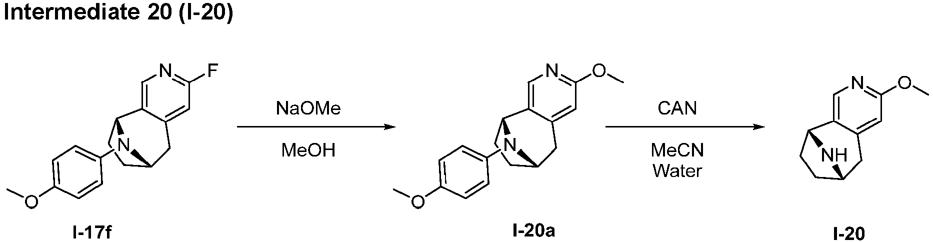

**Step 1:** To a solution of (6*S*,9*R*)-3-fluoro-10-(4-methoxyphenyl)-6,7,8,9-tetrahydro-5H-6,9-epiminocyclohepta[c]pyridine **I-17f** (100.0 mg, 351.7 µmol) in MeOH (1 ml) was added sodium methoxide (5.4 M in MeOH, 391 µl, 2.11 mmol) and the reaction mixture heated at 65 °C for 48 h. The reaction mixture was diluted with DCM (20 ml) and water (50 ml). The layers were separated and the aqueous layer was extracted with DCM (3 x 30 ml). The combined organics were dried over magnesium sulfate and concentrated *in vacuo* to give (6*S*,9*R*)-3-methoxy-10-(4-methoxyphenyl)-6,7,8,9-tetrahydro-5H-6,9-epiminocyclohepta[c]pyridine **I-20a** as a pale yellow oil. LCMS (method 2) m/z 297.1 (M+H)⁺ (ES⁺), at 2.14 min.

**Step 2:** (6*S*,9*R*)-3-Methoxy-6,7,8,9-tetrahydro-5H-6,9-epiminocyclohepta[c]pyridine **I-20** was synthesised from (6S,9R)-3-methoxy-10-(4-methoxyphenyl)-6,7,8,9-tetrahydro-5H-6,9-epiminocyclohepta[c]pyridine **I-20a** using a procedure essentially the same as for **I-13**. LCMS (method 1) m/z 191.1 (M+H)⁺ (ES⁺), at 1.33 min.

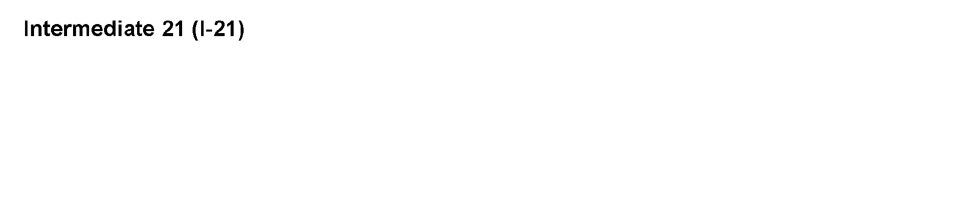

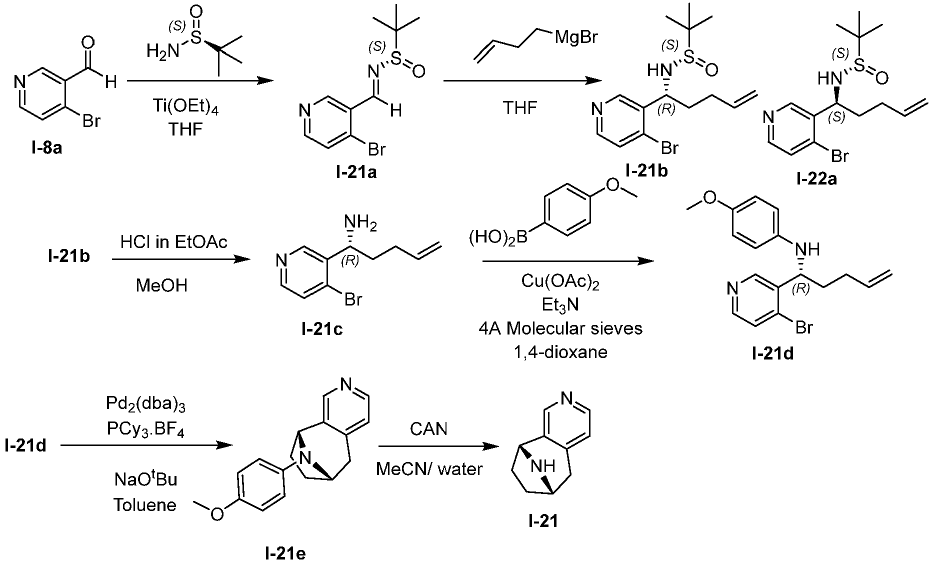

**Step 1:** (*S*)-*N*-((4-Bromopyridin-3-yl)methylene)-2-methylpropane-2-sulfinamide **I-21a** was synthesised from 4-bromo-3-pyridinecarboxaldehyde (**I-8a**) and (*S*)-*tert*-butylsulfinamide using a procedure essentially the same as for **I-7b**. ¹H NMR (400 MHz, CDCl₃) δ 9.13 (s, 1H), 8.92 (s, 1H), 8.46 (d, *J* = 5.2 Hz, 1H), 7.60 (d, *J* = 5.6 Hz, 1H), 1.29 (s, 9H).

**Step 2:** (*S*)-*N*-((*R*)-1-(4-Bromopyridin-3-yl)pent-4-en-1-yl)-2-methylpropane-2-sulfinamide **I-21b** and (*S*)-*N*-((*S*)-1-(4-bromopyridin-3-yl)pent-4-en-1-yl)-2-methylpropane-2-sulfinamide **I-22a** was synthesised from (*S*)-*N*-((4-bromopyridin-3-yl)methylene)-2-methylpropane-2-sulfinamide **I-21a** using a procedure essentially the same as for **I-7c.** ¹H NMR (400 MHz, CDCl₃) δ: 8.52 (s, 1H), 8.29 (d, *J =* 5.6 Hz, 1H), 7.50 (t, *J* = 9.6 Hz, 1H), 5.83 - 5.76 (m, 1H), 5.09 - 5.01 (m, 2H), 4.91 - 4.89 (m, 1H), 3.60 (t, *J =* 2.8 Hz, 1H), 2.19 - 1.96 (m, 4H), 1.18 (s, 9H).

**Step 3:** (*R*)-1-(4-Bromopyridin-3-yl)pent-4-en-1-amine **I-21c** was synthesised from *(S)*-*N-((R)*-1-(4-bromopyridin-3-yl)pent-4-en-1-yl)-2-methylpropane-2-sulfinamide **I-21b** using a procedure essentially the same as for **I-7d**. ¹H NMR (400 MHz, CDCl₃) δ 8.70 (s, 1H), 8.27 - 8.22 (m, 1H), 7.45 (t, *J =* 5.6 Hz, 1H), 5.88 - 5.80 (m, 1H), 5.07 - 4.98 (m, 2H), 4.37 - 4.33 (m, 1H), 2.22 - 2.13 (m, 2H), 1.90 - 1.75 (m, 2H). (NH₂ not observed)

**Step 4:** (*R*)-*N*-(1-(4-Bromopyridin-3-yl)pent-4-en-1-yl)-4-methoxyaniline **I-21d** was synthesised from (*R*)-1-(4-bromopyridin-3-yl)pent-4-en-1-amine **I-21c** using a procedure essentially the same as for **I-7e**. ¹H NMR (400 MHz, CDCl₃) δ 8.68 (s, 1H), 8.40 (d, *J* = 5.2 Hz, 1H), 7.34 (d, *J* = 5.2 Hz, 1H), 6.70 (d, *J* = 9.2 Hz, 2H), 6.39 - 6.36 (m, 2H), 5.87 - 5.83 (m, 1H), 5.09 - 5.02 (m, 2H), 4.66 - 4.63 (m, 1H), 3.95 (s, 1H), 3.87 (s, 3H), 2.33 - 2.25 (m, 2H), 1.95 - 1.93 (m, 1H), 1.75 - 1.71 (m, 1H), (NH not observed)

**Step 5:** To a solution of Pd₂(dba)₃ (0.68 g, 0.75 mmol), tricyclohexylphosphonium tetrafluoroborate (551 mg, 1.50 mmol and NaO^{t}Bu (5.69 g, 59.2 mmol) in toluene (50 ml). The flask was purged with N₂ and (*R*)-*N*-(1-(4-bromopyridin-3-yl)pent-4-en-1-yl)-4-methoxyaniline **I-21d** (2.6 g, 7.5 mmol) was added dropwise. The resulting mixture was heated to 90 °C for 12 h. The reaction mixture was filtered, and the filter cake was washed with EtOAc (3 x 50 ml). The filtrate was concentrated *in vacuo.* The product was purified by silica gel chromatography (10%-100% EtOAc/Petroleum ether to give (6S,9R)-10-(4-methoxyphenyl)-6,7,8,9-tetrahydro-5H-6,9-epiminocyclohepta[c]pyridine **I-21e** ¹H NMR (400 MHz, CDCl₃) δ 8.40 (s, 1H), 8.28 (d, *J* = 5.2 Hz, 1H), 6.85 (d, *J =* 5.2 Hz, 1H), 6.77 - 6.71 (m, 4H), 4.73 (d, *J* = 5.2 Hz, 1H), 4.46 (t, *J =* 5.2 Hz, 1H), 3.69 (s, 3H), 3.25 - 3.20 (m, 1H), 2.42 - 2.35 (m, 3H), 1.97 - 1.93 (m, 1H), 1.79 - 1.77 (m, 1H).

**Step 6:** To a solution of (6*S*,9*R*)-10-(4-methoxyphenyl)-6,7,8,9-tetrahydro-5H-6,9-epiminocyclohepta[c]pyridine **(I-21e)** (1.08 g, 4.05 mmol) in MeCN (50 ml) was added a solution of CAN (6.67 g, 12.2 mmol) in water (50 ml) dropwise at 0 °C. The reaction mixture was stirred at 0 °C for 1 h before the reaction mixture was warmed to RT for 1 h. The reaction was cooled to 0 °C and a further portion of CAN (4.45 g, 8.11 mmol) in water (20 ml) was added and the mixture was stirred at 0 °C for 1 h. A solution of 2 M NaOH (50 ml) was added and the resultant mixture was filtered through a celite pad. The layers were separated and the aqueous layer was extracted with DCM (3 x 100 ml). The combined organics were dried over magnesium sulfate and concentrated *in vacuo* to give (6S,9R)-6,7,8,9-tetrahydro-5H-6,9-epiminocyclohepta[c]pyridine **I-21** as an off white solid. ¹H NMR (500 MHz, DMSO-*d*₆) δ 8.27 - 8.18 (m, 2H), 7.04 (d, *J =* 5.0 Hz, 1H), 4.17 (d, *J* = 5.7 Hz, 1H), 3.71 (s, 1H), 2.99 (dd, *J* = 17.4, 5.0 Hz, 1H), 2.72 (s, 1H), 2.47 (dd, *J* = 17.2, 1.1 Hz, 1H), 1.98 - 1.86 (m, 2H), 1.71 (t, *J =* 8.9 Hz, 1H), 1.49 - 1.42 (m, 1H).

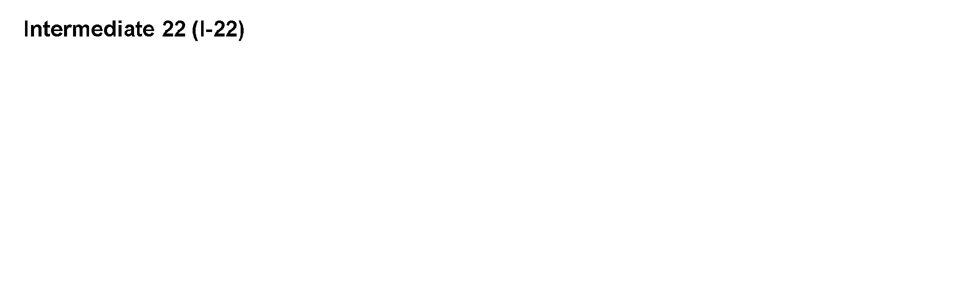

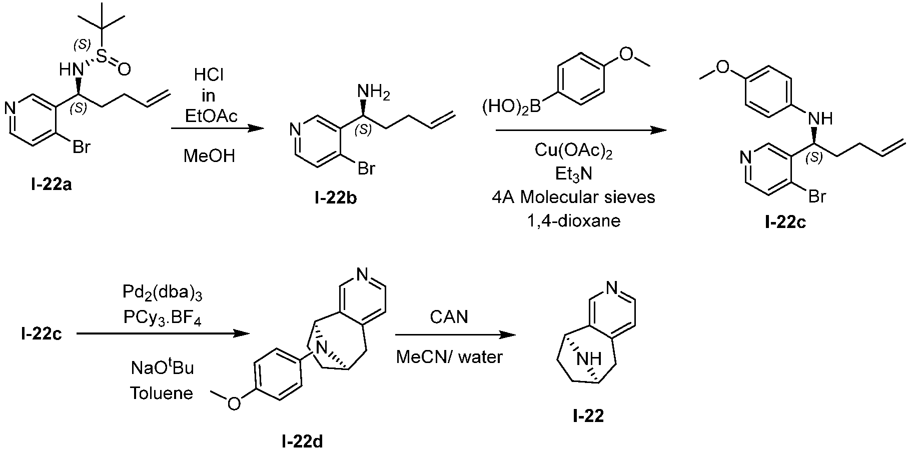

**Step 1:** (*S*)-1-(4-Bromopyridin-3-yl)pent-4-en-1-amine **I-22b** was synthesised from (*S*)-*N-*((*S*)-1-(4-bromopyridin-3-yl)pent-4-en-1-yl)-2-methylpropane-2-sulfinamide **I-22a** and using a procedure essentially the same as for **I-7d**. ¹H NMR (400 MHz, CDCl₃) δ 8.56 (d, *J* = 7.2 Hz, 1H), 8.25 (d, *J* = 5.2 Hz, 1H), 7.45 (d, *J* = 5.2 Hz, 1H), 5.87 - 5.79 (m, 1H), 5.06 - 4.97 (m, 2H), 4.36 - 4.33 (m, 1H), 2.20 - 2.12 (m, 2H), 1.89 - 1.85 (m, 1H), 1.78 - 1.75 (m, 1H). (NH₂ not observed)

**Step 2:** (*S*)-*N*-(1-(4-Bromopyridin-3-yl)pent-4-en-1-yl)-4-methoxyaniline **I-22c** was synthesised from *(S)*-1-(4-bromopyridin-3-yl)pent-4-en-1-amine **I-22b** using a procedure essentially the same as for **I-7e**. ¹H NMR (400 MHz, CDCl₃) δ: 8.57 (s, 1H), 8.26 (s, 1H), 7.50 (s, 1H), 6.70 (d, *J =* 2.4 Hz, 2H), 6.45 - 6.42 (m, 2H), 5.86 - 5.82 (m, 2H), 4.73 - 4.71 (m, 2H), 4.69 - 4.67 (m, 1H), 3.70 (s, 3H), 2.31 - 2.23 (m, 2H), 1.96-1.94 (m, 1H), 1.85-1.82 (m, 1H).

**Step 3:** (6*S*,9*R*)-10-(4-Methoxyphenyl)-6,7,8,9-tetrahydro-5H-6,9-epiminocyclohepta[c]pyridine) **I-22d** was synthesised from (S)-N-(1-(4-bromopyridin-3-yl)pent-4-en-1-yl)-4-methoxyaniline **I-22c** using a procedure essentially the same as for **I-21e.** ¹H NMR (400 MHz, CDCl₃) δ 8.66 (s, 1H), 8.29 - 8.24 (m, 1H), 7.48 - 7.45 (t, *J =* 8.8 Hz, 1H), 5.86 - 5.79 (m, 1H), 5.06 - 4.97 (m, 2H), 4.36 - 4.33 (m, 1H), 2.20 - 2.12 (m, 2H), 1.89 - 1.85 (m, 1H), 1.78 - 1.75 (m, 1H), 1.56 (s, 2H).

**Step 4:** (6*S*,9*R*)-6,7,8,9-tetrahydro-5H-6,9-epiminocyclohepta[c]pyridine **I-22** was synthesised from 1-(4-bromopyridin-3-yl)pent-4-en-1-amine **I-22d** using a procedure essentially the same as for **I-21**. ¹ H NMR (500 MHz, DMSO-*d*₆) δ 8.23 (d, *J =* 5.0 Hz, 1H), 8.19 (s, 1H), 7.03 (d, *J =* 5.0 Hz, 1H), 4.16 (d, *J =* 5.7 Hz, 1H), 3.70 (t, *J* = 6.0 Hz, 1H), 2.99 (dd, J = 17.4, 5.1 Hz, 1H), 2.00 - 1.86 (m, 2H), 1.70 (t, *J =* 8.9 Hz, 1H), 1.49 - 1.41 (m, 1H). (NH not observed, 1CH under DMSO peak)

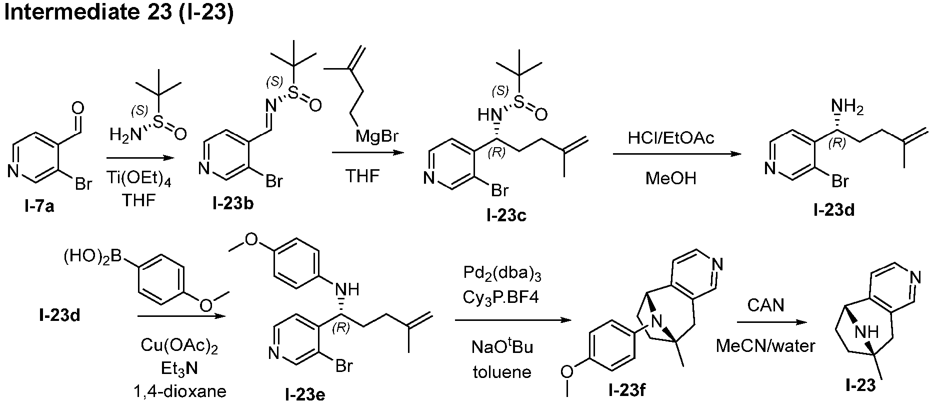

**Step 1:** (*S*)*-N*-((3-Bromopyridin-3-yl)methylene)-2-methylpropane-2-sulfinamide **I-23b** was synthesised from 3-bromo-4-pyridinecarboxaldehyde **I-7a** using a procedure essentially the same as for **I-7b**. ¹H NMR (400 MHz, CDCl₃) δ 8.92 (s, 1H), 8.87 (s, 1H), 8.64 - 8.63 (m, 1H), 7.85 - 7.84 (m, 1H), 1.30 (s, 3H).

**Step 2:** To a solution of (*S*)-*N*-((3-bromopyridin-3-yl)methylene)-2-methylpropane-2-sulfinamide (**I-23b**) (6.00 g, 20.7 mmol) in THF (120 ml) was added (3-methylbut-3-en-1-yl)magnesium bromide (0.5 M in THF, 124 ml) then the mixture was stirred at 20 °C for 1 h. Saturated NH₄Cl solution (50 ml) was added, and the product was extracted with EtOAc (3 x 20 ml). The combined organics were washed with water (20 ml) and brine (200 ml), dried with sodium sulfate, and concentrated *in vacuo.* The product was purified by silica gel chromatography (EtOAc/Petroleum ether 1%-100%) to give (*S*)-N-((*R*)-1-(3-bromopyridin-3-yl)-4-methylpent-4-en-1-yl)-2-methylpropane-2-sulfinamide **I-23c** as a yellow oil. ¹H NMR (400 MHz, CDCl₃) δ 8.72 - 8.64 (m, 1H), 8.61 (s, 1H), 7.33 - 7.32 (m, 1H), 4.85 - 4.81 (m, 1H), 4.77 - 4.73 (m, 2H), 2.14 - 1.93 (m, 4H), 1.72 (s, 3H), 1.19, (s, 9H)

**Step 3:** (*R*)-1-(3-Bromopyridin-3-yl)-4-methylpent-4-en-1-amine **I-23d** was synthesised from (*S*)-*N*-((*R*)-1-(3-bromopyridin-3-yl)-4-methylpent-4-en-1-yl)-2-methylpropane-2-sulfinamide I-**23c** using a procedure essentially the same as for **I-7d**. ¹H NMR (400 MHz, CDCl₃) δ 8.64 - 8.61 (m, 1H), 8.49 - 8.45 (m, 1H), 7.43 (t, *J =* 4.8 Hz, 1H), 4.72 - 4.69 (m, 2H), 4.30 - 4.24 (m, 1H), 2.14 - 2.05 (m, 2H), 1.87 - 1.70 (m, 1H), 1.68 - 1.64 (m, 1H).

**Step 4:** (*R*)-*N*-(1-(3-Bromopyridin-3-yl)-4-methylpent-4-en-1-yl)-4-methoxyaniline **I-23e** was synthesised from (R)-1-(3-bromopyridin-3-yl)-4-methylpent-4-en-1-amine **I-23d** using a procedure essentially the same as for **I-7e**. ¹H NMR (400 MHz, CDCl₃) δ 8.70 (s, 1H), 8.42 (s, 1H), 7.37 (s, 1H), 6.70 (d, *J* = 8.8 Hz, 2H), 6.36 (t, *J* = 8.8 Hz, 2H), 4.75 (d, *J* = 25.5 Hz, 2H), 4.65 - 4.62 (m, 1H), 3.70 (s, 3H), 2.26 - 2.20 (m, 2H), 2.12 - 1.98 (m, 3H), 1.77 - 1.75 (m, 4H).

**Step 5:** (5*R*,8*S*)-10-(4-Methoxyphenyl)-8-methyl-6,7,8,9-tetrahydro-5H-5,8-epiminocyclohepta[c]pyridine **I-23f** was synthesised from (*R*)-*N*-(1-(3-bromopyridin-3-yl)-4-methylpent-4-en-1-yl)-4-methoxyaniline **I-23e** using a procedure essentially the same as for **I-21e** ¹H NMR (400 MHz, CDCl₃) δ 8.42 (s, 1H), 8.22 (s, 1H), 7.09 (d, *J =* 4.8 Hz, 1H), 6.81 (d, *J* = 8.8 Hz, 2H), 6.67 (d, *J =* 8.8 Hz, 2H), 4.44 (d, *J =* 6.4 Hz, 2H), 3.71 (s, 3H), 2.72 - 2.68 (m, 1H), 2.49 - 2.45 (m, 1H), 2.33 - 2.30 (m, 1H), 2.05 - 2.02 (m, 1H), 1.99-1.93 (m, 2H), 1.72 - 1.70 (m, 1H), 1.56 (s, 3H).

**Step 6:** (5*R*,8*S*)-8-Methyl-6,7,8,9-tetrahydro-5H-5,8-epiminocyclohepta[c]pyridine (**I-23**) was synthesised from (5*R*,8*S*)-10-(4-Methoxyphenyl)-8-methyl-6,7,8,9-tetrahydro-5H-5,8-epiminocyclohepta[c]pyridine (**I-23f**) using a procedure essentially the same as for **I-21**. LCMS (method 2) m/z 175 (M+H)⁺ (ES⁺), at 1.15 min.

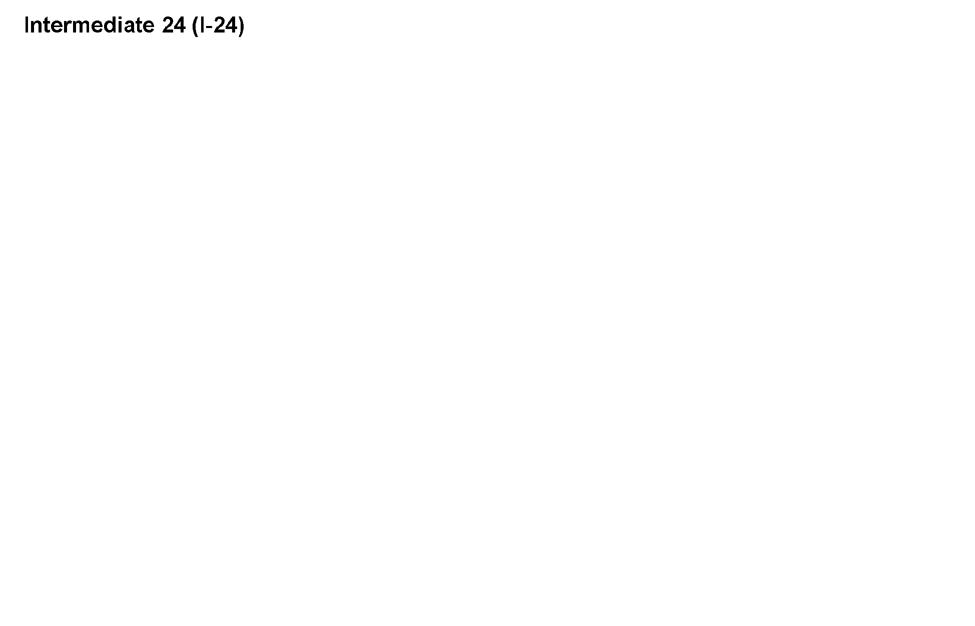

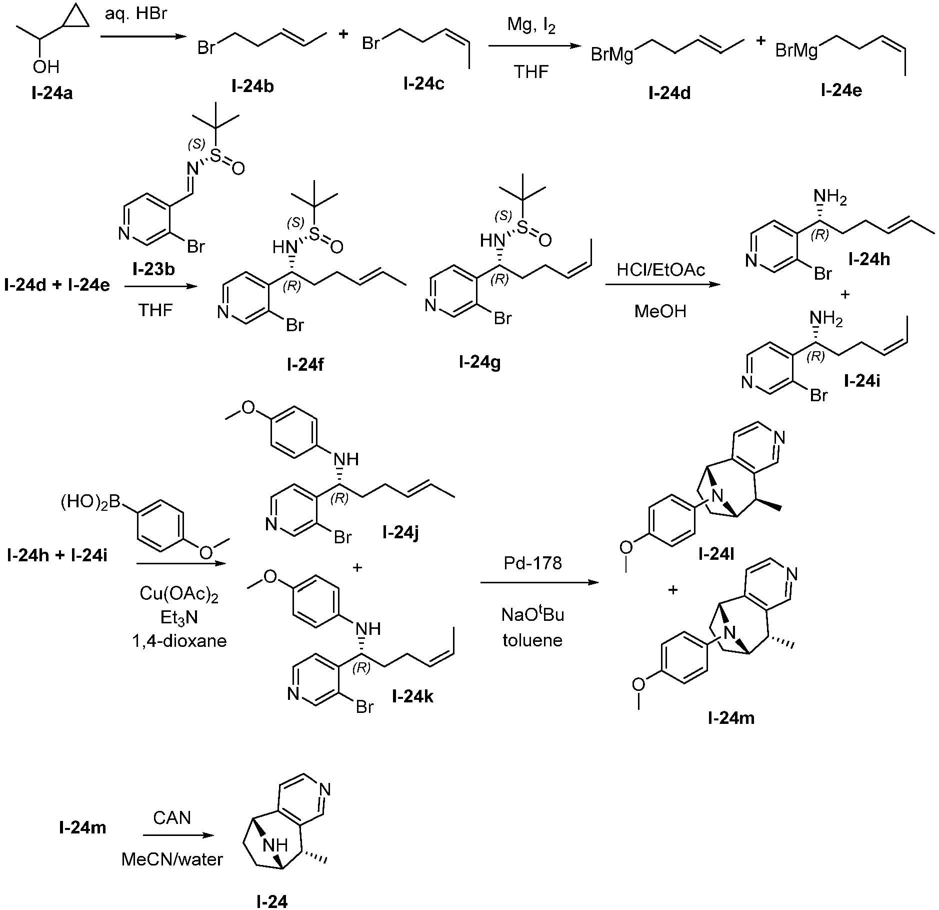

**Step 1:** 1-Cyclopropylethan-1-ol (11.3 ml, 116 mmol) was dissolved in aqueous HBr (45 ml, 47% w/w) at 0 °C. The mixture was warmed to RT for 12 h. The product was distilled *in vaccuo* (60 °C, 750 Torr) to give a mixture of (*E*)-5-bromopent-2-ene **I-24b** and (Z)-5-bromopent-2-ene **I-24c** as a yellow oil. ¹H NMR (400 MHz, CDCl₃) δ 5.58 - 5.52 (m, 1H), 5.43 - 5.36 (m, 1H), 3.37 - 3.33 (m, 2H), 2.56 - 2.50 (m, 2H), 1.67 - 1.65 (m, 3H).

**Step 2:** To a solution of I₂ (1.69 ml, 8.39 mmol) and Mg (8.66 g, 356 mmol) in THF (250 ml) at 20 °C was added a mixture of (*E*)-5-bromopent-2-ene **I-24b** and (*Z*)-5-bromopent-2-ene **I-24c** (25.0 g, 167 mmol) in THF (100 ml) at RT. The mixture was stirred at 40 °C for 1 h. The crude mixture of (*E*)-pent-3-en-1-ylmagnesium bromide **I-24d** and (Z)-pent-3-en-1-ylmagnesium **I-24e** was used into the next step without any work up or further purification.

**Step 3:** (*S*)-*N*-((*R*,*E*)-1-(3-Bromopyridin-4-yl)hex-4-en-1-yl)-2-methylpropane-2-sulfinamide **I-24f** and (*S*)-*N*-((*R*,*Z*)-1-(3-bromopyridin-4-yl)hex-4-en-1-yl)-2-methylpropane-2-sulfinamide **I-24g** was synthesised from (*S*,*E*)-*N*-((3-bromopyridin-3-yl)methylene)-2-methylpropane-2-sulfinamide **I-23b** and a mixture of mixture of (*E*)-pent-3-en-1-ylmagnesium bromide **I-24d** and (Z)-pent-3-en-1-ylmagnesium **I-24e** using a procedure essentially the same as for **I-7c**. Data for **I-24f** as **I-24g** was a minor proportion of the mixture. ¹H NMR (400 MHz, CDCl₃) δ 8.67 (s, 1H), 8.48 (d, *J* = 5.2 Hz, 1H), 5.50 - 5.39 (m, 2H), 4.88 - 4.81 (m, 1H), 3.59 (d, *J* = 3.6 Hz, 1H), 2.11 - 2.03 (m, 3H), 1.93 - 1.90 (m, 2H), 1.64-1.63 (m, 3H), 1.16 (s, 9H)

**Step 4:** (*R*,*E*)-1-(3-Bromopyridin-4-yl)hex-4-en-1-amine **I-24h** and (*R*,*Z*)-1-(3-Bromopyridin-4-yl)hex-4-en-1-amine **I-24i** was synthesised from (*S*)-*N*-((*R*,*E*)-1-(3-bromopyridin-4-yl)hex-4-en-1-yl)-2-methylpropane-2-sulfinamide **I-24f** and (*S*)-*N*-((*R*,*Z*)-1-(3-bromopyridin-4-yl)hex-4-en-1-yl)-2-methylpropane-2-sulfinamide **I-24g** using a procedure essentially the same as for **I-7d.**

**Step 5:** ((*R*,*E*)-*N*-(1-(3-Bromopyridin-4-yl)hex-4-en-1-yl)-4-methoxyaniline **I-24j** and (*R*,*Z*)-*N-*(1-(3-bromopyridin-4-yl)hex-4-en-1-yl)-4-methoxyaniline **I-24k** was synthesised from (*R*,*E*)-1-(3-bromopyridin-4-yl)hex-4-en-1-amine **I-24h** and (*R*,*Z*)-1-(3-bromopyridin-4-yl)hex-4-en-1-amine **I-24i** using a procedure essentially the same as for **I-7e**. Data for **I-24j** as **I-24k** was a minor proportion of the mixture. ¹H NMR (400 MHz, CDCl₃) δ 8.69 (s, 1H), 8.41 (s, 1H), 7.39 (s, 1H), 6.70 - 6.67 (m, 2H), 6.38 - 6.36 (m, 2H), 5.50 - 5.41 (m, 2H), 4.63 - 4.60 (m, 1H), 3.68 (s, 3H), 2.19 - 2.15 (m, 2H), 1.71 - 1.64 (m, 4H).

**Step 6:** To a solution of *((R,E)*-*N*-(1-(3-bromopyridin-4-yl)hex-4-en-1-yl)-4-methoxyaniline I-**24j** and (*R*,*Z*)-*N*-(1-(3-bromopyridin-4-yl)hex-4-en-1-yl)-4-methoxyaniline **I-24k** (0.54 g, 1.49 mmol) in dioxane (5 ml) was added NaO^{t}Bu (287 mg, 2.99 mmol) and Pd-178 (71.3 mg, 149 umol) at RT. The mixture was heated at 105 °C for 16 hours. The reaction mixture was filtered, the filter cake was washed with EtOAc (3 x 50 ml). The filtrate was concentrated *in vacuo.* The residue was purified by prep-HPLC (30-60% MeCN/10 mM NH₄HCO₃) to afford (5*R*,8*S*,9*R*)-10-(4-methoxyphenyl)-9-methyl-6,7,8,9-tetrahydro-5H-5,8-epiminocyclohepta[c]pyridine **I-24l** and as a white solid and (5*R*,8S,9S)-10-(4-methoxyphenyl)-9-methyl-6,7,8,9-tetrahydro-5H-5,8-epiminocyclohepta[c]pyridine **I-24k** as a white solid.

(5*R*,8*S*,9*R*)-10-(4-methoxyphenyl)-9-methyl-6,7,8,9-tetrahydro-5H-5,8-epiminocyclohepta[c]pyridine **I-24l**: ¹H NMR (400 MHz, CDCl₃) δ 8.37 (s, 2H), 7.06 - 7.01 (m, 1H), 6.88 - 6.78 (m, 4H), 4.67 (d, *J* = 5.2 Hz, 1H), 4.14 (d, *J* = 6.0, 1H), 3.75 (s, 3H), 2.81 (d, *J* = 7.6 Hz, 1H), 2.23 - 2.16 (m, 2H), 1.79 - 1.74 (m, 1H), 1.58 - 1.50 (m, 1H), 1.37 (d, *J* = 7.6 Hz, 3H).

(5*R*,8S,9S)-10-(4-methoxyphenyl)-9-methyl-6,7,8,9-tetrahydro-5H-5,8-epiminocyclohepta[c]pyridine **I-24k**: ¹H NMR (400 MHz, CDCl₃) δ 8.35 - 8.32 (m, 2H), 7.05 (d, *J* = 5.2 Hz, 1H), 6.99 - 6.73 (m, 4H), 4.57 (d, *J =* 7.6 Hz, 1H), 4.22 - 4.19 (m, 1H), 3.70 (s, 3H), 3.35 - 3.32 (m, 1H), 2.34 - 2.31 (m, 1H), 2.22 - 2.13 (m, 1H), 1.98 - 1.83 (m, 2H), 1.31 (d, *J* = 8.0 Hz, 3H).

**Step 7:** To a solution of (5*R*,8*S*,9*S*)-10-(4-methoxyphenyl)-9-methyl-6,7,8,9-tetrahydro-5H-5,8-epiminocyclohepta[c]pyridine **I-24k** (11.0 mg, 39.2 µmol) in MeCN (0.55 ml) was added a solution of CAN (65 mg, 118 µmol) in water (0.55 ml) was added dropwise at 0 °C. The mixture was stirred at 0 °C for 1 h. Water (10 ml) and 2 M NaOH (10 ml) was added, and the product was extracted with DCM (3 x 20 ml). The combined organics were concentrated *in vacuo* to give (5R,8S,9S)-9-methyl-6,7,8,9-tetrahydro-5H-5,8-epiminocyclohepta[c]pyridine **I-24** as a colourless oil. The product was used in the next step without further purification or analysis.

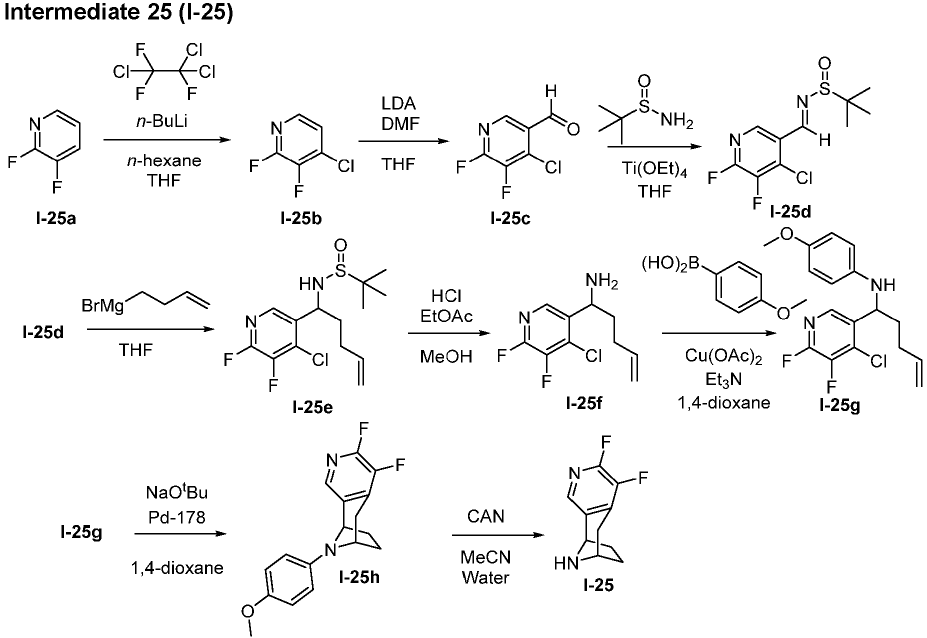

**Step 1:** To a solution of 2,3-difluoropyridine **I-25a** (50.0 g, 434 mmol) in hexane (275 ml) and THF (450 ml) was added *n*-BuLi (2.5 M in hexane, 173 ml) at -70 °C. The mixture was stirred at -70 °C for 2 h before 1,1,2-trichloro-1,2,2-trifluoroethane (52 ml, 434 mmol) was added at -70 °C and the resultant mixture was stirred at -70 °C for 1 h. Aqueous ammonium chloride solution (300 ml) was added at 0 °C and the product was extracted with EtOAc (3 x 100 ml). The combined organics were washed with brine (100 ml), dried with sodium sulfate and concentrated *in vacuo* to afford 4-chloro-2,3-difluoropyridine **I-25b** as a yellow oil. ¹H NMR (400 MHz, CDCl₃) δ 7.92 (d, *J* = 5.2 Hz, 1H), 7.29 (d, *J* = 2.0 Hz, 1H).

**Step 2:** To a solution of 4-chloro-2,3-difluoropyridine **I-25b** (17 g, 113 mmol) in THF (170 ml) was added a solution of LDA in THF/n-heptane/ethylbenzene (2 M, 68 ml) at -90 °C. The mixture was warmed -70 °C for 2 h. DMF (10.5 ml, 136 mmol) in THF (170 ml) was added slowly at -90 °C. The resultant mixture was slowly warmed to RT and stirred at RT for 1 h. An aqueous 1 M HCl solution (200 ml) was added at 0°C. The product was extracted with MTBE (3 x 100 ml). The combined organics were washed with brine (100 ml), dried over sodium sulfate and concentrated *in vacuo* to give 4-chloro-5,6-difluoronicotinaldehyde **I-25c** as a yellow oil. ¹H NMR (400 MHz, CDCl₃) δ 10.3 (s, 1H), 8.52 (s, 1H).

**Step 3:** *N*-((4-Chloro-5,6-difluoropyridin-3-yl)methylene)-2-methylpropane-2-sulfinamide **I-25d** was synthesised from 4-chloro-5,6-difluoronicotinaldehyde **I-25c** using a procedure essentially the same as for **I-7b**. ¹H NMR (400 MHz, CDCl₃) δ 8.89 (d, J = 2.4 Hz, 1H), 8.64 (s, 1H), 1.31 (s, 9H).

**Step 4:** *N-*(1-(4-Chloro-5,6-difluoropyridin-3-yl)pent-4-en-1-yl)-2-methylpropane-2-sulfinamide **I-25e** was synthesised from *N*-((4-chloro-5,6-difluoropyridin-3-yl)methylene)-2-methylpropane-2-sulfinamide **I-25d** using a procedure essentially the same as for **I-7c.** ¹H NMR (400 MHz, CDCl₃) δ 8.00 (s, 1H), 5.81 - 5.76 (m, 1H), 5.09 - 4.99 (m, 2H), 4.84 - 4.68 (m, 1H), 3.73 - 3.56 (m, 1H), 2.13 - 2.11 (m, 2H), 2.00 - 1.97 (m, 2H), 1.17 (s, 9H).

**Step 5:** 1-(4-Chloro-5,6-difluoropyridin-3-yl)pent-4-en-1-amine **I-25f** was synthesised from *N*-(1-(4-chloro-5,6-difluoropyridin-3-yl)pent-4-en-1-yl)-2-methylpropane-2-sulfinamide **I-25e** using a procedure essentially the same as for **I-7d**. ¹H NMR (400 MHz, CDCl₃) δ 8.11 (s, 1H), 5.84 - 5.76 (m, 1H), 5.06 - 4.98 (m, 2H), 4.33 - 4.30 (m, 1H), 2.18 - 2.12 (m, 2H), 1.85-1.73 (m, 2H).

**Step 6:** *N*-(1-(4-Chloro-5,6-difluoropyridin-3-yl)pent-4-en-1-yl)-4-methoxyaniline **I-25g** was synthesised from 1-(4-chloro-5,6-difluoropyridin-3-yl)pent-4-en-1-amine **I-25f** using a procedure essentially the same as for **I-7e**. ¹H NMR (400 MHz, CDCl₃) δ 8.00 (s, 1H), 6.71 - 6.69 (m, 2H), 6.42 - 6.39 (m, 2H), 5.84 - 5.79 (m, 1H), 5.08 - 5.03 (m, 2H), 4.67 - 4.65 (m, 1H), 3.70 (s, 3H), 2.29 - 2.20 (m, 2H), 1.95 - 1.86 (m, 2H).

**Step 7:** To a solution of *N*-(1-(4-chloro-5,6-difluoropyridin-3-yl)pent-4-en-1-yl)-4-methoxyaniline **I-25g** (0.30 g, 885 umol) in 1,4-dioxane (10 ml) was added NaO^{t}Bu (127 mg, 1.32 mmol) and Pd-178 (42 mg, 88.5 umol) at RT. The resultant mixture was heated at 95 °C for 16 h. The reaction mixture was filtered. The filter cake was washed with EtOAc (3 x 10 ml), the combined organics were dried over sodium sulfate and concentrated *in vacuo.* The product was purified by chromatography on silica (1-100%,EtOAc/Petroleum ether) to afford (±)-3,4-difluoro-10-(4-methoxyphenyl)-6,7,8,9-tetrahydro-5H-6,9-epiminocyclohepta[c]pyridine **I-25h** as a yellow solid. ¹H NMR (400 MHz, CDCl₃) δ 7.79 (s, 1H), 6.74 - 6.70 (m, 4H), 4.80 - 4.78 (m, 1H), 4.52 - 4.49 (m, 1H), 3.71 (s, 3H), 3.17 - 3.13 (m, 1H), 2.55 - 2.38 (m, 2H), 1.96 - 1.91 (m, 1H), 1.75-1.70 (m, 1H).

**Step 8:** (±)-3,4-Difluoro-6,7,8,9-tetrahydro-5H-6,9-epiminocyclohepta[c]pyridine **I-25** was synthesised from (±)-3,4-difluoro-10-(4-methoxyphenyl)-6,7,8,9-tetrahydro-5H-6,9-epiminocyclohepta[c]pyridine **I-25h** using a procedure essentially the same as for **I-24**. ¹H NMR (500 MHz, DMSO-*d*₆) δ 7.75 (d, *J* = 1.6 Hz, 1H), 4.31 - 4.26 (m, 1H), 3.76 (t, *J* = 5.9 Hz, 1H), 3.00 - 2.91 (m, 1H), 2.74 (s, 1H), 2.59 (dd, *J* = 18.0, 1.3 Hz, 1H), 2.00 - 1.87 (m, 2H), 1.78 - 1.66 (m, 1H), 1.57 - 1.46 (m, 1H)

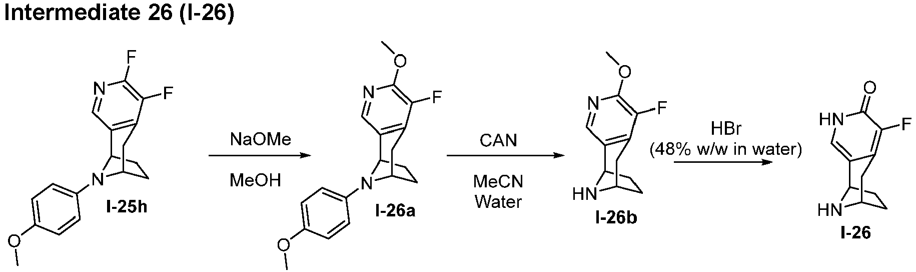

**Step 1:** To a solution of (±)-3,4-difluoro-10-(4-methoxyphenyl)-6,7,8,9-tetrahydro-5H-6,9-epiminocyclohepta[c]pyridine **I-25h** (145 mg, 480 µmol) in MeOH (5 ml) was added a solution of sodium methoxide in MeOH (133 µl, 5.4 M, 719 µmol). The reaction mixture was heated to 65 °C for 18 h. The reaction mixture was diluted with MeOH (5 ml) and an additional portion of sodium methoxide in MeOH (133 µl, 5.4 M, 719 µmol) was added and the reaction mixture was stirred at 65 °C for a further 3 h. The reaction mixture was diluted with DCM (30 ml) and water (10 ml) was added and the layers were separated. The aqueous was extracted with DCM (3 x 20 ml) and the combined organic layer were dried over sodium sulfate and concentrated *in vacuo* to give (±)-4-fluoro-3-methoxy-10-(4-methoxyphenyl)-6,7,8,9-tetrahydro-5H-6,9-epiminocyclohepta[c]pyridine **I-26a** as a brown oil. ¹H NMR (500 MHz, DMSO-*d*₆) δ 7.88 (s, 1H), 6.84 - 6.78 (m, 2H), 6.73 - 6.68 (m, 2H), 4.92 (d, *J* = 5.3 Hz, 1H), 4.51 (t, *J =* 5.9 Hz, 1H), 3.85 (s, 3H), 3.61 (d, *J* = 2.4 Hz, 3H), 2.97 (dd, *J* = 18.0, 5.0 Hz, 1H), 2.46 (s, 1H), 2.25 (q, *J =* 6.5, 4.5 Hz, 2H), 1.88 - 1.70 (m, 2H).

**Step 2:** (±)-4-Fluoro-3-methoxy-6,7,8,9-tetrahydro-5H-6,9-epiminocyclohepta[c]pyridine **I-26b** was synthesised from (±)-4-fluoro-3-methoxy-10-(4-methoxyphenyl)-6,7,8,9-tetrahydro-5H-6,9-epiminocyclohepta[c]pyridine **I-26a** using a procedure essentially the same as for **I-24.** ¹H NMR (500 MHz, DMSO-*d*₆) δ 7.66 (s, 1H), 4.20 (d, *J* = 4.9 Hz, 1H), 3.87 (s, 3H), 3.73 (t, *J* = 5.9 Hz, 1H), 2.88 (dd, *J* = 17.6, 5.2 Hz, 1H), 2.70 (s, 1H), 2.53 (d, *J =* 1.3 Hz, 1H), 1.95 - 1.86 (m, 2H), 1.74 - 1.64 (m, 1H), 1.54 - 1.44 (m, 1H).

**Step 3:** A solution of (±)-4-fluoro-3-methoxy-6,7,8,9-tetrahydro-5H-6,9-epiminocyclohepta[c]pyridine **I-26b** (190 mg, 999 µmol) in aqueous HBr (2.8 M, 48% Wt, 25.0 mmol) was refluxed for 16 h. The reaction mixture was concentrated *in vacuo.* The residue was dissolved in MeOH, loaded onto a SCX cartridge (10 g), the cartridge was washed with MeOH and the product was eluted with 0.7 M ammonia in MeOH solution. The filtrate was concentrated *in vacuo* to afford (±)-2,5,6,7,8,9-hexahydro-3H-6,9-epiminocyclohepta[c]pyridin-3-one **I-26** as a brown solid. LCMS (method 1) m/z 177.2 (M+H)⁺ (ES⁺), at 0.40 min. ¹H NMR (500 MHz, DMSO-*d*₆) δ 11.07 (s, 1H), 7.02 (s, 1H), 6.00 (s, 1H), 4.01 (d, *J* = 5.5 Hz, 1H), 3.60 (t, *J =* 6.1 Hz, 1H), 2.82 (dd, *J* = 17.6, 5.1 Hz, 1H), 2.42 - 2.34 (m, 1H), 1.90 - 1.77 (m, 2H), 1.63 - 1.55 (m, 1H), 1.48 - 1.39 (m, 1H).

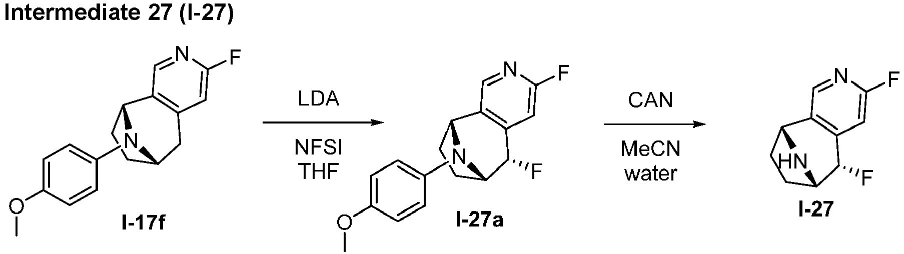

**Step 1:** To a solution of (6S,9R)-3-fluoro-10-(4-methoxyphenyl)-6,7,8,9-tetrahydro-5H-6,9-epiminocyclohepta[c]pyridine **I-17f** (100 mg, 352 µmol) in THF (3.5 ml) at 0 °C was added a soution of LDA (2 M in THF) (194 µl, 387 µmol) and the reaction was stirred at 0 °C for 1 h. A solution of NFSI (222 mg, 703 µmol) in THF (1 ml) was added and the reaction was stirred at 0 °C for 5 min and warmed to RT for 16 h. The reaction mixture was diluted with EtOAc (5 ml) and water (10 ml) was added. The layers were separated. The aqueous layer was extracted with EtOAc (2 x 10 ml). The combined organics were dried over magnesium sulfate and concentrated *in vacuo* to give a brown oil. The product was purified by chromatography on silica gel (0-100% EtOAc/isohexane) to afford (5*S*,6*S*,9*R*)-3,5-difluoro-10-(4-methoxyphenyl)-6,7,8,9-tetrahydro-5H-6,9-epiminocyclohepta[c]pyridine **I-27a** as a pale yellow oil. LCMS (method 5) m/z 303.1 (M+H)⁺ (ES⁺), at 2.30 min. ¹H NMR (500 MHz, CDCl₃) δ 8.08 (s, 1H), 6.94 (d, *J* = 2.3 Hz, 1H), 6.86 - 6.63 (m, 4H), 5.70 (ddd, *J* = 50.9, 5.0, 1.3 Hz, 1H), 4.87 - 4.69 (m, 1H), 4.61 (t, *J =* 5.8 Hz, 1H), 3.72 (s, 3H), 2.46 - 2.27 (m, 1H), 2.28 - 2.10 (m, 2H), 1.79 (ddd, *J* = 11.6, 8.6, 2.8 Hz, 1H)

**Step 2:** (5*S*,6*S*,9*R*)-3,5-difluoro-6,7,8,9-tetrahydro-5H-6,9-epiminocyclohepta[c]pyridine **I-27** was synthesised from (5S,6S,9R)-3,5-difluoro-10-(4-methoxyphenyl)-6,7,8,9-tetrahydro-5H-6,9-epiminocyclohepta[c]pyridine **I-27a** using a procedure essentially the same as for **I-24**.

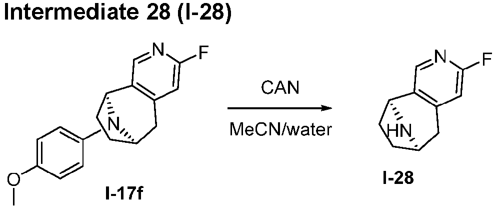

**Step 1:** (6*R*,9*S*)-3-fluoro-6,7,8,9-tetrahydro-5H-6,9-epiminocyclohepta[c]pyridine (**I-28**) was synthesised from (6*R*,9*S*)-3-fluoro-10-(4-methoxyphenyl)-6,7,8,9-tetrahydro-5H-6,9-epiminocyclohepta[c]pyridine **(I-17g)** using a procedure essentially the same as for **I-13**. LCMS (method 1) m/z 179.2 (M+H)⁺ (ES⁺), at 0.71 min, ¹H NMR (500 MHz, Chloroform-d) δ 7.80 (s, 1H), 6.58 (d, *J* = 2.0 Hz, 1H), 4.28 (d, *J =* 5.6 Hz, 1H), 3.93 - 3.69 (m, 1H), 3.10 (ddd, *J* = 17.8, 5.2, 1.5 Hz, 1H), 2.57 (d, *J* = 17.8 Hz, 1H), 2.14 - 1.91 (m, 3H), 1.90 - 1.77 (m, 1H), 1.67 - 1.42 (m, 1H).

### Experimental Scheme 2

**Compound 45:** (±)-*N*-(3-Chloro-4-(trifluoromethyl)phenyl)-2-fluoro-6,7,8,9-tetrahydro-5H-5,8-epiminocyclohepta[d]pyrimidine-10-carboxamide.

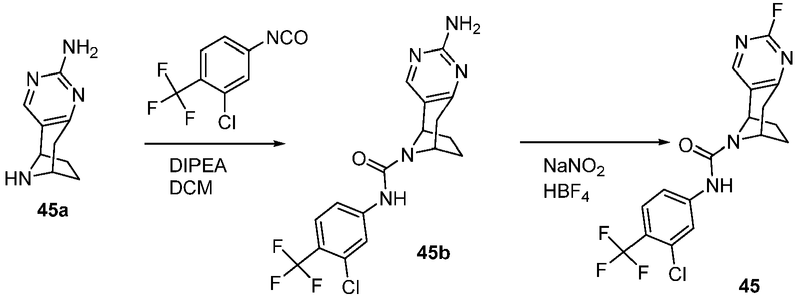

**Step 1:** 2-Amino-*N*-(3-chloro-4-(trifluoromethyl)phenyl)-6,7,8,9-tetrahydro-5H-5,8-epiminocyclohepta[d]pyrimidine-10-carboxamide **45b** was synthesised from 2-amino-6,7,8,9-tetrahydro-5H-5,8-epiminocyclohepta[d]pyrimidin-2-amine **45a** using a procedure essentially the same as for 1. LCMS (method 1): m/z 398.1, 400.0 (M+H)⁺ (ES⁺); at 1.19 min. ¹H NMR (500 MHz, DMSO-d6) δ 9.16 (s, 1H), 8.01 (s, 1H), 7.91 (d, *J* = 2.0 Hz, 1H), 7.70 (d, *J =* 8.8 Hz, 1H), 7.62 (dd, *J* = 8.8, 2.1 Hz, 1H), 6.45 (s, 2H), 5.12 (d, *J* = 5.9 Hz, 1H), 4.71 (dd, *J* = 7.9, 5.0 Hz, 1H), 3.11 (ddd, *J* = 18.3, 5.3, 1.4 Hz, 1H), 2.23 (q, *J =* 11.3, 10.8 Hz, 1H), 2.11 (tt, *J* = 11.8, 6.2 Hz, 1H), 1.84 - 1.66 (m, 2H). (1 H obscured by residual solvent peak).

**Step 2:** To a solution of 2-amino-*N*-(3-chloro-4-(trifluoromethyl)phenyl)-6,7,8,9-tetrahydro-5H-5,8-epiminocyclohepta[d]pyrimidine-10-carboxamide **45b** (100 mg, 251 µmol) in THF (2 ml) was added tetrafluoroboric acid in water (2.34 ml, 48% w/w, 15.1 mmol) and the resultant mixture was cooled to 0 °C. A solution of sodium nitrite (34.7 mg, 503 µmol) in water (2 ml) was added dropwise and the reaction mixture was stirred at 0 °C for 1 h before warming to RT for 16 h. The reaction mixture was re-cooled to 0 °C and an additional portion of sodium nitrite (34.7 mg, 503 µmol) in water (2 ml) was added. The reaction was stirred for 1 h. The reaction mixture was added to a solution of ice cold NaHCO₃ (10 ml) and the product was extracted using 10% MeOH in DCM solution (3 x 50 ml). The solvent was dried over sodium sulfate, filtered and concentrated *in vacuo.* The product was purified by mass directed HPLC (20-50 % MeCN/10 mM aqueous ammonium bicarbonate solution, C18) to yield (±)-N-(3-chloro-4-(trifluoromethyl)phenyl)-2-hydroxy-6,7,8,9-tetrahydro-5H-5,8-epiminocyclohepta[d]pyrimidine-10-carboxamide as a light yellow solid. LC-MS (method 1) m/z 399.3, 401.3 (M+H)⁺ (ES⁺); at 1.15 min. ¹H NMR (500 MHz, DMSO-d6) δ 9.30 (s, 1H), 8.62 (d, *J =* 1.7 Hz, 1H), 7.89 (d, *J* = 2.1 Hz, 1H), 7.72 (d, *J* = 8.8 Hz, 1H), 7.60 (d, *J* = 9.0 Hz, 1H), 5.39 (d, *J* = 6.1 Hz, 1H), 4.83 (t, *J* = 6.4 Hz, 1H), 3.38 (m, 1H), 2.84 (d, *J* = 18.8 Hz, 1H), 2.32 - 2.23 (m, 1H), 2.18 (dt, *J =* 11.7, 5.7 Hz, 1H), 1.91 (t, *J =* 10.7 Hz, 1H), 1.78 (dd, *J* = 17.8, 10.6 Hz, 1H).

The following compound was prepared using appropriate starting materials in an analogous procedure to that described in Experimental Scheme 2.

| Compound | Structure | UPLC method 1 Rt [M+H]⁺ | NMR |
|---|---|---|---|
| 46 | 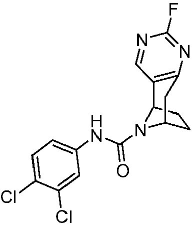 (±)-*N*-(3,4-Dichlorophenyl)-2-fluoro-6,7,8,9-tetrahydro-5H-5,8-epiminocyclohepta[d]pyrimidi ne-10-carboxamide | 367.2, 369.4 at 1.40 min | ¹H NMR (500 MHz, DMSO-d6) δ 9.04 (s, 1H), 8.60 (d, *J* = 1.7 Hz, 1H), 7.82 (d, *J* = 2.4 Hz, 1H), 7.48 (d, *J* = 8.9 Hz, 1H), 7.43 (dd, *J* = 8.8, 2.4 Hz, 1H), 5.35 (d, *J* = 6.2 Hz, 1H), 4.79 (t, *J* = 6.4 Hz, 1H), 3.35 (d, *J* = 5.1 Hz, 1H), 2.81 (d, *J* = 18.8 Hz, 1H), 2.29 - 2.21 (m, 1H), 2.16 (tt, *J* = 12.1, 6.3 Hz, 1H), 1.89 (t, *J* = 10.1 Hz, 1H), 1.77 (dt, *J* = 15.1, 7.7 Hz, 1H) |

### Experimental Scheme 3

### Compound 47: (±)-N-(3-Chloro-4-(trifluoromethyl)phenyl)-9-(hydroxyimino)-2-oxo-3,5,6,7,8,9-hexahydro-2H-5,8-epiminocyclohepta[d]pyrimidine-10-carboxamide

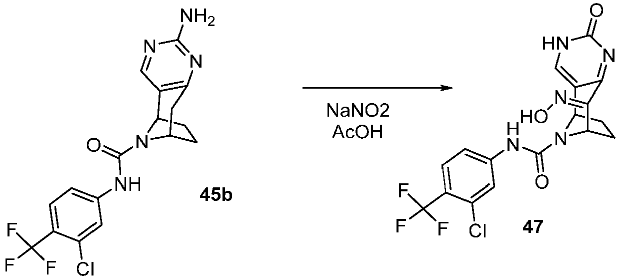

To a solution of 2-amino-*N*-(3-chloro-4-(trifluoromethyl)phenyl)-6,7,8,9-tetrahydro-5H-5,8-epiminocyclohepta[d]pyrimidine-10-carboxamide **45b** (200 mg, 503 µmol) in AcOH (5 ml) was added a solution of sodium nitrite (208 mg, 3.02 mmol) in water (2 ml) dropwise. The reaction mixture was stirred for 2 h. A further portion of water (2 ml) was added the reaction mixture was stirred for 72 h. The precipitate was washed with water and dried *in vacuo* to give (±)-*N*-(3-chloro-4-(trifluoromethyl)phenyl)-9-(hydroxyimino)-2-oxo-3,5,6,7,8,9-hexahydro-2H-5,8-epiminocyclohepta[d]pyrimidine-10-carboxamide **47** as a yellow solid. LC-MS (method 1) m/z 428.3, 430.3 (M+H)⁺ (ES⁺); at 1.15 min. ¹H NMR (500 MHz, DMSO-d6) δ 12.40 (s, 1H), 11.82 (s, 1H), 9.42 (s, 1H), 7.83 (d, *J* = 2.0 Hz, 1H), 7.71 (d, *J =* 8.8 Hz, 1H), 7.55 (dd, *J* = 8.5, 2.1 Hz, 1H), 5.80 (d, *J* = 6.8 Hz, 1H), 5.23 (s, 1H), 2.35 - 2.24 (m, 2H), 1.80 (d, *J =* 9.3 Hz, 1H), 1.71 (d, *J* = 11.6 Hz, 1H). (1NH not observed)

### Experimental Scheme 4

### Compound 11 (±)-N-(3-chloro-4-cyanophenyl)-6,7,8,9-tetrahydro-5H-5,8-epiminocyclohepta[d]pyrimidine-10-carboxamide

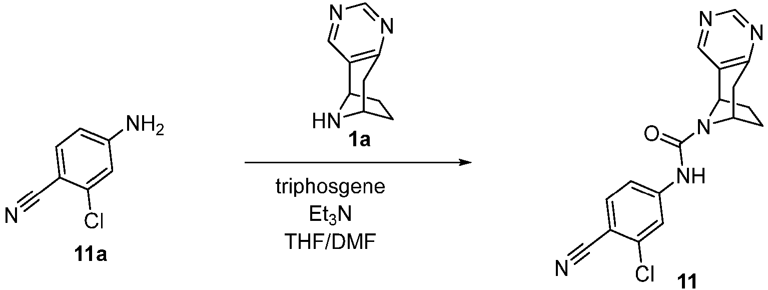

To a solution of 4-amino-2-chlorobenzonitrile (47 mg, 0.31 mmol) in THF (1 ml) was added a solution of triphosgene (37 mg, 0.12 mmol) in THF (1 ml) followed by Et₃N (130 µl, 0.93 mmol). The resultant mixture was stirred at RT for 30 min. A solution of 6,7,8,9-tetrahydro-5H-5,8-epiminocyclohepta[d]pyrimidine (50 mg, 0.31 mmol) in DMF (0.5 ml) was added and the resultant mixture was stirred at RT for 72 h. A solution of 2 M NaOH (2 ml) was added and the product was extracted with DCM (3 ml). The organics were passed through a hydrophobic frit and the filtrate was concentrated *in vacuo.* The product was purified by product mass directed HPLC (20-50% MeCN/10 mM aqueous ammonium bicarbonate solution, C18) to afford (±)-*N*-(5-(trifluoromethyl)pyrazin-2-yl)-6,7,8,9-tetrahydro-5H-5,8-epiminocyclohepta[d]pyrimidine-10-carboxamide as an off white solid. LC-MS (method 1) m/z 340.3, 342.3 (M+H)⁺ (ES⁺) at 1.05 min, ¹H NMR (500 MHz, DMSO-d6) δ 9.38 (s, 1H), 8.96 (s, 1H), 8.61 (s, 1H), 7.91 (d, *J* = 2.0 Hz, 1H), 7.80 (d, *J =* 8.7 Hz, 1H), 7.59 (dd, *J =* 8.7, 2.0 Hz, 1H), 5.31 (d, *J* = 6.2 Hz, 1H), 4.84 (t, *J* = 6.5 Hz, 1H), 3.37 - 3.34 (m, 1H), 2.80 (d, *J* = 18.4 Hz, 1H), 2.34 - 2.14 (m, 2H), 1.96 - 1.86 (m, 1H), 1.81 - 1.72 (m, 1H).

The following compounds were prepared using appropriate starting materials in an analogous procedure to that described in Experimental Scheme 4. Where the starting materials are not described in the literature, their synthesis is described below.

Key: (a) Reaction performed in DCM. (b) purification was by silica gel chromatography (EtOAc/isohexane). (c) purification was by silica gel chromatography ((0.7 M Ammonia in MeOH/DCM) (d) (M-H)⁻ ES⁽⁻⁾ as no ionisation in ES⁽⁺⁾ (e) LCMS method 6 (f) purified by prep TLC (MeOH/DCM)

| Compound | Structure | LCMS method 1 Rt [M+H]⁺ | NMR |
|---|---|---|---|
| 15(a) | 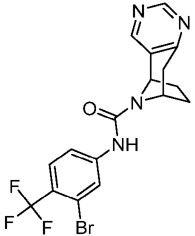 (±)-*N*-(3-Bromo-4-(trifluoromethyl)phenyl)-6,7,8,9-tetrahydro-5H-5,8-epiminocyclohepta[d]pyrimidi ne-10-carboxamide | 427.3, 429.3 at 1.34 min | ¹H NMR (500 MHz, DMSO-d6) δ 9.26 (s, 1H), 8.96 (s, 1H), 8.61 (s, 1H), 8.06 (d, *J =* 2.0 Hz, 1H), 7.70 (d, *J* = 8.8 Hz, 1H), 7.66 (dd, *J* = 8.9, 2.0 Hz, 1H), 5.31 (d, *J* = 6.2 Hz, 1H), 4.87 - 4.81 (m, 1H), 3.39 - 3.33 (m, 1H), 2.80 (d, *J* = 18.6 Hz, 1H), 2.34 - 2.15 (m, 2H), 1.96 - 1.86 (m, 1H), 1.81 - 1.72 (m, 1H). |
| 79^{(a)(b)} | 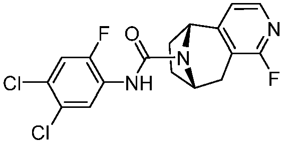 (5*R*,8*S*)-*N*-(4,5-Dichloro-2-fluorophenyl)-1-fluoro-6,7,8,9-tetrahydro-5H-5,8-epiminocyclohepta[c]pyridine -10-carboxamide | 382.0, 384.1, 386.3 at 1.47 min | ¹H NMR (500 MHz, DMSO-d6) δ 8.82 (s, 1H), 8.07-7.93 (m, 1H), 7.88-7.78 (m, 1H), 7.71-7.59 (m, 1H), 7.19 (s, 1H), 5.20 (br s, 1H), 4.77 (br s, 1H), 3.24-3.10 (m, 2H), 2.32-2.12 (m, 2H), 1.89-1.68 (m, 2H). |
| 88^{(a)(c)} | 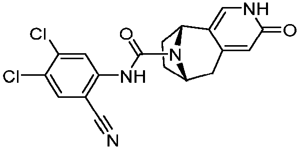 (6*S*,9*R*)-*N*-(4,5-Dichloro-2-cyanophenyl)-3-oxo-3,5,6,7,8,9-hexahydro-2H-6,9-epiminocyclohepta[c]pyridine -10-carboxamide | 389.4, 390.3, 393.3 at 1.05 min | ¹H NMR (500 MHz, DMSO-*d*₆) δ 11.26 (s, 1H), 9.17 (s, 1H), 8.17 (s, 1H), 7.76 (s, 1H), 7.19 (s, 1H), 6.09 (s, 1H), 5.04 (d, *J* = 6.0 Hz, 1H), 4.57 (t, *J* = 6.5 Hz, 1H), 3.16 (dt, *J* = 10.8, 5.7 Hz, 1H), 2.58 (d, *J* = 17.8 Hz, 1H), 2.18 (q, *J* = 11.0 Hz, 1H), 2.08 (tt, *J =* 11.8, 6.3 Hz, 1H), 1.77 - 1.70 (m, 1H), 1.70 - 1.63 (m, 1H). |
| 93^{(a)} | 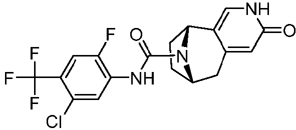 (6*S*,9*R*)-*N*-(5-Chloro-2-fluoro-4-(trifluoromethyl)phenyl)-3-oxo-3,5,6,7,8,9-hexahydro-2H-6,9-epiminocyclohepta[c]pyridine -10-carboxamide | 416.3, 418.3 at 1.19 min | ¹H NMR (500 MHz, DMSO-d6) δ 11.27 (s, 1H), 8.89 (s, 1H), 8.04 (d, *J* = 6.9 Hz, 1H), 7.77 (d, *J =* 10.9 Hz, 1H), 7.21 (s, 1H), 6.10 (s, 1H), 5.08 (d, *J* = 6.0 Hz, 1H), 4.61 (t, *J* = 6.4 Hz, 1H), 3.13 (dd, *J* = 17.6, 5.2 Hz, 1H), 2.56 (d, *J* = 17.9 Hz, 1H), 2.17 (q, *J* = 11.0 Hz, 1H), 2.07 (tt, *J =* 11.9, 6.3 Hz, 1H), 1.80 - 1.56 (m, 2H). |
| 94(a) | 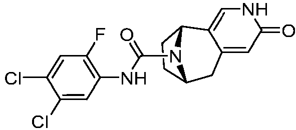 (6*S*,9*R*)-*N*-(4,5-Dichloro-2-fluorophenyl)-3-oxo-3,5,6,7,8,9-hexahydro-2H-6,9-epiminocyclohepta[c]pyridine -10-carboxamide | 382.6, 384.6 at 1.08 min | ¹H NMR (500 MHz, DMSO-d6) δ 7.83 (d, *J* = 7.5 Hz, 1H), 7.66 (d, *J =* 10.3 Hz, 1H), 7.19 (s, 1H), 6.10 (s, 1H), 5.03 (d, *J* = 6.0 Hz, 1H), 4.67 - 4.53 (m, 1H), 3.12 (dd, *J =* 18.0, 5.1 Hz, 1H), 2.54 (d, *J* = 18.2 Hz, 1H), 2.16 (q, *J =* 11.1, 10.7 Hz, 1H), 2.12 - 2.00 (m, 1H), 1.76 - 1.58 (m, 2H), 2 NH not observed. |
| 98^{(a)(c)} | 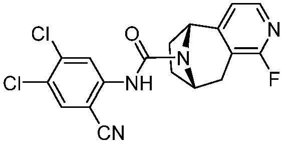 (5*R*,8*S*)-*N*-(4,5-Dichloro-2-cyanophenyl)-1-fluoro-6,7,8,9-tetrahydro-5H-5,8-epiminocyclohepta[c]pyridine -10-carboxamide | 391.3, 393.3 at 1.36 min | ¹H NMR (500 MHz, DMSO-*d*₆) δ 9.36 (s, 1H), 8.15 (s, 1H), 7.99 (d, *J* = 5.0 Hz, 1H), 7.72 (s, 1H), 7.19 (dd, *J =* 5.1, 1.5 Hz, 1H), 5.18 (d, *J* = 6.1 Hz, 1H), 4.75 (t, *J =* 6.1 Hz, 1H), 3.25 (dd, *J* = 17.4, 4.9 Hz, 1H), 2.60 (d, *J =* 17.3 Hz, 1H), 2.32 - 2.16 (m, 2H), 1.88 (q, *J* = 10.9, 10.5 Hz, 1H), 1.77 (t, *J* = 6.3 Hz, 1H). |
| 101^{(a)(c)} | 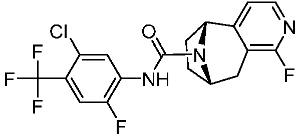 (5*R*,8*S*)-*N*-(5-Chloro-2-fluoro-4-(trifluoromethyl)phenyl)-1-fluoro-6,7,8,9-tetrahydro-5H-5,8-epiminocyclohepta[c]pyridine -10-carboxamide | 418.2, 420.2 at 1.54min | ¹H NMR (500 MHz, DMSO-*d*₆) δ 9.08 (s, 1H), 8.01 (dd, *J* = 6.0, 3.3 Hz, 2H), 7.77 (d, *J =* 11.0 Hz, 1H), 7.21 (dd, *J* = 5.2, 1.6 Hz, 1H), 5.24 (d, *J* = 6.1 Hz, 1H), 4.82 (t, *J* = 6.1 Hz, 1H), 3.19 (dd, *J* = 17.2, 5.1 Hz, 1H), 2.60 (d, *J =* 17.3 Hz, 1H), 2.28 - 2.15 (m, 2H), 1.85 (t, *J =* 9.8 Hz, 1H), 1.80 - 1.71 (m, 1H). |
| **117** ^{(a)(b)} | 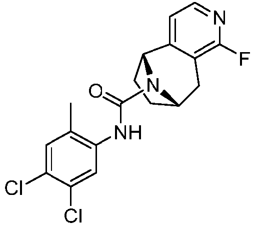 (5*R*,8*S*)-*N*-(4,5-Dichloro-2-methylphenyl)-1-fluoro-6,7,8,9-tetrahydro-5H-5,8-epiminocyclohepta[c]pyridine -10-carboxamide | 380.3, 382.1 at 1.44 min | ¹H NMR (500 MHz, DMSO-d6) δ 8.44 (s, 1H), 8.00 (d, *J* = 5.0 Hz, 1H), 7.46 (d, *J =* 27.8 Hz, 2H), 7.19 (d, *J* = 5.0 Hz, 1H), 5.17 (d, *J =* 6.1 Hz, 1H), 4.73 (d, *J =* 6.4 Hz, 1H), 3.21 (dd, *J* = 17.2, 5.1 Hz, 1H), 2.57 (d, *J* = 17.3 Hz, 1H), 2.30 - 2.16 (m, 2H), 2.01 (s, 3H), 1.90 - 1.81 (m, 1H), 1.81 - 1.70 (m, 1H). |
| 118^{(a)(b)} | 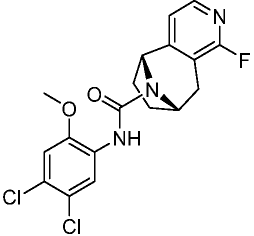 (5*R*,8*S*)-*N*-(4,5-Dichloro-2-methoxyphenyl)-1-fluoro-6,7,8,9-tetrahydro-5H-5,8-epiminocyclohepta[c]pyridine -10-carboxamide | 396.3, 398.5 at 1.52 min | ¹H NMR (500 MHz, DMSO-d6) δ 8.13 (s, 1H), 8.00 (d, *J* = 5.0 Hz, 1H), 7.81 (s, 1H), 7.24 (s, 1H), 7.20 (dd, *J =* 5.1, 1.6 Hz, 1H), 5.18 (d, *J* = 6.2 Hz, 1H), 4.74 (t, *J* = 6.2 Hz, 1H), 3.81 (s, 3H), 3.18 (dd, *J* = 17.3, 5.0 Hz, 1H), 2.56 (d, *J* = 17.4 Hz, 1H), 2.22 (ddd, *J* = 21.7, 12.2, 7.1 Hz, 2H), 1.91 - 1.79 (m, 1H), 1.75 (dt, *J* = 14.3, 6.8 Hz, 1H). |
| 119^{(a)} | 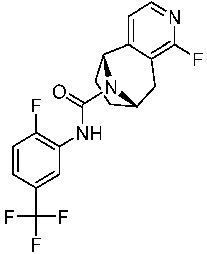 (5*R*,8*S*)-1-Fluoro-*N*-(2-fluoro-5-(trifluoromethyl)phenyl)-6,7,8,9-tetrahydro-5H-5,8-epiminocyclohepta[c]pyridine -10-carboxamide | 384.1 at 1.40 min | ¹H NMR (500 MHz, DMSO-d6) δ 8.85 (s, 1H), 8.01 (d, *J* = 5.0 Hz, 1H), 7.93 (dd, *J =* 7.1, 2.4 Hz, 1H), 7.57 - 7.30 (m, 2H), 7.20 (dd, *J* = 5.1, 1.6 Hz, 1H), 5.22 (d, *J* = 6.2 Hz, 1H), 4.80 (t, *J* = 6.2 Hz, 1H), 3.20 (dd, *J* = 17.5, 5.0 Hz, 1H), 2.59 (d, *J* = 17.3 Hz, 1H), 2.32 - 2.11 (m, 2H), 1.90 - 1.80 (m, 1H), 1.76 (dt, *J* = 14.6, 7.1 Hz, 1H). |
| 120^{(a)} | 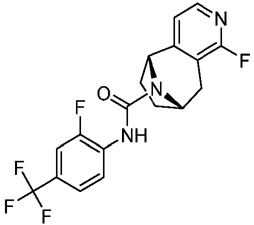 (5*R*,8*S*)-1-Fluoro-N-(2-fluoro-4-(trifluoromethyl)phenyl)-6,7,8,9-tetrahydro-5H-5,8-epiminocyclohepta[c]pyridine -10-carboxamide | 384.4 at 1.43 min | ¹H NMR (500 MHz, DMSO-d6) δ 8.91 (s, 1H), 8.01 (d, *J* = 5.0 Hz, 1H), 7.76 (t, *J* = 8.1 Hz, 1H), 7.64 (dd, *J* = 11.0, 2.1 Hz, 1H), 7.47 (d, *J =* 8.5 Hz, 1H), 7.21 (dd, *J =* 5.1, 1.6 Hz, 1H), 5.23 (d, *J* = 6.1 Hz, 1H), 4.80 (t, *J* = 6.3 Hz, 1H), 3.19 (dd, *J* = 17.2, 5.0 Hz, 1H), 2.59 (d, *J* = 17.3 Hz, 1H), 2.31 - 2.15 (m, 2H), 1.89 - 1.81 (m, 1H), 1.76 (dt, *J* = 13.2, 6.9 Hz, 1H). |
| 121^{(a)} | 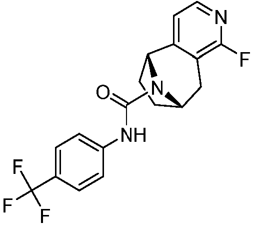 (5*R*,8*S*)-1-Fluoro-*N*-(4-(trifluoromethyl)phenyl)-6,7,8,9-tetrahydro-5H-5,8-epiminocyclohepta[c]pyridine -10-carboxamide | 364.3 at 1.41 min^{(j)} | ¹H NMR (500 MHz, DMSO-d6) δ 9.11 (s, 1H), 8.01 (d, *J* = 5.0 Hz, 1H), 7.68 (d, *J =* 8.6 Hz, 2H), 7.57 (d, *J* = 8.6 Hz, 2H), 7.21 (dd, *J* = 5.0, 1.6 Hz, 1H), 5.26 (d, *J* = 6.3 Hz, 1H), 5.02 - 4.77 (m, 1H), 3.15 (dd, *J* = 17.4, 5.0 Hz, 1H), 2.60 (d, *J* = 17.4 Hz, 1H), 2.31 - 2.13 (m, 2H), 1.89 - 1.82 (m, 1H), 1.80 - 1.71 (m, 1H). |
| 122^{(a)(b)} | 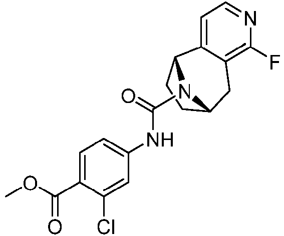 Methyl 2-chloro-4-((5*R*,8*S*)-1-fluoro-6,7,8,9-tetrahydro-5H-5,8-epiminocyclohepta[c]pyridine -10-carboxamido)benzoate | 390.1, 392.1 at 1.26 min | ¹H NMR (500 MHz, DMSO-d6) δ 9.19 (s, 1H), 8.01 (d, *J* = 5.0 Hz, 1H), 7.81 - 7.75 (m, 2H), 7.53 (dd, *J =* 8.7, 2.1 Hz, 1H), 7.21 (dd, *J* = 5.1, 1.6 Hz, 1H), 5.26 (d, *J* = 6.1 Hz, 1H), 4.83 (t, *J* = 6.4 Hz, 1H), 3.80 (s, 3H), 3.15 (dd, *J* = 17.3, 5.0 Hz, 1H), 2.60 (d, *J* = 17.3 Hz, 1H), 2.30 - 2.10 (m, 2H), 1.94 - 1.80 (m, 1H), 1.78 -1.74 (m 1H) |
| 123^{(a)(b)} | 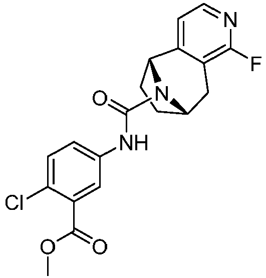 Methyl 2-chloro-5-((5*R*,8*S*)-1-fluoro-6,7,8,9-tetrahydro-5H-5,8-epiminocyclohepta[c]pyridine -10-carboxamido)benzoate | 390.1, 392.1 at 1.25 min | ¹H NMR (500 MHz, DMSO-d6) δ 9.04 (s, 1H), 7.99 (dd, *J* = 14.5, 3.8 Hz, 2H), 7.70 (dd, *J* = 8.9, 2.7 Hz, 1H), 7.42 (d, *J* = 8.8 Hz, 1H), 7.20 (dd, *J* = 5.1, 1.5 Hz, 1H), 5.24 (d, *J* = 6.3 Hz, 1H), 4.81 (t, *J* = 6.3 Hz, 1H), 3.84 (s, 3H), 3.15 (dd, *J* = 17.5, 5.0 Hz, 1H), 2.59 (d, *J* = 17.3 Hz, 1H), 2.25 - 2.16 (m ,2H), 1.88 - 1.80 (m, 1H), 1.80 - 1.71 (m, 1H). |
| 124^{(a)(b)} | 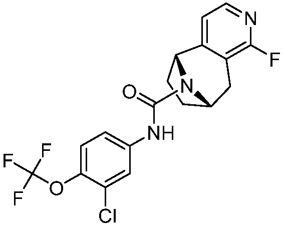 (5*R*,8*S*)-*N*-(3-Chloro-4-(trifluoromethoxy)phenyl)-1-fluoro-6,7,8,9-tetrahydro-5H-5,8-epiminocyclohepta[c]pyridine -10-carboxamide | 416.1, 418.1 at 1.53 min | ¹H NMR (500 MHz, DMSO-d6) δ 9.07 (s, 1H), 8.01 (d, *J =* 5.0 Hz, 1H), 7.84 (d, *J* = 2.5 Hz, 1H), 7.51 (dd, *J* = 9.1, 2.6 Hz, 1H), 7.43 (d, *J* = 9.1 Hz, 1H), 7.23 - 7.18 (m, 1H), 5.23 (d, *J* = 6.2 Hz, 1H), 4.81 (t, *J* = 6.3 Hz, 1H), 3.15 (dd, *J* = 17.3, 5.1 Hz, 1H), 2.59 (d, *J* = 17.3 Hz, 1H), 2.28 - 2.13 (m, 2H), 1.87 - 1.83 (m, 1H), 1.78 - 1.74 (m 1H) |
| 125^{(a)(b)} | 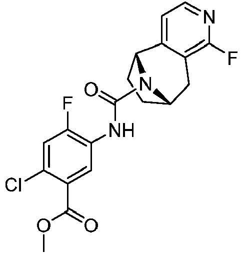 Methyl 2-chloro-4-fluoro-5-((5*R*,8*S*)-1-fluoro-6,7,8,9-tetrahydro-5H-5,8-epiminocyclohepta[c]pyridine -10-carboxamido)benzoate | 408.1, 410.1 at 1.25 min | ¹H NMR (500 MHz, DMSO-d6) δ 8.83 (s, 1H), 8.04 - 7.98 (m, 2H), 7.58 (d, *J =* 10.4 Hz, 1H), 7.19 (dd, *J* = 5.0, 1.6 Hz, 1H), 5.20 (d, *J* = 6.2 Hz, 1H), 4.81 - 4.74 (m, 1H), 3.82 (s, 3H), 3.18 (dd, *J* = 17.2, 5.0 Hz, 1H), 2.58 (d, *J* = 17.3 Hz, 1H), 2.30 - 2.17 (m, 2H), 1.88 - 1.80 (m, 1H), 1.80 1.71 (m, 1H). |
| 126^{(a)(b)} | 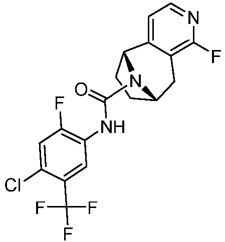 (5*R*,8*S*)-*N*-(4-Chloro-2-fluoro-5-(trifluoromethyl)phenyl)-1-fluoro-6,7,8,9-tetrahydro-5H-5,8-epiminocyclohepta[c]pyridine -10-carboxamide | 418.1, 420.1 at 1.48 min | ¹H NMR (500 MHz, DMSO-d6) δ 8.94 (s, 1H), 8.07 (d, *J* = 7.8 Hz, 1H), 8.01 (d, *J =* 5.0 Hz, 1H), 7.77 (d, *J* = 10.2 Hz, 1H), 7.20 (dd, *J* = 5.1, 1.6 Hz, 1H), 5.22 (d, *J* = 6.2 Hz, 1H), 4.82 - 4.76 (m, 1H), 3.19 (dd, *J* = 17.4, 5.0 Hz, 1H), 2.59 (d, *J* = 17.3 Hz, 1H), 2.26 - 2.18 (m, 2H), 1.89 - 1.78 (m 1H), 1.80 - 1.71 (m, 1H). |
| **127** ^{(a)(c)} | 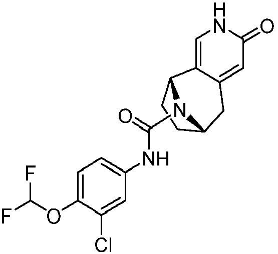 (6*S*,9*R*)-*N*-(3-Chloro-4-(difluoromethoxy)phenyl)-3-oxo-3,5,6,7,8,9-hexahydro-2H-6,9-epiminocyclohepta[c]pyridine -10-carboxamide | 396.4, 398.4 at 1.07 min | ¹H NMR (500 MHz, DMSO-d6) δ 11.30 (s, 1H), 9.24 (s, 1H), 8.64 (d, *J* = 2.7 Hz, 1H), 8.38 (d, *J* = 6.0 Hz, 1H), 7.13 (t, *J* = 73.5 Hz, 1H), 7.23 (s, 1H), 6.10 (s, 1H), 5.13 (d, *J* = 6.0 Hz, 1H), 4.66 (s, 1H), 3.17 (d, *J* = 7.2 Hz, 1H), 2.58 (d, *J =* 17.8 Hz, 1H), 2.21 - 2.13 (m, 1H), 2.08 (dt, *J =* 11.8, 5.8 Hz, 1H), 1.77 - 1.67 (m, 1H), 1.66 (s, 1H) |
| **128** ^{(a)(c)} | 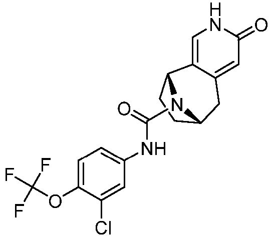 (6*S*,9*R*)-*N*-(3-Chloro-4-(trifluoromethoxy)phenyl)-3-oxo-3,5,6,7,8,9-hexahydro-2H-6,9-epiminocyclohepta[c]pyridine -10-carboxamide | 414.3, 416.3 at 1.22 min | ¹H NMR (500 MHz, DMSO-d6) δ 11.27 (s, 1H), 8.91 (s, 1H), 7.86 (dd, *J* = 2.6, 1.1 Hz, 1H), 7.55 - 7.50 (m, 1H), 7.43 (d, *J* = 9.1 Hz, 1H), 7.19 (s, 1H), 6.09 (s, 1H), 5.07 (d, *J* = 6.0 Hz, 1H), 4.60 (s, 1H), 3.10 (d, *J =* 19.4 Hz, 1H), 2.55 (d, *J =* 17.9 Hz, 1H), 2.17 (d, *J =* 10.8 Hz, 1H), 2.06 (dd, *J* = 11.9, 6.0 Hz, 1H), 1.72 (t, *J =* 10.5 Hz, 1H), 1.66 (d, *J =* 13.5 Hz, 1H) |
| 129^{(a)(c)} | 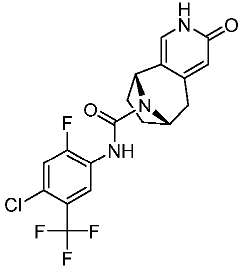 (6*S*,9*R*)-*N*-(4-Chloro-2-fluoro-5-(trifluoromethyl)phenyl)-3-oxo-3,5,6,7,8,9-hexahydro-2H-6,9-epiminocyclohepta[c]pyridine -10-carboxamide | 416.3, 418.3 at 1.19 min | ¹H NMR (500 MHz, DMSO-d6) δ 11.27 (s, 1H), 8.76 (s, 1H), 8.09 (d, *J* = 7.8 Hz, 1H), 7.77 (d, *J =* 10.2 Hz, 1H), 7.19 (s, 1H), 6.10 (s, 1H), 5.05 (d, *J* = 6.0 Hz, 1H), 4.59 (t, *J* = 6.5 Hz, 1H), 3.13 (dd, *J* = 17.8, 5.1 Hz, 1H), 2.55 (d, *J* = 17.8 Hz, 1H), 2.18 (q, *J =* 11.2 Hz, 1H), 2.10 - 2.03 (m, 1H), 1.76 - 1.60 (m, 2H) |
| 130^{(a)(c)} | 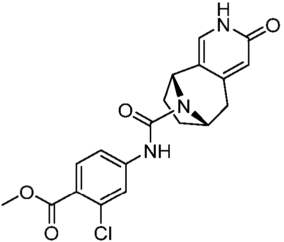 Methyl 2-chloro-4-((6*S*,9*R*)-3-oxo-3,5,6,7,8,9-hexahydro-2H-6,9-epiminocyclohepta[c]pyridine -10-carboxamido)benzoate | 388.3, 390.3 at 0.96 min | ¹H NMR (500 MHz, DMSO-d6) δ 11.28 (s, 1H), 9.03 (s, 1H), 7.84 - 7.74 (m, 2H), 7.55 (dd, *J* = 8.7, 2.1 Hz, 1H), 7.20 (s, 1H), 6.10 (s, 1H), 5.09 (d, *J* = 6.0 Hz, 1H), 4.65 - 4.59 (m, 1H), 3.80 (s, 3H), 3.10 (dd, *J* = 17.8, 5.1 Hz, 1H), 2.57 (d, *J =* 17.9 Hz, 1H), 2.17 (q, *J =* 10.7 Hz, 1H), 2.06 (tt, *J* = 11.8, 6.1 Hz, 1H), 1.72 (t, *J =* 10.5 Hz, 1H), 1.69 - 1.60 (m, 1H). |
| 131 ^{(a)(c)} | 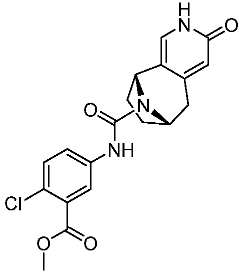 Methyl 2-chloro-5-((6S,9R)-3-oxo-3,5,6,7,8,9-hexahydro-2H-6,9-epiminocyclohepta[c]pyridine -10-carboxamido)benzoate | 388.4, 390.4 at 0.97 min | ¹H NMR (500 MHz, DMSO-d6) δ 11.27 (s, 1H), 8.88 (s, 1H), 7.99 (d, *J* = 2.7 Hz, 1H), 7.71 (dd, *J* = 8.8, 2.7 Hz, 1H), 7.42 (d, *J =* 8.8 Hz, 1H), 7.18 (s, 1H), 6.09 (s, 1H), 5.07 (d, *J = 6.0* Hz, 1H), 4.63 - 4.57 (m, 1H), 3.84 (s, 3H), 3.10 (dd, *J* = 17.9, 5.1 Hz, 1H), 2.55 (d, *J =* 17.9 Hz, 1H), 2.17 (q, *J =* 10.9 Hz, 1H), 2.10 - 2.00 (m, 1H), 1.75 - 1.60 (m, 2H). |
| 132^{(a)(c)} | 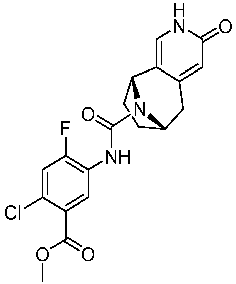 Methyl 2-chloro-4-fluoro-5-((6S,9*R*)-3-oxo-3,5,6,7,8,9-hexahydro-2H-6,9-epiminocyclohepta[c]pyridine -10-carboxamido)benzoate | 406.4, 408.4 at 0.74 min | ¹H NMR (500 MHz, DMSO-d6) δ 11.27 (s, 1H), 8.65 (s, 1H), 8.04 (d, *J* = 8.4 Hz, 1H), 7.58 (d, *J* = 10.4 Hz, 1H), 7.18 (s, 1H), 6.10 (s, 1H), 5.03 (d, *J* = 5.9 Hz, 1H), 4.60 - 4.54 (m, 1H), 3.83 (s, 3H), 3.12 (dd, *J* = 17.7, 5.0 Hz, 1H), 2.55 (d, *J* = 17.8 Hz, 1H), 2.17 (q, *J =* 10.6 Hz, 1H), 2.11-2.03 (m, 1H), 1.74 - 1.61 (m, 2H) |
| 136^{(a)(b)} | 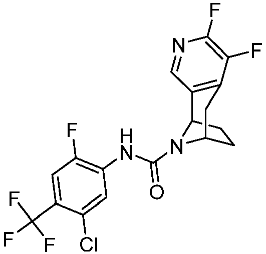 (±)-*N*-(5-Chloro-2-fl uoro-4-(trifluoromethyl)phenyl)-3,4-difluoro-6,7,8,9-tetrahydro-5H-6,9-epiminocyclohepta[c]pyridine -10-carboxamide | 434.3, 436.3 at 1.77 min (d) | ¹H NMR (500 MHz, DMSO-d6) δ 9.09 (s, 1H), 8.02 (d, *J* = 6.8 Hz, 1H), 7.92 (s, 1H), 7.79 (d, *J =* 10.9 Hz, 1H), 5.36 (d, *J* = 5.6 Hz, 1H), 4.79 (t, *J =* 6.0 Hz, 1H), 3.28 (d, *J = 5.0* Hz, 1H), 2.83 (d, *J* = 18.1 Hz, 1H), 2.29 - 2.14 (m, 2H), 1.85 (t, *J =* 9.9 Hz, 1H), 1.75 (dd, *J* = 12.4, 5.8 Hz, 1H). |
| 137^{(a)(b)} | 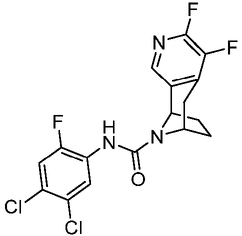 (±)-*N*-(4,5-Dichloro-2-fluorophenyl)-3,4-difluoro-6,7,8,9-tetrahydro-5H-6,9-epiminocyclohepta[c]pyridine -10-carboxamide | 400.3, 402.3 at 1.66 min (d) | ¹H NMR (500 MHz, DMSO-d6) δ 8.83 (s, 1H), 7.90 (s, 1H), 7.82 (d, *J* = 7.5 Hz, 1H), 7.67 (d, *J =* 10.3 Hz, 1H), 5.32 (d, *J =* 5.7 Hz, 1H), 4.74 (t, *J* = 6.0 Hz, 1H), 3.26 (d, *J =* 5.0 Hz, 1H), 2.83 (s, 1H), 2.23 (d, *J =* 12.3 Hz, 1H), 2.18 (td, *J* = 11.6, 6.0 Hz, 1H), 1.83 (t, *J* = 10.1 Hz, 1H), 1.75 (dd, *J* = 12.4, 5.7 Hz, 1H) |
| 138^{(a)(c)} | 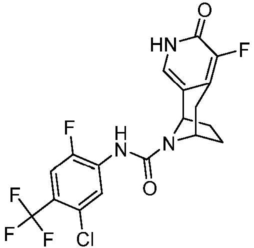 (±)-*N*-(5-Chloro-2-fl uoro-4-(trifluoromethyl)phenyl)-4-fluoro-3-oxo-3,5,6,7,8,9-hexahydro-2H-6,9-epiminocyclohepta[c]pyridine -10-carboxamide | 434.3, 436.3 at 1.28 min | ¹H NMR (500 MHz, DMSO-d6) δ 11.84 (s, 1H), 8.94 (s, 1H), 8.04 (d, *J* = 6.9 Hz, 1H), 7.78 (d, *J =* 11.0 Hz, 1H), 7.13 (s, 1H), 5.13 (d, *J =* 6.1 Hz, 1H), 4.68 (t, *J* = 6.3 Hz, 1H), 3.10 (d, *J =* 18.7 Hz, 1H), 2.66 (d, *J =* 18.2 Hz, 1H), 2.18 (q, *J =* 11.1, 10.6 Hz, 1H), 2.11 (dt, *J =* 11.9, 5.8 Hz, 1H), 1.78 - 1.67 (m, 2H) |
| 139 | 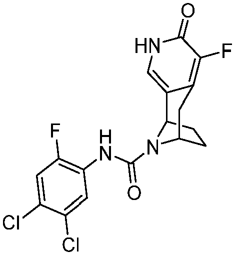 (±)-*N*-(4,5-Dichloro-2-fluorophenyl)-4-fluoro-3-oxo-3,5,6,7,8,9-hexahydro-2H-6,9-epiminocyclohepta[c]pyridine -10-carboxamide | 400.2, 402.2 at 1.11 min | ¹H NMR (500 MHz, DMSO-d6) δ 11.83 (s, 1H), 8.67 (s, 1H), 7.83 (d, *J* = 7.6 Hz, 1H), 7.67 (d, *J =* 10.3 Hz, 1H), 7.10 (s, 1H), 5.09 (d, *J* = 6.0 Hz, 1H), 4.67 - 4.60 (m, 1H), 3.09 (d, *J =* 14.6 Hz, 1H), 2.64 (d, *J =* 17.8 Hz, 1H), 2.18 (q, *J =* 11.9, 11.3 Hz, 1H), 2.09 (dd, *J* = 11.7, 6.0 Hz, 1H), 1.73 (q, *J =* 11.8, 11.1 Hz, 2H). |
| 141(a)(c) | 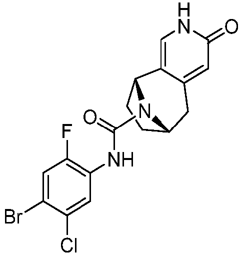 (6*S*,9*R*)-*N*-(4-Bromo-5-chloro-2-fluorophenyl)-3-oxo-3,5,6,7,8,9-hexahydro-2H-6,9-epiminocyclohepta[c]pyridine -10-carboxamide | 426.1, 428.1 at 1.15 min | ¹H NMR (500 MHz, DMSO-d6) δ 11.26 (s, 1H), 8.62 (s, 1H), 7.84 (d, *J* = 7.5 Hz, 1H), 7.75 (d, *J =* 10.1 Hz, 1H), 7.19 (s, 1H), 6.10 (s, 1H), 5.04 (d, *J* = 6.0 Hz, 1H), 4.57 (t, *J* = 6.5 Hz, 1H), 3.12 (dd, *J* = 17.9, 5.1 Hz, 1H), 2.54 (d, *J* = 18.0 Hz, 1H), 2.16 (q, *J* = 11.0 Hz, 1H), 2.06 (tt, *J =* 11.6, 6.2 Hz, 1H), 1.68 (ddd, *J* = 28.8, 11.9, 7.5 Hz, 2H) |
| 142^{(a)(c)} | 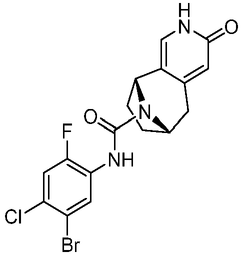 (6S,9R)-*N*-(5-Bromo-4-chloro-2-fluorophenyl)-3-oxo-3,5,6,7,8,9-hexahydro-2H-6,9-epiminocyclohepta[c]pyridine -10-carboxamide | 426.1, 428.1 at 1.15 min | ¹H NMR (500 MHz, DMSO-d6) δ 11.26 (s, 1H), 8.61 (s, 1H), 7.94 (d, *J* = 7.8 Hz, 1H), 7.66 (d, *J* = 10.4 Hz, 1H), 7.19 (s, 1H), 6.10 (s, 1H), 5.03 (d, *J* = 6.0 Hz, 1H), 4.74 - 4.35 (m, 1H), 3.12 (dd, *J =* 18.0, 5.1 Hz, 1H), 2.54 (d, *J* = 18.0 Hz, 1H), 2.17 (q, *J =* 11.1 Hz, 1H), 2.06 (tt, *J =* 11.6, 6.2 Hz, 1H), 1.75 - 1.58 (m, 2H) |
| 143^{(a)(c)} | 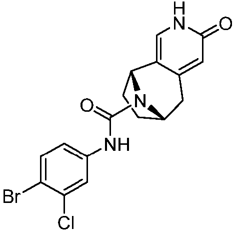 (6S,9R)-N-(4-Bromo-3-chlorophenyl)-3-oxo-3,5,6,7,8,9-hexahydro-2H-6,9-epiminocyclohepta[c]pyridine -10-carboxamide | 408.1, 410.1 at 1.14 min | ¹H NMR (500 MHz, DMSO-d6) δ 8.86 (s, 1H), 7.86 (d, *J* = 2.5 Hz, 1H), 7.59 (d, *J =* 8.8 Hz, 1H), 7.40 (dd, *J* = 8.9, 2.5 Hz, 1H), 7.20 (s, 1H), 6.09 (s, 1H), 5.08 (d, *J* = 6.0 Hz, 1H), 4.60 (t, *J* = 6.6 Hz, 1H), 3.09 (dd, *J* = 17.9, 5.1 Hz, 1H), 2.55 (d, *J =* 17.9 Hz, 1H), 2.16 (q, *J =* 11.0 Hz, 1H), 2.05 (tt, *J =* 11.6, 6.1 Hz, 1H), 1.69 (tdd, *J* = 28.1, 13.3, 8.6 Hz, 2H), 1 NH not visible |
| 144^{(a)(c)} | 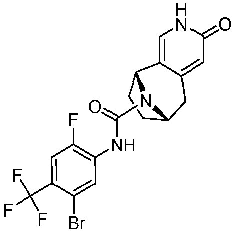 (6S,9R)-*N*-(5-Bromo-2-fluoro-4-(trifluoromethyl)phenyl)-3-oxo-3,5,6,7,8,9-hexahydro-2H-6,9-epiminocyclohepta[c]pyridine -10-carboxamide | 460.1, 462.1 at 1.23 min | ¹H NMR (500 MHz, DMSO-d6) δ 11.28 (s, 1H), 8.88 (s, 1H), 8.19 (d, *J =* 7.1 Hz, 1H), 7.76 (d, *J =* 11.1 Hz, 1H), 7.21 (s, 1H), 6.10 (s, 1H), 5.08 (d, *J* = 6.0 Hz, 1H), 4.68 - 4.34 (m, 1H), 3.14 (dd, *J =* 17.9, 5.1 Hz, 1H), 2.56 (d, *J* = 17.9 Hz, 1H), 2.23 - 2.01 (m, 2H), 1.78 - 1.59 (m, 2H). |
| 145^{(a)(c)} | 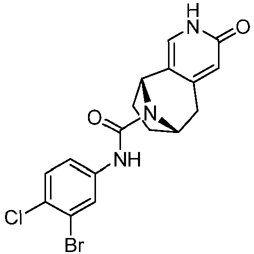 (6*S*,9*R*)-*N*-(3-Bromo-4-chlorophenyl)-3-oxo-3,5,6,7,8,9-hexahydro-2H-6,9-epiminocyclohepta[c]pyridine -10-carboxamide | 408.2, 410.1 at 1.13 min | ¹H NMR (500 MHz, DMSO-d6) δ 8.81 (s, 1H), 7.97 (d, *J* = 2.4 Hz, 1H), 7.51 (dd, *J =* 8.9, 2.5 Hz, 1H), 7.46 (d, *J* = 8.8 Hz, 1H), 7.19 (s, 1H), 6.09 (s, 1H), 5.06 (d, *J = 6.0* Hz, 1H), 4.67 - 4.46 (m, 1H), 3.09 (dd, *J* = 17.9, 5.2 Hz, 1H), 2.55 (d, *J =* 17.9 Hz, 1H), 2.16 (q, *J =* 11.1 Hz, 1H), 2.05 (tt, *J* = 11.6, 6.1 Hz, 1H), 1.68 (ddd, *J* = 29.8, 11.8, 7.4 Hz, 2H), 1 NH not visible. |
| 151^{(a)(b)} | 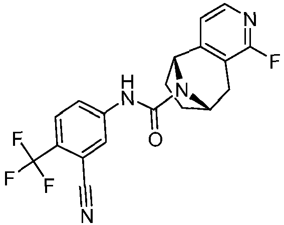 (5*R*,8*S*)-*N*-(3-cyano-4-(trifluoromethyl)phenyl)-1-fluoro-6,7,8,9-tetrahydro-5H-5,8-epiminocyclohepta[c]pyridine -10-carboxamide | 389.2 at 1.49 min^{(d)} | ¹H NMR (400 MHz, DMSO-d6) δ 9.43 (s, 1H), 8.21 (d, *J* = 2.2 Hz, 1H), 8.02 (d, *J* = 5.0 Hz, 1H), 7.98 - 7.91 (m, 1H), 7.87 (d, *J =* 8.9 Hz, 1H), 7.22 (dd, *J* = 5.0, 1.6 Hz, 1H), 5.27 (d, *J* = 6.1 Hz, 1H), 4.85 (t, *J* = 6.1 Hz, 1H), 3.17 (dd, *J* = 17.5, 5.0 Hz, 1H), 2.62 (d, *J* = 17.3 Hz, 1H), 2.28 - 2.16 (m, 2H), 1.92 - 1.83 (m, 1H), 1.83 - 1.72 (m, 1H). |
| 152 | 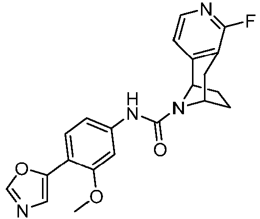 (±)-1-Fluoro-*N*-(3-methoxy-4-(oxazol-5-yl)phenyl)-6,7,8,9-tetrahydro-5H-5,8-epiminocyclohepta[c]pyridine -10-carboxamide | 393.1 at 1.12 min^{(d)} | ¹H NMR (400 MHz, CDCl₃) δ 8.38 (s, 1H), 8.15 - 7.93 (m, 2H), 7.64 - 7.41 (m, 3H), 7.02 (t, *J* = 6.4 Hz, 1H), 6.83 (dd, *J* = 8.4, 1.9 Hz, 1H), 5.12 (dd, *J* = 23.1, 6.3 Hz, 1H), 4.70 (dt, *J* = 13.7, 6.6 Hz, 1H), 3.94 (d, *J* = 6.9 Hz, 3H), 3.47 - 3.28 (m, 1H), 2.73 - 2.54 (m, 3H), 2.02 (q, *J =* 10.6 Hz, 1H), 1.85 (ddd, *J* = 21.5, 14.1, 8.4 Hz, 2H). |
| 153 | 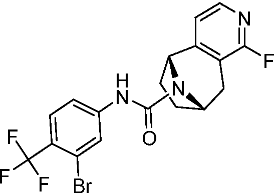 (5*R*,8*S*)-N-(3-Bromo-4-(trifluoromethyl)phenyl)-1-fluoro-6,7,8,9-tetrahydro-5H-5,8-epiminocyclohepta[c]pyridine -10-carboxamide | 442.0, 444.0 at 1.64 min^{(d)} | ¹H NMR (400 MHz, DMSO-d6) δ 9.23 (s, 1H), 8.06 (d, *J* = 1.9 Hz, 1H), 8.01 (d, *J = 5.0* Hz, 1H), 7.69 (d, *J* = 8.8 Hz, 1H), 7.66 (dd, *J* = 8.8, 1.9 Hz, 1H), 7.21 (dd, *J* = 5.0, 1.7 Hz, 1H), 5.26 (d, *J = 6.1* Hz, 1H), 4.90 - 4.77 (m, 1H), 3.16 (dd, *J =* 17.3, 5.0 Hz, 1H), 2.61 (d, *J* = 17.4 Hz, 1H), 2.34 - 2.10 (m, 2H), 1.91 - 1.81 (m, 1H), 1.81 - 1.66 (m, 1H). |
| 154 | 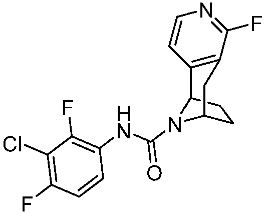 (±)-*N*-(3-Chloro-2,4-difluorophenyl)-1-fluoro-6,7,8,9-tetrahydro-5H-5,8-epiminocyclohepta[c]pyridine -10-carboxamide | 366.0 at 1.26 min^{(d)} | ¹H NMR (400 MHz, DMSO-d6) δ 8.78 (s, 1H), 8.00 (d, *J* = 5.0 Hz, 1H), 7.38 (td, *J =* 8.7, 5.9 Hz, 1H), 7.23 (td, *J* = 9.0, 2.1 Hz, 1H), 7.19 (dd, *J* = 5.1, 1.8 Hz, 1H), 5.17 (d, *J =* 6.0 Hz, 1H), 4.75 (d, *J* = 6.1 Hz, 1H), 3.23 - 3.14 (m, 1H), 2.58 (d, *J* = 17.3 Hz, 1H), 2.26 (d, *J =* 11.7 Hz, 1H), 2.20 (dd, *J =* 12.2, 6.4 Hz, 1H), 1.89 - 1.77 (m, 1H), 1.75 (s, 1H). |
| 155 | 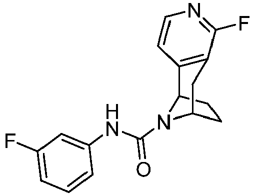 (±)-1-Fluoro-*N*-(3-fluorophenyl)-6,7,8,9-tetrahydro-5H-5,8-epiminocyclohepta[c]pyridine -10-carboxamide | 316.3 at 1.17 min | ¹H NMR (400 MHz, DMSO-d6) δ 8.92 (s, 1H), 8.01 (d, *J* = 5.0 Hz, 1H), 7.42 (dt, *J =* 11.7, 1.8 Hz, 1H), 7.30 - 7.17 (m, 3H), 6.79 - 6.69 (m, 1H), 5.24 (d, *J =* 6.1 Hz, 1H), 4.81 (t, *J = 6.1* Hz, 1H), 3.14 (dd, *J* = 18.1, 5.2 Hz, 1H), 2.58 (d, *J* = 17.3 Hz, 1H), 2.31 - 2.21 (m, 1H), 2.17 (td, *J* = 12.0, 11.5, 6.5 Hz, 1H), 1.89 - 1.80 (m, 1H), 1.80 - 1.70 (m, 1H). |
| 156 | 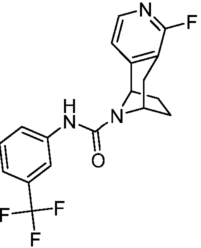 (±)-1-Fluoro-*N*-(3-(trifluoromethyl)phenyl)-6,7,8,9-tetrahydro-5H-5,8-epiminocyclohepta[c]pyridine -10-carboxamide | 366.3 at 1.36 min | ¹H NMR (400 MHz, DMSO-d6) δ 9.05 (s, 1H), 8.01 (d, *J* = 5.0 Hz, 1H), 7.94 - 7.88 (m, 1H), 7.77 - 7.70 (m, 1H), 7.46 (t, *J* = 8.0 Hz, 1H), 7.27 (d, *J* = 7.7 Hz, 1H), 7.21 (dd, *J =* 5.1, 1.7 Hz, 1H), 5.25 (d, *J* = 6.1 Hz, 1H), 4.87 - 4.79 (m, 1H), 3.16 (dd, *J* = 17.4, 5.0 Hz, 1H), 2.60 (d, *J* = 17.3 Hz, 1H), 2.21 (ddt, *J* = 17.8, 11.5, 8.7 Hz, 2H), 1.90 - 1.81 (m, 1H), 1.76 (dt, *J =* 14.1, 6.7 Hz, 1H). |
| 157 | 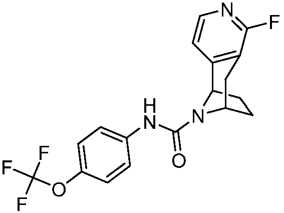 (±)-1-Fluoro-*N*-(4-(trifluoromethoxy)phenyl)-6,7,8,9-tetrahydro-5H-5,8-epiminocyclohepta[c]pyridine -10-carboxamide | 382.3 at 1.39 min | ¹H NMR (400 MHz, DMSO-d6) δ 8.93 (s, 1H), 8.00 (d, *J* = 5.0 Hz, 1H), 7.59 - 7.51 (m, 2H), 7.26 - 7.17 (m, 3H), 5.23 (d, *J* = 6.0 Hz, 1H), 4.84 - 4.77 (m, 1H), 3.15 (dd, *J =* 17.4, 5.0 Hz, 1H), 2.58 (d, *J* = 17.4 Hz, 1H), 2.20 (ddd, *J =* 17.3, 14.8, 8.5 Hz, 2H), 1.89 - 1.80 (m, 1H), 1.80 - 1.70 (m, 1H). |
| 158 | 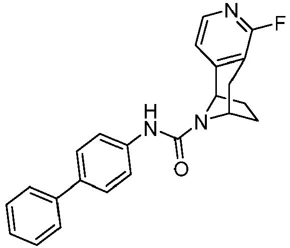 (±)-*N*-([1,1'-Biphenyl]-4-yl)-1-fluoro-6,7,8,9-tetrahydro-5H-5,8-epiminocyclohepta[c]pyridine -10-carboxamide | 374.3 at 1.43 min | ¹H NMR (400 MHz, DMSO-d6) δ 8.84 (s, 1H), 8.01 (d, *J* = 5.0 Hz, 1H), 7.64 - 7.58 (m, 2H), 7.54 (s, 4H), 7.42 (dd, *J* = 8.4, 7.0 Hz, 2H), 7.33 - 7.25 (m, 1H), 7.21 (dd, *J =* 5.0, 1.7 Hz, 1H), 5.26 (d, *J* = 6.0 Hz, 1H), 4.83 (t, *J* = 6.1 Hz, 1H), 3.17 (dd, *J* = 17.5, 5.0 Hz, 1H), 2.59 (d, *J* = 17.4 Hz, 1H), 2.30 - 2.09 (m, 2H), 1.92 - 1.80 (m, 1H), 1.80 - 1.67 (m, 1H). |
| 159 | 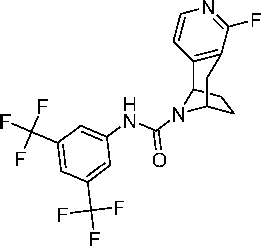 (±)-*N*-(3,5-Bis(trifluoromethyl)phenyl)-1-fluoro-6,7,8,9-tetrahydro-5H-5,8-epiminocyclohepta[c]pyridine -10-carboxamide | 432.2 at 1.61 min^{(d)} | ¹H NMR (400 MHz, DMSO-d6) δ 9.35 (s, 1H), 8.25 - 8.16 (m, 2H), 8.02 (d, *J =* 5.0 Hz, 1H), 7.60 (s, 1H), 7.22 (dd, *J* = 5.0, 1.6 Hz, 1H), 5.27 (d, *J* = 6.1 Hz, 1H), 4.91 - 4.79 (m, 1H), 3.19 (dd, *J =* 17.5, 5.0 Hz, 1H), 2.62 (d, *J* = 17.4 Hz, 1H), 2.35 - 2.13 (m, 2H), 1.93 - 1.83 (m, 1H), 1.78 (dt, *J* = 14.2, 6.8 Hz, 1H). |
| 160 | 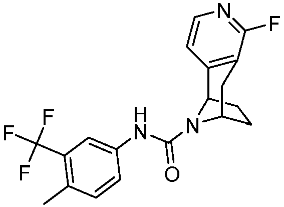 (±)-1-Fluoro-*N*-(4-methyl-3-(trifluoromethyl)phenyl)-6,7,8,9-tetrahydro-5H-5,8-epiminocyclohepta[c]pyridine -10-carboxamide | 380.2 at 1.44 min | ¹H NMR (400 MHz, DMSO-d6) δ 8.93 (s, 1H), 8.01 (d, *J* = 5.0 Hz, 1H), 7.84 (d, *J =* 2.3 Hz, 1H), 7.63 (dd, J = 8.4, 2.3 Hz, 1H), 7.28 (d, *J* = 8.4 Hz, 1H), 7.20 (dd, *J* = 5.0, 1.7 Hz, 1H), 5.24 (d, *J* = 6.1 Hz, 1H), 4.81 (t, *J = 6.1* Hz, 1H), 3.15 (dd, *J* = 17.4, 5.0 Hz, 1H), 2.58 (d, *J* = 17.3 Hz, 1H), 2.33 (t, *J* = 2.0 Hz, 3H), 2.29 - 2.09 (m, 2H), 1.90 - 1.80 (m, 1H), 1.75 (dt, *J* = 13.9, 6.7 Hz, 1H). |
| 161 | 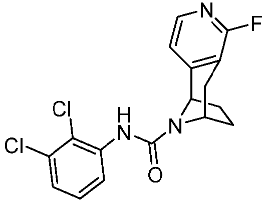 (±)-*N*-(2,3-Dichlorophenyl)-1-fluoro-6,7,8,9-tetrahydro-5H-5,8-epiminocyclohepta[c]pyridine -10-carboxamide | 366.3, 368.2 at 1.35 min | ¹H NMR (400 MHz, DMSO-d6) δ 8.69 (s, 1H), 8.00 (d, *J* = 5.0 Hz, 1H), 7.44 - 7.37 (m, 2H), 7.32 - 7.24 (m, 1H), 7.20 (dd, *J* = 5.0, 1.7 Hz, 1H), 5.18 (d, *J* = 5.9 Hz, 1H), 4.75 (t, *J = 6.1* Hz, 1H), 3.24 (dd, *J* = 17.4, 5.1 Hz, 1H), 2.58 (d, *J* = 17.4 Hz, 1H), 2.31 - 2.13 (m, 2H), 1.90 - 1.81 (m, 1H), 1.81 - 1.70 (m, 1H). |
| 162 | 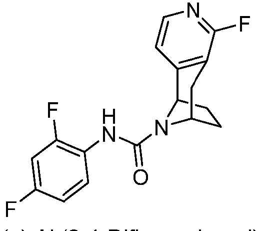 (±)-*N*-(2,4-Ditluorophenyl)-1-fluoro-6,7,8,9-tetrahydro-5H-5,8-epiminocyclohepta[c]pyridine -10-carboxamide | 334.2 at 1.11 min | ¹H NMR (400 MHz, DMSO-d6) δ 8.56 (s, 1H), 8.00 (d, *J* = 5.0 Hz, 1H), 7.37 (td, *J =* 9.0, 6.2 Hz, 1H), 7.26 - 7.14 (m, 2H), 6.99 (tdd, *J* = 8.6, 2.9, 1.4 Hz, 1H), 5.17 (d, *J* = 6.0 Hz, 1H), 4.77 - 4.66 (m, 1H), 3.18 (dd, *J* = 17.4, 5.0 Hz, 1H), 2.56 (d, *J* = 17.3 Hz, 1H), 2.20 (dtd, *J* = 17.6, 11.9 7.1 Hz, 2H), 1.91 - 1.79 (m, 1H), 1.79 - 1.63 (m, 1H). |
| 163 | 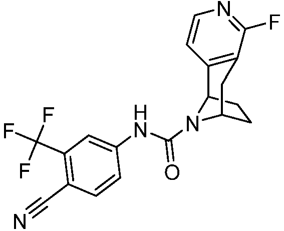 (±)-*N*-(4-Cyano-3-(trifluoromethyl)phenyl)-1-fluoro-6,7,8,9-tetrahydro-5H-5,8-epiminocyclohepta[c]pyridine -10-carboxamide | 389.2 at 1.33 min^{(d)} | ¹H NMR (400 MHz, DMSO-d6) δ 8.03 (d, *J* = 5.0 Hz, 1H), 7.88 (d, *J =* 2.1 Hz, 1H), 7.78 (dd, *J* = 8.6, 2.2 Hz, 1H), 7.70 (d, *J* = 8.6 Hz, 1H), 7.08 - 6.94 (m, 2H), 5.12 (d, *J =* 6.4 Hz, 1H), 4.71 (dd, *J =* 7.0, 5.5 Hz, 1H), 3.31 (dd, *J* = 17.5, 4.9 Hz, 1H), 2.71 - 2.61 (m, 1H), 2.51 - 2.39 (m, 1H), 2.34 (dq, *J =* 11.9, 5.8 Hz, 1H), 2.01 (ddd, *J =* 12.2, 9.2, 2.5 Hz, 1H), 1.84 (ddd, *J* = 12.4, 9.0, 6.2 Hz, 1H). |
| 164 | 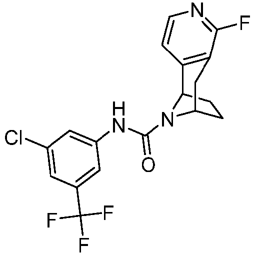 (±)-*N*-(3-Chloro-5-(trifluoromethyl)phenyl)-1-fluoro-6,7,8,9-tetrahydro-5H-5,8-epiminocyclohepta[c]pyridine -10-carboxamide | 398.2 at 1.55 min^{(d)} | ¹H NMR (400 MHz, DMSO-d6) δ 9.19 (s, 1H), 8.02 (d, *J* = 5.0 Hz, 1H), 7.98 - 7.80 (m, 2H), 7.37 (d, *J* = 1.9 Hz, 1H), 7.21 (dd, *J* = 5.0, 1.6 Hz, 1H), 5.25 (d, *J* = 6.2 Hz, 1H), 4.83 (t, *J = 6.2* Hz, 1H), 3.17 (dd, *J* = 17.5, 5.1 Hz, 1H), 2.61 (d, *J* = 17.3 Hz, 1H), 2.32 - 2.08 (m, 2H), 1.95 - 1.81 (m, 1H), 1.77 (dt, *J* = 14.0, 6.7 Hz, 1H). |
| 165 | 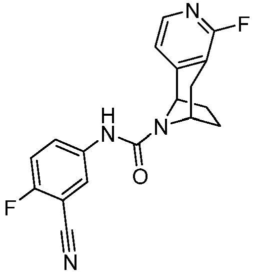 (±)-*N*-(3-Cyano-4-fluorophenyl)-1-fluoro-6,7,8,9-tetrahydro-5H-5,8-epiminocyclohepta[c]pyridine -10-carboxamide | 339.2 at 1.15 min^{(d)} | ¹H NMR (400 MHz, DMSO-d6) δ 9.06 (s, 1H), 8.01 (d, *J* = 5.0 Hz, 1H), 7.94 (dd, *J =* 5.8, 2.8 Hz, 1H), 7.76 (ddd, *J* = 9.3, 4.9, 2.8 Hz, 1H), 7.41 (t, *J =* 9.2 Hz, 1H), 7.21 (dd, *J* = 5.1, 1.7 Hz, 1H), 5.23 (d, *J* = 6.1 Hz, 1H), 4.90 - 4.68 (m, 1H), 3.16 (dd, *J* = 17.5, 5.0 Hz, 1H), 2.59 (d, *J* = 17.4 Hz, 1H), 2.31 - 2.02 (m, 2H), 1.92 - 1.79 (m, 1H), 1.79 - 1.62 (m, 1H). |
| 166 | 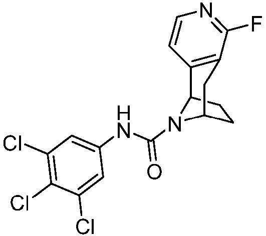 (±)-1-Fluoro-*N*-(3,4,5-trichlorophenyl)-6,7,8,9-tetrahydro-5H-5,8-epiminocyclohepta[c]pyridine -10-carboxamide | 398.1, 400.2, 402.2 at 1.58 min^{(d)} | ¹H NMR (400 MHz, DMSO-d6) δ 9.09 (s, 1H), 8.01 (d, *J* = 5.0 Hz, 1H), 7.80 (s, 2H), 7.20 (dd, *J* = 5.1, 1.6 Hz, 1H), 5.23 (d, *J* = 6.1 Hz, 1H), 4.80 (t, *J* = 6.2 Hz, 1H), 3.15 (dd, *J* = 17.0, 5.0 Hz, 1H), 2.60 (d, *J* = 17.4 Hz, 1H), 2.29 - 2.03 (m, 2H), 1.93 - 1.81 (m, 1H), 1.76 (dt, *J* = 14.2, 6.7 Hz, 1H). |
| 167 | 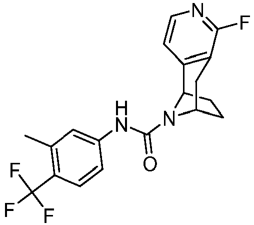 (±)-1-Fluoro-*N*-(3-methyl-4-(trifluoromethyl)phenyl)-6,7,8,9-tetrahydro-5H-5,8-epiminocyclohepta[c]pyridine -10-carboxamide | 378.3 at 1.45 min^{(d)} | ¹H NMR (400 MHz, DMSO-d6) δ 9.02 (s, 1H), 8.01 (d, *J* = 4.7 Hz, 1H), 7.58 - 7.39 (m, 3H), 7.20 (dd, *J =* 5.1, 1.7 Hz, 1H), 5.25 (d, *J* = 6.1 Hz, 1H), 4.82 (t, *J* = 6.2 Hz, 1H), 3.23 - 3.02 (m, 1H), 2.65 - 2.52 (m, 1H), 2.35 (d, *J* = 1.8 Hz, 3H), 2.21 (ddt, *J* = 17.3, 11.7, 6.6 Hz, 2H), 1.93 - 1.80 (m, 1H), 1.76 (dt, *J =* 13.6, 6.7 Hz, 1H). |
| 168 | 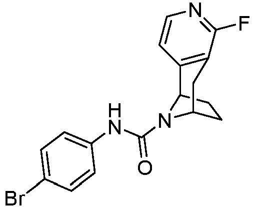 (±)-*N*-(4-Bromophenyl)-1-fluoro-6,7,8,9-tetrahydro-5H-5,8-epiminocyclohepta[c]pyridine -10-carboxamide | 376.1, 378.2 at 1.29 min | ¹H NMR (400 MHz, DMSO-d6) δ 8.85 (s, 1H), 8.00 (d, *J* = 5.0 Hz, 1H), 7.46 - 7.41 (m, 2H), 7.38 (d, *J* = 9.0 Hz, 2H), 7.20 (dd, *J* = 5.0, 1.7 Hz, 1H), 5.23 (d, *J* = 6.0 Hz, 1H), 4.79 (t, *J* = 6.2 Hz, 1H), 3.14 (dd, *J* = 20.3, 5.1 Hz, 1H), 2.57 (d, *J* = 17.4 Hz, 1H), 2.27 - 2.06 (m, 2H), 1.91 - 1.78 (m, 1H), 1.78 - 1.65 (m, 1H). |
| 169 | 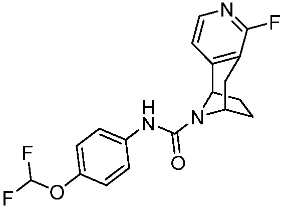 (±)-*N*-(4-(Difluoromethoxy)phenyl)-1-fluoro-6,7,8,9-tetrahydro-5H-5,8-epiminocyclohepta[c]pyridine -10-carboxamide | 364.2 at 1.21 min | ¹H NMR (400 MHz, DMSO-d6) δ 8.80 (s, 1H), 8.00 (d, *J* = 5.0 Hz, 1H), 7.50 - 7.43 (m, 2H), 7.19 (dd, *J* = 5.0, 1.7 Hz, 1H), 7.08 (d, *J* = 74.7 Hz, 1H), 7.08 - 7.01 (m, 2H), 5.22 (d, *J* = 6.0 Hz, 1H), 4.79 (t, *J* = 6.1 Hz, 1H), 3.15 (dd, *J* = 17.4, 5.0 Hz, 1H), 2.59 (s, 1H), 2.31 - 2.11 (m, 2H), 1.88 - 1.79 (m, 1H), 1.79 - 1.69 (m, 1H). |
| 170 | 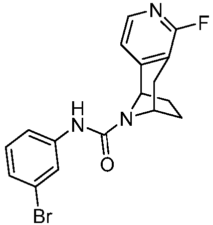 (±)-*N*-(3-Bromophenyl)-1-fluoro-6,7,8,9-tetrahydro-5H-5,8-epiminocyclohepta[c]pyridine -10-carboxamide | 376.2, 378.2 at 1.30 min | ¹H NMR (400 MHz, DMSO-d6) δ 8.88 (s, 1H), 8.01 (d, *J* = 5.0 Hz, 1H), 7.77 (t, *J =* 2.0 Hz, 1H), 7.44 (ddd, *J* = 8.2, 2.1, 1.1 Hz, 1H), 7.22 - 7.18 (m, 1H), 7.17 (d, *J =* 8.1 Hz, 1H), 7.11 (ddd, *J* = 7.9, 2.0, 1.1 Hz, 1H), 5.23 (d, *J =* 6.0 Hz, 1H), 4.86 - 4.69 (m, 1H), 3.14 (dd, *J* = 17.4, 5.1 Hz, 1H), 2.58 (d, *J =* 17.4 Hz, 1H), 2.31 - 2.12 (m, 2H), 1.90 - 1.80 (m, 1H), 1.75 (ddd, *J* = 13.6, 9.5, 4.8 Hz, 1H). |
| 171 | 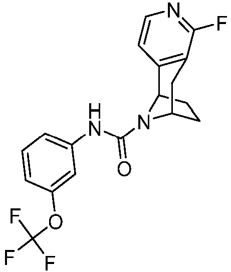 (±)-1-Fluoro-*N*-(3-(trifluoromethoxy)phenyl)-6,7,8,9-tetrahydro-5H-5,8-epiminocyclohepta[c]pyridine -10-carboxamide | 382.3 at 1.40 min | ¹H NMR (400 MHz, DMSO-d6) δ 8.99 (s, 1H), 8.01 (d, *J* = 5.0 Hz, 1H), 7.61 (s, 1H), 7.49 - 7.39 (m, 1H), 7.34 (t, *J* = 8.2 Hz, 1H), 7.21 (dd, *J =* 4.9, 1.7 Hz, 1H), 6.90 (ddt, *J* = 8.1, 2.3, 1.0 Hz, 1H), 5.25 (d, *J = 6.0* Hz, 1H), 4.82 (t, *J* = 6.1 Hz, 1H), 3.15 (dd, *J =* 17.5, 4.9 Hz, 1H), 2.59 (d, *J* = 17.3 Hz, 1H), 2.30 - 2.09 (m, 2H), 1.89 - 1.80 (m, 1H), 1.80 - 1.66 (m, 1H). |
| 172 | 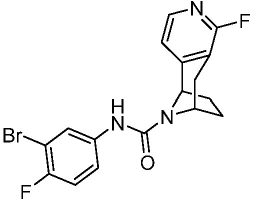 (±)-*N*-(3-Bromo-4-fluorophenyl)-1-fluoro-6,7,8,9-tetrahydro-5H-5,8-epiminocyclohepta[c]pyridine -10-carboxamide | 394.2, 396.2 at 1.31 min | ¹H NMR (400 MHz, DMSO-d6) δ 8.89 (s, 1H), 8.01 (d, *J* = 5.0 Hz, 1H), 7.85 (dd, *J =* 6.4, 2.6 Hz, 1H), 7.44 (ddd, *J* = 9.0, 4.4, 2.6 Hz, 1H), 7.24 (t, *J* = 8.8 Hz, 1H), 7.20 (dd, *J* = 5.1, 1.7 Hz, 1H), 5.22 (d, *J* = 6.1 Hz, 1H), 4.79 (t, *J* = 6.1 Hz, 1H), 3.15 (dd, *J* = 17.4, 5.0 Hz, 1H), 2.58 (d, *J* = 17.3 Hz, 1H), 2.20 (ddd, *J* = 25.0, 11.9, 6.7 Hz, 2H), 1.90 - 1.80 (m, 1H), 1.80 - 1.70 (m, 1H). |
| 173 | 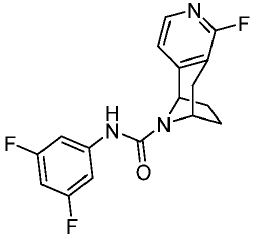 (±)-*N*-(3,5-Difluorophenyl)-1-fluoro-6,7,8,9-tetrahydro-5H-5,8-epiminocyclohepta[c]pyridine -10-carboxamide | 334.2 at 1.26 min | ¹H NMR (400 MHz, DMSO-d6) δ 9.06 (s, 1H), 8.01 (d, *J* = 5.0 Hz, 1H), 7.32 - 7.15 (m, 3H), 6.75 (tt, *J* = 9.3, 2.4 Hz, 1H), 5.24 (d, *J* = 6.1 Hz, 1H), 4.81 (t, *J* = 6.1 Hz, 1H), 3.14 (dd, *J* = 17.4, 5.0 Hz, 1H), 2.60 (d, *J* = 17.4 Hz, 1H), 2.20 (dtd, *J* = 23.7, 12.1, 11.7, 7.4 Hz, 2H), 1.96 - 1.79 (m, 1H), 1.79 - 1.67 (m, 1H). |
| 174 | 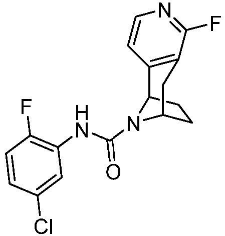 (±)-*N*-(5-Chloro-2-fluorophenyl)-1-fluoro-6,7,8,9-tetrahydro-5H-5,8-epiminocyclohepta[c]pyridine -10-carboxamide | 350.2 at 1.27 min | ¹H NMR (400 MHz, DMSO-d6) δ 8.69 (s, 1H), 8.00 (d, *J* = 5.0 Hz, 1H), 7.60 (dd, *J =* 6.9, 2.7 Hz, 1H), 7.23 (dd, *J* = 10.4, 8.8 Hz, 1H), 7.19 (dd, *J* = 5.0, 1.7 Hz, 1H), 7.14 (ddd, *J* = 8.8*,* 4.2, 2.7 Hz, 1H), 5.20 (d, *J* = 5.9 Hz, 1H), 4.77 (t, *J* = 6.1 Hz, 1H), 3.18 (dd, *J =* 17.4, 5.0 Hz, 1H), 2.57 (d, *J* = 17.3 Hz, 1H), 2.29 - 2.10 (m, 2H), 1.89 - 1.80 (m, 1H), 1.80 - 1.69 (m, 1H). |
| 175 | 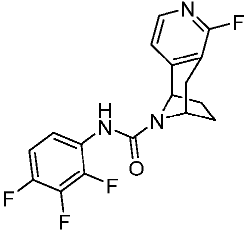 (±)-1-Fluoro-*N*-(2,3,4-trifluorophenyl)-6,7,8,9-tetrahydro-5H-5,8-epiminocyclohepta[c]pyridine -10-carboxamide | 352.2 at 1.18 min | ¹H NMR (400 MHz, DMSO-d6) δ 8.75 (s, 1H), 8.00 (d, *J* = 5.0 Hz, 1H), 7.32 - 7.09 (m, 3H), 5.18 (d, *J =* 6.0 Hz, 1H), 4.75 (t, *J =* 6.2 Hz, 1H), 3.18 (dd, *J* = 17.5, 5.0 Hz, 1H), 2.58 (d, *J* = 17.3 Hz, 1H), 2.31 - 2.12 (m, 2H), 1.89 - 1.80 (m, 1H), 1.80 - 1.70 (m, 1H). |
| 176 | 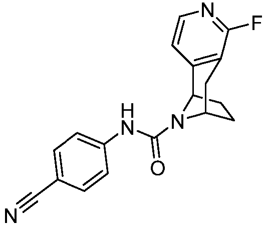 (±)-*N*-(4-Cyanophenyl)-1-fluoro-6,7,8,9-tetrahydro-5H-5,8-epiminocyclohepta[c]pyridine -10-carboxamide | 321.1 at 1.09 min^{(d)} | ¹H NMR (400 MHz, DMSO-d6) δ 9.09 (s, 1H), 8.01 (d, *J* = 5.0 Hz, 1H), 7.66 (s, 3H), 7.21 (dd, *J* = 5.0, 1.6 Hz, 1H), 5.26 (d, *J* = 6.1 Hz, 1H), 4.84 (t, *J* = 6.2 Hz, 1H), 3.15 (dd, *J* = 17.5, 5.1 Hz, 1H), 2.60 (d, *J* = 17.4 Hz, 1H), 2.30 - 2.08 (m, 2H), 1.91 - 1.80 (m, 1H), 1.76 (dt, *J* = 13.9, 6.5 Hz, 1H). |
| 177 | 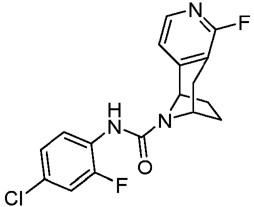 (±)-*N*-(4-Chloro-2-fluorophenyl)-1-fluoro-6,7,8,9-tetrahydro-5H-5,8-epiminocyclohepta[c]pyridine -10-carboxamide | 350.2 at 1.25 min | ¹H NMR (400 MHz, DMSO-d6) δ 8.62 (s, 1H), 8.00 (d, *J* = 4.7 Hz, 1H), 7.44 (t, *J =* 8.7 Hz, 1H), 7.39 (dd, *J* = 10.5, 2.4 Hz, 1H), 7.25 - 7.10 (m, 2H), 5.19 (d, *J =* 5.9 Hz, 1H), 4.76 (dd, *J* = 7.1, 5.0 Hz, 1H), 3.17 (dd, *J* = 17.4, 5.0 Hz, 1H), 2.57 (d, *J* = 17.3 Hz, 1H), 2.29 - 2.08 (m, 2H), 1.91 - 1.79 (m, 1H), 1.79 - 1.64 (m, 1H). |
| 178 | 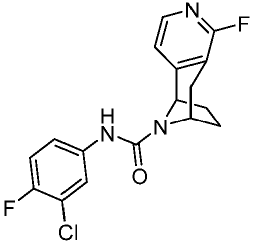 (±)-*N*-(3-Chloro-4-fluorophenyl)-1-fluoro-6,7,8,9-tetrahydro-5H-5,8-epiminocyclohepta[c]pyridine -10-carboxamide | 350.2 at 1.29 min | ¹H NMR (400 MHz, DMSO-d6) δ 8.90 (s, 1H), 8.01 (d, *J* = 5.0 Hz, 1H), 7.73 (dd, *J =* 6.9, 2.6 Hz, 1H), 7.39 (ddd, *J* = 9.1, 4.3, 2.6 Hz, 1H), 7.28 (t, *J =* 9.1 Hz, 1H), 7.20 (dd, *J* = 5.1, 1.7 Hz, 1H), 5.22 (d, *J* = 6.1 Hz, 1H), 4.85 - 4.77 (m, 1H), 3.15 (dd, *J* = 17.4, 4.9 Hz, 1H), 2.58 (d, *J* = 17.4 Hz, 1H), 2.19 (dtd, *J* = 17.8, 11.7, 7.1 Hz, 2H), 1.90 - 1.79 (m, 1H), 1.79 - 1.67 (m, 1H). |
| 179 | 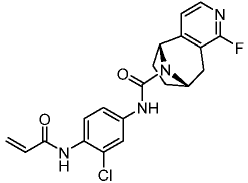 (5R,8S)-*N*-(4-Acrylamido-3-chlorophenyl)-1-fluoro-6,7,8,9-tetrahydro-5H-5,8-epiminocyclohepta[c]pyridine -10-carboxamide | 401.3, 403.5 at 1.16 min | ¹H NMR (400 MHz, DMSO-d6) δ 9.60 (s, 1H), 8.90 (s, 1H), 8.01 (d, *J* = 5.0 Hz, 1H), 7.71 (d, *J =* 2.4 Hz, 1H), 7.54 (d, *J* = 8.8 Hz, 1H), 7.36 (dd, *J* = 8.8, 2.4 Hz, 1H), 7.20 (dd, *J =* 5.0, 1.6 Hz, 1H), 6.54 (dd, *J =* 17.0, 10.2 Hz, 1H), 6.23 (dd, J = 17.1, 2.0 Hz, 1H), 5.74 (dd, *J =* 10.1, 1.9 Hz, 1H), 5.23 (d, *J* = 6.0 Hz, 1H), 4.80 (t, *J* = 6.1 Hz, 1H), 3.15 (dd, *J* = 17.5, 4.9 Hz, 1H), 2.58 (d, *J* = 17.4 Hz, 1H), 2.35 - 2.12 (m, 2H), 1.92 - 1.67 (m, 2H). |
| 180 | 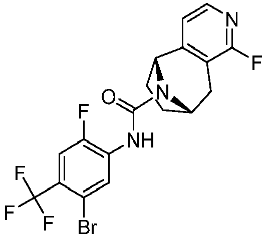 (5R,8S)-*N*-(5-Bromo-2-fluoro-4-(trifluoromethyl)phenyl)-1-fluoro-6,7,8,9-tetrahydro-5H-5,8-epiminocyclohepta[c]pyridine -10-carboxamide | 460.0, 462.0 at 1.71min^{(d)} | ¹H NMR (400 MHz, DMSO-d6) δ 9.06 (s, 1H), 8.17 (d, *J* = 7.1 Hz, 1H), 8.01 (d, *J =* 5.0 Hz, 1H), 7.76 (d, *J* = 11.2 Hz, 1H), 7.21 (dd, *J* = 5.1, 1.6 Hz, 1H), 5.24 (d, *J* = 5.9 Hz, 1H), 4.81 (t, *J = 6.2* Hz, 1H), 3.19 (dd, *J* = 17.4, 5.0 Hz, 1H), 2.60 (d, *J* = 17.4 Hz, 1H), 2.32 - 2.10 (m, 2H), 1.95 - 1.79 (m, 1H), 1.80-1.70 (m, 1H). |
| 181 | 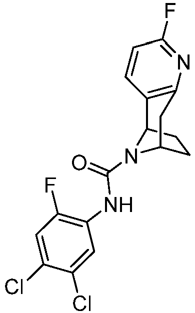 (±)-N-(4,5-Dichloro-2-fluorophenyl)-2-fluoro-6,7,8,9-tetrahydro-5H-5,8-epiminocyclohepta[b]pyridine -10-carboxamide | 382.1, 384.1 at 1.55 min^{(d)} | ¹H NMR (400 MHz, DMSO-d6) δ 8.79 (s, 1H), 7.81 (d, *J* = 7.6 Hz, 1H), 7.77 (t, *J* = 8.2 Hz, 1H), 7.66 (d, *J* = 10.3 Hz, 1H), 6.94 (dd, *J* = 8.3, 2.6 Hz, 1H), 5.21 (d, *J* = 5.9 Hz, 1H), 4.74 (t, *J* = 6.2 Hz, 1H), 3.34 (dd, *J* = 17.3, 5.0 Hz, 1H), 2.67 (d, *J =* 17.9 Hz, 1H), 2.32 - 2.08 (m, 2H), 1.87 - 1.77 (m, 1H), 1.76 1.61 (m, 1H) |
| 182 | 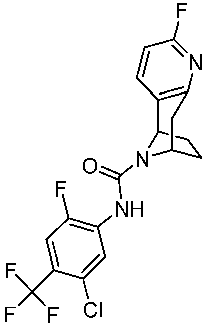 (±)-*N*-(5-Chloro-2-fluoro-4-(trifluoromethyl)phenyl)-2-fluoro-6,7,8,9-tetrahydro-5H-5,8-epiminocyclohepta[b]pyridine -10-carboxamide | 416.2, 418.2 at 1.68 min^{(d)} | ¹H NMR (400 MHz, DMSO-d6) δ 9.05 (s, 1H), 8.02 (d, *J* = 6.9 Hz, 1H), 7.83 - 7.72 (m, 2H), 6.94 (dd, *J* = 8.2, 2.6 Hz, 1H), 5.26 (d, *J* = 5.9 Hz, 1H), 4.85 - 4.63 (m, 1H), 3.35 (d, *J =* 17.1, 5.0 Hz, 1H), 2.68 (d, *J* = 17.9 Hz, 1H), 2.31 - 2.10 (m, 2H), 1.89 - 1.78 (m, 1H), 1.78 - 1.67 (m, 1H). |
| 183 | 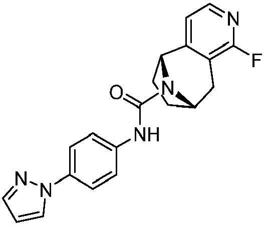 (5*R*,8*S*)-*N*-(4-(1H-Pyrazol-1-yl)phenyl)-1-fluoro-6,7,8,9-tetrahydro-5H-5,8-epiminocyclohepta[c]pyridine -10-carboxamide | 364.3 at 1.22 min | ¹H NMR (400 MHz, DMSO-d6) δ 8.86 (s, 1H), 8.36 (d, *J* = 2.4 Hz, 1H), 8.01 (d, *J =* 5.0 Hz, 1H), 7.70 = 7.65 (m, 3H), 7.58 - 7.52 (m, 2H), 7.21 (dd, *J* = 5.0, 1.7 Hz, 1H), 6.49 (t, *J* = 2.1 Hz, 1H), 5.25 (d, *J* = 6.0 Hz, 1H), 4.82 (t, *J* = 6.2 Hz, 1H), 3.17 (dd, *J* = 17.6, 4.9 Hz, 1H), 2.59 (d, *J* = 17.3 Hz, 1H), 2.29 - 2.13 (m, 2H), 1.88 - 1.81 (m, 1H), 1.80 - 1.71 (m, 1H). |
| 184 | 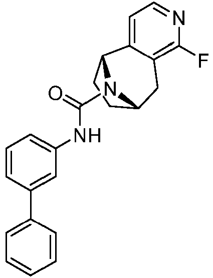 (5*R*,8*S*)-*N*-([1,1'-Biphenyl]-3-yl)-1-fluoro-6,7,8,9-tetrahydro-5H-5,8-epiminocyclohepta[c]pyridine -10-carboxamide | 374.3 at 1.62 min | ¹H NMR (400 MHz, DMSO-d6) δ 8.82 (s, 1H), 8.01 (d, *J* = 5.0 Hz, 1H), 7.77 (t, *J =* 2.0 Hz, 1H), 7.61 - 7.54 (m, 2H), 7.52 - 7.41 (m, 3H), 7.39 - 7.27 (m, 2H), 7.25 - 7.18 (m, 2H), 5.26 (d, *J* = 6.0 Hz, 1H), 4.83 (t, *J = 6.1* Hz, 1H), 3.18 (dd, *J* = 17.2, 5.0 Hz, 1H), 2.59 (d, *J* = 17.3 Hz, 1H), 2.30 - 2.13 (m, 2H), 1.90 - 1.81 (m, 1H), 1.81 - 1.69 (m, 1H). |
| 185 | 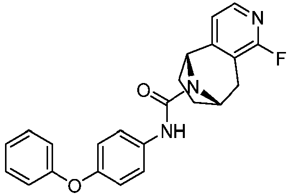 (5*R*,8*S*)-1-Fluoro-*N*-(4-phenoxyphenyl)-6,7,8,9-tetrahydro-5H-5,8-epiminocyclohepta[c]pyridine -10-carboxamide | 390.3 at 1.60 min | ¹H NMR (400 MHz, DMSO-d6) δ 8.76 (s, 1H), 8.00 (d, *J* = 5.0 Hz, 1H), 7.51 - 7.38 (m, 2H), 7.38 - 7.28 (m, 2H), 7.20 (dd, *J* = 5.0, 1.7 Hz, 1H), 7.07 (t, *J* = 7.4 Hz, 1H), 6.97 - 6.83 (m, 4H), 5.23 (d, *J =* 6.0 Hz, 1H), 4.79 (t, *J* = 6.0 Hz, 1H), 3.16 (dd, *J* = 17.3, 4.9 Hz, 1H), 2.57 (d, *J =* 17.3 Hz, 1H), 2.30 - 2.11 (m, 2H), 1.90 - 1.80 (m, 1H), 1.79 - 1.69 (m, 1H). |
| 186 | 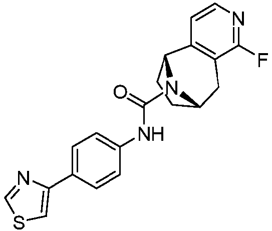 (5*R*,8S)-1-Fluoro-*N*-(4-(thiazol-4-yl)phenyl)-6,7,8,9-tetrahydro-5H-5,8-epiminocyclohepta[c]pyridine -10-carboxamide | 381.2 at 1.23 min | ¹H NMR (400 MHz, DMSO-d6) δ 9.14 (d, *J =* 1.9 Hz, 1H), 8.85 (s, 1H), 8.01 (d, *J =* 5.0 Hz, 1H), 7.99 (d, *J* = 1.9 Hz, 1H), 7.88 - 7.82 (m, 2H), 7.56 - 7.51 (m, 2H), 7.21 (dd, *J* = 5.0, 1.6 Hz, 1H), 5.26 (d, *J* = 6.0 Hz, 1H), 4.83 (t, *J* = 6.2 Hz, 1H), 3.21 - 3.10 (m, 1H), 2.59 (d, *J =* 17.4 Hz, 1H), 2.29 - 2.12 (m, 2H), 1.85 (dd, *J* = 11.3, 8.9 Hz, 1H), 1.80 - 1.69 (m, 1H). |
| 187 | 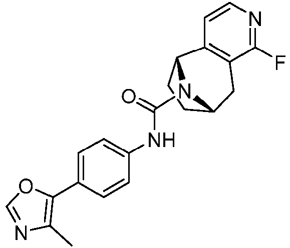 (5*R*,8*S*)-1-Fluoro-*N*-(4-(4-methyloxazol-5-yl)phenyl)-6,7,8,9-tetrahydro-5H-5,8-epiminocyclohepta[c]pyridine -10-carboxamide | 379.2 at 1.20 min | ¹H NMR (400 MHz, DMSO-d6) δ 8.91 (s, 1H), 8.25 (s, 1H), 8.01 (d, *J* = 5.0 Hz, 1H), 7.64 - 7.56 (m, 2H), 7.52 - 7.44 (m, 2H), 7.21 (dd, *J =* 5.0, 1.7 Hz, 1H), 5.26 (d, *J* = 6.0 Hz, 1H), 4.83 (t, *J* = 6.1 Hz, 1H), 3.16 (dd, *J* = 17.5, 5.0 Hz, 1H), 2.59 (d, *J* = 17.4 Hz, 1H), 2.31 (s, 3H), 2.28 - 2.12 (m, 2H), 1.89 - 1.81 (m, 1H), 1.80 - 1.69 (m, 1H). |
| 188 | 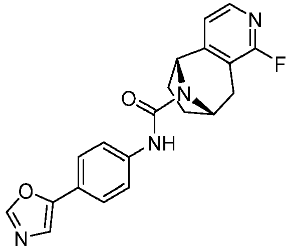 (5*R*,8*S*)-1-Fluoro-*N*-(4-(oxazol-5-yl)phenyl)-6,7,8,9-tetrahydro-5H-5,8-epiminocyclohepta[c]pyridine -10-carboxamide | 365.3 at 1.15 min | ¹H NMR (400 MHz, DMSO-d6) δ 8.91 (s, 1H), 8.36 (s, 1H), 8.01 (d, *J* = 5.0 Hz, 1H), 7.58 (s, 4H), 7.52 (s, 1H), 7.21 (dd, *J* = 5.0, 1.7 Hz, 1H), 5.26 (d, *J* = 6.0 Hz, 1H), 4.83 (t, *J* = 6.0 Hz, 1H), 3.16 (dd, *J* = 17.3, 5.0 Hz, 1H), 2.59 (d, *J* = 17.3 Hz, 1H), 2.31 -2.10 (m, 2H), 1.90 - 1.80 (m, 1H), 1.76 (dt, *J* = 13.7, 6.7 Hz, 1H). |
| 189 | 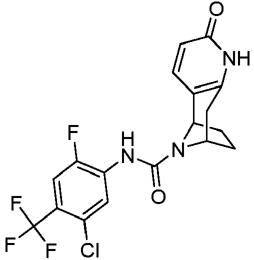 (±)-*N*-(5-Chloro-2-fluoro-4-(trifluoromethyl)phenyl)-2-oxo-2,5,6,7,8,9-hexahydro-1 H-5,8-epiminocyclohepta[b]pyridine -10-carboxamide | 416.1, 418.2 at 1.27 min | ¹H NMR (400 MHz, DMSO-d6) δ 11.50 (s, 1H), 8.96 (s, 1H), 8.06 (d, *J* = 6.9 Hz, 1H), 7.78 (d, *J =* 11.0 Hz, 1H), 7.28 (d, *J =* 9.2 Hz, 1H), 6.11 (d, *J* = 9.2 Hz, 1H), 4.97 (d, *J* = 5.5 Hz, 1H), 4.65 (t, *J* = 6.4 Hz, 1H), 3.18 - 3.05 (m, 1H), 2.38 (d, *J =* 17.7 Hz, 1H), 2.25 - 2.13 (m, 1H), 2.10 - 1.96 (m, 1H), 1.86 - 1.78 (m, 1H), 1.73 - 1.64 (m, 1H). |
| 190^{(f)} | 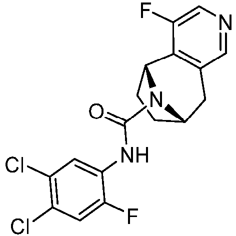 (5*R*,8*S*)-*N*-(4,5-Dichloro-2-fluorophenyl)-4-fluoro-6,7,8,9-tetrahydro-5H-5,8-epiminocyclohepta[c]pyridine -10-carboxamide | 384.1, 386.1 at 2.73 min^{(e)} | ¹H NMR (400 MHz, DMSO-d6) δ 8.91 (s, 1H), 8.33 (s, 1H), 8.22 (s, 1H),7.81 (d, *J* = 7.6 Hz, 1H), 7.65 (d, *J* = 10.0 Hz, 1H), 5.39 (d, *J* = 5.6 Hz, 1H), 4.76 (t, *J* = 5.2 Hz, 1H), 3.35 (d, *J =* 4.0 Hz, 1H), 2.75 - 2.70 (m, 1H), 2.32 - 2.14 (m, 2H), 1.89 - 1.83 (m, 1H), 1.75 -1.68 (m, 1H) |
| 191(a)(c) | 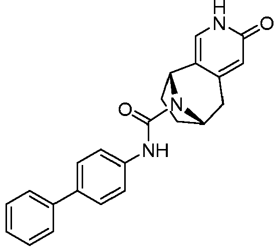 (6*S*,9*R*)-*N*-([1,1'-Biphenyl]-4-yl)-3-oxo-3,5,6,7,8,9-hexahydro-2H-6,9-epiminocyclohepta[c]pyridine -10-carboxamide | 372.3 at 1.19 min | ¹H NMR (500 MHz, DMSO-d6) δ 11.26 (s, 1H), 8.67 (s, 1H), 7.64 - 7.59 (m, 2H), 7.59 - 7.52 (m, 4H), 7.42 (t, *J* = 7.7 Hz, 2H), 7.32 - 7.28 (m, 1H), 7.19 (s, 1H), 6.10 (s, 1H), 5.09 (d, *J* = 6.0 Hz, 1H), 4.62 (t, *J* = 6.5 Hz, 1H), 3.13 (dd, *J* = 18.1, 5.1 Hz, 1H), 2.58 - 2.53 (m, 1H), 2.23 - 2.13 (m, 1H), 2.12 - 2.03 (m, 1H), 1.77 - 1.69 (m, 1H), 1.69 - 1.60 (m, 1H). |
| 192^{(a)(b)} | 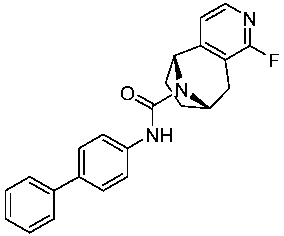 (5*R*,8*S*)-*N*-([1,1'-Biphenyl]-4-yl)-1-fluoro-6,7,8,9-tetrahydro-5H-5,8-epiminocyclohepta[c]pyridine -10-carboxamide | 374 at 1.56 min | ¹H NMR (500 MHz, DMSO-d6) δ 8.84 (s, 1H), 8.01 (d, *J* = 5.0 Hz, 1H), 7.63 - 7.58 (m, 2H), 7.54 (s, 4H), 7.42 (t, *J* = 7.7 Hz, 2H), 7.32 - 7.26 (m, 1H), 7.21 (dd, J = 5.0, 1.6 Hz, 1H), 5.26 (d, *J* = 6.2 Hz, 1H), 4.83 (t, *J* = 6.3 Hz, 1H), 3.17 (dd, *J* = 17.4, 5.0 Hz, 1H), 2.59 (d, *J* = 17.4 Hz, 1H), 2.31 - 2.15 (m, 2H), 1.91 - 1.82 (m, 1H), 1.80 - 1.71 (m, 1H). |
| 193^{(a)(c)} | 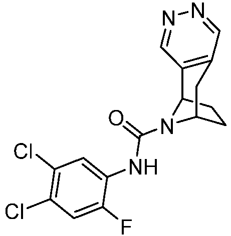 (±)-*N*-(4,5-Dichloro-2-fluorophenyl)-6,7,8,9-tetrahydro-5H-5,8-epiminocyclohepta[d]pyridazi ne-10-carboxamide | 367.2, 369.1 at 1.19 min | ¹H NMR (400 MHz, DMSO-d6) δ 9.04 (d, *J* = 1.2 Hz, 1H), 8.99 (s, 1H), 8.86 (s, 1H), 7.80 (dd, *J* = 7.6, 3.7 Hz, 1H), 7.67 (d, *J =* 10.3 Hz, 1H), 5.18 (d, *J* = 5.9 Hz, 1H), 4.76 (t, *J* = 6.2 Hz, 1H), 3.28 (d, *J* = 5.3 Hz, 1H), 2.68 (d, *J =* 18.1 Hz, 1H), 2.31 - 2.09 (m, 2H), 1.92 - 1.78 (m, 1H), 1.72 (dd, *J* = 12.6, 5.7 Hz, 1H) |
| 194^{(a)(b)} | 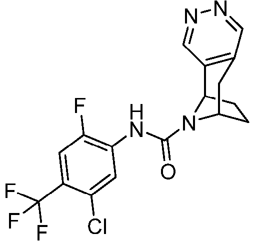 (±)-*N*-(5-Chloro-2-fluoro-4-(trifluoromethyl) phenyl)-6,7,8,9-tetrahydro-5H-5,8-epiminocyclohepta[d]pyridazi ne-10-carboxamide | 401.2, 403.2 at 1.33 min | ¹H NMR (400 MHz, DMSO-d6) δ 9.13 (s, 1H), 9.05 (d, *J =* 1.2 Hz, 1H), 8.99 (s 1H), 8.00 (d, *J* = 6.9 Hz, 1H), 7.79 (d, *J =* 11.0 Hz, 1H), 5.22 (d, *J* = 5.8 Hz, 1H), 4.81 (t, *J* = 6.1 Hz, 1H), 3.28 (d, *J* = 5.7 Hz, 1H), 2.69 (d, *J* = 18.1 Hz, 1H), 2.31 - 2.14 (m, 2H), 1.94 - 1.82 (m, 1H), 1.78 - 1.64 (m, 1H) |
| 195^{(a)(b)} | 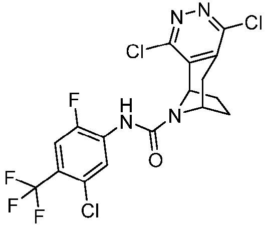 (±)-1,4-Dichloro-*N*-(5-chloro-2-fluoro-4-(trifluoromethyl)phenyl)-6,7,8,9-tetrahydro-5H-5,8-epiminocyclohepta[d]pyridazi ne-10-carboxamide | 467.0, 469.0 at 1.75 min^{(d)} | ¹H NMR (400 MHz, DMSO-d6) δ 9.30 (s, 1H), 8.05 (d, *J* = 6.9 Hz, 1H), 7.79 (d, *J =* 11.0 Hz, 1H), 5.34 (d, *J* = 5.7 Hz, 1H), 4.86 (d, *J* = 6.2 Hz, 1H), 3.28 - 3.20 (m, 1H), 2.68 (d, *J =* 18.6 Hz, 1H), 2.35 - 2.14 (m, 2H), 2.04 - 1.88 (m, 1H), 1.85 - 1.72 (m, 1H). |
| 199^{(a)(b)} | 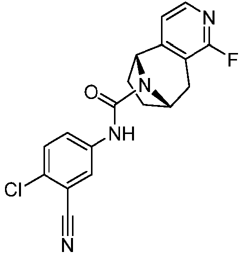 (5*R*,8*S*)-*N*-(4-Chloro-3-cyanophenyl)-1-fluoro-6,7,8,9-tetrahydro-5H-5,8-epiminocyclohepta[c]pyridine -10-carboxamide | 355.1, 357.2 at 1.38 min^{(d)} | ¹H NMR (400 MHz, DMSO-d6) δ 9.15 (s, 1H), 8.04 (d, *J* = 2.6 Hz, 1H), 8.01 (d, *J* = 5.0 Hz, 1H), 7.76 (dd, *J* = 9.0, 2.6 Hz, 1H), 7.60 (d, *J* = 9.0 Hz, 1H), 7.21 (dd, *J* = 5.0, 1.7 Hz, 1H), 5.24 (d, *J* = 6.1 Hz, 1H), 4.81 (t, *J = 6.1* Hz, 1H), 3.15 (dd, *J* = 17.5, 5.0 Hz, 1H), 2.60 (d, *J* = 17.4 Hz, 1H), 2.31 - 2.10 (m, 2H), 1.91 - 1.82 (m, 1H), 1.76 (dt, *J* = 13.9, 6.8 Hz, 1H). |
| 200^{(f)} | 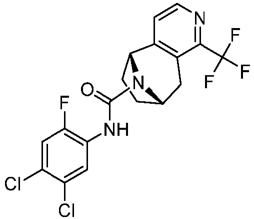 (5*R*,8*S*)-*N*-(4,5-dichloro-2-fluorophenyl)-1-(trifluoromethyl)-6,7,8,9-tetrahydro-5H-5,8-epiminocyclohepta[c]pyridine -10-carboxamide | 434.2, 436.2 at 4.40 min | ¹H NMR (400 MHz, DMSO-d6):δ ppm: 8.87 (s, 1H), 8.49 (d, *J* = 4.8Hz, 1H), 7.80 (d, *J* = 7.6 Hz, 1H), 7.65 (d, *J =* 10.4 Hz, 1H), 7.54 (d, *J* = 4.8 Hz, 1H), 5.26 (d, *J* = 4.8Hz, 1H), 4.77 (t, *J* = 6.0 Hz, 1H), 3.45 (dd, *J* = 2.8 Hz, 4.0 Hz, 1H), 2.80 (d, *J* = 17.6 Hz, 1H), 2.28 - 2.15 (m, 2H), 1.88 - 1.83 (m, 1H), 1.76 - 1.70(m, 1H). |

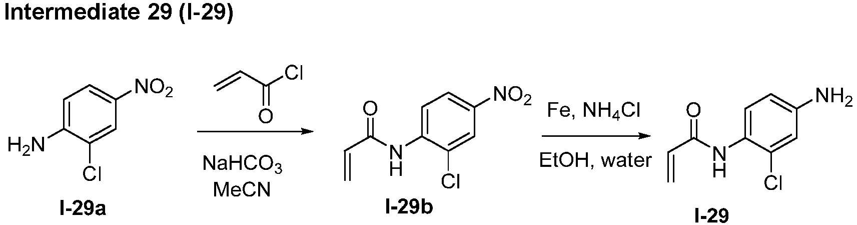

**Step 1:** To a solution of 2-chloro-4-nitroaniline **I-29a** (1.00 g, 5.79 mmol) and sodium bicarbonate (1.46 g, 17.4 mmol) in MeCN (10 ml) was added acryloyl chloride (706 µl, 8.69 mmol) at 0 °C over 5 min. The reaction was warmed to RT for 16 h. The reaction mixture was diluted with EtOAc (30 ml) and washed with water (3 x 20 ml). The organic layer was dried over magnesium sulfate and concentrated *in vacuo* to obtain N-(2-chloro-4-nitrophenyl)acrylamide **I-29b** as a yellow solid. The compound was used in the next step without further purification. ¹H NMR (400 MHz, DMSO-d6) δ 10.03 (s, 1H), 8.37 (d, *J = 2.6* Hz, 1H), 8.31 (d, *J =* 9.1 Hz, 1H), 8.23 (dd, *J* = 9.1, 2.6 Hz, 1H), 6.77 (dd, *J* = 17.0, 10.3 Hz, 1H), 6.36 (dd, *J =* 17.0, 1.8 Hz, 1H), 5.88 (dd, *J =* 10.2, 1.8 Hz, 1H).

**Step 2:** To a solution of *N*-(2-chloro-4-nitrophenyl)acrylamide (1.40 g, 5.232 mmol) in a mixture of EtOH (30 ml) and water (6 ml) was added iron powder (585 mg, 10.46 mmol) followed by 1 ml of saturated ammonium chloride solution. The reaction mixture was heated at 80 °C for 4 h. The reaction mixture was cooled to RT and filtered through celite. The celite was washed with EtOH and ethyl acetate. The filtrate was concentrated *in vacuo* and the residue was partitioned between 10% MeOH in DCM (50 ml) and water (50 ml). The layers were separated and the aqueous layer was extracted with 10% MeOH in DCM (2 x 30 ml). The organic layers were combined, dried over magnesium sulfate and concentrated *in vacuo* to obtain *N*-(4-amino-2-chlorophenyl)acrylamide **I-29** as a yellow oil. The compound was used in the next step without any further purification. ¹H NMR (400 MHz, DMSO-d6) δ 9.37 (s, 1H), 7.18 (d, *J* = 8.6 Hz, 1H), 6.65 (d, *J* = 2.5 Hz, 1H), 6.56 - 6.35 (m, 2H), 6.18 (dd, *J =* 17.1, 2.1 Hz, 1H), 5.69 (dd, *J =* 10.2, 2.1 Hz, 1H), 5.33 (s, 2H)

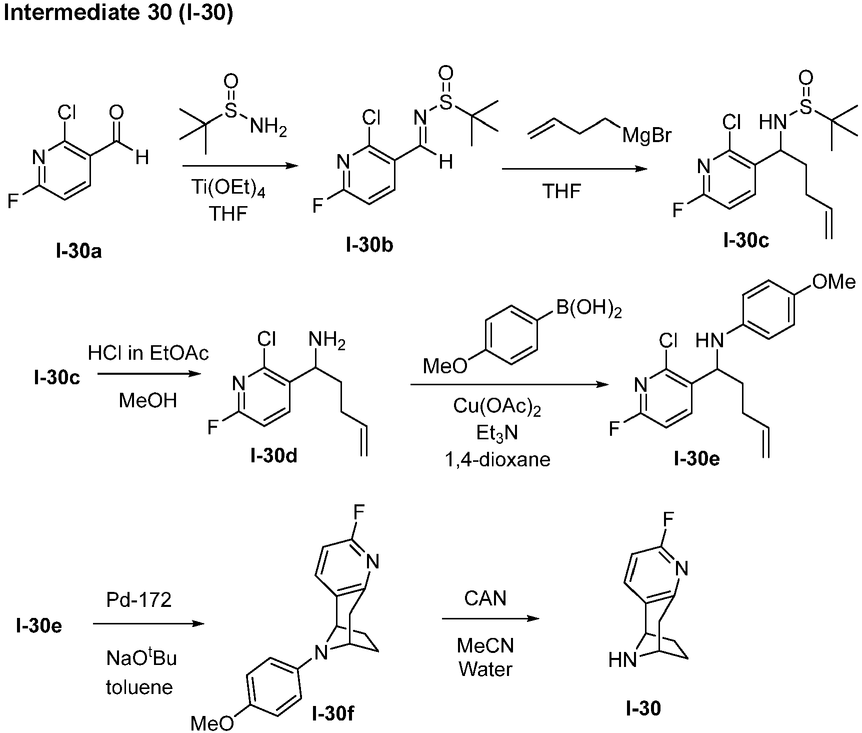

**Step 1:** (*E*)-*N*-((2-Chloro-6-fluoropyridin-3-yl)methylene)-2-methylpropane-2-sulfinamide **(I30b)** was synthesised from 2-chloro-6-fluoronicotinaldehyde **(I-30a)** using a procedure essentially the same as for **I-7b.** ¹H NMR: (400 MHz, CDCl₃) δ 8.93 (s, 1H), 8.48 (t, *J* = 8.4 Hz, 1H), 7.02 - 6.99 (m, 1H), 1.28 (m, 9H).

**Step 2:** *N*-(1-(2-Chloro-6-fluoropyridin-3-yl)pent-4-en-1-yl)-2-methylpropane-2-sulfinamide **(I30c)** was synthesised from (*E*)-*N*-((2-chloro-6-fluoropyridin-3-yl)methylene)-2-methylpropane-2-sulfinamide **(I30b)** using a procedure essentially the same as for **I-7c.** ¹H NMR: (400 MHz, CDCl₃) δ 7.82 - 7.87 (m, 1H), 6.87 - 6.90 (m, 1H), 5.76 (t, *J* = 10.4 Hz, 1H), 5.01 - 5.05 (m, 2H), 4.98 - 4.84 (m, 1H), 5.82 - 3.79 (m, 1H), 2.20- 2.05 (m, 2H), 1.94-1.88 (m, 2H) 1.16-1.22 (m, 9H)

**Step 3:** 1-(2-Chloro-6-fluoropyridin-3-yl)pent-4-en-1-amine **(I30d)** was synthesised from N-(1-(2-Chloro-6-fluoropyridin-3-yl)pent-4-en-1-yl)-2-methylpropane-2-sulfinamide **(I30c)** using a procedure essentially the same as for **I-7d.** ¹H NMR: (400 MHz, CDCl₃) δ 8.00 (t, *J =* 8.4 Hz, 1H), 6.87-6.90 (m, 1H), 5.72-5.83 (m 1H), 5.96-5.05 (m, 2H), 4.36 (t, *J* = 5.6 Hz, 1H) 2.09-2.15 (m, 2H), 1.65-1.69 (m, 2H), 1.48 (s, 2H).

**Step 4:** *N*-(1-(2-Chloro-6-fluoropyridin-3-yl)pent-4-en-1-yl)-4-methoxyaniline **(I-30e)** was synthesised from 1-(2-chloro-6-fluoropyridin-3-yl)pent-4-en-1-amine **(I30d)** using a procedure essentially the same as for **I-7e.** ¹H NMR: (400 MHz, CDCl₃) δ 7.88 (t, *J* = 8.0 Hz, 1H), 6.78 - 6.83 (m, 1H), 6.70 (t, *J =* 4.4 Hz, 2H), 6.40 (d, *J* = 4.4 Hz, 2H), 5.80 - 5.87 (m, 1H), 5.01 - 5.05 (m, 2H), 3.70 (s, 3H), 2.19 - 2.31 (m, 2H), 1.77 - 1.92 (m, 2H), 1.43 (s, 1H) (1 exchangeable NH not observed)

**Step 5:** To a solution of *N*-(1-(2-chloro-6-fluoropyridin-3-yl)pent-4-en-1-yl)-4-methoxyaniline **(I-30e)** (3.00 g, 9.35 mmol) in toluene (60 ml) was added NaO^{t}Bu (1.35 g, 14.0 mmol) and Pd-172 (567 mg, 935 umol) at 20 °C. The mixture was stirred at 110 °C for 16 h. The reaction mixture was concentrated *in vacuo.* The product was purified by silica gel chromatography (EtOAc/Petroleum ether 1%-100%) to give (±)-2-fluoro-10-(4-methoxyphenyl)-6,7,8,9-tetrahydro-5H-5,8-epiminocyclohepta[b]pyridine **(I-30f)** as a yellow solid. ¹H NMR: (400 MHz, CDCl₃) δ 7.55 (t, *J =* 8.0 Hz, 1H), 6.68 - 6.77 (m, 5H), 4.72 (d, *J =* 2.4 Hz, 1H), 4.52 (d, *J =* 5.6 Hz, 1H), 3.71 (s, 3H), 3.28 (dd, *J*=8.8, 4.4 Hz,1H), 2.53 (d, *J* = 8.8 Hz, 1H), 2.42 - 2.56 (m, 3H), 1.82 - 1.96 (m, 2H).

**Step 6:** (±)-2-fluoro-6,7,8,9-tetrahydro-5H-5,8-epiminocyclohepta[b]pyridine **(I-30)** was synthesised from (±)-2-fluoro-10-(4-methoxyphenyl)-6,7,8,9-tetrahydro-5H-5,8-epiminocyclohepta[b]pyridine **(I-30f)** using a procedure essentially the same as for **I-9.** ¹H NMR (400 MHz, DMSO-d6) δ 7.60 (t, *J* = 8.3 Hz, 1H), 6.90 - 6.76 (m, 1H), 4.19 (d, *J* = 5.6 Hz, 1H), 3.84 - 3.68 (m, 1H), 3.02 (dd, *J* = 17.7, 5.2 Hz, 1H), 2.79 (s, 1H), 2.45 (s, 1H), 2.00 - 1.82 (m, 2H), 1.77 - 1.65 (m, 1H), 1.58 - 1.39 (m, 1H)

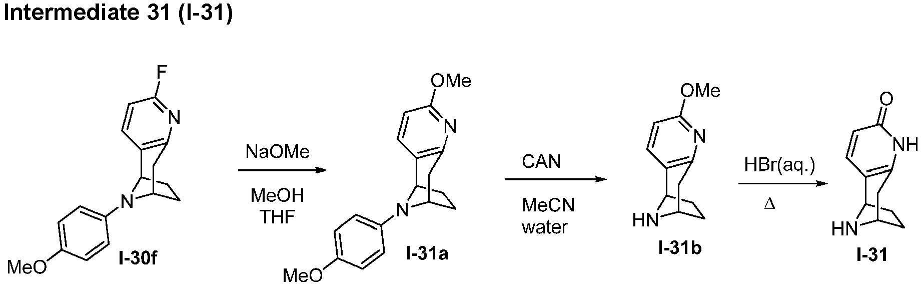

**Step 1:** To a solution of (±)-2-fluoro-10-(4-methoxyphenyl)-6,7,8,9-tetrahydro-5H-5,8-epiminocyclohepta[b]pyridine (113 mg, 397 µmol) in MeOH (4 ml) was added a solution of sodium methoxide in methanol (110 µl, 5.4 molar, 596 µmol). The resultant mixture was heated at 65 °C for 20 h. THF (1 ml) was added followed by a further portion of sodium methoxide (110 µl, 5.4 molar, 596 µmol). The reaction was stirred at 65 °C for a further 24 h. The reaction was cooled. The solid was filtered off and washed with MeOH (2 x 5 ml) and the solid was dried *in vacuo* to give (±)-2-methoxy-10-(4-methoxyphenyl)-6,7,8,9-tetrahydro-5H-5,8-epiminocyclohepta[b]pyridine **(I-31a)** as a white solid. LC-MS (method 1) m/z 297.1 (M+H)⁺ (ES⁺); at 1.68 min

**Step 2:** (±)-2-Methoxy-6,7,8,9-tetrahydro-5H-5,8-epiminocyclohepta[b]pyridine (I-31b) was synthesised from (±)-2-methoxy-10-(4-methoxyphenyl)-6,7,8,9-tetrahydro-5H-5,8-epiminocyclohepta[b]pyridine (**I-31a**) using a procedure essentially the same as for **I-9**. ¹H NMR (400 MHz, Chloroform-d) δ 7.17 (d, *J* = 8.2 Hz, 1H), 6.45 (d, *J* = 8.2 Hz, 1H), 4.17 (d, *J =* 5.5 Hz, 1H), 3.97 - 3.89 (m, 1H), 3.86 (s, 3H), 3.21 - 3.05 (m, 1H), 2.59 (d, *J =* 17.7 Hz, 1H), 2.35 (s, 1H), 2.19 - 2.00 (m, 2H), 1.89 - 1.78 (m, 1H), 1.68 - 1.57 (m, 1H).

**Step 3:**(±)-1,5,6,7,8,9-Hexahydro-2H-5,8-epiminocyclohepta[b]pyridin-2-one **(I-31)** was synthesised from (±)-2-methoxy-6,7,8,9-tetrahydro-5H-5,8-epiminocyclohepta[b]pyridine **(I-31b)** using a procedure essentially the same as for **I-10.** ¹H NMR (400 MHz, DMSO-d6) δ 11.32 (s, 1H), 7.15 (d, *J =* 9.1 Hz, 1H), 6.04 (d, *J =* 9.1 Hz, 1H), 3.91 (d, *J* = 5.3 Hz, 1H), 3.80 - 3.59 (m, 1H), 2.80 (dd, *J* = 17.5, 4.9 Hz, 1H), 2.18 (d, *J* = 17.5 Hz, 1H), 2.06 - 1.58 (m, 3H), 1.55 - 1.26 (m, 1H), 1 exchangeable proton not visible.

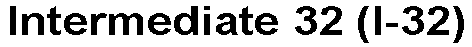

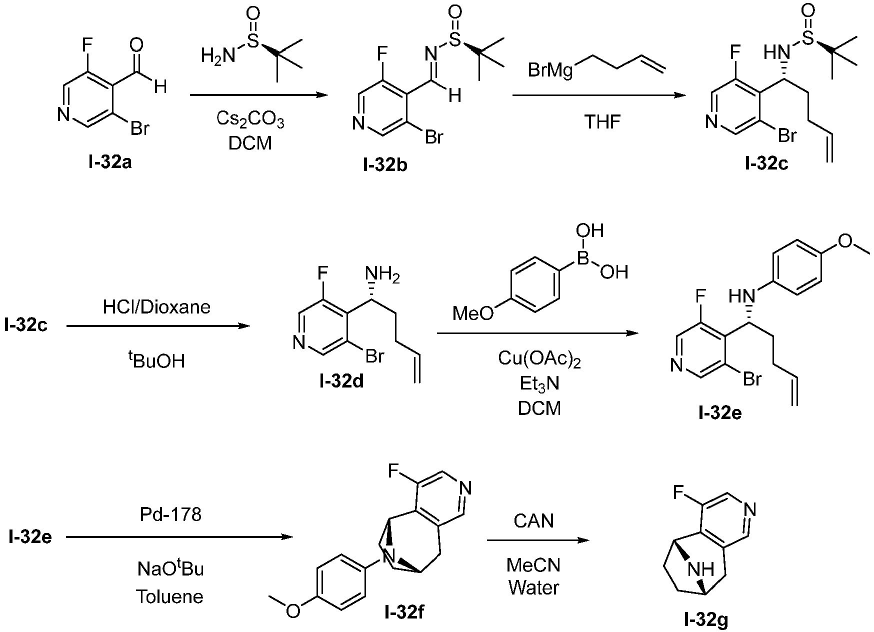

**Step** 1: To a solution of 3-bromo-5-fluoroisonicotinaldehyde **I-32a** (4.5 g, 22.2 mmol) and (*S*)-2-methylpropane-2-sulfinamide (2.7 g, 22.2 mmol) in DCM (50 ml) was added cesium carbonate (7.2 g, 22.2 mmol). After stirring at RT for 16 h, the reaction mixture was filtered and the filter cake was washed with DCM (50 ml). The filtrate was concentrated under reduced pressure to give (*S*)-*N*-((3-bromo-5-fluoropyridin-4-yl)methylene)-2-methylpropane-2-sulfinamide **I-32b** as a yellow solid. ¹H NMR (400 MHz, DMSO-d6) δ 8.81 (s, 1H), 8.77 (d, *J* = 1.6 Hz, 1H), 8.63 (s, 1H), 1.22 (s, 9H)

**Step 2:** (*S*)-*N*-((R)-1-(3-Bromo-5-fluoropyridin-4-yl)pent-4-en-1-yl)-2-methylpropane-2-sulfinamide **(I-32c)** was synthesised from (*S*)-*N*-((3-bromo-5-fluoropyridin-4-yl)methylene)-2-methylpropane-2-sulfinamide **(I-32b)** using a procedure essentially the same as for **I-13c.** ¹H NMR (400 MHz, DMSO-d6) δ 8.63 (s, 1H), 8.54 (d, *J* = 1.6 Hz, 1H), 5.84 - 5.72 (m, 2H), 5.08 - 4.97 (m, 2H), 4.79 (q, *J* = 6.4 Hz, 1H), 2.18 - 2.10 (m, 2H), 2.05 - 2.00(m, 1H), 1.99 - 1.87 (m, 1H), 1.01 (s, 9H).

**Step 3:** To a solution of (*S*)-*N*-((R)-1-(3-bromo-5-fluoropyridin-4-yl)pent-4-en-1-yl)-2-methylpropane-2-sulfinamide **(I-32c)** (5.4 g, 14.9 mmol) in ^{t}BuOH (60 ml) at 0 °C was added a solution of HCl in dioxane (4 M, 19 ml). The reaction mixture was stirred at RT for 2.5 h. Water was added (50 ml) and the mixture extracted with EtOAc (3 x 50 ml). The aqueous layer was basified with saturated sodium bicarbonate aqueous solution and extracted with EtOAc (3 x 50 ml). The combined organic layers were dried over sodium sulfate and concentrated *in vacuo* to give (*R*)-1-(3-bromo-5-fluoropyridin-4-yl)pent-4-en-1-amine (I-32d) as a yellow oil. ¹H NMR (400 MHz, DMSO-d6) δ 8.58 (s, 1H), 8.50 (d, *J =* 2.4 Hz, 1H), 5.84 - 5.74 (m, 1H), 5.03 - 4.92 (m, 2H), 4.19 (t, *J* = 7.2 Hz, 1H), 2.15 - 2.10 (m, 3H), 2.02 - 1.93 (m, 1H), 1.88 - 1.70 (m, 2H).

**Step 4:** To a solution of (*R*)-1-(3-bromo-5-fluoropyridin-4-yl)pent-4-en-1-amine (3 g, 11.6 mmol) **(I-32d)** in DCM (40 ml) were added (4-methoxyphenyl)boronic acid (5.3 g, 34.8 mmol), copper(ll)acetate (3.15 g, 17.4 mmol) and Et₃N (8.1 ml, 58 mmol). The dark blue mixture was vigorously stirred at RT, fully opened to the air, for 20 h. 2 M NaOH aqueous solution (20 ml) was added and the resultant biphasic mixture was filtered and the filter cake was washed with DCM. The layers were separated, and the aqueous layer extracted with DCM (2 x 50 ml). The combined organic layers were dried over Na₂SO₄ and concentrated *in vacuo.* The product was purified by silica gel chromatography (10 % EtOAc/ petroleum ether) to give (*R*)-*N*-(1-(3-bromo-5-fluoropyridin-4-yl)pent-4-en-1-yl)-4-methoxyaniline **(I-32e)** as a yellow oil. ¹H NMR (400 MHz, DMSO-d6) δ 8.56 (s, 1H), 8.45 (d, *J* = 2.0 Hz, 1H), 6.64 (d, *J =* 8.8 Hz, 2H), 6.47 (d, *J* = 8.8 Hz, 2H), 5.88 - 5.77 (m, 2H), 5.06 - 5.00 (m, 2H), 4.81 - 4.75 (m, 1H), 3.57 (s, 3H), 2.32 - 2.26 (m, 1H), 2.15 - 2.01 (m, 2H), 1.98 -1.80 (m, 1H).

**Step 5:** (5*R*,8*S*)-4-fluoro-10-(4-methoxyphenyl)-6,7,8,9-tetrahydro-5H-5,8-epiminocyclohepta[c]pyridine **(I-32f)** was synthesised from (*R*)-*N*-(1-(3-bromo-5-fluoropyridin-4-yl)pent-4-en-1-yl)-4-methoxyaniline **(I-32e)** using a procedure essentially the same as for **I-13f**. ¹H NMR (400 MHz, DMSO-d6) δ 8.28 (s, 1H), 8.04 (s, 1H), 6.77 - 6.71 (m, 4H), 5.04 (d, *J = 5.6* Hz, 1H), 4.55 (t, *J = 6.4* Hz, 1H), 3.60 (s, 3H), 3.06 (dd, J= 4.8, 4.8 Hz, 1H), 2.52 - 2.47 (m, 1H), 2.35 - 2.24 (m, 2H), 1.90 - 1.85 (m, 1H), 1.78 - 1.73 (m, 1H)

**Step 6:** (5*R*,8*S*)-4-fluoro-6,7,8,9-tetrahydro-5H-5,8-epiminocyclohepta[c]pyridine **(I-32)** was synthesised from (5*R*,8*S*)-4-fluoro-10-(4-methoxyphenyl)-6,7,8,9-tetrahydro-5H-5,8-epiminocyclohepta[c]pyridine **(I-32f)** using a procedure essentially the same as for **I-13.** ¹H NMR (400 MHz, DMSO-d6) δ 8.23 (s, 1H), 8.11 (s, 1H), 4.38 (d, *J =* 1.6 Hz, 1H), 3.76 (t, *J =* 6.4 Hz, 1H), 3.02 (dd, J= 4.2, 4.2 Hz, 1H), 2.56-2.50 (m, 1H), 2.02-1.89 (m, 2H), 1.76 - 1.71 (m, 1H), 1.52 -1.46 (m,1H)

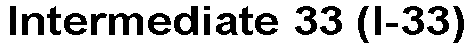

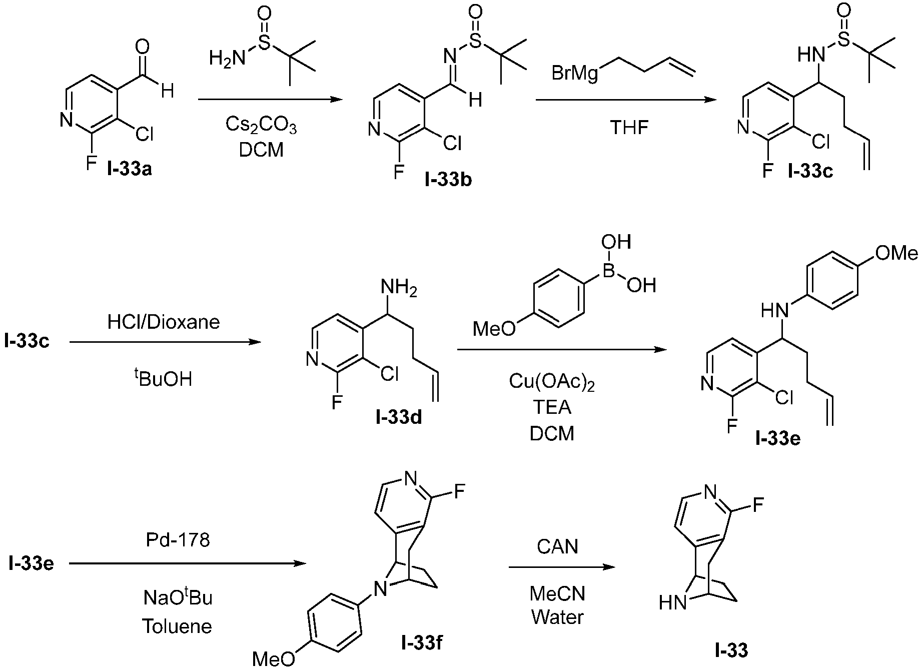

**Step 1**: *N*-((3-Chloro-2-fluoropyridin-4-yl)methylene)-2-methylpropane-2-sulfinamide **(I-33b)** was synthesised from 3-chloro-2-fluoroisonicotinaldehyde **(I-33a)** using a procedure essentially the same as for **I-32b.** ¹H NMR (500 MHz, DMSO-d6) δ 8.81 (s, 1H), 8.33 (d, *J* = 5.1 Hz, 1H), 7.90 (d, *J* = 5.1 Hz, 1H), 1.22 (s, 9H).

**Step 2:** A solution of but-3-en-1-ylmagnesium bromide in THF (133 ml, 0.5 M, 66.55 mmol) was slowly added to a solution of *N*-((3-chloro-2-fluoropyridin-4-yl)methylene)-2-methylpropane-2-sulfinamide **(I-33b)** (14.6 g, 55.46 mmol) in THF (150 ml) at -78 °C. The reaction mixture was allowed to slowly warm to RT over 16 h. The reaction mixture was quenched with staurated ammonium chloride solution (150 ml) and extracted with EtOAc (2 x 200 ml). The organic layers were combined, dried over magnesium sulfate and concentrated *in vacuo* to give *N*-(1-(3-chloro-2-fluoropyridin-4-yl)pent-4-en-1-yl)-2-methylpropane-2-sulfinamide **(I-33c)** as an orange oil. LC-MS (method 2) m/z 319.0, 321.0 (M+H)⁺ (ES⁺); at 2.12 min.

**Step 3:** 1-(3-chloro-2-fluoropyridin-4-yl)pent-4-en-1-amine **(I-33d)** was synthesised from N-(1-(3-chloro-2-fluoropyridin-4-yl)pent-4-en-1-yl)-2-methylpropane-2-sulfinamide **(I-33c)** using a procedure essentially the same as for **I-13d.** ¹H NMR (500 MHz, DMSO-d6) δ 8.16 (dd, *J* = 5.1, 0.9 Hz, 1H), 7.62 (d, *J =* 5.1 Hz, 1H), 5.86 - 5.74 (ddt, *J* = 16.9, 10.2, 6.6 Hz, 1H), 5.02 (dq, *J* = 17.2, 1.8 Hz, 1H), 4.98 - 4.92 (m, 1H), 4.20 (dd, *J* = 8.2, 5.0 Hz, 1H), 3.31 (s, 1H), 2.27 (br s, 2H), 2.20 - 2.02 (m, 1H), 1.69 - 1.51 (m, 2H).

**Step 4:** *N*-(1-(3-Chloro-2-fluoropyridin-4-yl)pent-4-en-1-yl)-4-methoxyaniline **(I-33e)** was synthesised from 1-(3-chloro-2-fluoropyridin-4-yl)pent-4-en-1-amine **(I-33d)** using a procedure essentially the same as for **I-32e.** ¹H NMR (500 MHz, DMSO-d6) δ 8.10 (dd, *J* = 5.2, 0.8 Hz, 1H), 7.41 (d, *J =* 5.1 Hz, 1H), 6.69 - 6.61 (m, 2H), 6.42 - 6.35 (m, 2H), 6.09 (d, *J* = 8.3 Hz, 1H), 5.80 - 5.81 (m, 1H), 5.07 - 4.96 (m, 2H), 4.68 (td, *J* = 8.5, 4.7 Hz, 1H), 3.57 (s, 3H), 2.29 (dd, *J =* 14.3, 8.4 Hz, 1H), 2.24 - 2.13 (m, 1H), 1.86 - 1.70 (m, 2H).

**Step 5:** (±)-1-Fluoro-10-(4-methoxyphenyl)-6,7,8,9-tetrahydro-5H-5,8-epiminocyclohepta[c]pyridine **(I-33f)** was synthesised from *N*-(1-(3-chloro-2-fluoropyridin-4-yl)pent-4-en-1-yl)-4-methoxyaniline **(I-33e)** using a procedure essentially the same as for **I-7f.** ¹H NMR (500 MHz, DMSO-d6) δ 7.95 (d, *J = 5.0* Hz, 1H), 7.28 (d, *J* = 4.9 Hz, 1H), 6.85 - 6.76 (m, 2H), 6.71 (d, *J =* 8.6 Hz, 2H), 4.92 (d, *J* = 5.4 Hz, 1H), 4.56 (s, 1H), 3.61 (s, 3H), 2.92 (d, *J = 16.4* Hz, 1H), 2.35 (d, *J =* 17.4 Hz, 1H), 2.33 - 2.27 (m, 2H), 1.88 - 1.75 (m, 2H)

**Step 6:** (±)-1-Fluoro-6,7,8,9-tetrahydro-5H-5,8-epiminocyclohepta[c]pyridine **(I-33)** was synthesised from (±)-1-fluoro-10-(4-methoxyphenyl)-6,7,8,9-tetrahydro-5H-5,8-epiminocyclohepta[c]pyridine **(I-33f)** using a procedure essentially the same as for **I-14.** ¹H NMR (400 MHz, DMSO-d6) δ 7.91 (dd, *J* = 5.0, 1.0 Hz, 1H), 7.04 (dd, *J* = 5.0, 1.9 Hz, 1H), 4.17 (d, *J* = 5.4 Hz, 1H), 3.78 (t, *J =* 5.9 Hz, 1H), 3.04 - 2.68 (m, 2H), 2.38 (d, *J* = 17.1 Hz, 1H), 1.93 (ddt, *J* = 8.6, 5.9, 2.6 Hz, 2H), 1.73 (t, *J =* 9.0 Hz, 1H), 1.63 - 1.34 (m, 1H).

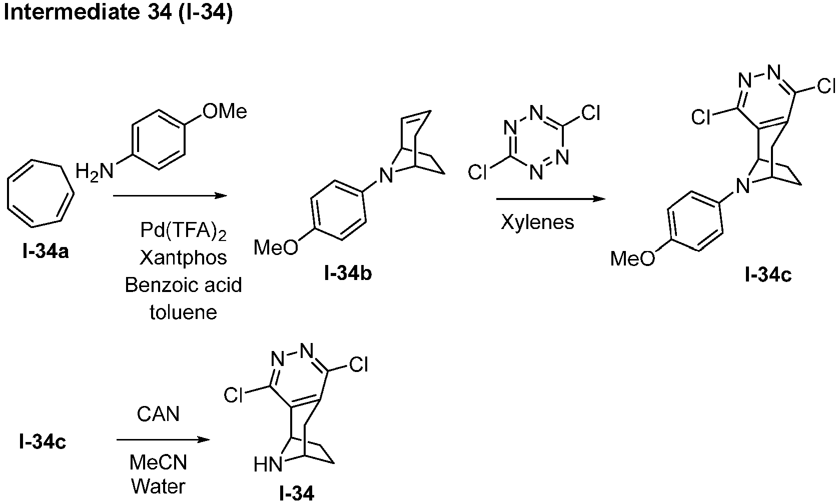

**Step 1:** A flask containing palladium(ll) trifluoroacetate (270 mg, 812 µmol), Xantphos (940 mg, 1.62 mmol) and benzoic acid (496 mg, 4.06 mmol) and the flask was evacuated under vacuum and backfilled with N₂ (3 times). Toluene (100 ml) was added followed by cyclohepta-1,3,5-triene (**I-34a**) (16.8 ml, 162 mmol) and the flask was evacuated under vacuum and backfilled with N₂ (3 times). The mixture was stirred at RT for 2-3 min before a solution of 4-methoxyaniline (5.00 g, 40.6 mmol) in toluene (50 ml) which had been sparged with N₂ for 2 minutes. The reaction was heated at 110 °C for 3 h and left to cool overnight. The organics were washed with saturate sodium bicarbonate solution (100 ml) and brine (100 ml). The organic layer was dried over magnesium sulfate and concentrated *in vacuo.* The product was purified by chromatography on silica gel (0-10% EtOAc/isohexane) to afford (±)-8-(4-methoxyphenyl)-8-azabicyclo[3.2.1]oct-2-ene **(I-34b)** as a clear orange oil. ¹H NMR (500 MHz, CDCl₃) δ 6.88 (d, *J* = 8.6 Hz, 2H), 6.84 - 6.78 (m, 2H), 6.07 (m, 1H), 5.50 - 5.38 (m, 1H), 4.28 - 4.19 (m, 1H), 4.08 (t, *J =* 5.3 Hz, 1H), 3.76 (s, 3H), 2.57 (d, *J* = 18.0 Hz, 1H), 2.42-2.42 (m, 1H), 2.20-2.08 (m, 1H), 2.06 - 1.97 (m, 1H), 1.91 - 1.62 (m, 2H)

**Step 2:** (±)-8-(4-Methoxyphenyl)-8-azabicyclo[3.2.1]oct-2-ene **(I-34b)** (1.03 g, 4.07 mmol) and 3,6-dichloro-1,2,4,5-tetrazine (737 mg, 4.88 mmol) were dissolved in xylenes (40 ml) and the mixture was stirred at 100 °C for 72 h. The reaction mixture was cooled to RT and solvent was concentrated *in vacuo.* The product was purified by chromatography on silica gel (0-50% EtOAc/isohexane) to afford (±)-1,4-dichloro-10-(4-methoxyphenyl)-6,7,8,9-tetrahydro-5H-5,8-epiminocyclohepta[d]pyridazine **(I-34c)** as a pale brown solid. ¹H NMR (400 MHz, DMSO-d6) δ 6.85 - 6.64 (m, 4H), 5.00 (d, *J* = 5.8 Hz, 1H), 4.64 (t, *J =* 5.7 Hz, 1H), 3.62 (s, 3H), 2.91 (dd, *J* = 18.9, 5.0 Hz, 1H), 2.40 - 2.20 (m, 2H), 2.04 - 1.91 (m, 1H), 1.89 - 1.75 (m, 1H) (1H under DMSO peak)

**Step 3:** (±)-1,4-dichloro-6,7,8,9-tetrahydro-5H-5,8-epiminocyclohepta[d]pyridazine **(I-34)** was synthesised (±)-1,4-dichloro-10-(4-methoxyphenyl)-6,7,8,9-tetrahydro-5H-5,8-epiminocyclohepta[d]pyridazine **(I-34c)** using a procedure essentially the same as for **I-14.** ¹H NMR (400 MHz, DMSO-d6) δ 4.31 (d, *J =* 5.1 Hz, 1H), 3.83 (s, 1H), 2.92 (d, *J* = 4.4 Hz, 1H), 2.44 (d, *J* = 18.6 Hz, 1H), 2.07 - 1.93 (m, 2H), 1.82 (t, *J =* 9.2 Hz, 1H), 1.61 - 1.46 (m, 1H). (Exchangeable -NH proton not visible)

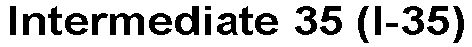

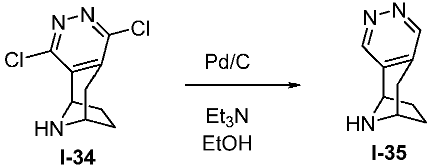

To a solution of (±)-1,4-dichloro-6,7,8,9-tetrahydro-5H-5,8-epiminocyclohepta[d]pyridazine **(I-34)** (84 mg, 0.37 mmol) in EtOH (2 ml) was added Et₃N (200 µl, 1.5 mmol) followed by Pd/C (86 mg, 5% Wt, 37 µmol). The reaction mixture was stirred for 16 h at RT under H₂ (5 bar). The reaction mixture was filtered and concentrated *in vacuo.* The resultant residue was dissolved in DCM (30 ml) and washed with distilled water (5 ml). A solution of 2 M NaOH was added to the aqueous until pH ~10 was reached. The aqueous was extracted with DCM (3 x 20 ml). The combined organics were dried over magnesium sulfate and concentrated *in vacuo* to provide (±)-6,7,8,9-tetrahydro-5H-5,8-epiminocyclohepta[d]pyridazine as a pale yellow oil, which was used without further purification. ¹H NMR (400 MHz, DMSO-d6) δ 8.88 (d, *J =* 1.9 Hz, 2H), 4.16 (d, *J =* 6.1 Hz, 1H), 3.78 (t, *J* = 5.9 Hz, 1H), 2.97 (dd, *J* = 17.9, 5.0 Hz, 1H), 2.51 - 2.43 (m, 1H), 2.02 - 1.86 (m, 2H), 1.75 (t, *J* = 9.1 Hz, 1H), 1.54 - 1.38 (m, 1H). (Exchangeable -NH proton not observed)

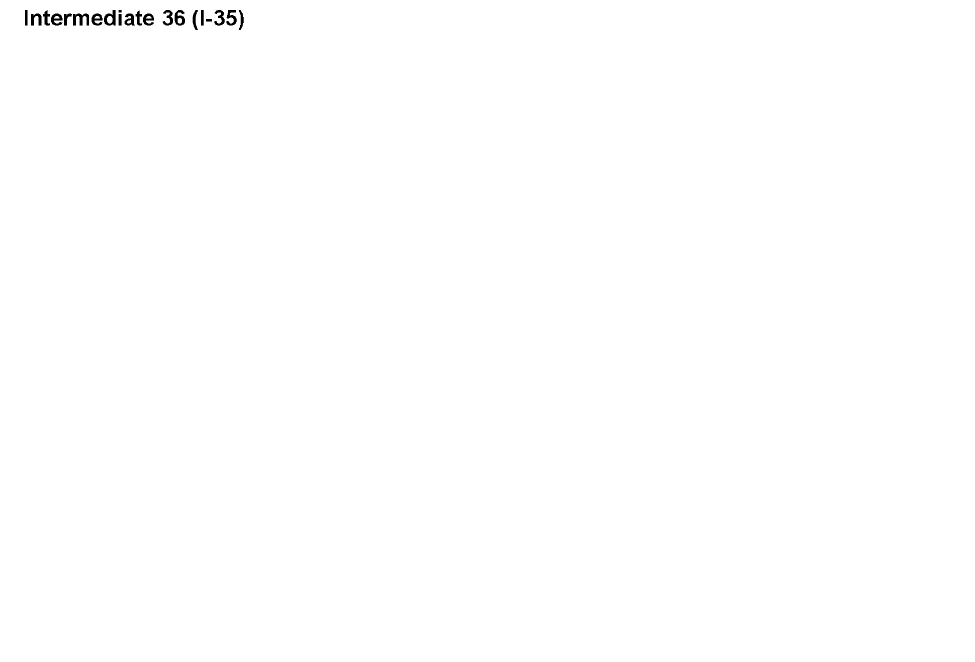

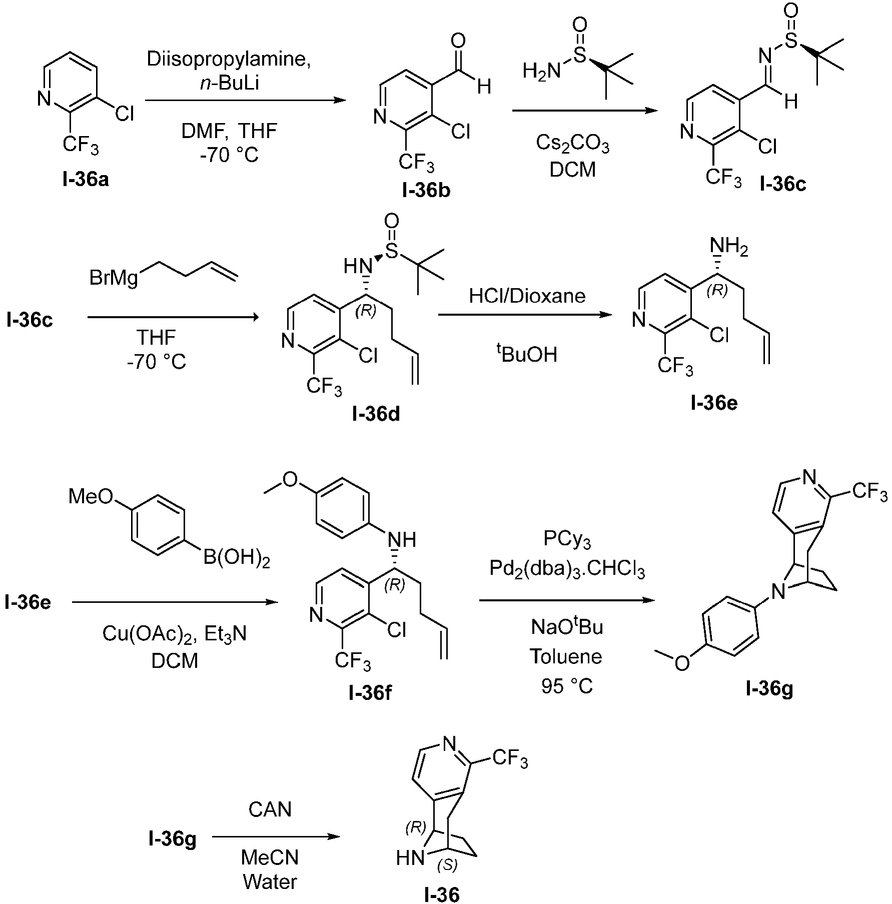

**Step** 1: To a solution of diisopropylamine (9.2 ml, 60 mmol) in dry-THF (20 m) at -70 °C was added a solution of *n*-BuLi in hexanes (2.5 M, 26.5 ml, 60 mmol). The mixture was stirred at -70 °C for 0.5 h. A solution of 3-chloro-2-(trifluoromethyl)pyridine **I-36a** (8 g, 40 mmol) in THF (80 ml) was added to the reaction slowly and the mixture was stirred at -70 °C for 1 h. A solution of DMF (8.5 ml, 110 mmol) in THF (30 ml) was added slowly and the mixture was stirred at -70 °C for 1 h. The reaction was quenched with saturated aqueous ammonium chloride solution (60 ml) and the mixture was extracted with EtOAc (3 x 100 ml). The combined organics were washed with brine, dried over sodium sulfate and concentrated *in vacuo.* The product was purified by chromatography on silica gel (20% EtOAc/ petroleum ether) to give 3-chloro-2-(trifluoromethyl)isonicotinaldehyde **I-36b** as a yellow solid. ¹H NMR: (400 MHz, DMSO-d6) δ 10.39 (s, 1H), 8.90 (d, *J* = 4.8 Hz, 1H), 8.07 (d, *J*= 4.8 Hz, 1H)

**Step 2:** (*S*)-*N*-((3-Chloro-2-(trifluoromethyl)pyridin-4-yl)methylene)-2- methylpropane-2-sulfinamide **I-36c** was synthesised from 3-chloro-2-(trifluoromethyl)isonicotinaldehyde **I-36b** using a procedure essentially the same as for **I-32b.** ¹H NMR: (400 MHz, DMSO-d6) δ 8.89 (s, 1H), 8.82 (d, *J =* 4.8 Hz, 1H), 8.23 (d, *J* = 4.8 Hz, 1H), 1.23 (s, 9H).

**Step 3:** (*S*)-*N*-((*R*)-1-(3-Chloro-2-(trifluoromethyl)pyridin-4-yl)pent-4-en-1-yl)-2-methylpropane-2-sulfinamide **I-36d** was synthesised from (*S*)-*N*-((3-chloro-2-(trifluoromethyl)pyridin-4-yl)methylene)-2- methylpropane-2-sulfinamide **I-36c** using a procedure essentially the same as for **I-13c.** ¹H NMR (400 MHz, DMSO-d6): δ 8.69 (d, *J* = 4.8 Hz, 1H), 7.89 (d, *J* = 4.8 Hz, 1H), 5.93 (d, *J* = 7.2 Hz, 1H), 5.85 - 5.74 (m, 1H), 5.13 - 4.99 (m, 2H), 4.78 - 4.72 (m, 1H), 2.27 - 2.13 (m, 2H), 1.96 - 1.86 (m, 1H), 1.81 - 1.72 (m, 1H), 1.07 (s, 9H).

**Step 4:** (*R*)-1-(3-Chloro-2-(trifluoromethyl)pyridin-4-yl)pent-4-en-1-amine **I-36e** was synthesised from (*S*)-*N*-((*R*)-1-(3-Chloro-2-(trifluoromethyl)pyridin-4-yl)pent-4-en-1-yl)-2-methylpropane-2-sulfinamide **I-36d** using a procedure essentially the same as for **I-32d.** ¹H NMR (400 MHz, DMSO-d6): δ 8.63 (d, *J =* 4.8 Hz, 1H), 8.00 (d, *J* = 4.8 Hz, 1H), 5.85 - 5.75 (m, 1H), 5.05 - 4.93 (m, 2H), 4.30 - 4.27 (m, 1H), 2.22- 2.05 (m, 4H), 1.69 - 1.51 (m, 2H).

**Step 5:** (*R*)-*N*-(1-(3-chloro-2-(trifluoromethyl)pyridin-4-yl)pent-4-en-1-yl)-4-methoxyaniline I-**36f** was synthesised from (*R*)-1-(3-Chloro-2-(trifluoromethyl)pyridin-4-yl)pent-4-en-1-amine **I-36e** using a procedure essentially the same as for **I-32d.** ¹H NMR (400 MHz, DMSO-d6): δ 8.59 (d, *J* = 4.8 Hz, 1H), 7.77 (d, *J* = 4.8 Hz, 1H), 6.66 - 6.63 (m, 2H), 6.40 - 6.36 (m, 2H), 6.13 (d, *J* = 8.4 Hz, 1H), 5.89 - 5.79 (m, 1H), 5.05 - 4.97 (m, 2H), 4.77 (q, *J =* 8.8 Hz, 1H), 3.56 (s, 3H), 2.36 - 2.16 (m, 2H), 1.80 - 1.74 (m, 2H)

**Step 6:** A mixture of (*R*)-*N*-(1-(3-chloro-2-(trifluoromethyl)pyridin-4-yl)pent-4-en-1- yl)-4-methoxyaniline **I-36f** (2 g, 5.4 mmol), NaO^{t}Bu (778 mg, 8.1 mmol), Pd₂(dba)₃.CHCl₃ (559 mg, 0.54 mmol) and tricyclohexyl phosphine (302 mg, 1.08 mmol) in toluene (30 ml) was stirred at 95 °C for 4 h under N₂. The mixture was cooled to RT, filtered, and the filter cake was washed with EtOAc (3 x 20 ml). The filtrate was concentrated *in vacuo* and the product was purified by chromatography on silica gel (10% EtOAc/ petroleum ether) to give (5R,8S)-10-(4-methoxyphenyl)-1-(trifluoromethyl)-6,7,8,9-tetrahydro-5H-5,8-epiminocyclohepta[c]pyridine **I-36g** as a yellow solid. ¹H NMR (400 MHz, DMSO-d6): δ 8.43 (d, *J* = 4.8 Hz, 1H), 7.62 (d, *J* = 4.8 Hz, 1H), 6.84 - 6.80 (m, 2H), 6.73 - 6.69 (m, 2H), 5.00 (d, *J = 6.0* Hz, 1H), 4.57 (t, *J =* 5.2 Hz, 1H), 3.61 (s, 3H), 3.20 (dd, *J'* = 4.8, 4.8 Hz, 1H), 2.58 (d, *J* = 17.6 Hz, 1H), 2.32 - 2.23 (m, 2H), 1.87 - 1.74 (m, 2H)

**Step 7:** (5*R*,8*S*)-1-(trifluoromethyl)-6,7,8,9-tetrahydro-5H-5,8-epiminocyclohepta[c]pyridine **I-36** was synthesised from (5*R*,8*S*)-10-(4-methoxyphenyl)-1-(trifluoromethyl)-6,7,8,9-tetrahydro-5H-5,8-epiminocyclohepta[c]pyridine **I-36g** using a procedure essentially the same as for **I-14.** ¹H NMR (400 MHz, DMSO-d6) δ 8.39 (d, *J* = 4.8 Hz, 1H), 7.37 (d, *J* = 4.8 Hz, 1H), 4.24 (d, *J =* 5.2 Hz, 1H), 3.81 - 3.77 (m, 1H), 3.12 (dd, *J =* 5.2, 5.2 Hz, 1H), 2.64 - 2.59 (m, 1H), 1.98 - 1.91 (m, 2H), 1.73 (t, J= 9.6 Hz, 1H), 1.51 - 1.46 (m, 1H).

### Experimental Scheme 5

### Compound 58 (±)-10-((3,4-Dichlorophenyl)carbamoyl)-6,7,8,9-tetrahydro-5H-5,8-epiminocyclohepta[c]pyridine 2-oxide

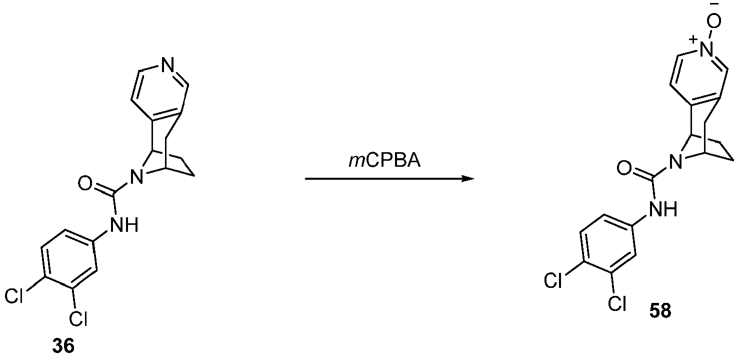

To a solution of (±)-*N*-(3,4-dichlorophenyl)-6,7,8,9-tetrahydro-5H-5,8-epiminocyclohepta[c]pyridine-10-carboxamide **36** (50 mg, 0.14 mmol) in DCM (1 ml) was added *m*CPBA (62 mg, 0.29 mmol, 80% w/w) at 0 °C. The reaction mixture was warmed to RT for 16 h. Water (10 ml) was added and the product was extracted with DCM (3 x 5 ml). The combined organics were washed with brine (10 ml), dried with sodium sulfate, and concentrated *in vacuo.* The product was purified by prep-TLC (eluted with 25% MeOH/EtOAc) to afford (±)-10-((3,4-dichlorophenyl)carbamoyl)-6,7,8,9-tetrahydro-5H-5,8-epiminocyclohepta[c]pyridine 2-oxide **58** as a white solid. LC-MS (method 1) m/z 364.1, 366.1 (M+H)⁺ (ES⁺) at 1.07 min, ¹H NMR: (400 MHz, CDCl₃) δ 8.01 - 7.96 (m, 2 H), 7.60 (d, *J* = 2.7 Hz, 1 H), 7.33 (d, *J* = 8.8 Hz, 1 H), 7.22 (dd, *J* = 8.8, 2.7 Hz, 1 H), 7.04 - 7.00 (m, 1 H), 6.73 (s, 1 H), 5.03 (d, *J = 6.2* Hz, 1 H), 4.66- 4.59 (m, 1 H), 3.37 (dd, *J* = 17.0, 4.9 Hz, 1 H), 2.62 (d, *J* = 17.2 Hz, 1 H), 2.47 - 2.36 (m, 1 H), 2.36 - 2.25 (m, 1 H), 2.00 - 1.91 (m, 1 H), 1.84 - 1.74 (m, 1 H)

The following compounds were prepared using appropriate starting materials in an analogous procedure to that described in Experimental Scheme 5

| Compound | Structure | LCMS method 1 Rt [M+H]⁺ | NMR |
|---|---|---|---|
| 92 | 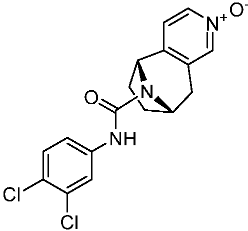 (5*R*,8*S*)-10-((3,4-Dichlorophenyl)carbamoyl)-6,7,8,9-tetrahydro-5H-5,8-epiminocyclohepta[c]pyridine 2-oxide | 364.1, 366.1 at 1.12 min | ¹H NMR (400 MHz, CDCl₃) δ 7.98 (d, *J* = 6.5 Hz, 1H), 7.95 (s, 1H), 7.61 (d, *J* = 2.4 Hz, 1H), 7.35 - 7.30 (m, 1H), 7.26 - 7.21 (m, 1H), 7.08 - 7.01 (m, 2H), 5.06 (d, *J* = 6.2 Hz, 1H), 4.69 - 4.61 (m, 1H), 3.33 (dd, *J* = 4.7, 17.3 Hz, 1H), 2.60 (d, *J* = 17.2 Hz, 1H), 2.46 - 2.24 (m, 2H), 1.98 - 1.90 (m, 1H), 1.82 - 1.71 (m, 1H) |

### Experimental Scheme 6

### Compound 59 (±)-10-((3,4-Dichlorophenyl)carbamoyl)-6,7,8,9-tetrahydro-5H-6,9-epiminocyclohepta[c]pyridine 2-oxide

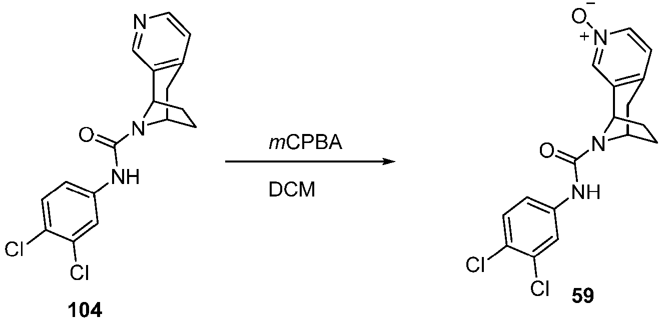

(±)-10-((3,4-Dichlorophenyl)carbamoyl)-6,7,8,9-tetrahydro-5H-6,9-epiminocyclohepta[c]pyridine 2-oxide **59** was synthesised from (±)-*N*-(3,4-dichlorophenyl)-6,7,8,9-tetrahydro-5H-6,9-epiminocyclohepta[c]pyridine-10-carboxamide **59a** using a procedure essentially the same as for **58.** LC-MS (method 1) m/z 364.0, 366.1 at 1.07 min (M+H)⁺ (ES⁺), ¹H NMR: (400 MHz, CDCl₃-*d*) δ 8.06 (s, 2H), 7.91 (dd, *J =* 6.6, 1.5 Hz, 1H), 7.73 (d, *J =* 2.2 Hz, 1H), 7.40-7.35 (m, 1H), 7.33-7.28 (m, 1H), 6.98 (d, *J =* 6.4 Hz, 1H), 5.15 (d, *J =* 5.5 Hz, 1H), 4.82 (t, *J =* 5.8 Hz, 1H), 3.31 (dd, *J =* 17.9, 4.9 Hz, 1H), 2.62 (d, *J* = 17.9 Hz, 1H), 2.42-2.24 (m, 2H), 2.03-1.92 (m, 1H), 1.77-1.67 (m, 1H)

### Experimental Scheme 7

### Compound 60 (±)-2-Chloro-N-(3,4-dichlorophenyl)-6,7,8,9-tetrahydro-5H-5,8-epiminobenzo[7]annulene-10-carboxamide

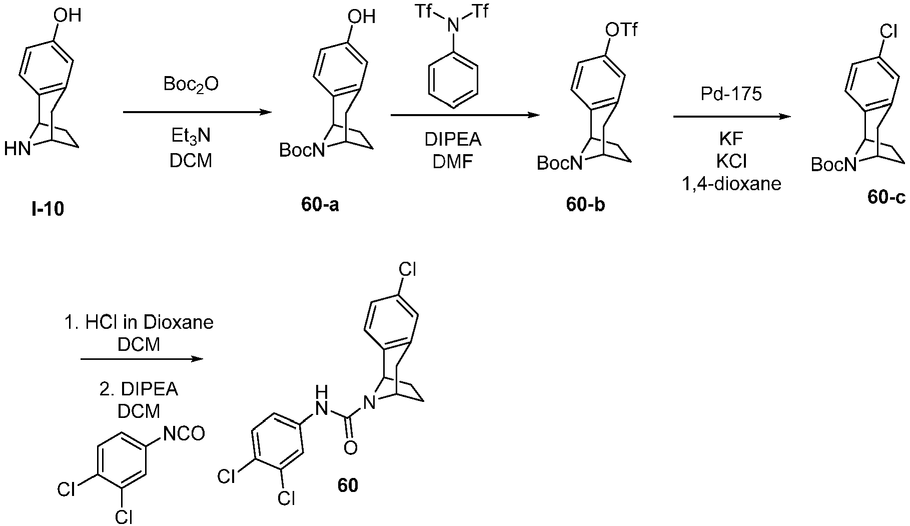

**Step 1:** To a solution of (±)-6,7,8,9-tetrahydro-5H-5,8-epiminobenzo[7]annulen-2-ol **I-10** (176 mg, 0.93 mmol) in DCM (5 ml) was added di-*tert*-butyl dicarbonate (224 mg, 1.03 mmol) at 0 °C, and then warmed up back to RT for 1 h. Saturated sodium bicarbonate solution (10 ml) and the product was extracted with DCM (3 x 10 ml). The organic layers were combined, dried over magnesium sulfate and concentrated *in vacuo* to afford *tert-butyl* (±)-2-hydroxy-6,7,8,9-tetrahydro-5H-5,8-epiminobenzo[7]annulene-10-carboxylate 60a as a pale brown solid. The crude product was used for the next step without further purification. LC-MS (method 2) m/z 274 (M-H)⁻ (ES⁻) at 2.02 min

**Step 2:** To a solution of *tert-butyl* (±)-2-hydroxy-6,7,8,9-tetrahydro-5H-5,8-epiminobenzo[7]annulene-10-carboxylate **60a** (247 mg, 0.83 mmol) in DMF (7.3 ml) was added DIPEA (0.44 ml, 2.50 mmol), followed by 1,1,1-trifluoro-*N*-phenyl-*N-*((trifluoromethyl)sulfonyl)methanesulfonamide (447 mg, 1.25 mmol). The mixture was stirred at RT for 16 h. The reaction mixture was diluted with EtOAc (20 ml) and a 2:1 mixture of water and brine (50 ml) was added. The product was extracted with EtOAc (2 x 20 ml). The organics were combined and washed with water (5 x 20 ml) and brine (20 ml). The organics were dried over magnesium sulfate and concentrated *in vacuo.* The product was purified by chromatography on silica gel (0-50% EtOAc/isohexane) to afford *tert-butyl* (±)-2-(((trifluoromethyl)sulfonyl)oxy)-6,7,8,9-tetrahydro-5H-5,8-epiminobenzo[7]annulene-10-carboxylate **60b** as a pale yellow oil. LC-MS (method 2) m/z 308 (M+H-Boc)⁺ (ES⁺) at 1.86 min, ¹H NMR (500 MHz, DMSO-d6) δ 7.39 - 7.31 (m, 1H), 7.24 (d, *J* = 7.5 Hz, 2H), 4.90 (br s, 1H), 4.37 (br s, 1H), 3.30-3.15 (m, 1H), 2.67 (d, *J =* 17.3 Hz, 1H), 2.24-2.06 (m, 2H), 1.82-1.70 (m, 1H), 1.67 - 1.56 (m, 1H), 1.43-1.22 (m, 9H).

**Step 3:** To a microwave vial, Pd-175 (11 mg, 14 µmol), potassium chloride (34 mg, 0.46 mmol) and potassium fluoride (6.6 mg, 0.11 mmol), were added, which was sealed, then evacuated and back filled with N₂ (3 times). A solution of *tert-*butyl (±)-2-(((trifluoromethyl)sulfonyl)oxy)-6,7,8,9-tetrahydro-5H-5,8-epiminobenzo[7]annulene-10-carboxylate **60b** (93 mg 0.23 mmol) in 1,4-dioxane (3 ml) was added and the resultant mixture was heated to 130 °C for 16 h. The reaction was cooled to RT and filtered through a pad of celite, washing with EtOAc (20 ml). The filtrate was concentrated *in vacuo.* The product was purified by chromatography on silica gel (0-15% EtOAc/isohexane) to afford *tert-butyl* (±)-2-chloro-6,7,8,9-tetrahydro-5H-5,8-epiminobenzo[7]annulene-10-carboxylate **60c** as a colourless oil. LC-MS (method 2) m/z 194.7 (M+H-Boc)⁺ (ES⁺) at 1.82 min, ¹H NMR (500 MHz, CDCl₃) δ 7.16 - 7.02 (m, 2H), 6.98 (d, *J* = 8.1 Hz, 1H), 4.87 (s, 1H), 4.50 (s, 1H), 3.34 (s, 1H), 2.52 (d, *J* = 16.7 Hz, 1H), 2.18 (tdd, *J* = 18.0, 15.8, 9.0 Hz, 2H), 1.88 - 1.74 (m, 1H), 1.73 - 1.53 (m, 1H), 1.40 (s, 9H)

**Step 4.** A solution of HCl in 1,4-dioxane (294 µl, 4 M, 1.17 mmol) was added dropwise to a mixture of *tert-*butyl (±)-2-chloro-6,7,8,9-tetrahydro-5H-5,8-epiminobenzo[7]annulene-10-carboxylate **60c** (11.5 mg, 11.7 µmol) and the solution was stirred for 16 h at RT. A further portion of HCl in 1,4-dioxane (294 µl, 4 M, 1.17 mmol) was added and the reaction mixture was stirred at RT for 24 h. The reaction mixture was concentrated *in vacuo.* The resultant mixture was dissolved in DCM (0.2 ml). 1,2-Dichloro-4-isocyanatobenzene (16.6 mg, 88.1 µmol) and DIPEA (31 µl, 180 µmol) were added. The resultant mixture was stirred at RT for 48 h. The reaction mixture was diluted with 10% MeOH in DCM (2 ml) and washed with water (2 ml). The organic layer was dried over magnesium sulfate and concentrated *in vacuo.* The product was purified by mass directed reverse phase HPLC (45-75% MeCN/(0.1% ammonia in water) to give (t)-2-chloro-*N*-(3,4-dichlorophenyl)-6,7,8,9-tetrahydro-5H-5,8-epiminobenzo[7]annulene-10-carboxamide **60** as a colourless solid. LC-MS (method 1) m/z 381.2, 383.4 (M+H)⁺ (ES⁺) at 1.79 min, ¹H NMR (500 MHz, DMSO-d6) δ 8.90 (s, 1H), 7.84 (d, *J =* 1.8 Hz, 1H), 7.46 (s, 2H), 7.18 (s, 3H), 5.17 (d, *J =* 6.0 Hz, 1H), 4.69 (t, *J* = 6.2, 6.2 Hz, 1H), 3.33 - 3.26 (m, 1H), 2.64 (d, *J =* 17.2 Hz, 1H), 2.27 - 2.06 (m, 2H), 1.78 (t, *J =* 10.4, 10.4 Hz, 1H), 1.67 (dt, *J =* 15.2, 7.6, 7.6 Hz, 1H).

### Experimental Scheme 8

### Compound 61 (±)-1-Amino-N-(3,4-dichlorophenyl)-6,7,8,9-tetrahydro-5H-6,9-epiminocyclohepta[c]pyridine-10-carboxamide

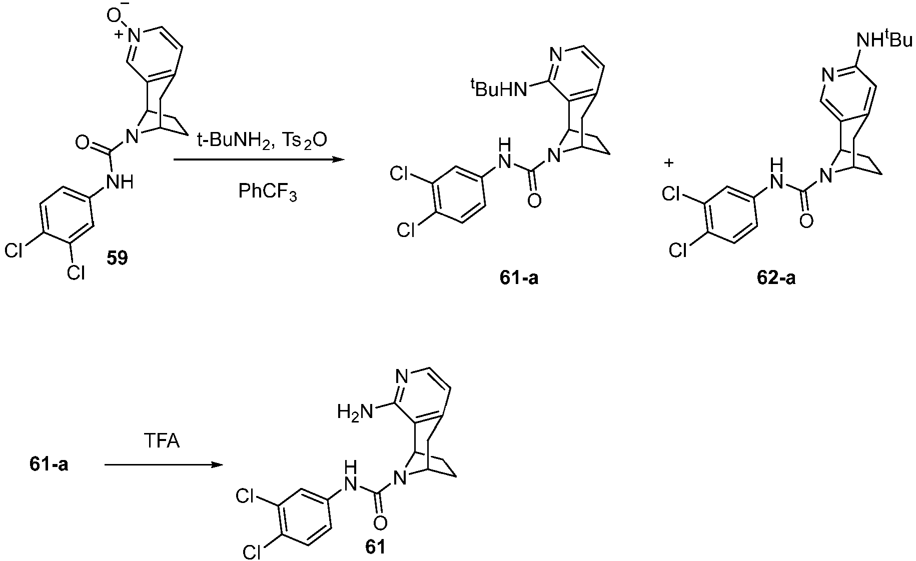

**Step 1:** To a solution of (±)-10-((3,4-dichlorophenyl)carbamoyl)-6,7,8,9-tetrahydro-5H-6,9-epiminocyclohepta[c]pyridine 2-oxide **59** (50 mg, 137 µmol) and *tert*-butylamine (87 µl, 824 µmol,) in trifluorotoluene (2.4 ml) and DCM (0.6 ml) were added p-tolylsulfonyl 4-methylbenzenesulfonate (112 mg, 343 µmol) at 0 °C. The reaction mixture was stirred at 0 °C for 6 h. The mixture was warmed to RT and concentrated *in vacuo* .The product was purified by prep-TLC (50% EtOAc/petroleum ether) and susbsequent purification by prep-HPLC (55-85% MeCN/10 mM NH₄HCO₃) to obtain (±)-1-(*tert*-butylamino)-*N*-(3,4-dichlorophenyl)-6,7,8,9-tetrahydro-5H-6,9-epiminocyclohepta[c]pyridine-10-carboxamide **61-a** as a white solid and (±)-3-(*tert*-butylamino)-*N*-(3,4-dichlorophenyl)-6,7,8,9-tetrahydro-5H-6,9-epiminocyclohepta[c]pyridine-10-carboxamide **62-a** as a white solid.

**61-a** ¹H NMR: (400 MHz, CDCl₃) δ 7.92 (d, *J =* 5.1 Hz, 1H), 7.60 (d, *J =* 2.4 Hz, 1H), 7.31 (d, *J = 8.8* Hz, 1H), 7.17 (dd, *J* = 8.8, 2.7 Hz, 1H), 6.32 (d, *J =* 2.4 Hz, 1H), 6.30 (s, 1H), 4.88 (d, *J* = 5.7 Hz, 1H), 4.54 - 4.45 (m, 1H), 3.94 (br s, 1H), 3.34 (dd, *J* = 17.2, 4.6 Hz, 1H), 2.48 (d, *J =* 17.0 Hz, 1H), 2.44 - 2.22 (m, 1H), 2.28 - 2.16 (m, 1H), 1.96 - 1.88 (m, 1H), 1.82 - 1.72 (m, 1H), 1.51 (s, 9H)
**62-a** ¹H NMR: (400 MHz, CDCl₃) δ 7.83 (s, 1H), 7.61 (d, *J* = 2.4 Hz, 1H), 7.30 (d, *J =* 8.8 Hz, 1H), 7.18 (dd, *J* = 8.7, 2.5 Hz, 1H), 6.35 (s, 1H), 6.20 (s, 1H), 4.79 (d, *J* = 5.1 Hz, 1H), 4.64 (t, *J* = 5.8 Hz, 1H), 4.57 - 4.34 (m, 1H), 3.35 (dd, *J* = 17.2, 4.6 Hz, 1H), 2.51 (d, *J =* 17.2 Hz, 1H), 2.37 - 2.22 (m, 2H), 1.95 (t, *J* = 9.2 Hz, 1H), 1.78 - 1.67 (m, 1H), 1.41 (s, 9H)

**Step 2:** To a solution of (±)-1-(tert-butylamino)-*N*-(3,4-dichlorophenyl)-6,7,8,9-tetrahydro-5H-6,9-epiminocyclohepta[c]pyridine-10-carboxamide **61a** (25 mg, 60 µmol) in DCM (1 ml) was added TFA (0.3 ml, 4.05 mmol) at RT. The resultant mixture was warmed to 50 °C for 6 h. The reaction mixture was concentrated *in vacuo.* The residue was diluted with water (5 ml) and DCM (3 ml). The pH was adjusted to 10 with 50% aqueous NaOH. The product was extracted with DCM (3 x 3 ml). The combined organic layers were washed with brine (2 x 5 ml), dried over sodium sulfate and concentrated *in vacuo.* The product was purified by prep-HPLC (35-55% MeCN/10 mM NH₄HCO₃) to obtain (±)-1-amino-N-(3,4-dichlorophenyl)-6,7,8,9-tetrahydro-5H-6,9-epiminocyclohepta[c]pyridine-10-carboxamide 61 as a white solid. LC-MS (method 1) m/z 363.1, 365.1 (M+H)⁺ (ES⁺) at 1.23 min, ¹H NMR: (400 MHz, CDCl₃) δ 7.78 (d, *J* = 5.5 Hz, 1H), 7.60 (d, *J* = 2.5 Hz, 1H), 7.32 - 7.28 (m, 1H), 7.25 - 7.19 (m, 1H), 6.80 (br s, 1H), 6.51 (d, *J* = 5.50 Hz, 1H), 5.52 - 5.24 (m, 1H), 5.10 (d, *J =* 5.75 Hz, 1H) 4.69 - 4.57 (m, 1H), 3.88 (s, 1H), 3.43 (dd, *J* = 17.7, 4.5 Hz, 1H), 2.56 (d, *J =* 17.7 Hz, 1H), 2.48 - 2.37 (m, 1H), 2.29 (ddd, *J* = 17.9, 11.7, 6.2 Hz, 1H), 1.82 - 1.71 (m, 1H), 2.02-1.95 (m, 1H)

### Experimental Scheme 9

### Compound 62 (±)-3-Amino-N-(3,4-dichlorophenyl)-6,7,8,9-tetrahydro-5H-6,9-epiminocyclohepta[c]pyridine-10-carboxamide

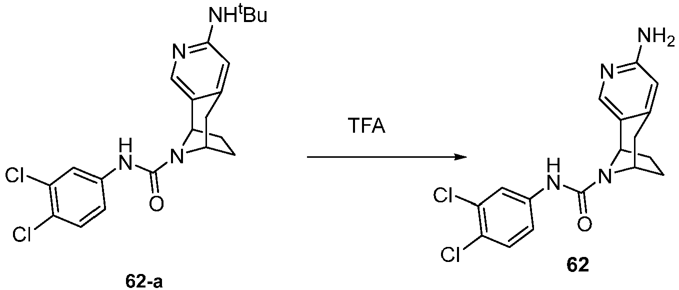

(±)-3-Amino-*N*-(3,4-dichlorophenyl)-6,7,8,9-tetrahydro-5H-6,9-epiminocyclohepta[c]pyridine-10-carboxamide **62** was synthesised from (±)-3-(*tert*-butylamino)-*N*-(3,4-dichlorophenyl)-6,7,8,9-tetrahydro-5H-6,9-epiminocyclohepta[c]pyridine-10-carboxamide **62-a** using a procedure essentially the same as for **61.** LC-MS (method 1) m/z 363.1, 365.1 (M+H)⁺ (ES⁺) at 1.21 min, ¹H NMR: (400 MHz, CDCl₃-*d*) δ 7.77 (s, 1H), 7.60 (d, *J =* 2.43 Hz, 1H), 7.32 - 7.28 (m, 1H), 7.20 (dd, *J* = 8.8, 2.4 Hz, 1H), 6.62 (br s, 1H), 6.28 (s, 1H), 4.86 (d, *J* = 5.5 Hz, 1H), 4.67-4.62 (m, 1H), 3.35 (dd, *J =* 17.4, 4.6 Hz, 1H), 2.53 (d, *J =* 17.4 Hz, 1H), 2.38 - 2.22 (m, 2H), 1.90 (br s, 1H), 1.76 - 1.67 (m, 1H). (NH₂ protons not observed)

### Experimental Scheme 10

### Compound 63 (±)-1-Amino-N-(3,4-dichlorophenyl)-6,7,8,9-tetrahydro-5H-5,8-epiminocyclohepta[c]pyridine-10-carboxamide, Compound 65 (±)-1-(tert-Butylamino)-N-(3,4-dichlorophenyl)-6,7,8,9-tetrahydro-5H-5,8-epiminocyclohepta[c]pyridine-10-carboxamide and Compound 66 (±)-3-(tert-Butylamino)-N-(3,4-dichlorophenyl)-6,7,8,9-tetrahydro-5H-5,8-epiminocyclohepta[c]pyridine-10-carboxamide

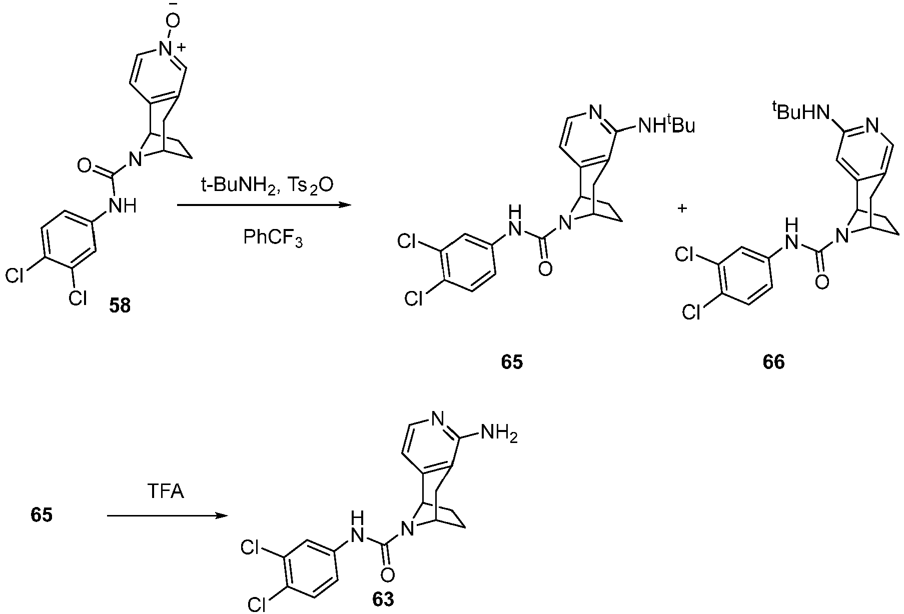

**Step 1:** (±)-1-(*tert*-Butylamino)-*N*-(3,4-dichlorophenyl)-6,7,8,9-tetrahydro-5H-5,8-epiminocyclohepta[c]pyridine-10-carboxamide **65** and (±)-3-(tert-butylamino)-*N*-(3,4-dichlorophenyl)-6,7,8,9-tetrahydro-5H-5,8-epiminocyclohepta[c]pyridine-10-carboxamide **66** were synthesised from (±)-10-((3,4-dichlorophenyl)carbamoyl)-6,7,8,9-tetrahydro-5H-5,8-epiminocyclohepta[c]pyridine 2-oxide **58** using a procedure essentially the same as for **61a** and **62a 65** LC-MS (method 1) m/z 419.2, 421.1 (M+H)⁺ (ES⁺) at 1.77 min, ¹H NMR (400 MHz, CDCl₃) δ 7.96 (d, *J =* 5.1 Hz, 1H), 7.59 (d, *J* = 2.5 Hz, 1H), 7.30 (d, *J =* 8.8 Hz, 1H), 7.18 (dd, *J* = 8.7, 2.5 Hz, 1H), 6.34 (d, *J* = 5.1 Hz, 1H), 6.31 (s, 1H), 4.77 - 4.72 (m, 1H), 4.67 (d, *J* = 5.6 Hz, 1H), 3.89 (s, 1H), 3.01 (dd, *J* = 15.9, 4.8 Hz, 1H), 2.39 - 2.25 (m, 2H), 2.09-1.99 (m, 2 H), 1.80-1.72 (m, 1 H), 1.46 (s, 9 H)
**66** LC-MS (method 1) m/z 419.1, 421.1 (M+H)⁺ (ES⁺) at 1.66 min, ¹H NMR (400 MHz, CDCl₃) δ 7.82 (s, 1H), 7.86 - 7.79 (m, 1H), 7.60 (d, *J* = 2.5 Hz, 1H), 7.33 - 7.30 (m, 1H), 7.23 - 7.18 (m, 1H), 6.33 (s, 1H), 6.19 (s, 1H), 4.83 (d, *J* = 6.1 Hz, 1H), 4.57 (d, *J* = 4.5 Hz, 1H), 3.34 - 3.23 (m, 1H), 2.54 (d, *J* = 16.0 Hz, 1H), 2.39 - 2.20 (m, 2H), 1.95 (t, *J* = 10.2 Hz, 1H), 1.85 - 1.74 (m, 1H), 1.42 (s, 9H).

**Step 2:** (±)-1-Amino-*N*-(3,4-dichlorophenyl)-6,7,8,9-tetrahydro-5H-5,8-epiminocyclohepta[c]pyridine-10-carboxamide **63** was synthesised from (±)-1-(*tert-*butylamino)-*N*-(3,4-dichlorophenyl)-6,7,8,9-tetrahydro-5H-5,8-epiminocyclohepta[c]pyridine-10-carboxamide **65** using a procedure essentially the same as for **61.** LC-MS (method 1) m/z 363.1, 365.1 (M+H)⁺ (ES⁺) at 1.98 min, ¹H NMR (400 MHz, CDCl₃-*d*) δ 7.94 (d, *J =* 5.1 Hz, 1H), 7.58 (d, *J =* 2.4 Hz, 1H), 7.31 (d, *J =* 8.8 Hz, 1H), 7.18 (dd, *J =* 8.7, 2.5 Hz, 1H), 6.51 (d, *J* = 5.1 Hz, 1H), 6.35 (s, 1H), 4.81 - 4.77 (m, 1H), 4.78 (d, *J = 6.2* Hz, 1H), 4.73 (t, *J* = 6.0 Hz, 1H), 4.34 (s, 2H), 3.12 (dd, *J* = 16.1, 4.9 Hz, 1H), 2.43 - 2.26 (m, 2H), 2.22 (d, *J* = 16.1Hz, 1H). 2.06 - 1.98 (m, 1H), 1.83 - 1.74 (m, 1H).

### Experimental Scheme 11

### Compound 64 (±)-3-Amino-N-(3,4-dichlorophenyl)-6,7,8,9-tetrahydro-5H-5,8-epiminocyclohepta[c]pyridine-10-carboxamide

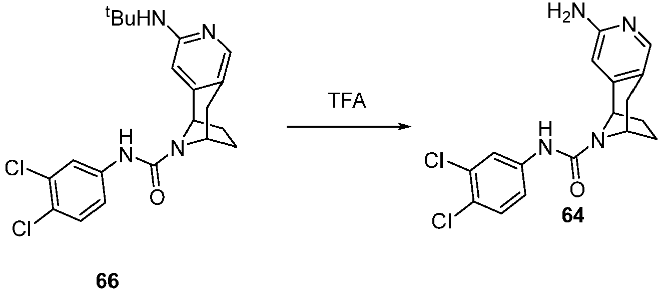

(±)-3-Amino-*N*-(3,4-dichlorophenyl)-6,7,8,9-tetrahydro-5H-5,8-epiminocyclohepta[c]pyridine-10-carboxamide **64** was synthesised from (±)-3-(tert-butylamino)-*N*-(3,4-dichlorophenyl)-6,7,8,9-tetrahydro-5H-5,8-epiminocyclohepta[c]pyridine-10-carboxamide **66** using a procedure essentially the same as for **61.** LC-MS (method 1) m/z 363.1, 365.1 (M+H)⁺ (ES⁺) at 1.21 min, ¹H NMR (400 MHz, CDCl₃) δ 7.83 (s, 1 H), 7.61 (d, *J = 2.4* Hz, 1H), 7.31 (d, *J =* 8.6 Hz, 1H), 7.20 (dd, J = 8.7, 2.5 Hz, 1H), 6.35 (s, 1H), 6.29 (s, 1H), 4.85 (d, *J =* 6.2 Hz, 1H), 4.57 (t, *J* = 6.2 Hz, 1H), 4.33 (s, 2H), 3.31 (dd, J = 16.0, 4.7 Hz, 1H), 2.57 (d, *J* = 16.1 Hz, 1H), 2.41 - 2.21 (m, 2H), 2.00 - 1.90 (m, 1H), 1.84 - 1.75 (m, 1H)

### Experimental Scheme 12

### Compound 67 (±)-N-(3,4-Dichlorophenyl)-3-fluoro-6,7,8,9-tetrahydro-5H-6,9-epiminocyclohepta[c]pyridine-10-carboxamide

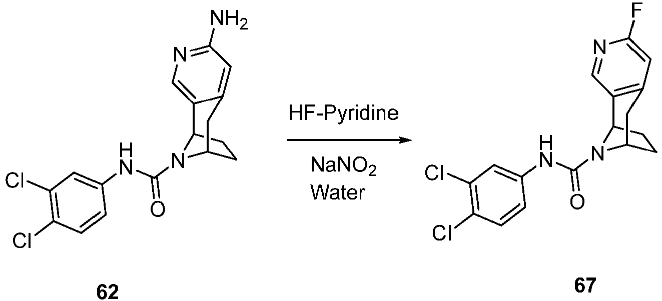

To a solution of (±)-3-amino-*N*-(3,4-dichlorophenyl)-6,7,8,9-tetrahydro-5H-6,9-epiminocyclohepta[c]pyridine-10-carboxamide **62** (10 mg, 27.52 µmol) in HF:Pyridine (0.5 ml, 5.55 mmol, 70% HF) was added a solution of NaNO₂ (4.75 mg, 68.82 µmol) in water (0.25 ml) dropwise at 0 °C for 5 min. After stirring at 0 °C for 0.5 h, the reaction mixture was warmed to RT for 2.5 h. Water (5 ml) was added and the product was extracted with EtOAc (3 x 2 ml). The combined organics were washed with brine (2 x 5 ml), dried over sodium sulfate and concentrated *in vacuo.* The product was purified by prep-HPLC (35-55% MeCN/10 mM NH₄HCO₃) to give (±)-*N*-(3,4-dichlorophenyl)-3-fluoro-6,7,8,9-tetrahydro-5H-6,9-epiminocyclohepta[c]pyridine-10-carboxamide **67** as a white solid. LC-MS (method 1 m/z 364.5, 366.5, 368.4 (M-H)⁻ (ES⁻) at 1.47 min, ¹H NMR: (400 MHz, CDCl₃) δ 8.00 (s, 1H), 7.59 (d, *J = 2.6* Hz, 1H), 7.32 (d, *J* = 8.7 Hz, 1H), 7.18 (dd, *J* = 8.7, 2.5 Hz, 1H), 6.72 (s, 1H), 6.40 (s, 1H), 5.04 (d, *J* = 5.6 Hz, 1H), 4.64 (br t, *J = 6.1* Hz, 1H), 3.48 (dd, *J* = 17.7, 4.3 Hz, 1H), 2.71 (d, *J =* 17.7 Hz, 1H), 2.47 - 2.27 (m, 2H), 2.05 - 1.91 (m, 1H), 1.82 - 1.71 (m, 1H).

### Experimental Scheme 13

### Compound 68 (±)-N-(3,4-Dichlorophenyl)-1-fluoro-6,7,8,9-tetrahydro-5H-6,9-epiminocyclohepta[c]pyridine-10-carboxamide

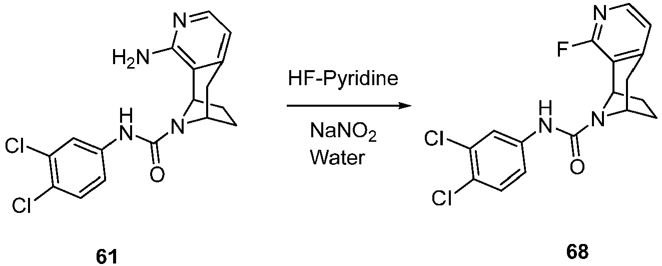

(±)-*N*-(3,4-Dichlorophenyl)-1-fluoro-6,7,8,9-tetrahydro-5H-6,9-epiminocyclohepta[c]pyridine-10-carboxamide **68** was synthesised from (±)-1-amino-N-(3,4-dichlorophenyl)-6,7,8,9-tetrahydro-5H-6,9-epiminocyclohepta[c]pyridine-10-carboxamide **61** using a procedure essentially the same as for **67.** LC-MS (method 1) m/z 366.1, 368.1, (M+H)⁺ (ES⁺) at 1.43 min, ¹H NMR: (400 MHz, CDCl₃) δ 8.01 (dd, *J =* 5.1, 0.7 Hz, 1H), 7.61 (d, *J =* 2.5 Hz, 1H), 7.32 (d, *J =* 8.8 Hz, 1H), 7.19 (dd, *J =* 8.7, 2.6 Hz, 1H), 6.96 (d, *J =* 4.5 Hz, 1H), 6.46 (s, 1H), 5.17 (d, *J* = 5.8 Hz, 1H), 4.75 - 4.68 (m, 1H), 3.49 (dd, *J =* 17.8, 4.7 Hz, 1H), 2.66 (d, *J* = 17.7 Hz, 1H), 2.47 - 2.27 (m, 2H), 2.11 - 1.99 (m, 1H), 1.82 - 1.69 (m, 1H).

### Experimental Scheme 14

### Compound 69 (±)-N-(3,4-Dichlorophenyl)-3-fluoro-6,7,8,9-tetrahydro-5H-5,8-epiminocyclohepta[c]pyridine-10-carboxamide

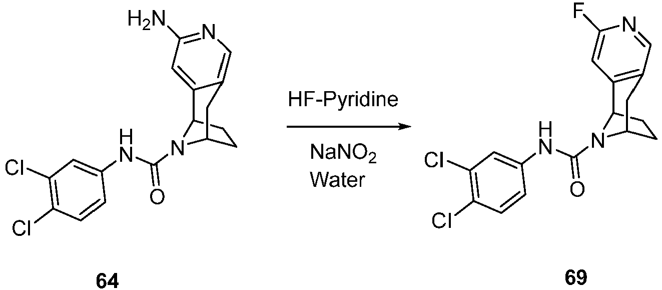

(±)-*N*-(3,4-Dichlorophenyl)-3-fluoro-6,7,8,9-tetrahydro-5H-5,8-epiminocyclohepta[c]pyridine-10-carboxamide **69** was synthesised from (±)-3-amino-*N*-(3,4-dichlorophenyl)-6,7,8,9-tetrahydro-5H-5,8-epiminocyclohepta[c]pyridine-10-carboxamide **64** using a procedure essentially the same as for **67** LC-MS (method 1) m/z 366.1, 368.1 (M+H)⁺ (ES⁺) at 1.42 min, ¹H NMR (400 MHz, CDCl₃) δ 7.97 (s, 1 H), 7.60 (d, *J = 2.6* Hz, 1 H), 7.33 (d, *J =* 8.8 Hz, 1 H), 7.19 (dd, J = 8.7, 2.6 Hz, 1 H), 6.73 (d, *J =* 2.7 Hz, 1 H), 6.38 (s, 1 H), 5.03 (d, *J =* 6.2 Hz, 1 H), 4.65 - 4.57 (m, 1 H), 3.41 (d, *J* = 16.4 Hz, 1 H), 2.70 (d, *J* = 16.6 Hz, 1 H), 2.47 - 2.27 (m, 2 H), 1.94 - 2.03 (m, 1 H), 1.86 - 1.76 (m, 1 H).

### Experimental Scheme 15

### Compound 71 (±)-2-Chloro-N-(3-chloro-4-(trifluoromethyl)phenyl)-6,7,8,9-tetrahydro-5H-5,8-epiminobenzo[7]annulene-10-carboxamide

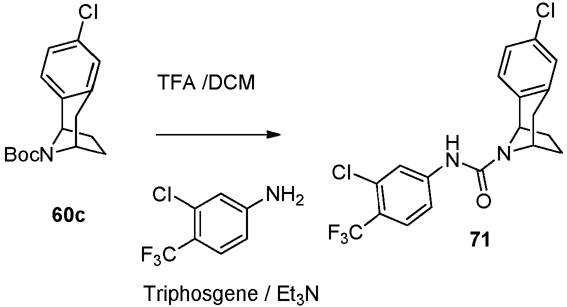

To a solution of *tert-butyl* (±)-2-chloro-6,7,8,9-tetrahydro-5H-5,8-epiminobenzo[7]annulene-10-carboxylate **60c** (19 mg, 65.4 µmol) in DCM (1 ml) was added TFA (50 µl, 654 µmol) dropwise. The resultant mixture was stirred at RT for 16 h. The reaction mixture was concentrated *in vacuo.* The residue was dissolved in DCM (1 ml). In a separate flask a solution of 3-chloro-4-(trifluoromethyl)aniline (14 mg, 71.9 µmol) was added to a solution of triphosgene (9 mg, 30 µmol) in DCM (1 ml).Triethylamine (28 µl, 203 µmol) was added and the resultant mixture was stirred for 10 min. This solution was then added to the TFA salt solution and the mixture was stirred at RT for 18 h. The reaction was diluted with 10% MeOH in DCM solution (8 ml) and sat. sodium bicarbonate solution (5 ml). The product was extracted with 10% MeOH in DCM solution (2 x 8 ml). The combined organic layers were passed through a hydrophobic frit and concentrated under reduced pressure. The product was purified by prep HPLC (45-75% MeCN/0.1% NH₄OH in water) to give (±)-2-chloro-*N*-(3-chloro-4-(trifluoromethyl)phenyl)-6,7,8,9-tetrahydro-5H-5,8-epiminobenzo[7]annulene-10-carboxamide **71** as a colourless solid. LC-MS (method 1) m/z 415.6, 417.5 (M+H)⁺ (ES⁺) at 1.82 min, ¹H NMR (500 MHz, DMSO-d6) δ 9.17 (s, 1H), 7.91 (d, *J* = 2.0 Hz, 1H), 7.69 (d, *J* = 8.8 Hz, 1H), 7.64 - 7.58 (m, 1H), 7.19 (d, *J =* 1.2 Hz, 3H), 5.20 (d, *J* = 6.0 Hz, 1H), 4.73 (t, *J* = 6.4 Hz, 1H), 2.66 (d, *J* = 17.4 Hz, 1H), 2.27 - 2.09 (m, 2H), 1.83 - 1.75 (m, 1H), 1.73 - 1.64 (m, 1H). (1 proton obscured by residual water peak)

### Experimental Scheme 16

### Compound 72 (±)-2-Cyano-N-(3,4-dichlorophenyl)-6,7,8,9-tetrahydro-5H-5,8-epiminobenzo[7]annulene-10-carboxamide

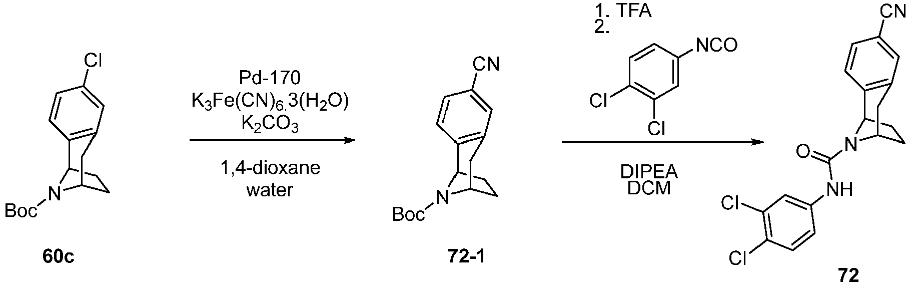

**Step 1:** A vial was charged with Pd-170 (1.0 mg, 1.5 µmol), potassium carbonate (3.4 mg, 25 µmol) and potassium ferrocyanate trihydrate (21 mg, 49 µmol), sealed and evacuated/ backfilled with N₂ (3 times). A solution of *tert-butyl* (±)-2-chloro-6,7,8,9-tetrahydro-5H-5,8-epiminobenzo[7]annulene-10-carboxylate (29 mg, 99 µmol) **60c** in 1,4-dioxane:water (1:1, 0.3 ml) was added and the reaction mixture heated to 120 °C for 17 h. The reaction mixture was cooled to RT and filtered through a pad of celite washing with EtOAc (10 ml). The filtrate was concentrated *in vacuo.* The product was purified by silica gel chromatography (0%-50% EtOAc/isohexane) to give *tert-butyl* (±)-2-cyano-6,7,8,9-tetrahydro-5H-5,8-epiminobenzo[7]annulene-10-carboxylate **(72-1)** as a sticky colourless oil. LC-MS (method 1) m/z 185.3 (M-Boc+H)⁺ (ES⁺) at 1.54 min,

**Step 2:** To a solution of *tert-butyl* (±)-2-cyano-6,7,8,9-tetrahydro-5H-5,8-epiminobenzo[7]annulene-10-carboxylate **(72-1)** (20 mg, 70 µmol) in DCM (1 ml) was added TFA (54 µl, 0.70 mmol) and the reaction mixture stirred at RT for 16 h. The reaction mixture was concentrated *in vacuo.* The resultant mixture was dissolved in DCM (1 ml). 1,2-Dichloro-4-isocyanatobenzene (15 mg, 77 µmol) and DIPEA (37 µl, 210 µmol) were added. The resultant mixture was stirred at RT for 16 h. The reaction mixture was diluted with 10% MeOH in DCM (8 ml) and washed with saturated aqueous sodium bicarbonate solution (5 ml). The aqueous layer was extracted with 10% MeOH in DCM (2 x 8 ml) and the combined organics dried *via* hydrophobic frit and concentrated *in vacuo.* The product was purified by mass directed reverse phase HPLC (35- 65% MeCN/(0.1% ammonia in water) to give (±)-2-cyano-*N*-(3,4-dichlorophenyl)-6,7,8,9-tetrahydro-5H-5,8-epiminobenzo[7]annulene-10-carboxamide **(72)** as colourless solid LC-MS (method 1) m/z 370.3, 372.6 (M-H)⁻ (ES⁻) at 1.57 min, ¹H NMR (500 MHz, DMSO-*d₆*) δ 8.95 (s, 1H), 7.83 (d, *J* = 2.2 Hz, 1H), 7.61 (d, *J* = 9.2 Hz, 2H), 7.50 - 7.41 (m, 2H), 7.37 (d, *J* = 7.7 Hz, 1H), 5.23 (d, *J =* 6.0 Hz, 1H), 4.73 (s, 1H), 2.70 (d, *J* = 17.3 Hz, 1H), 2.18 (dd, *J* = 12.1, 6.4 Hz, 2H), 1.80 (t, *J =* 10.0 Hz, 1H), 1.72 - 1.63 (m, 1H) (urea-NH not observed).

### Experimental Scheme 17

### Compound 76: (±)-N-(3,4-Dichlorophenyl)-3-oxo-3,5,6,7,8,9-hexahydro-2H-6,9-epiminocyclohepta[c]pyridine-10-carboxamide

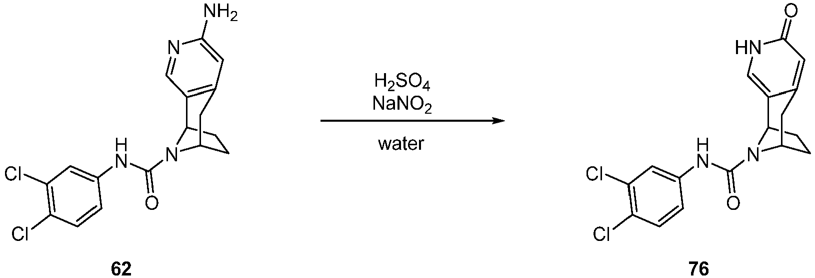

To a solution of (±)-3-amino-*N*-(3,4-dichlorophenyl)-6,7,8,9-tetrahydro-5H-6,9-epiminocyclohepta[c]pyridine-10-carboxamide **62** (5 mg, 13.76 µmol) in H₂SO₄ (0.5 ml, 1.41 mmol, 15%) was added a solution of NaNO₂ (2.85 mg, 41.29 µmol) in water (0.2 ml) dropwise at 0 °C for 5 min. The reaction mixture was stirred at 0 °C for 3 h. The reaction mixture was poured into water (3 ml) and basified with ammonium hydroxide to pH 8 and the mixture was extracted with DCM (3 x 3 ml). The combined organic phase was washed with brine (2 x 5 ml), dried over sodium sulfate and concentrated *in vacuo.* The product was purified by reverse phase HPLC (5-95% (0.018% TFA in MeCN)/(0.037% TFA in water)) to give (±)-*N*-(3,4-dichlorophenyl)-3-oxo-3,5,6,7,8,9-hexahydro-2H-6,9-epiminocyclohepta[c]pyridine-10-carboxamide **76** as a white solid. LC-MS (method 1) m/z 364.1, 366.1 (M+H)⁺ (ES⁺) at 1.09 min, ¹H NMR (400 MHz, CDCl₃-*d*) δ 11.98 - 11.17 (m, 1H), 7.66 (d, *J* = 2.0 Hz, 1H), 7.35 - 7.30 (m, 1H), 7.29 (br d, *J* = 2.5 Hz, 1H), 7.05 (s, 1H), 7.01 (s, 1H), 6.34 (s, 1H), 4.90 (br d, *J =* 5.5 Hz, 1H), 4.57 (t, *J = 6.0* Hz, 1H), 3.24 (d, *J =* 13.8 Hz, 1H), 2.59 (d, *J =* 17.9 Hz, 1H), 2.39 - 2.16 (m, 2H), 1.85 (t, *J =* 10.2 Hz, 1H), 1.78 - 1.68 (m, 1H).

### Experimental Scheme 18

### Compound 77: (±)-1-Chloro-N-(3,4-dichlorophenyl)-6,7,8,9-tetrahydro-5H-5,8-epiminocyclohepta[c]pyridine-10-carboxamide

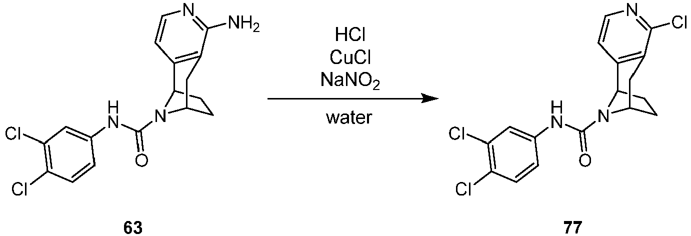

To a solution of (±)-1-amino-*N*-(3,4-dichlorophenyl)-6,7,8,9-tetrahydro-5H-5,8-epiminocyclohepta[c]pyridine-10-carboxamide **(63)** (15 mg, 41.29 µmol) in HCl (1.62 ml, 16.77 mmol, 37% w/w) was added a solution of NaNO₂ (14.25 mg, 206.47 µmol) in water (0.25 ml) and CuCl (4.09 mg, 41.29 µmol) at RT. The reaction mixture was stirred at RT for 3 h. The reaction mixture was quenched with water (5 ml), basified with aqueous NaOH (15% w/w) to pH 7 and extracted with DCM (3 x 3 ml). The combined organic phases were washed with brine (2 x 5 ml), dried over sodium sulfate, and concentrated *in vacuo.* The product was purified by reverse phase HPLC (35-100% MeCN/(10 mM NH₄HCO₃ in water)) to give (±)-1-chloro-*N*-(3,4-dichlorophenyl)-6,7,8,9-tetrahydro-5H-5,8-epiminocyclohepta[c]pyridine-10-carboxamide **77** as a white solid. LC-MS (method 1) m/z 382.0, 384.0 (M+H)⁺ (ES⁺) at 2.63 min, ¹H NMR (400 MHz, CDCl₃) δ 8.22 (d, *J* = 4.9 Hz, 1 H), 7.59 (d, *J = 2.6* Hz, 1 H), 7.32 (d, *J* = 8.8 Hz, 1 H), 7.20 (dd, *J* = 8.8, 2.6 Hz, 1 H), 7.01 (s, 1 H), 7.04 (d, *J* = 4.9 Hz, 1 H), 6.47 (s, 1 H), 5.04 (d, *J* = 6.4 Hz, 1 H), 4.67 - 4.58 (m, 1 H), 3.31 (dd, *J* = 17.7, 4.8 Hz, 1 H), 2.74 - 2.64 (m, 1 H), 2.48 - 2.26 (m, 1 H), 2.02 - 1.93 (m, 1 H), 1.87 - 1.77 (m, 1 H).

### Experimental Scheme 19

### Compound 82: tert-Butyl ((±)-10-((3,4-dichlorophenyl)carbamoyl)-6,7,8,9-tetrahydro-5H-6,9-epiminocyclohepta[c]pyridin-1-yl)carbamate

The following scheme makes use of synthetic methodology described in Fier, Kim and Cohen, J. Am. Chem. Soc., 2020, 142, (19), 8614-8618

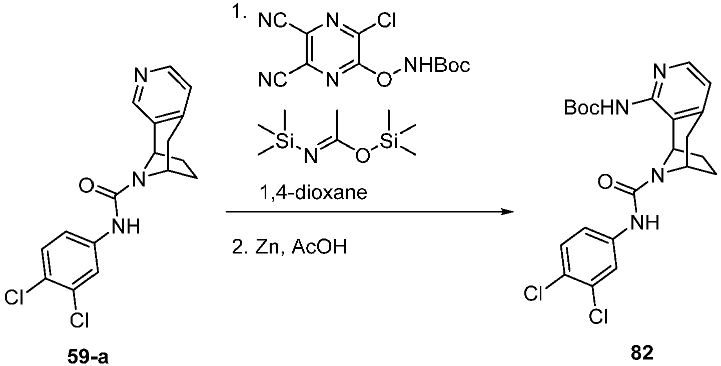

To a solution of (±)-*N*-(3,4-dichlorophenyl)-6,7,8,9-tetrahydro-5H-6,9-epiminocyclohepta[c]pyridine-10-carboxamide **59a** (20 mg, 57.43 µmol) and trimethylsilyl-*N-*trimethylsilylethanimidate (35.05 mg, 172.30 µmol) in 1,4-dioxane (0.23 ml) was added *tert-*butyl ((3-chloro-5,6-dicyanopyrazin-2-yl)oxy)carbamate (25.47 mg, 86.15 µmol) at RT and the reaction mixture was heated at 80 °C for 3 h. After cooling to RT, the reaction mixture was diluted with glacial AcOH (0.23 ml) and zinc (18.8 mg, 287 µmol) was added at once. The reaction mixture was stirred at RT for 3 h. The reaction mixture was quenched with water (5 ml), neutralised with aqueous NaOH (15% w/w) to pH 7, and extracted with EtOAc (3 x 5 ml). The combined organics were washed with brine (10 ml), dried over sodium sulfate, and concentrated *in vacuo.* The product was purified by reverse phase HPLC (45-100% MeCN/(10 mM NH₄HCO₃ in water)) to give *tert-*butyl ((±)-10-((3,4-dichlorophenyl)carbamoyl)-6,7,8,9-tetrahydro-5H-6,9-epiminocyclohepta[c]pyridin-1-yl)carbamate **82** as a yellow solid. LC-MS (method 1) m/z 363.1 (M-Boc+H)⁺ (ES⁺) at 1.69 min, ¹H NMR (400 MHz, CDCl₃) δ 8.76 (s, 1H), 8.15 (br d, *J* = 4.0 Hz, 1H), 7.75 (d, *J = 2.4* Hz, 1H), 7.59 (dd, *J* = 8.8, 2.4 Hz, 1H), 7.29 (s, 1H), 7.27 - 7.26 (m, 1H), 7.26 - 7.26 (m, 1H), 6.97 (br d, *J =* 4.6 Hz, 1H), 6.94 (s, 1H), 5.15 (d, *J = 6.0* Hz, 1H), 4.93 - 4.83 (m, 1H), 3.51 (br dd, *J* = 17.9, 4.9 Hz, 1H), 2.57 (d, *J* = 17.4 Hz, 1H), 2.44 - 2.26 (m, 2H), 1.97 - 1.86 (m, 1H), 1.72 - 1.66 (m, 1H), 1.60 (s, 9H).

### Experimental Scheme 20

### Compound 99: (±)-3-Bromo-N-(3,4-dichlorophenyl)-6,7,8,9-tetrahydro-5H-6,9-epiminocyclohepta[c]pyridine-10-carboxamide

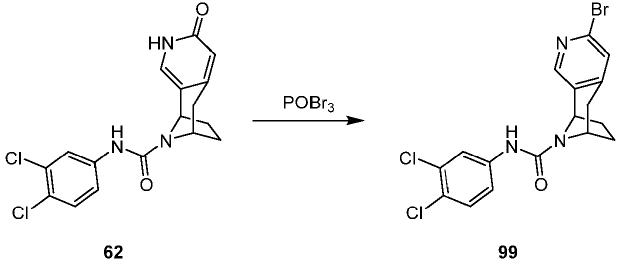

A mixture of (±)-*N*-(3,4-dichlorophenyl)-3-oxo-3,5,6,7,8,9-hexahydro-2H-6,9-epiminocyclohepta[c]pyridine-10-carboxamide **(62)** (5 mg, 2.75 µmol) and POBr₃ (4 mg, 13.75 µmol) was stirred at 110 °C for 2 h. The reaction mixture was cooled to 0 °C and an aqueous solution of NaOH (35% w/w) was added until pH 10 was reached. The mixture was extracted with EtOAc (3 x 5 ml) and the organics were combined, washed with brine (10 ml), dried over sodium sulfate and concentrated *in vacuo.* The product was purified by reverse phase HPLC (50-100% MeCN/(10 mM NH₄HCO₃ in water)) to give (±)-3-bromo-*N-*(3,4-dichlorophenyl)-6,7,8,9-tetrahydro-5H-6,9-epiminocyclohepta[c]pyridine-10-carboxamide **(99)** as white solid. LCMS (method 1) m/z 426.0, 428.0, 430.0 (M+H)⁺ (ES⁺), at 1.51 min, ¹H NMR (400 MHz, CDCl₃) δ 8.16 (s, 1H), 7.59 (d, *J* = 2.4 Hz, 1H), 7.32 (d, *J* = 8.7 Hz, 1H), 7.28 - 7.27 (m, 1H), 7.18 (dd, *J* = 2.6, 8.8 Hz, 1H), 6.38 (br s, 1H), 5.00 (d, *J* = 5.9 Hz, 1H), 4.64 (d, *J* = 5.1 Hz, 1H), 3.44 (dd, *J =* 4.4, 17.9 Hz, 1H), 2.65 (d, *J =* 17.6 Hz, 1H), 2.46 - 2.29 (m, 2H), 1.98 (t, *J =* 9.5 Hz, 1H), 1.83 - 1.71 (m, 1H).

### Experimental Scheme 21

### Compound 100: (±)-3-Chloro-N-(3,4-dichlorophenyl)-6,7,8,9-tetrahydro-5H-6,9-epiminocyclohepta[c]pyridine-10-carboxamide

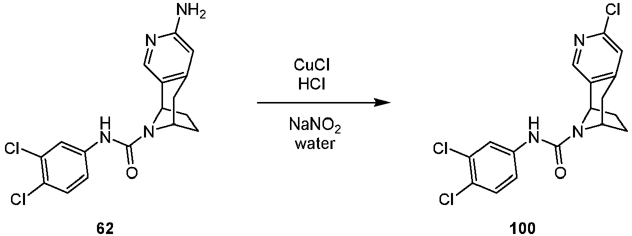

To a solution of (±)-3-amino-*N*-(3,4-dichlorophenyl)-6,7,8,9-tetrahydro-5H-6,9-epiminocyclohepta[c]pyridine-10-carboxamide (62) (3.5 mg, 9.64 umol) and CuCl (954 µg, 9.64 µmol) in HCl (0.5 ml) was added a solution of NaNO₂ (3.3 mg, 48.2 µmol) in water (0.2 ml) dropwise at 0 °C. Then the reaction mixture was stirred at RT for 2 h. The reaction mixture was diluted with water (5 ml) and basified by dropwise addition of ammonium hydroxide to pH 9. The aqueous mixture was extracted with EtOAc (3 x 5 ml) and the combined organics were washed with brine (2 x 5 ml), dried over sodium sulfate and concentrated *in vacuo.* The product was purified by reverse phase HPLC (45-100% MeCN/(10 mM NH₄HCO₃ in water)) to give (±)-3-chloro-*N*-(3,4-dichlorophenyl)-6,7,8,9-tetrahydro-5H-6,9-epiminocyclohepta[c]pyridine-10-carboxamide **(100)** as white solid. LCMS (method 1) m/z 382.0, 384.0, 386.0 (M+H)⁺ (ES⁺), at 1.48 min, ¹H NMR (400 MHz, CDCl₃) δ 8.17 (s, 1H), 7.59 (d, *J* = 2.4 Hz, 1H), 7.32 (d, *J* = 8.8 Hz, 1H), 7.18 (dd, *J* = 2.5, 8.7 Hz, 1H), 7.12 (s, 1H), 6.37 (s, 1H), 5.02 (d, *J =* 5.7 Hz, 1H), 4.63 (t, *J* = 6.0 Hz, 1H), 3.45 (dd, *J* = 4.9, 17.6 Hz, 1H), 2.66 (d, *J =* 17.6 Hz, 1H), 2.48 - 2.27 (m, 2H), 2.05 - 1.91 (m, 1H), 1.83 - 1.69 (m, 1H).

### Experimental Scheme 22

### Compound 109: (±)-N-(3,4-Dichlorophenyl)-1-fluoro-6,7,8,9-tetrahydro-5H-5,8-epiminocyclohepta[c]pyridine-10-carboxamide

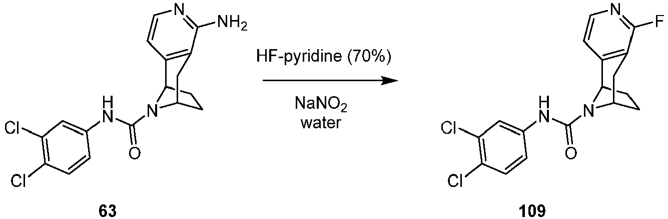

(±)-*N*-(3,4-dichlorophenyl)-1-fluoro-6,7,8,9-tetrahydro-5H-5,8-epiminocyclohepta[c]pyridine-10-carboxamide **109** was synthesised (±)-1-amino-*N*-(3,4-dichlorophenyl)-6,7,8,9-tetrahydro-5H-5,8-epiminocyclohepta[c]pyridine-10-carboxamide **63** using a procedure essentially the same as for **67.** LCMS (method 1) m/z 366.1, 368.0 (M+H)⁺ (ES⁺), at 1.45 min, ¹H NMR (400 MHz, CDCl₃) δ 8.03 (d, *J* = 5.0 Hz, 1H), 7.60 (d, *J* = 2.6 Hz, 1H), 7.33 (d, *J* = 8.8 Hz, 1H), 7.19 (dd, *J* = 8.8, 2.6 Hz, 1H), 7.02 - 6.97 (m, 1 H), 6.38 (s, 1 H), 5.06 (d, *J* = 6.5 Hz, 1H), 4.66 - 4.59 (m, 1 H), 3.31 (dd, *J* = 17.6, 4.28 Hz, 1 H)

### Experimental Scheme 23

### Compound 113: Cis-(±)-N-(3,4-Dichlorophenyl)-9-fluoro-6,7,8,9-tetrahydro-5H-5,8-epiminocyclohepta[d]pyrimidine-10-carboxamide

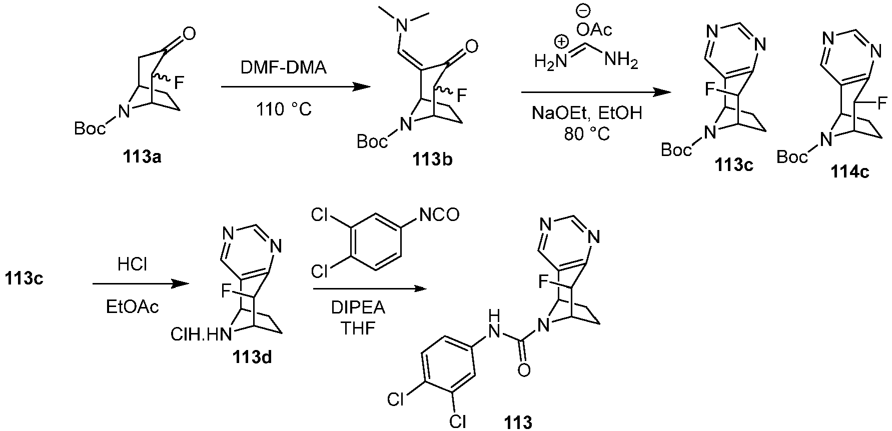

**Step 1:** A solution of *tert*-butyl-2-fluoro-3-oxo-8-azabicyclo[3.2.1]octane-8-carboxylate (220 mg, 904 µmol) and DMF-DMA (3 ml, 23 mmol) was stirred at 110 °C for 3 h. The reaction was cooled to RT and the reaction mixture was poured onto ice (30 ml). The product was extracted with EtOAc (3 x 10 ml). The combined organics were washed with brine (2 x 10 ml), dried over sodium sulfate and concentrated *in vacuo* to give *tert-*butyl-2-((dimethylamino)methylene)-4-fluoro-3-oxo-8-azabicyclo[3.2.1]octane-8-carboxylate **113b** as a yellow oil. The crude product was used in the next step without any further purification.

**Step 2:** To a solution of formamidine acetate (17 mg, 161 µmol) in EtOH (2 ml) was added sodium ethoxide (9.9 mg, 145 µmol) at RT. After stirring at RT for 30 min, a solution of *tert-*butyl-2-((dimethylamino)methylene)-4-fluoro-3-oxo-8-azabicyclo[3.2.1]octane-8-carboxylate **113b** (60 mg, 160 µmol) in EtOH (1 ml) was added and the reaction mixture was stirred at 80 °C for 16 h. The mixture was cooled to RT and poured onto water (50 ml), extracted with EtOAc (3 x 25 ml). The combined organics were washed with brine (2 x 20 ml), dried over sodium sulfate, and concentrated *in vacuo.* The product was purified by prep-TLC (25% EtOAc/ petroleum ether) to obtain *cis-tert-*butyl 9-fluoro-6,7,8,9-tetrahydro-5H-5,8-epiminocyclohepta[d]pyrimidine-10-carboxylate **113 c** as a yellow solid. ¹H NMR (400 MHz, CDCl₃) δ 9.23 (s, 1H), 8.67 (s, 1H), 5.23 (d, *J* = 4.6 Hz, 1H), 5.16 - 4.86 (m, 2H), 2.36 (d, *J* = 10.4 Hz, 1H), 2.17 (br s, 1H), 1.70 - 1.64 (m, 2H), 1.47 - 1.44 (m, 9H)
and *trans-tert-*butyl 9-fluoro-6,7,8,9-tetrahydro-5H-5,8-epiminocyclohepta[d]pyrimidine-10-carboxylate **114c** as a yellow solid. ¹H NMR (400 MHz, CDCl₃-*d*) δ 9.19 (s, 1H), 8.55 (s, 1H), 5.90 - 5.64 (m, 1H), 5.17 - 4.72 (m, 2H), 2.29 - 2.18 (m, 3H), 1.85 - 1.78 (m, 1H), 1.46 - 1.44 (m, 9H)

**Step 3:** A solution of *cis-tert*-butyl 9-fluoro-6,7,8,9-tetrahydro-5H-5,8-epiminocyclohepta[d]pyrimidine-10-carboxylate **113c** (170 mg, 609 µmol) in 4 M HCl/ EtOAc (10 ml, 40 mmol) was stirred at RT for 2 h. The reaction mixture was concentrated *in vacuo* to give *cis*-9-fluoro-6,7,8,9-tetrahydro-5H-5,8-epiminocyclohepta[d]pyrimidine hydrochloride **113d** as a yellow solid. The material was used in the next step without any further purification. ¹H NMR (400 MHz, CD₃OD) δ 9.33 (s, 1H), 8.95 (s, 1H), 5.61 - 5.42 (m, 1H), 5.20 (d, *J* = 5.7 Hz, 1H), 4.78 - 4.70 (m, 1H), 2.51 - 2.39 (m, 2H), 2.12 - 2.04 (m, 1H), 1.91 - 1.79 (m, 1H), (NH not observed)

**Step 4:** To a solution of cis-9-fluoro-6,7,8,9-tetrahydro-5H-5,8-epiminocyclohepta[d]pyrimidine hydrochloride **113d** (120 mg, 556 µmol) in DCM (5 ml) was added 1,2-dichloro-4-isocyanato-benzene (105 mg, 556 µmol) and DIPEA (291 µl, 1.67 mmol) at 0°C. Then the reaction mixture was warmed to RT for 16 h. The reaction mixture was diluted with water (20 ml) and extracted with DCM (3 x 10 ml). The combined organics were washed with brine (2 x 10 ml), dried over sodium sulfate and concentrated *in vacuo.* The product was purified by prep-HPLC (20-50% MeCN/(10 mM NH₄HCO₃ in water) to obtain *cis*-(±)-*N*-(3,4-dichlorophenyl)-9-fluoro-6,7,8,9-tetrahydro-5H-5,8-epiminocyclohepta[d]pyrimidine-10-carboxamide as a white solid. LCMS (method 1) m/z 365.2, 367.2 (M-H)⁻ (ES⁻) at 1.23 min, ¹H NMR (500 MHz, DMSO-d6) δ 9.20 (s, 1H), 9.13 (s, 1H), 8.91 (s, 1H), 7.81 (d, *J* = 2.5 Hz, 1H), 7.48 (d, *J* = 8.9 Hz, 1H), 7.42 (dd, *J* = 8.9, 2.5 Hz, 1H), 5.41 (d, *J = 6.2* Hz, 1H), 5.31 (dd, *J* = 48.5, 1.9 Hz, 1H), 5.21 (t, *J* = 10.1 Hz, 1H), 2.25 (d, *J* = 11.4 Hz, 1H), 2.14 - 1.95 (m, 1H), 1.72 - 1.59 (m, 1H), 1.54 (dt, *J* = 16.2, 8.4 Hz, 1H)

### Experimental Scheme 24

### Compound 114: trans-(±)-N-(3,4-Dichlorophenyl)-9-fluoro-6,7,8,9-tetrahydro-5H-5,8-epiminocyclohepta[d]pyrimidine-10-carboxamide

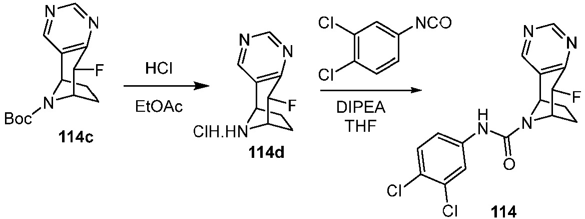

**Step 1:** *trans*-9-Fluoro-6,7,8,9-tetrahydro-5H-5,8-epiminocyclohepta[d]pyrimidine hydrochloride **114d** was synthesised from *trans-tert*-butyl 9-fluoro-6,7,8,9-tetrahydro-5H-5,8-epiminocyclohepta[d]pyrimidine-10-carboxylate **114c** using a procedure essentially the same as for **113d.** ¹H NMR (400 MHz, CD₃OD) δ 9.30 - 9.26 (m, 1H), 8.85 (s, 1H), 6.12 - 5.89 (m, 1H), 5.14 (d, *J* = 5.6 Hz, 1H), 4.72 (br t, *J* = 6.4 Hz, 1H), 2.53 - 2.28 (m, 3H), 2.25 - 2.14 (m, 1H), (NH not observed)

**Step 2:** *trans*-(±)-*N*-(3,4-dichlorophenyl)-9-fluoro-6,7,8,9-tetrahydro-5H-5,8-epiminocyclohepta[d]pyrimidine-10-carboxamide **114** was synthesised from trans-9-fluoro-6,7,8,9-tetrahydro-5H-5,8-epiminocyclohepta[d]pyrimidine hydrochloride **114d** using a procedure essentially the same as for **113.** LCMS (method 1) m/z 365.3, 367.2 (M-H)⁻ (ES⁻) at 1.30 min. ¹H NMR (500 MHz, DMSO-d6) δ 9.19 (s, 1H), 9.15 (s, 1H), 8.79 (d, *J* = 1.1 Hz, 1H), 7.83 (d, *J* = 2.5 Hz, 1H), 7.51 (d, *J =* 8.8 Hz, 1H), 7.44 (dd, *J* = 8.9, 2.5 Hz, 1H), 5.92 (dd, *J* = 49.5, 5.5 Hz, 1H), 5.40 (d, *J* = 4.9 Hz, 1H), 5.03 (t, *J* = 6.0 Hz, 1H), 2.22 - 2.12 (d, *J* = 7.9 Hz, 2H), 2.08 - 1.97 (s, 1H), 1.84 (t, *J* = 9.4 Hz, 1H).

### Separation of enantiomers by chiral chromatography

It will be appreciated that the enantiomers of the compounds described above can be isolated using techniques well known in the art, including, but not limited to, chiral chromatography. For example, a racemic mixture can be dissolved in a solvent, for example, methanol, followed by separation by chiral SFC on a Waters prep 15 with UV detection by DAD at 210 - 400 nm, 40 °C, 120 bar on a Chiralpak^{®} IG (Daicel Ltd.) column (1 x 25 cm, 5µm particle size), flow rate 15ml/ min-1 using 50 % ethanol in 0.1% DEA to afford both enantiomers as the separated pure compounds.

For example, (±)-*N*-(3,4-dichlorophenyl)-6,7,8,9-tetrahydro-5H-5,8-epiminocyclohepta[d]pyrimidine-10-carboxamide (compound **48)** was dissolved to 100 mg/ml in methanol and was then separated by chiral SFC on a Waters prep 15 with UV detection by DAD at 210 - 400 nm, 40 °C, 120 bar on a Chiralpak^{®} IG (Daicel Ltd.) column (1 x 25 cm, 5µm particle size), flow rate 15ml/ min-1 using 50 % ethanol in 0.1% DEA to afford (5R,8S)-N-(3,4-dichlorophenyl)-6,7,8,9-tetrahydro-5H-5,8-epiminocyclohepta[d]pyrimidine-10-carboxamide (compound **52)** and (5S,8R)-N-(3,4-dichlorophenyl)-6,7,8,9-tetrahydro-5H-5,8-epiminocyclohepta[d]pyrimidine-10-carboxamide (compound **53)** both as colourless solids. LCMS and NMR data for compounds **52** and **53** are found in the table below.

Key: (a) m/z from ES⁻

| Compound | Structure | LCMS method 1 Rt [M+H]⁺ | NMR |
|---|---|---|---|
| 52 | 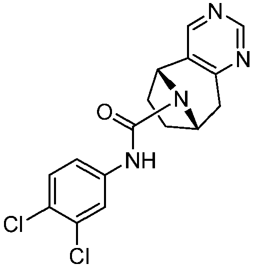 (5R,8S)-N-(3,4-Dichlorophenyl)-6,7,8,9-tetrahydro-5H-5,8-epiminocyclohepta[d]pyrimidine-10-carboxamide | 349.2, 351.2 at 1.52 min | ¹H NMR (500 MHz, DMSO-d6) δ 9.03 (s, 1H), 8.96 (s, 1H), 8.60 (s, 1H), 7.83 (d, *J* = 2.4 Hz, 1H), 7.51 - 7.41 (m, 2H), 5.28 (d, *J* = 6.2 Hz, 1H), 4.80 (t, *J* = 6.5 Hz, 1H), 3.29 (s, 1H), 2.78 (d, *J* = 18.4 Hz, 1H), 2.28 (q, *J* = 11.0, 10.2 Hz, 1H), 2.19 (tt, *J* = 12.1, 6.2 Hz, 1H), 1.94 - 1.85 (m, 1H), 1.76 (dt, *J* = 15.5, 7.7 Hz, 1H) |
| 53 | 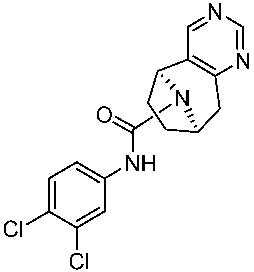 (5*S*,8*R)*-*N*-(3,4-Dichlorophenyl)-6,7,8,9-tetrahydro-5H-5,8-epiminocyclohepta[d]pyrimidine-10-carboxamide | 349.2, 351.2 at 1.52 min | ¹H NMR (500 MHz, DMSO-d6) δ 9.03 (s, 1H), 8.96 (s, 1H), 8.60 (s, 1H), 7.83 (d, *J* = 2.4 Hz, 1H), 7.51 - 7.41 (m, 2H), 5.28 (d, *J* = 6.2 Hz, 1H), 4.80 (t, *J* = 6.5 Hz, 1H), 3.29 (s, 1H), 2.78 (d, *J* = 18.4 Hz, 1H), 2.28 (q, *J* = 11.0, 10.2 Hz, 1H), 2.19 (tt, *J* = 12.1, 6.2 Hz, 1H), 1.94 - 1.85 (m, 1H), 1.76 (dt, *J* = 15.5, 7.7 Hz, 1H) |
| 42 | 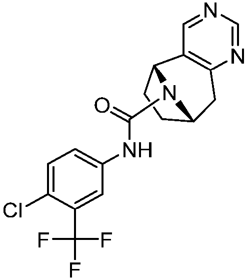 *(*5*R*,8*S)-N-*(4-Chloro-3-(trifluoromethyl)phenyl)-6,7,8,9-tetrahydro-5H-5,8-epiminocyclohepta[d]pyrimidine-10-carboxamide | 383.2, 385.2 at 1.62 min | ¹H NMR (500 MHz, DMSO-d6) δ 9.18 (s, 1H), 8.95 (d, *J* = 6.4 Hz, 1H), 8.60 (d, *J* = 5.8 Hz, 1H), 8.04 (d, *J* = 2.7 Hz, 1H), 7.79 (dd, *J* = 8.9, 2.6 Hz, 1H), 7.57 (dd, *J* = 10.7, 4.6 Hz, 1H), 5.30 (d, *J =* 6.4 Hz, 1H), 4.83 (t, *J =* 6.6 Hz, 1H), 3.36 (s, 1H), 2.78 (dd, *J* = 18.6, 5.8 Hz, 1H), 2.34 - 2.12 (m, 2H), 1.90 (t, *J* = 10.7 Hz, 1H), 1.77 (q, *J* = 9.9, 7.4 Hz, 1H) |
| 38 | 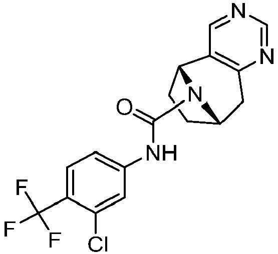 *(*5*R,*8*S)*-*N*-(3-Chloro-4-(trifluoromethyl)phenyl)-6,7,8,9-tetrahydro-5H-5,8-epiminocyclohepta[d]pyrimidine-10-carboxamide | 383.2, 385.2 at 1.61 min | ¹H NMR (500 MHz, DMSO-d6) δ 9.29 (s, 1H), 8.96 (s, 1H), 8.61 (s, 1H), 7.89 (d, *J* = 2.0 Hz, 1H), 7.71 (d, *J* = 8.8 Hz, 1H), 7.63 - 7.58 (m, 1H), 5.31 (d, *J* = 6.2 Hz, 1H), 4.84 (t, *J* = 6.5 Hz, 1H), 3.35 (d, *J* = 5.1 Hz, 1H), 2.82-2.78 (m, 1H), 2.32 - 2.24 (m, 1H), 2.20 (tt, *J* = 12.0, 6.0 Hz, 1H), 1.90 (t, *J =* 10.4 Hz, 1H), 1.76 (d, *J =* 7.8 Hz, 1H). |
| 39 | 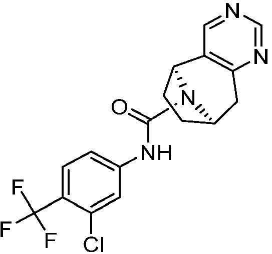 *(*5*S*,8*R)-N*-(3-Chloro-4-(trifluoromethyl)phenyl)-6,7,8,9-tetrahydro-5H-5,8-epiminocyclohepta[d]pyrimidine-10-carboxamide | 383.2, 385.3 at 1.61 min | ¹H NMR (500 MHz, DMSO-d6) δ 9.29 (s, 1H), 8.96 (s, 1H), 8.61 (s, 1H), 7.89 (d, *J* = 2.0 Hz, 1H), 7.71 (d, *J* = 8.8 Hz, 1H), 7.63 - 7.58 (m, 1H), 5.31 (d, *J* = 6.2 Hz, 1H), 4.84 (t, *J* = 6.5 Hz, 1H), 3.35 (d, *J* = 5.1 Hz, 1H), 2.82-2.78 (m, 1H), 2.32 - 2.24 (m, 1H), 2.20 (tt, *J* = 12.0, 6.0 Hz, 1H), 1.90 (t, *J =* 10.4 Hz, 1H), 1.76 (d, *J =* 7.8 Hz, 1H). |
| 43 | 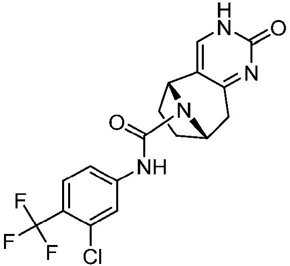 *(*5*R*,8*S)-N*-(3-Chloro-4-(trifluoromethyl)phenyl)-2-oxo-3,5,6,7,8,9-hexahydro-2H-5,8-epiminocyclohepta[d]pyrimidine-10-carboxamide | 399.3, 401.3 at 1.15 min | ¹H NMR (500 MHz, DMSO-d6) δ 11.73 (s, 1H), 9.20 (s, 1H), 7.95 (s, 1H), 7.91 (d, *J =* 2.0 Hz, 1H), 7.72 (d, *J =* 8.8 Hz, 1H), 7.64 - 7.59 (m, 1H), 5.12 (d, *J* = 5.9 Hz, 1H), 4.71 (t, *J =* 6.6 Hz, 1H), 3.10 (dd, *J* = 18.7, 5.0 Hz, 1H), 2.56 (d, *J =* 18.5 Hz, 1H), 2.22 (q, *J* = 11.3, 10.8 Hz, 1H), 2.13 - 2.01 (m, 1H), 1.82 (dd, *J* = 12.0, 9.1 Hz, 1H), 1.74 (dt, *J* = 15.8, 7.9 Hz, 1H) |
| 44 | 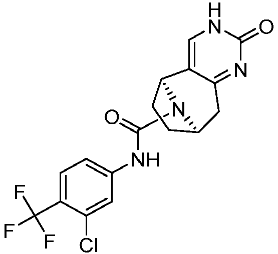 (5*S*,8*R*)-*N*-(3-Chloro-4-(trifluoromethyl) phenyl)-2-oxo-3,5,6,7,8,9-hexahydro-2H-5,8-epiminocyclohepta[d]pyrimidine-10-carboxamide | 399.3, 401.3 at 1.15 min | ¹H NMR (500 MHz, DMSO-d6) δ 11.73 (s, 1H), 9.20 (s, 1H), 7.95 (s, 1H), 7.91 (s, 1H), 7.72 (d, *J =* 8.7 Hz, 1H), 7.62 (d, *J =* 8.8 Hz, 1H), 5.12 (d, *J =* 5.9 Hz, 1H), 4.70 (t, *J =* 6.5 Hz, 1H), 3.10 (dd, *J* = 18.2, 5.1 Hz, 1H), 2.56 (d, *J =* 18.2 Hz, 1H), 2.27 - 2.19 (m, 1H), 2.08 (dq, *J* = 11.7, 5.9 Hz, 1H), 1.82 (t, *J* = 10.8 Hz, 1H), 1.73 (d, *J =* 8.9 Hz, 1H). |
| 105 | 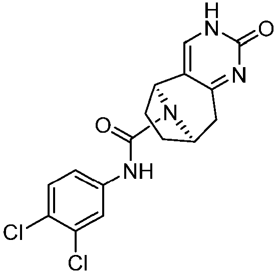 (5*S*,8*R*)-*N*-(3,4-Dichlorophenyl)-2-oxo-3,5,6,7,8,9-hexahydro-2H-5,8-epiminocyclohepta[d]pyrimidine-10-carboxamide | 365.3, 367.3 at 1.06 min | ¹H NMR (500 MHz, DMSO-*d*₆) δ 11.71 (s, 1H), 8.94 (s, 1H), 7.94 (s, 1H), 7.84 (d, *J* = 2.3 Hz, 1H), 7.58 - 7.37 (m, 2H), 5.09 (d, *J* = 5.9 Hz, 1H), 4.77 - 4.59 (m, 1H), 3.08 (dd, *J* = 18.4, 5.0 Hz, 1H), 2.55 (s, 1H), 2.21 (q, *J =* 11.3 Hz, 1H), 2.06 (tt, *J =* 11.7, 6.3 Hz, 1H), 1.81 (t, *J* = 10.5 Hz, 1H), 1.77 - 1.68 (m, 1H). |
| 106 | 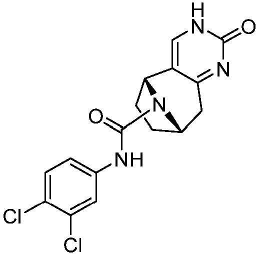 (5*R*,8*S*)-*N*-(3,4-Dichlorophenyl)-2-oxo-3,5,6,7,8,9-hexahydro-2H-5,8-epiminocyclohepta[d]pyrimidine-10-carboxamide | 365.3, 367.3 at 1.06 min | ¹H NMR (500 MHz, DMSO-*d*₆) δ 11.71 (s, 1H), 8.94 (s, 1H), 7.94 (s, 1H), 7.84 (d, *J* = 2.2 Hz, 1H), 7.52 - 7.43 (m, 2H), 5.09 (d, *J* = 5.8 Hz, 1H), 4.73 - 4.62 (m, 1H), 3.08 (dd, *J* = 18.7, 5.1 Hz, 1H), 2.55 (d, *J* = 5.0 Hz, 1H), 2.26 -2.15 (m, 1H), 2.06 (dq, *J =* 11.7, 6.0 Hz, 1H), 1.80 (t, *J* = 10.5 Hz, 1H), 1.74 (d, *J* = 13.3 Hz, 1H). |
| 107 | 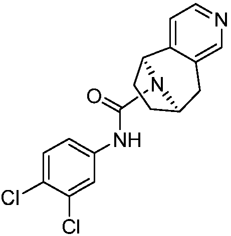 (5*S*,8*R*)-*N*-(3,4-Dichlorophenyl)-6,7,8,9-tetrahydro-5H-5,8-epiminocyclohepta[c]pyridine-10-carboxamide | 347.7, 348.6 at 1.35 min | ¹H NMR (500 MHz, DMSO-*d*₆) δ 8.96 (s, 1H), 8.42 - 8.11 (m, 2H), 7.84 (d, *J* = 2.1 Hz, 1H), 7.58 - 7.32 (m, 2H), 7.18 (d, *J =* 4.9 Hz, 1H), 5.16 (d, *J* = 6.2 Hz, 1H), 4.77 (t, *J* = 6.3 Hz, 1H), 3.29 (d, *J* = 11.8 Hz, 1H), 2.67 (d, *J =* 17.0 Hz, 1H), 2.31 - 2.09 (m, 2H), 1.86 - 1.76 (m, 1H), 1.70 (dt, *J* = 15.1, 7.0 Hz, 1H). |
| 108 | 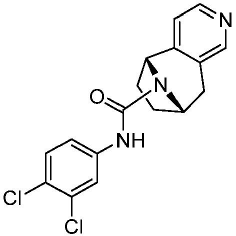 (5*R*,8*S*)-*N*-(3,4-Dichlorophenyl)-6,7,8,9-tetrahydro-5H-5,8-epiminocyclohepta[c]pyridine-10-carboxamide | 347.6, 348.5 at 1.35 min | ¹H NMR (500 MHz, DMSO-*d*₆) δ 8.95 (s, 1H), 8.32 (t, *J* = 2.4 Hz, 2H), 7.84 (d, *J* = 2.0 Hz, 1H), 7.59 - 7.34 (m, 2H), 7.18 (d, *J =* 4.9 Hz, 1H), 5.16 (d, *J* = 6.3 Hz, 1H), 4.88 - 4.68 (m, 1H), 3.29 (dd, *J =* 17.6, 5.4 Hz, 1H), 2.67 (d, *J* = 17.1 Hz, 1H), 2.30 - 2.10 (m, 2H), 1.88 - 1.75 (m, 1H), 1.78 - 1.62 (m, 1H). |
| 110 | 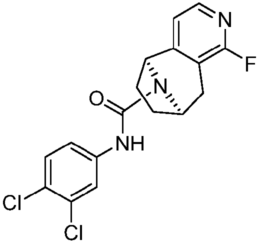 (5*S*,8*R*)-*N*-(3,4-Dichlorophenyl)-1-fluoro-6,7,8,9-tetrahydro-5H-5,8-epiminocyclohepta[c]pyridine-10-carboxamide | 366.1, 368.1 at 1.52 min | ¹H NMR (400 MHz, CDCl₃) δ 8.03 (d, *J* = 4.9 Hz, 1H), 7.60 (d, *J* = 2.4 Hz, 1H), 7.32 (d, *J* = 8.8 Hz, 1H), 7.19 (dd, *J* = 2.5, 8.7 Hz, 1H), 7.01 - 6.95 (m, 1H), 6.45 (s, 1H), 5.06 (d, *J =* 6.2 Hz, 1H), 4.66 - 4.58 (m, 1H), 3.31 (dd, *J =* 17.4, 4.5 Hz, 1H), 2.65 (d, *J* = 17.4 Hz, 1H), 2.49 - 2.26 (m, 2H), 2.04 - 1.94 (m, 1H), 1.83 (ddd, *J* = 12.7, 9.2, 6.3 Hz, 1H) |
| 115 | 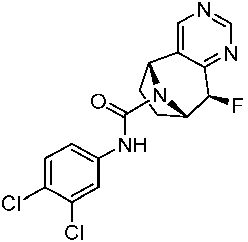 (5*R*,8*S*,9*S*)-*N*-(3,4-Dichlorophenyl)-9-fluoro-6,7,8,9-tetrahydro-5H-5,8-epiminocyclohepta[d]pyrimidine-10-carboxamide | 367.0, 369.4 at 1.22 min | ¹H NMR (500 MHz, DMSO-d6) δ 9.20 (s, 1H), 9.12 (s, 1H), 8.91 (s, 1H), 7.81 (d, *J* = 2.5 Hz, 1H), 7.48 (d, *J* = 8.9 Hz, 1H), 7.42 (dd, *J* = 8.9, 2.5 Hz, 1H), 5.40 (d, *J* = 6.2 Hz, 1H), 5.31 (dd, *J* = 48.4, 1.9 Hz, 1H), 5.21 (t, *J =* 10.1 Hz, 1H), 2.25 (q, *J* = 11.6 Hz, 1H), 2.05 (p, *J =* 8.5, 7.2 Hz, 1H), 1.65 (ddd, *J* = 11.8, 9.0, 2.3 Hz, 1H), 1.54 (dt, *J* = 15.3, 8.0 Hz, 1H). |
| 146 | 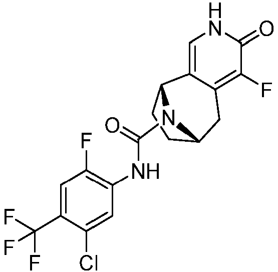 (6*S*,9*R*)-N-(5-Chloro-2-fluoro-4-(trifluoromethyl)phenyl)-4-fluoro-3-oxo-3,5,6,7,8,9-hexahydro-2H-6,9-epiminocyclohepta[c]pyridine-10-carboxamide | 434.3, 436.5 at 1.12 min | ¹H NMR (500 MHz, DMSO-d6) δ 8.06 (d, *J* = 6.9 Hz, 1H), 7.72 (d, *J* = 11.1 Hz, 1H), 7.12 (s, 1H), 5.13 (d, *J* = 6.0 Hz, 1H), 4.67 (t, *J* = 6.5 Hz, 1H), 3.10 (d, *J* = 16.9 Hz, 1H), 2.64 (d, *J* = 17.8 Hz, 1H), 2.17 (q, *J =* 11.2 Hz, 1H), 2.08 (dt, *J* = 11.5, 5.7 Hz, 1H), 1.72 (q, *J =* 15.4, 12.7 Hz, 2H). 2-NH protons not observed |
| 147 | 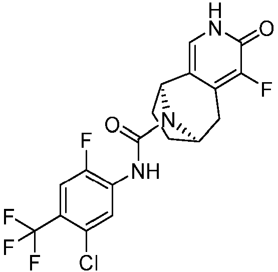 (6*R*,9*S*)-*N*-(5-Chloro-2-fluoro-4-(trifluoromethyl)phenyl)-4-fluoro-3-oxo-3,5,6,7,8,9-hexahydro-2H-6,9-epiminocyclohepta[c]pyridine-10-carboxamide | 434.4, 436.2 at 1.12 min | ¹H NMR (500 MHz, DMSO-d6) δ 8.06 (d, *J* = 6.9 Hz, 1H), 7.73 (d, *J =* 10.9 Hz, 1H), 7.12 (s, 1H), 5.13 (d, *J* = 6.0 Hz, 1H), 4.67 (d, *J* = 8.2 Hz, 1H), 3.10 (d, *J* = 15.7 Hz, 1H), 2.64 (d, *J* = 17.5 Hz, 1H), 2.17 (q, *J =* 10.5 Hz, 1H), 2.10 (dt, *J =* 12.1, 6.0 Hz, 1H), 1.73 (q, *J* = 14.8, 12.2 Hz, 2H). 2-NH protons not observed |
| 196 | 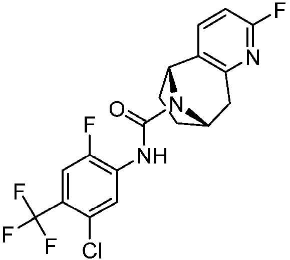 (5*R*,8*S*)-*N*-(5-Chloro-2-fluoro-4-(trifluoromethyl)phenyl)-2-fluoro-6,7,8,9-tetrahydro-5H-5,8-epiminocyclohepta[b]pyridine-10-carboxamide | 416.2, 418.2 at 1.68 min^{(a)} | ¹H NMR (400 MHz, DMSO-d6) δ 9.06 (s, 1H), 8.02 (d, *J* = 6.9 Hz, 1H), 7.84 - 7.72 (m, 2H), 6.94 (dd, *J* = 8.3, 2.5 Hz, 1H), 5.26 (d, *J* = 5.9 Hz, 1H), 4.78 (t, *J* = 6.2 Hz, 1H), 3.39 - 3.32 (m, 1H), 2.68 (d, *J* = 17.8 Hz, 1H), 2.31 -2.09 (m, 2H), 1.89 - 1.78 (m, 1H), 1.79 - 1.66 (m, 1H). |
| 197 | 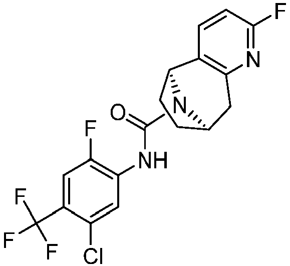 (5*S*,8*R*)-*N*-(5-Chloro-2-fluoro-4-(trifluoromethyl)phenyl)-2-fluoro-6,7,8,9-tetrahydro-5H-5,8-epiminocyclohepta[b]pyridine-10-carboxamide | 416.2, 418.2 at 1.67 min(a) | ¹H NMR (400 MHz, DMSO-d6) δ 9.05 (s, 1H), 8.02 (d, *J =* 6.9 Hz, 1H), 7.85 - 7.72 (m, 2H), 6.94 (dd, *J* = 8.2, 2.6 Hz, 1H), 5.26 (d, *J* = 5.9 Hz, 1H), 4.82 - 4.72 (m, 1H), 3.40 - 3.32 (m, 1H), 2.68 (d, *J* = 17.8 Hz, 1H), 2.29-2.11 (m, 2H), 1.91 - 1.80 (m, 1H), 1.79 1.66 (m, 1H). |

### Experimental Scheme 25

### Compound 133: (5R,8S)-N-(3-Chloro-4-(difluoromethoxy)phenyl)-1-fluoro-6,7,8,9-tetrahydro-5H-5,8-epiminocyclohepta[c]pyridine-10-carboxamide

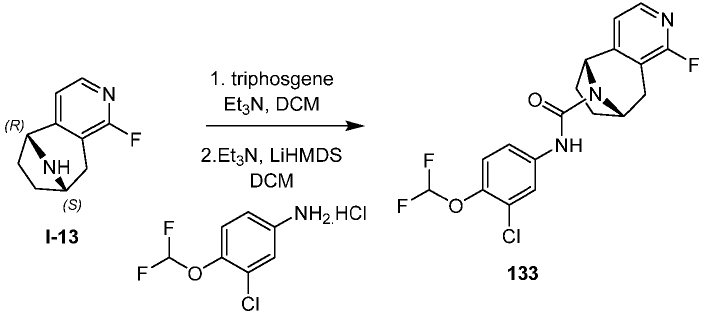

To a solution of (5*R*,8*S*)-1-fluoro-6,7,8,9-tetrahydro-5H-5,8-epiminocyclohepta[c]pyridine **I-13** (30 mg, 0.16 mmol) and Et₃N (65 µl, 0.47 mmol) in DCM (1 ml) was added a solution of triphosgene (46 mg, 0.16 mmol) in 1 ml of DCM. The resultant mixture was stirred at RT for 5 min. In a separate flask, 3-chloro-4-(difluoromethoxy)aniline, HCl (40 mg, 0.17 mmol) was dissolved in DCM (1 ml), Et₃N (48 mg, 65 µl, 3 Eq, 0.47 mmol) was added and the mixture was stirred until a homogenous solution was formed. The carbamoyl chloride solution was added dropwise into the aniline solution. The mixture was stirred at RT for 4 h. After this time LiHMDS (52 mg, 0.31 mmol) was added, and the reaction was left to stir for 18 h. The reaction mixture was concentrated *in vacuo.* The product was purified by chromatography on silica gel (0-70% EtOAc/isohexane) to afford (5*R*,8*S*)-*N*-(3-chloro-4-(difluoromethoxy)phenyl)-1-fluoro-6,7,8,9-tetrahydro-5H-5,8-epiminocyclohepta[c]pyridine-10-carboxamide **133** as a pale brown solid. LCMS (method 1) m/z 398.4, 400.4 (M+H)⁺ (ES⁺) at 1.39 min, ¹H NMR (500 MHz, DMSO-d6) δ 8.95 (s, 1H), 8.01 (d, *J* = 5.0 Hz, 1H), 7.76 (d, *J* = 2.6 Hz, 1H), 7.43 (dd, *J* = 9.0, 2.6 Hz, 1H), 7.30 - 7.16 (m, 2H), 7.05 (t, *J =* 73.6 Hz, 1H), 5.23 (d, *J* = 6.3 Hz, 1H), 4.80 (t, *J* = 6.3 Hz, 1H), 3.21 - 3.07 (m, 1H), 2.58 (d, *J* = 17.3 Hz, 1H), 2.31 - 2.06 (m, 2H), 1.92 - 1.79 (m, 1H), 1.78 - 1.67 (m, 1H).

### Experimental Scheme 26

### Compound 148: (±)-1-Bromo-N-(3,4-dichlorophenyl)-6,7,8,9-tetrahydro-5H-5,8-epiminocyclohepta[c]pyridine-10-carboxamide

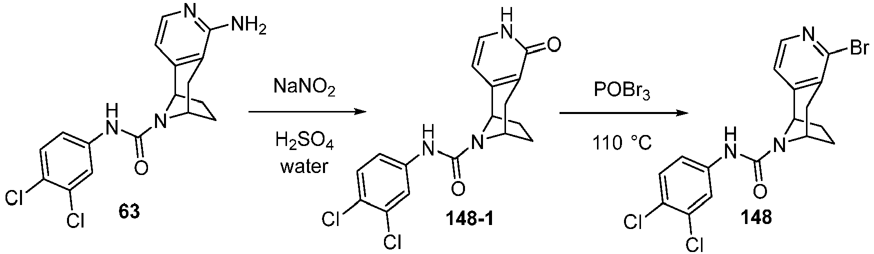

**Step one:** To a solution of (±)-1-amino-*N*-(3,4-dichlorophenyl)-6,7,8,9-tetrahydro-5H-5,8-epiminocyclohepta[c]pyridine-10-carboxamide **compound 63** (7 mg, 19 µmol) in H₂SO₄ (0.8 ml, 15% w/w) was added a solution of NaNO₂ (3 mg, 42 µmol) in water (0.2 ml) dropwise at 0 °C. The reaction mixture was stirred at RT for 6 h. Water (5 ml) was added and the aqueous was basified with an aqueous solution of NaOH (15%) to pH = 8. The product was extracted with EtOAc (3 x 2 ml). The combined organics were washed with brine (2 x 5 ml), dried with sodium sulfate and concentrated *in vacuo.* The product was purified by prep-HPLC (20-55% MeCN/10 mM NH₄HCO₃) to obtain (±)-*N*-(3,4-dichlorophenyl)-1-oxo-2,5,6,7,8,9-hexahydro-1H-5,8-epiminocyclohepta[c]pyridine-10-carboxamide **148-1** as a white solid. ¹H NMR (400 MHz, CDCl₃) δ 11.03 (br s, 1H), 7.65 (d, *J* = 2.2 Hz, 1H), 7.34 - 7.30 (m, 1H), 7.24 - 7.17 (m, 2H), 6.63 (br s, 1H), 6.12 (d, *J =* 6.6 Hz, 1H), 4.87 (d, *J* = 6.4 Hz, 1H), 4.56 - 4.47 (m, 1H), 3.11 (br dd, *J* = 4.2, 17.9 Hz, 1H), 2.49 (d, *J =* 17.9 Hz, 1H), 2.44 - 2.32 (m, 1H), 2.24 (tt, *J* = 6.1, 12.0 Hz, 1H), 2.05 - 1.94 (m, 1H), 1.86 - 1.75 (m, 1H)

**Step two:** A solution of (±)-*N*-(3,4-dichlorophenyl)-1-oxo-2,5,6,7,8,9-hexahydro-1H-5,8-epiminocyclohepta[c]pyridine-10-carboxamide (8 mg, 22 µmol) and phosphorus(V) oxybromide (31 mg, 110 µmol) was stirred at 110 °C for 3 h. The reaction mixture was poured onto water (5 ml), basified with an aqueous solution of NaOH (15% w/w) to pH = 9. The product was extracted with EtOAc (3 x 3 ml). The combined organics were washed with brine (2 x 5 ml), dried with sodium sulfate and concentrated *in vacuo.* The product was purified by prep-HPLC (40-70% MeCN/10 mM NH₄HCO₃) to obtain (±)-1-bromo-*N*-(3,4-dichlorophenyl)-6,7,8,9-tetrahydro-5H-5,8-epiminocyclohepta[c]pyridine-10-carboxamide **148** as a white solid. LCMS (method 1) m/z 428.1, 430.1 (M+H)⁺ (ES⁺) at 1.55 min, ¹H NMR (400 MHz, CDCl₃) δ 8.20 (d, *J* = 4.9 Hz, 1H), 7.60 (d, *J =* 2.4 Hz, 1H), 7.33 (d, *J* = 8.6 Hz, 1H), 7.20 (dd, *J* = 2.5, 8.7 Hz, 1H), 7.06 (d, *J =* 4.9 Hz, 1H), 6.43 (s, 1H), 5.01 (d, *J* = 6.4 Hz, 1H), 4.65 - 4.58 (m, 1H), 3.28 (dd, *J* = 5.2, 17.5 Hz, 1H), 2.66 (d, *J =* 17.6 Hz, 1H), 2.47 - 2.27 (m, 2H), 2.01 - 1.92 (m, 1H), 1.88 - 1.78 (m, 1H)

### Experimental Scheme 27

### Compound 149: (5R,8S)-N-(4,5-Dichloro-2-hydroxyphenyl)-1-fluoro-6,7,8,9-tetrahydro-5H-5,8-epiminocyclohepta[c]pyridine-10-carboxamide

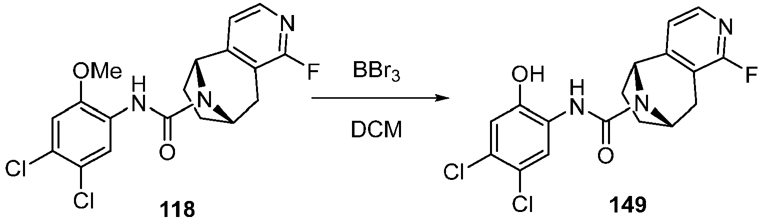

A solution of (5*R*,8*S*)-*N*-(4,5-dichloro-2-methoxyphenyl)-1-fluoro-6,7,8,9-tetrahydro-5H-5,8-epiminocyclohepta[c]pyridine-10-carboxamide **118** (20.0 mg, 50.5 µmol) in DCM (0.5 ml) was cooled down to -78°C. A solution of tribromoborane in DCM (66 µl, 1 M, 65.6 µmol) was added dropwise at -78°C, and the mixture was stirred at -78 °C for 5 min, before warming to 0 °C for 1 h. The reaction mixture was cooled down to -78°C and anhydrous MeOH (1 ml) was added. The reaction mixture was warmed to RT for 30 min. The reaction mixture was concentrated *in vacuo.* The product was purified by chromatography on RP Flash C18 (5-40% MeCN/10 mM aqueous ammonium bicarbonate) to afford (5*R*,8*S*)-*N-*(4,5-dichloro-2-hydroxyphenyl)-1-fluoro-6,7,8,9-tetrahydro-5H-5,8-epiminocyclohepta[c]pyridine-10-carboxamide **149** as a pale yellow solid. LCMS (method 1) m/z 382.1, 384.0 (M+H)⁺ (ES⁺) at 0.74 min. 1H NMR (500 MHz, DMSO-d6) δ 8.15 (s, 1H), 8.00 (d, *J* = 5.0 Hz, 1H), 7.71 (s, 1H), 7.21 (dd, *J* = 5.1, 1.6 Hz, 1H), 6.98 (s, 1H), 5.20 (d, *J* = 6.2 Hz, 1H), 5.00 - 4.52 (m, 1H), 3.19 (dd, *J* = 17.3, 5.0 Hz, 1H), 2.57 (d, *J =* 17.3 Hz, 1H), 2.21 (dtd, *J* = 23.8, 12.1, 7.2 Hz, 2H), 1.91 - 1.80 (m, 1H), 1.79 - 1.69 (m, 1H), 1 exchangeable proton not visible.

### Experimental Scheme 28

### Compound 150: (6S,9R)-N-(3,4-Dichlorophenyl)-2-methyl-3-oxo-3,5,6,7,8,9-hexahydro-2H-6,9-epiminocyclohepta[c]pyridine-10-carboxamide

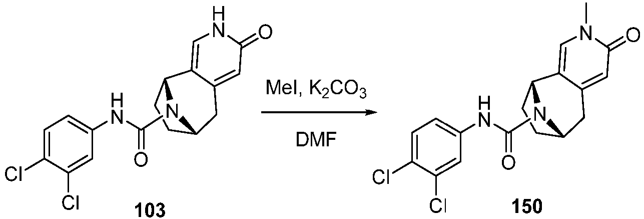

To a solution of (6*S*,9*R*)-*N*-(3,4-dichlorophenyl)-3-oxo-3,5,6,7,8,9-hexahydro-2H-6,9-epiminocyclohepta[c]pyridine-10-carboxamide (35 mg, 96 µmol) in DMF (2 ml) was added Mel (6.0 µl, 96 µmol) and K₂CO₃ (40 mg, 0.29 mmol). The reaction mixture was stirred for 16 h. A further portion of Mel (3 ul, 48 µmol) was added and the reaction mixture was stirred for a further 16 h. A solution of saturated aqueous sodium bicarbonate solution (5 ml) was added and the product was extracted with 20% 0.7 M Ammonia/MeOH in DCM. The organics were concentrated *in vacuo.* The product was purified by chromatography on RP Flash C18 (15-50% MeCN/10 mM Ammonium Bicarbonate) to afford (6S,9R)-N-(3,4-dichlorophenyl)-2-methyl-3-oxo-3,5,6,7,8,9-hexahydro-2H-6,9-epiminocyclohepta[c]pyridine-10-carboxamide **150** as a clear white solid. LCMS (method 1) m/z 378.3, 380.3 (M+H)⁺ (ES⁺) at 1.16 min. ¹H NMR (500 MHz, DMSO-d6) δ 8.88 (s, 1H), 7.84 (d, *J =* 2.1 Hz, 1H), 7.51 (s, 1H), 7.50 - 7.42 (m, 2H), 6.16 (s, 1H), 5.03 (d, *J* = 6.0 Hz, 1H), 4.60 (t, *J =* 6.4 Hz, 1H), 3.35 (s, 3H), 3.10 (dd, *J* = 17.9, 5.0 Hz, 1H), 2.56 (d, *J =* 17.8 Hz, 1H), 2.17 (t, *J =* 10.5 Hz, 1H), 2.08 (tt, *J =* 12.0, 6.5 Hz, 1H), 1.73 (t, *J* = 10.4 Hz, 1H), 1.64 (d, *J =* 8.3 Hz, 1H).

### Determination of Absolute Stereochemistry

The absolute stereochemistry of compounds according to the invention was determined using X-ray diffraction.

### Description of Equipment and Data Collection

Data was obtained using a Rigaku Oxford Diffraction XtaLAB Synergy four-circle diffractometer equipped with a HyPix-6000HE area detector.
Cryogenic system: Oxford Cryostream 800
Cu: *λ*=1.54184 Å, 50W, Micro focus source with multilayer mirror (µ-CMF).
Distance from the crystal to the CCD detector: d = 35 mm
Tube Voltage: 50 kV
Tube Current: 1 mA

An example of stereochemistry determination using X-ray diffraction for compounds 52 and 53 are shown below. It will be appreciated that similar methods can be used to identify the absolute stereochemistry of other compounds.

### Compound 52: (5R,8S)-N-(3,4-dichlorophenyl)-6,7,8,9-tetrahydro-5H-5,8-epiminocyclohepta[d]pyrimidine-10-carboxamide

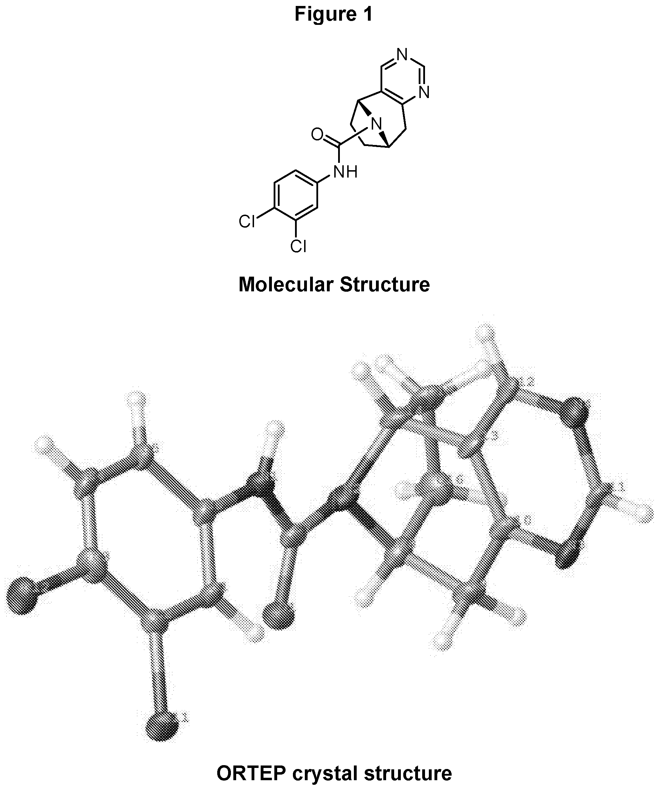

A total of 13407 reflections were collected in the 2θ range from 10.794 to 133.11. The limiting indices were: -12 ≤ h ≤ 12, -7 ≤ k ≤ 7, -13 ≤ l ≤ 12; which yielded 2535 unique reflections (Rint = 0.0996). The structure was solved using SHELXT (Sheldrick, G. M. 2015. Acta Cryst. A71, 3-8) and refined using SHELXL (against F²) (Sheldrick, G. M. 2015. Acta Cryst. C71, 3-8). The total number of refined parameters was 208, compared with 2535 data. All reflections were included in the refinement. The goodness of fit on F² was 1.038 with a final R value for [I > 2σ (I)] R₁ = 0.0934 and wR₂= 0.2203. The largest differential peak and hole were 1.28 and -0.56 Å⁻³, respectively. The solved Oak Ridge Thermal Ellipsoid Plot (ORTEP) crystal structure for compound **52** is shown in Figure 1.

### Compound 53: (5S,8R)-N-(3,4-dichlorophenyl)-6,7,8,9-tetrahydro-5H-5,8-epiminocyclohepta[d]pyrimidine-10-carboxamide

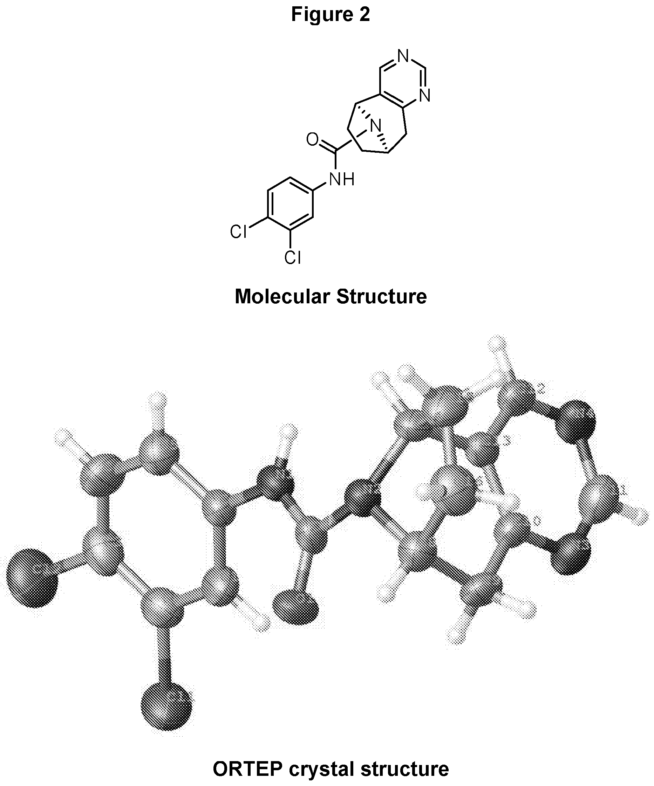

A total of 11160 reflections were collected in the 2θ range from 7.75 to 133.182. The limiting indices were: -12 ≤ h ≤ 12, -7 ≤ k ≤ 7, -13 ≤ l ≤ 10; which yielded 2527 unique reflections (Rint = 0.0914). The structure was solved using SHELXT (Sheldrick, G. M. 2015. Acta Cryst. A71, 3-8) and refined using SHELXL (against F²) (Sheldrick, G. M. 2015. Acta Cryst. *C71*, 3-8). The total number of refined parameters was 208, compared with 2527 data. All reflections were included in the refinement. The goodness of fit on F² was 1.066 with a final R value for [I > 2σ (I)] R₁ = 0.0947 and wR₂ = 0.2243. The largest differential peak and hole were 0.87 and -0.39 Å⁻³, respectively. The solved Oak Ridge Thermal Ellipsoid Plot (ORTEP) crystal structure for compound **53** is shown in Figure 2.

### Human GPR65 cyclic adenosine monophosphate (cAMP) Homogeneous Time Resolved Fluorescence (HTRF) antagonist assay procedure

IC₅₀ data was obtained by the following procedure:
1321N1 human astrocytoma cells stably expressing human recombinant GPR65 (1321N1-hrGPR65 cells, EuroscreenFast) were cultured according to the vendor's instructions. Compounds were tested for their ability to antagonise GPR65, through measuring the concentration of cytoplasmic cAMP following treatment of the cells at a pH of 7.2 to activate GPR65 signalling and addition of the compound to be tested. The extent to which the expected rise in cAMP concentration upon GPR65 activation was suppressed by the added compound is indicative of its potency. The assay was carried out according to EuroscreenFast assay methodology as follows.

On the day of the assay, test compounds were added to 384-well, low volume, white microtiter plates by acoustic dispensing. KRH buffer (5 mM KCI, 1.25 mM MgSO₄, 124 mM NaCl, 25 mM HEPES, 13.3 mM Glucose, 1.25 mM KH₂PO₄ and 1.45 mM CaCl₂) was adjusted to pH 6.5, pH 7.6 and pH 8.4 by adding NaOH. 1321N1-hGPR65 cells were rapidly thawed and diluted in KRH, pH 7.6 prior to centrifugation at 300 xg for 5 min and resuspension in assay buffer (KRH, pH 7.6, supplemented with 1 mM 3-isobutyl-1-methylxanthine (IBMX) and 200 µM ethylenediaminetetraacetic acid (EDTA)). Cells were added to assay plates at a density of 2,000 cells per well in a volume of 5 µl. Assay plates were briefly centrifuged at 100 xg and then incubated at room temperature for 30 min. Cells were stimulated by the addition of 5 µL KRH, pH 6.5, to achieve an assay pH of 7.2, while control wells received 5 µl KRH, pH 8.4 to achieve an assay pH of 7.9. Assay plates were briefly centrifuged at 100 xg and then incubated at room temperature for 30 min. Accumulation of cAMP was detected by cAMP HTRF kit (Cisbio). d2-labeled cAMP and cryptate-labeled anti-cAMP antibody in Lysis and Detection Buffer (Cisbio) were added to assay plates, and the plates were incubated at room temperature for 1 h. HTRF measurements were performed using a Pherastar FSX instrument. Acceptor and donor emission signals were measured at 665 nm and 620 nm, respectively, and HTRF ratios were calculated as signal_{665 nm}/signal₆₂₀ₙₘ x 10⁴. Data were normalised to high and low control values and fitted with 4-parameter logistic regression to determine hGPR65 IC50 values for the test compounds, which are shown in Table 1.

**Table 1: Activity of selected compounds according to the invention**

| | |
|---|---|
| **1** | Medium |
| **2** | High |
| **3** | Medium |
| **4** | Medium |
| **5** | Low |
| **6** | Low |
| **7** | High |
| **8** | Low |
| **11** | Medium |
| **12** | Medium |
| **13** | Low |
| **15** | High |
| **16** | High |
| **17** | High |
| **18** | High |
| **24** | Low |
| **25** | Low |
| **26** | Medium |
| **27** | Low |
| **28** | Low |
| **29** | Medium |
| **31** | High |
| **32** | High |
| **34** | Medium |
| **35** | High |
| **36** | High |
| **37** | Medium |
| **38** | High |
| **39** | Low |
| **42** | High |
| **43** | High |
| **44** | Low |
| **45** | High |
| **46** | High |
| **47** | Medium |
| **48** | High |
| **49** | Low |
| **50** | Low |
| **51** | Medium |
| **52** | High |
| **53** | Low |
| **54** | Medium |
| **55** | Medium |
| **56** | Medium |
| **57** | Medium |
| **58** | High |
| **59** | Low |
| **60** | High |
| **61** | Medium |
| **62** | Low |
| **63** | Low |
| **64** | Medium |
| **65** | Medium |
| **66** | Medium |
| **67** | Medium |
| **68** | Medium |
| **69** | Medium |
| **71** | Medium |
| **72** | Medium |
| **73** | Low |
| **74** | High |
| **75** | High |
| **76** | High |
| **77** | High |
| **78** | High |
| **79** | High |
| **80** | Medium |
| **81** | Medium |
| **82** | Medium |
| **83** | Medium |
| **84** | Medium |
| **85** | High |
| **86** | Low |
| **87** | Low |
| **88** | High |
| **91** | High |
| **92** | High |
| **93** | High |
| **94** | High |
| **95** | Medium |
| **98** | High |
| **99** | Low |
| **100** | Medium |
| **101** | High |
| **103** | High |
| **104** | Medium |
| **105** | Low |
| **106** | High |
| **107** | Low |
| **108** | High |
| **109** | High |
| **110** | Low |
| **111** | Medium |
| **112** | Low |
| **113** | Medium |
| **114** | Medium |
| **115** | Medium |
| **116** | Medium |
| **117** | High |
| **118** | Medium |
| **119** | High |
| **120** | High |
| **122** | High |
| **123** | High |
| **124** | High |
| **125** | High |
| **126** | High |
| **127** | High |
| **128** | High |
| **129** | High |
| **130** | Medium |
| **131** | Medium |
| **132** | Medium |
| **133** | High |
| **134** | High |
| **135** | Medium |
| **136** | Medium |
| **137** | Medium |
| **138** | High |
| **139** | High |
| **140** | High |
| **141** | High |
| **142** | High |
| **143** | High |
| **144** | High |
| **145** | High |
| **146** | High |
| **147** | High |
| **148** | Medium |
| **149** | High |
| **150** | Medium |
| **151** | High |
| **152** | Medium |
| **153** | High |
| **154** | Medium |
| **155** | Medium |
| **156** | High |
| **157** | High |
| **158** | High |
| **159** | Medium |
| **160** | High |
| **161** | Medium |
| **162** | Medium |
| **163** | High |
| **164** | High |
| **165** | Medium |
| **166** | High |
| **167** | High |
| **168** | High |
| **169** | Medium |
| **170** | High |
| **171** | High |
| **172** | High |
| **173** | Medium |
| **174** | High |
| **175** | Medium |
| **176** | Medium |
| **177** | High |
| | |
| **178** | High |
| **179** | Medium |
| **180** | High |
| **181** | High |
| **182** | High |
| **183** | Medium |
| **184** | High |
| **185** | High |
| **186** | Medium |
| **187** | Medium |
| **188** | Medium |
| **189** | High |
| **190** | High |
| **191** | High |
| **192** | High |
| **193** | Medium |
| **194** | Medium |
| **195** | Medium |
| **196** | High |
| **197** | Medium |
| **198** | Medium |
| **199** | High |
| **200** | Medium |

| | |
|---|---|
| High = IC50 < 500 nM; Medium = IC50 > 500 nM and < 5 µM; Low > 5 µM | |

### REFERENCES

Bohn, T. et al. (2018). Tumor immunoevasion via acidosis-dependent induction of regulatory tumor-associated macrophages. Nature Immunology, 1319-1326.

Damaghi, M. et al. (2013). pH Sensing and Regulation in Cancer. Frontiers in Physiology*.*

Gaublomme, J. et al. (2015). Single-Cell Genomics Unveils Critical Regulators of Th17 Cell Pathogenicity. Cell, 1400-1412.

Hernandez, J. (2018). GPR65, a critical regulator of Th17 cell pathogenicity, is regulated by the CRTC2/CREB pathway. The Journal of Immunology, 200 (Supplement).

Korn, T. et al. (2009). IL-17 and Th17 Cells. Annual Reviews in Immunology, 485-517.

Wang, J. et al. (2004). TDAG8 is a proton-sensing and psychosine-sensitive G-protein-coupled receptor. Journal of Biological Chemistry, 45626-45633.

Yoshida, N. et al. (2016). ICER is requisite for Th17 differentiation. Nature Communications, 12993.

Hardin, M. et al. (2014). The clinical and genetic features of COPD-asthma overlap syndrome. Eur Respir J. 2014 Aug;44(2):341-50.

Kottyan, L. et al. (2009). Eosinophil viability is increased by acidic pH in a cAMP-and GPR65-dependent manner. Blood. 2009 Sep 24;114(13):2774-82.

Tsurumaki, H. et al (2015). Int J Mol Sci. Protective Role of Proton-Sensing TDAG8 in Lipopolysaccharide-Induced Acute Lung Injury. Dec 4;16(12):28931-42.

Schultz and Wolfe (2011), Organic Letters, Intramolecular Alkene Carboamination Reactions for the Synthesis of Enantiomerically Enriched Tropane Derivatives , 13 (11), 2962-2965

Fier, Kim and Cohen (2020), J. Am. Chem. Soc., 142, (19), 8614-8618

## Claims

1. A compound of formula (If), or a pharmaceutically acceptable salt or solvate thereof, 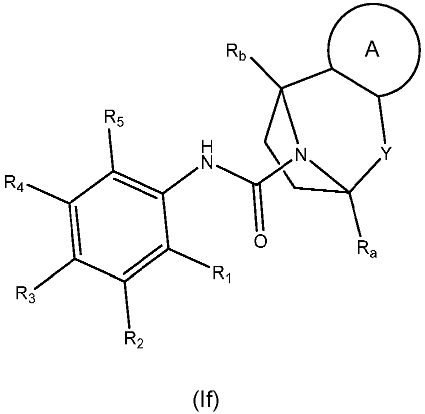 wherein:
ring A is a pyridinyl ring or tautomer thereof, or a phenyl ring, each of which may be optionally substituted with one or more substituents selected from H, F, Cl, Br, I, CN, alkoxy, NR₁₁R₁₁', OH, alkyl, phenyl, and haloalkyl;
Y is selected from C=N-OH and CR₁₀R₁₀', wherein R₁₀ and R₁₀' are each independently selected from H, F, alkyl, and haloalkyl;
Rₐ and R_{b} are each independently selected from H and alkyl;
R₁, R₄, and R₅ are each independently selected from H, CN, alkyl, alkoxy, haloalkyl, OH, F, Cl, Br, and I;
R₂ and R₃ are each independently selected from H, F, Cl, Br, I, CN, alkoxy, haloalkyl, haloalkoxy, alkyl, aryl, heteroaryl, O-aryl, NHCO-alkenyl and CO₂-alkyl, wherein said aryl, heteroaryl and O-aryl groups are each optionally further substituted by one or more groups independently selected from halo, alkyl and alkoxy; and
R₁₁ and R₁₁' are each independently selected from H, alkyl, haloalkyl, COR₁₂, CO₂R₁₂ and SO₂R₁₃, wherein R₁₂ and R₁₃ are both independently alkyl.

2. A compound according to claim 1 wherein ring A is selected from the following: 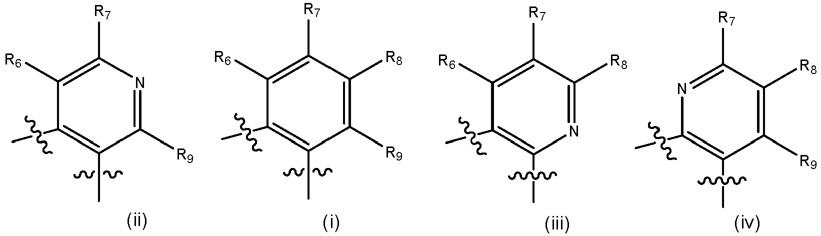 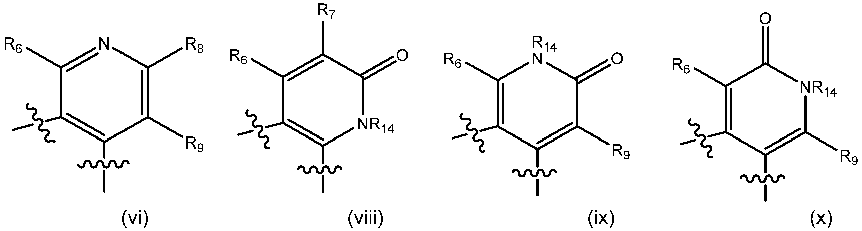 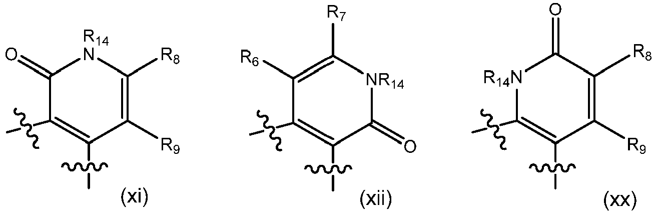 wherein R₆, R₇, R₈, and R₉ are each independently selected from H, F, Cl, Br, I, CN, alkoxy, NR₁₁R₁₁', OH, alkyl, phenyl, and haloalkyl, and R₁₄ is H or alkyl, more preferably H.

3. A compound of formula (Ib), or a pharmaceutically acceptable salt or solvate thereof, 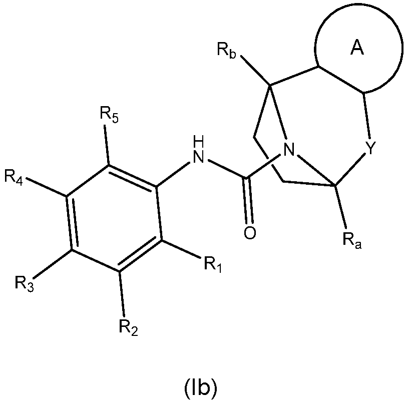 wherein:
ring A is selected from the groups (i)-(xx): 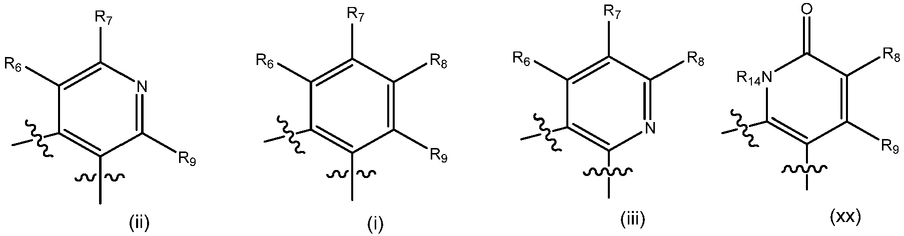 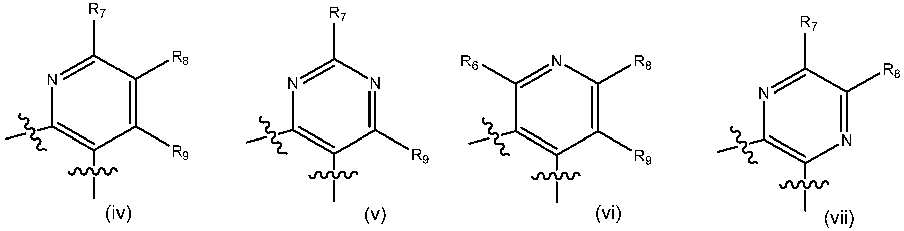 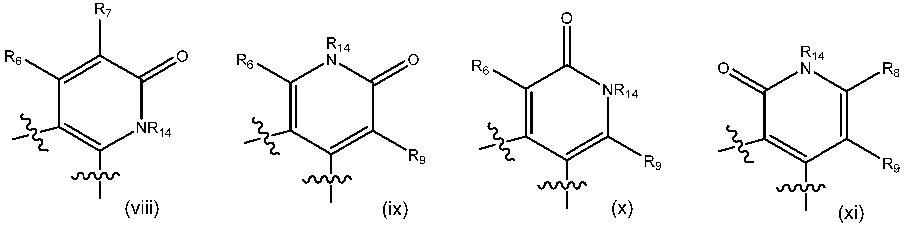 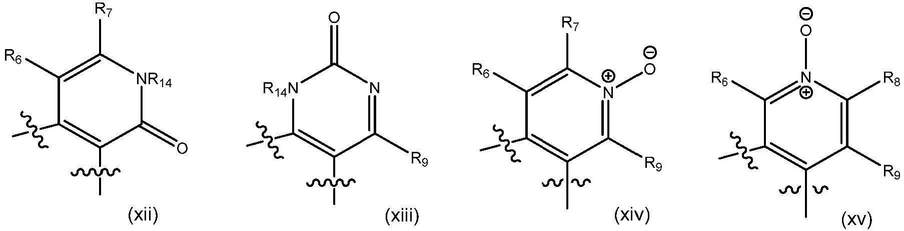 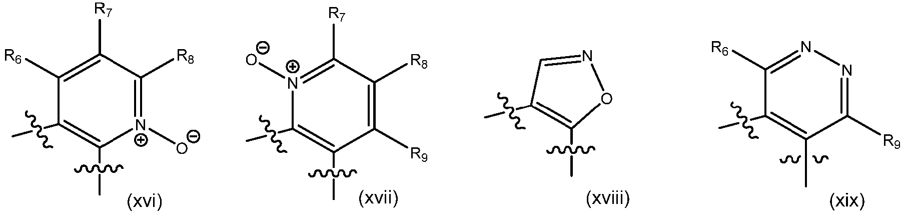 wherein R₆, R₇, R₈, and R₉ are each independently selected from H, F, Cl, Br, I, CN, alkoxy, NR₁₁R₁₁', OH, alkyl, phenyl, and haloalkyl, and R₁₄ is H or alkyl, more preferably H;
Y is selected from C=N-OH and CR₁₀R₁₀', wherein R₁₀ and R₁₀' are each independently selected from H, F, alkyl, and haloalkyl;
Rₐ and R_{b} are each independently selected from H and alkyl;
R₁, R₄, and R₅ are each independently selected from H, CN, alkyl, alkoxy, haloalkyl, OH, F, Cl, Br, and I;
R₂ and R₃ are each independently selected from H, F, Cl, Br, I, CN, alkoxy, haloalkyl, haloalkoxy, alkyl, aryl, heteroaryl, O-aryl, NHCO-alkenyl and CO₂-alkyl, wherein said aryl, heteroaryl and O-aryl groups are each optionally further substituted by one or more groups independently selected from halo, alkyl and alkoxy; and
R₁₁ and R₁₁' are each independently selected from H, alkyl, haloalkyl, COR₁₂, CO₂R₁₂ and SO₂R₁₃, wherein R₁₂ and R₁₃ are both independently alkyl.

4. A compound according to claim 3, wherein ring A is selected from (i), (ii), (iii), (iv), (v), (vi), (viii), (ix), (xiv), (xv) and (xix), and is preferably selected from (i), (ii), (iii), (v), (vi) and (ix), and is even more preferably selected from (i), (ii), (vi) and (ix).

5. A compound according to any preceding claim, which is of formula: 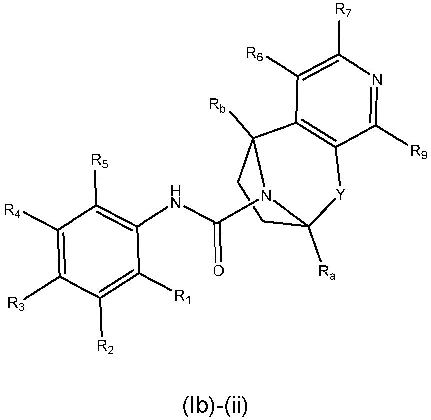
wherein Y, Rₐ, R_{b}, R₁-R₅ are as defined in claim 1, and R₆, R₇ and R₉ are as defined in claim 2; or 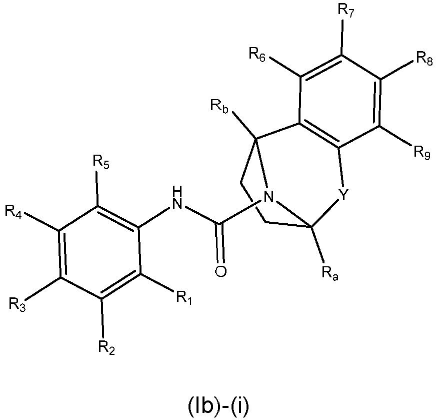
wherein Y, Rₐ, R_{b}, R₁-R₅ are as defined in claim 1, and R₆-R₉ are as defined in claim 2; or 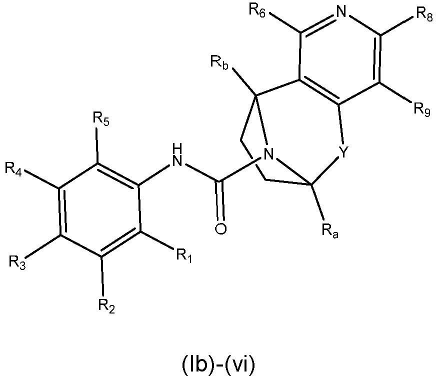
wherein Y, Rₐ, R_{b}, R₁-R₅ are as defined in claim 1, and R₆, R₈ and R₉ are as defined in claim 2; or 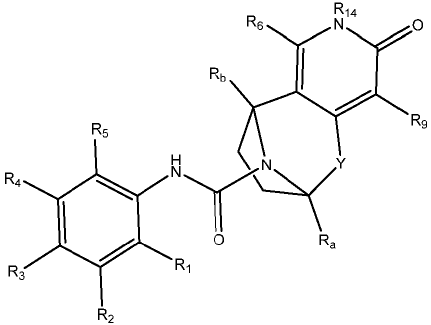 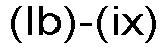
wherein Y, Rₐ, R_{b}, R₁-R₅ are as defined in claim 1, and R₆, R₉ and R₁₄ are as defined in claim 2.

6. A compound according to any preceding claim, wherein Y is selected from CH₂ and C=N-OH, and is preferably CH₂.

7. A compound according to any preceding claim, wherein R₂ and R₃ are:
(i) each independently selected from H, F, Cl, Br, CN, methoxy, OCF₃, CF₃, OCHF₂, Me, Ph, pyrazolyl, oxazolyl, thiazolyl, OPh, NHCO-CH=CH₂ and CO₂Me, wherein said Ph, OPh, pyrazolyl, oxazolyl and thiazolyl groups are each optionally further substituted by one or more alkyl groups;
(ii) each independently selected from F, Cl, Br, I, CN, C₁-C₆ haloalkyl, C₁-C₆ haloalkoxy and CO₂-alkyl.
(iii) each independently selected from Cl, Br, and CF₃, and more preferably are each independently selected from Cl and CF₃; or
(iv) both Cl, or one of R₂ and R₃ is Cl and the other is selected from OCF₃, CO₂Me, OCHF₂ and CF₃.

8. A compound according to any preceding claim, wherein R₁ and R₄ are both H.

9. A compound according to any preceding claim, wherein R₅ is selected from H, F, Me, MeO, Cl, OH and CN, and is preferably H or F, more preferably H.

10. A compound according to any one of claims 2 to 9, wherein R₆, R₇, R₈, and R₉ are each independently selected from H, F, Cl, Br, CN, OMe, NH₂, NHBu, NHCO₂Bu and OH.

11. A compound according to any preceding claim, which is of formula (Ib.1): 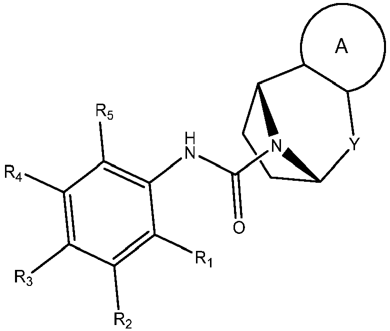 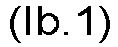 where A, Y and R₁-R₅ are as defined in any of claims 1-10, or which is in the form of a mixture that is enantiomerically enriched with a compound of formula (Ib.1).

12. A compound according to any one of claims 3-11 which is selected from the following:
| | | | |
|---|---|---|---|
| 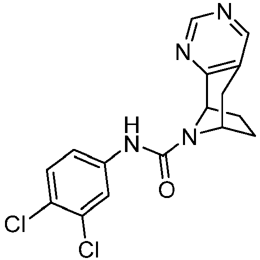 | (17) | 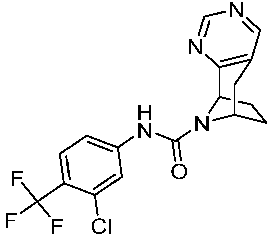 | (18) |
| 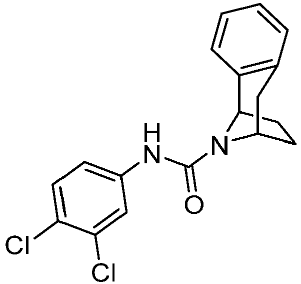 | (34) | 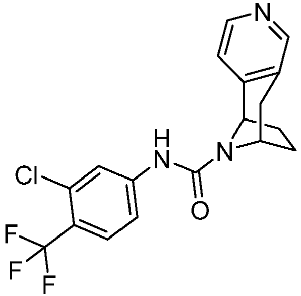 | (35) |
| 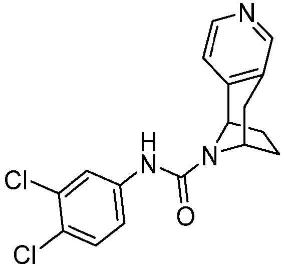 | (36) | 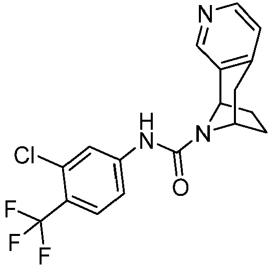 | (37) |
| | | 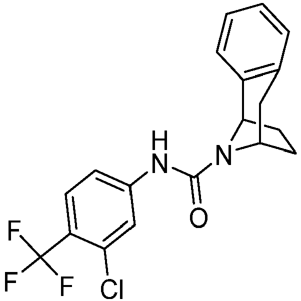 | (54) |
| 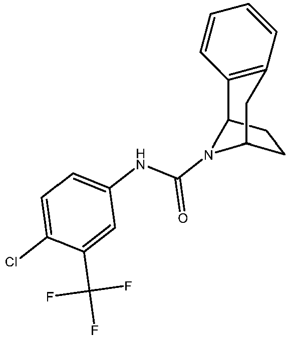 | (55) | 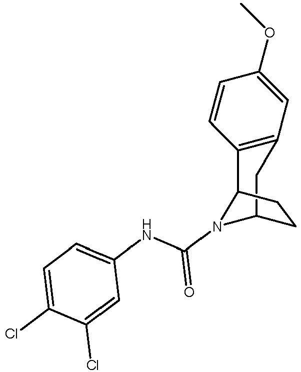 | (56) |
| 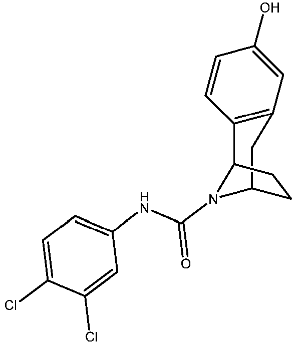 | (57) | 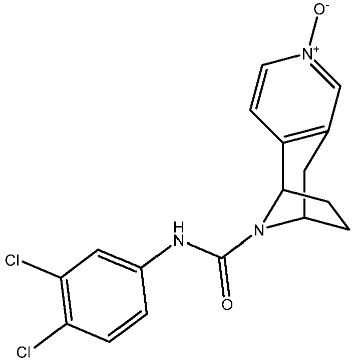 | (58) |
| 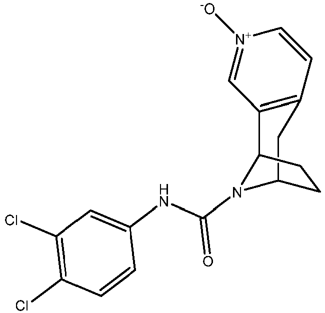 | (59) | 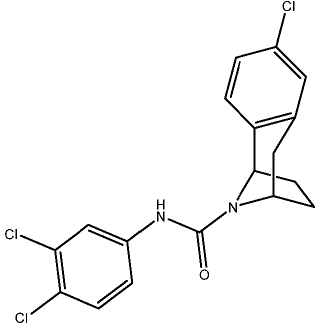 | (60) |
| 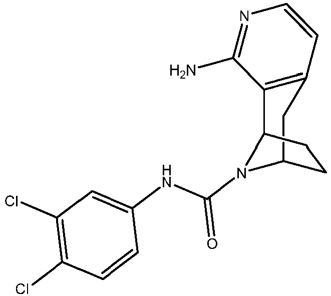 | (61) | 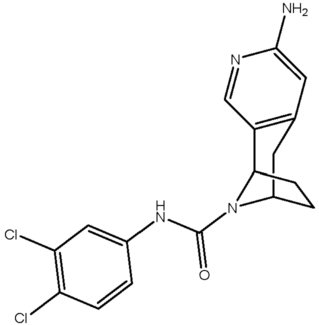 | (62) |
| 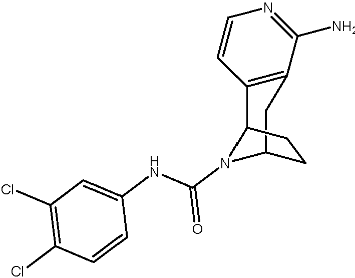 | (63) | 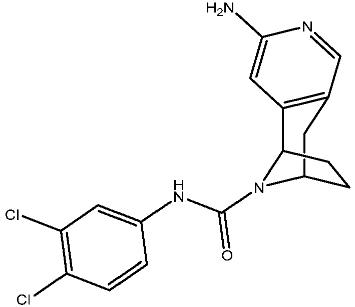 | (64) |
| 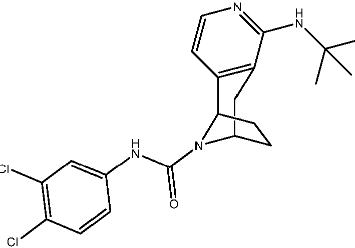 | (65) | 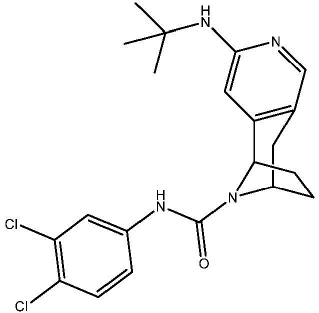 | (66) |
| 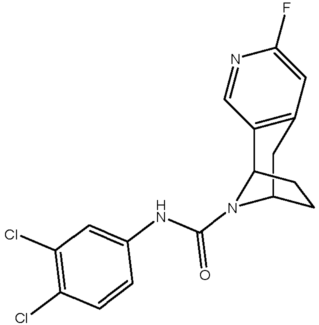 | (67) | 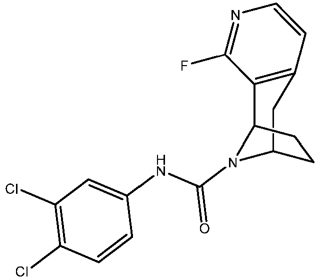 | (68) |
| 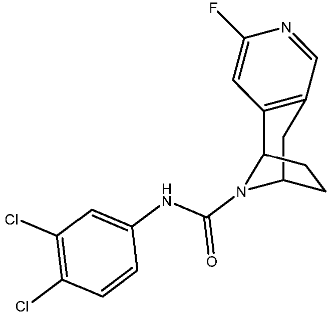 | (69) | 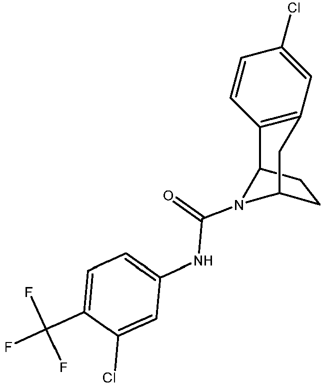 | (71) |
| 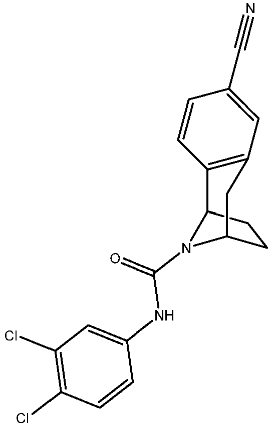 | (72) | 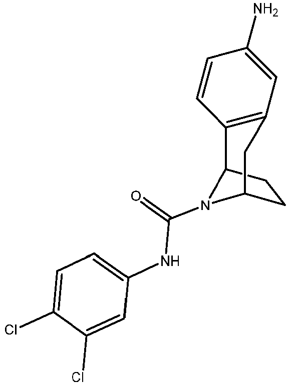 | (73) |
| 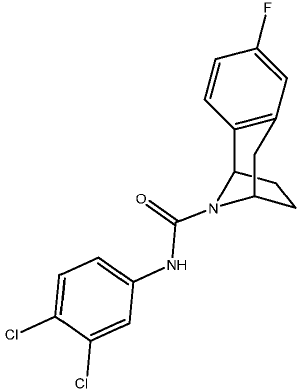 | (74) | 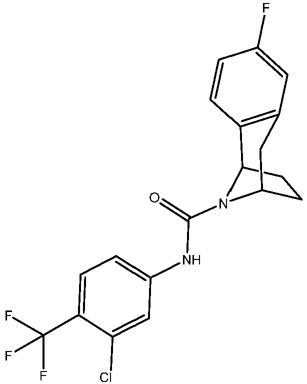 | (75) |
| 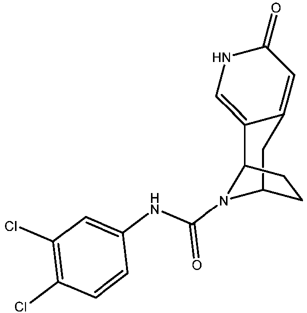 | (76) | 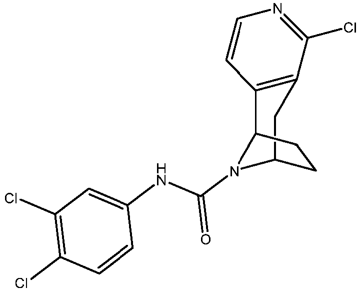 | (77) |
| 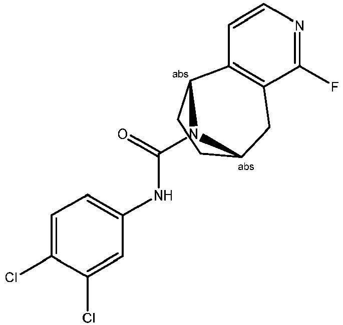 | (78) | 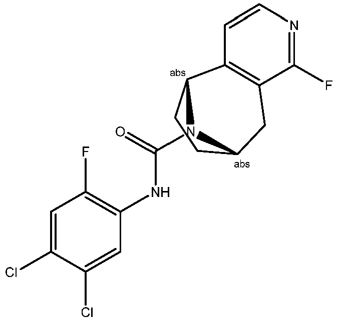 | (79) |
| 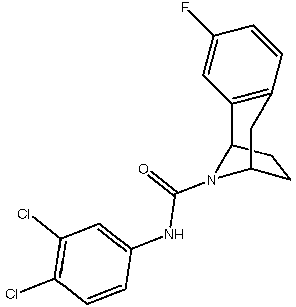 | (80) | 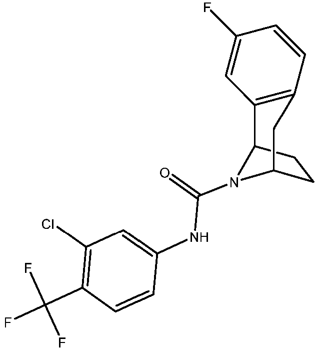 | (81) |
| 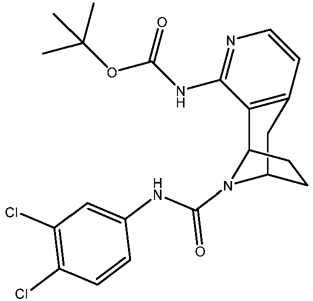 | (82) | 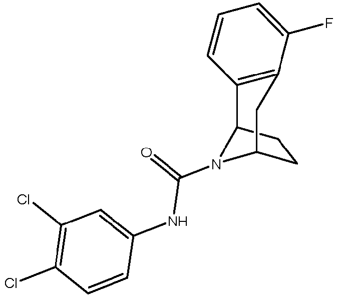 | (83) |
| 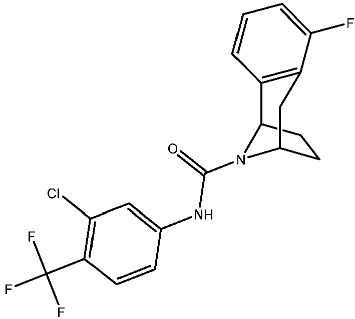 | (84) | 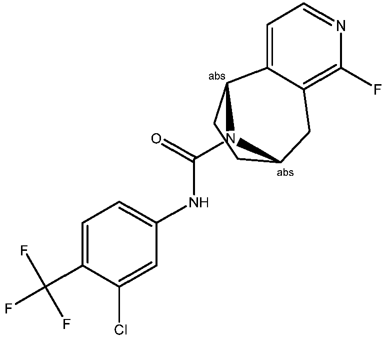 | (85) |
| 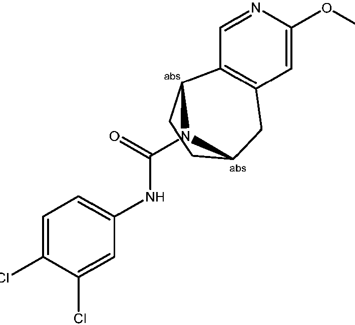 | (86) | 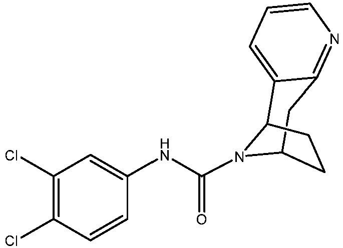 | (87) |
| 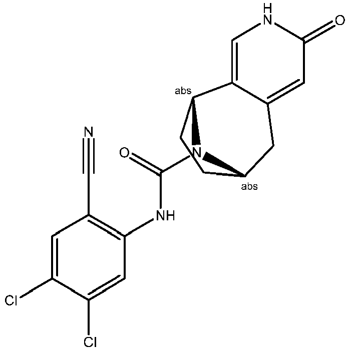 | (88) | 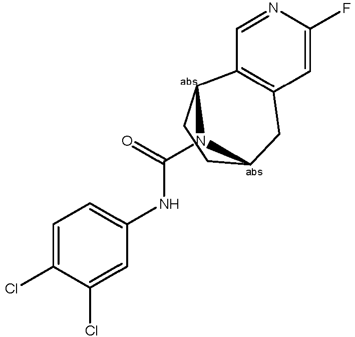 | (91) |
| 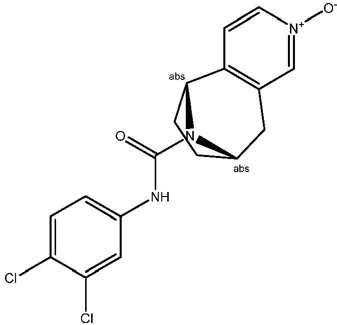 | (92) | 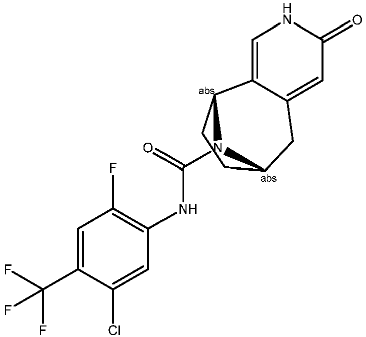 | (93) |
| 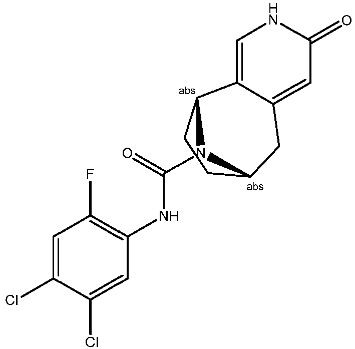 | (94) | 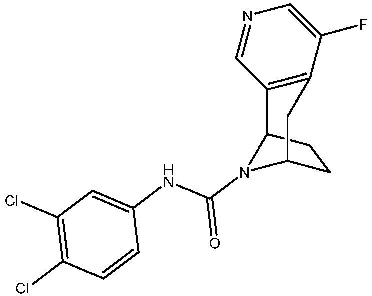 | (95) |
| 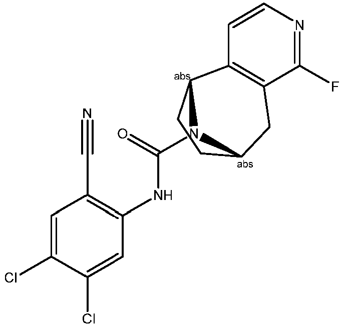 | (98) | 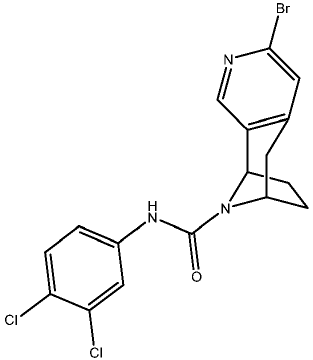 | (99) |
| 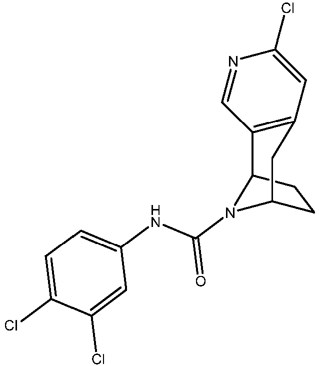 | (100) | 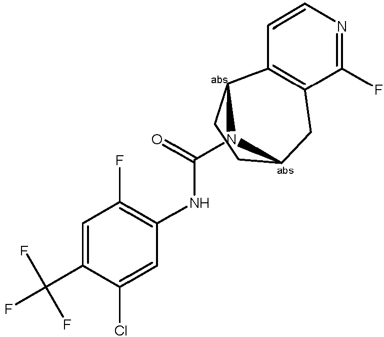 | (101) |
| 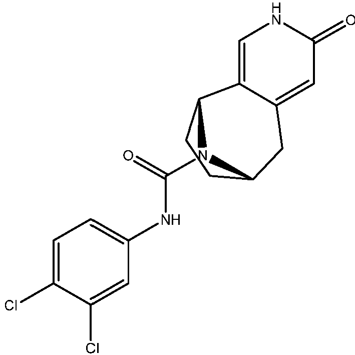 | (103) | 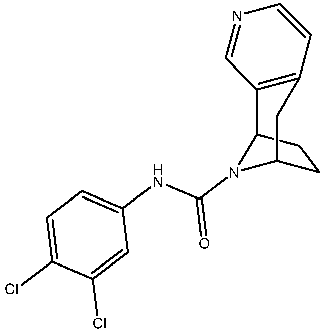 | (104) |
| 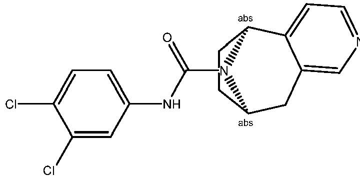 | (107) | 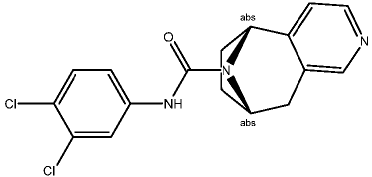 | (108) |
| 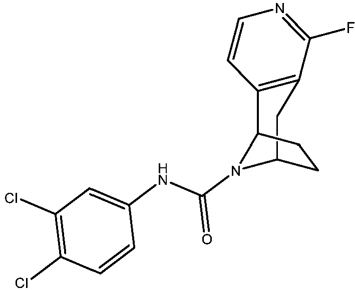 | (109) | 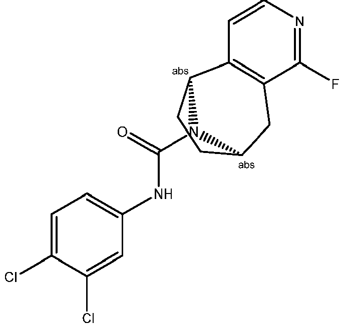 | (110) |
| 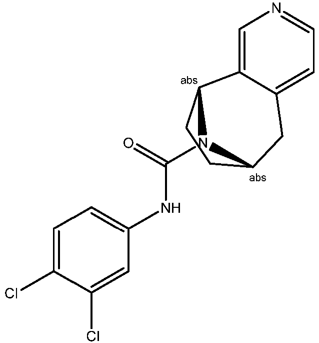 | (111) | 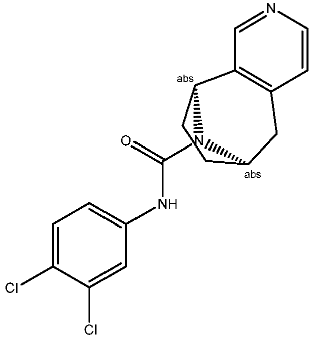 | (112) |
| 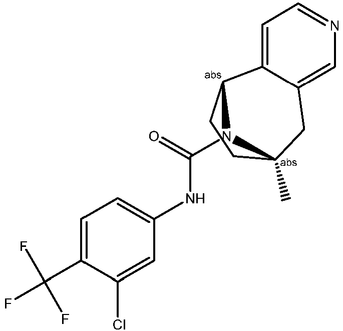 | (116) | 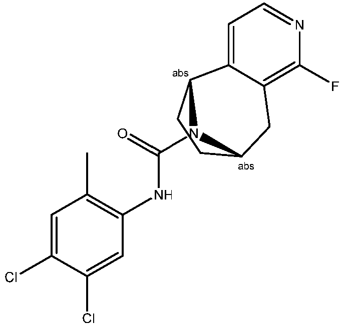 | (117) |
| 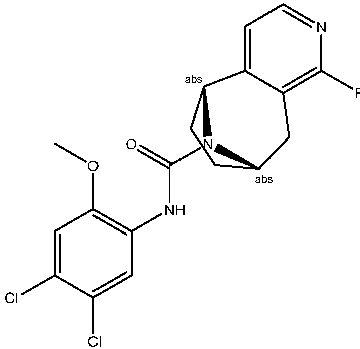 | (118) | 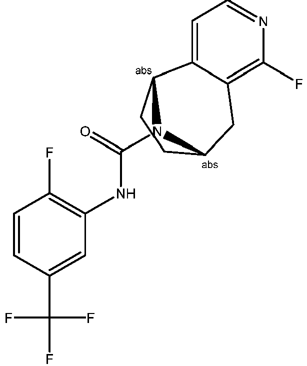 | (119) |
| 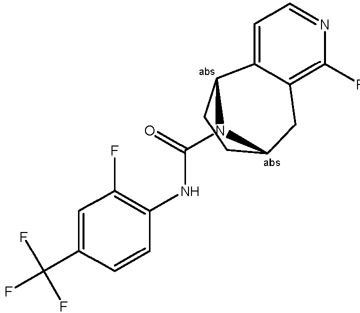 | (120) | 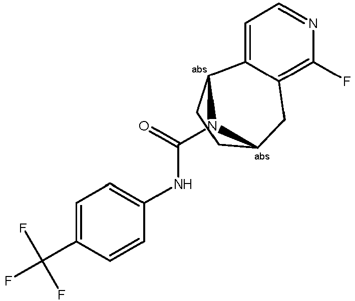 | (121) |
| 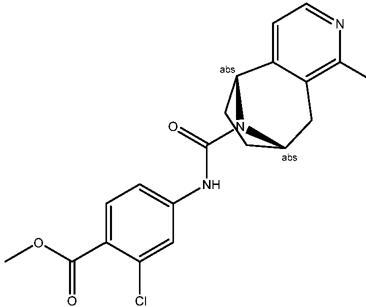 | (122) | 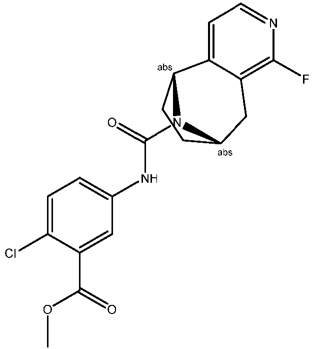 | (123) |
| 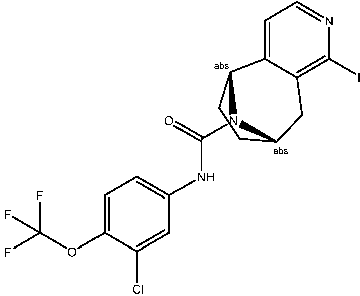 | (124) | 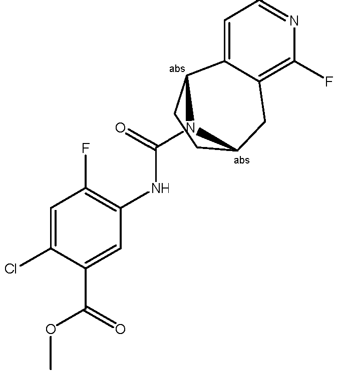 | (125) |
| 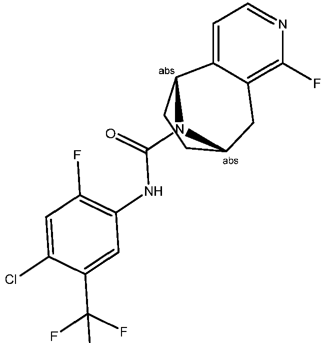 | (126) | 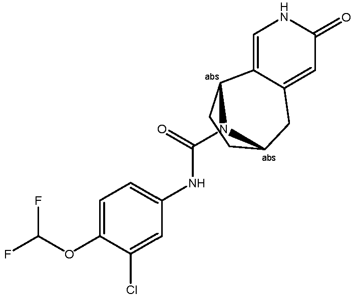 | (127) |
| 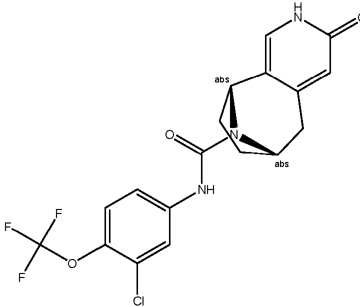 | (128) | 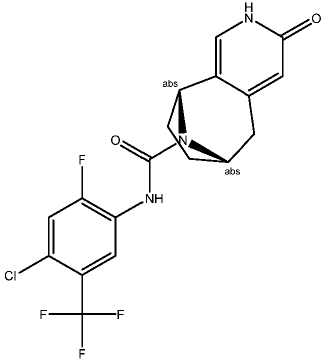 | (129) |
| 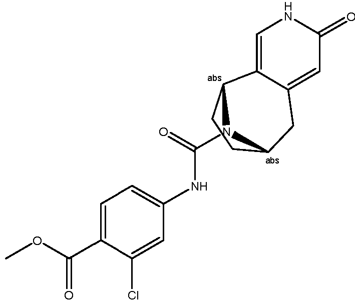 | (130) | 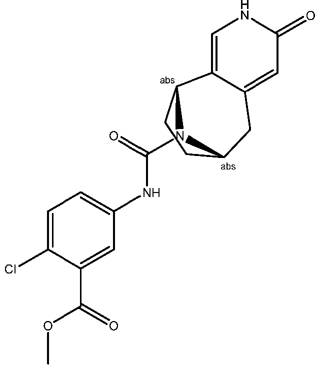 | (131) |
| 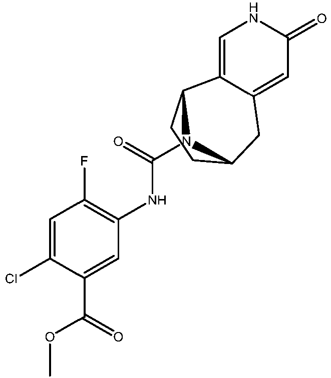 | (132) | 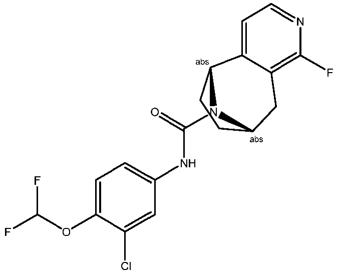 | (133) |
| 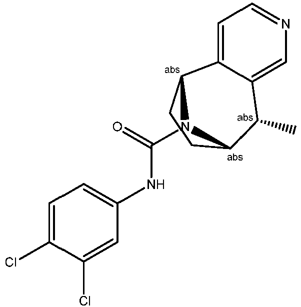 | (134) | 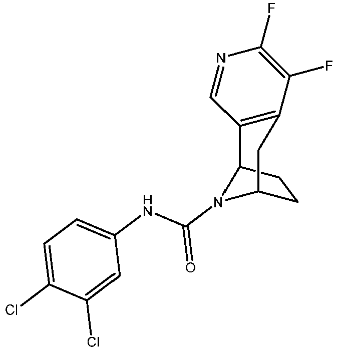 | (135) |
| 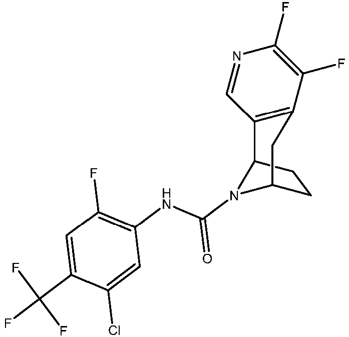 | (136) | 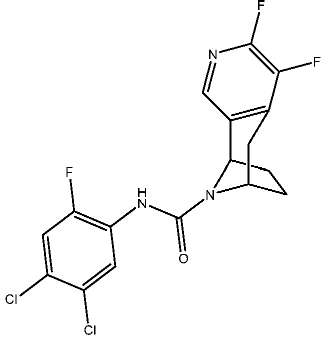 | (137) |
| 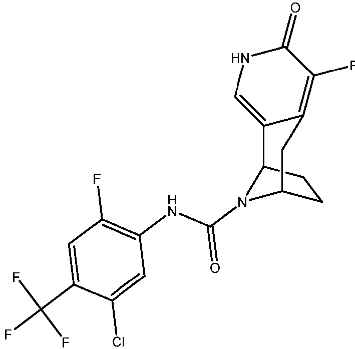 | (138) | 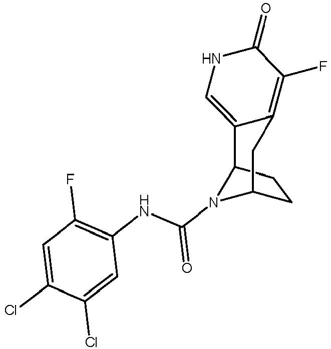 | (139) |
| 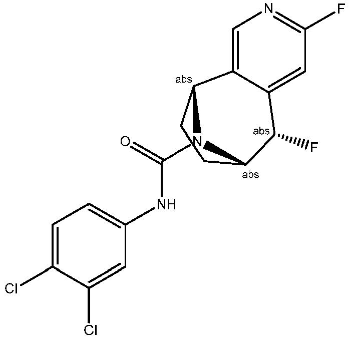 | (140) | 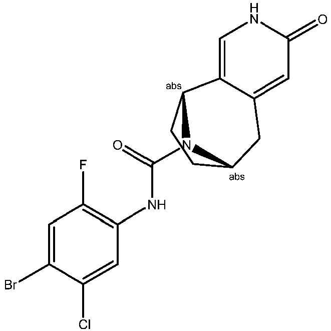 | (141) |
| 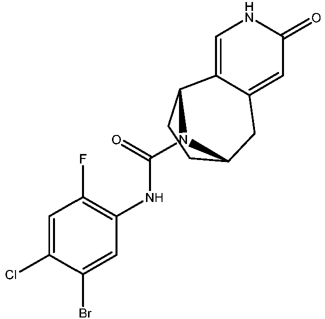 | (142) | 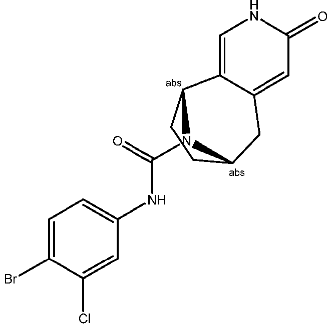 | (143) |
| 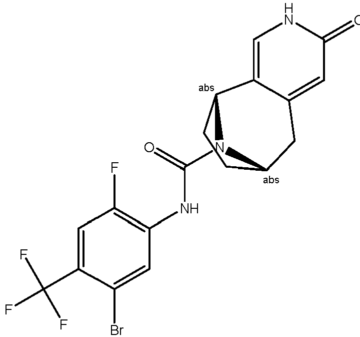 | (144) | 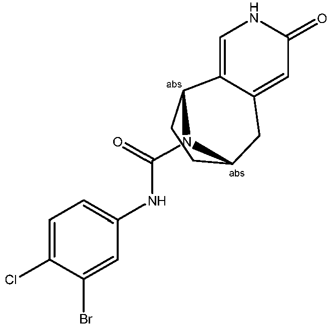 | (145) |
| 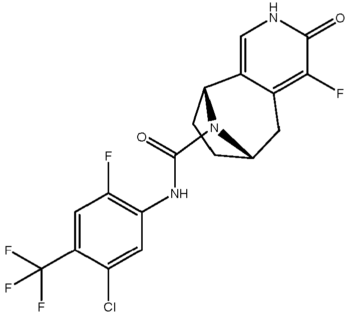 | (146) | 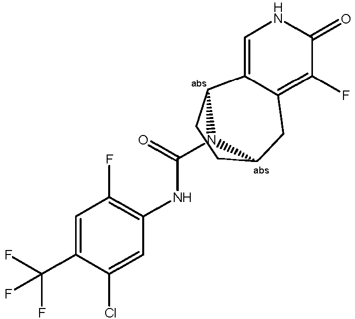 | (147) |
| 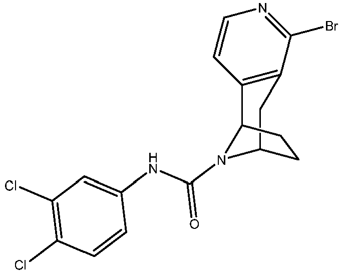 | (148) | 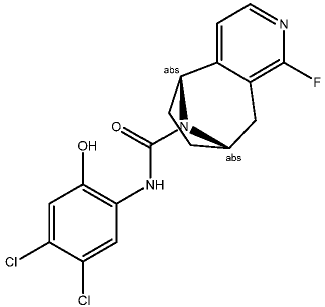 | (149) |
| 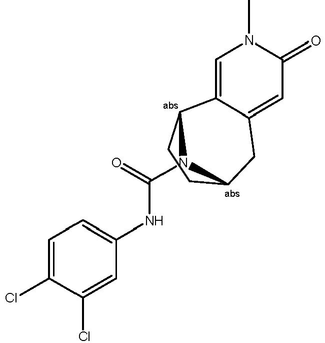 | (150) | | |
| 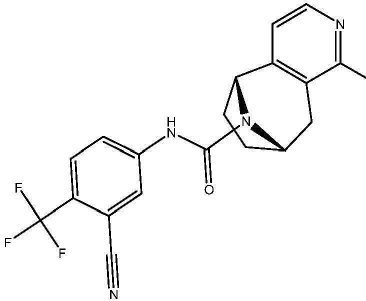 | (151) | 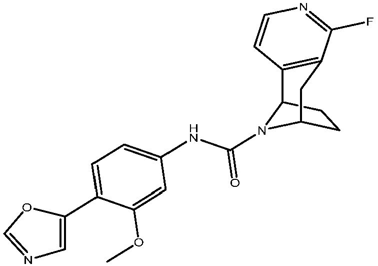 | (152) |
| 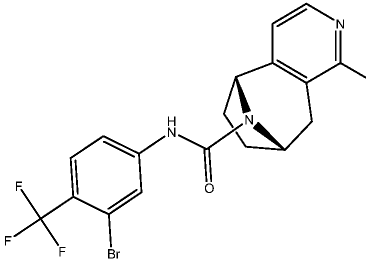 | (153) | 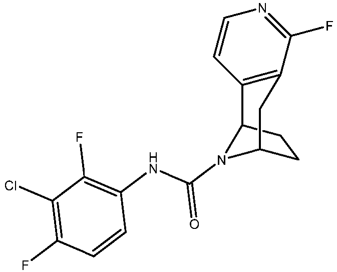 | (154) |
| 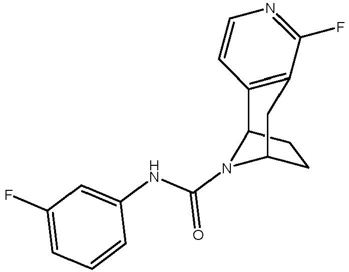 | (155) | 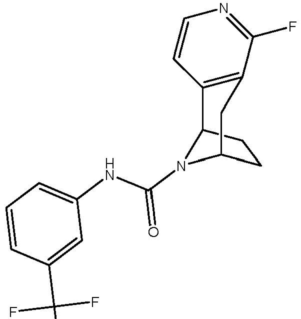 | (156) |
| 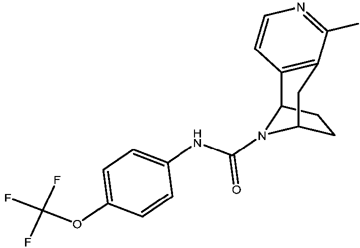 | (157) | 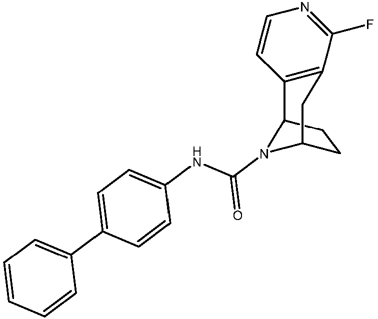 | (158) |
| 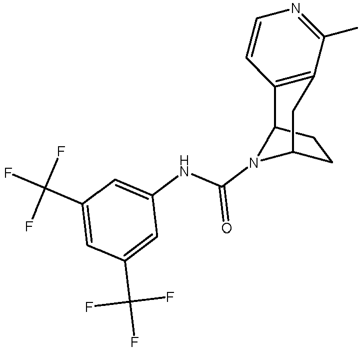 | (159) | 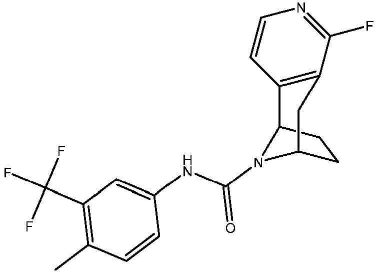 | (160) |
| 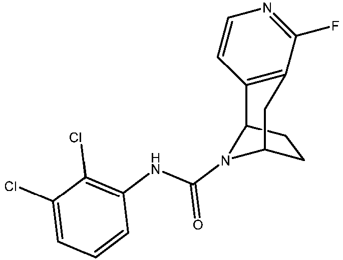 | (161) | 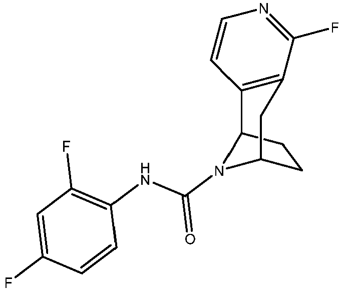 | (162) |
| 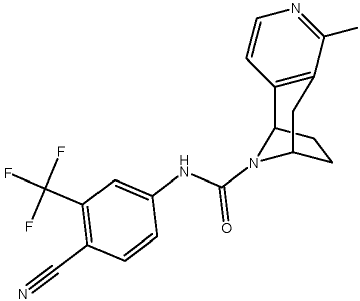 | (163) | 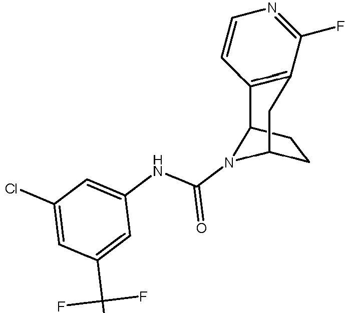 | (164) |
| 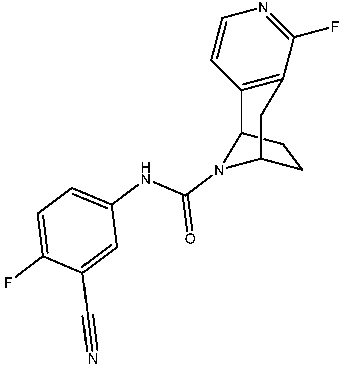 | (165) | 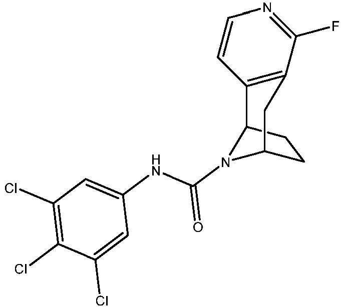 | (166) |
| 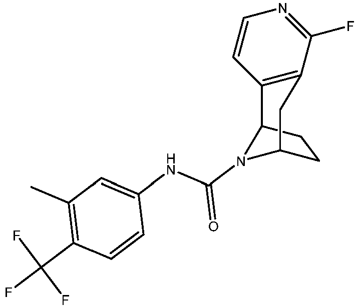 | (167) | 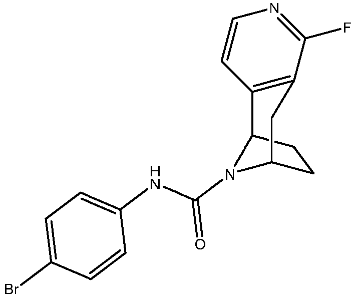 | (168) |
| 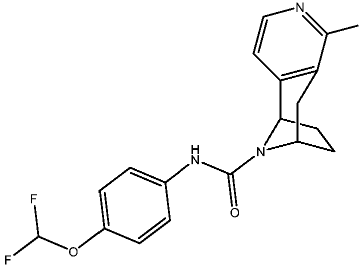 | (169) | 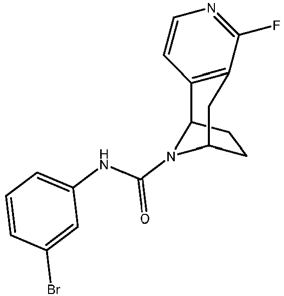 | (170) |
| 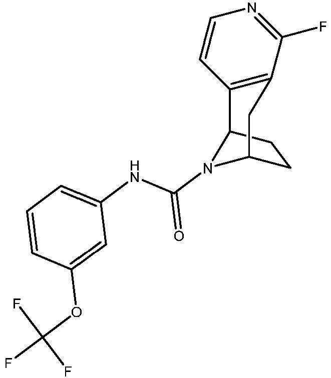 | (171) | 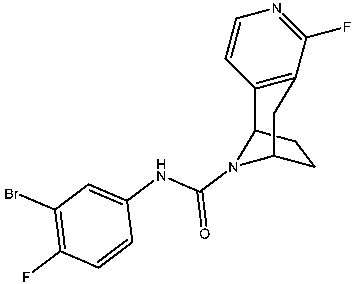 | (172) |
| 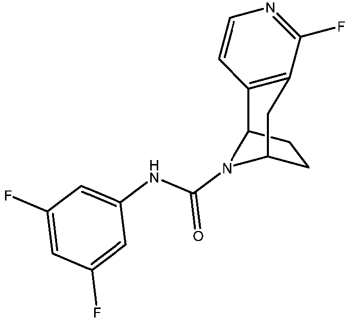 | (173) | 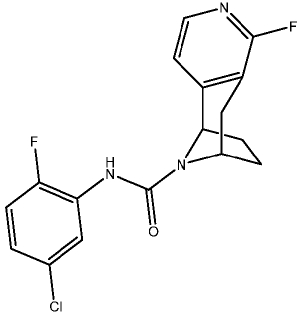 | (174) |
| 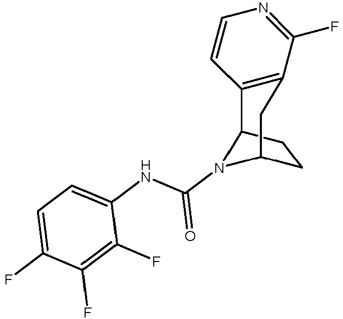 | (175) | 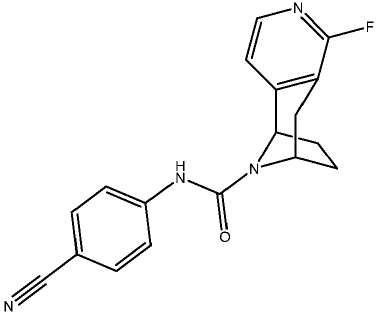 | (176) |
| 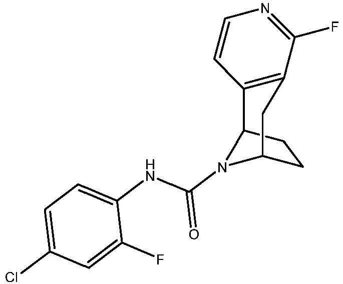 | (177) | 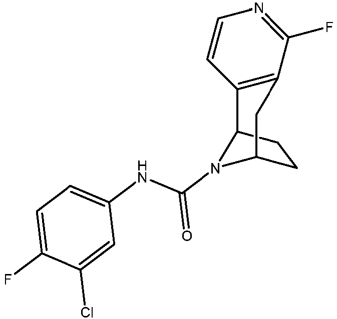 | (178) |
| 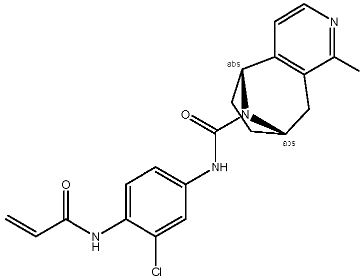 | (179) | 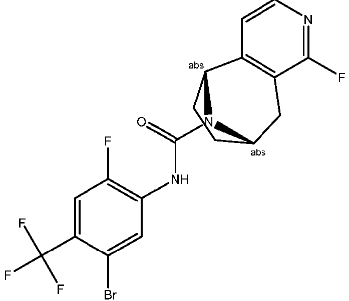 | (180) |
| 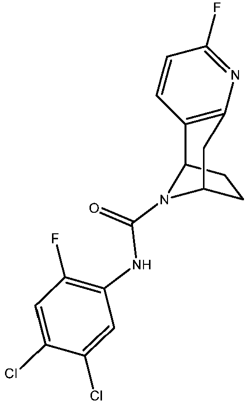 | (181) | 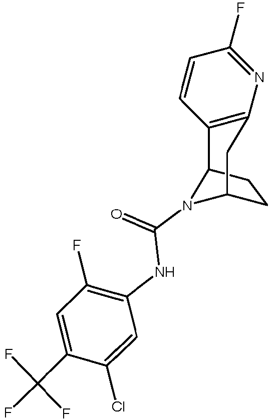 | (182) |
| 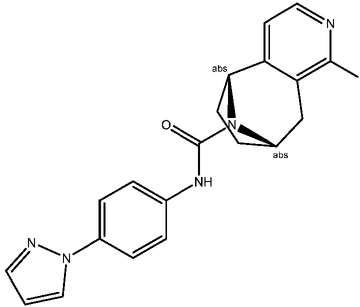 | (183) | 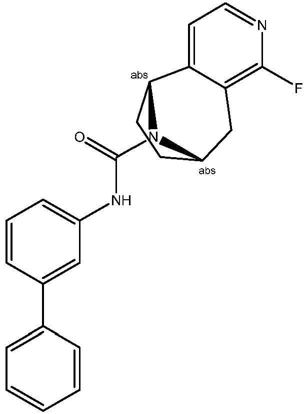 | (184) |
| 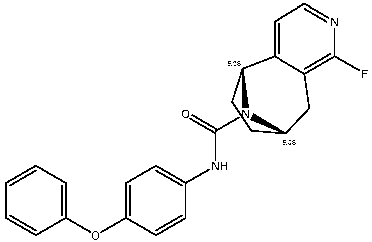 | (185) | 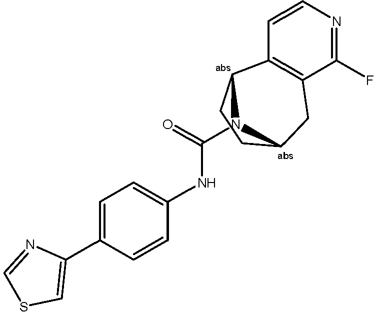 | (186) |
| 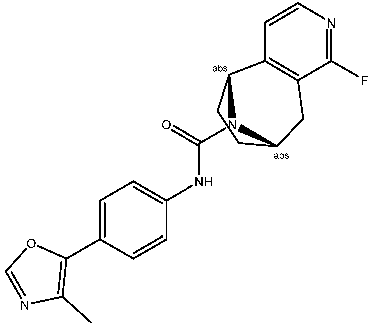 | (187) | 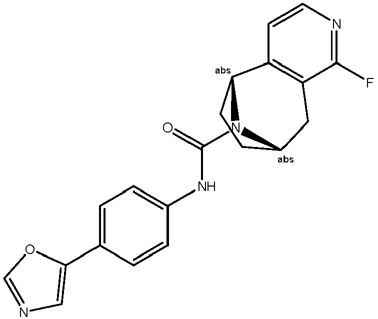 | (188) |
| 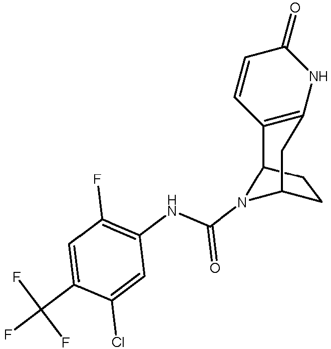 | (189) | 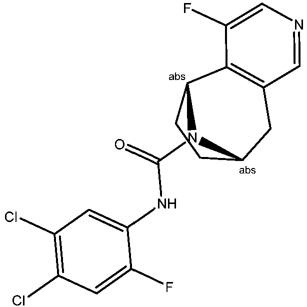 | (190) |
| 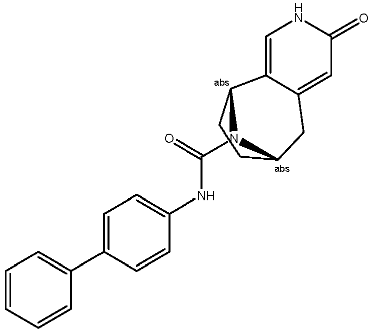 | (191) | 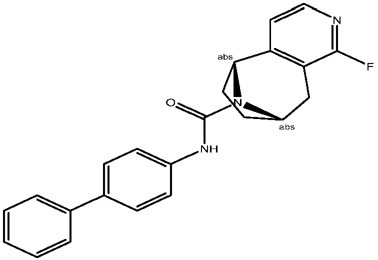 | (192) |
| 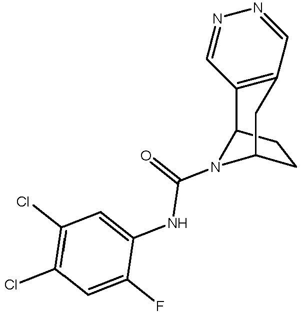 | (193) | 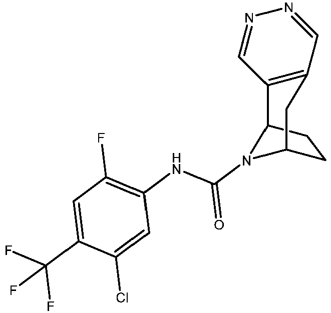 | (194) |
| 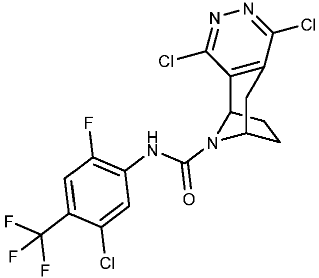 | (195) | 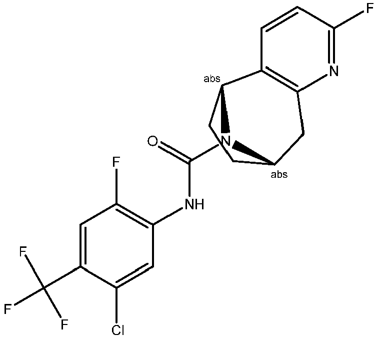 | (196) |
| 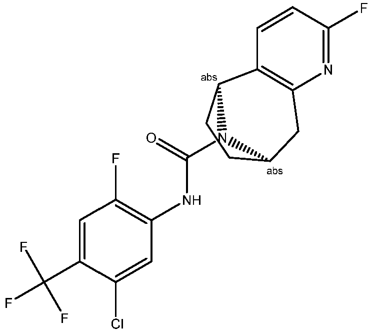 | (197) | 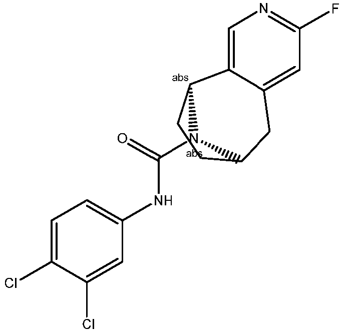 | (198) |
| 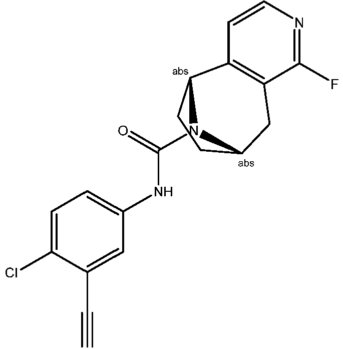 | (199) | 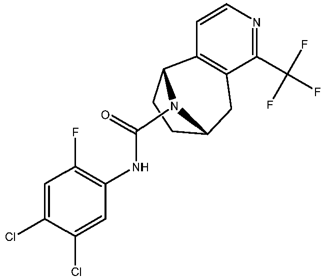 | 200 |
and enantiomers thereof, and mixtures of enantiomers thereof, including racemic mixtures, and pharmaceutically acceptable salts and solvates thereof.

13. A compound of formula (le), or a pharmaceutically acceptable salt or solvate thereof, 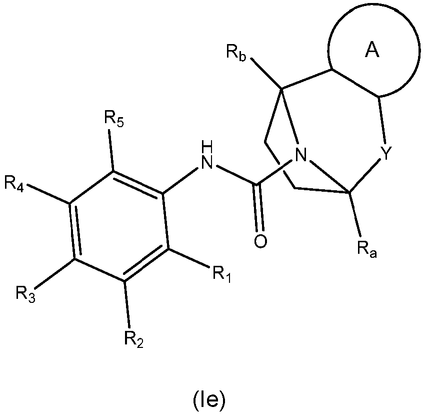 wherein:
ring A is 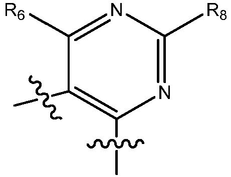 wherein R₆ and R₈ are each independently selected from H, F, Cl, Br, I, CN, alkoxy, OH, phenyl, and haloalkyl;
Y is selected from C=N-OH and CR₁₀R₁₀', wherein R₁₀ and R₁₀' are each independently selected from H, F, alkyl, and haloalkyl;
Rₐ and R_{b} are each independently selected from H and alkyl;
R₁, R₄, and R₅ are each independently selected from H, F, Cl, Br, and I; and
R₂ and R₃ are each independently selected from Cl, Br, I, CN, and haloalkyl;
with the proviso that when Y is CH₂, and Rₐ, R_{b}, R₁, R₄, R₅, R₆ and R₈ are all H:
R₃ is not CN, when R₂ is Cl; and
R₂ and R₃ are not both Cl.

14. A compound according to claim 13 wherein R₂ is selected from Cl, Br and CF₃, and is more preferably selected from Cl and CF₃.

15. A compound according to claim 13 or claim 14 wherein R₃ is selected from Cl, Br, CN and CF₃, more is more preferably selected from Cl and CF₃.

16. A compound according to any one of claims 13 to 15 wherein R₁, R₄ and R₅ are all H.

17. A compound according to any one of claims 13 to 16 wherein Y is selected from CH₂ and CHF, and is preferably CH₂.

18. A compound according to any one of claims 13 to 17 wherein R₆ is H.

19. A compound according to any one of claims 13 to 18 wherein R₈ is selected from H, CF₃, phenyl, OH and F, optionally wherein R₈ is OH, and ring A is:

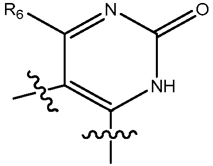

20. A compound according to any one of claims 13 to 19, which is of formula (le.1): 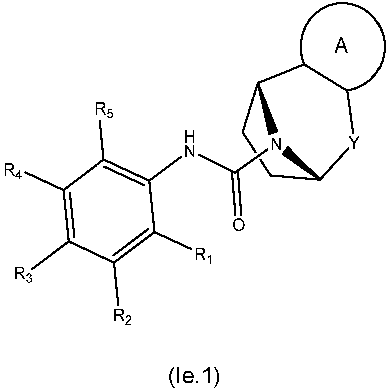 where A, Y and R₁-R₅ are as defined in any of claims 13-19, or which is in the form of a mixture that is enantiomerically enriched with a compound of formula (le.1).

21. A compound which is selected from the following:
| | | | |
|---|---|---|---|
| 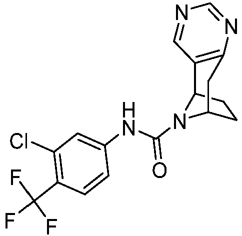 | (2) | | |
| 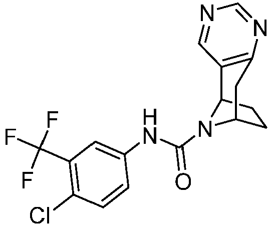 | (3) | 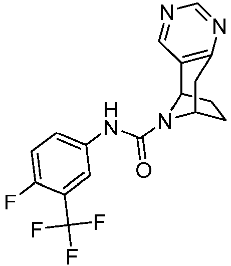 | (5) |
| 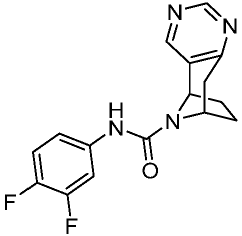 | (6) | 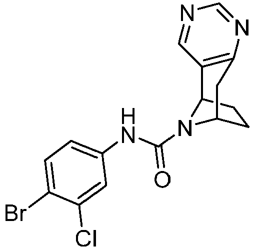 | (7) |
| 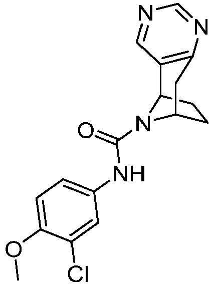 | (8) | 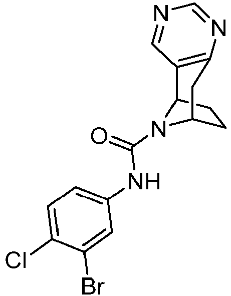 | (12) |
| 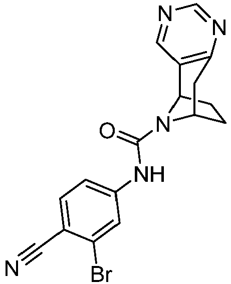 | (13) | 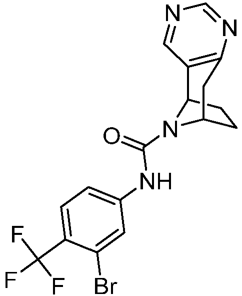 | (15) |
| 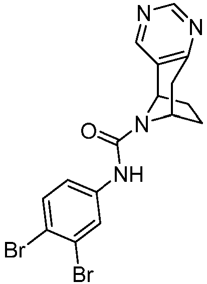 | (16) | 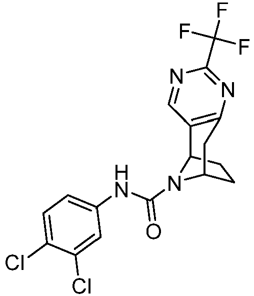 | (24) |
| 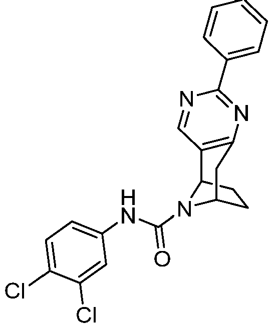 | (26) | 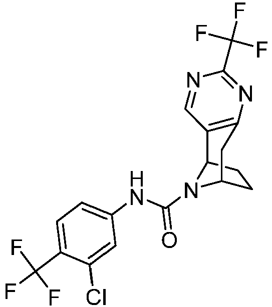 | (27) |
| 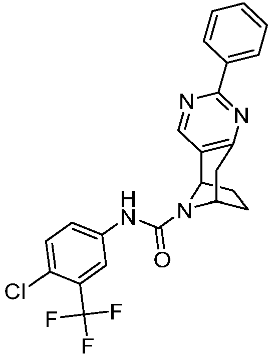 | (28) | 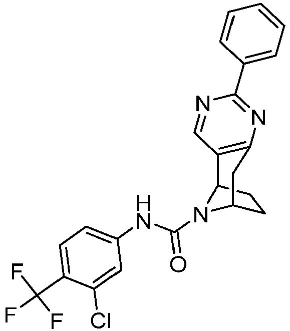 | (29) |
| 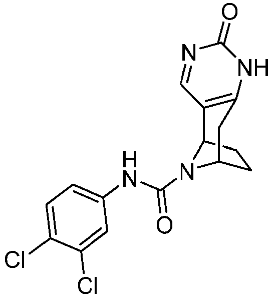 | (31) | 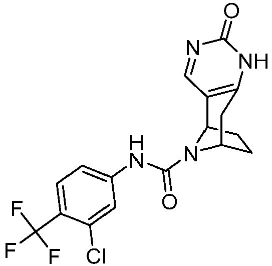 | (32) |
| | | 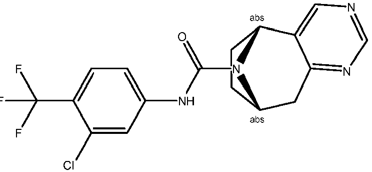 Enantiomer of (2) | (38) |
| 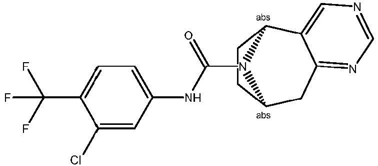 Enantiomer of (2) | (39) | 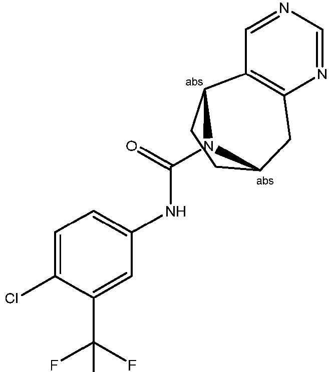 Enantiomer of (3) | (42) |
| 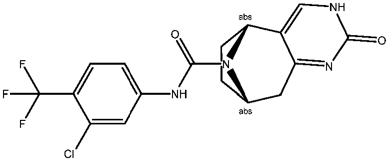 Enantiomer of (32) | (43) | 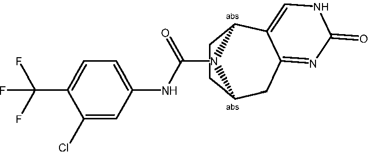 Enantiomer of (32) | (44) |
| 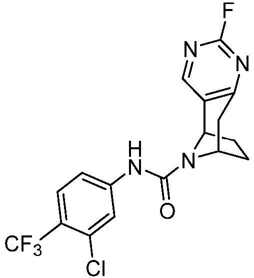 | (45) | 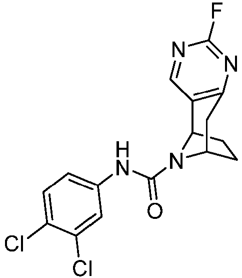 | (46) |
| 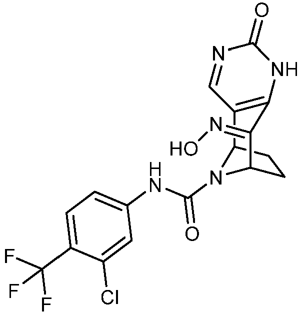 | (47) | 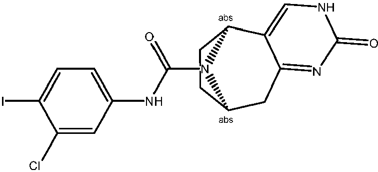 Enantiomer of (31) | (105) |
| 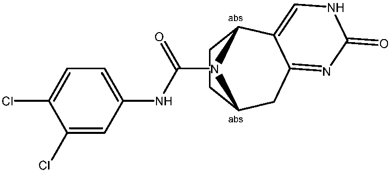 Enantiomer of (31) | (106) | 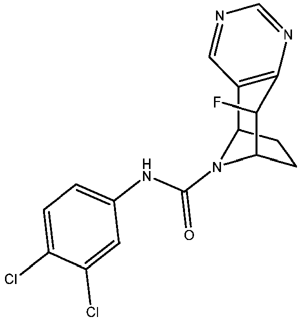 | (113) |
| 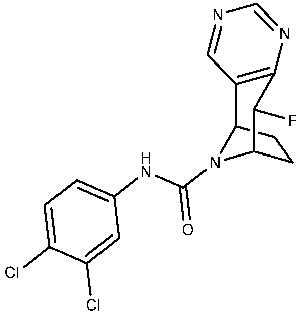 | (114) | 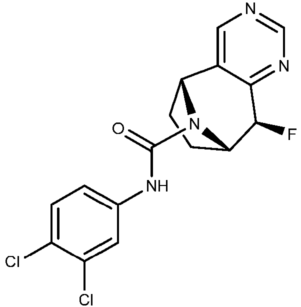 | (115) |
and enantiomers thereof, and mixtures of enantiomers thereof, including racemic mixtures, and pharmaceutically acceptable salts and solvates thereof.

22. A pharmaceutical composition comprising a compound according to any of claims 1-21, and a pharmaceutically acceptable diluent, excipient, or carrier.

23. A compound according to any one of claims 1-21, or a pharmaceutical composition according to claim 22, for use as a medicament.

24. A compound according to any one of claims 1-21, or a pharmaceutical composition according to claim 22, for use in treating or preventing a disorder selected from a proliferative disorder, an immune disorder, asthma, chronic obstructive pulmonary disease (COPD) and acute respiratory distress syndrome (ARDS).

25. A compound or pharmaceutical composition for use according to claim 24, wherein the use comprises modulating GPR65, preferably wherein the use comprises inhibiting GPR65 signalling.

26. A compound or pharmaceutical composition for use according to claim 24, wherein the disorder is a proliferative disorder, preferably:
wherein the proliferative disorder is a cancer, and is preferably a solid tumour and/or metastases thereof; or
wherein the proliferative disorder is a cancer selected from melanoma, renal cell carcinoma (RCC), gastric cancer, acute myeloid leukaemia (AML), triple negative breast cancer (TNBC), colorectal cancer, head and neck cancer, colorectal adenocarcinoma, pancreatic adenocarcinoma, sarcoma, lung cancer, ovarian cancer and gliomas, preferably glioblastoma (GBM).

27. A compound or pharmaceutical composition for use according to claim 24 wherein the disorder is an immune disorder, preferably wherein the immune disorder is an autoimmune disease, more preferably wherein the autoimmune disease is selected from psoriasis, psoriatic arthritis, rheumatoid arthritis (RA), multiple sclerosis (MS), systemic lupus erythematosus (SLE), autoimmune thyroiditis (Hashimoto's thyroiditis), Graves' disease, uveitis (including intermediate uveitis), ulcerative colitis, Crohn's disease, autoimmune uveoretinitis, systemic vasculitis, polymyositis-dermatomyositis, systemic sclerosis (scleroderma), Sjogren's Syndrome, ankylosing spondylitis and related spondyloarthropathies, sarcoidosis, autoimmune hemolytic anemia, immunological platelet disorders, and autoimmune polyendocrinopathies, most preferably wherein the autoimmune disease is selected from psoriasis, psoriatic arthritis, ankylosing spondylitis, Crohn's disease, and multiple sclerosis (MS).

28. A compound or pharmaceutical composition for use according to claim 24, wherein the use comprises treating or preventing a disorder selected from asthma, chronic obstructive pulmonary disease (COPD) and acute respiratory distress syndrome (ARDS).

29. A compound as defined in any one of claims 1-21, or a pharmaceutically acceptable salt or solvate thereof, or a pharmaceutical composition according to claim 22, for use in treating or preventing a GPR65-associated disease or disorder.

30. A compound selected from the following:
| | | | |
|---|---|---|---|
| 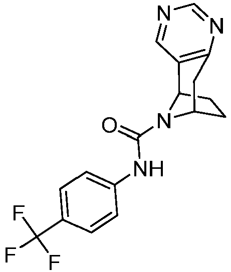 | (1) | 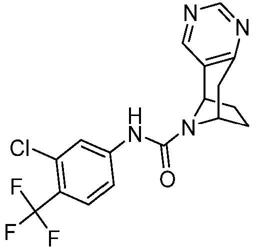 | (2) |
| 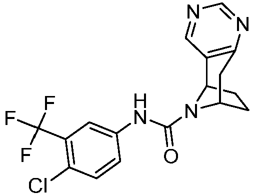 | (3) | 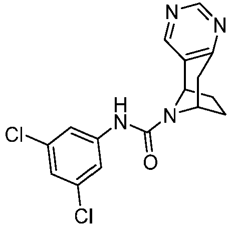 | (4) |
| 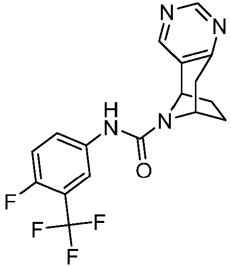 | (5) | 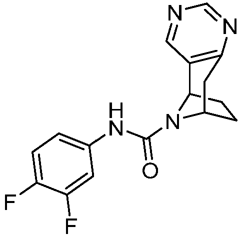 | (6) |
| 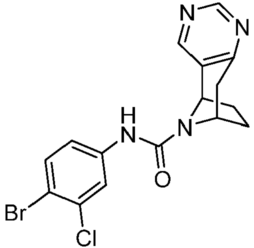 | (7) | 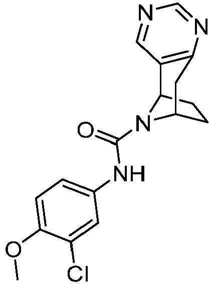 | (8) |
| 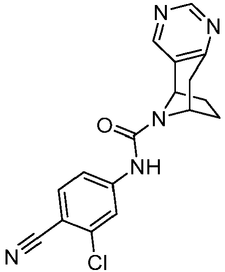 | (11) | 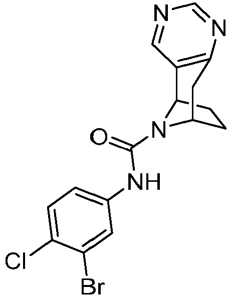 | (12) |
| 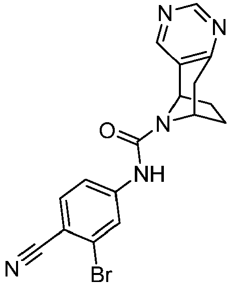 | (13) | 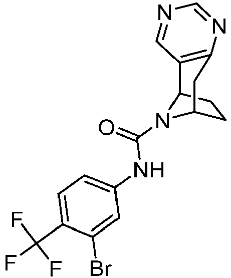 | (15) |
| 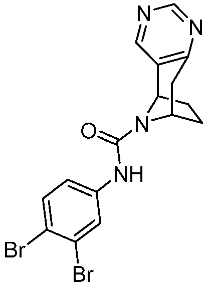 | (16) | 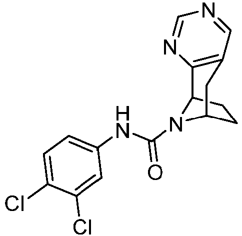 | (17) |
| 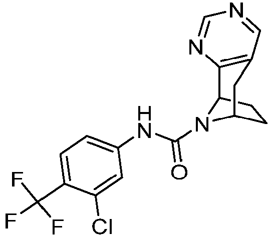 | (18) | 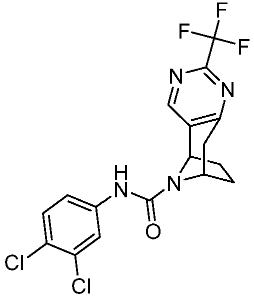 | (24) |
| 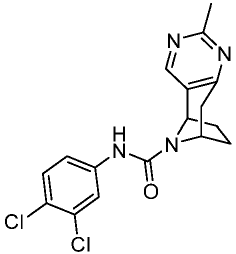 | (25) | 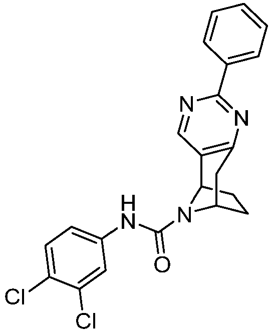 | (26) |
| 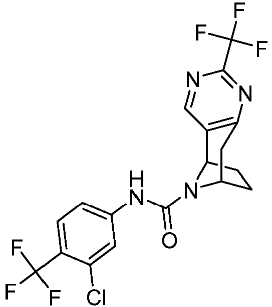 | (27) | 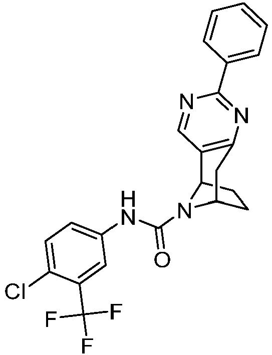 | (28) |
| 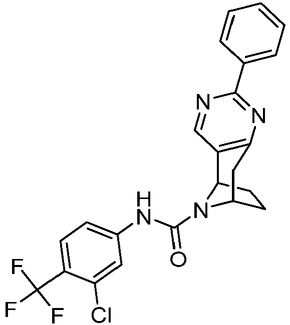 | (29) | 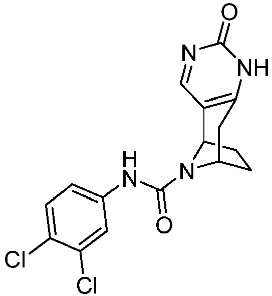 | (31) |
| 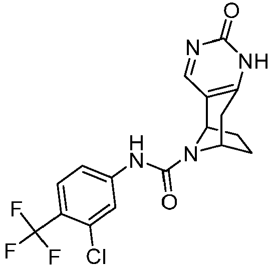 | (32) | 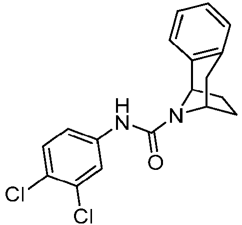 | (34) |
| 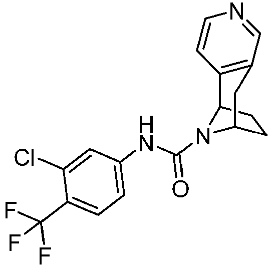 | (35) | 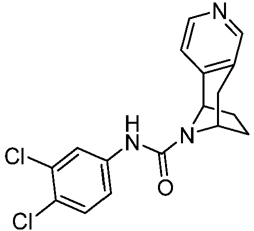 | (36) |
| 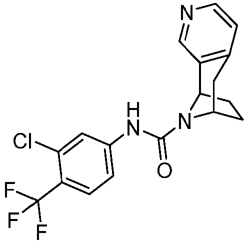 | (37) | 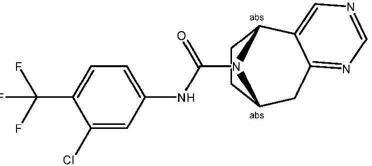 Enantiomer of (2) | (38) |
| 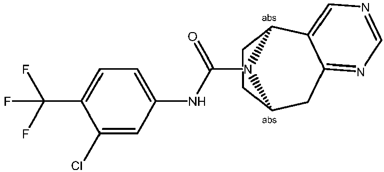 Enantiomer of (2) | (39) | 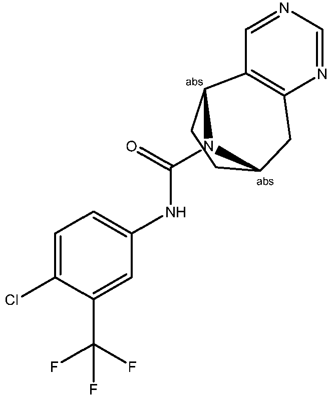 Enantiomer of (3) | (42) |
| 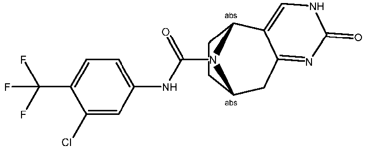 Enantiomer of (32) | (43) | 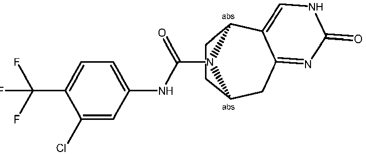 Enantiomer of (32) | (44) |
| 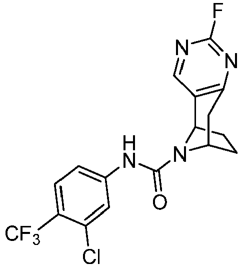 | (45) | 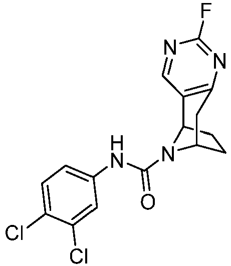 | (46) |
| 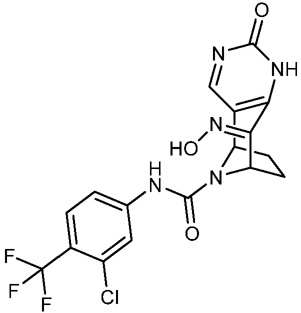 | (47) | 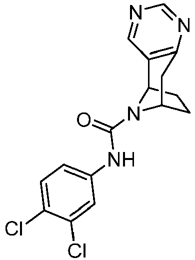 | (48) |
| 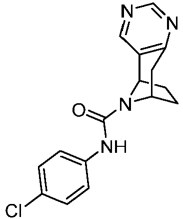 | (49) | 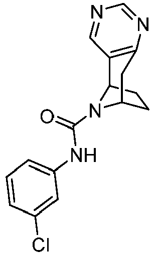 | (50) |
| 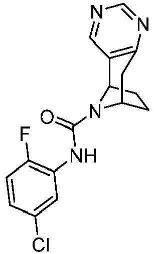 | (51) | 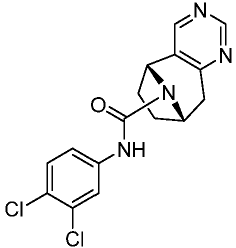 | (52) |
| 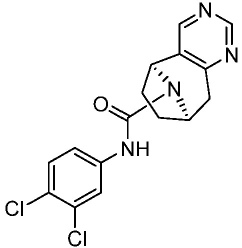 | (53) | 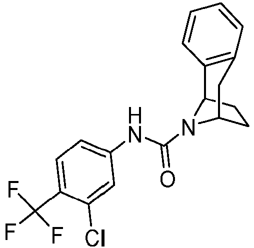 | (54) |
| 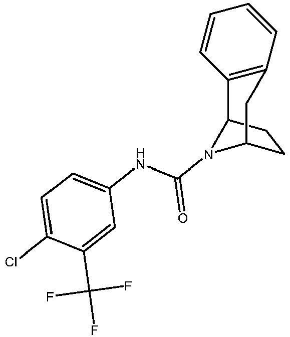 | (55) | 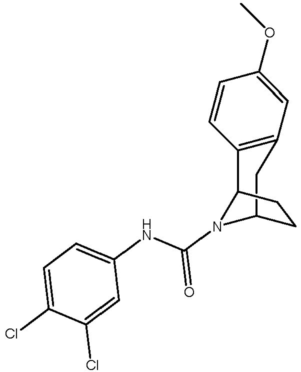 | (56) |
| | (57) | 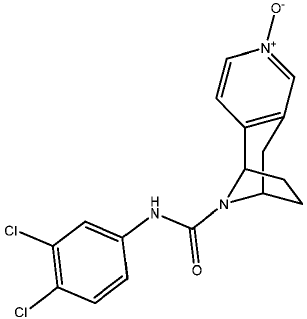 | (58) |
| 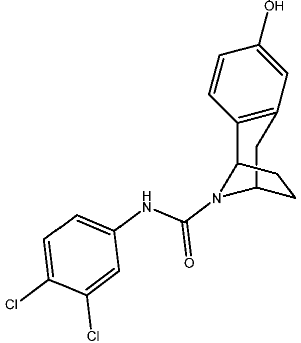 | | | |
| 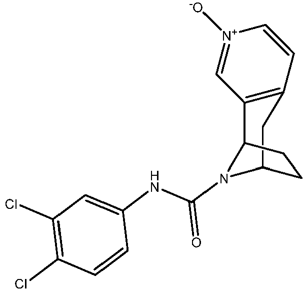 | (59) | 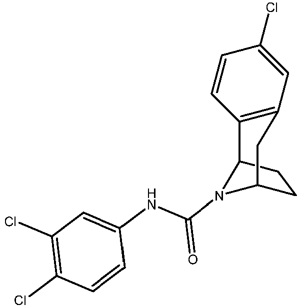 | (60) |
| 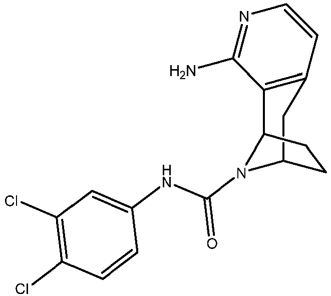 | (61) | 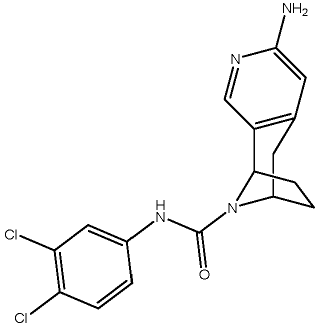 | (62) |
| 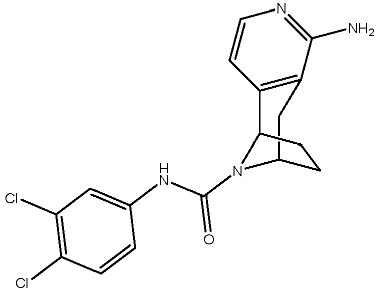 | (63) | 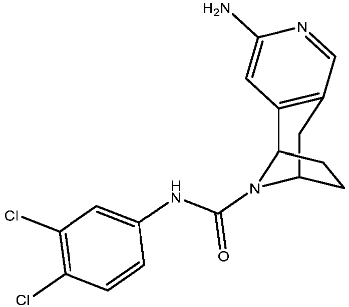 | (64) |
| 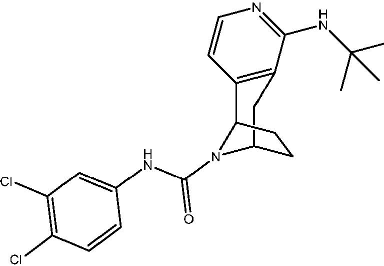 | (65) | 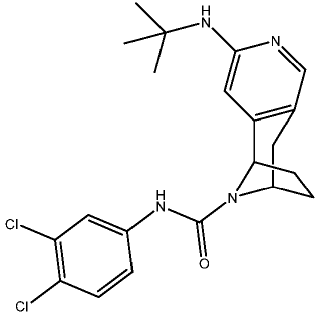 | (66) |
| 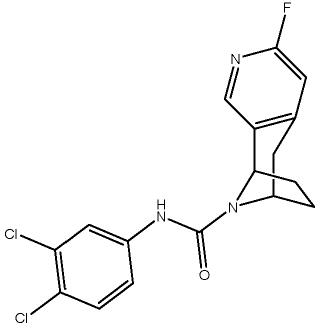 | (67) | 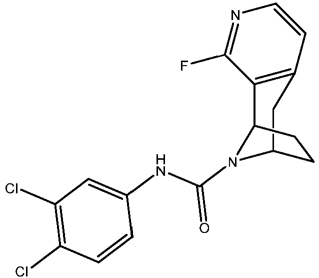 | (68) |
| 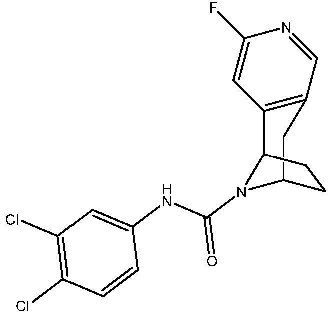 | (69) | 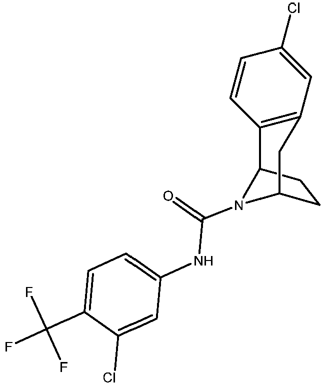 | (71) |
| 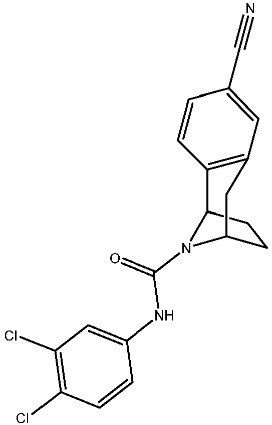 | (72) | 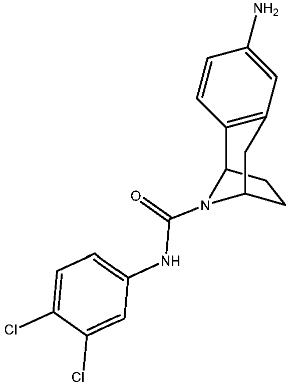 | (73) |
| 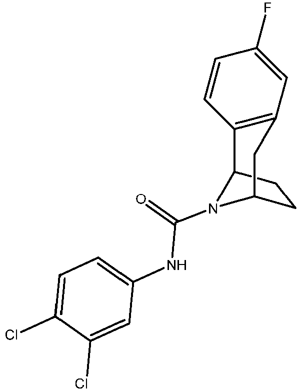 | (74) | 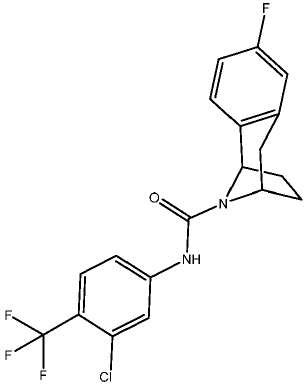 | (75) |
| 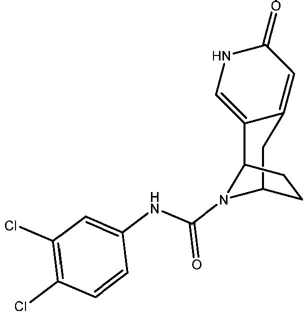 | (76) | 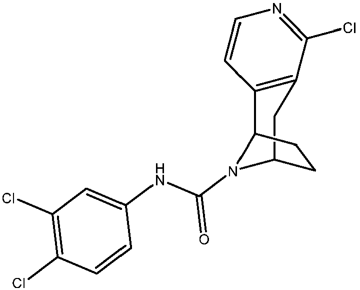 | (77) |
| 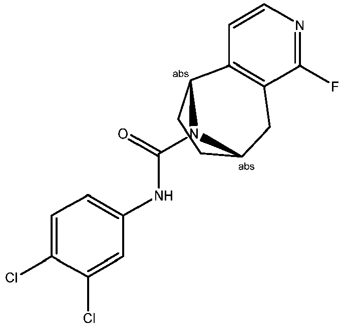 | (78) | 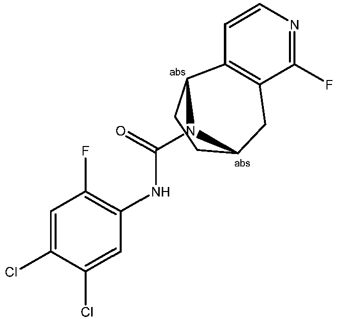 | (79) |
| 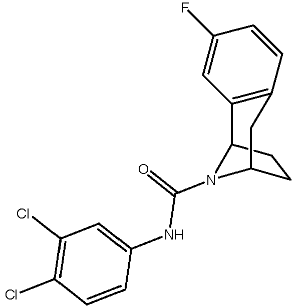 | (80) | 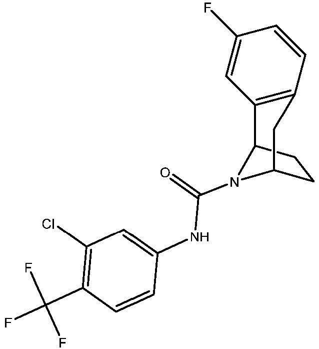 | (81) |
| 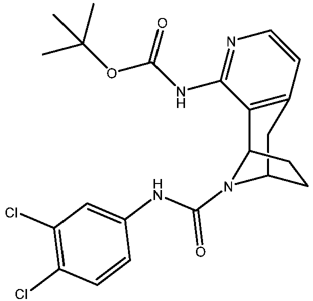 | (82) | 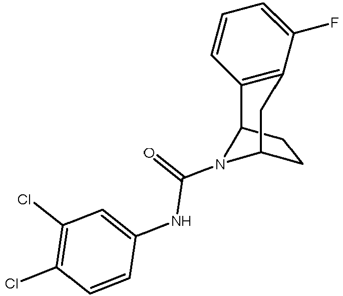 | (83) |
| 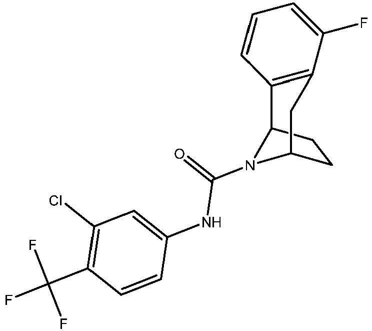 | (84) | 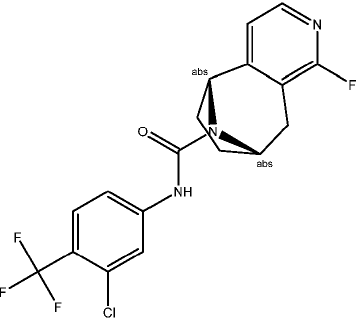 | (85) |
| 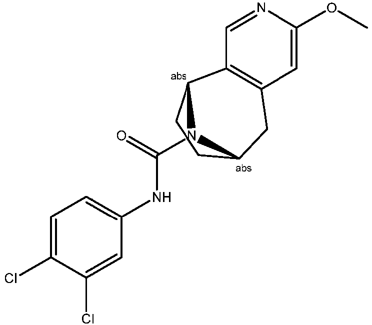 | (86) | 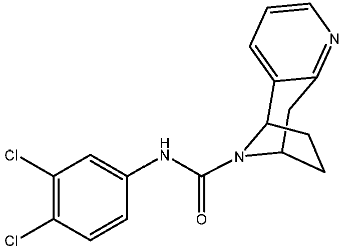 | (87) |
| 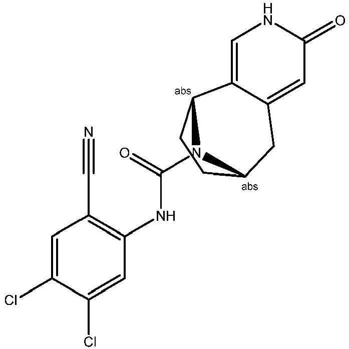 | (88) | 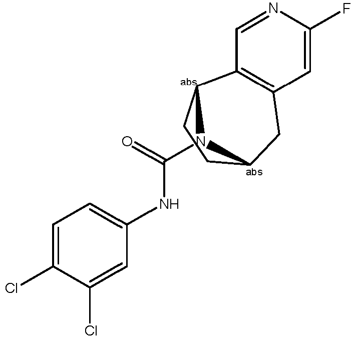 | (91) |
| 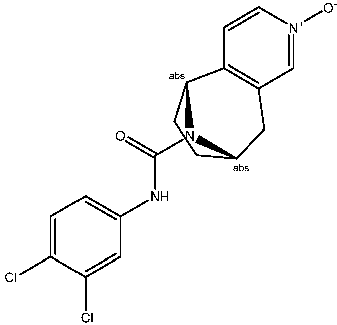 | (92) | 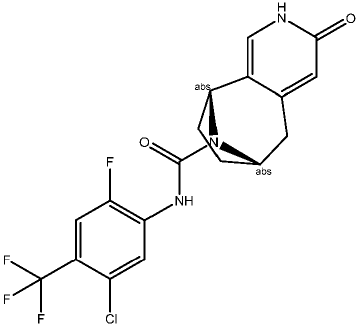 | (93) |
| 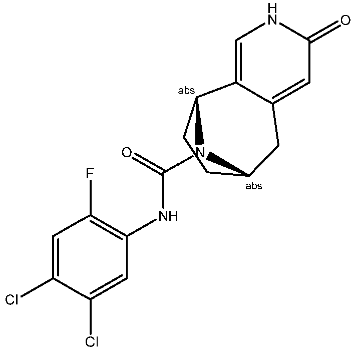 | (94) | 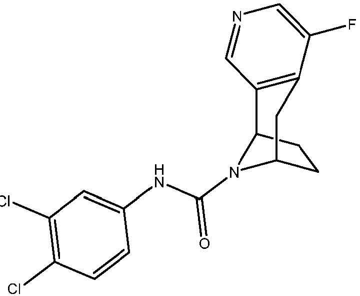 | (95) |
| 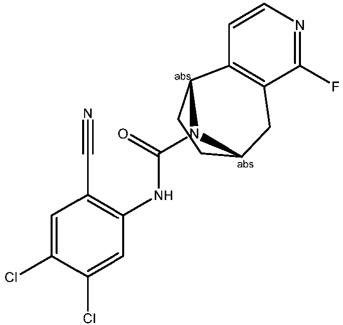 | (98) | 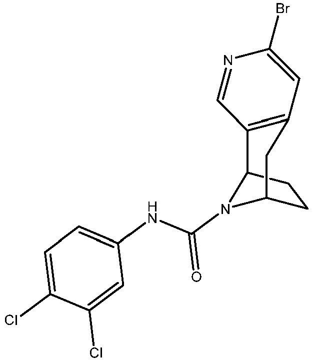 | (99) |
| 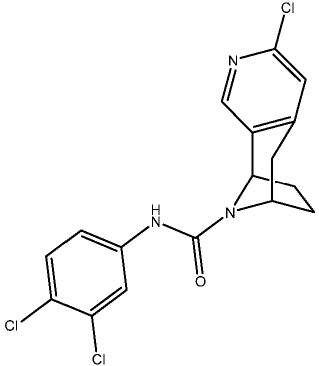 | (100) | F 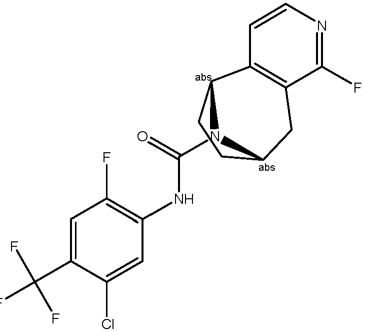 | (101) |
| 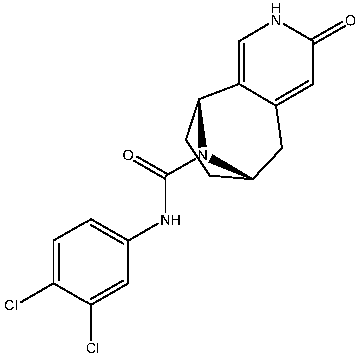 | (103) | 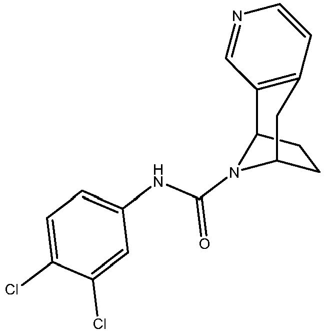 | (104) |
| 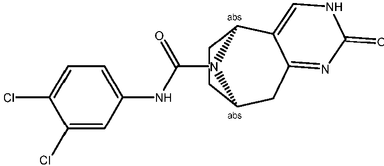 Enantiomer of (31) | (105) | 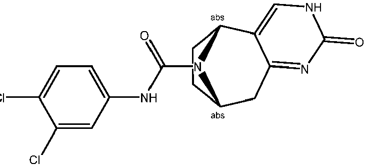 Enantiomer of (31) | (106) |
| 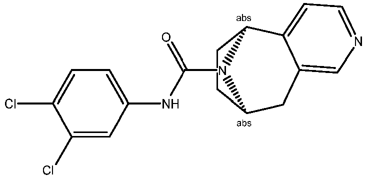 | (107) | 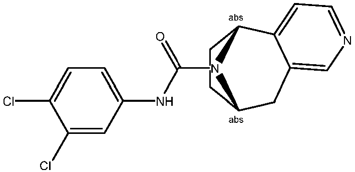 | (108) |
| 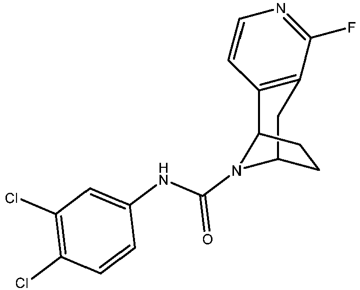 | (109) | 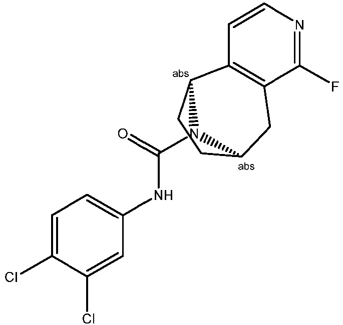 | (110) |
| 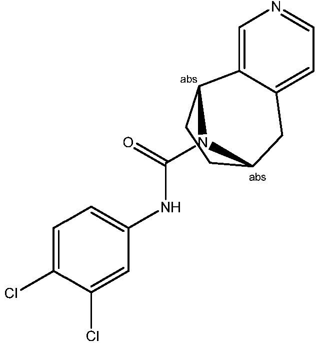 | (111) | 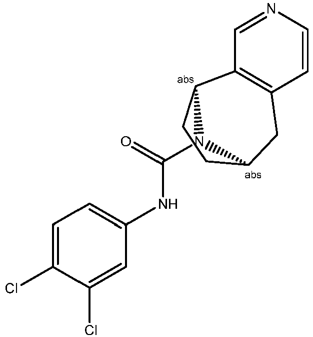 | (112) |
| 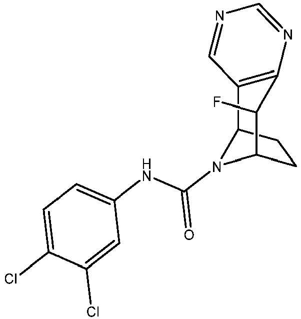 | (113) | 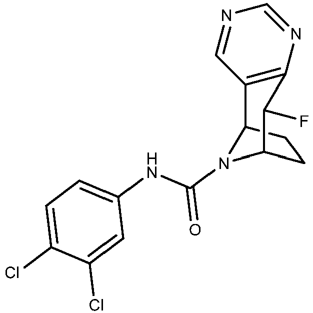 | (114) |
| 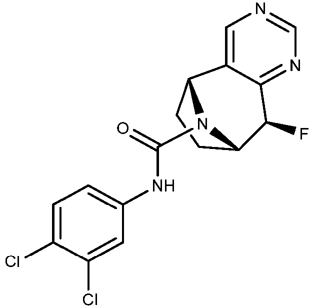 | (115) | | |
| 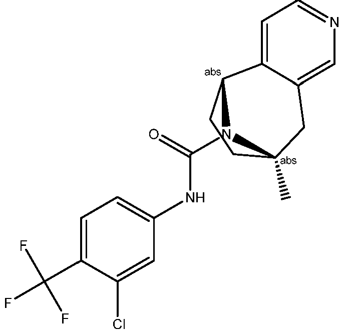 | (116) | 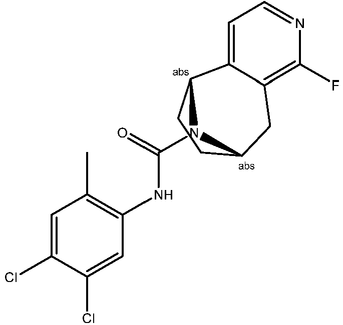 | (117) |
| 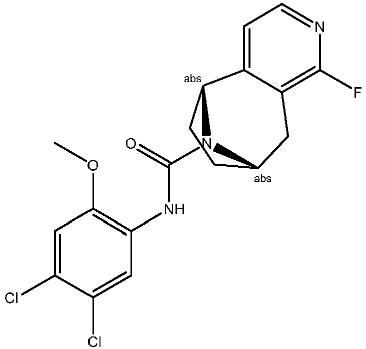 | (118) | 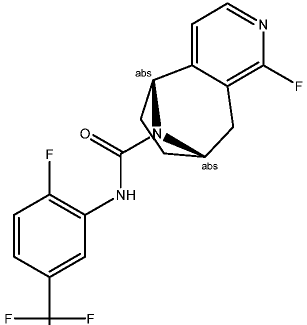 | (119) |
| 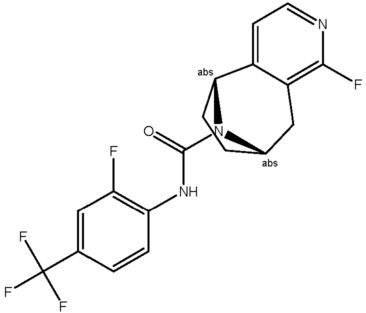 | (120) | 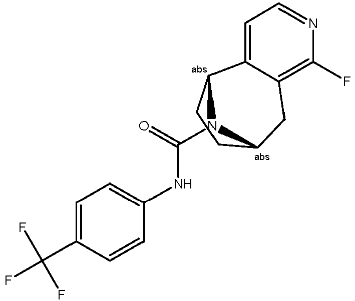 | (121) |
| 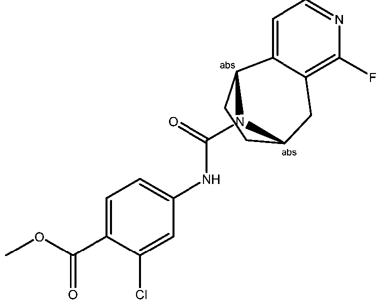 | (122) | 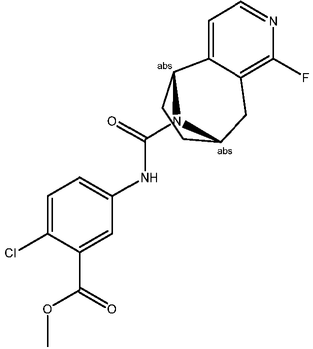 | (123) |
| 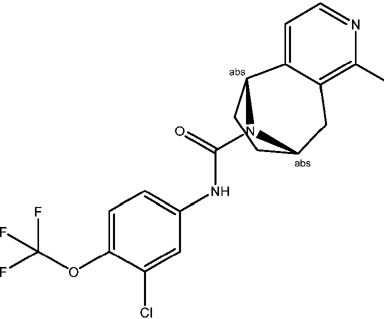 | (124) | 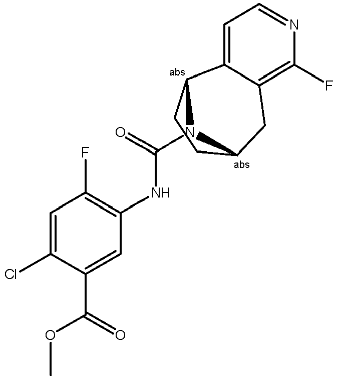 | (125) |
| 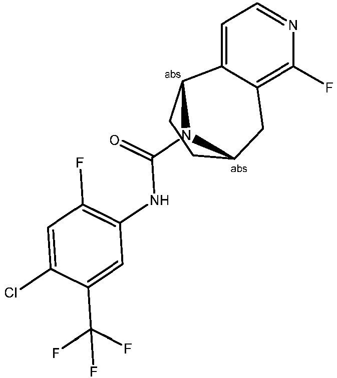 | (126) | 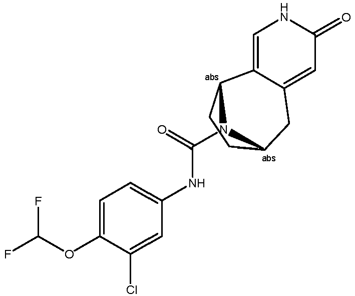 | (127) |
| 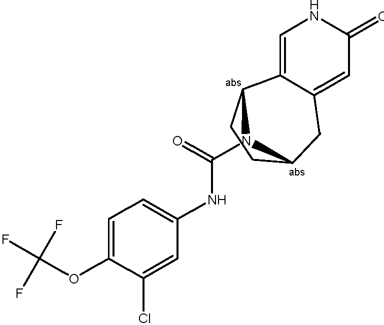 | (128) | 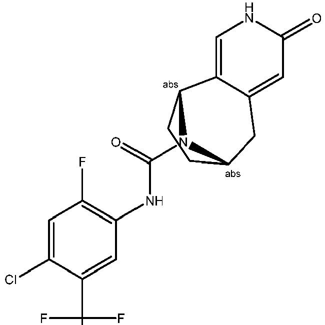 | (129) |
| 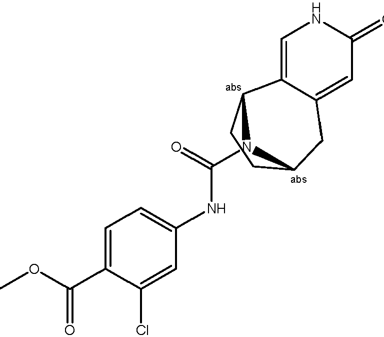 | (130) | 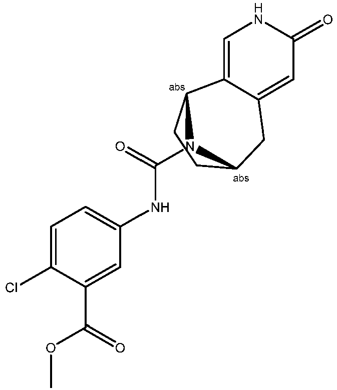 | (131) |
| 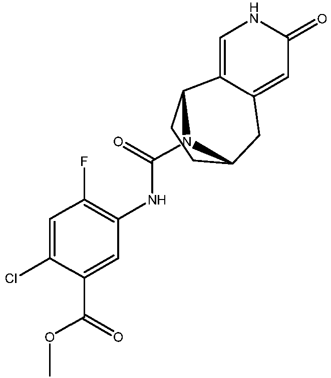 | (132) | 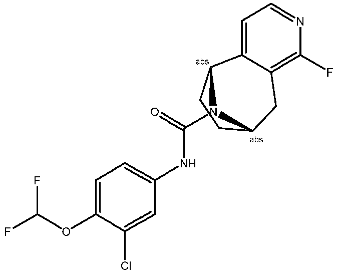 | (133) |
| 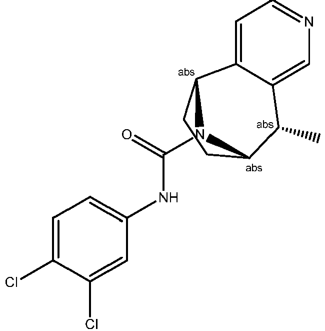 | (134) | 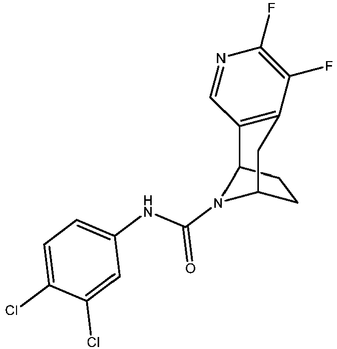 | (135) |
| 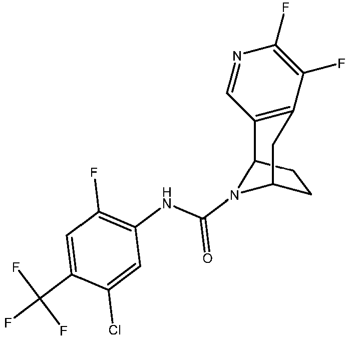 | (136) | 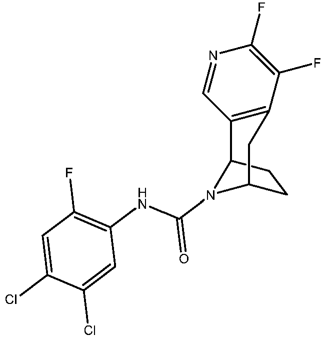 | (137) |
| 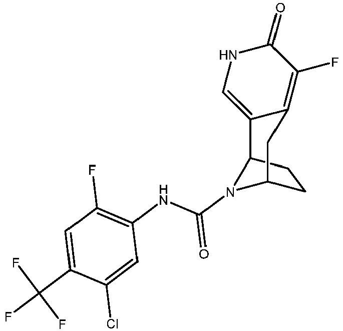 | (138) | 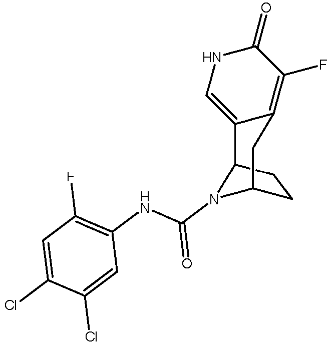 | (139) |
| 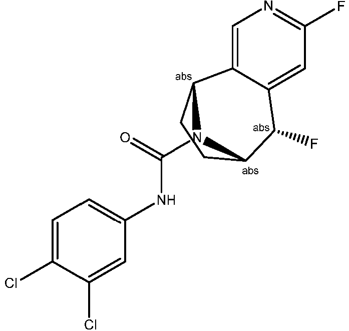 | (140) | 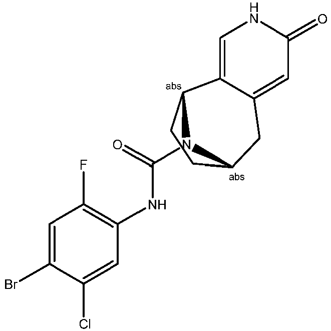 | (141) |
| 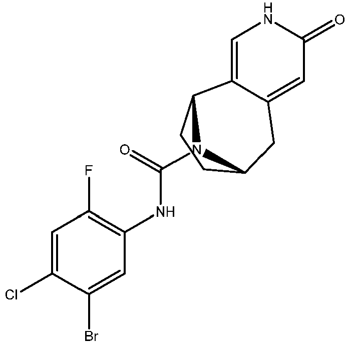 | (142) | 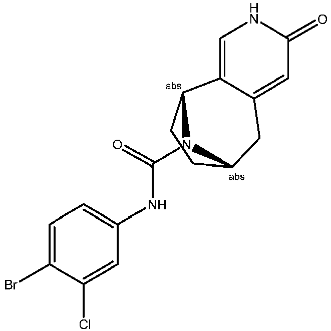 | (143) |
| 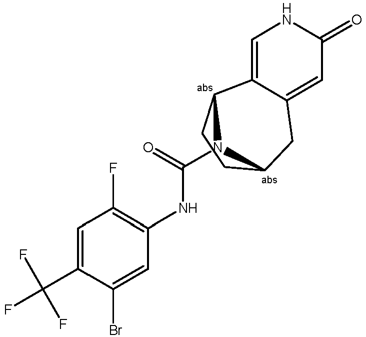 | (144) | 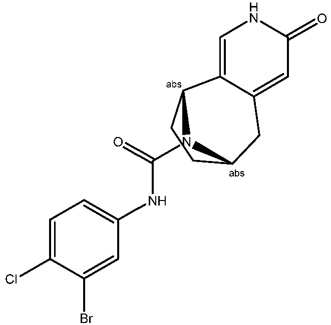 | (145) |
| 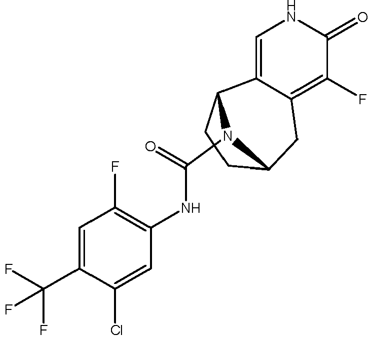 | (146) | 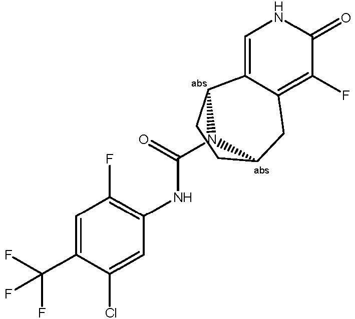 | (147) |
| 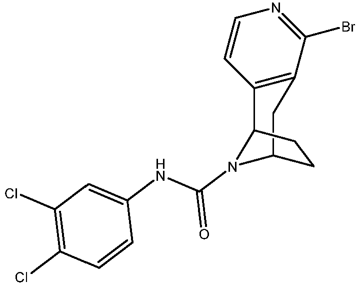 | (148) | 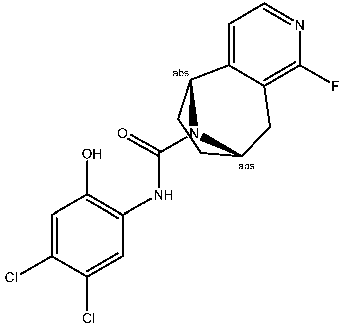 | (149) |
| 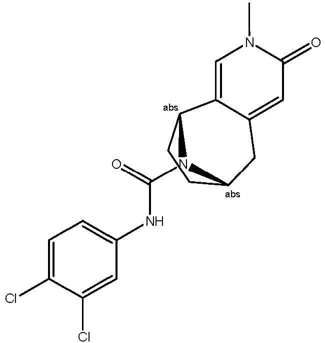 | (150) | | |
| 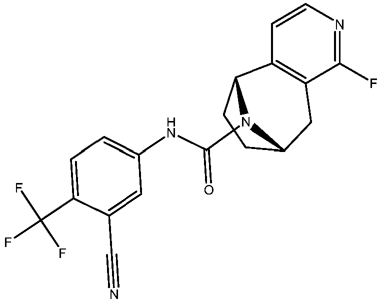 | (151) | 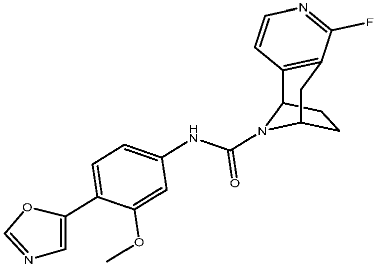 | (152) |
| 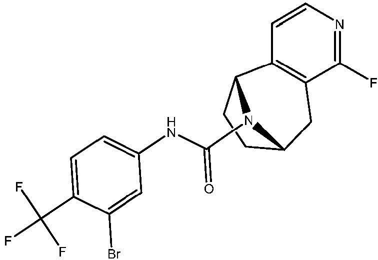 | (153) | 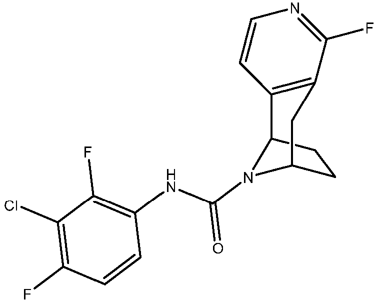 | (154) |
| 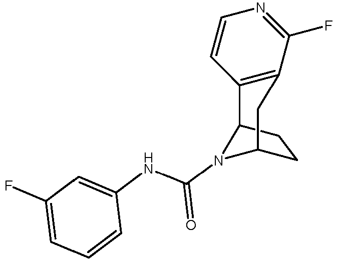 | (155) | 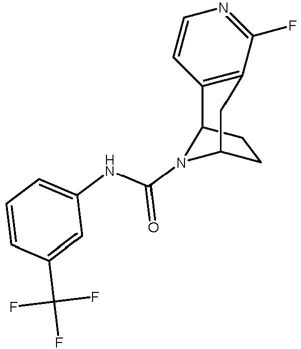 | (156) |
| 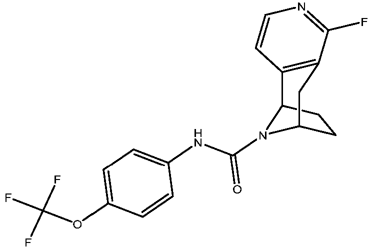 | (157) | 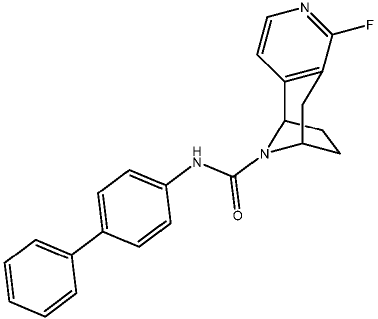 | (158) |
| 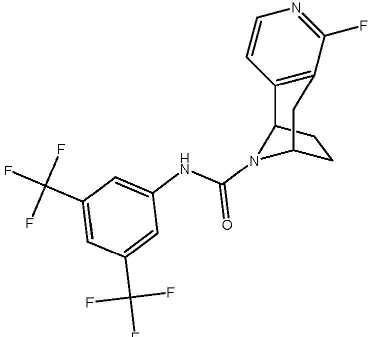 | (159) | 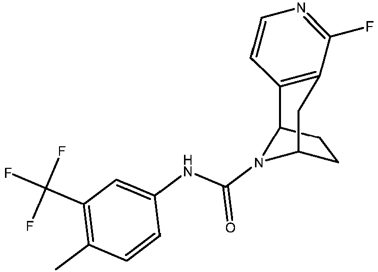 | (160) |
| 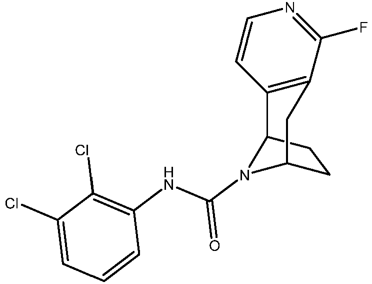 | (161) | 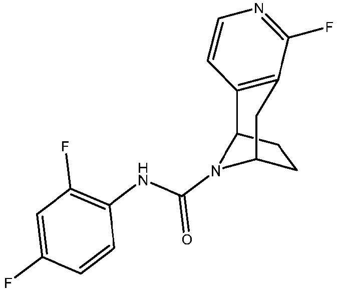 | (162) |
| 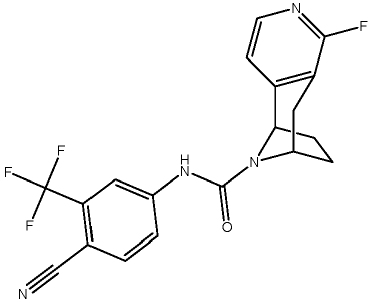 | (163) | 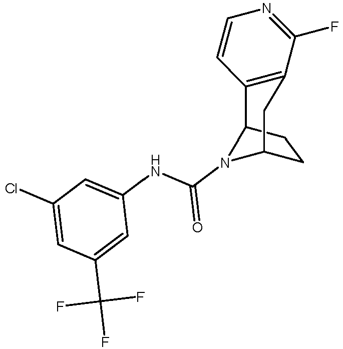 | (164) |
| 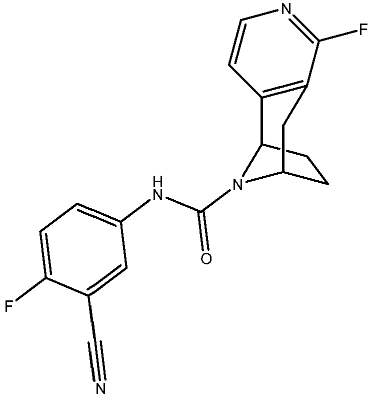 | (165) | 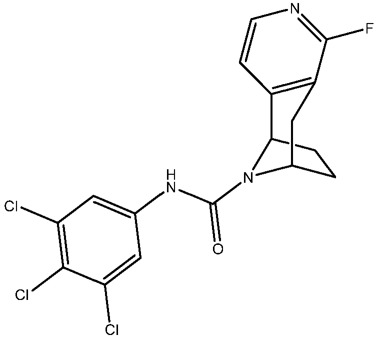 | (166) |
| 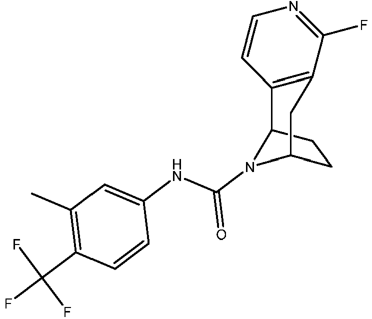 | (167) | 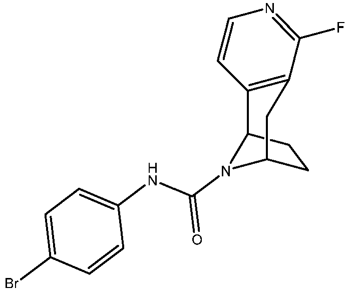 | (168) |
| 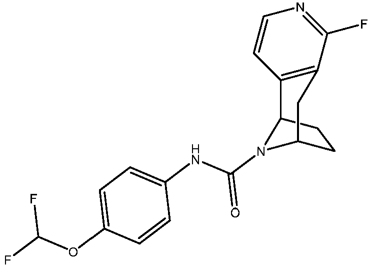 | (169) | 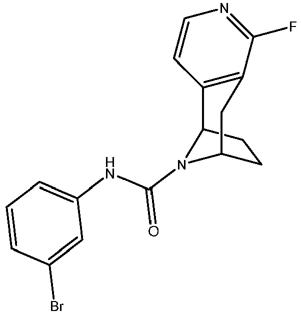 | (170) |
| 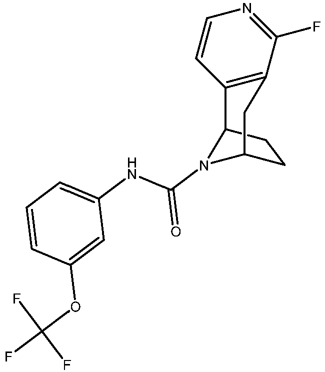 | (171) | 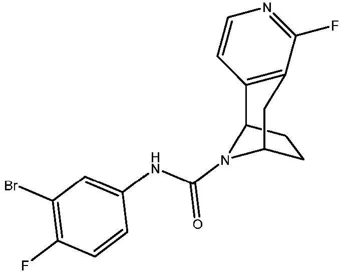 | (172) |
| 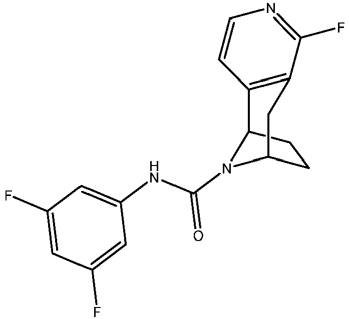 | (173) | 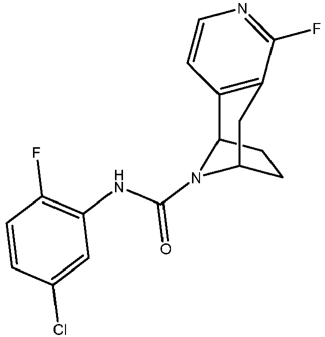 | (174) |
| 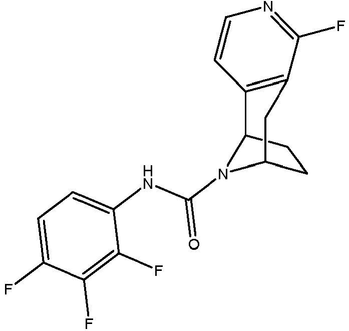 | (175) | 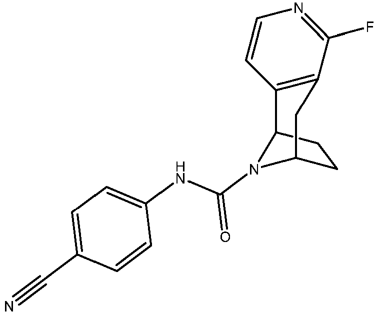 | (176) |
| 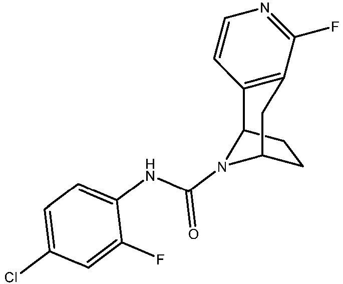 | (177) | 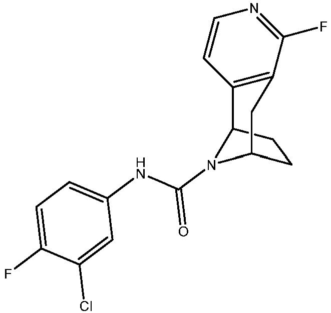 | (178) |
| 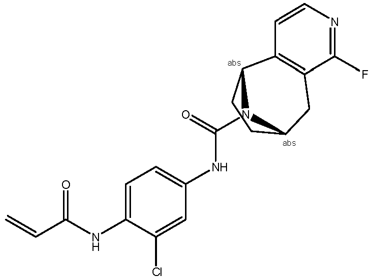 | (179) | 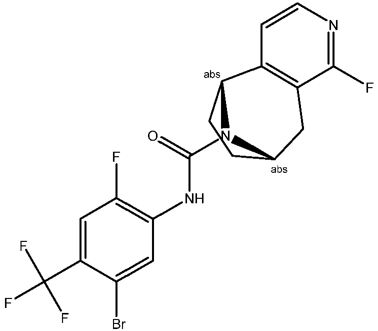 | (180) |
| 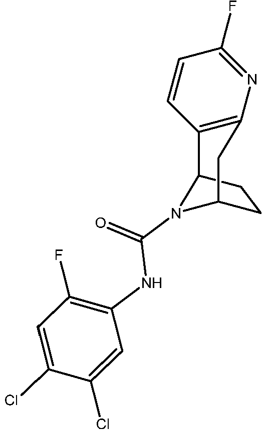 | (181) | 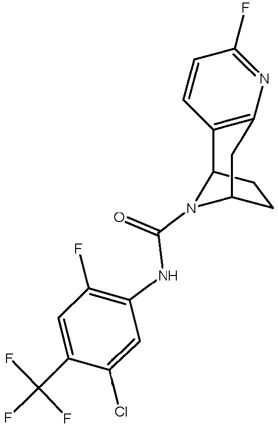 | (182) |
| 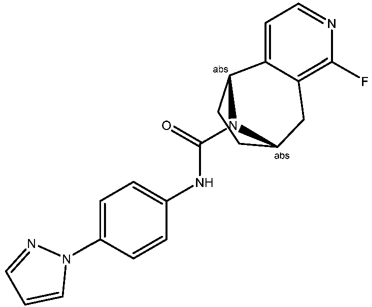 | (183) | 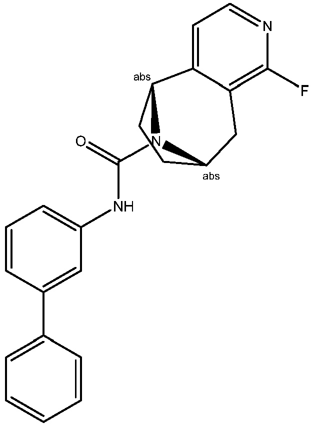 | (184) |
| 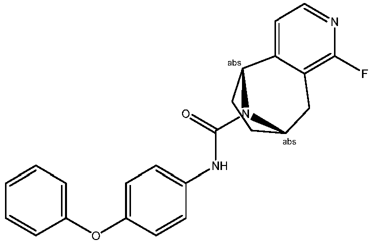 | (185) | 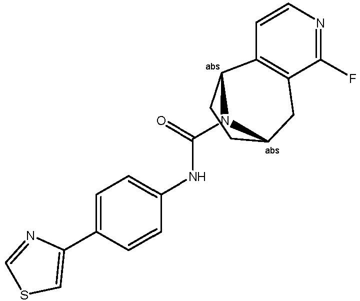 | (186) |
| 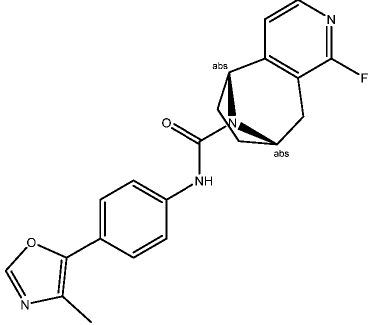 | (187) | 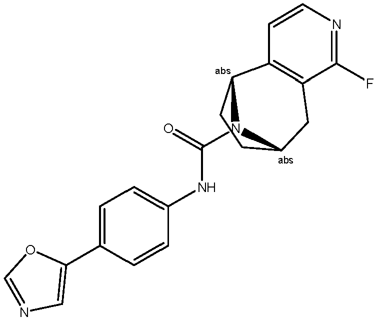 | (188) |
| 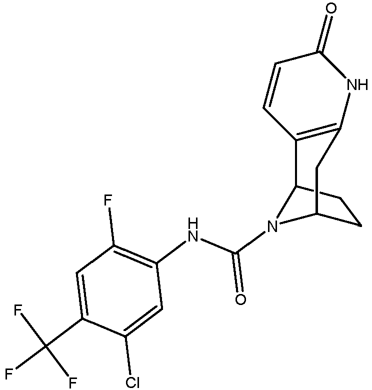 | (189) | 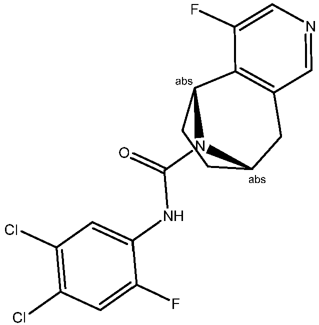 | (190) |
| 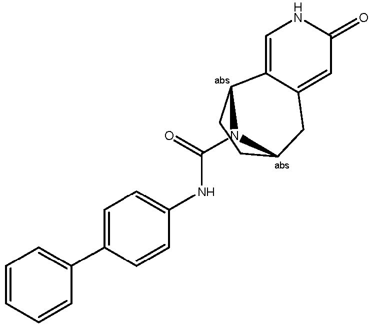 | (191) | 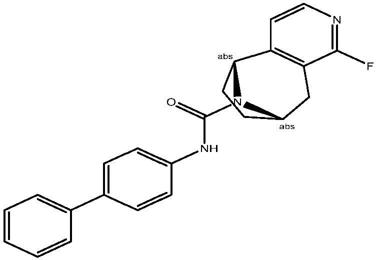 | (192) |
| 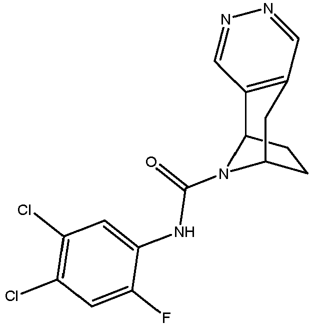 | (193) | 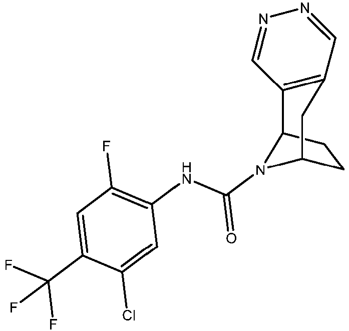 | (194) |
| 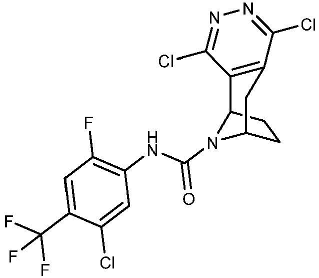 | (195) | 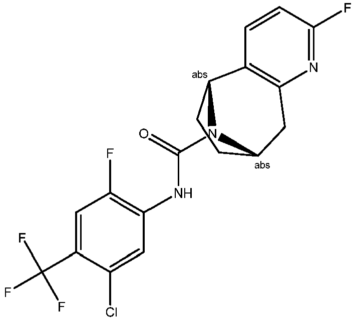 | (196) |
| 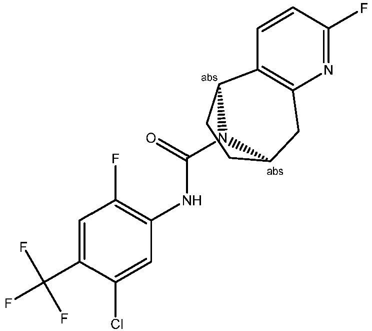 | (197) | 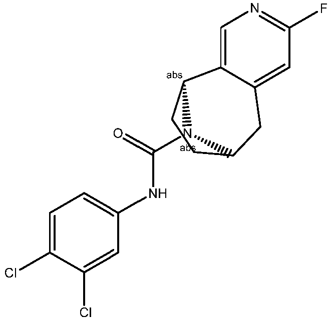 | (198) |
| 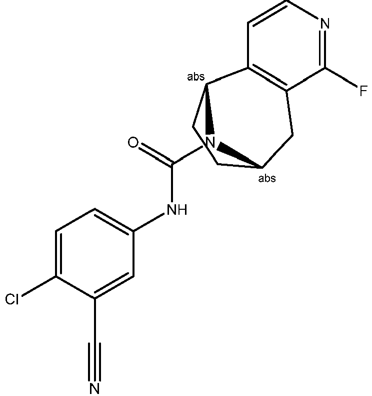 | (199) | 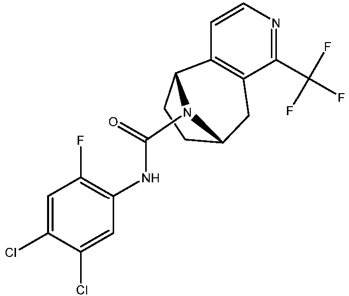 | 200 |
and enantiomers thereof, and mixtures of enantiomers thereof, including racemic mixtures, and pharmaceutically acceptable salts and solvates thereof; for use in treating or preventing a disorder selected from a proliferative disorder, an immune disorder, asthma, chronic obstructive pulmonary disease (COPD) and acute respiratory distress syndrome (ARDS).

## Patentansprüche

1. Verbindung der Formel (If) oder pharmazeutisch unbedenkliches Salz oder Solvat davon, 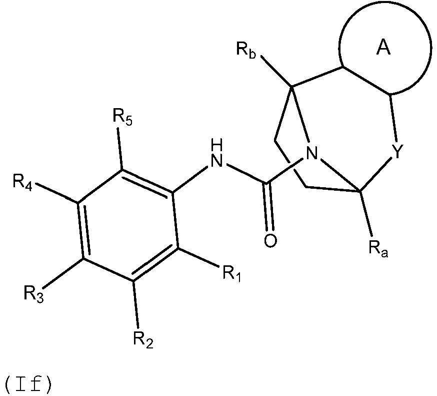 wobei:
Ring A ein Pyridinylring oder Tautomer davon oder ein Phenylring ist, wobei jeder dieser Ringe gegebenenfalls durch einen oder mehrere Substituenten, die aus H, F, Cl, Br, I, CN, Alkoxy, NR₁₁R₁₁', OH, Alkyl, Phenyl und Halogenalkyl ausgewählt sind, substituiert sein kann;
Y aus C=N-OH und CR₁₀R₁₀' ausgewählt ist, wobei R₁₀ und R₁₀' jeweils unabhängig aus H, F, Alkyl und Halogenalkyl ausgewählt sind;
Rₐ und R_{b} jeweils unabhängig aus H und Alkyl ausgewählt sind;
R₁, R₄ und R₅ jeweils unabhängig aus H, CN, Alkyl, Alkoxy, Halogenalkyl, OH, F, Cl, Br und I ausgewählt sind;
R₂ und R₃ jeweils unabhängig aus H, F, Cl, Br, I, CN, Alkoxy, Halogenalkyl, Halogenalkoxy, Alkyl, Aryl, Heteroaryl, O-Aryl, NHCO-Alkenyl und CO₂-Alkyl ausgewählt sind, wobei die Aryl-, Heteroaryl- und O-Aryl-Gruppen jeweils gegebenenfalls weiter durch eine oder mehrere Gruppen, die unabhängig aus Halogen, Alkyl und Alkoxy ausgewählt sind, substituiert sind; und
R₁₁ und R₁₁' jeweils unabhängig aus H, Alkyl, Halogenalkyl, COR₁₂, CO₂R₁₂ und SO₂R₁₃ ausgewählt sind, wobei R₁₂ und R₁₃ beide unabhängig für Alkyl stehen.

2. Verbindung nach Anspruch 1, wobei Ring A aus den folgenden Gruppen ausgewählt ist: 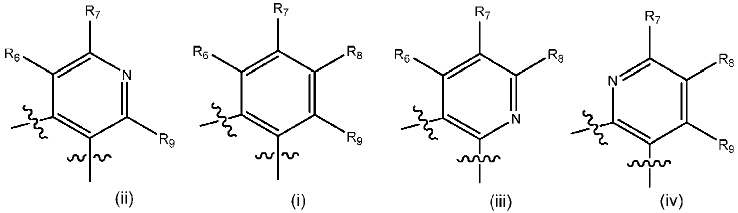 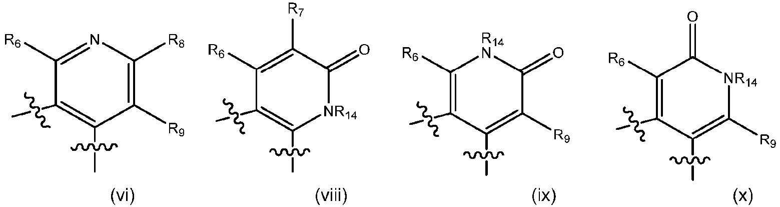 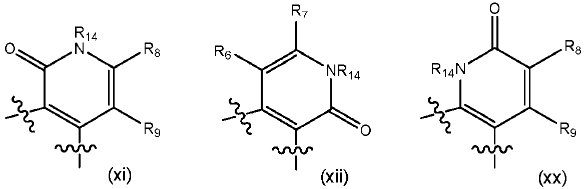 wobei R₆, R₇, R₈ und R₉ jeweils unabhängig aus H, F, Cl, Br, I, CN, Alkoxy, NR₁₁R₁₁', OH, Alkyl, Phenyl und Halogenalkyl ausgewählt sind und R₁₄ für H oder Alkyl, weiter bevorzugt H, steht.

3. Verbindung der Formel (Ib) oder pharmazeutisch unbedenkliches Salz oder Solvat davon, 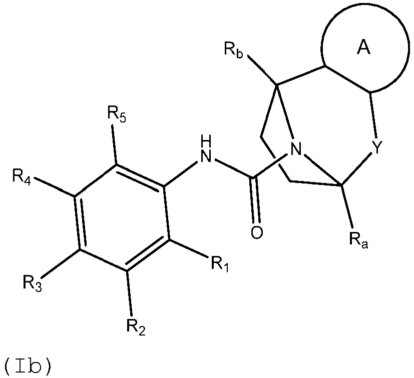 wobei:
Ring A aus den Gruppen (i)-(xx) ausgewählt ist: 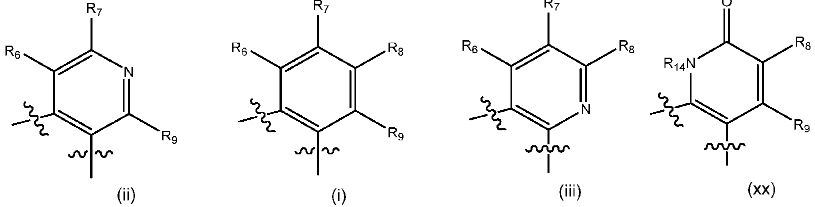 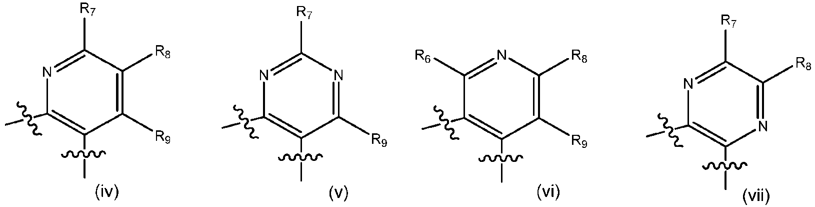 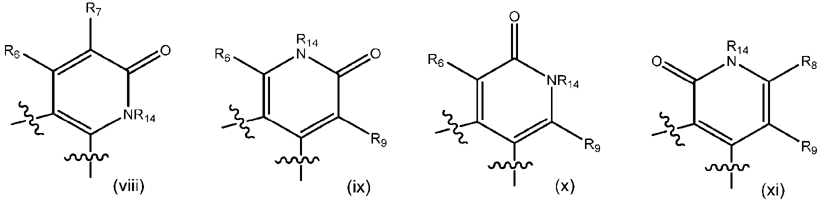 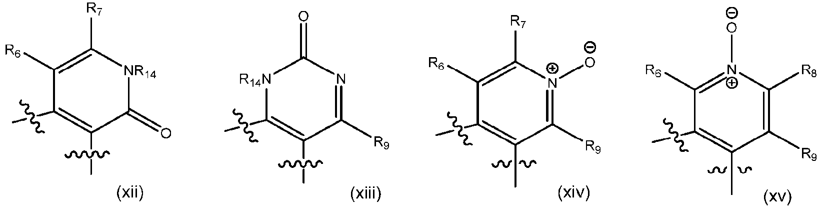 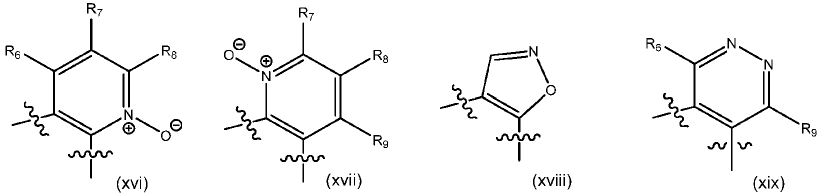
wobei R₆, R₇, R₈ und R₉ jeweils unabhängig aus H, F, Cl, Br, I, CN, Alkoxy, NR₁₁R₁₁', OH, Alkyl, Phenyl und Halogenalkyl ausgewählt sind und R₁₄ für H oder Alkyl, weiter bevorzugt H, steht;
Y aus C=N-OH und CR₁₀R₁₀' ausgewählt ist, wobei R₁₀ und R₁₀' jeweils unabhängig aus H, F, Alkyl und Halogenalkyl ausgewählt sind;
Rₐ und R_{b} jeweils unabhängig aus H und Alkyl ausgewählt sind;
R₁, R₄ und R₅ jeweils unabhängig aus H, CN, Alkyl, Alkoxy, Halogenalkyl, OH, F, Cl, Br und I ausgewählt sind;
R₂ und R₃ jeweils unabhängig aus H, F, Cl, Br, I, CN, Alkoxy, Halogenalkyl, Halogenalkoxy, Alkyl, Aryl, Heteroaryl, O-Aryl, NHCO-Alkenyl und CO₂-Alkyl ausgewählt sind, wobei die Aryl-, Heteroaryl- und O-Aryl-Gruppen jeweils gegebenenfalls weiter durch eine oder mehrere Gruppen, die unabhängig aus Halogen, Alkyl und Alkoxy ausgewählt sind, substituiert sind; und
R₁₁ und R₁₁' jeweils unabhängig aus H, Alkyl, Halogenalkyl, COR₁₂, CO₂R₁₂ und SO₂R₁₃ ausgewählt sind, wobei R₁₂ und R₁₃ beide unabhängig für Alkyl stehen.

4. Verbindung nach Anspruch 3, wobei Ring A aus (i), (ii), (iii), (iv), (v), (vi), (viii), (ix), (xiv), (xv) and (xix) ausgewählt ist und vorzugsweise aus ((i), (ii), (iii), (v), (vi) und (ix) ausgewählt ist und noch weiter bevorzugt aus (i), (ii), (vi) und (ix) ausgewählt ist.

5. Verbindung nach einem der vorhergehenden Ansprüche mit der Formel: 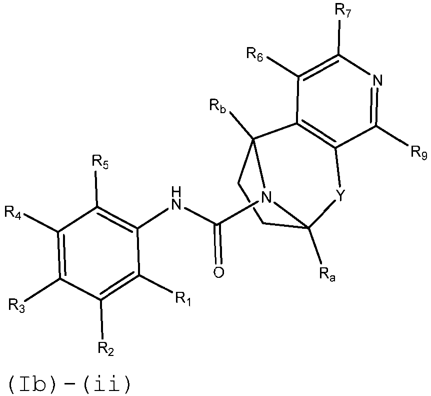
wobei Y, Rₐ, R_{b}, R₁-R₅ wie in Anspruch 1 definiert sind und R₆, R₇ und R₉ wie in Anspruch 2 definiert sind; oder 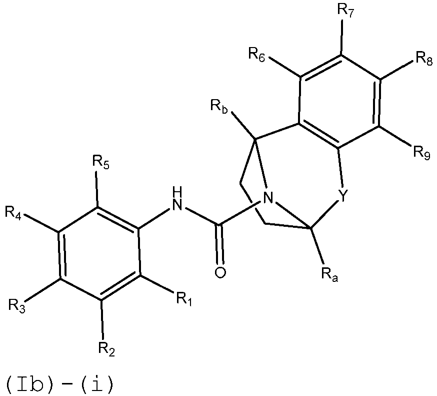
wobei Y, Rₐ, R_{b}, R₁-R₅ wie in Anspruch 1 definiert sind und R₆-R₉ wie in Anspruch 2 definiert sind; oder 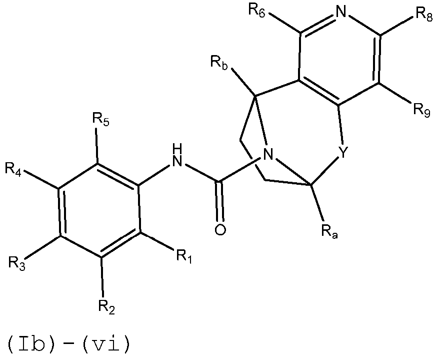
wobei Y, Rₐ, R_{b}, R₁-R₅ wie in Anspruch 1 definiert sind und R₆, R₈ und R₉ wie in Anspruch 2 definiert sind; oder 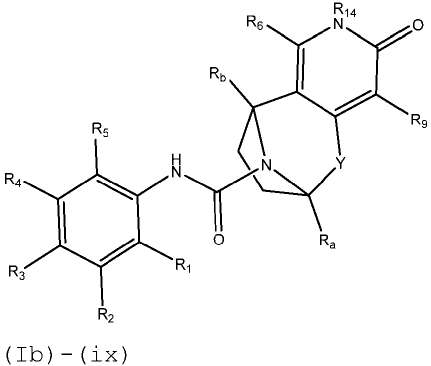 wobei Y, Rₐ, R_{b}, R₁-R₅ wie in Anspruch 1 definiert sind und R₆, R₉ und R₁₄ wie in Anspruch 2 definiert sind.

6. Verbindung nach einem der vorhergehenden Ansprüche, wobei Y aus CH₂ und C=N-OH ausgewählt ist und vorzugsweise für CH₂ steht.

7. Verbindung nach einem der vorhergehenden Ansprüche, wobei R₂ und R₃:
(i) jeweils unabhängig aus H, F, Cl, Br, CN, Methoxy, OCF₃, CF₃, OCHF₂, Me, Ph, Pyrazolyl, Oxazolyl, Thiazolyl, OPh, NHCO-CH=CH₂ und CO₂Me ausgewählt sind, wobei die Ph- , OPh-, Pyrazolyl-, Oxazolyl- und Thiazolylgruppen jeweils gegebenenfalls weiter durch eine oder mehrere Alkylgruppen substituiert sind;
(ii) jeweils unabhängig aus F, Cl, Br, I, CN, C₁-C₆-Halogenalkyl, C₁-C₆-Halogenalkoxy und CO₂-Alkyl ausgewählt sind;
(iii) jeweils unabhängig aus Cl, Br und CF₃ ausgewählt sind und weiter bevorzugt jeweils unabhängig aus Cl und CF₃ ausgewählt sind; oder
(iv) beide für Cl stehen oder eines von R₂ und R₃ für Cl steht und das andere aus OCF₃, CO₂Me, OCHF₂ und CF₃ ausgewählt ist.

8. Verbindung nach einem der vorhergehenden Ansprüche, wobei R₁ und R₄ beide für H stehen.

9. Verbindung nach einem der vorhergehenden Ansprüche, wobei R₅ aus H, F, Me, MeO, Cl, OH und CN ausgewählt ist und vorzugsweise für H oder F, weiter bevorzugt H, steht.

10. Verbindung nach einem der Ansprüche 2 bis 9, wobei R₆, R₇, R₈ und R₉ jeweils unabhängig aus H, F, Cl, Br, CN, OMe, NH₂, NHBu, NHCO₂Bu und OH ausgewählt sind.

11. Verbindung nach einem der vorhergehenden Ansprüche mit der Formel (Ib.1): 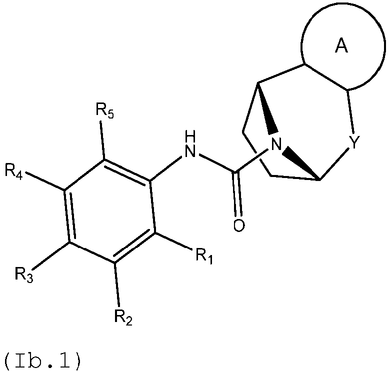 wobei A, Y und R₁-R₅ wie in einem der Ansprüche 1-10 definiert sind, oder die in Form eines Gemischs vorliegt, das mit einer Verbindung der Formel (Ib.1) enantiomerenangereichert ist.

12. Verbindung nach einem der Ansprüche 3-11, die aus den Folgenden ausgewählt ist:
| | | | |
|---|---|---|---|
| 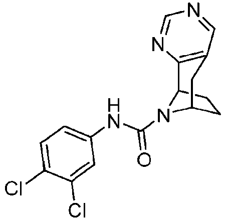 | (17) | 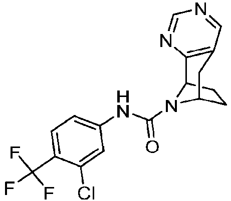 | (18) |
| 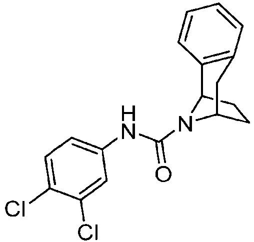 | (34) | 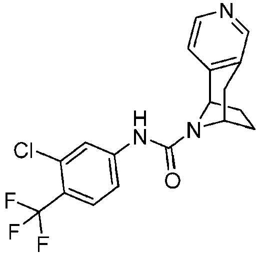 | (35) |
| 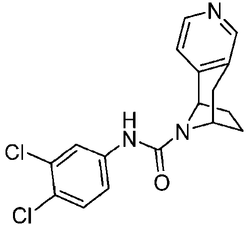 | (36) | 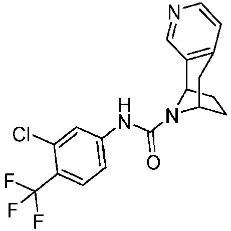 | (37) |
| | | 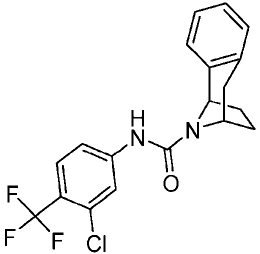 | (54) |
| 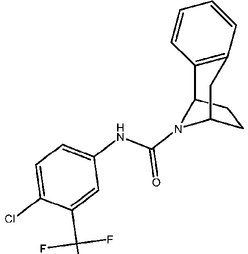 | (55) | 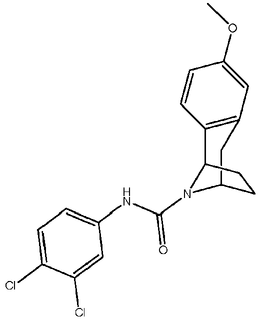 | (56) |
| 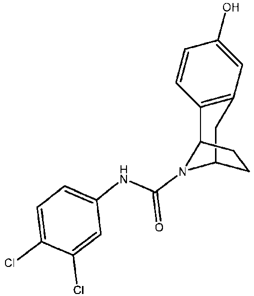 | (57) | 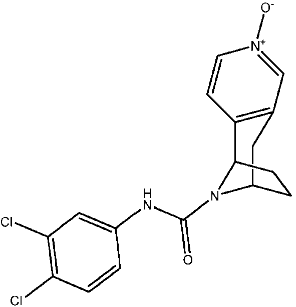 | (58) |
| 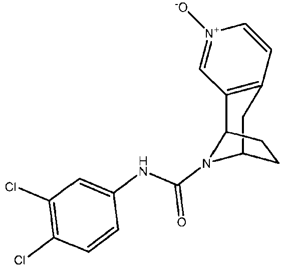 | (59) | 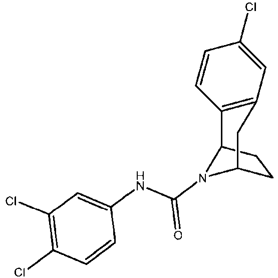 | (60) |
| 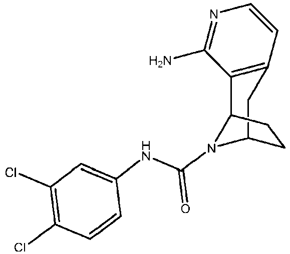 | (61) | 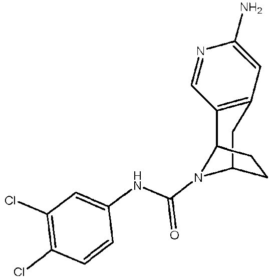 | (62) |
| 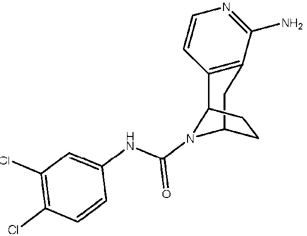 | (63) | 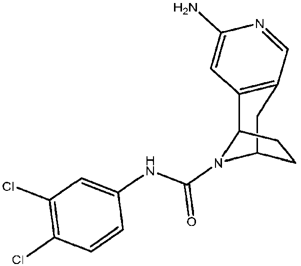 | (64) |
| 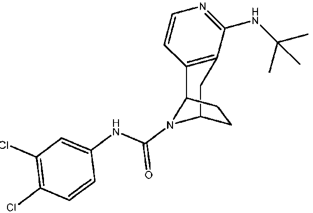 | (65) | 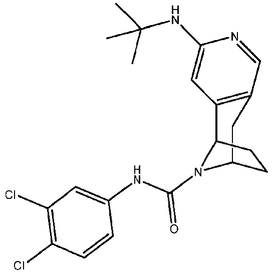 | (66) |
| 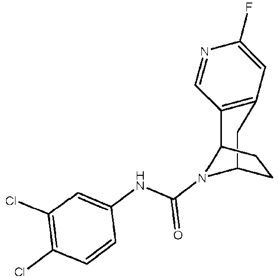 | (67) | 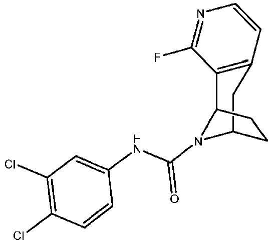 | (68) |
| 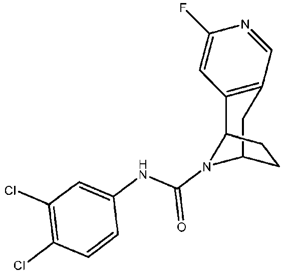 | (69) | 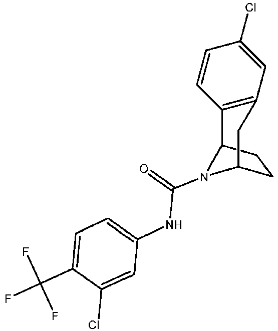 | (71) |
| 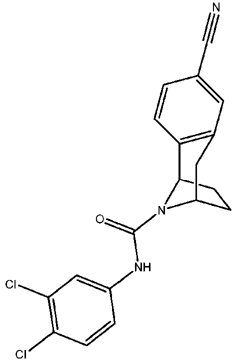 | (72) | 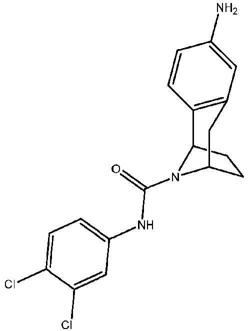 | (73) |
| 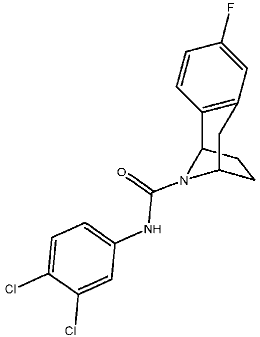 | (74) | 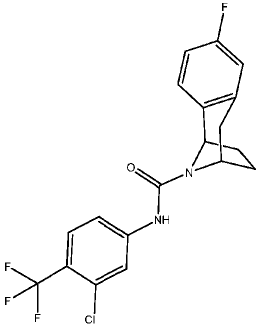 | (75) |
| 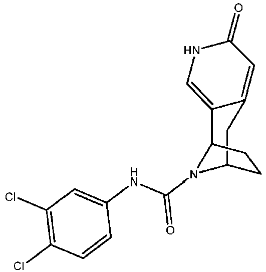 | (76) | 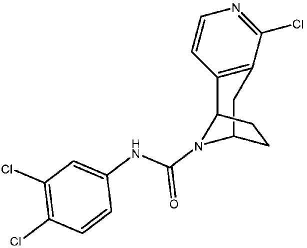 | (77) |
| 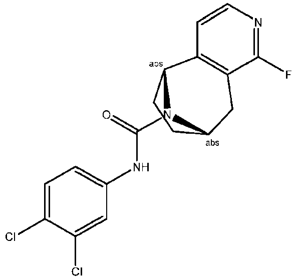 | (78) | 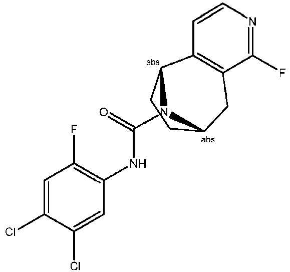 | (79) |
| 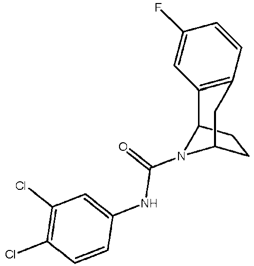 | (80) | 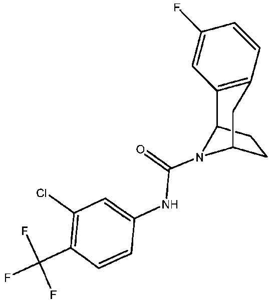 | (81) |
| 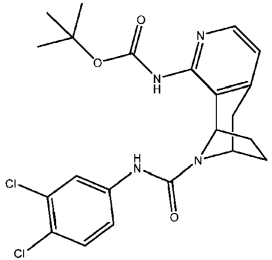 | (82) | 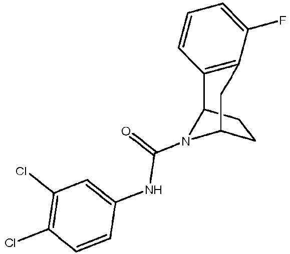 | (83) |
| 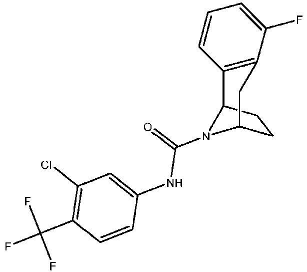 | (84) | 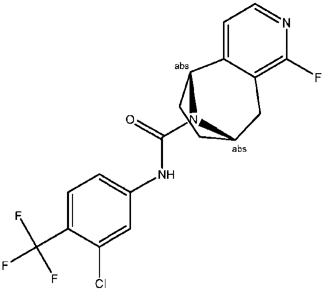 | (85) |
| 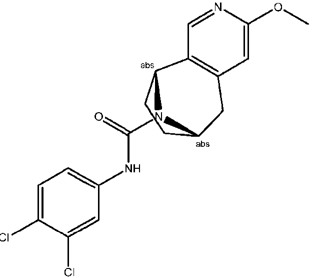 | (86) | 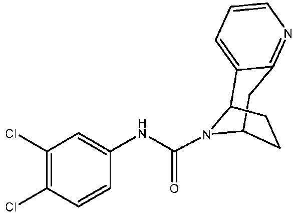 | (87) |
| 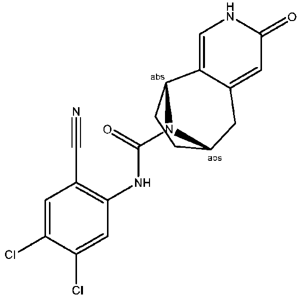 | (88) | 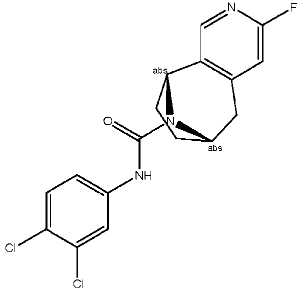 | (91) |
| 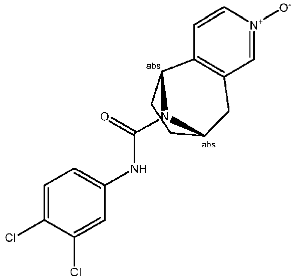 | (92) | 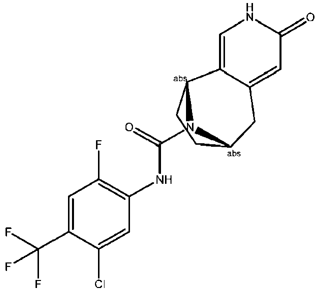 | (93) |
| 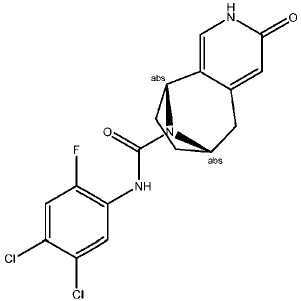 | (94) | 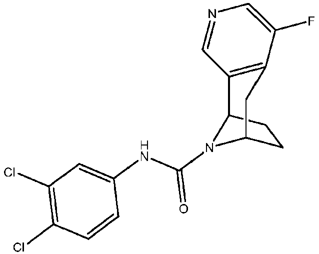 | (95) |
| 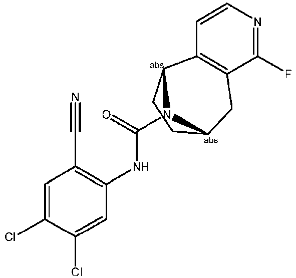 | (98) | 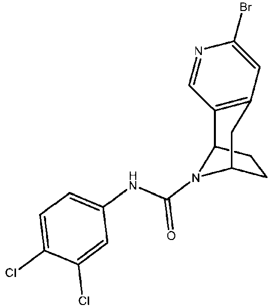 | (99) |
| 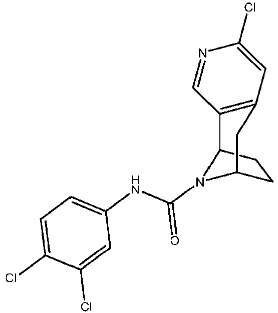 | (100) | 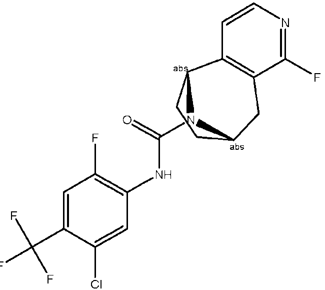 | (101) |
| 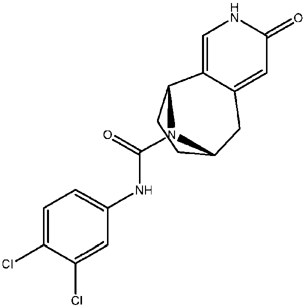 | (103) | 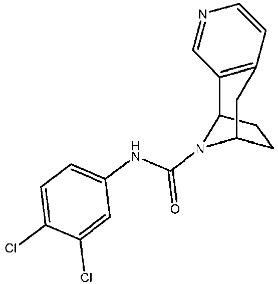 | (104) |
| 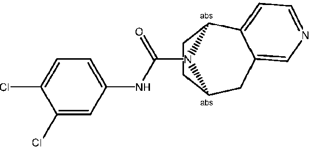 | (107) | 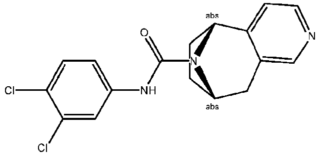 | (108) |
| 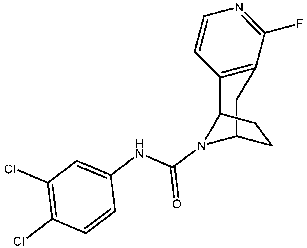 | (109) | 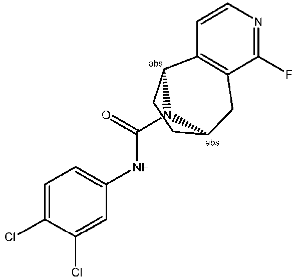 | (110) |
| 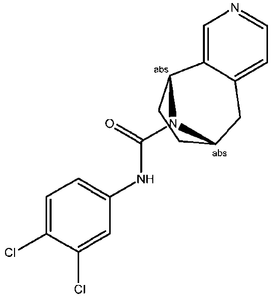 | (111) | 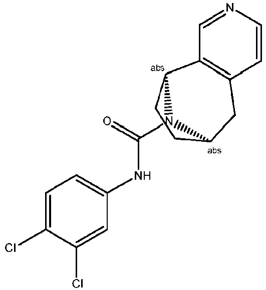 | (112) |
| 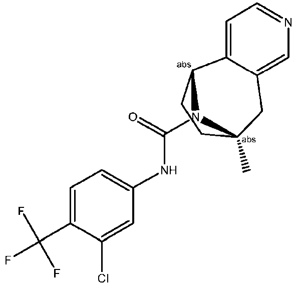 | (116) | 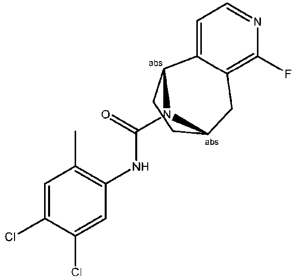 | (117) |
| 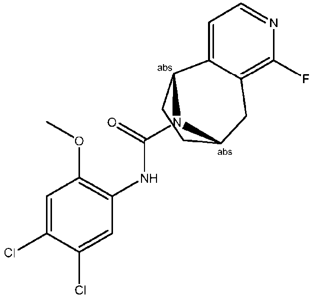 | (118) | 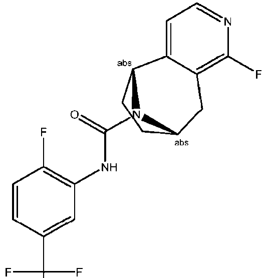 | (119) |
| 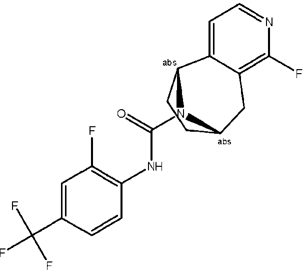 | (120) | 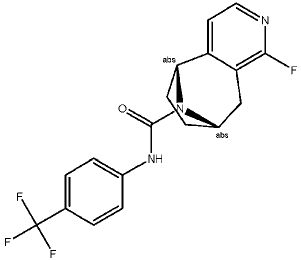 | (121) |
| 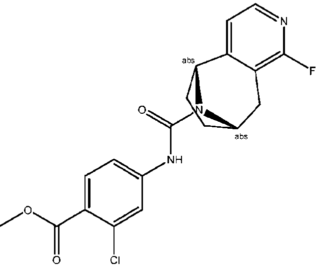 | (122) | 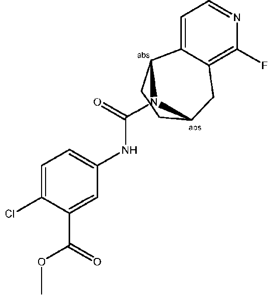 | (123) |
| 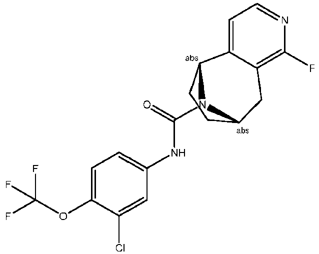 | (124) | 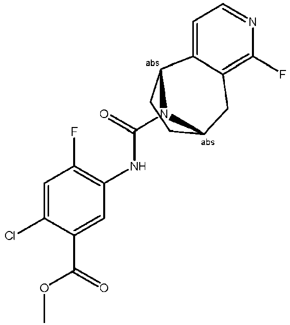 | (125) |
| 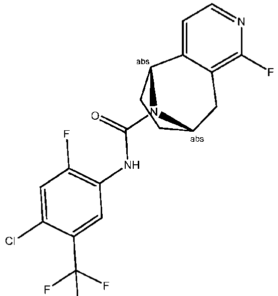 | (126) | 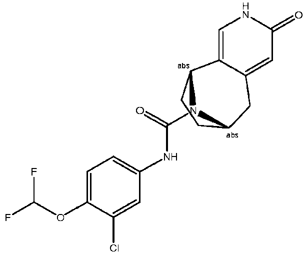 | (127) |
| 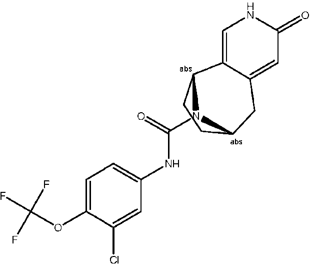 | (128) | 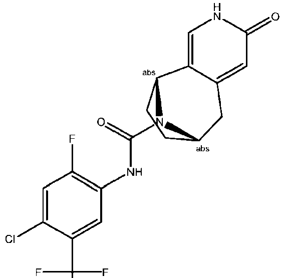 | (129) |
| 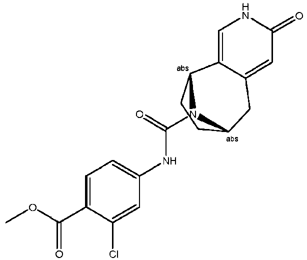 | (130) | 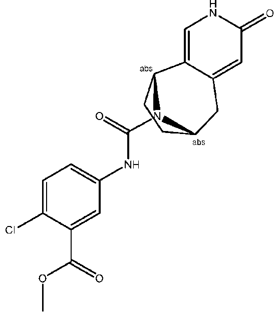 | (131) |
| 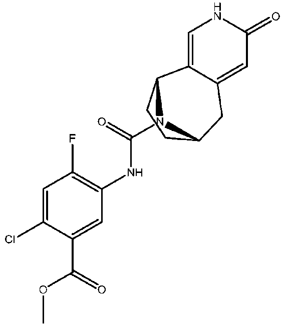 | (132) | 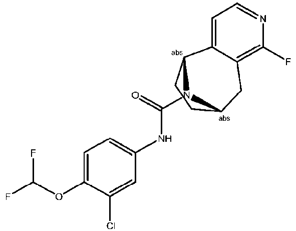 | (133) |
| 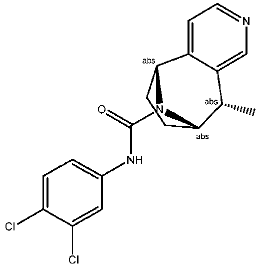 | (134) | 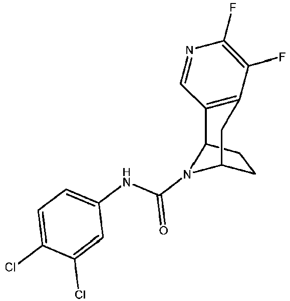 | (135) |
| 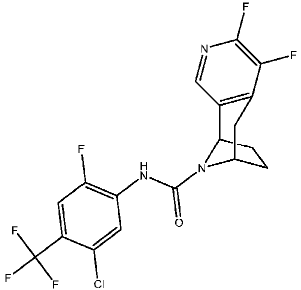 | (136) | 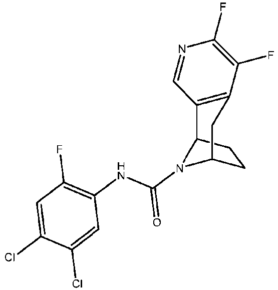 | (137) |
| 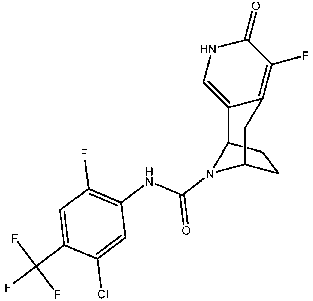 | (138) | 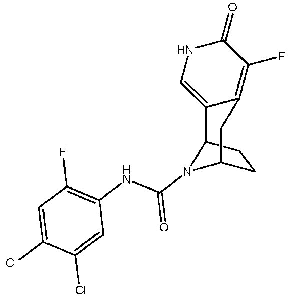 | (139) |
| 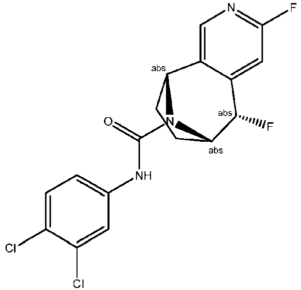 | (140) | 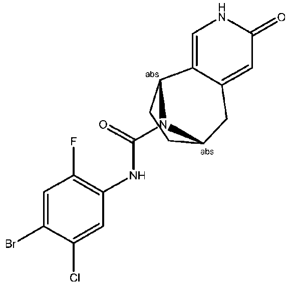 | (141) |
| 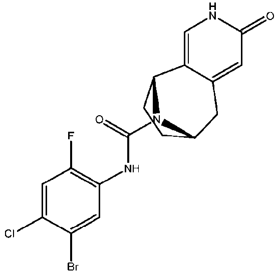 | (142) | 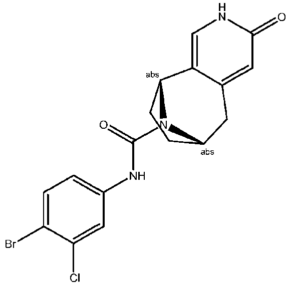 | (143) |
| 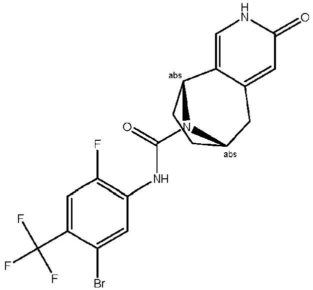 | (144) | 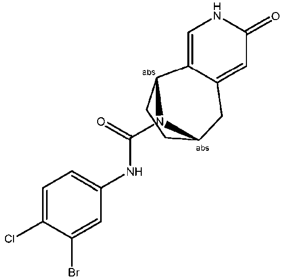 | (145) |
| 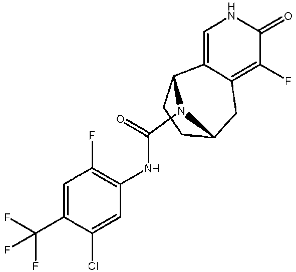 | (146) | 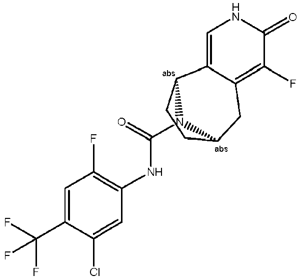 | (147) |
| 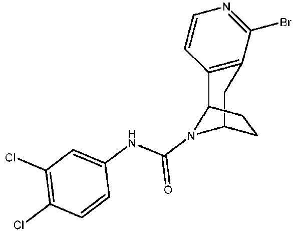 | (148) | 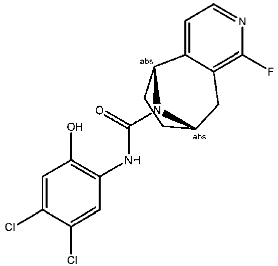 | (149) |
| 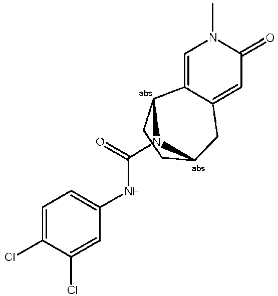 | (150) | | |
| 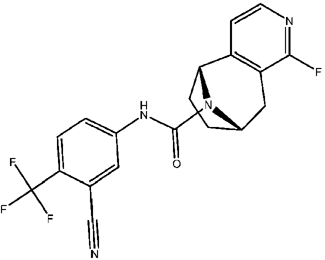 | (151) | 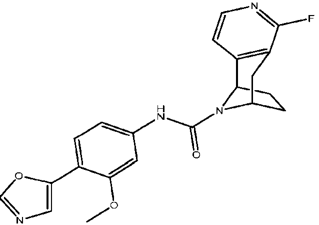 | (152) |
| 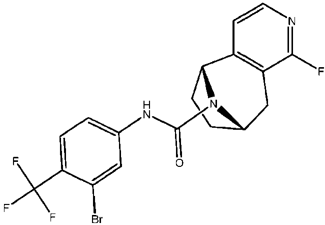 | (153) | 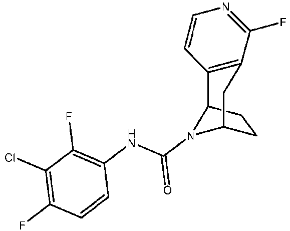 | (154) |
| 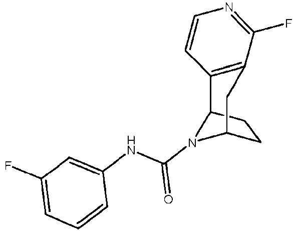 | (155) | 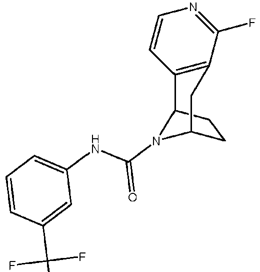 | (156) |
| 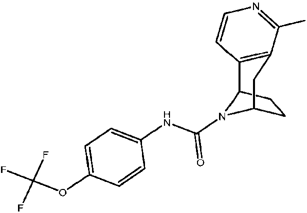 | (157) | 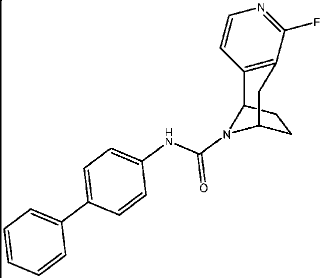 | (158) |
| 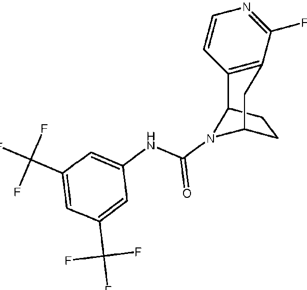 | (159) | 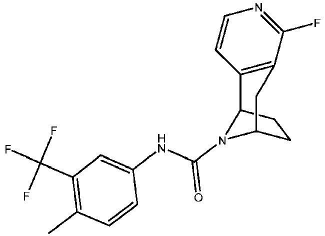 | (160) |
| 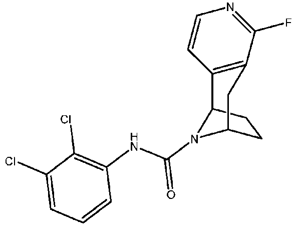 | (161) | 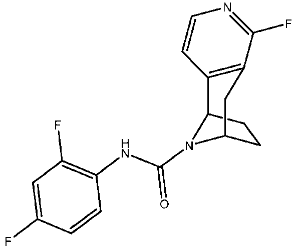 | (162) |
| 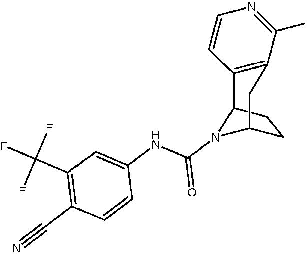 | (163) | 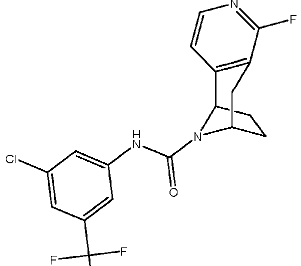 | (164) |
| 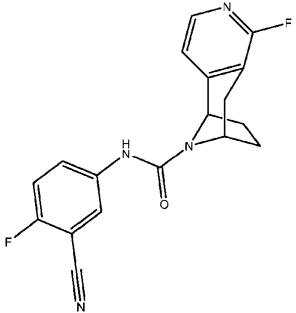 | (165) | 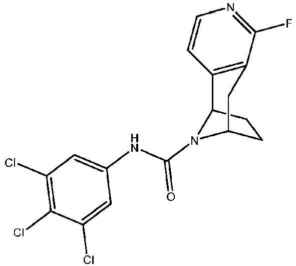 | (166) |
| 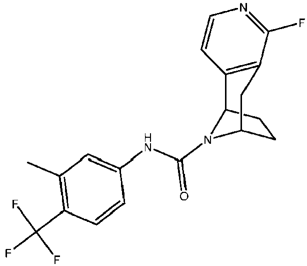 | (167) | 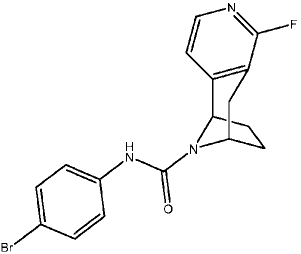 | (168) |
| 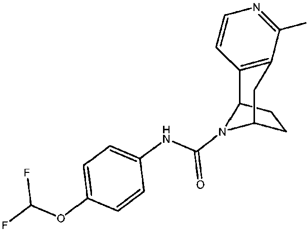 | (169) | 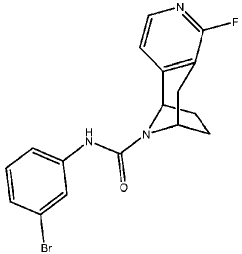 | (170) |
| 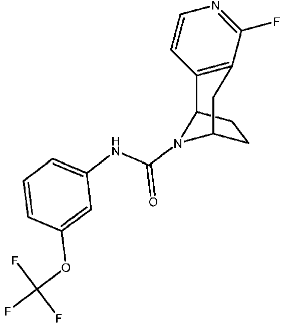 | (171) | 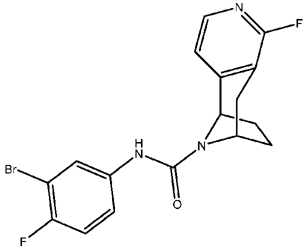 | (172) |
| 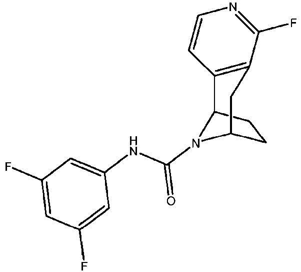 | (173) | 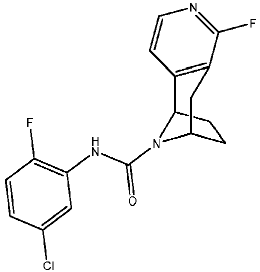 | (174) |
| 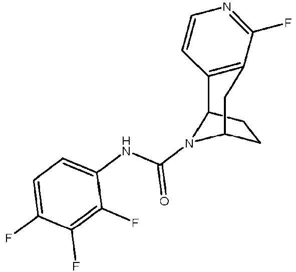 | (175) | 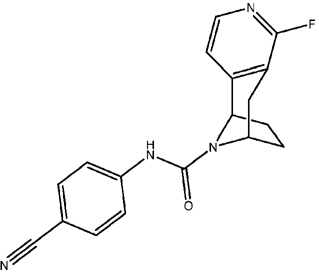 | (176) |
| 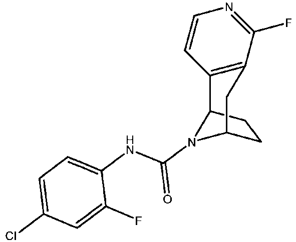 | (177) | 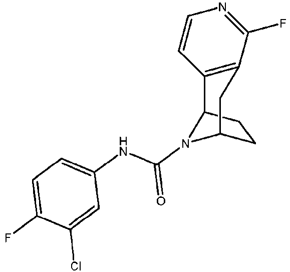 | (178) |
| 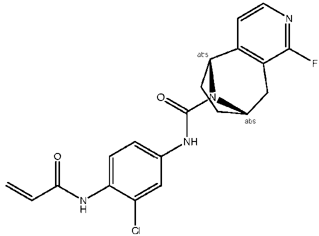 | (179) | 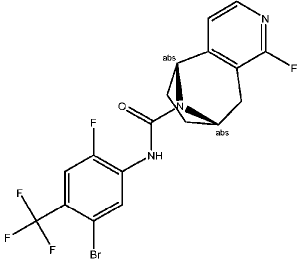 | (180) |
| 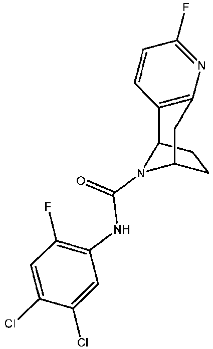 | (181) | 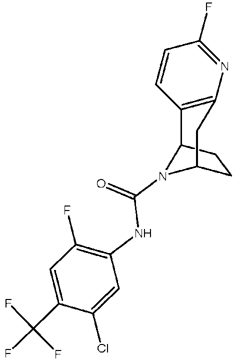 | (182) |
| 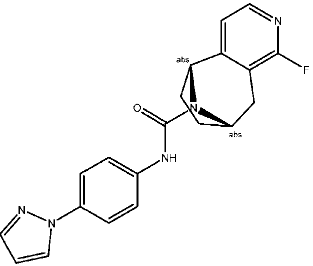 | (183) | 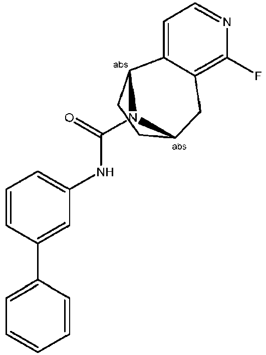 | (184) |
| 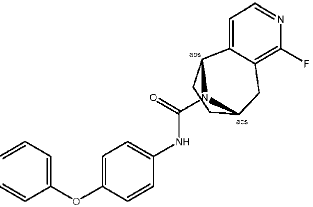 | (185) | 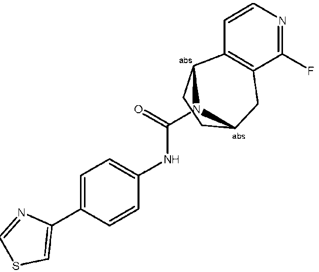 | (186) |
| 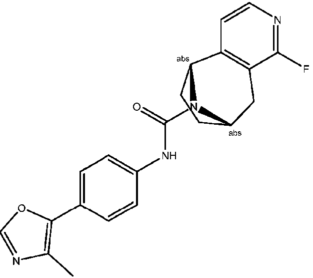 | (187) | 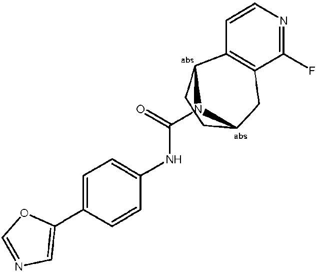 | (188) |
| 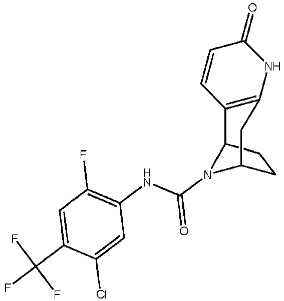 | (189) | 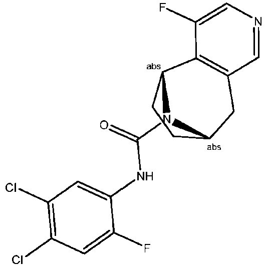 | (190) |
| 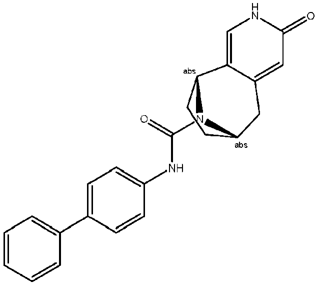 | (191) | 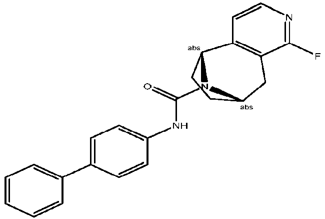 | (192) |
| 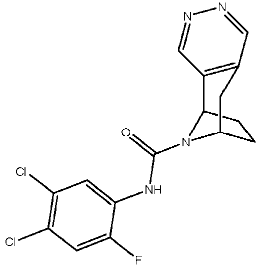 | (193) | 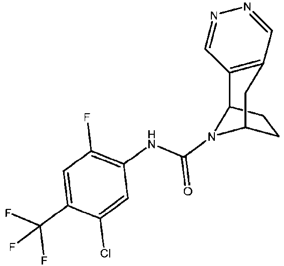 | (194) |
| 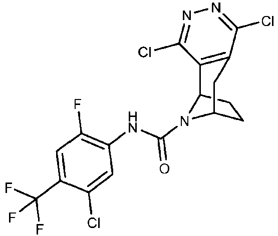 | (195) | 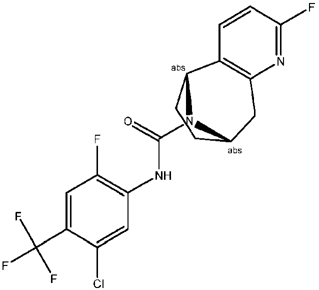 | (196) |
| 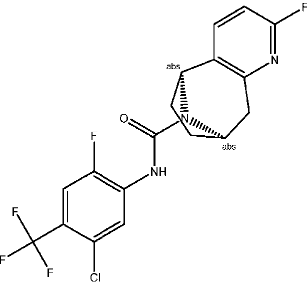 | (197) | 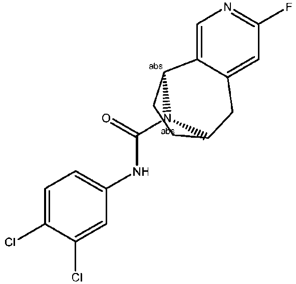 | (198) |
| 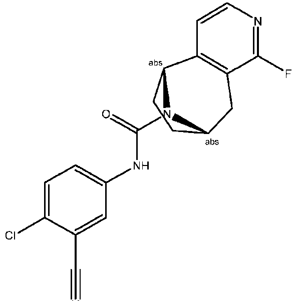 | (199) | 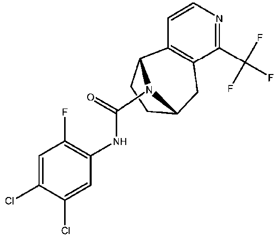 | 200 |
und Enantiomere davon und Gemische von Enantiomeren davon einschließlich racemischer Gemische und pharmazeutisch unbedenkliche Salze und Solvate davon.

13. Verbindung der Formel (Ie) oder pharmazeutisch unbedenkliches Salz oder Solvat davon, 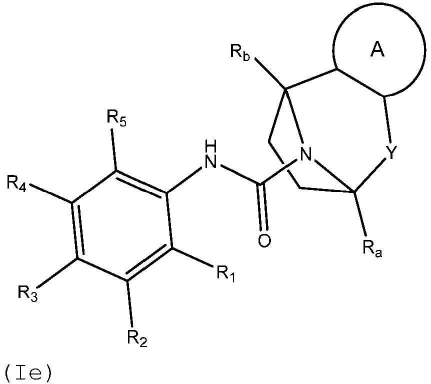 wobei:
Ring A für Folgendes steht: 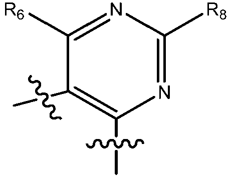
wobei R₆ und R₈ jeweils unabhängig aus H, F, Cl, Br, I, CN, Alkoxy, OH, Phenyl und Halogenalkyl ausgewählt sind; Y aus C=N-OH und CR₁₀R₁₀' ausgewählt ist, wobei R₁₀ und R₁₀' jeweils unabhängig aus H, F, Alkyl und Halogenalkyl ausgewählt sind;
Rₐ und R_{b} jeweils unabhängig aus H und Alkyl ausgewählt sind;
R₁, R₄ und R₅ jeweils unabhängig aus H, F, Cl, Br und I ausgewählt sind; und
R₂ und R₃ jeweils unabhängig aus Cl, Br, I, CN und Halogenalkyl ausgewählt sind;
mit der Maßgabe, dass dann, wenn Y für CH₂ steht und Rₐ, R_{b}, R₁, R₄, R₅, R₆ und R₈ alle für H stehen:
R₃ nicht für CN steht, wenn R₂ für Cl steht; und
R₂ und R₃ nicht beide für Cl stehen.

14. Verbindung nach Anspruch 13, wobei R₂ aus Cl, Br und CF₃ ausgewählt ist und weiter bevorzugt aus Cl and CF₃ ausgewählt ist.

15. Verbindung nach Anspruch 13 oder Anspruch 14, wobei R₃ aus Cl, Br, CN and CF₃ ausgewählt ist und weiter bevorzugt aus Cl and CF₃ ausgewählt ist.

16. Verbindung nach einem der Ansprüche 13 bis 15, wobei R₁, R₄ und R₅ alle für H stehen.

17. Verbindung nach einem der Ansprüche 13 bis 16, wobei Y aus CH₂ und CHF ausgewählt ist und vorzugsweise für CH₂ steht.

18. Verbindung nach einem der Ansprüche 13 bis 17, wobei R₆ für H steht.

19. Verbindung nach einem der Ansprüche 13 bis 18, wobei R₈ aus H, CF₃, Phenyl, OH und F ausgewählt ist, gegebenenfalls wobei R₈ für OH steht und Ring A für Folgendes steht:

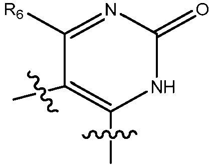

20. Verbindung nach einem der Ansprüche 13 bis 19 mit der Formel (Ie.1): 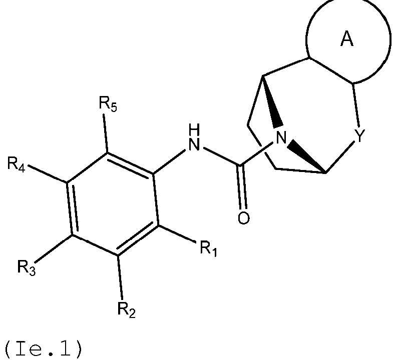 wobei A, Y und R₁-R₅ wie in einem der Ansprüche 13-19 definiert sind, oder die in Form eines Gemischs vorliegt, das mit einer Verbindung der Formel (Ie.1) enantiomerenangereichert ist.

21. Verbindung, ausgewählt aus den Folgenden:
| | | | |
|---|---|---|---|
| 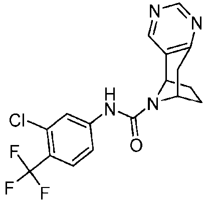 | (2) | | |
| 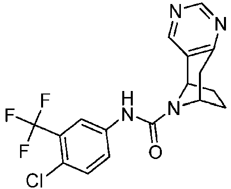 | (3) | 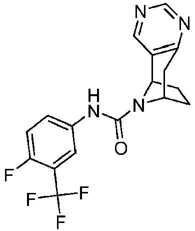 | (5) |
| 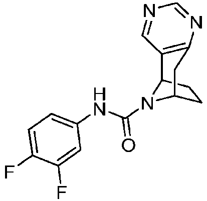 | (6) | 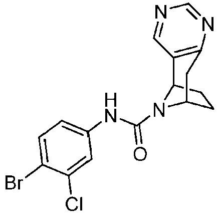 | (7) |
| 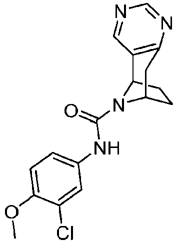 | (8) | 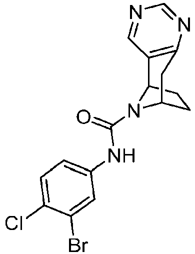 | (12) |
| 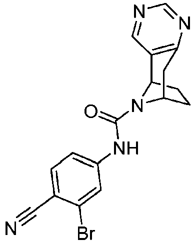 | (13) | 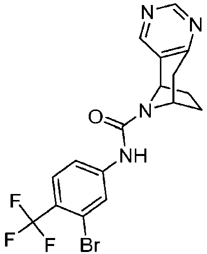 | (15) |
| 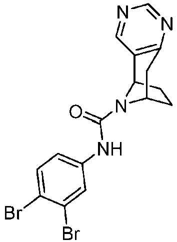 | (16) | 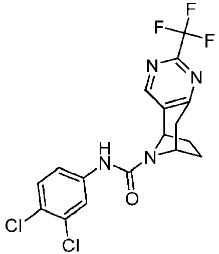 | (24) |
| 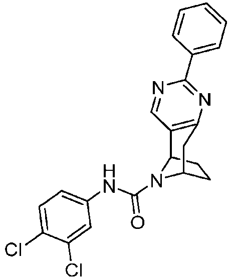 | (26) | 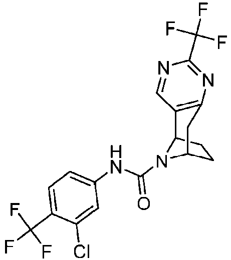 | (27) |
| 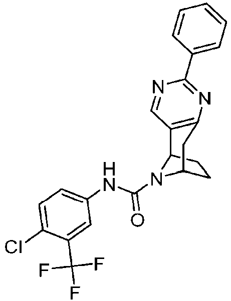 | (28) | 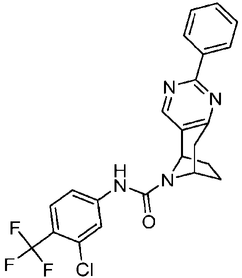 | (29) |
| 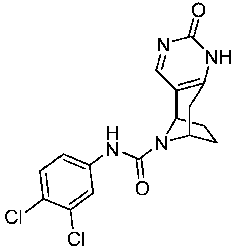 | (31) | 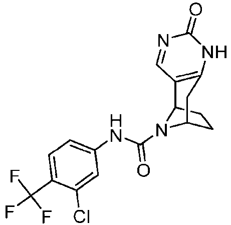 | (32) |
| | | 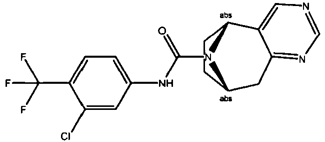 Enantiomer von (2) | (38) |
| 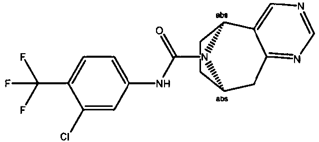 Enantiomer von (2) | (39) | 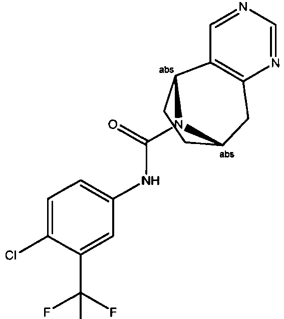 Enantiomer von (3) | (42) |
| 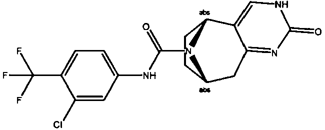 Enantiomer von (32) | (43) | 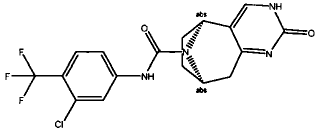 Enantiomer von (32) | (44) |
| 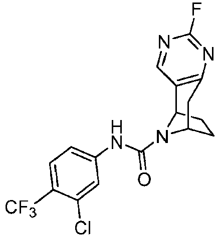 | (45) | 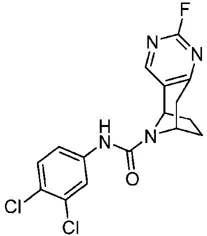 | (46) |
| 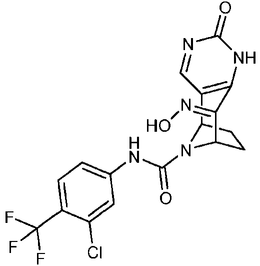 | (47) | 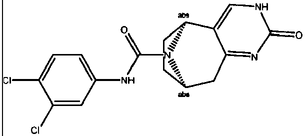 Enantiomer von (31) | (105) |
| 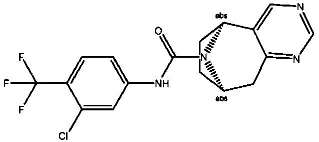 Enantiomer von (31) | (106) | 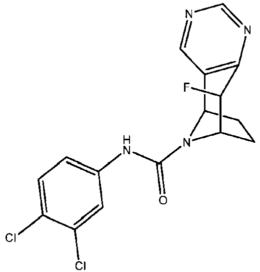 | (113) |
| 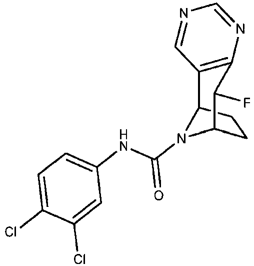 | (114) | 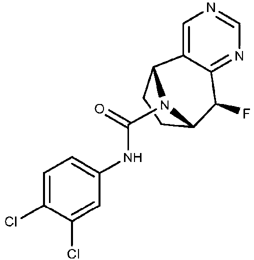 | (115) |
und Enantiomere davon und Gemische von Enantiomeren davon einschließlich racemischer Gemische und pharmazeutisch unbedenkliche Salze und Solvate davon.

22. Pharmazeutische Zusammensetzung, umfassend eine Verbindung nach einem der Ansprüche 1-21 und ein pharmazeutisch unbedenkliches Verdünnungsmittel, einen pharmazeutisch unbedenklichen Hilfsstoff oder einen pharmazeutisch unbedenklichen Träger.

23. Verbindung nach einem der Ansprüche 1-21 oder pharmazeutische Zusammensetzung nach Anspruch 22 zur Verwendung als Medikament.

24. Verbindung nach einem der Ansprüche 1-21 oder pharmazeutische Zusammensetzung nach Anspruch 22 zur Verwendung bei der Behandlung oder Prävention einer Störung, die aus einer Proliferationsstörung, einer Immunstörung, Asthma, chronisch-obstruktiver Lungenerkrankung (Chronic Obstructive Pulmonary Disease, COPD) und akutem Atemnotsyndrom (Acute Respiratory Distress Syndrome, ARDS) ausgewählt ist.

25. Verbindung oder pharmazeutische Zusammensetzung zur Verwendung nach Anspruch 24, wobei die Verwendung die Modulierung von GPR65 umfasst, vorzugsweise wobei die Verwendung das Inhibieren der GPR65-Signalgebung umfasst.

26. Verbindung oder pharmazeutische Zusammensetzung zur Verwendung nach Anspruch 24, wobei es sich bei der Störung um eine Proliferationsstörung handelt, vorzugsweise:
wobei es sich bei der Proliferationsstörung um eine Krebserkrankung und vorzugsweise um einen soliden Tumoren und/oder Metastasen davon handelt; oder
wobei es sich bei der Proliferationsstörung um eine Krebserkrankung handelt, die aus Melanom, Nierenzellkarzinom (Renal Cell Carcinoma, RCC), Magenkrebs, akuter myeloischer Leukämie (AML), dreifach negativem Brustkrebs (Triple Negative Breast Cancer, TNBC), Kolorektalkrebs, Kopf- und Halskrebs, kolorektalem Adenokarzinom, Pankreas-Adenokarzinom, Sarkom, Lungenkrebs, Eierstockkrebs und Gliomen, vorzugsweise Glioblastom (GBM), ausgewählt ist.

27. Verbindung oder pharmazeutische Zusammensetzung zur Verwendung nach Anspruch 24, wobei es sich bei der Störung um eine Immunstörung handelt, vorzugsweise wobei es sich bei der Immunstörung um eine Autoimmunstörung handelt, weiter bevorzugt wobei die Autoimmunstörung aus Psoriasis, Arthritis psoriatica, rheumatoider Arthritis (RA), multipler Sklerose (MS), systemischem Lupus erythematodes (SLE), Autoimmunthyroiditis (Hashimoto-Thyroiditis), Morbus Basedow, Uveitis (einschließlich intermediäre Uveitis), Colitis ulcerosa, Morbus Crohn, Autoimmunuveoretinitis, systemischer Vaskulitis, Polymyositis-Dermatomyositis, systemischer Sklerose (Sklerodermie), Sjogren-Syndrom, Spondylitis ankylosans und verwandten Spondyloarthropathien, Sarkoidose, autoimmunhämolytischer Anämie, immunologischen Thrombozytenstörungen und Autoimmunpolyendokrinopathien ausgewählt ist, ganz besonders bevorzugt wobei die Autoimmunerkrankung aus Psoriasis, Arthritis psoriatica, Spondylitis ankylosans, Morbus Crohn und multipler Sklerose (MS) ausgewählt ist.

28. Verbindung oder pharmazeutische Zusammensetzung zur Verwendung nach Anspruch 24, wobei die Verwendung die Behandlung oder Prävention einer Störung, die aus Asthma, chronisch-obstruktiver Lungenerkrankung (COPD) und akutem Atemnotsyndrom (ARDS) ausgewählt ist, umfasst.

29. Verbindung nach einem der Ansprüche 1-21 oder pharmazeutisch unbedenkliches Salz oder Solvat davon oder pharmazeutische Zusammensetzung nach Anspruch 22 zur Verwendung bei der Behandlung oder Prävention einer mit GPR65 assoziierten Erkrankung oder Störung.

30. Verbindung, ausgewählt aus den Folgenden:
| | | | |
|---|---|---|---|
| 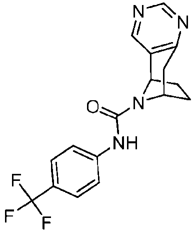 | (1) | 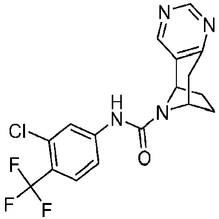 | (2) |
| 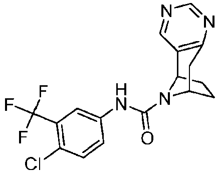 | (3) | 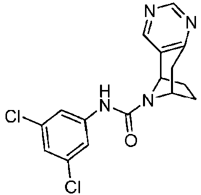 | (4) |
| 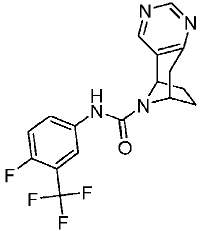 | (5) | 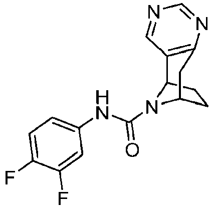 | (6) |
| 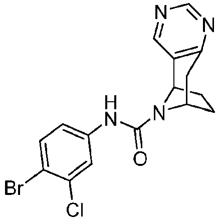 | (7) | 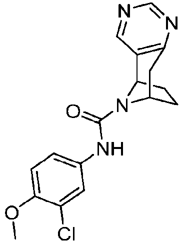 | (8) |
| 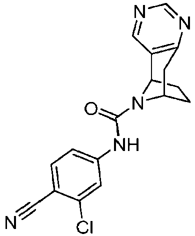 | (11) | 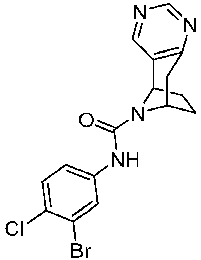 | (12) |
| 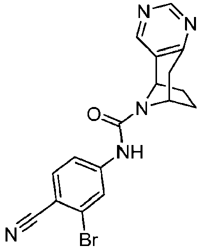 | (13) | 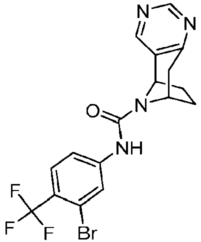 | (15) |
| 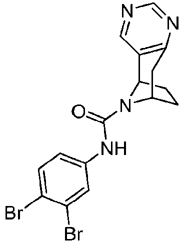 | (16) | 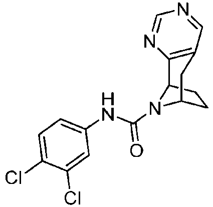 | (17) |
| 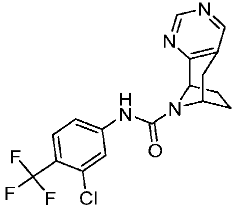 | (18) | 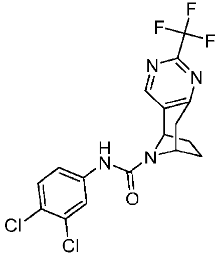 | (24) |
| 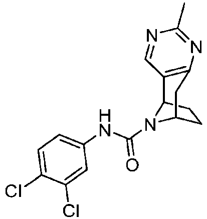 | (25) | 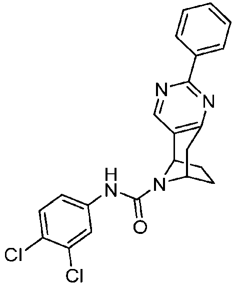 | (26) |
| 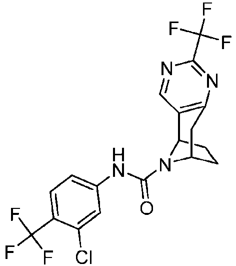 | (27) | 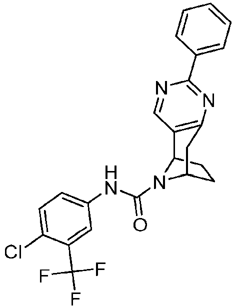 | (28) |
| 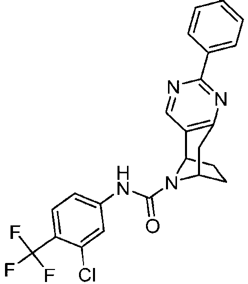 | (29) | 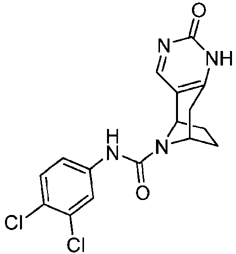 | (31) |
| 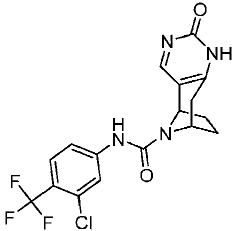 | (32) | 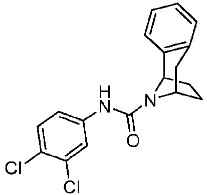 | (34) |
| 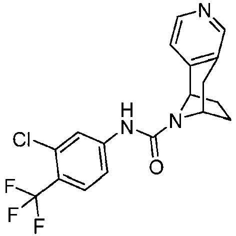 | (35) | 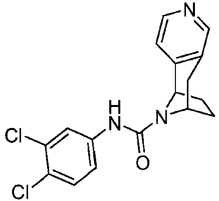 | (36) |
| 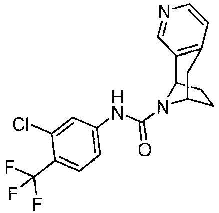 | (37) | 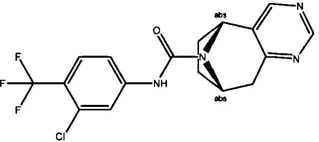 Enantiomer von (2) | (38) |
| 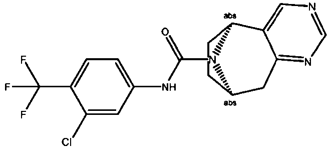 | (39) | 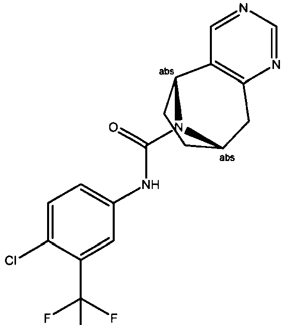 | (42) |
| Enantiomer von (2) | | Enantiomer von (3) | |
| 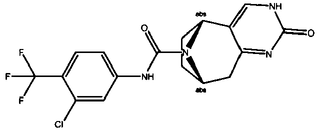 | (43) | 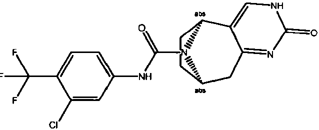 | (44) |
| Enantiomer von (32) | | Enantiomer von (32) | |
| 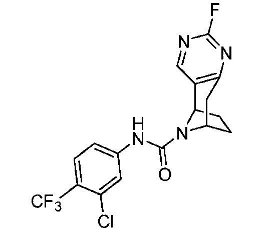 | (45) | 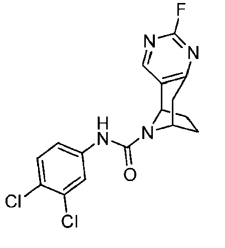 | (46) |
| 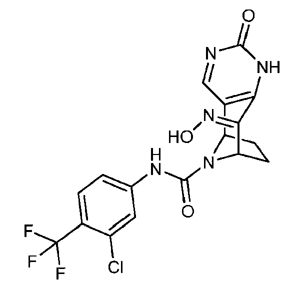 | (47) | 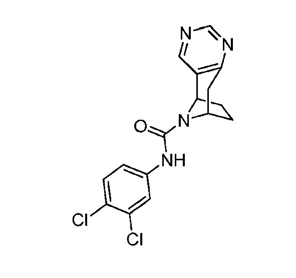 | (48) |
| 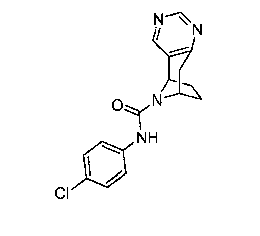 | (49) | 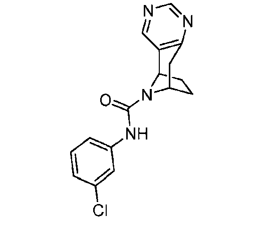 | (50) |
| 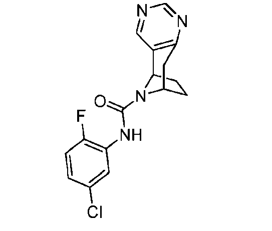 | (51) | 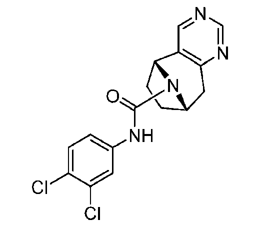 | (52) |
| 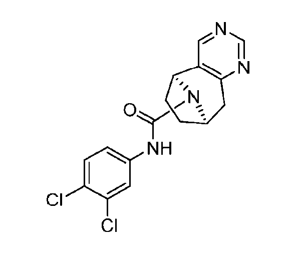 | (53) | 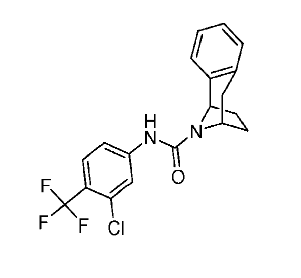 | (54) |
| 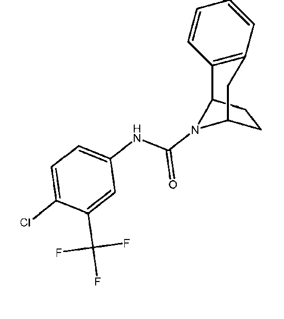 | (55) | 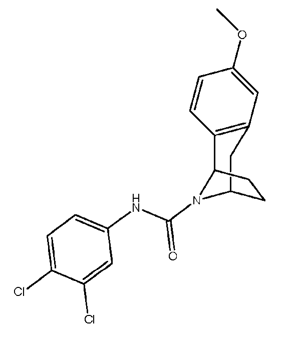 | (56) |
| | (57) | 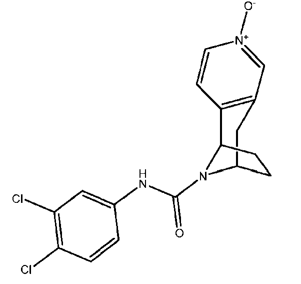 | (58) |
| 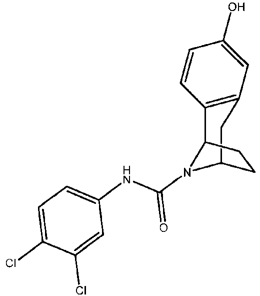 | | | |
| 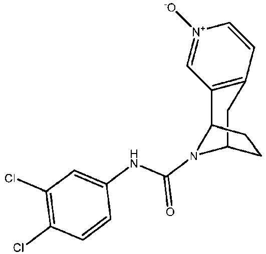 | (59) | 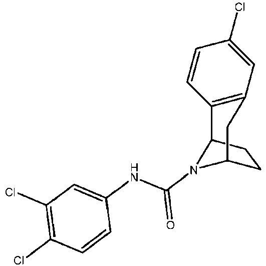 | (60) |
| 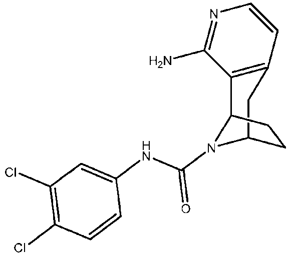 | (61) | 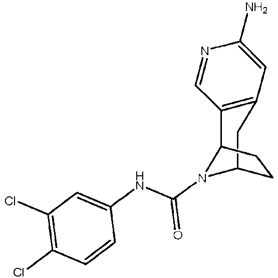 | (62) |
| 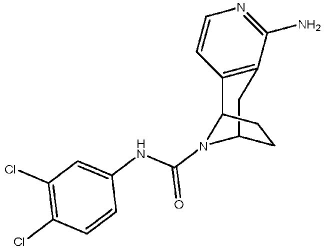 | (63) | 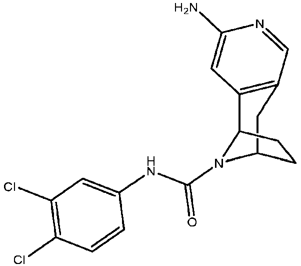 | (64) |
| 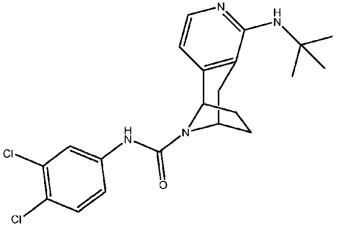 | (65) | 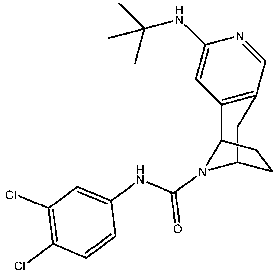 | (66) |
| 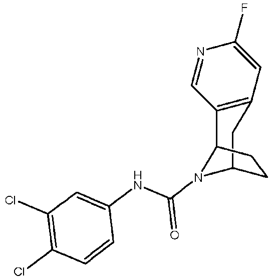 | (67) | 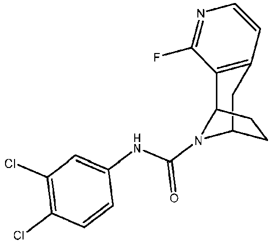 | (68) |
| 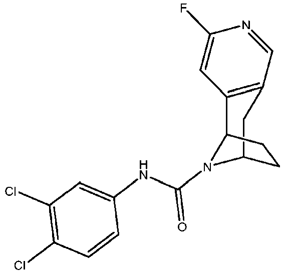 | (69) | 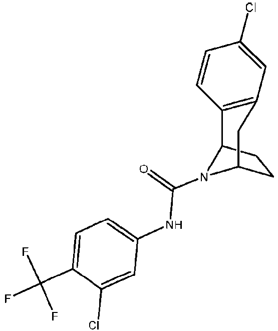 | (71) |
| 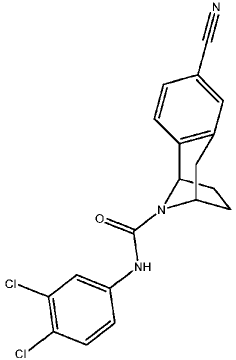 | (72) | 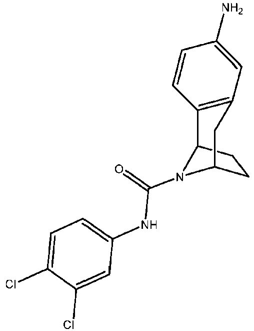 | (73) |
| 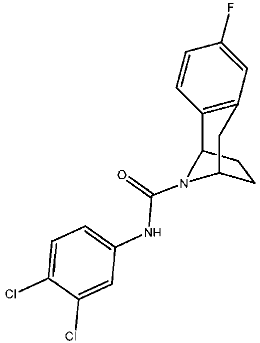 | (74) | 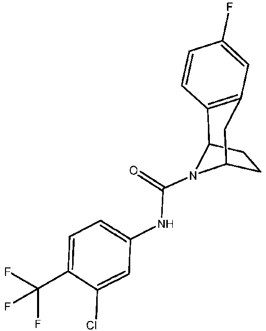 | (75) |
| 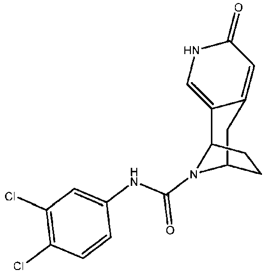 | (76) | 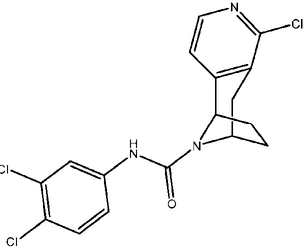 | (77) |
| 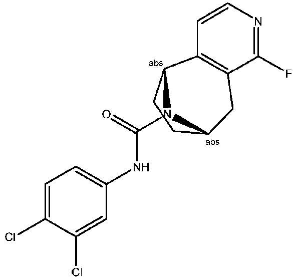 | (78) | 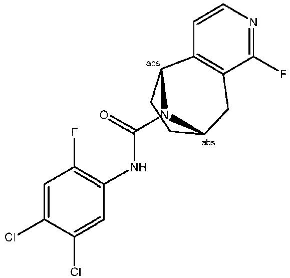 | (79) |
| 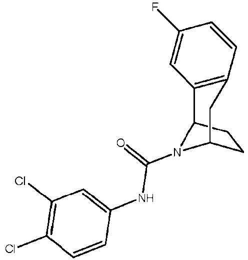 | (80) | 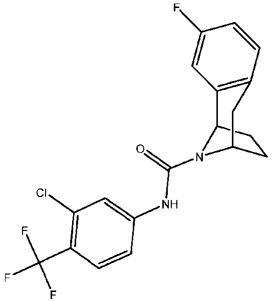 | (81) |
| 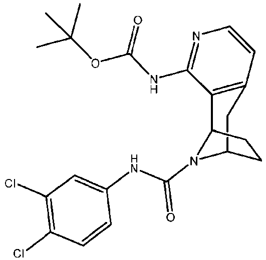 | (82) | 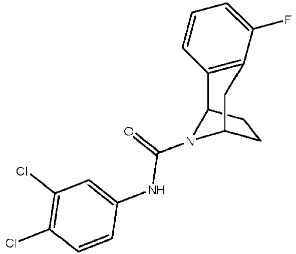 | (83) |
| 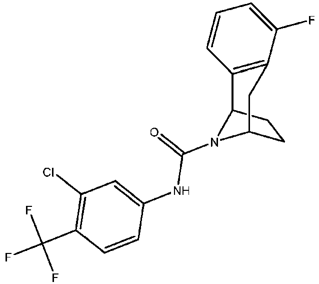 | (84) | 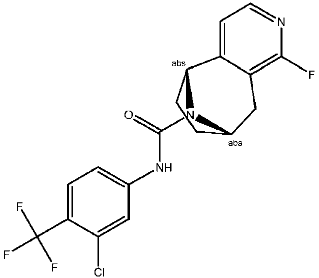 | (85) |
| 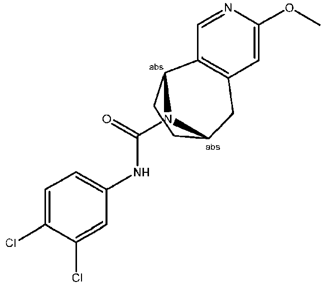 | (86) | 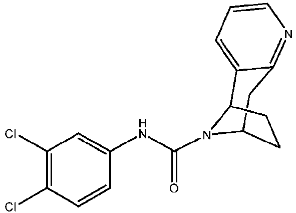 | (87) |
| 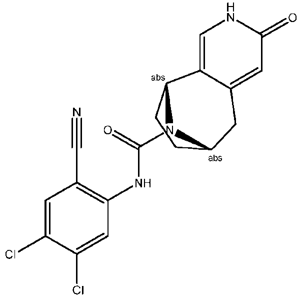 | (88) | 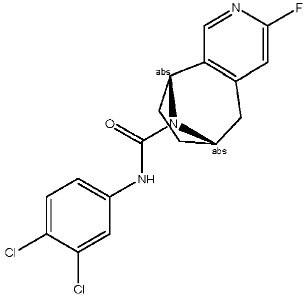 | (91) |
| 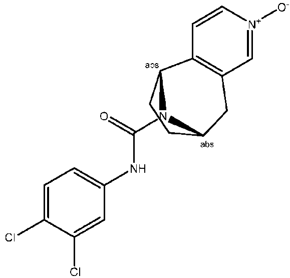 | (92) | 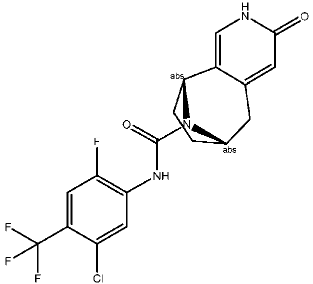 | (93) |
| 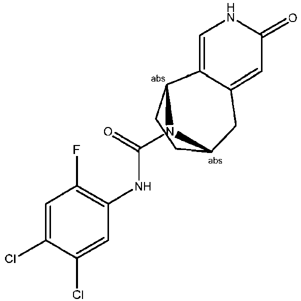 | (94) | 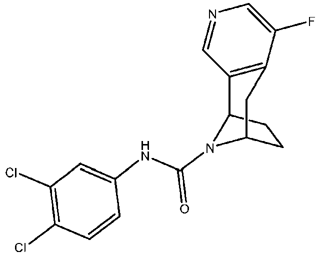 | (95) |
| 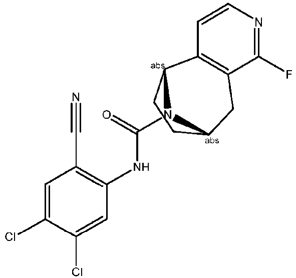 | (98) | 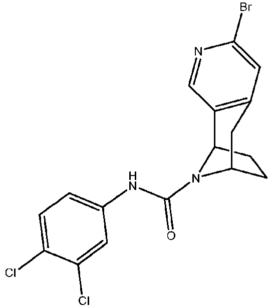 | (99) |
| 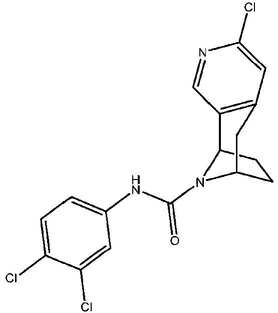 | (100) | 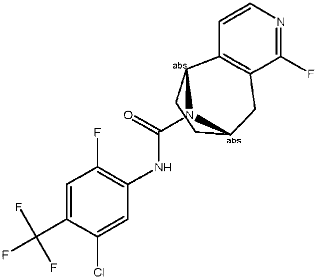 | (101) |
| 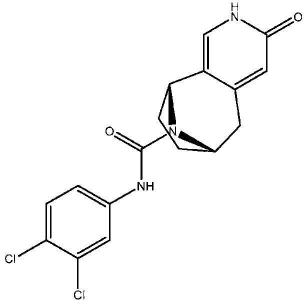 | (103) | 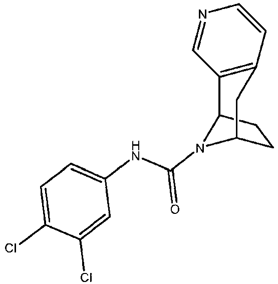 | (104) |
| 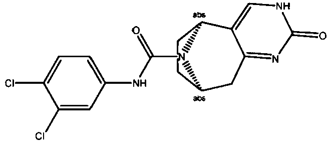 Enantiomer von (31) | (105) | 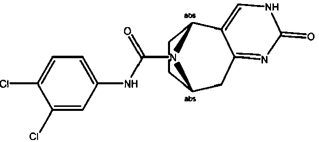 Enantiomer von (31) | (106) |
| 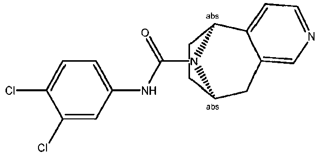 | (107) | 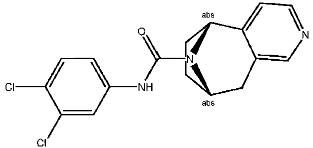 | (108) |
| 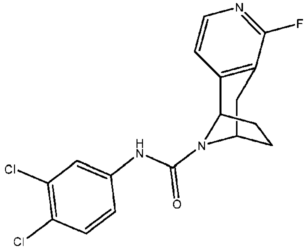 | (109) | 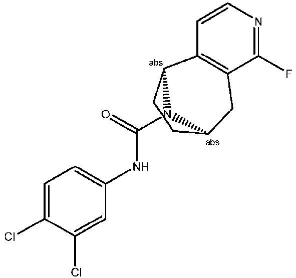 | (110) |
| 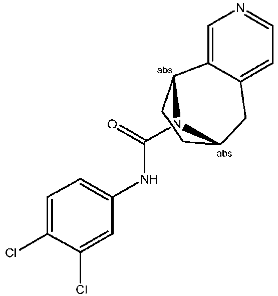 | (111) | 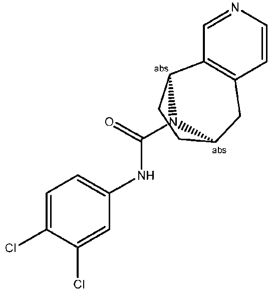 | (112) |
| 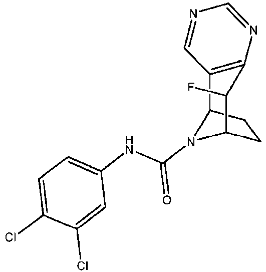 | (113) | 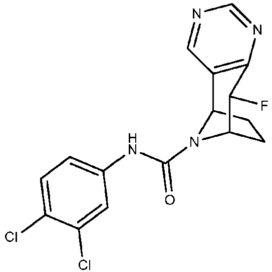 | (114) |
| 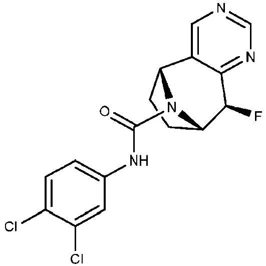 | (115) | | |
| 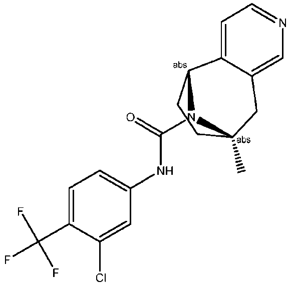 | (116) | 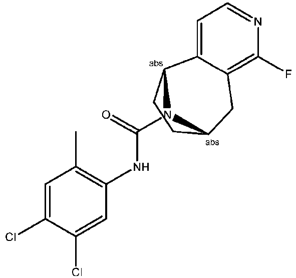 | (117) |
| 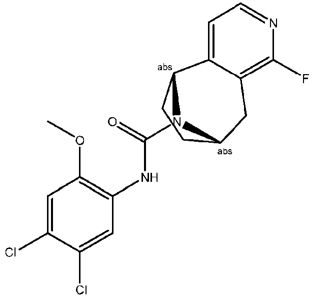 | (118) | 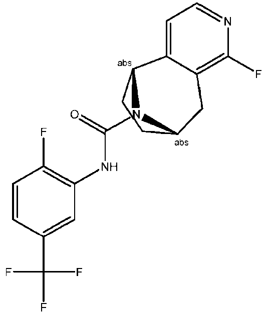 | (119) |
| 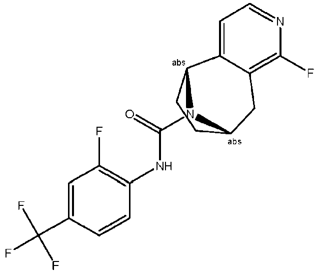 | (120) | 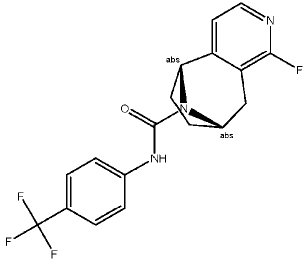 | (121) |
| 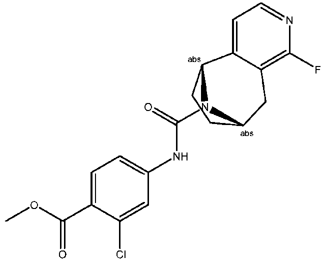 | (122) | 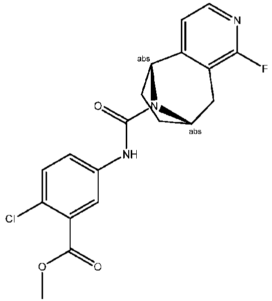 | (123) |
| 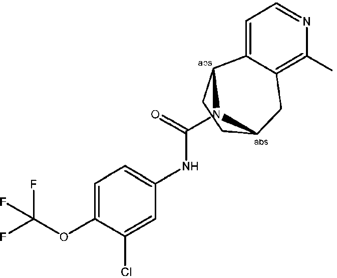 | (124) | 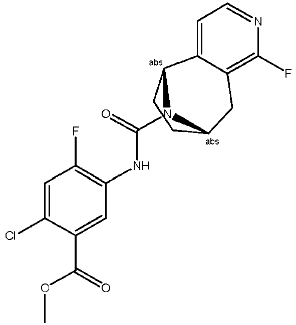 | (125) |
| 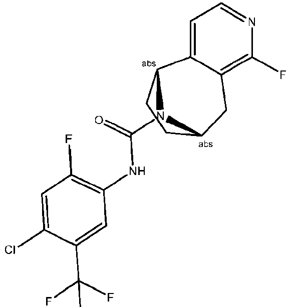 | (126) | 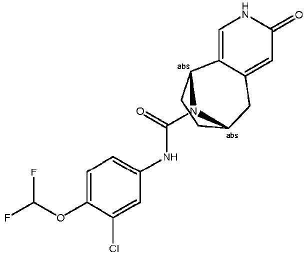 | (127) |
| 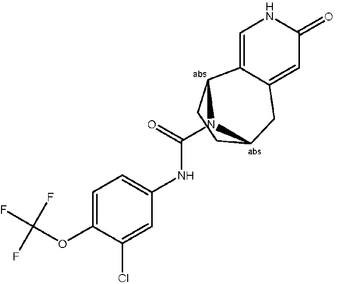 | (128) | 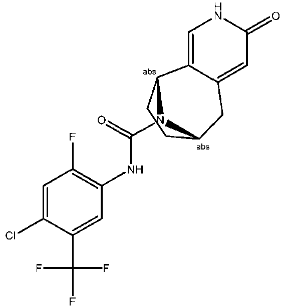 | (129) |
| 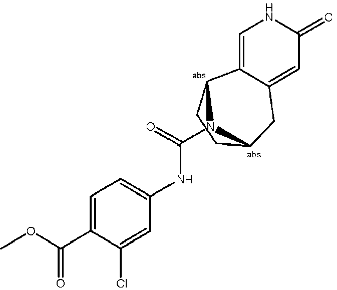 | (130) | 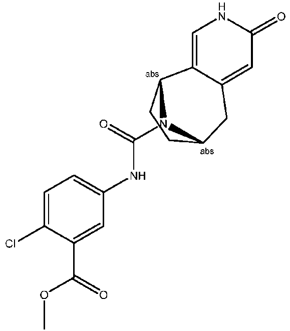 | (131) |
| 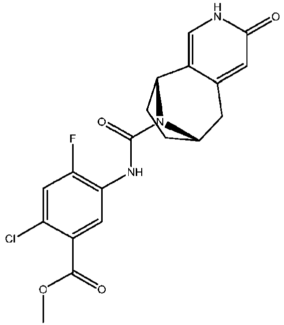 | (132) | 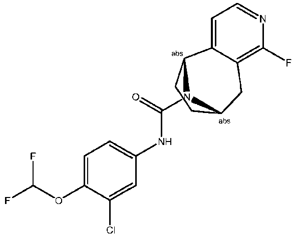 | (133) |
| 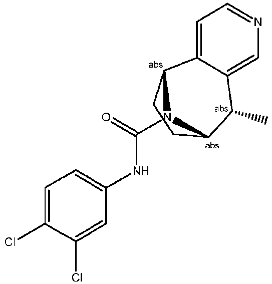 | (134) | 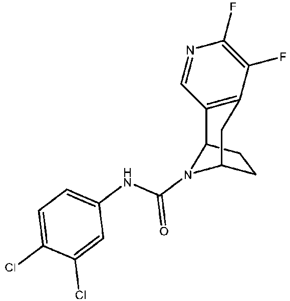 | (135) |
| 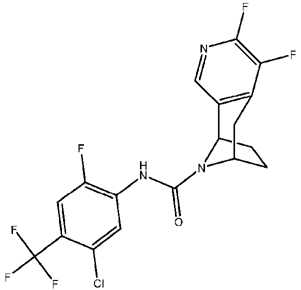 | (136) | 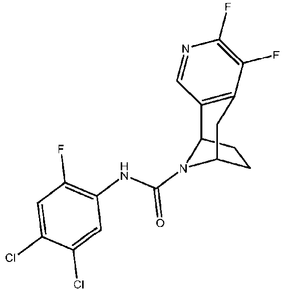 | (137) |
| 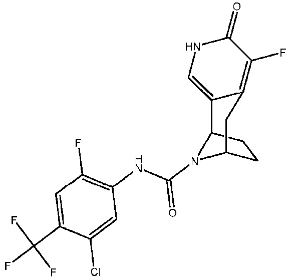 | (138) | 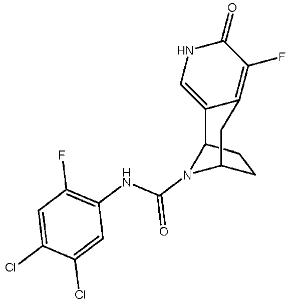 | (139) |
| 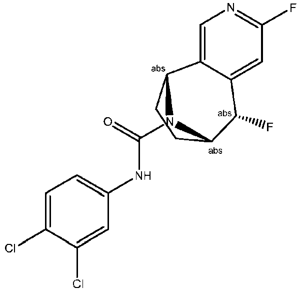 | (140) | 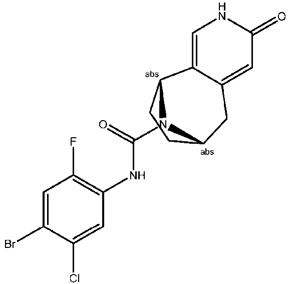 | (141) |
| 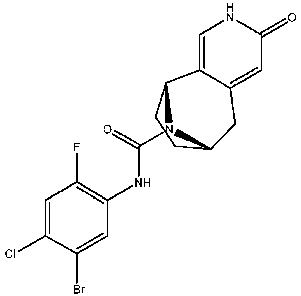 | (142) | 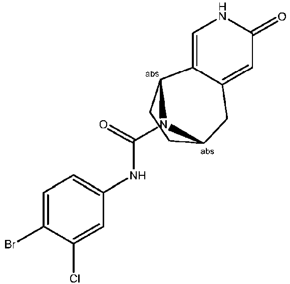 | (143) |
| 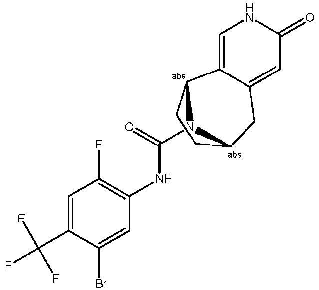 | (144) | 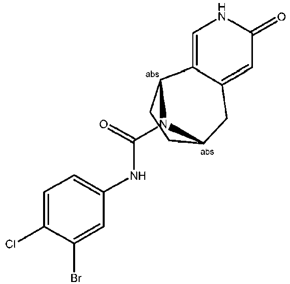 | (145) |
| 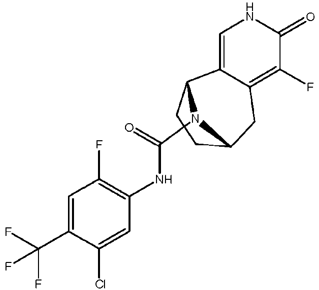 | (146) | 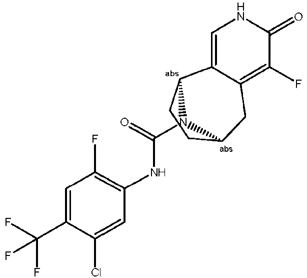 | (147) |
| 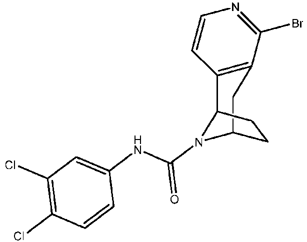 | (148) | 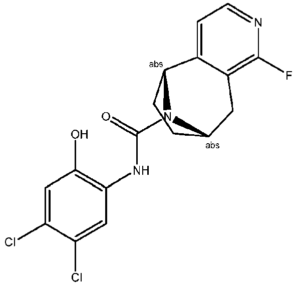 | (149) |
| 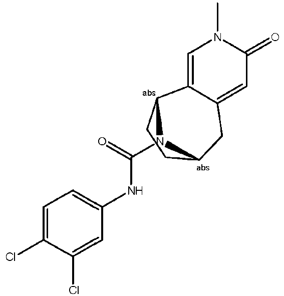 | (150) | | |
| 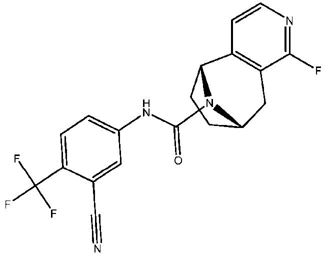 | (151) | 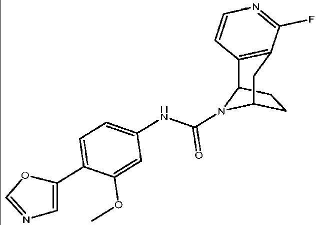 | (152) |
| 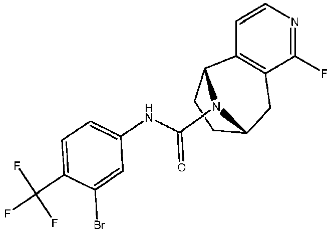 | (153) | 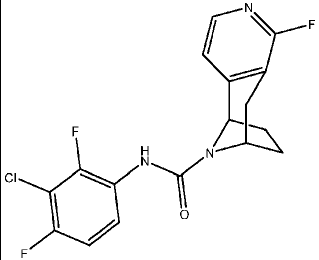 | (154) |
| 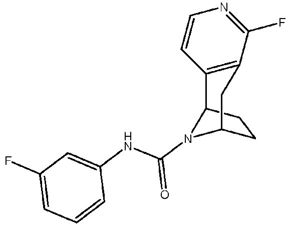 | (155) | 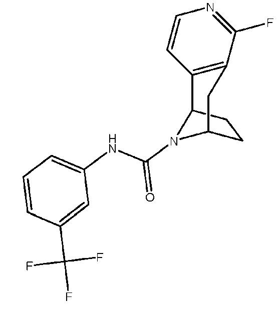 | (156) |
| 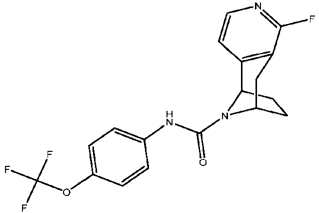 | (157) | 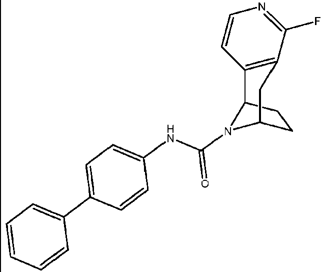 | (158) |
| 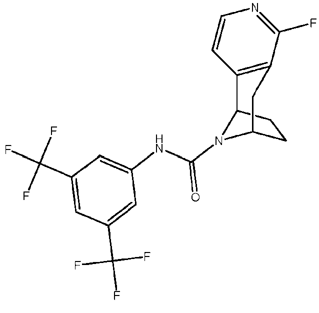 | (159) | 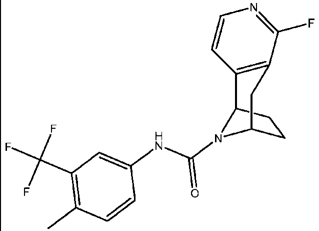 | (160) |
| 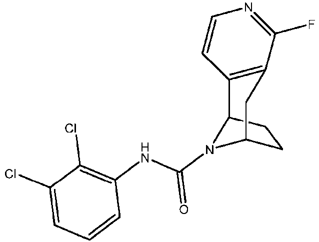 | (161) | 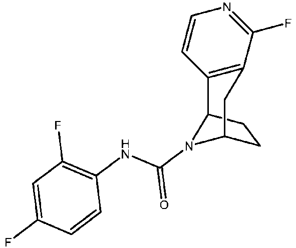 | (162) |
| 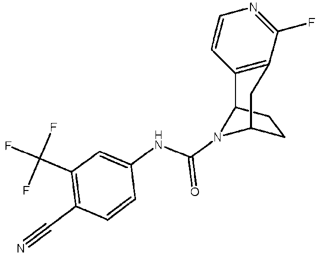 | (163) | 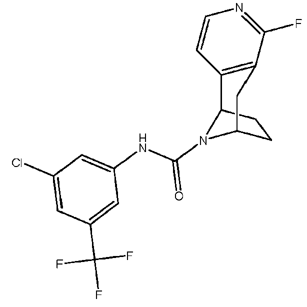 | (164) |
| 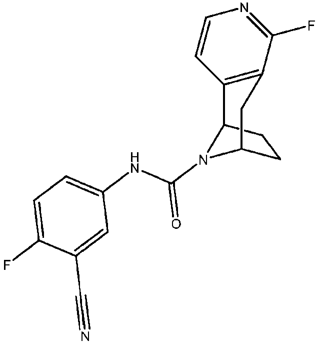 | (165) | 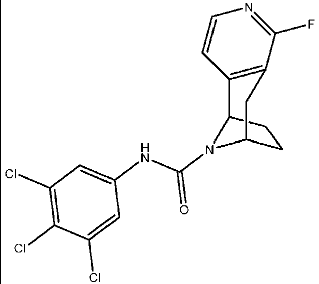 | (166) |
| 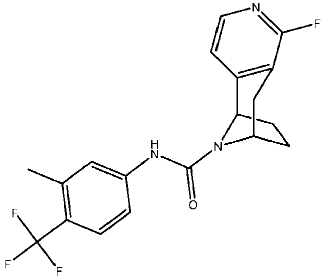 | (167) | 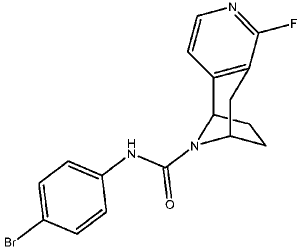 | (168) |
| 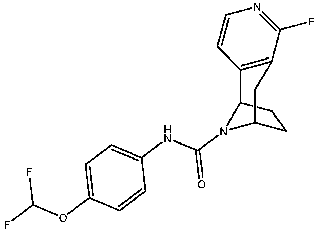 | (169) | 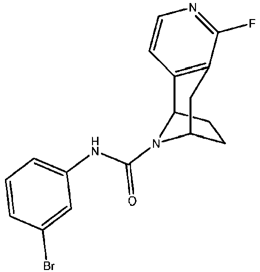 | (170) |
| 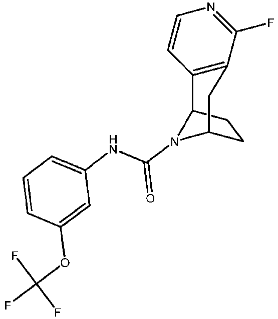 | (171) | 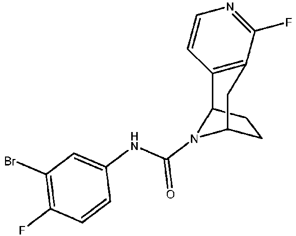 | (172) |
| 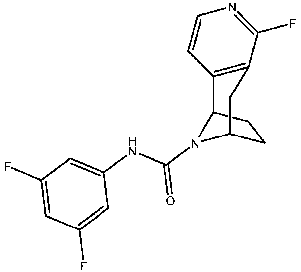 | (173) | 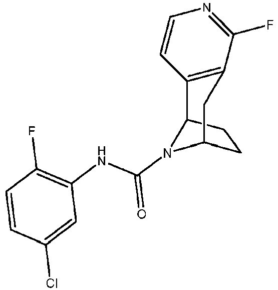 | (174) |
| 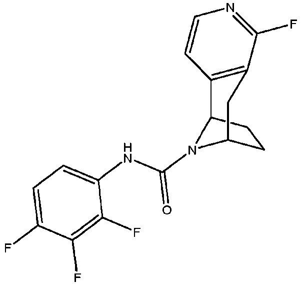 | (175) | 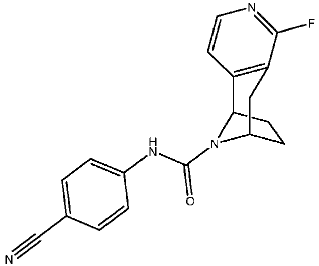 | (176) |
| 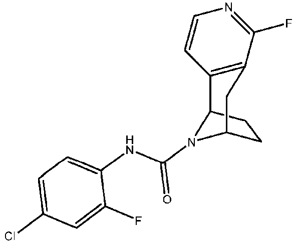 | (177) | 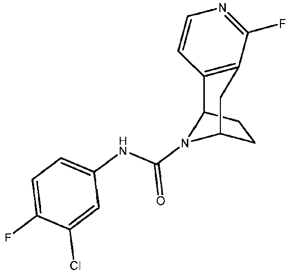 | (178) |
| 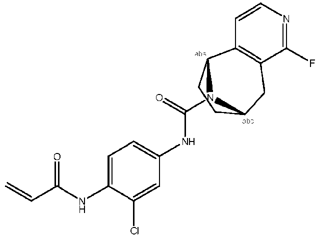 | (179) | 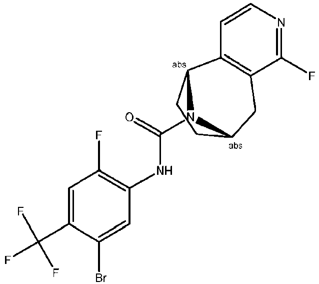 | (180) |
| 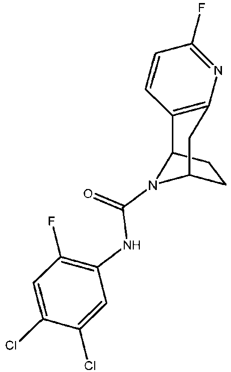 | (181) | 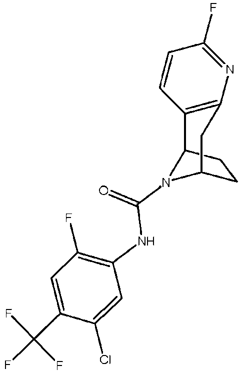 | (182) |
| 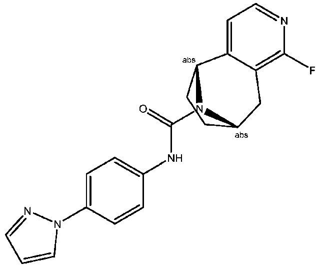 | (183) | 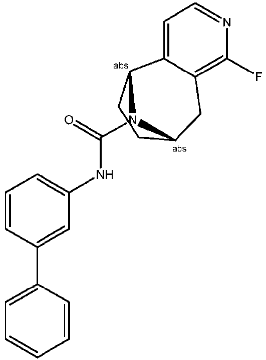 | (184) |
| 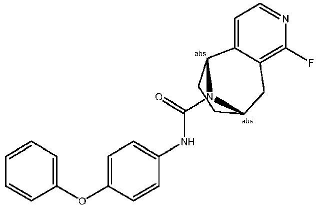 | (185) | 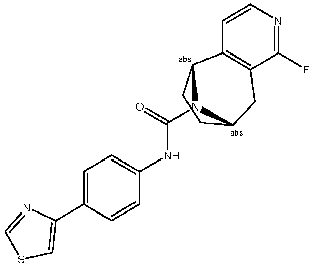 | (186) |
| 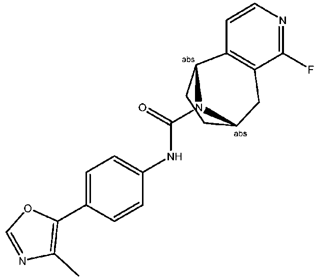 | (187) | 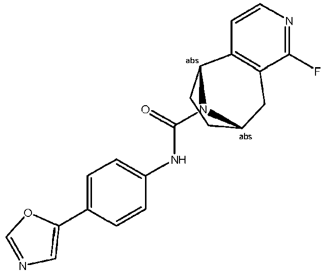 | (188) |
| 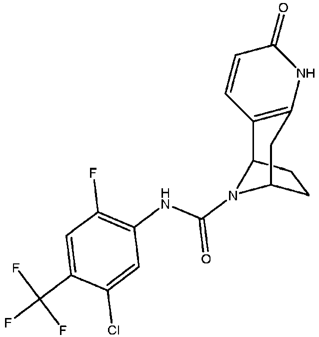 | (189) | 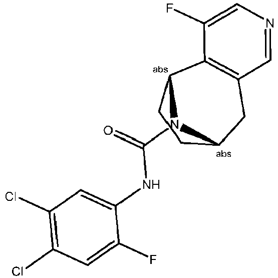 | (190) |
| 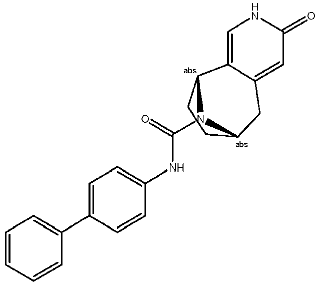 | (191) | 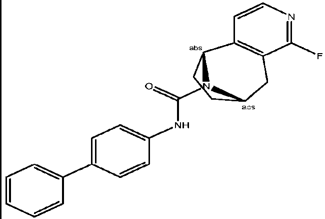 | (192) |
| 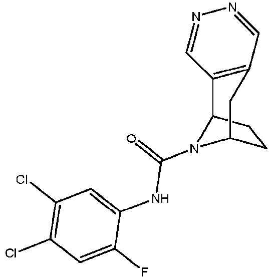 | (193) | 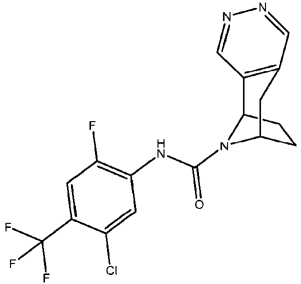 | (194) |
| 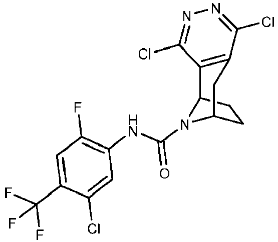 | (195) | 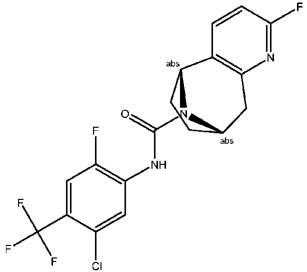 | (196) |
| 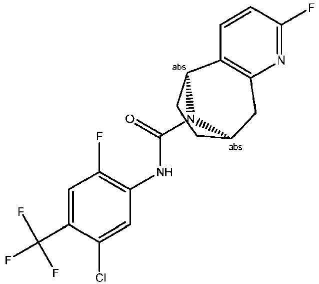 | (197) | 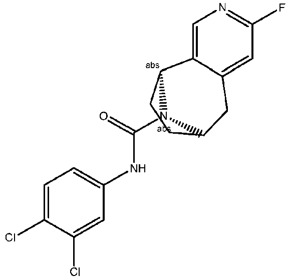 | (198) |
| 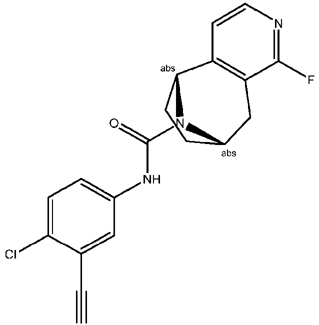 | (199) | 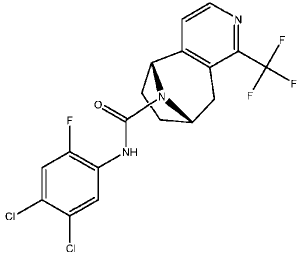 | 200 |
und Enantiomere davon und Gemische von Enantiomeren davon einschließlich racemischer Gemische und pharmazeutisch unbedenkliche Salze und Solvate davon zur Verwendung bei der Behandlung oder Prävention einer Störung, die aus einer Proliferationsstörung, einer Immunstörung, Asthma, chronisch-obstruktiver Lungenerkrankung (COPD) und akutem Atemnotsyndrom (ARDS) ausgewählt ist.

## Revendications

1. Composé de formule (If), ou sel ou solvate pharmaceutiquement acceptable correspondant, 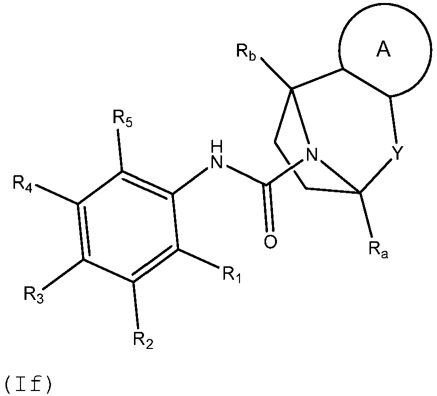
le cycle A étant un cycle pyridinyle ou une forme tautomère correspondante, ou un cycle phényle, chacun desquels pouvant éventuellement être substitué par un ou plusieurs substituants choisis parmi H, F, Cl, Br, I, CN, alcoxy, NR₁₁R₁₁', OH, alkyle, phényle, et halogénoalkyle ; Y étant choisi parmi C=N-OH et CR₁₀R₁₀', R₁₀ et R₁₀' étant chacun indépendamment choisis parmi H, F, alkyle, et halogénoalkyle ;
Rₐ et R_{b} étant chacun indépendamment choisis parmi H et alkyle ;
R₁, R₄, et R₅ étant chacun indépendamment choisis parmi H, CN, alkyle, alcoxy, halogénoalkyle, OH, F, Cl, Br, et I ;
R₂ et R₃ étant chacun indépendamment choisis parmi H, F, Cl, Br, I, CN, alcoxy, halogénoalkyle, halogénoalcoxy, alkyle, aryle, hétéroaryle, O-aryle, NHCO-alcényle et CO₂-alkyle, lesdits groupes aryle, hétéroaryle et O-aryle étant chacun éventuellement en outre substitués par un ou plusieurs groupes indépendamment choisis parmi halogéno, alkyle et alcoxy ; et
R₁₁ et R₁₁' étant chacun indépendamment choisis parmi H, alkyle, halogénoalkyle, COR₁₂, CO₂R₁₂ et SO₂R₁₃, R₁₂ et R₁₃ étant tous deux indépendamment alkyle.

2. Composé selon la revendication 1, le cycle A étant choisi parmi les suivants : 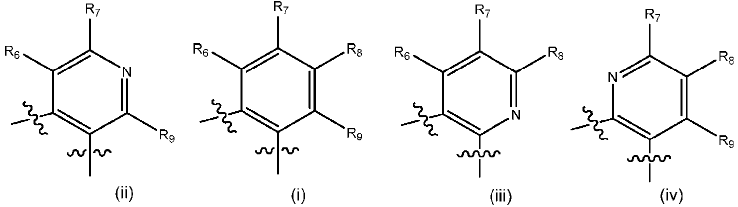 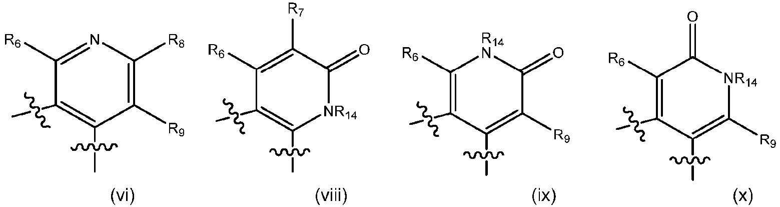 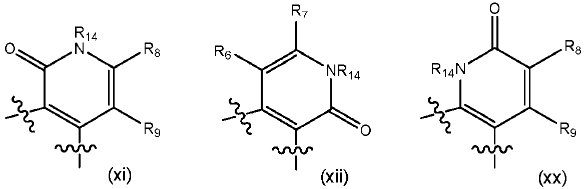 R₆, R₇, R₈, et R₉ étant chacun indépendamment choisis parmi H, F, Cl, Br, I, CN, alcoxy, NR₁₁R₁₁', OH, alkyle, phényle, et halogénoalkyle, et R₁₄ étant H ou alkyle, plus préférablement H.

3. Composé de formule (Ib), ou sel ou solvate pharmaceutiquement acceptable correspondant, 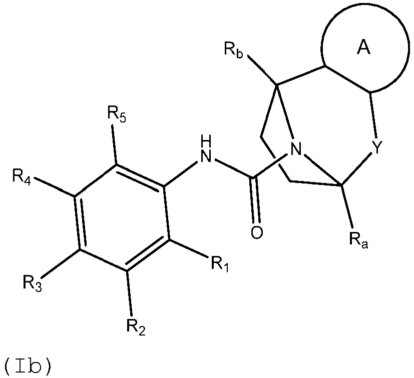
le cycle A étant choisi parmi les groupes (i)-(xx) : 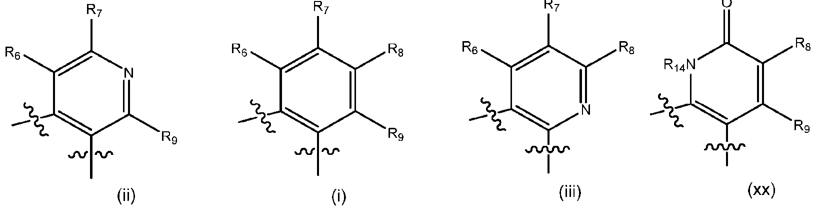 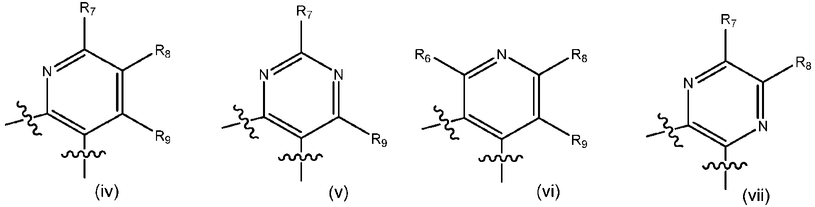 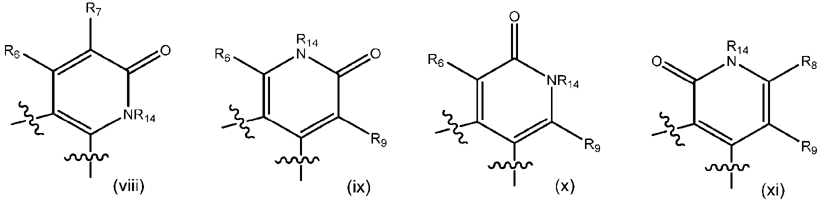 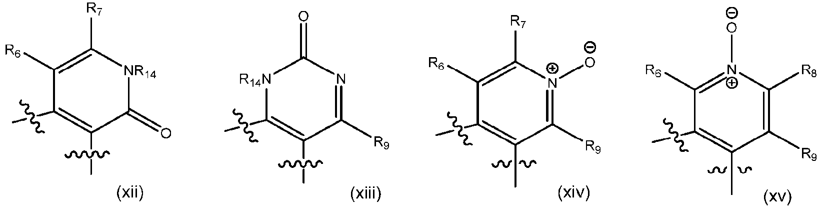 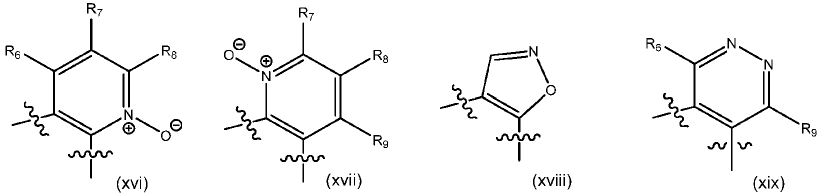
R₆, R₇, R₈, et R₉ étant chacun indépendamment choisis parmi H, F, Cl, Br, I, CN, alcoxy, NR₁₁R₁₁', OH, alkyle, phényle, et halogénoalkyle, et R₁₄ étant H ou alkyle, plus préférablement H ;
Y étant choisi parmi C=N-OH et CR₁₀R₁₀', R₁₀ et R₁₀' étant chacun indépendamment choisis parmi H, F, alkyle, et halogénoalkyle ;
Rₐ et R_{b} étant chacun indépendamment choisis parmi H et alkyle ;
R₁, R₄, et R₅ étant chacun indépendamment choisis parmi H, CN, alkyle, alcoxy, halogénoalkyle, OH, F, Cl, Br, et I ;
R₂ et R₃ étant chacun indépendamment choisis parmi H, F, Cl, Br, I, CN, alcoxy, halogénoalkyle, halogénoalcoxy, alkyle, aryle, hétéroaryle, O-aryle, NHCO-alcényle et CO₂-alkyle, lesdits groupes aryle, hétéroaryle et O-aryle étant chacun éventuellement en outre substitués par un ou plusieurs groupes indépendamment choisis parmi halogéno, alkyle et alcoxy ; et
R₁₁ et R₁₁' étant chacun indépendamment choisis parmi H, alkyle, halogénoalkyle, COR₁₂, CO₂R₁₂ et SO₂R₁₃, R₁₂ et R₁₃ étant tous deux indépendamment alkyle.

4. Composé selon la revendication 3, le cycle A étant choisi parmi (i), (ii), (iii), (iv), (v), (vi), (viii), (ix), (xiv), (xv) et (xix), et étant préférablement choisi parmi (i), (ii), (iii), (v), (vi) et (ix), et étant encore plus préférablement choisi parmi (i), (ii), (vi) et (ix).

5. Composé selon l'une quelconque des revendications précédentes, qui est de formule : 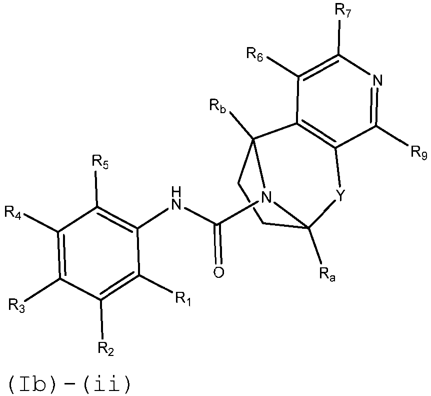
Y, Rₐ, R_{b}, R₁-R₅ étant tels que définis dans la revendication 1, et R₆, R₇ et R₉ étant tels que définis dans la revendication 2 ; ou 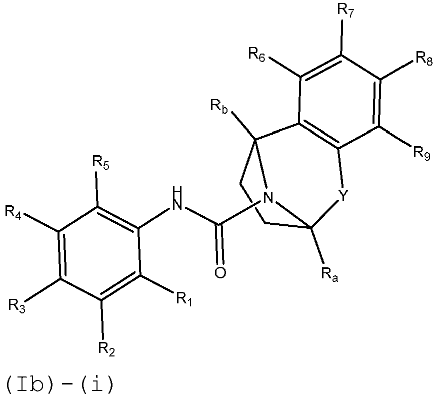
Y, Rₐ, R_{b}, R₁-R₅ étant tels que définis dans la revendication 1, et R₆-R₉ étant tels que définis dans la revendication 2 ; ou 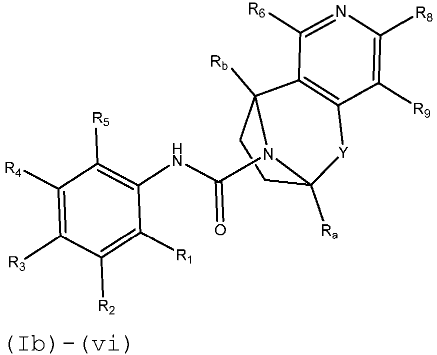
Y, Rₐ, R_{b}, R₁-R₅ étant tels que définis dans la revendication 1, et R₆, R₈ et R₉ étant tels que définis dans la revendication 2 ; ou 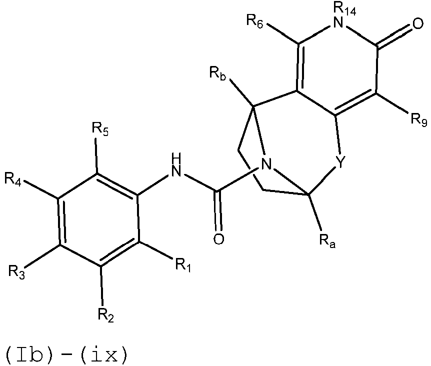
Y, Rₐ, R_{b}, R₁-R₅ étant tels que définis dans la revendication 1, et R₆, R₉ et R₁₄ étant tels que définis dans la revendication 2.

6. Composé selon l'une quelconque des revendications précédentes, Y étant choisi parmi CH₂ et C=N-OH, et étant préférablement CH₂.

7. Composé selon l'une quelconque des revendications précédentes, R₂ et R₃ étant :
(i) chacun indépendamment choisis parmi H, F, Cl, Br, CN, méthoxy, OCF₃, CF₃, OCHF₂, Me, Ph, pyrazolyle, oxazolyle, thiazolyle, OPh, NHCO-CH=CH₂ et CO₂Me, lesdits groupes Ph, OPh, pyrazolyle, oxazolyle et thiazolyle étant chacun éventuellement en outre substitués par un ou plusieurs groupes alkyle ;
(ii) chacun indépendamment choisis parmi F, Cl, Br, I, CN, C₁-C₆ halogénoalkyle, C₁-C₆ halogénoalcoxy et CO₂-alkyle.
(iii) chacun indépendamment choisis parmi Cl, Br, et CF₃, et plus préférablement étant chacun indépendamment choisis parmi Cl et CF₃ ; ou
(iv) tous deux Cl, ou l'un parmi R₂ et R₃ étant Cl et l'autre étant choisi parmi OCF₃, CO₂Me, OCHF₂ et CF₃.

8. Composé selon l'une quelconque des revendications précédentes, R₁ et R₄ étant tous deux H.

9. Composé selon l'une quelconque des revendications précédentes, R₅ étant choisi parmi H, F, Me, MeO, Cl, OH et CN, et étant préférablement H ou F, plus préférablement H.

10. Composé selon l'une quelconque des revendications 2 à 9, R₆, R₇, R₈, et R₉ étant chacun indépendamment choisis parmi H, F, Cl, Br, CN, OMe, NH₂, NHBu, NHCO₂Bu et OH.

11. Composé selon l'une quelconque des revendications précédentes, qui est de formule (Ib.1) : 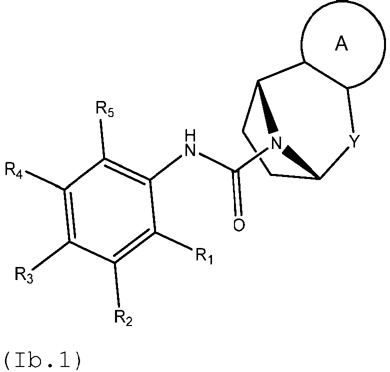 où A, Y et R₁-R₅ sont tels que définis dans l'une quelconque des revendications 1 à 10, ou qui est sous la forme d'un mélange qui est énantiomériquement enrichi en composé de formule (Ib.1).

12. Composé selon l'une quelconque des revendications précédentes 3 à 11, qui est choisi parmi les suivants :
| | | | |
|---|---|---|---|
| 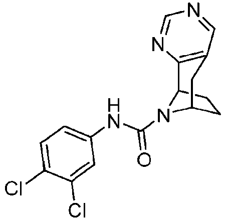 | (17) | 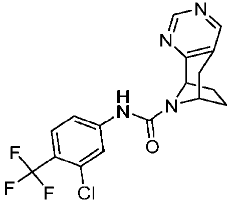 | (18) |
| 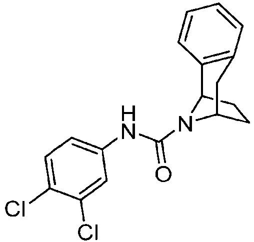 | (34) | 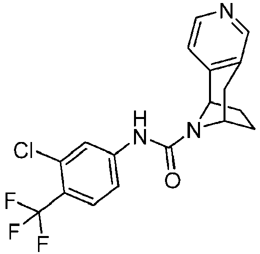 | (35) |
| 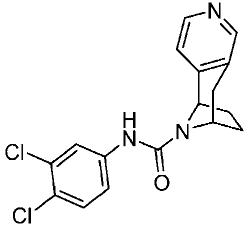 | (36) | 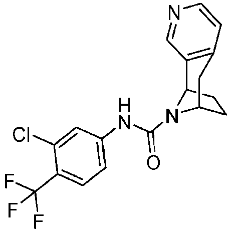 | (37) |
| | | 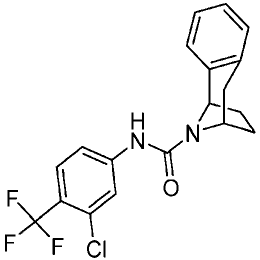 | (54) |
| 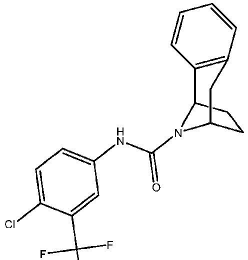 | (55) | 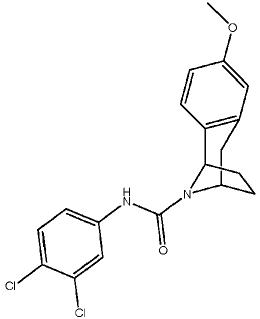 | (56) |
| 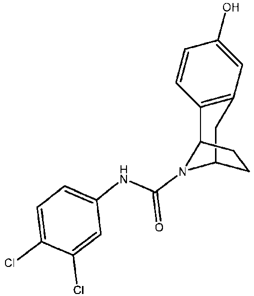 | (57) | 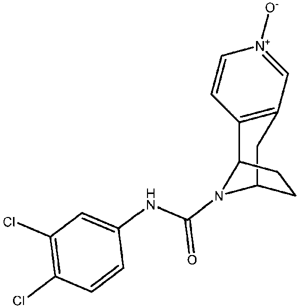 | (58) |
| 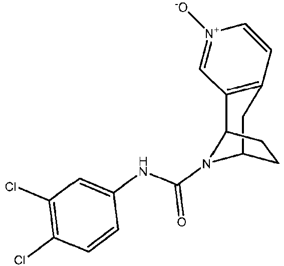 | (59) | 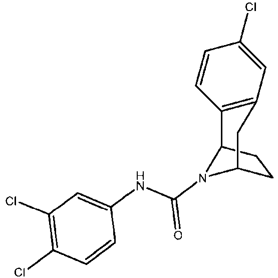 | (60) |
| 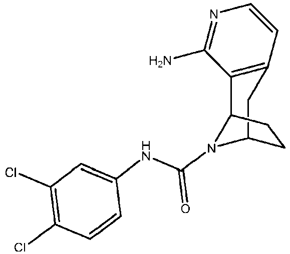 | (61) | 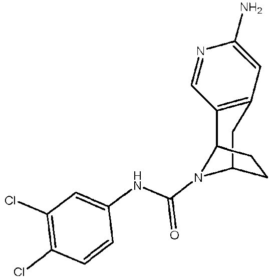 | (62) |
| 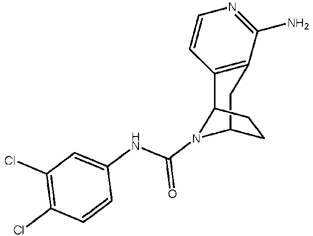 | (63) | 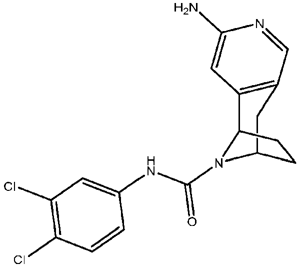 | (64) |
| 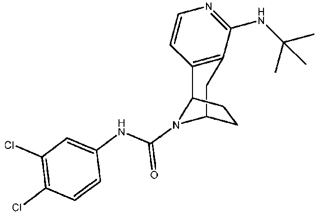 | (65) | 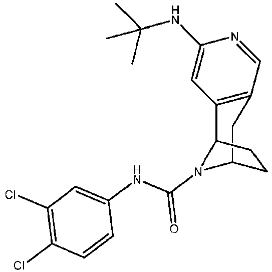 | (66) |
| 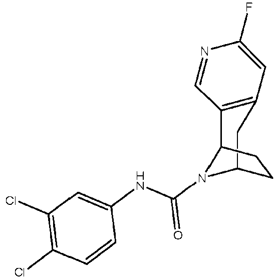 | (67) | 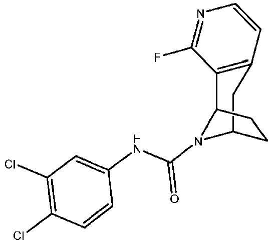 | (68) |
| 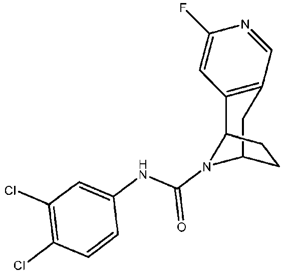 | (69) | 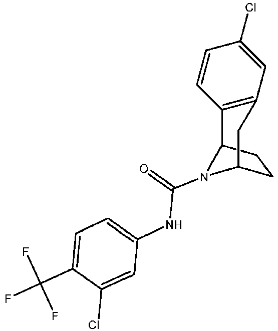 | (71) |
| 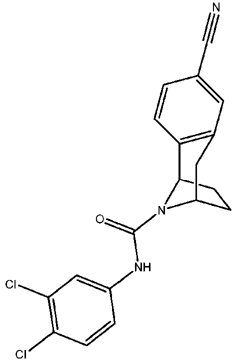 | (72) | 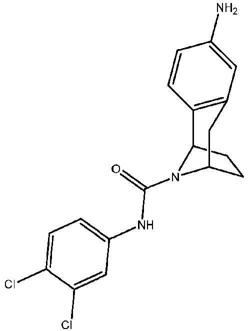 | (73) |
| 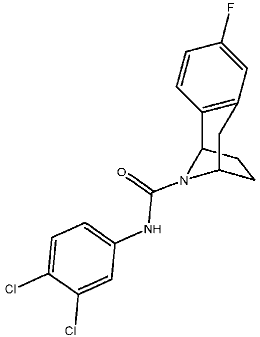 | (74) | 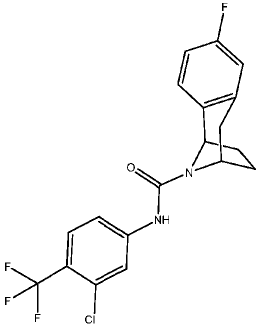 | (75) |
| 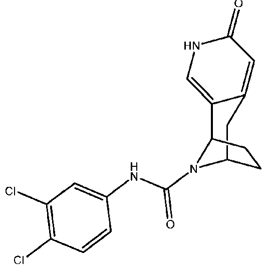 | (76) | 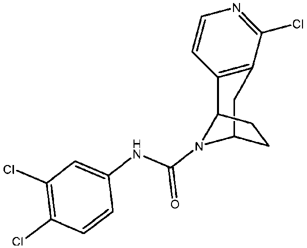 | (77) |
| 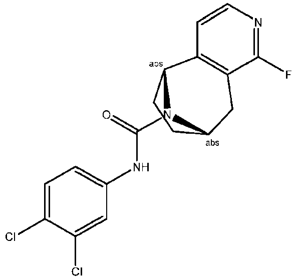 | (78) | 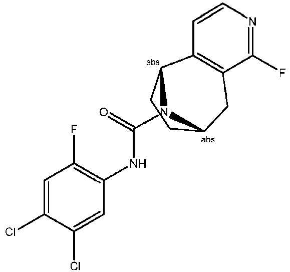 | (79) |
| 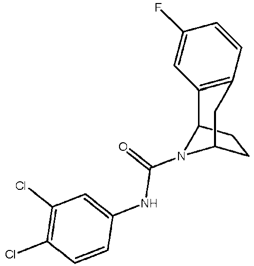 | (80) | 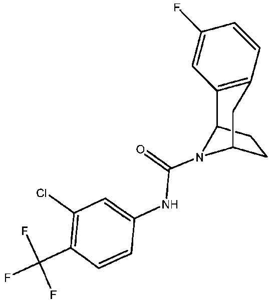 | (81) |
| 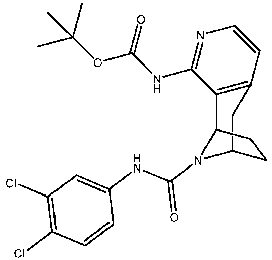 | (82) | 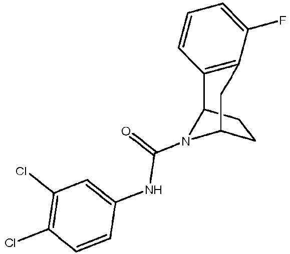 | (83) |
| 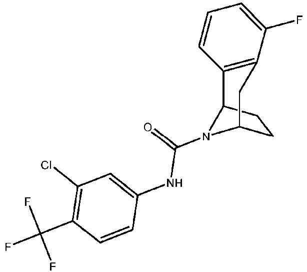 | (84) | 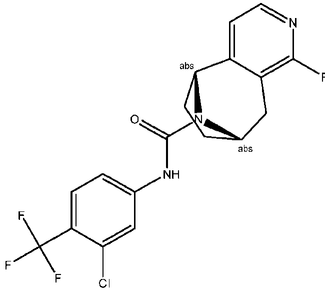 | (85) |
| 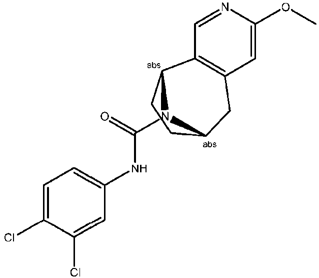 | (86) | 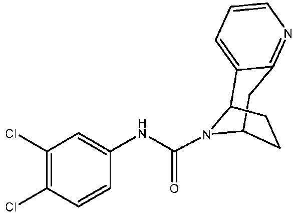 | (87) |
| 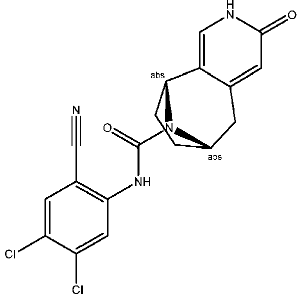 | (88) | 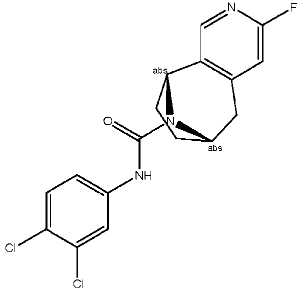 | (91) |
| 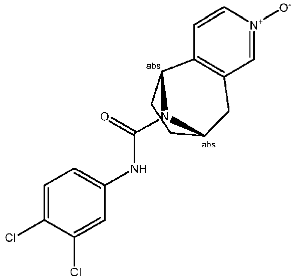 | (92) | 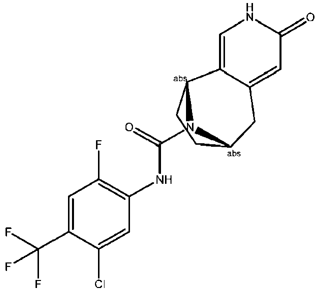 | (93) |
| 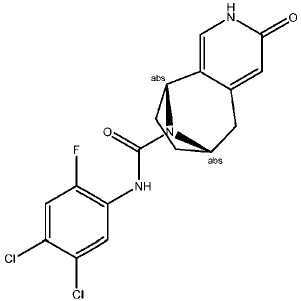 | (94) | 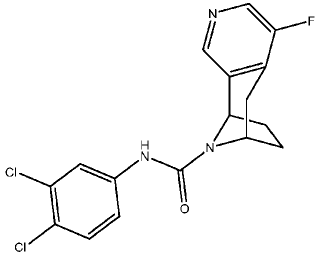 | (95) |
| 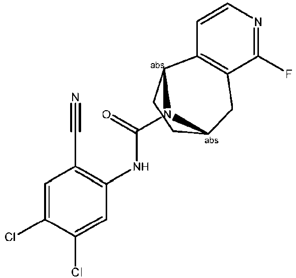 | (98) | 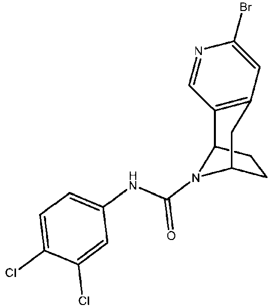 | (99) |
| 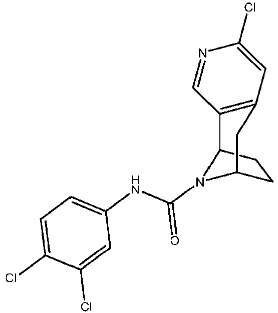 | (100) | 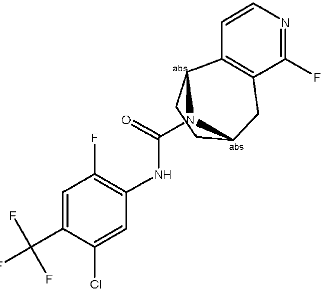 | (101) |
| 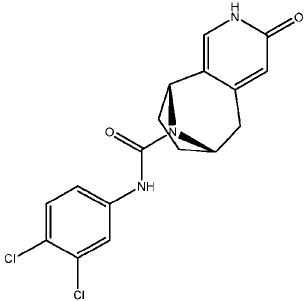 | (103) | 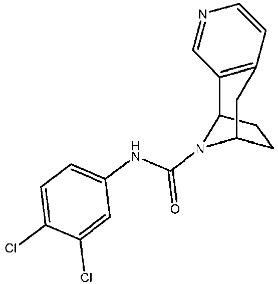 | (104) |
| 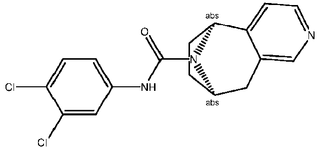 | (107) | 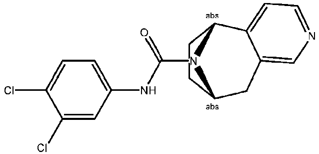 | (108) |
| 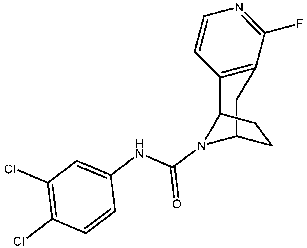 | (109) | 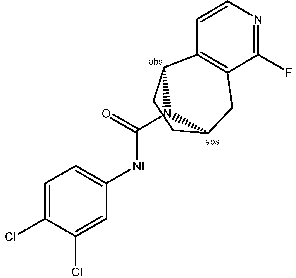 | (110) |
| 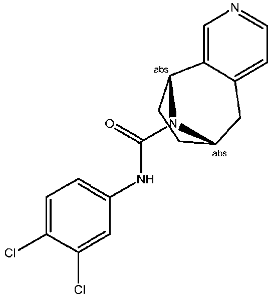 | (111) | 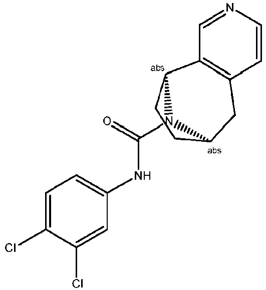 | (112) |
| 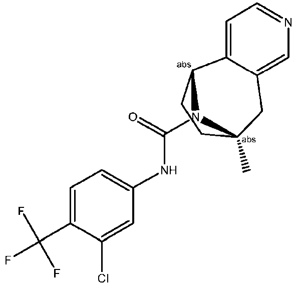 | (116) | 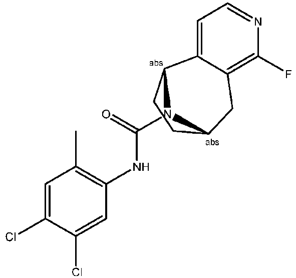 | (117) |
| 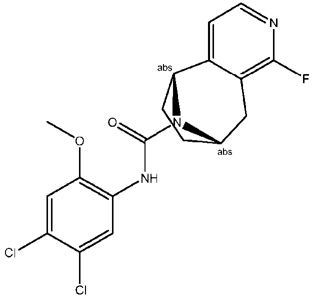 | (118) | 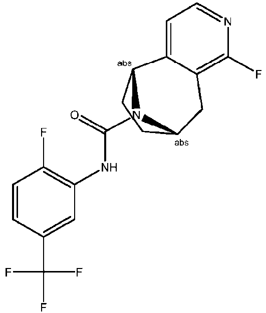 | (119) |
| 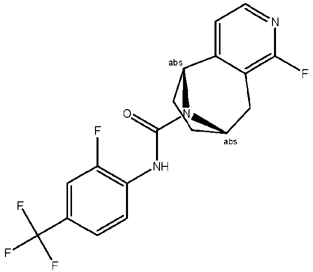 | (120) | 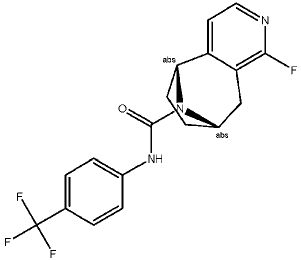 | (121) |
| 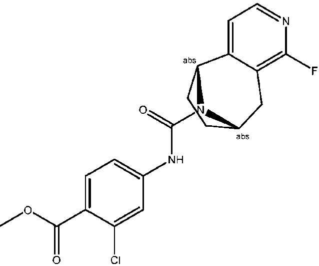 | (122) | 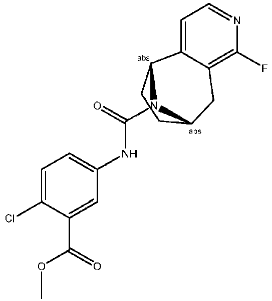 | (123) |
| 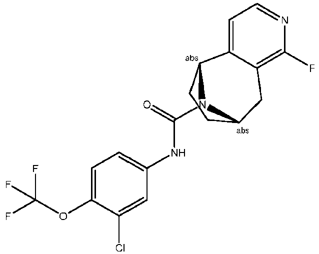 | (124) | 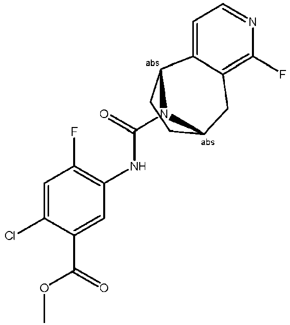 | (125) |
| 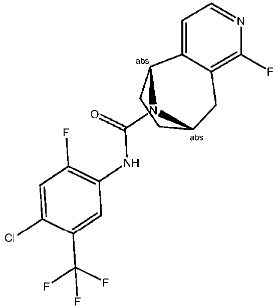 | (126) | 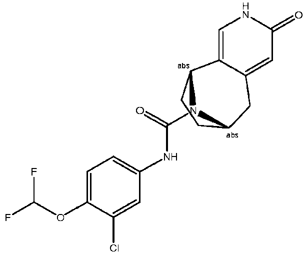 | (127) |
| 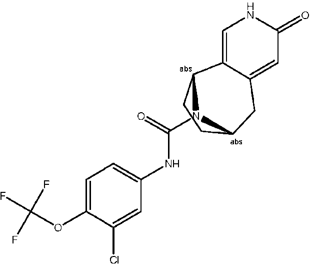 | (128) | 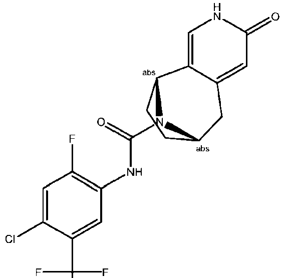 | (129) |
| 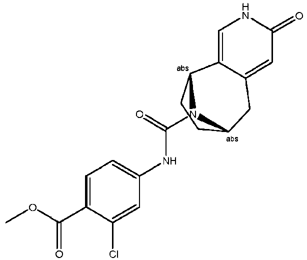 | (130) | 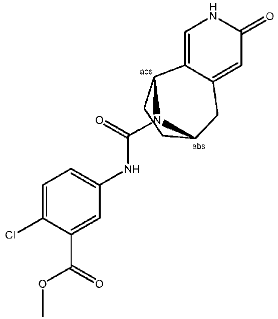 | (131) |
| 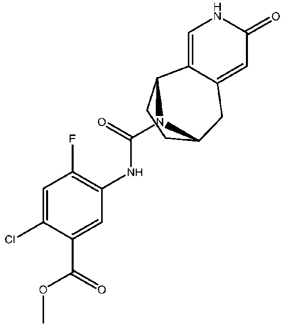 | (132) | 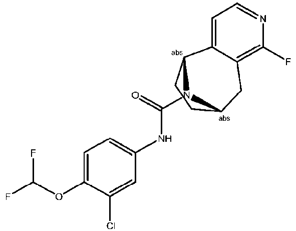 | (133) |
| 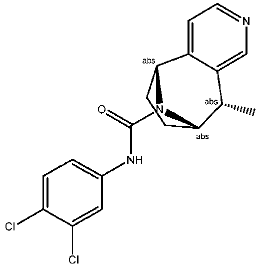 | (134) | 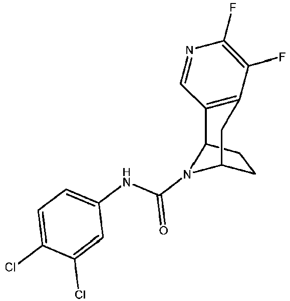 | (135) |
| 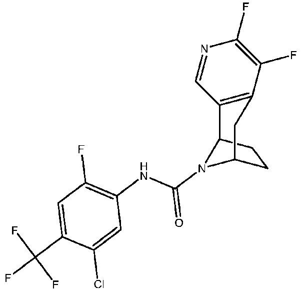 | (136) | 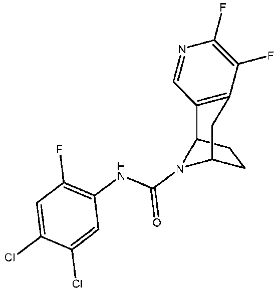 | (137) |
| 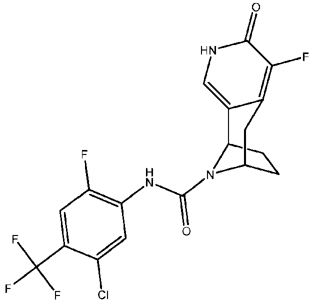 | (138) | 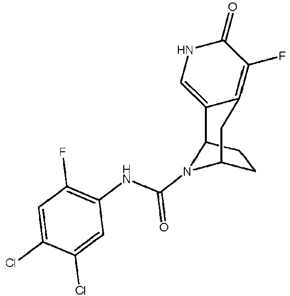 | (139) |
| 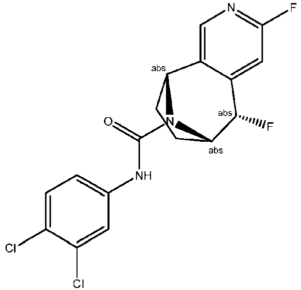 | (140) | 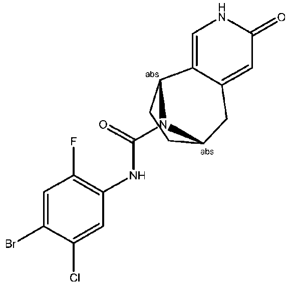 | (141) |
| 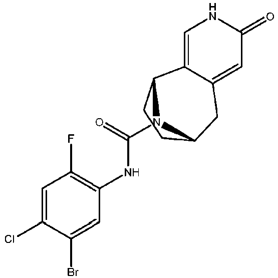 | (142) | 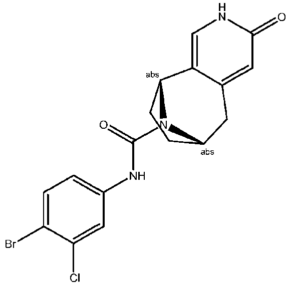 | (143) |
| 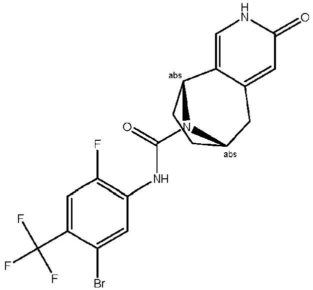 | (144) | 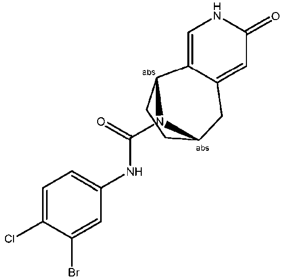 | (145) |
| 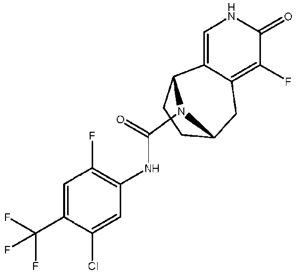 | (146) | 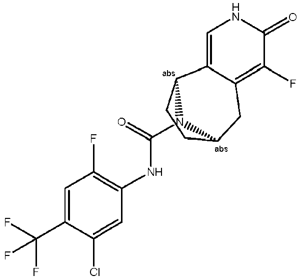 | (147) |
| 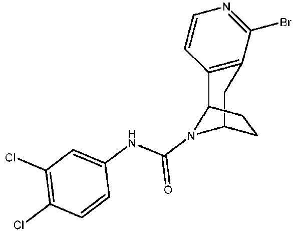 | (148) | 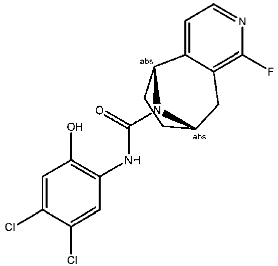 | (149) |
| 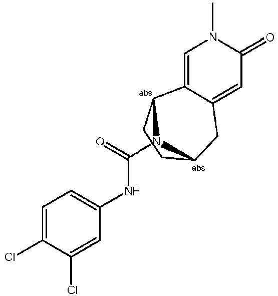 | (150) | | |
| 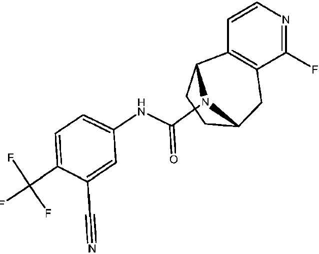 | (151) | 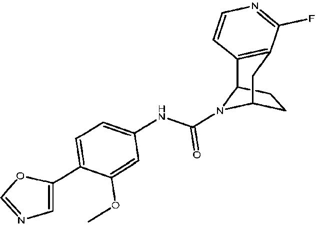 | (152) |
| 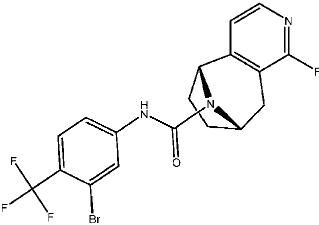 | (153) | 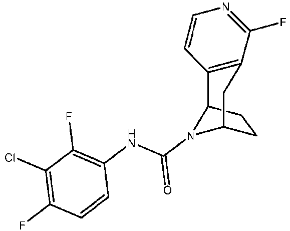 | (154) |
| 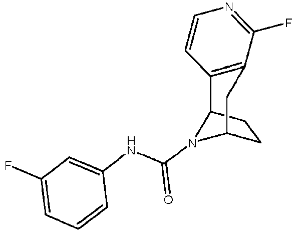 | (155) | 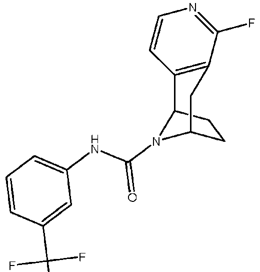 | (156) |
| 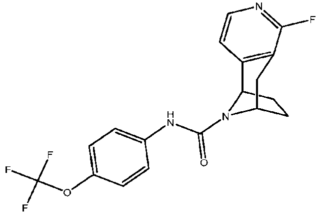 | (157) | 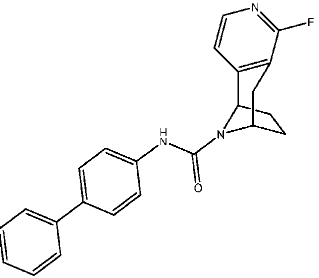 | (158) |
| 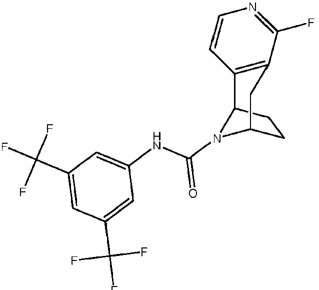 | (159) | 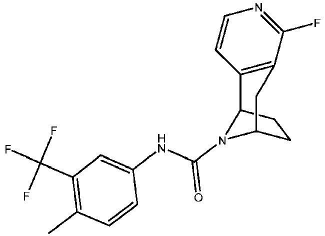 | (160) |
| 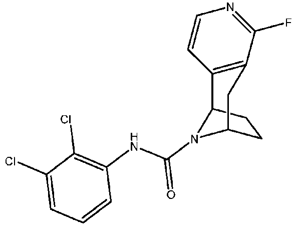 | (161) | 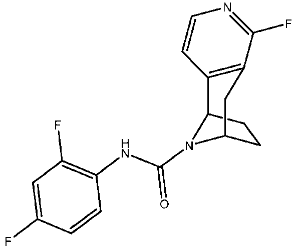 | (162) |
| 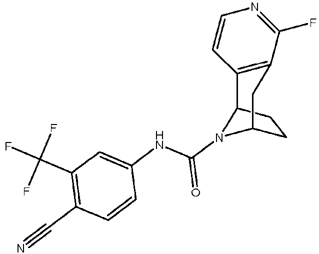 | (163) | 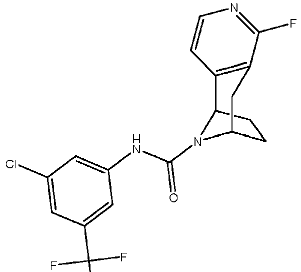 | (164) |
| 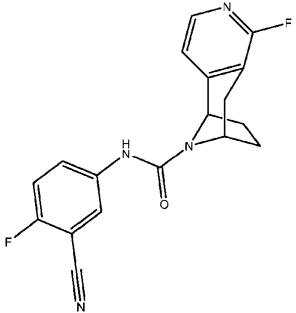 | (165) | 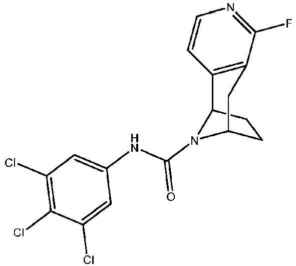 | (166) |
| 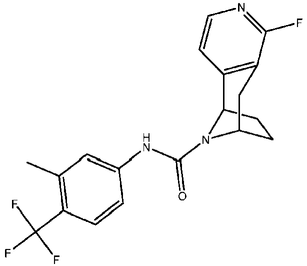 | (167) | 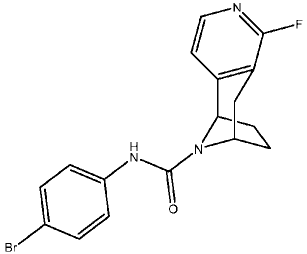 | (168) |
| 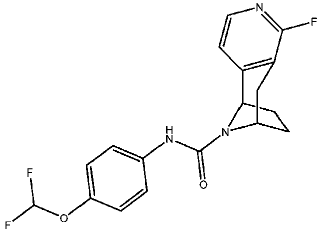 | (169) | 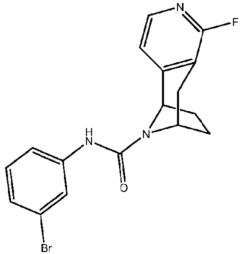 | (170) |
| 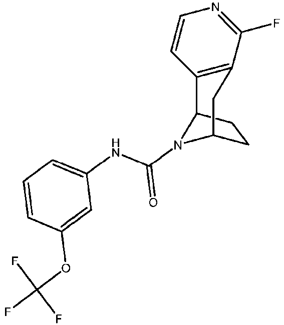 | (171) | 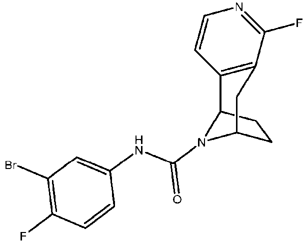 | (172) |
| 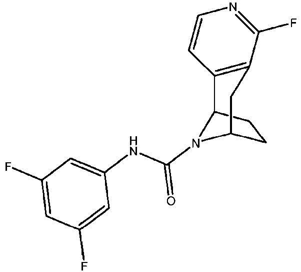 | (173) | 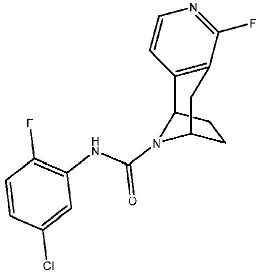 | (174) |
| 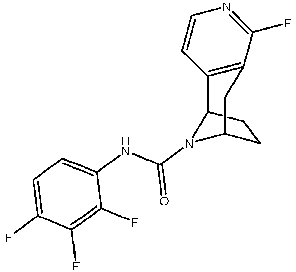 | (175) | 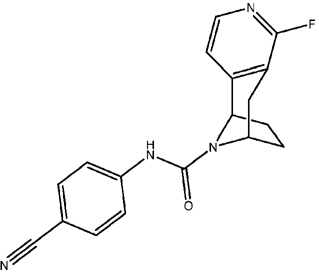 | (176) |
| 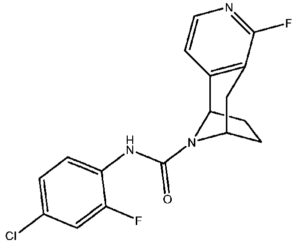 | (177) | 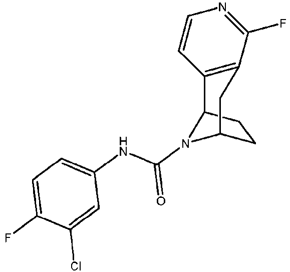 | (178) |
| 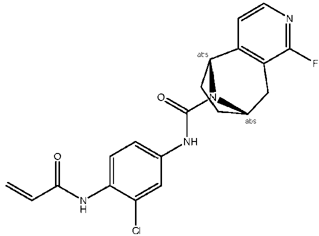 | (179) | 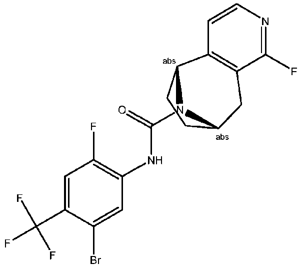 | (180) |
| 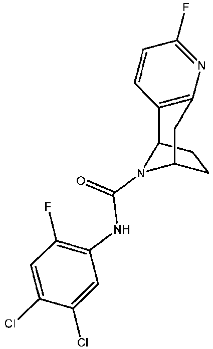 | (181) | 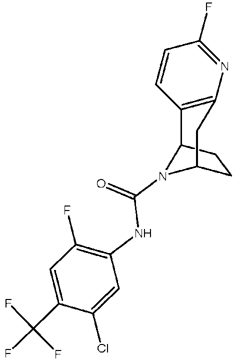 | (182) |
| 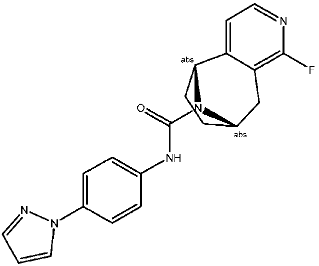 | (183) | 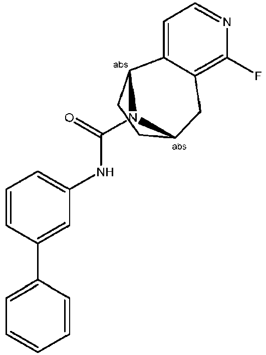 | (184) |
| 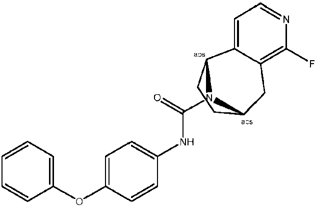 | (185) | 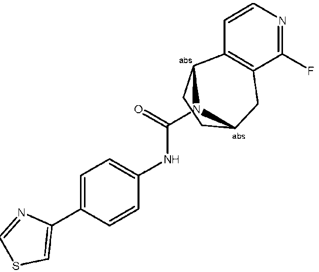 | (186) |
| 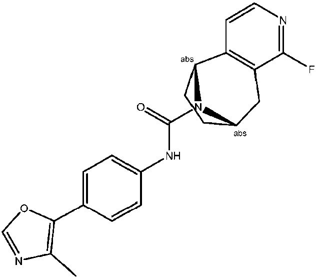 | (187) | 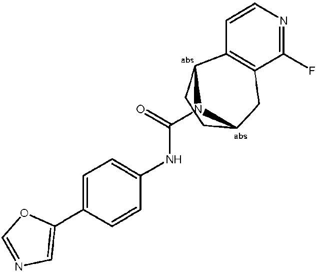 | (188) |
| 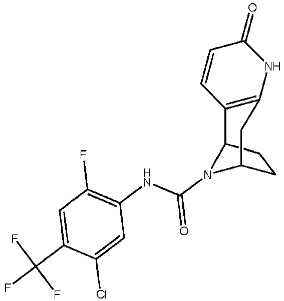 | (189) | 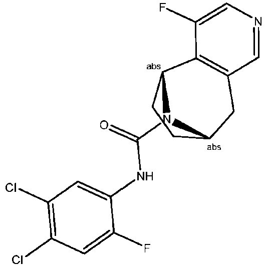 | (190) |
| 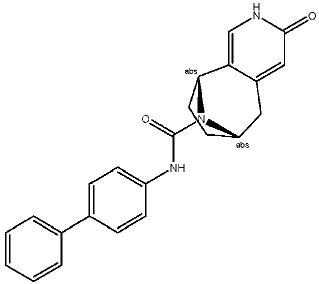 | (191) | 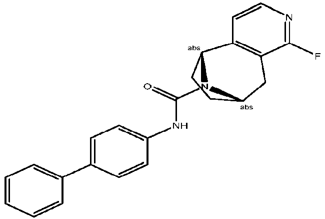 | (192) |
| 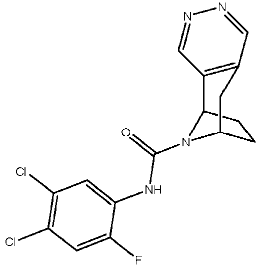 | (193) | 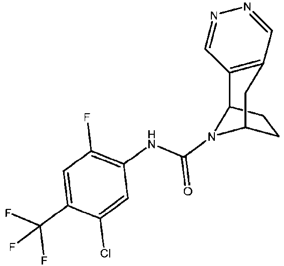 | (194) |
| 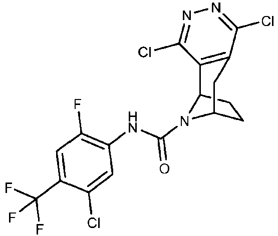 | (195) | 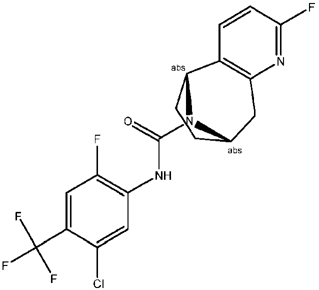 | (196) |
| 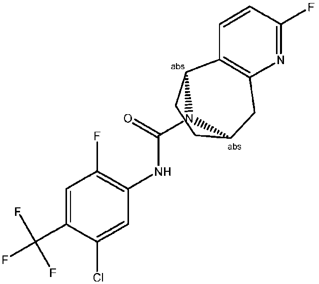 | (197) | 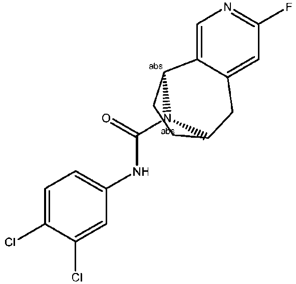 | (198) |
| 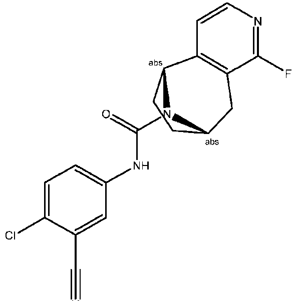 | (199) | 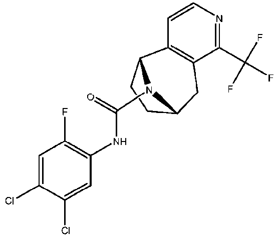 | 200 |
et énantiomères correspondants, et mélanges d'énantiomères correspondants, y compris mélanges racémiques, et sels et solvates pharmaceutiquement acceptables correspondants.

13. Composé de formule (Ie), ou sel ou solvate pharmaceutiquement acceptable correspondant, 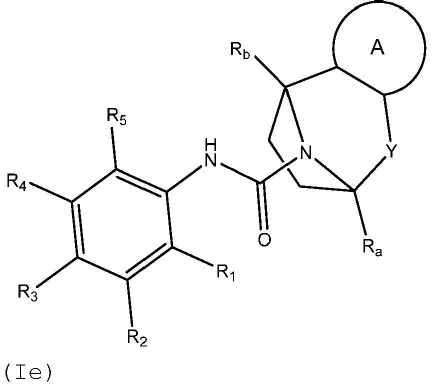
le cycle A étant 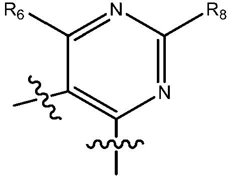
R₆ et R₈ étant chacun indépendamment choisis parmi H, F, Cl, Br, I, CN, alcoxy, OH, phényle, et halogénoalkyle ;
Y étant choisi parmi C=N-OH et CR₁₀R₁₀', R₁₀ et R₁₀' étant chacun indépendamment choisis parmi H, F, alkyle, et halogénoalkyle ;
Rₐ et R_{b} étant chacun indépendamment choisis parmi H et alkyle ;
R₁, R₄, et R₅ étant chacun indépendamment choisis parmi H, F, Cl, Br, et I ; et
R₂ et R₃ étant chacun indépendamment choisis parmi Cl, Br, I, CN et halogénoalkyle ;
à la condition que lorsque Y est CH₂, et Rₐ, R_{b}, R₁, R₄, R₅, R₆ et R₈ sont tous H :
R₃ ne soit pas CN, lorsque R₂ est Cl ; et
R₂ et R₃ ne soient pas tous deux Cl.

14. Composé selon la revendication 13, R₂ étant choisi parmi Cl, Br et CF₃, et étant plus préférablement choisi parmi Cl et CF₃.

15. Composé selon la revendication 13 ou la revendication 14, R₃ étant choisi parmi Cl, Br, CN et CF₃, étant plus préférablement choisi parmi Cl et CF₃.

16. Composé selon l'une quelconque des revendications 13 à 15, R₁, R₄ et R₅ étant tous H.

17. Composé selon l'une quelconque des revendications 13 à 16, Y étant choisi parmi CH₂ et CHF, et étant préférablement CH₂.

18. Composé selon l'une quelconque des revendications 13 à 17, R₆ étant H.

19. Composé selon l'une quelconque des revendications 13 à 18, R₈ étant choisi parmi H, CF₃, phényle, OH et F, éventuellement R₈ étant OH, et le cycle A étant :

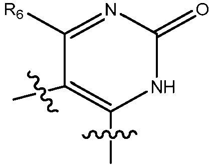

20. Composé selon l'une quelconque des revendications 13 à 19, qui est de formule (Ie.1) : 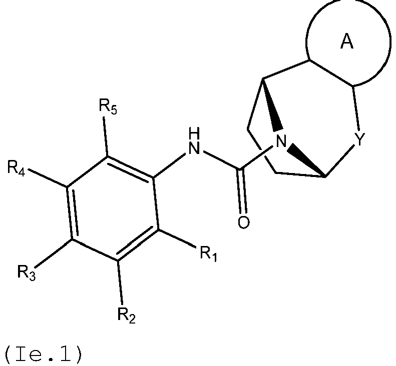 où A, Y et R₁-R₅ sont tels que définis dans l'une quelconque des revendications 13 à 19, ou qui est sous la forme d'un mélange qui est énantiomériquement enrichi en composé de formule (Ie.1).

21. Composé choisi parmi les suivants :
| | | | |
|---|---|---|---|
| 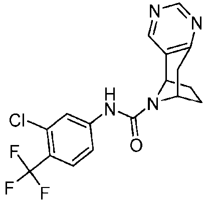 | (2) | | |
| 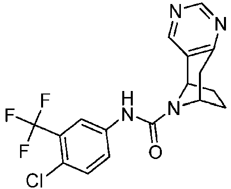 | (3) | 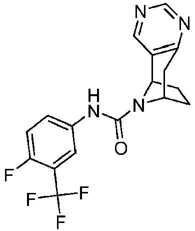 | (5) |
| 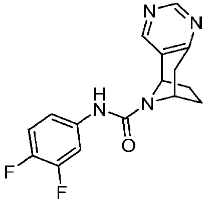 | (6) | 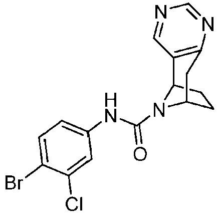 | (7) |
| 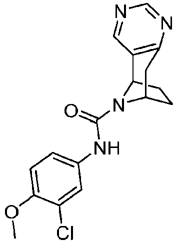 | (8) | 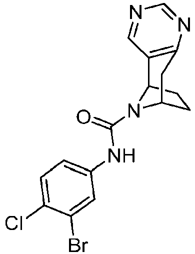 | (12) |
| 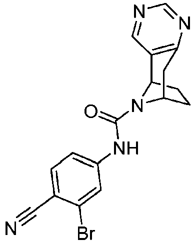 | (13) | 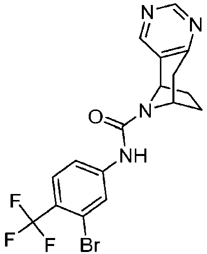 | (15) |
| 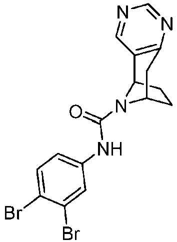 | (16) | 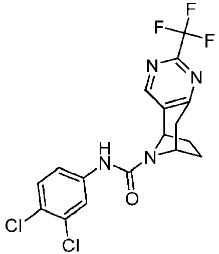 | (24) |
| 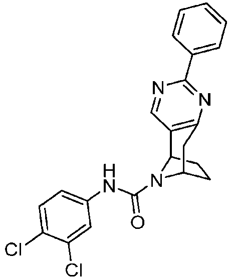 | (26) | 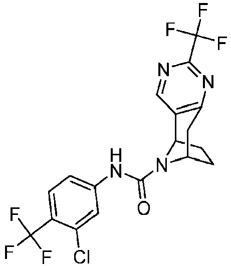 | (27) |
| 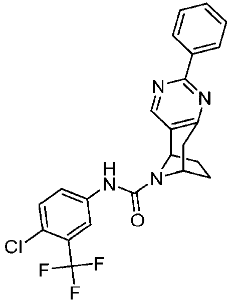 | (28) | 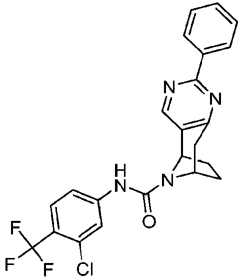 | (29) |
| 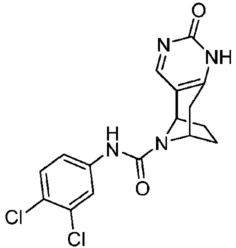 | (31) | 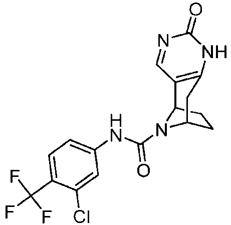 | (32) |
| | | 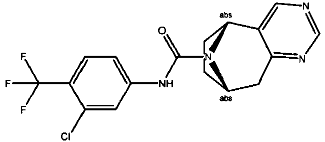 Énantiomère de (2) | (38) |
| 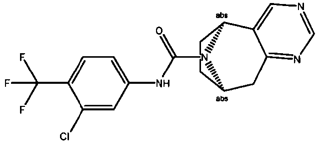 Énantiomère de (2) | (39) | 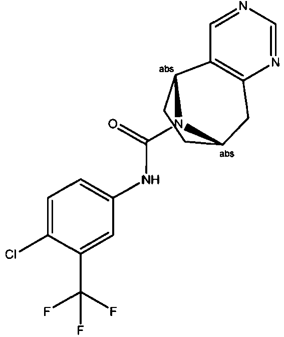 Énantiomère de (3) | (42) |
| 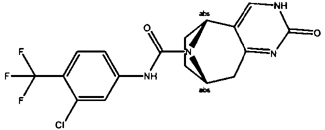 Énantiomère de (32) | (43) | 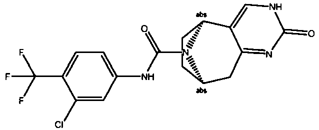 Énantiomère de (32) | (44) |
| 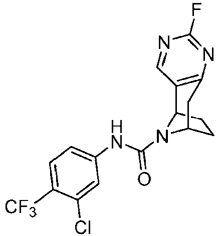 | (45) | 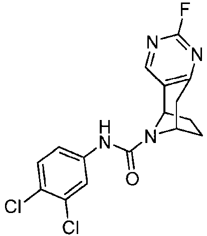 | (46) |
| 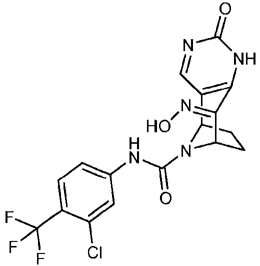 | (47) | 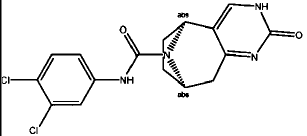 Énantiomère de (31) | (105) |
| 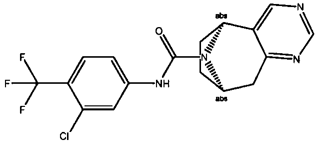 Énantiomère de (31) | (106) | 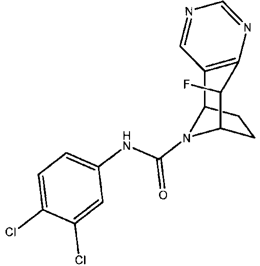 | (113) |
| 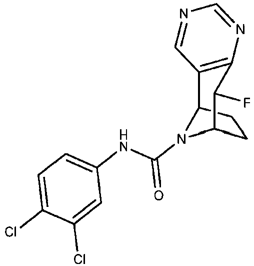 | (114) | 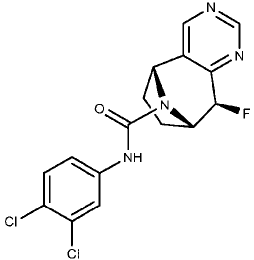 | (115) |
et énantiomères correspondants, et mélanges d'énantiomères correspondants, y compris mélanges racémiques, et sels et solvates pharmaceutiquement acceptables correspondants.

22. Composition pharmaceutique comprenant un composé selon l'une quelconque des revendications 1 à 21, et un diluant, excipient ou support pharmaceutiquement acceptable.

23. Composé selon l'une quelconque des revendications 1 à 21, ou composition pharmaceutique selon la revendication 22, pour une utilisation comme médicament.

24. Composé selon l'une quelconque des revendications 1 à 21, ou composition pharmaceutique selon la revendication 22, pour une utilisation dans le traitement ou la prévention d'un trouble choisi parmi un trouble prolifératif, un trouble immunitaire, l'asthme, une maladie pulmonaire obstructive chronique (COPD) et un syndrome de détresse respiratoire aiguë (ARDS).

25. Composé ou composition pharmaceutique pour une utilisation selon la revendication 24, dans lequel l'utilisation comprend la modulation de GPR65, de préférence dans lequel l'utilisation comprend l'inhibition de la signalisation de GPR65.

26. Composé ou composition pharmaceutique pour une utilisation selon la revendication 24, dans lequel le trouble est un trouble prolifératif, de préférence :
dans lequel le trouble prolifératif est un cancer, et est de préférence une tumeur solide et/ou des métastases de celui-ci ; ou
dans lequel le trouble prolifératif est un cancer choisi parmi le mélanome, le carcinome à cellules rénales (RCC), le cancer gastrique, la leucémie myéloïde aiguë (AML), le cancer du sein triple négatif (TNBC), le cancer colorectal, le cancer de la tête et du cou, l'adénocarcinome colorectal, l'adénocarcinome pancréatique, le sarcome, le cancer du poumon, le cancer de l'ovaire et les gliomes, de préférence le glioblastome (GBM) .

27. Composé ou composition pharmaceutique pour une utilisation selon la revendication 24, dans lequel le trouble est un trouble immunitaire, de préférence dans lequel le trouble immunitaire est une maladie auto-immune, de préférence dans lequel la maladie auto-immune est choisie parmi le psoriasis, le rhumatisme psoriasique, la polyarthrite rhumatoïde (PR), la sclérose en plaques (SEP), le lupus érythémateux disséminé (LED), la thyroïdite auto-immune (thyroïdite de Hashimoto), la maladie de Graves, l'uvéite (y compris l'uvéite intermédiaire), la rectocolite hémorragique, la maladie de Crohn, l'uvéorétinite auto-immune, la vascularite systémique, la polymyosite-dermatomyosite, la sclérodermie systémique, le syndrome de Sjögren, la spondylarthrite ankylosante et les spondylarthropathies apparentées, la sarcoïdose, l'anémie hémolytique auto-immune, les troubles plaquettaires immunologiques et les polyendocrinopathies auto-immunes, de préférence dans lequel la maladie auto-immune est choisie parmi le psoriasis, le rhumatisme psoriasique, la spondylarthrite ankylosante, la maladie de Crohn et la sclérose en plaques (MS).

28. Composé ou composition pharmaceutique pour une utilisation selon la revendication 24, dans lequel l'utilisation comprend le traitement ou la prévention d'un trouble choisi parmi l'asthme, une maladie pulmonaire obstructive chronique (COPD) et un syndrome de détresse respiratoire aiguë (ARDS).

29. Composé selon l'une quelconque des revendications 1 à 21, ou sel ou solvate pharmaceutiquement acceptable correspondant, ou composition pharmaceutique selon la revendication 22, pour une utilisation dans le traitement ou la prévention d'une maladie ou d'un trouble associé à GPR65.

30. Composé choisi parmi les suivants :
| | | | |
|---|---|---|---|
| 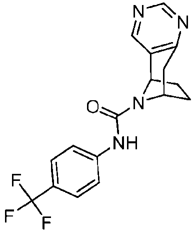 | (1) | 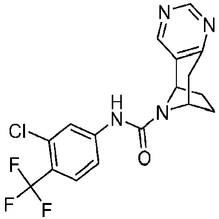 | (2) |
| 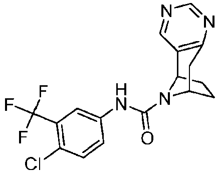 | (3) | 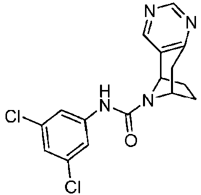 | (4) |
| 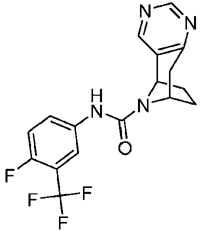 | (5) | 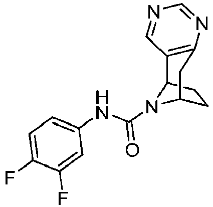 | (6) |
| 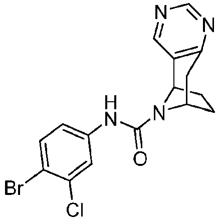 | (7) | 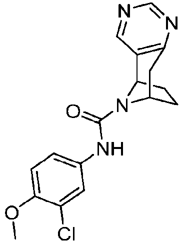 | (8) |
| 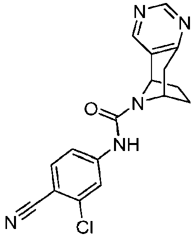 | (11) | 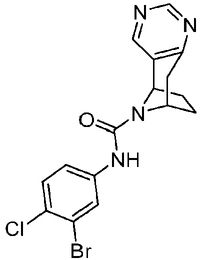 | (12) |
| 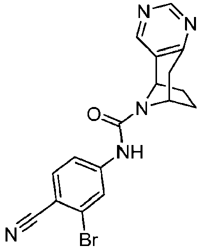 | (13) | 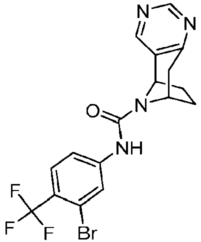 | (15) |
| 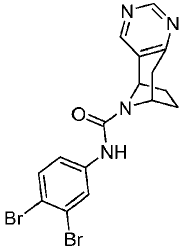 | (16) | 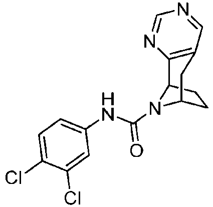 | (17) |
| 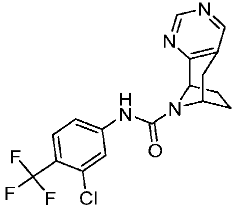 | (18) | 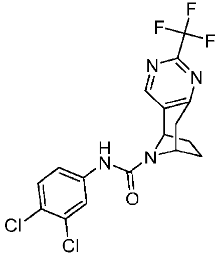 | (24) |
| 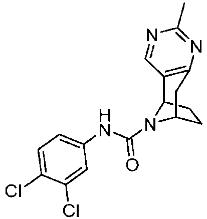 | (25) | 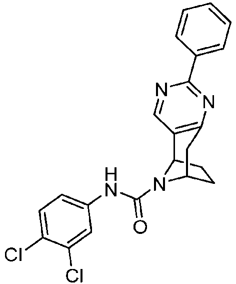 | (26) |
| 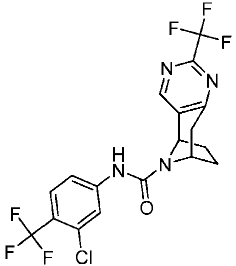 | (27) | 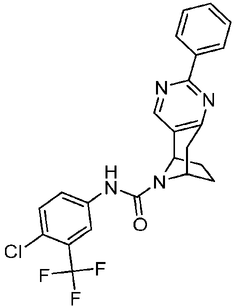 | (28) |
| 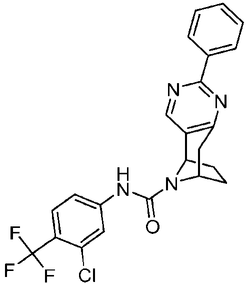 | (29) | 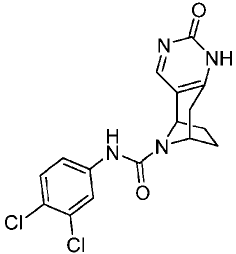 | (31) |
| 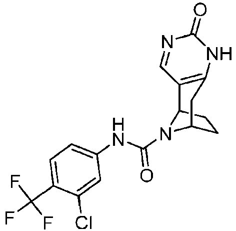 | (32) | 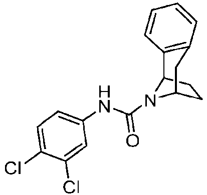 | (34) |
| 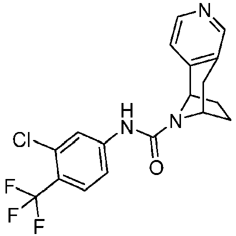 | (35) | 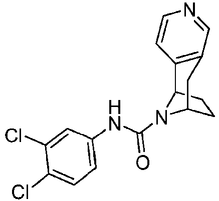 | (36) |
| 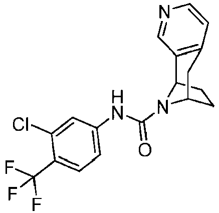 | (37) | 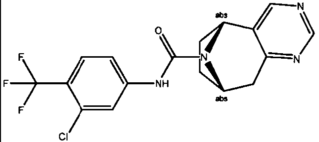 Énantiomère de (2) | (38) |
| 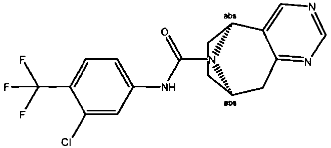 Énantiomère de (2) | (39) | 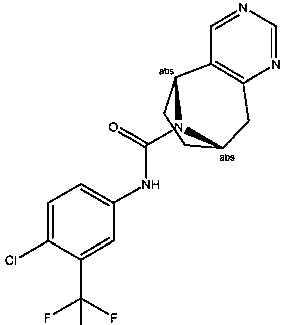 Énantiomère de (3) | (42) |
| 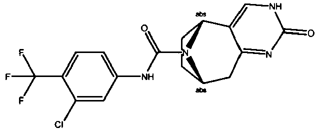 Énantiomère de (32) | (43) | 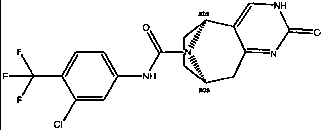 Énantiomère de (32) | (44) |
| 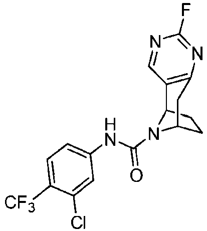 | (45) | 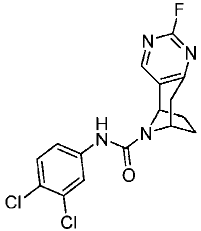 | (46) |
| 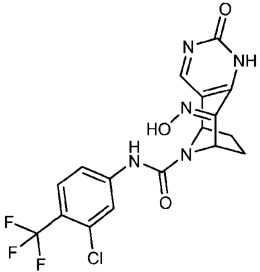 | (47) | 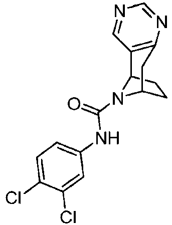 | (48) |
| 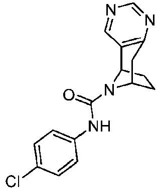 | (49) | 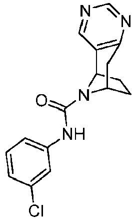 | (50) |
| 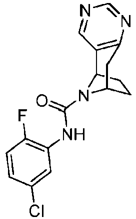 | (51) | 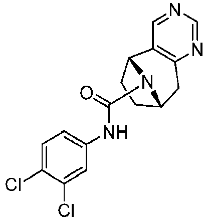 | (52) |
| 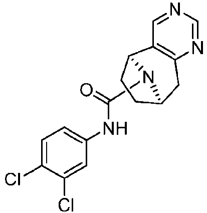 | (53) | 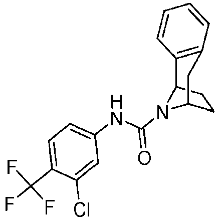 | (54) |
| 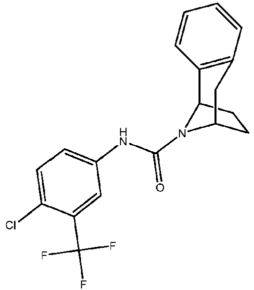 | (55) | 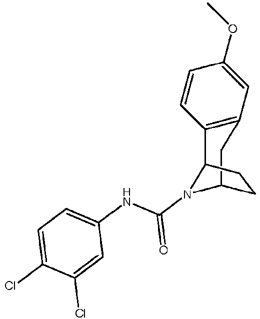 | (56) |
| | (57) | 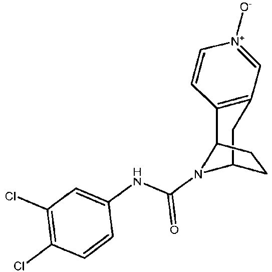 | (58) |
| 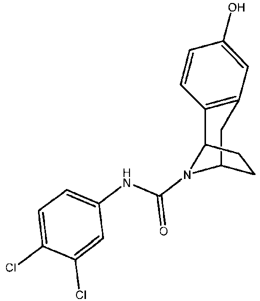 | | | |
| 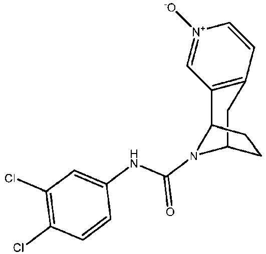 | (59) | 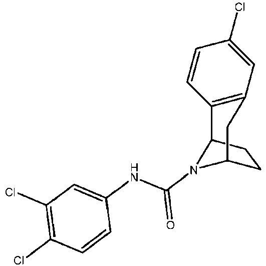 | (60) |
| 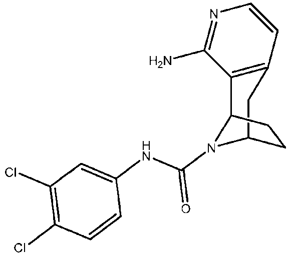 | (61) | 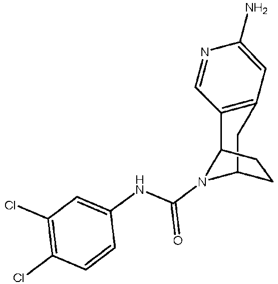 | (62) |
| 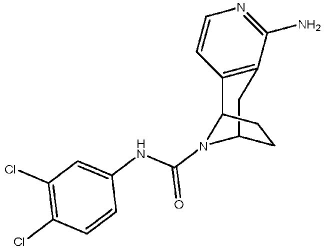 | (63) | 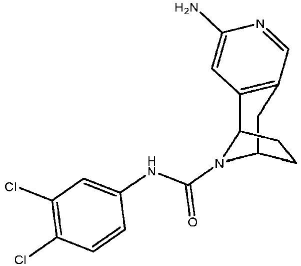 | (64) |
| 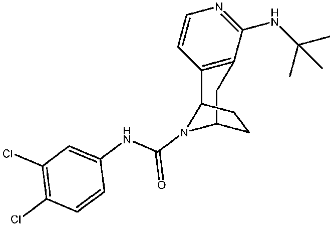 | (65) | 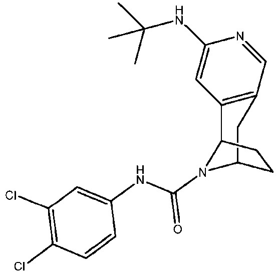 | (66) |
| 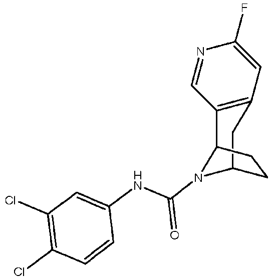 | (67) | 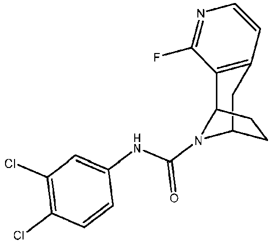 | (68) |
| 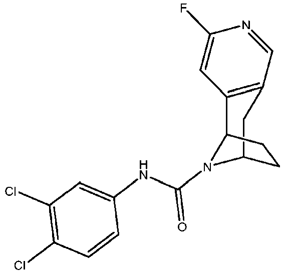 | (69) | 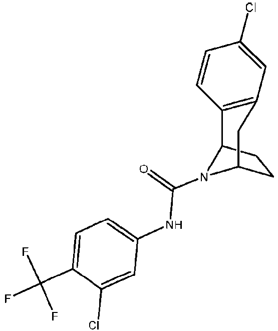 | (71) |
| 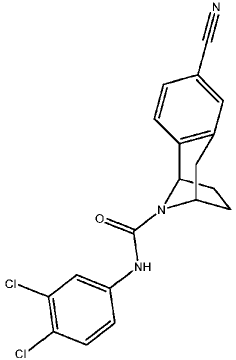 | (72) | 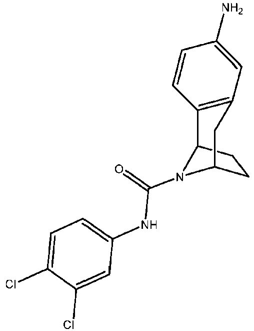 | (73) |
| 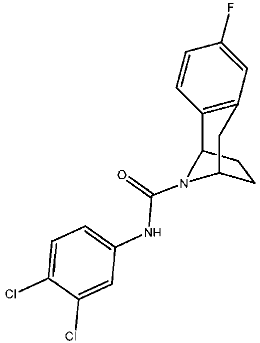 | (74) | 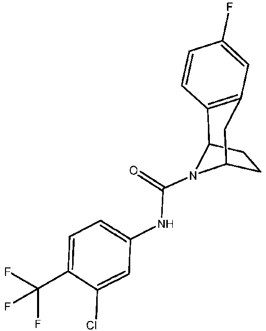 | (75) |
| 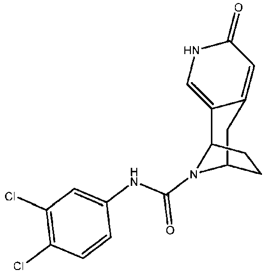 | (76) | 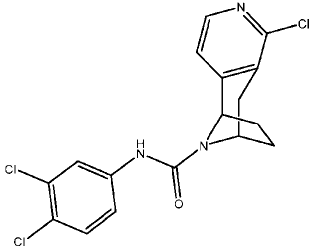 | (77) |
| 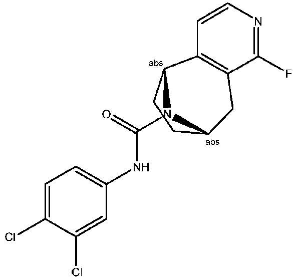 | (78) | 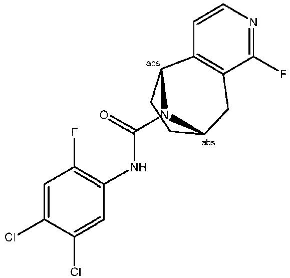 | (79) |
| 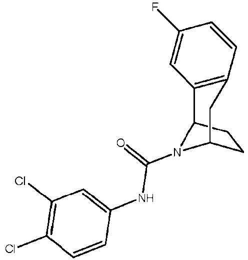 | (80) | 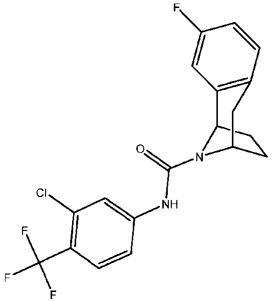 | (81) |
| 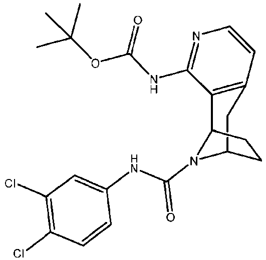 | (82) | 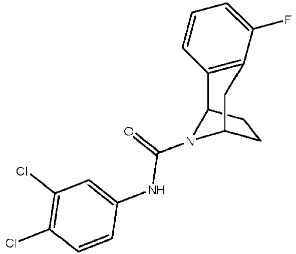 | (83) |
| 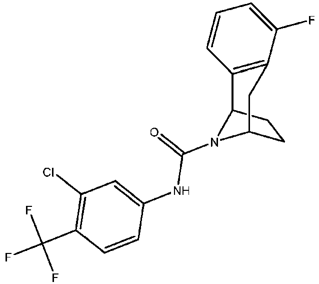 | (84) | 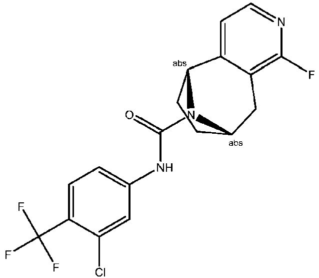 | (85) |
| 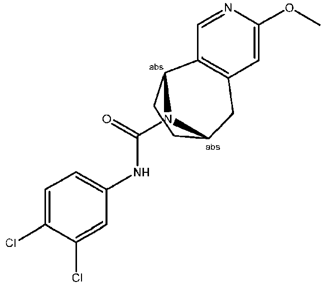 | (86) | 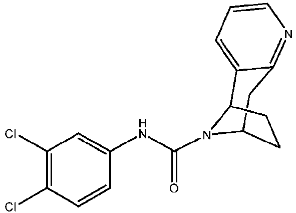 | (87) |
| 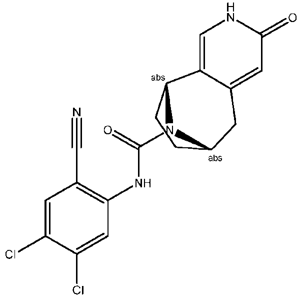 | (88) | 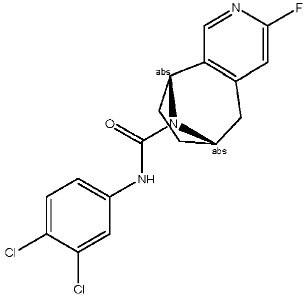 | (91) |
| 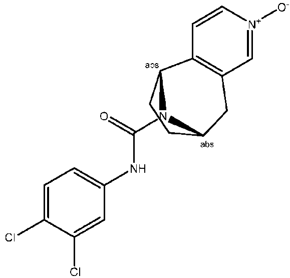 | (92) | 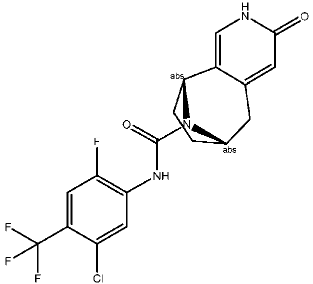 | (93) |
| 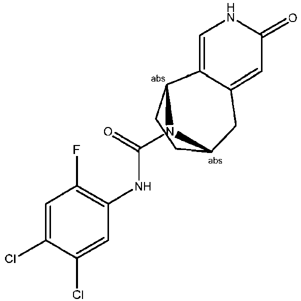 | (94) | 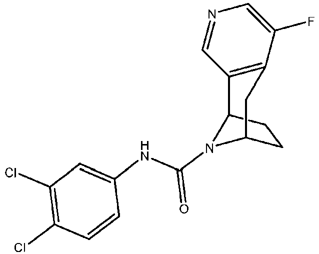 | (95) |
| 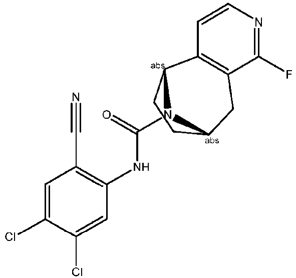 | (98) | 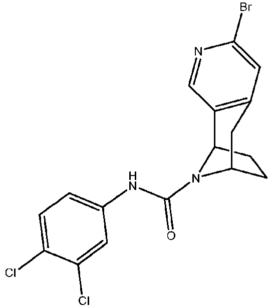 | (99) |
| 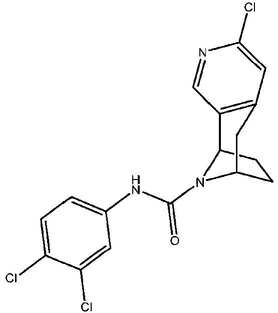 | (100) | 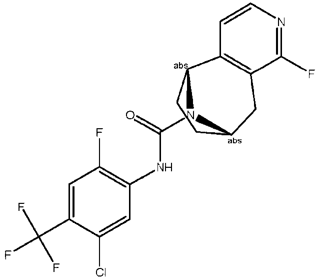 | (101) |
| 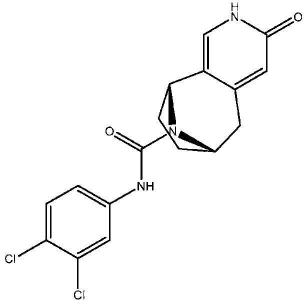 | (103) | 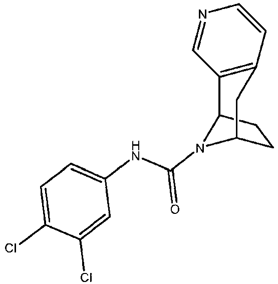 | (104) |
| 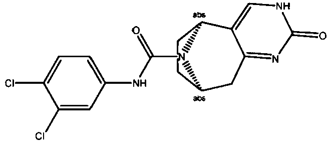 Énantiomère de (31) | (105) | 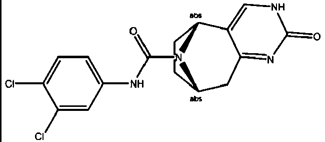 Énantiomère de (31) | (106) |
| 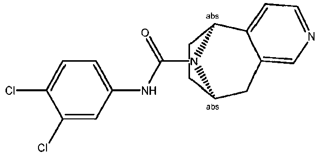 | (107) | 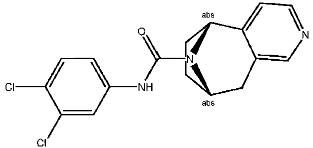 | (108) |
| 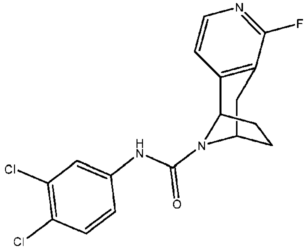 | (109) | 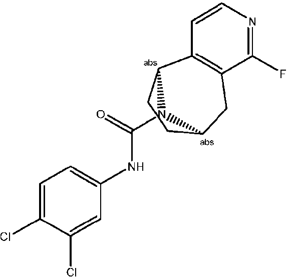 | (110) |
| 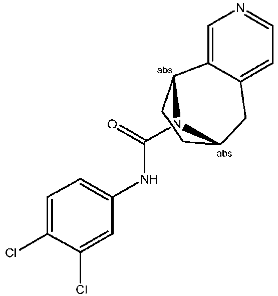 | (111) | 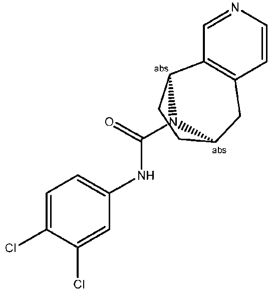 | (112) |
| 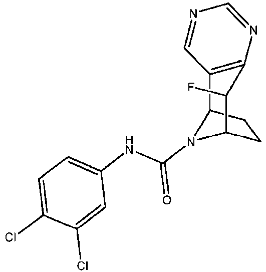 | (113) | 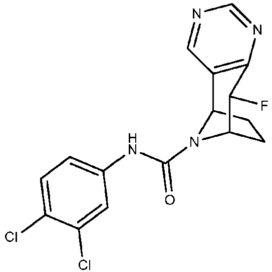 | (114) |
| 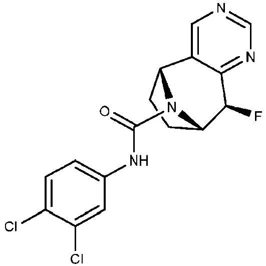 | (115) | | |
| 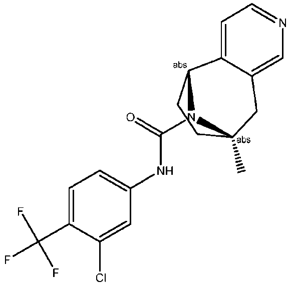 | (116) | 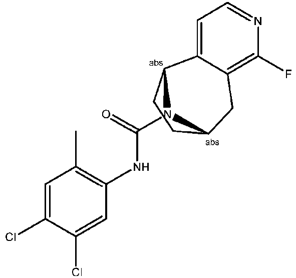 | (117) |
| 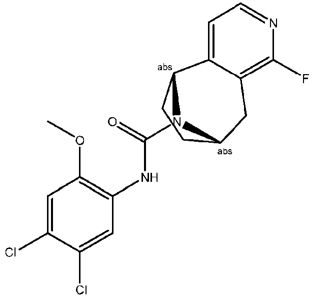 | (118) | 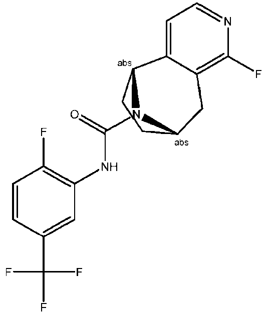 | (119) |
| 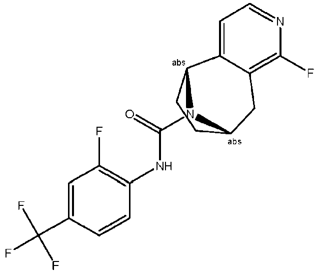 | (120) | 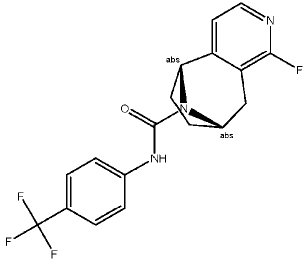 | (121) |
| 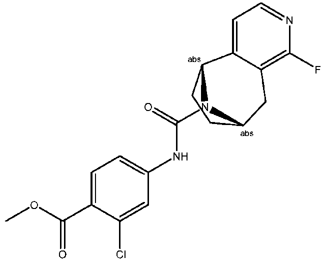 | (122) | 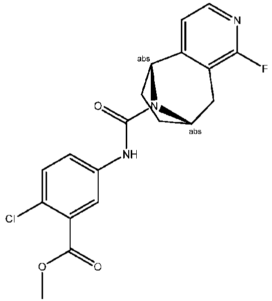 | (123) |
| 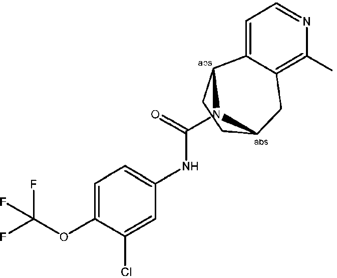 | (124) | 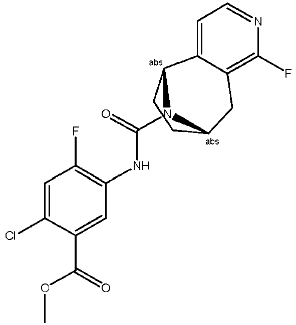 | (125) |
| 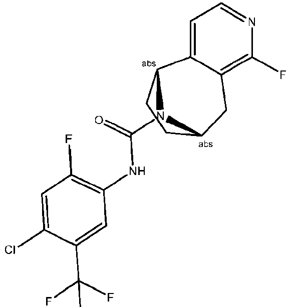 | (126) | 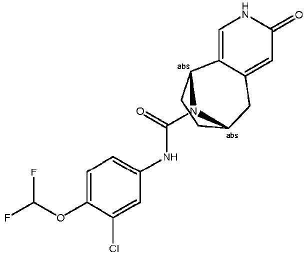 | (127) |
| 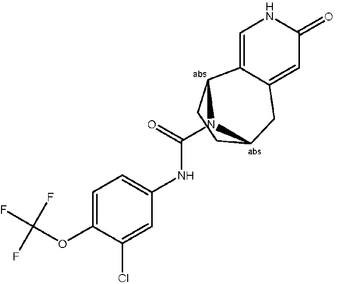 | (128) | 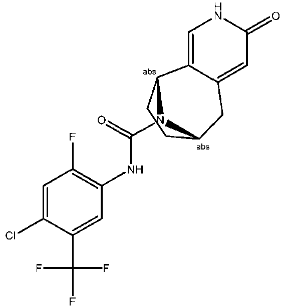 | (129) |
| 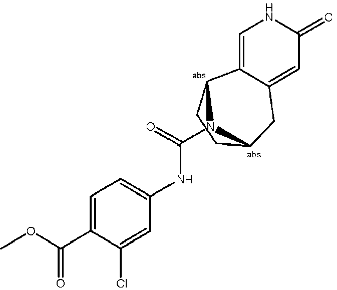 | (130) | 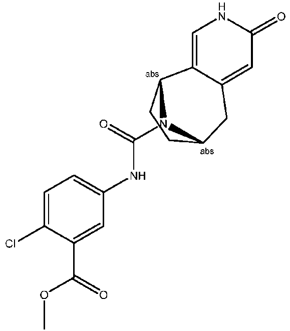 | (131) |
| 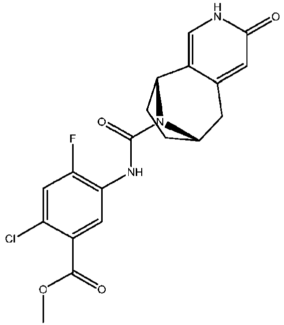 | (132) | 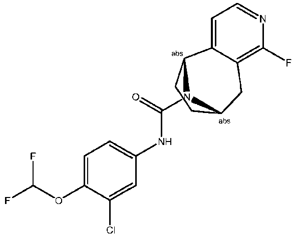 | (133) |
| 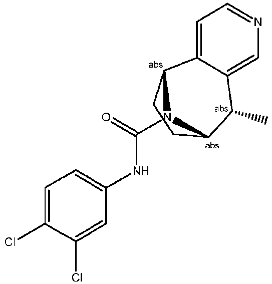 | (134) | 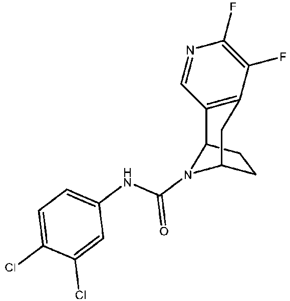 | (135) |
| 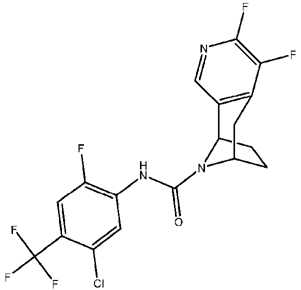 | (136) | 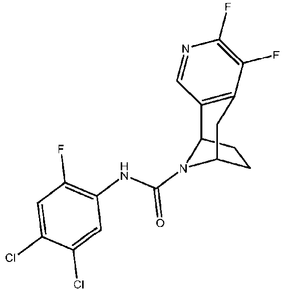 | (137) |
| 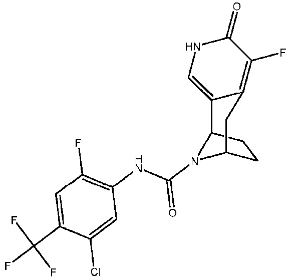 | (138) | 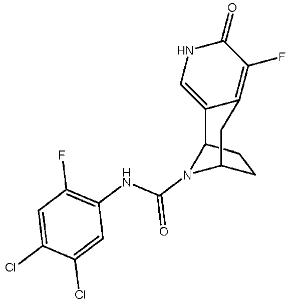 | (139) |
| 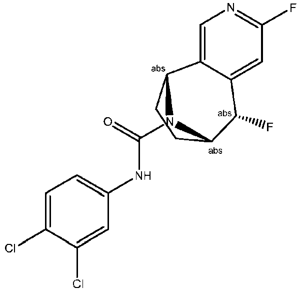 | (140) | 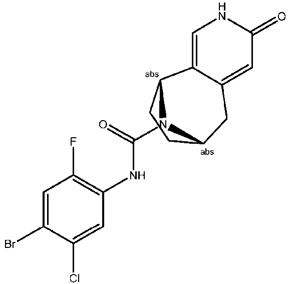 | (141) |
| 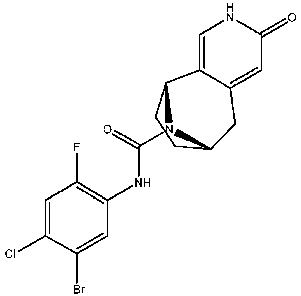 | (142) | 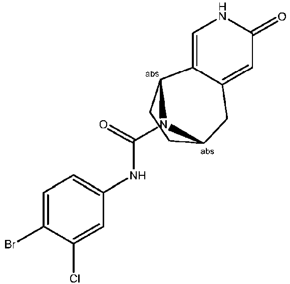 | (143) |
| 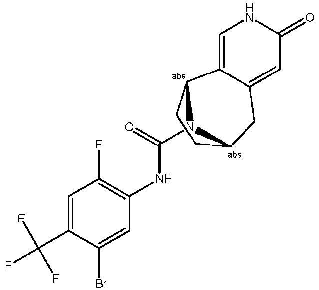 | (144) | 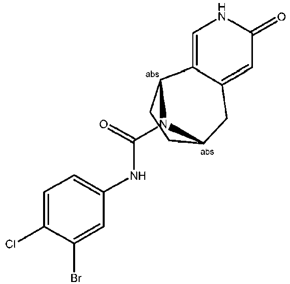 | (145) |
| 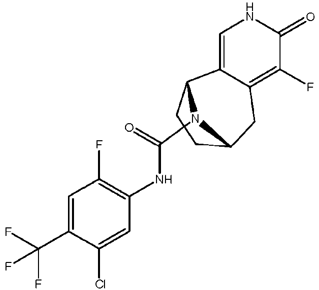 | (146) | 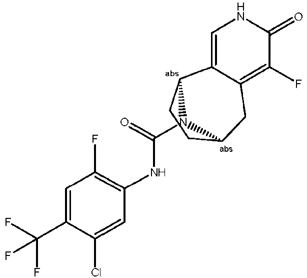 | (147) |
| 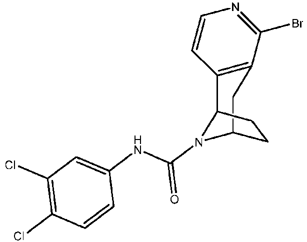 | (148) | 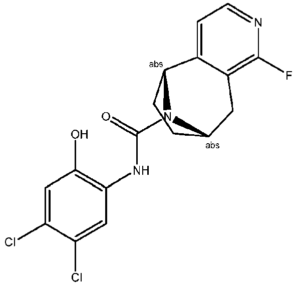 | (149) |
| 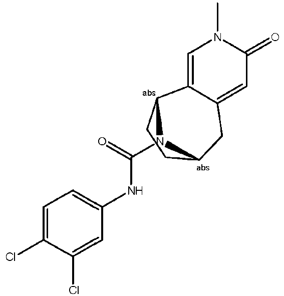 | (150) | | |
| 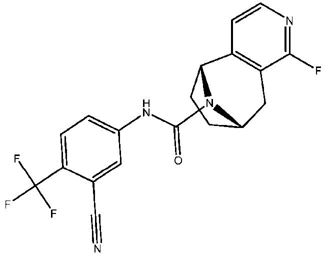 | (151) | 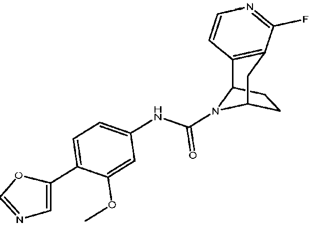 | (152) |
| 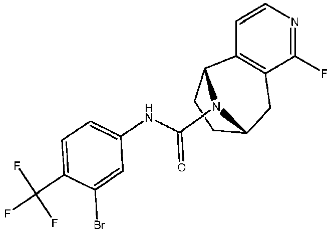 | (153) | 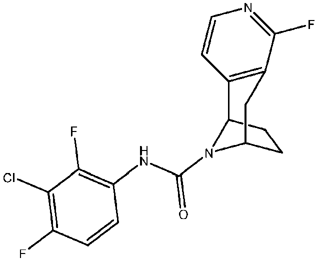 | (154) |
| 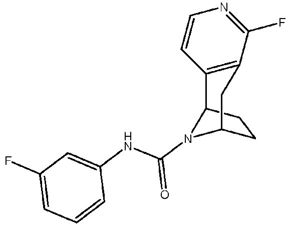 | (155) | 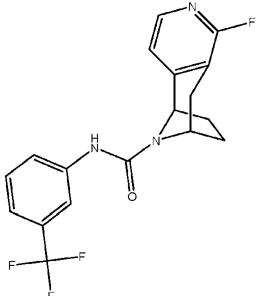 | (156) |
| 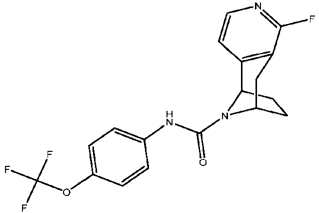 | (157) | 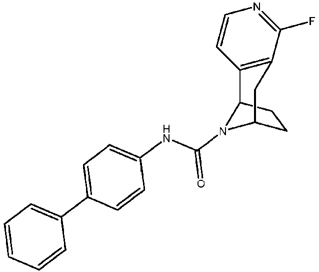 | (158) |
| 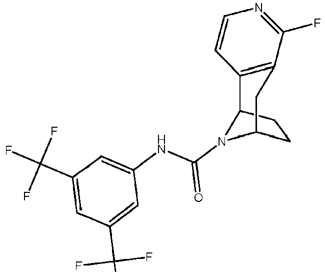 | (159) | 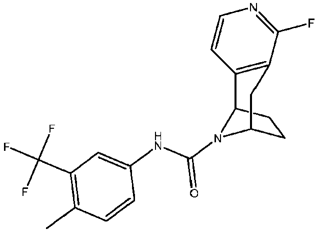 | (160) |
| 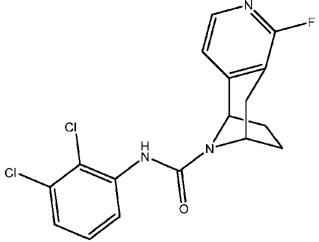 | (161) | 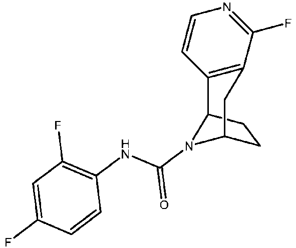 | (162) |
| 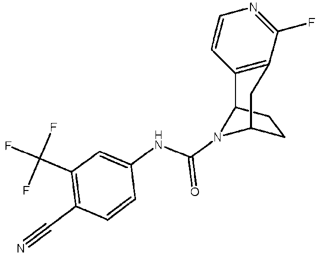 | (163) | 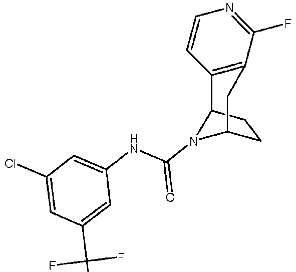 | (164) |
| 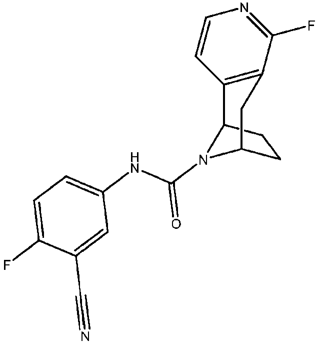 | (165) | 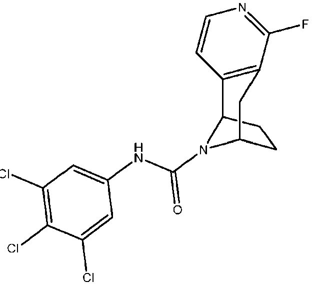 | (166) |
| 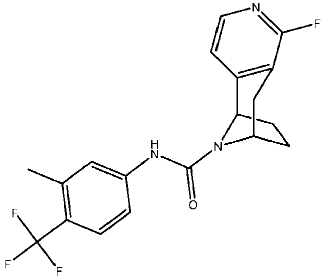 | (167) | 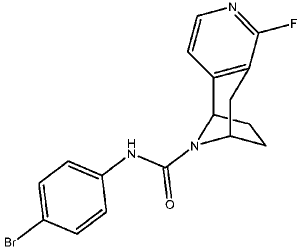 | (168) |
| 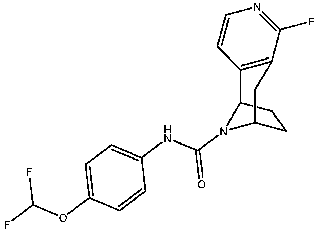 | (169) | 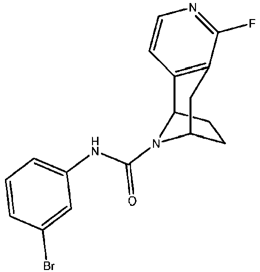 | (170) |
| 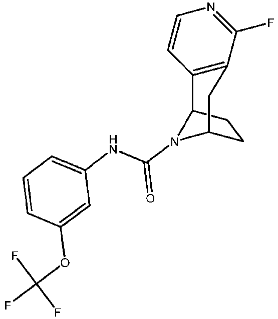 | (171) | 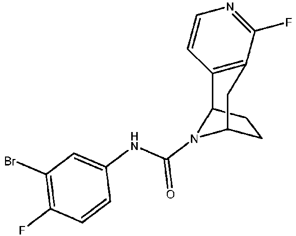 | (172) |
| 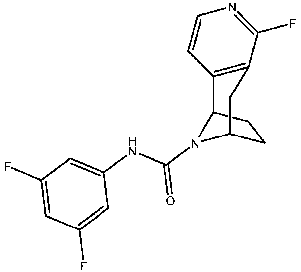 | (173) | 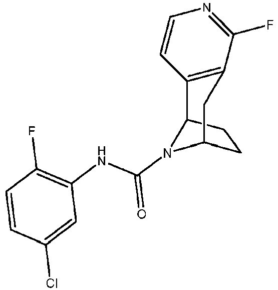 | (174) |
| 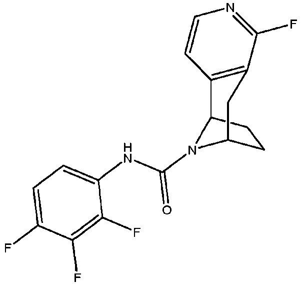 | (175) | 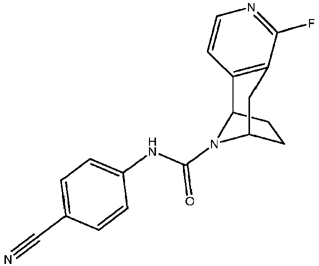 | (176) |
| 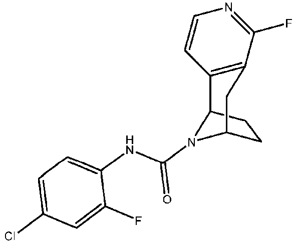 | (177) | 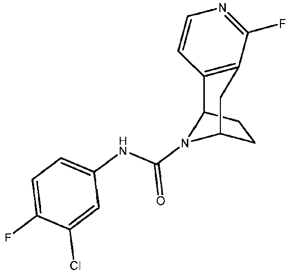 | (178) |
| 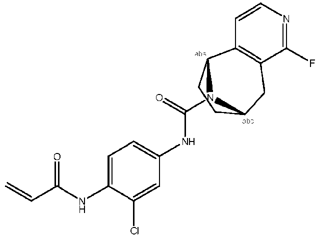 | (179) | 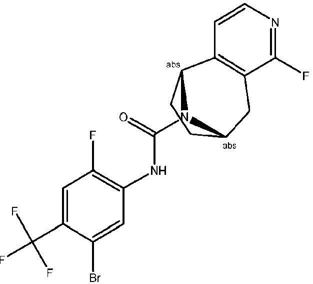 | (180) |
| 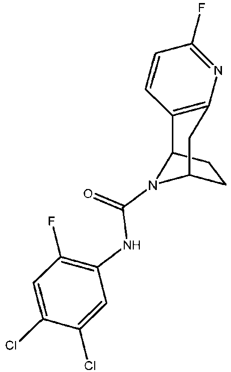 | (181) | 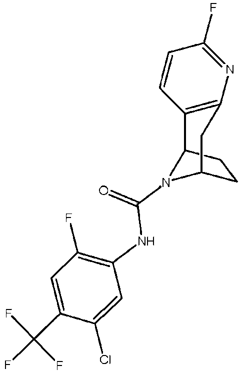 | (182) |
| 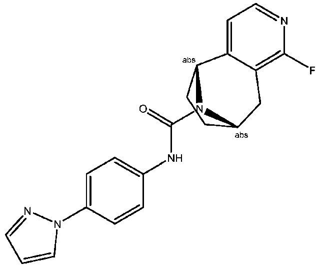 | (183) | 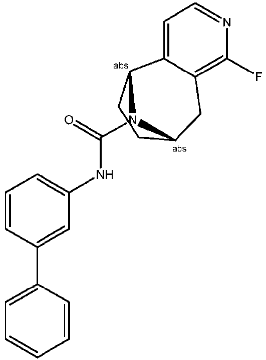 | (184) |
| 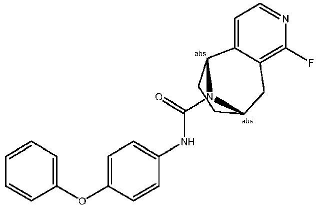 | (185) | 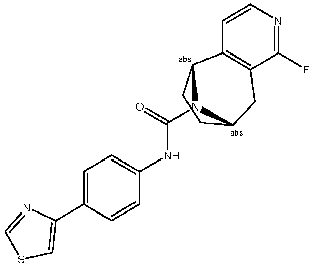 | (186) |
| 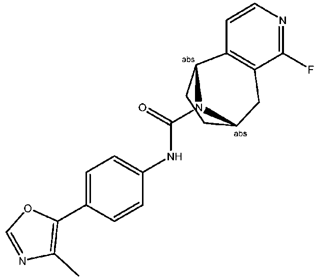 | (187) | 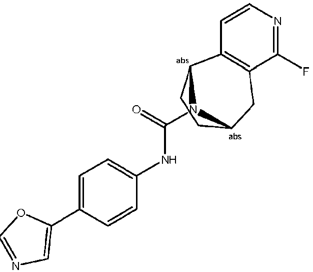 | (188) |
| 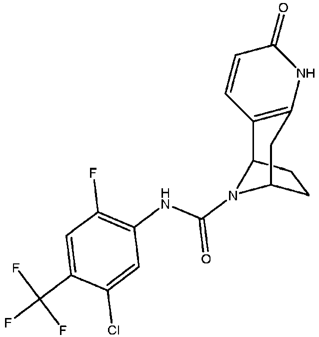 | (189) | 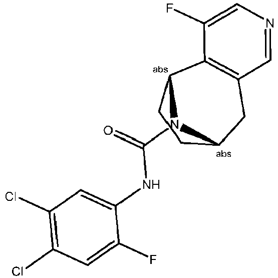 | (190) |
| 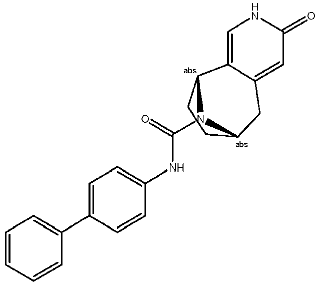 | (191) | 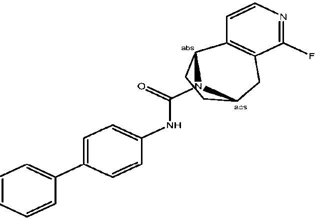 | (192) |
| 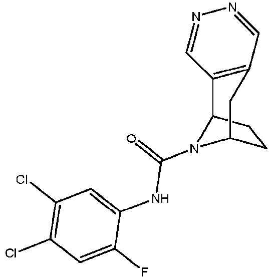 | (193) | 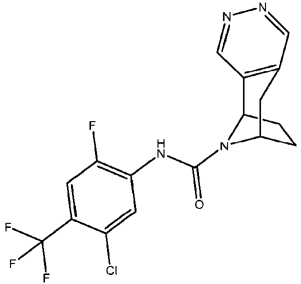 | (194) |
| 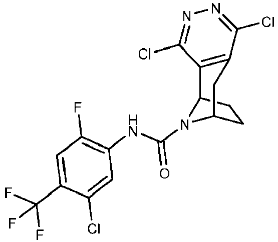 | (195) | 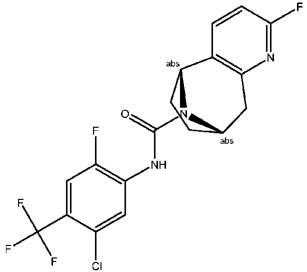 | (196) |
| 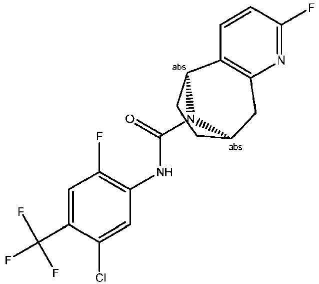 | (197) | 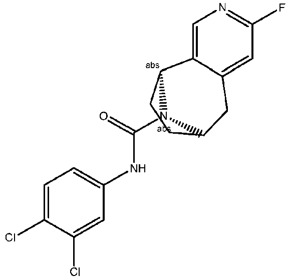 | (198) |
| 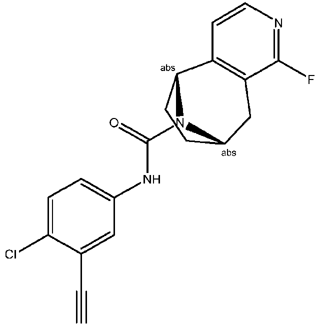 | (199) | 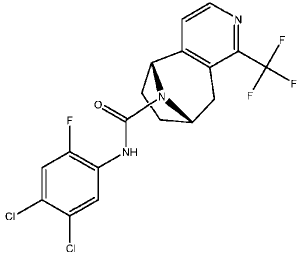 | 200 |
et énantiomères correspondants, et mélanges d'énantiomères correspondants, y compris mélanges racémiques, et sels et solvates pharmaceutiquement acceptables correspondants, pour une utilisation dans le traitement ou la prévention d'un trouble choisi parmi un trouble prolifératif, un trouble auto-immun, l'asthme, une maladie pulmonaire obstructive chronique (COPD) et un syndrome de détresse respiratoire aiguë (ARDS).
